# EUROPEAN PATENT APPLICATION

(11) **EP 4 570 805 A1**
(43) Date of publication of application: **18.06.2025**
(21) Application number: 23851967.2
(22) Date of filing: 11.08.2023
(51) Int. Cl.: C07D 498/16, C07D 471/16, C07D 491/16, A61K 31/551, A61K 31/553, A61K 31/5365, A61K 31/4985, A61P 35/00

(54) **TRICYCLIC COMPOUNDS AND MEDICAL USE THEREOF**

(30) Priority: 12.08.2022 CN 202210967635; 16.01.2023 CN 202310200012; 08.02.2023 CN 202310103403; 17.03.2023 CN 202310264699; 28.07.2023 CN 202310948052; 07.08.2023 CN 202310991185
(71) Applicant: Chia Tai Tianqing Pharmaceutical Group Co., Ltd., Lianyungang, Jiangsu 222062 (CN)
(72) Inventor: LIU, Fei, Lianyungang, Jiangsu 222062 (CN); ZHU, Yizhong, Lianyungang, Jiangsu 222062 (CN); XU, Hongjiang, Lianyungang, Jiangsu 222062 (CN); JIANG, Qiushi, Lianyungang, Jiangsu 222062 (CN); JI, Lei, Lianyungang, Jiangsu 222062 (CN); WANG, Jinan, Lianyungang, Jiangsu 222062 (CN); ZHAO, Wei, Lianyungang, Jiangsu 222062 (CN); WANG, Bin, Lianyungang, Jiangsu 222062 (CN)
(74) Representative: Grünecker Patent- und Rechtsanwälte PartG mbB
(86) International application number: PCT/CN2023/112440
(87) International publication number: WO 2024/032747

(57) **Abstract**

The present application relates to tricyclic compounds and medical use thereof. The structure is represented by formula (I). Specifically, the present application further relates to a preparation method for the compounds, a pharmaceutical composition, and use thereof in the treatment of cancers.

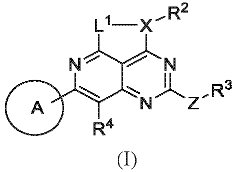

## Description

### THE PRESENT APPLICATION CLAIMS THE FOLLOWING PRIORITIES:

The present application claims 1) the priority and benefit to the Chinese Patent Application No. 202210967635.3 filed with China National Intellectual Property Administration on August 12, 2022, 2) the priority and benefit to the Chinese Patent Application No. 202310200012.8 filed with China National Intellectual Property Administration on January 16, 2023, 3) the priority and benefit to the Chinese Patent Application 202310103403.8 filed with China National Intellectual Property Administration on February 8, 2023, 4) the priority and benefit to the Chinese Patent Application No. 202310264699.1 filed with China National Intellectual Property Administration on March 17, 2023, 5) the priority and benefit to the Chinese Patent Application No. 202310948052.0 filed with China National Intellectual Property Administration on July 28, 2023, and 6) the priority and benefit to the Chinese Patent Application No. 202310991185.6 filed with China National Intellectual Property Administration on August 7, 2023, the contents of which are incorporated herein by reference in their entireties.

### TECHNICAL FIELD

The present disclosure relates to a tricyclic compound, a preparation method therefor, a pharmaceutical composition comprising the compound, and use thereof in the treatment of cancers.

### BACKGROUND

The *Ras* gene is an important proto-oncogene, named after its discovery in the rat sarcoma virus. The Ras protein it encodes is localized on the inner side of the cell membrane. This protein has the ability to bind to GTP/GDP and, with the assistance of GTPase activating protein (GAP), can hydrolyze GTP. By interconverting between its active (GTP-bound) and inactive (GDP-bound) conformations, the Ras protein controls the "on" and "off" states in signal transduction processes involving growth factors and cytokines, playing an important role in cellular processes such as proliferation, differentiation, senescence, and apoptosis (Bos J. L. et al., Cell, 2007, 129(5): 865-877). The human *Ras* gene family has three members: Harvey rat sarcoma viral oncogene homolog *(HRas),* neuroblastoma rat sarcoma viral oncogene homolog *(NRas),* and Kirsten rat sarcoma viral oncogene homolog (*Kras*), with *Kras* being primarily expressed in the intestine, lung, and thymus (Rajalingam K. et al., Biochim Biophys Acta, 2007, 1773(8): 1177-1195).

Studies have shown that *Ras* gene mutations are present in over 30% of human tumors, with *Kras* mutations accounting for about 86% of these cases (Riely G. J. et al., Proc Am Thorac Soc, 2009, 6(2): 201-205). For *Kras* mutations, mutations of glycine at position 12 (G12) account for about 80%, and the G12D mutation (where glycine at position 12 is mutated to aspartic acid) is the primary mutation form of G12 mutations (Prior I. A. et al., Cancer Res, 2012, 72(10): 2457-2467). G12 mutations reduce the catalytic activity of GAP, ultimately leading to the sustained activation of Ras, which makes Ras unable to effectively regulate cell signal transduction, thereby promoting the occurrence and development of tumors.

Currently, Kras (e.g., Kras^{G12D}) has become an attractive anti-cancer target.

### SUMMARY

The present disclosure relates to a compound of formula (I), a stereoisomer thereof, a tautomer thereof, a deuterated compound thereof, or a pharmaceutically acceptable salt thereof, 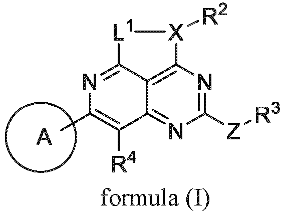 wherein
X is selected from the group consisting of -N- and -CH-, the -CH- optionally substituted with R^{x};
R^{x} is selected from the group consisting of deuterium, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
L¹ is selected from the group consisting of -O-, -S-, and the following groups optionally substituted with one or more R¹: -NH-, C₁₋₅ alkylene, C₁₋₄ heteroalkylene, C₂₋₅ alkenylene, and C₁₋₄ heteroalkenylene;
each R¹ is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R² is selected from the group consisting of H, deuterium, halogen, -OH, -NH₂, -CN, and the following groups optionally substituted with one or more R^{2a}: C₁₋₁₂ alkyl, C₁₋₁₂ heteroalkyl, 3- to 12-membered cycloalkyl-L²-, 4- to 12-membered heterocyclyl-L²-, 6- to 10-membered aryl-L²-, and 5- to 10-membered heteroaryl-L²-;
L² is selected from the group consisting of a single bond, -O-, -S-, -NH-, -N(C₁₋₆ alkyl)-, C₁₋₆ alkylene, C₁₋₆ heteroalkylene, C₂₋₆ alkenylene, and C₁₋₆ heteroalkenylene;
each R^{2a} is independently selected from the group consisting of deuterium, halogen, -OH, oxo, -NH₂, -CN, -C(O)NR^{2c}R^{2d}, -NR^{2c}C(O)R^{2d}, -NHC(O)NR^{2c}R^{2d}, -NR^{2c}C(O)OR^{2d}, -OC(O)R^{2d}, -C(O)OR^{2d}, -OC(O)OR^{2d}, -OC(O)NR^{2c}R^{2d}, -SO₂R^{2d}, -NHSO₂R^{2d}, -C₁₋₆ alkylene C(O)NR^{2c}R^{2d}, -C₁₋₆ alkylene NR^{2c}C(O)R^{2d}, -C₁₋₆ alkylene NHC(O)NR^{2c}R^{2d}, -C₁₋₆ alkylene NR^{2c}C(O)OR^{2d}, -C₁₋₆ alkylene OC(O)R^{2d}, -C₁₋₆ alkylene C(O)OR^{2d}, -C₁₋₆ alkylene OC(O)NR^{2c}R^{2d}, -C₁₋₆ alkylene SO₂R^{2d}, -C₁₋₆ alkylene OSO₂R^{2d}, -C₁₋₆ alkylene NHSO₂R^{2d}, and the following groups optionally substituted with one or more R^{2b}: C₁₋₆ alkyl, C₁₋₆ alkoxy, C₁₋₆ alkylamino, di-C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{2c} is independently selected from the group consisting of H, deuterium, and C₁₋₆ alkyl;
R^{2d} is independently selected from the group consisting of H, deuterium, and the following groups optionally substituted with one or more R^{2e}: C₁₋₆ alkyl, C₁₋₆ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{2e} is independently selected from the group consisting of deuterium, halogen, -OH, oxo, -NH₂, -CN, C₁₄ alkyl, C₁₋₄ alkoxy, C₁₋₄ haloalkyl, and C₁₋₄ haloalkoxy;
each R^{2b} is independently selected from the group consisting of deuterium, halogen, -OH, -NH₂, -CN, C₁₋₄ alkyl, C₁₋₄ alkoxy, C₁₋₄ alkylamino, di-C₁₋₄ alkylamino, C₁₋₄ haloalkyl, and C₁₋₄ haloalkoxy;
Z is selected from the group consisting of a single bond, -S-, -O-, and the following groups optionally substituted with one or more R^{z}: -NH- and -N(C₁₋₁₂ alkyl)-;
each R^{z} is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R³ is selected from the group consisting of H, deuterium, and the following groups optionally substituted with one or more R^{3a}: C₁₋₁₂ alkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3a} is independently selected from the group consisting of deuterium, halogen, -OH, oxo, -NH₂, -CN, -C(O)NR^{3c}R^{3d}, -NR^{3c}C(O)R^{3d}, -NHC(O)NR^{3c}R^{3d}, -NR^{3c}C(O)OR^{3d}, -OC(O)R^{3d}, -C(O)OR^{3d}, -OC(O)OR^{3d}, -OC(O)NR^{3c}R^{3d}, -SO₂R^{3d}, -NHSO₂R^{3d}, -C₁₋₆ alkylene C(O)NR^{3c}R^{3d}, -C₁₋₆ alkylene NR^{3c}C(O)R^{3d}, -C₁₋₆ alkylene NHC(O)NR^{3c}R^{3a}, -C₁₋₆ alkylene NR^{3c}C(O)OR^{3d}, -C₁₋₆ alkylene OC(O)R^{3d}, -C₁₋₆ alkylene C(O)OR^{3d}, -C₁₋₆ alkylene OC(O)NR^{3c}R^{3d}, -C₁₋₆ alkylene SO₂R^{3d}, -C₁₋₆ alkylene OSO₂R^{3d}, -C₁₋₆ alkylene NHSO₂R^{3d}, and the following groups optionally substituted with one or more R^{3b}: =NH, =CH₂, =N(C₁₋₆ alkyl), =CH(C₁₋₆ alkyl), =C(C₁₋₆ alkyl)₂, C₁₋₆ alkyl, C₁₋₆ alkoxy, C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{3c} is independently selected from the group consisting of H, deuterium, and C₁₋₆ alkyl;
R^{3d} is independently selected from the group consisting of H, deuterium, and the following groups optionally substituted with one or more R^{3e}: C₁₋₁₂ alkyl, C₁₋₁₂ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3b} is independently selected from the group consisting of deuterium, halogen, -OH, oxo, -NH₂, -CN, C₁₋₄ alkyl, C₁₋₄ alkoxy, C₁₋₄ alkylamino, and di-C₁₋₄ alkylamino;
each R^{3e} is independently selected from the group consisting of deuterium, halogen, -OH, formyl, oxo, C₁₋₄ alkyl, and C₁₋₄ haloalkyl;
R⁴ is selected from the group consisting of H, deuterium, halogen, -CN, C₁₋₁₂ alkyl, C₁₋₁₂ alkoxy, C₁₋₁₂ haloalkyl, C₁₋₁₂ haloalkoxy, 3- to 12-membered cycloalkyl, 3- to 12-membered heterocyclyl, 6- to 10-membered aryl, and 5-to 10-membered heteroaryl;
ring A is selected from the group consisting of the following groups optionally substituted with one or more R⁵: 3-to 12-membered cycloalkyl, 3- to 12-membered heterocyclyl, 6- to 10-membered aryl, and 5- to 10-membered heteroaryl;
each R⁵ is independently selected from the group consisting of deuterium, halogen, -CN, -OH, -NH₂, and the following groups optionally substituted with one or more R^{5a}: C₁₋₁₂ alkyl, C₂₋₁₂ alkenyl, C₂₋₁₂ alkynyl, C₁₋₁₂ alkoxy, C₁₋₁₂ alkylamino, di-C₁₋₁₂ alkylamino, C₁₋₁₂ alkylthio, 3- to 12-membered cycloalkyl, 6- to 10-membered aryl, 5-to 10-membered heteroaryl, and 3- to 12-membered heterocyclyl;
each R^{5a} is independently selected from the group consisting of deuterium, halogen, -OH, oxo, -NH₂, -CN, C₁₋₄ alkyl, C₁₋₄ alkoxy, C₁₋₄ alkylamino, and di-C₁₋₄ alkylamino;
each L¹, L², R¹, R², R^{2a}, R^{2b} , R^{2c}, R^{2d}, R^{2e}, R³, R^{3a}, R^{3b}, R^{3c}, R^{3d} , R^{3e}, R⁴, R⁵, R^{5a}, R^{x}, or R^{z} is independently optionally substituted with one or more substituents.

In some embodiments, provided is the compound of formula (I), the stereoisomer thereof, the tautomer thereof, or the pharmaceutically acceptable salt thereof,
wherein,
X is selected from the group consisting of -N- and -CH-, the -CH- optionally substituted with R^{x};
R^{x} is selected from the group consisting of halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
L¹ is selected from the group consisting of -O-, -S-, and the following groups optionally substituted with one or more R¹: -NH-, C₁₋₅ alkylene, C₁₋₄ heteroalkylene, C₂₋₅ alkenylene, and C₁₋₄ heteroalkenylene;
each R¹ is independently selected from the group consisting of oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R² is selected from the group consisting of H, halogen, -OH, -NH₂, -CN, and the following groups optionally substituted with one or more R^{2a}: C₁₋₁₂ alkyl, C₁₋₁₂ heteroalkyl, 3- to 12-membered cycloalkyl-L²-, 4- to 12-membered heterocyclyl-L²-, 6- to 10-membered aryl-L²-, and 5- to 10-membered heteroaryl-L²-;
L² is selected from the group consisting of a single bond, -O-, -S-, -NH-, -N(C₁₋₆ alkyl)-, C₁₋₆ alkylene, C₁₋₆ heteroalkylene, C₂₋₆ alkenylene, and C₁₋₆ heteroalkenylene;
each R^{2a} is independently selected from the group consisting of halogen, -OH, oxo, -NH₂, -CN, -C(O)NR^{2c}R^{2d}, -NR^{2c}C(O)R^{2d}, -NHC(O)NR^{2c}R^{2d}, -NR^{2c}C(O)OR^{2d}, -OC(O)R^{2d}, -C(O)OR^{2d}, -OC(O)OR^{2d}, -OC(O)NR^{2c}R^{2d}, -SO₂R^{2d}, -NHSO₂R^{2d}, -C₁₋₆ alkylene C(O)NR^{2c}R^{2d}, -C₁₋₆ alkylene NR^{2c}C(O)R^{2d}, -C₁₋₆ alkylene NHC(O)NR^{2c}R^{2d}, -C₁₋₆ alkylene NR^{2c}C(O)OR^{2d}, -C₁₋₆ alkylene OC(O)R^{2d}, -C₁₋₆ alkylene C(O)OR^{2d}, -C₁₋₆ alkylene OC(O)NR^{2c}R^{2d}, -C₁₋₆ alkylene SO₂R^{2d}, -C₁₋₆ alkylene OSO₂R^{2d}, -C₁₋₆ alkylene NHSO₂R^{2d}, and the following groups optionally substituted with one or more R^{2b}: C₁₋₆ alkyl, C₁₋₆ alkoxy, C₁₋₆ alkylamino, di-C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{2c} is independently selected from the group consisting of H and C₁₋₆ alkyl;
R^{2d} is independently selected from the group consisting of H and the following groups optionally substituted with one or more R^{2e}: C₁₋₆ alkyl, C₁₋₆ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{2e} is independently selected from the group consisting of halogen, -OH, oxo, -NH₂, -CN, C₁₋₄ alkyl, C₁₋₄ alkoxy, C₁₋₄ haloalkyl, and C₁₋₄ haloalkoxy;
each R^{2b} is independently selected from the group consisting of halogen, -OH, -NH₂, -CN, C₁₋₄ alkyl, C₁₋₄ alkoxy, C₁₋₄ alkylamino, di-C₁₋₄ alkylamino, C₁₋₄ haloalkyl, and C₁₋₄ haloalkoxy;
Z is selected from the group consisting of a single bond, -S-, -O-, and the following groups optionally substituted with one or more R^{z}: -NH- and -N(C₁₋₁₂ alkyl)-;
each R^{z} is independently selected from the group consisting of oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R³ is selected from the group consisting of H and the following groups optionally substituted with one or more R^{3a}: C₁₋₁₂ alkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3a} is independently selected from the group consisting of halogen, -OH, oxo, -NH₂, -CN, -C(O)NR^{3c}R^{3d}, -NR^{3c}C(O)R^{3d}, -NHC(O)NR^{3c}R^{3d}, -NR^{3c}C(O)OR^{3d}, -OC(O)R^{3d}, -C(O)OR^{3d}, -OC(O)OR^{3d}, -OC(O)NR^{3c}R^{3d}, -SO₂R^{3d}, -NHSO₂R^{3d}, -C₁₋₆ alkylene C(O)NR^{3c}R^{3a}, -C₁₋₆ alkylene NR^{3c}C(O)R^{3d}, -C₁₋₆ alkylene NHC(O)NR^{3c}R^{3d}, -C₁₆ alkylene NR^{3c}C(O)OR^{3d}, -C₁₋₆ alkylene OC(O)R^{3d}, -C₁₋₆ alkylene C(O)OR^{3d}, -C₁₋₆ alkylene OC(O)NR^{3c}R^{3d}, -C₁₋₆ alkylene SO₂R^{3d}, -C₁₋₆ alkylene OSO₂R^{3d}, -C₁₋₆ alkylene NHSO₂R^{3d}, and the following groups optionally substituted with one or more R^{3b}: =NH, =CH₂, =N(C₁₋₆ alkyl), =CH(C₁₋₆ alkyl), =C(C₁₋₆ alkyl)₂, C₁₋₆ alkyl, C₁₋₆ alkoxy, C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{3c} is independently selected from the group consisting of H and C₁₋₆ alkyl;
R^{3d} is independently selected from the group consisting of H and the following groups optionally substituted with one or more R^{3e}: C₁₋₁₂ alkyl, C₁₋₁₂ heteroalkyl, 3- to 12-membered cycloalkyl, 3- to 12-membered heterocyclyl, 6-to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3b} is independently selected from the group consisting of halogen, -OH, oxo, -NH₂, -CN, C₁₋₄ alkyl, C₁₋₄ alkoxy, C₁₋₄ alkylamino, and di-C₁₋₄ alkylamino;
each R^{3e} is independently selected from the group consisting of halogen, -OH, formyl, oxo, C₁₋₄ alkyl, and C₁₋₄ haloalkyl;
R⁴ is selected from the group consisting of H, halogen, -CN, C₁₋₁₂ alkyl, C₁₋₁₂ alkoxy, C₁₋₁₂ haloalkyl, C₁₋₁₂ haloalkoxy, 3- to 12-membered cycloalkyl, 3- to 12-membered heterocyclyl, 6- to 10-membered aryl, and 5- to 10-membered heteroaryl;
ring A is selected from the group consisting of the following groups optionally substituted with one or more R⁵: 3-to 12-membered cycloalkyl, 3- to 12-membered heterocyclyl, 6- to 10-membered aryl, and 5- to 10-membered heteroaryl;
each R⁵ is independently selected from the group consisting of halogen, -CN, -OH, -NH₂, and the following groups optionally substituted with one or more R^{5a}: C₁₋₁₂ alkyl, C₂₋₁₂ alkenyl, C₂₋₁₂ alkynyl, C₁₋₁₂ alkoxy, C₁₋₁₂ alkylamino, di-C₁₋₁₂ alkylamino, C₁₋₁₂ alkylthio, 3- to 12-membered cycloalkyl, 6- to 10-membered aryl, 5- to 10-membered heteroaryl, and 3- to 12-membered heterocyclyl;
each R^{5a} is independently selected from the group consisting of halogen, -OH, oxo, -NH₂, -CN, C₁₋₄ alkyl, C₁₋₄ alkoxy, C₁₋₄ alkylamino, and di-C₁₋₄ alkylamino;
each L¹, L², R¹, R², R^{2a}, R^{2b} , R^{2c}, R^{2d}, R^{2e}, R³, R^{3a}, R^{3b}, R^{3c}, R^{3d} , R^{3e}, R⁴, R⁵, R^{5a}, R^{x}, or R^{z} is independently optionally substituted with one or more substituents.

In some embodiments, provided is the compound of formula (I), the stereoisomer thereof, the tautomer thereof, or the pharmaceutically acceptable salt thereof,
wherein,
X is selected from the group consisting of -N- and -CH-, the -CH- optionally substituted with R^{x};
R^{x} is selected from the group consisting of halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
L¹ is selected from the group consisting of -O-, -S-, and the following groups optionally substituted with one or more R¹: -NH-, C₁₋₅ alkylene, C₁₋₄ heteroalkylene, C₂₋₅ alkenylene, and C₁₋₄ heteroalkenylene;
each R¹ is independently selected from the group consisting of oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R² is selected from the group consisting of H, halogen, -OH, -NH₂, -CN, and the following groups optionally substituted with one or more R^{2a}: C₁₋₁₂ alkyl, C₁₋₁₂ heteroalkyl, 3- to 12-membered cycloalkyl-L²-, 4- to 12-membered heterocyclyl-L²-, 6- to 10-membered aryl-L²-, and 5- to 10-membered heteroaryl-L²-;
L² is selected from the group consisting of a single bond, -O-, -S-, -NH-, -N(C₁₋₆ alkyl)-, C₁₋₆ alkylene, C₁₋₆ heteroalkylene, C₂₋₆ alkenylene, and C₁₋₆ heteroalkenylene;
each R^{2a} is independently selected from the group consisting of halogen, -OH, oxo, -NH₂, -CN, -C(O)NR^{2c}R^{2d}, -NR^{2c}C(O)R^{2d}, -NHC(O)NR^{2c}R^{2d}, -NR^{2c}C(O)OR^{2d}, -OC(O)R^{2d}, -C(O)OR^{2d}, -OC(O)OR^{2d}, -OC(O)NR^{2c}R^{2d}, -SO₂R^{2d}, -NHSO₂R^{2d}, -C₁₋₆ alkylene C(O)NR^{2c}R^{2d}, -C₁₋₆ alkylene NR^{2c}C(O)R^{2d}, -C₁₋₆ alkylene NHC(O)NR^{2c}R^{2d}, -C₁₋₆ alkylene NR^{2c}C(O)OR^{2d}, -C₁₋₆ alkylene OC(O)R^{2d}, -C₁₋₆ alkylene C(O)OR^{2d}, -C₁₋₆ alkylene OC(O)NR^{2c}R^{2d}, -C₁₋₆ alkylene SO₂R^{2d}, -C₁₋₆ alkylene OSO₂R^{2d}, -C₁₋₆ alkylene NHSO₂R^{2d}, and the following groups optionally substituted with one or more R^{2b}: C₁₋₆ alkyl, C₁₋₆ alkoxy, C₁₋₆ alkylamino, di-C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{2c} is independently selected from the group consisting of H and C₁₋₆ alkyl;
R^{2d} is independently selected from the group consisting of H and the following groups optionally substituted with one or more R^{2e}: C₁₋₆ alkyl, C₁₋₆ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{2e} is independently selected from the group consisting of halogen, -OH, oxo, -NH₂, -CN, C₁₋₄ alkyl, C₁₋₄ alkoxy, C₁₋₄ haloalkyl, and C₁₋₄ haloalkoxy;
each R^{2b} is independently selected from the group consisting of halogen, -OH, -NH₂, -CN, C₁₋₄ alkyl, C₁₋₄ alkoxy, C₁₋₄ alkylamino, di-C₁₋₄ alkylamino, C₁₋₄ haloalkyl, and C₁₋₄ haloalkoxy;
Z is selected from the group consisting of a single bond, -S-, -O-, and the following groups optionally substituted with one or more R^{z}: -NH- and -N(C₁₋₁₂ alkyl)-;
each R^{z} is independently selected from the group consisting of oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R³ is selected from the group consisting of H and the following groups optionally substituted with one or more R^{3a}: C₁₋₁₂ alkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3a} is independently selected from the group consisting of halogen, -OH, oxo, -NH₂, -CN, -C(O)NR^{3c}R^{3d}, -NR^{3c}C(O)R^{3d}, -NHC(O)NR^{3c}R^{3d}, -NR^{3c}C(O)OR^{3d}, -OC(O)R^{3d}, -C(O)OR^{3d}, -OC(O)OR^{3d}, -OC(O)NR^{3c}R^{3d}, -SO₂R^{3d}, -NHSO₂R^{3d}, -C₁₋₆ alkylene C(O)NR^{3c}R^{3a}, -C₁₋₆ alkylene NR^{3c}C(O)R^{3d}, -C₁₋₆ alkylene NHC(O)NR^{3c}R^{3d}, -C₁₋₆ alkylene NR^{3c}C(O)OR^{3d}, -C₁₋₆ alkylene OC(O)R^{3d}, -C₁₋₆ alkylene C(O)OR^{3d}, -C₁₋₆ alkylene OC(O)NR^{3c}R^{3d}, -C₁₋₆ alkylene SO₂R^{3d}, -C₁₋₆ alkylene OSO₂R^{3d}, -C₁₋₆ alkylene NHSO₂R^{3d}, and the following groups optionally substituted with one or more R^{3b}: C₁₋₆ alkyl, C₁₋₆ alkoxy, C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{3c} is independently selected from the group consisting of H and C₁₋₆ alkyl;
R^{3d} is independently selected from the group consisting of H and the following groups optionally substituted with one or more R^{3e}: C₁₋₁₂ alkyl, C₁₋₁₂ heteroalkyl, 3- to 12-membered cycloalkyl, 3- to 12-membered heterocyclyl, 6-to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3b} is independently selected from the group consisting of halogen, -OH, -NH₂, -CN, C₁₋₄ alkyl, C₁₋₄ alkoxy, C₁₋₄ alkylamino, and di-C₁₋₄ alkylamino;
each R^{3e} is independently selected from the group consisting of halogen, -OH, formyl, oxo, C₁₋₄ alkyl, and C₁₋₄ haloalkyl;
R⁴ is selected from the group consisting of H, halogen, -CN, C₁₋₁₂ alkyl, C₁₋₁₂ alkoxy, C₁₋₁₂ haloalkyl, C₁₋₁₂ haloalkoxy, 3- to 12-membered cycloalkyl, 3- to 12-membered heterocyclyl, 6- to 10-membered aryl, and 5- to 10-membered heteroaryl;
ring A is selected from the group consisting of the following groups optionally substituted with one or more R⁵: 3-to 12-membered cycloalkyl, 3- to 12-membered heterocyclyl, 6- to 10-membered aryl, and 5- to 10-membered heteroaryl;
each R⁵ is independently selected from the group consisting of halogen, -CN, -OH, -NH₂, and the following groups optionally substituted with one or more R^{5a}: C₁₋₁₂ alkyl, C₂₋₁₂ alkenyl, C₂₋₁₂ alkynyl, C₁₋₁₂ alkoxy, C₁₋₁₂ alkylamino, di-C₁₋₁₂ alkylamino, C₁₋₁₂ alkylthio, 3- to 12-membered cycloalkyl, 6- to 10-membered aryl, 5- to 10-membered heteroaryl, and 3- to 12-membered heterocyclyl;
each R^{5a} is independently selected from the group consisting of halogen, -OH, -NH₂, -CN, C₁₋₄ alkyl, C₁₋₄ alkoxy, C₁₋₄ alkylamino, and di-C₁₋₄ alkylamino;
each L¹, L², R¹, R², R^{2a}, R^{2b} , R^{2c}, R^{2d}, R^{2e}, R³, R^{3a}, R^{3b}, R^{3c}, R^{3d} , R^{3e}, R⁴, R⁵, R^{5a}, R^{x}, or R^{z} is independently optionally substituted with one or more substituents.

In some embodiments, R^{x} is selected from deuterium.

In some embodiments, R^{x} is selected from the group consisting of halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, R^{x} is selected from the group consisting of halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, and C₁₋₆ haloalkoxy.

In some embodiments, R^{x} is selected from the group consisting of halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy.

In some embodiments, R^{x} is selected from the group consisting of F, Cl, Br, -OH, -NH₂, -CN, C₁₋₃ alkyl, C₁₋₃ alkoxy, C₁₋₃ haloalkyl, and C₁₋₃ haloalkoxy.

In some embodiments, R^{x} is selected from the group consisting of F, Cl, Br, -OH, -NH₂, -CN, methyl, ethyl, isopropyl, methoxy, ethoxy, isopropoxy, trifluoromethyl, and trifluoromethoxy.

In some embodiments, X is selected from the group consisting of -N-, -CH-, -C(C₁₋₆ alkyl)-, and -C(C₁₋₆ haloalkyl)-.

In some embodiments, X is selected from the group consisting of -N-, -CH-, -C(C₁₋₃ alkyl)-, and -C(C₁₋₃ haloalkyl)-.

In some embodiments, X is selected from -N-.

In some embodiments, L¹ is selected from the group consisting of -O-, -S-, and the following groups optionally substituted with one or more R¹: -NH-, C₁₋₅ alkylene, C₁₋₄ heteroalkylene, C₂₋₅ alkenylene, and C₂₋₄ heteroalkenylene.

In some embodiments, L¹ is selected from the group consisting of -O- and the following groups optionally substituted with one or more R¹: -NH-, C₂₋₄ alkylene, C₁₋₃ heteroalkylene, C₂₋₄ alkenylene, and C₁₋₃ heteroalkenylene.

In some embodiments, L¹ is selected from the group consisting of -O- and the following groups optionally substituted with one or more R¹: -NH-, C₂₋₄ alkylene, C₁₋₃ heteroalkylene, C₂₋₄ alkenylene, and C₂₋₃ heteroalkenylene.

In some embodiments, L¹ is selected from the group consisting of -O- and the following groups optionally substituted with one or more R¹: -NH-, C₂₋₄ alkylene, and C₁₋₃ heteroalkylene.

In some embodiments, L¹ is selected from the group consisting of -O- and the following groups optionally substituted with one or more R¹: -NH-, -CH₂CH₂-, -(CH₂)₃-, -(CH₂)₄-, -OCH₂-, -CH₂O-, -OCH₂CH₂-, -CH₂OCH₂-, -CH₂CH₂O-, -O(CH₂)₃-, -CH₂OCH₂CH₂-, -CH₂CH₂OCH₂-, -CH₂CH₂CH₂O-, -NHCH₂-, -NHCH₂CH₂-, -CH₂NHCH₂-, -CH₂CH₂NH-, -NH(CH₂)₃-, -CH₂NHCH₂CH₂-, -CH₂CH₂NHCH₂-, and -CH₂CH₂CH₂NH-.

In some embodiments, L¹ is selected from the group consisting of -O- and the following groups optionally substituted with one or more R¹: -NH-, -CH₂CH₂-, -(CH₂)₃-, -(CH₂)₄-, -OCH₂-, -OCH₂CH₂-, -O(CH₂)₃-, -NHCH₂-, -NHCH₂CH₂-, and -NH(CH₂)₃-,

In some embodiments, L¹ is selected from the group consisting of -O-, -NH-, -N(C₁₋₃ alkyl)-, and the following groups optionally substituted with one or more R¹: -CH₂CH₂-, -(CH₂)₃-, -(CH₂)₄-, -OCH₂-, -OCH₂CH₂-, -O(CH₂)₃-, -NHCH₂-, -NHCH₂CH₂-, and -NH(CH₂)₃-.

In some embodiments, L¹ is selected from the group consisting of -O-, -NH-, -N(CH₃)-, and the following groups optionally substituted with one or more R¹: -CH₂CH₂-, -(CH₂)₃-, -(CH₂)₄-, -OCH₂-, -OCH₂CH₂-, -O(CH₂)₃-, -NHCH₂-, -NHCH₂CH₂-, and -NH(CH₂)₃-.

In some embodiments, L¹ is selected from the group consisting of the following groups optionally substituted with one or more R¹: -(CH₂)₃-, -OCH₂CH₂-, and -NHCH₂CH₂-.

In some embodiments, L¹ is selected from -OCH₂CH₂- optionally substituted with one or more R¹.

In some embodiments, each R¹ may further be selected from deuterium.

In some embodiments, each R¹ is independently selected from the group consisting of oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, each R¹ is independently selected from the group consisting of oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, and C₁₋₆ haloalkoxy.

In some embodiments, each R¹ is independently selected from the group consisting of oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy.

In some embodiments, each R¹ is independently selected from the group consisting of F, Cl, Br, -OH, oxo, -NH₂, -CN, C₁₋₃ alkyl, C₁₋₃ alkoxy, C₁₋₃ haloalkyl, and C₁₋₃ haloalkoxy.

In some embodiments, each R¹ is independently selected from the group consisting of F, Cl, Br, -OH, oxo, -NH₂, -CN, methyl, ethyl, isopropyl, methoxy, ethoxy, isopropoxy, trifluoromethyl, and trifluoromethoxy. In some embodiments, each R¹ is independently selected from -CH₂F.

In some embodiments, each R¹ is independently selected from the group consisting of methyl, ethyl, isopropyl, trifluoromethyl, and -CH₂F.

In some embodiments, R² is selected from deuterium.

In some embodiments, R² is selected from the group consisting of H, deuterium, halogen, -OH, -NH₂, -CN, and the following groups optionally substituted with one or more R^{2a}: C₁₋₆ alkyl, C₁₋₆ heteroalkyl, 3- to 10-membered cycloalkyl-L²-, 4- to 10-membered heterocyclyl-L²-, 6- to 10-membered aryl-L²-, and 5- to 10-membered heteroaryl-L²-.

In some embodiments, R² is selected from the group consisting of H, halogen, -OH, -NH₂, -CN, and the following groups optionally substituted with one or more R^{2a}: C₁₋₆ alkyl, C₁₋₆ heteroalkyl, 3- to 10-membered cycloalkyl-L²-, 4- to 10-membered heterocyclyl-L²-, 6- to 10-membered aryl-L²-, and 5- to 10-membered heteroaryl-L²-.

In some embodiments, R² is selected from the group consisting of H, halogen, -OH, -NH₂, -CN, and the following groups optionally substituted with one or more R^{2a}: C₁₋₄ alkyl, C₁₋₄ heteroalkyl, 3- to 10-membered cycloalkyl-L²-, 4- to 10-membered heterocyclyl-L²-, phenyl-L²-, naphthyl-L²-, 5- to 6-membered heteroaryl-L²-, and benzo 5- to 6-membered heteroaryl-L²-.

In some embodiments, R² is selected from the group consisting of H, F, Cl, Br, -OH, -NH₂, -CN, and the following groups optionally substituted with one or more R^{2a}: C₁₋₄ alkyl, C₁₋₄ heteroalkyl, 3- to 8-membered cycloalkyl-L²-, 4- to 8-membered heterocyclyl-L²-, phenyl-L²-, naphthyl-L²-, 5- to 6-membered heteroaryl-L²-, benzo 5- to 6-membered heterocyclyl-L²-, and benzo 5- to 6-membered heteroaryl-L²-. In some embodiments, R² is selected from 5- to 6-membered heteroaryl-fused 5- to 6-membered cycloalkenyl-L²- optionally substituted with one or more R^{2a}.

In some embodiments, R² is selected from the group consisting of H, deuterium, F, Cl, Br, -OH, -NH₂, -CN, and the following groups optionally substituted with one or more R^{2a}: C₁₋₄ alkyl, C₁₋₄ heteroalkyl, 3- to 8-membered cycloalkyl-L²-, 4- to 8-membered heterocycloalkyl-L²-, phenyl-L²-, naphthyl-L²-, 5- to 6-membered heteroaryl-L²-, benzo 5- to 6-membered heterocyclyl-L²-, benzo 5- to 6-membered heteroaryl-L²-, and 5- to 6-membered heteroaryl-fused 5- to 6-membered cycloalkenyl-L²-.

In some embodiments, R² is selected from the group consisting of H, F, Cl, Br, -OH, -NH₂, -CN, and the following groups optionally substituted with one or more R^{2a}: C₁₋₄ alkyl, C₁₋₄ heteroalkyl, 3- to 8-membered cycloalkyl-L²-, 4- to 8-membered heterocycloalkyl-L²-, phenyl-L²-, naphthyl-L²-, 5- to 6-membered heteroaryl-L²-, benzo 5- to 6-membered heterocyclyl-L²-, and benzo 5- to 6-membered heteroaryl-L²-. In some embodiments, R² is selected from 5- to 6-membered heteroaryl-fused 5- to 6-membered cycloalkenyl-L²- optionally substituted with one or more R^{2a}.

In some embodiments, R² is selected from the group consisting of the following groups optionally substituted with one or more R^{2a}: C₁₋₄ alkyl, 3- to 8-membered cycloalkyl-L²-, 4- to 8-membered heterocyclyl-L²-, phenyl-L²-, and 5- to 6-membered heteroaryl-L²-. In some embodiments, R² is selected from the group consisting of the following groups optionally substituted with one or more R^{2a}: C₁₋₄ alkyl and 5- to 6-membered heteroaryl-L²-.

In some embodiments, L² is selected from the group consisting of a single bond, -O-, -S-, -NH-, -N(C₁₋₆ alkyl)-, C₁₋₆ alkylene, C₁₋₆ heteroalkylene, C₂₋₆ alkenylene, and C₂₋₆ heteroalkenylene.

In some embodiments, L² is selected from the group consisting of a single bond, -O-, -S-, -NH-, -N(C₁₋₆ alkyl)-, C₁₋₆ alkylene, and C₁₋₆ heteroalkylene.

In some embodiments, L² is selected from the group consisting of a single bond, -O-, -NH-, -N(C₁₋₃ alkyl)-, C₁₋₄ alkylene, and C₁₋₄ heteroalkylene.

In some embodiments, L² is selected from the group consisting of a single bond, -O-, -NH-, -N(C₁₋₃ alkyl)-, C₁₋₄ alkylene, -O-C₁₋₄ alkylene, and -NH-C₁₋₄ alkylene.

In some embodiments, L² is selected from the group consisting of a single bond and methylene.

In some embodiments, L² is selected from -CH(CH₃)-.

In some embodiments, R² is selected from the group consisting of H, deuterium, F, Cl, Br, -OH, -NH₂, -CN, and the following groups optionally substituted with one or more R^{2a}: methyl, ethyl, n-propyl, isopropyl, *tert*-butyl, n-butyl, isobutyl, sec-butyl, C₁₋₄ heteroalkyl, cyclopropanyl, cyclobutanyl, cyclopentanyl, 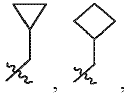 oxetanyl, azetidinyl, pyrrolidinyl, piperidinyl, 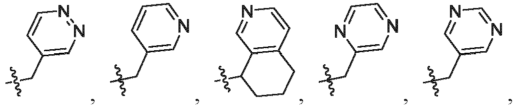 and pyrazolyl. In some embodiments, R² is selected from the group consisting of H, F, Cl, Br, -OH, -NH₂, -CN, and the following groups optionally substituted with one or more R^{2a}: methyl, ethyl, n-propyl, isopropyl, C₁₋₄ heteroalkyl, cyclopropanyl, cyclobutanyl, cyclopentanyl, 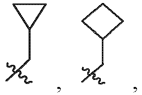 oxetanyl, azetidinyl, pyrrolidinyl, piperidinyl, 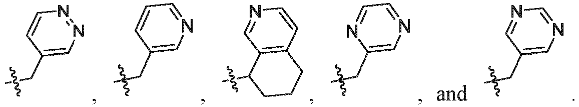 In some embodiments, R² is selected from *tert*-butyl optionally substituted with one or more R^{2a}. In some embodiments, R² is selected from the group consisting of the following groups optionally substituted with one or more R^{2a}: n-butyl, isobutyl, sec-butyl, and pyrazolyl.

In some embodiments, R² is selected from the group consisting of the following groups optionally substituted with one or more R^{2a}: methyl, ethyl, *n*-propyl, isopropyl, *tert-*butyl*, n*-butyl, isobutyl, *sec*-butyl, cyclopropanyl, 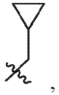 and

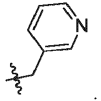

In some embodiments, R² is selected from 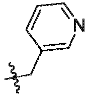 optionally substituted with one or more R^{2a}.

In some embodiments, the position of R^{2a} substitution is a carbon atom or a nitrogen atom in R².

In some embodiments, the position of R^{2a} substitution is a carbon atom in R².

In some embodiments, R^{2a} is independently selected from the group consisting of deuterium, halogen, -OH, oxo, -NH₂, -CN, -C(O)NR^{2c}R^{2d}, -NR^{2c}C(O)R^{2d}, -NHC(O)NR^{2c}R ^{2d}, -NR^{2c}C(O)OR^{2d}, -OC(O)R^{2d}, -C(O)OR^{2d}, -OC(O)OR^{2d}, -OC(O)NR^{2c}R^{2d}, -SO₂R^{2d}, -NHSO₂R^{2d}, -C₁₋₃ alkylene C(O)NR^{2c}R^{2d}, -C₁₋₃ alkylene NR^{2c}C(O)R^{2d}, -C₁₋₃ alkylene NHC(O)NR^{2c}R^{2d}, -C₁₋₃ alkylene NR^{2c}C(O)OR^{2d}, -C₁₋₃ alkylene OC(O)R^{2d}, -C₁₋₃ alkylene C(O)OR^{2d}, -C₁₋₃ alkylene OC(O)NR^{2c}R^{2d}, -C₁₋₃ alkylene SO₂R^{2d}, -C₁₋₃ alkylene OSO₂R^{2d}, -C₁₋₃ alkylene NHSO₂R^{2d}, and the following groups optionally substituted with one or more R^{2b}: C₁₋₄ alkyl, C₁₋₄ alkoxy, C₁₋₄ alkylamino, di-C₁₋₄ alkylamino, 3- to 6-membered cycloalkyl, 4- to 7-membered heterocyclyl, phenyl, 5- to 6-membered heteroaryl, 3- to 6-membered cycloalkyl C₁₋₃ alkylene, 4- to 7-membered heterocyclyl C₁₋₃ alkylene, phenyl C₁₋₃ alkylene, and 5- to 6-membered heteroaryl C₁₋₃ alkylene.

In some embodiments, R^{2a} may further be selected from deuterium.

In some embodiments, R^{2a} is independently selected from the group consisting of halogen, -OH, oxo, -NH₂, -CN, -C(O)NR^{2c}R^{2d}, -NR^{2c}C(O)R^{2d}, -NHC(O)NR^{2c}R^{2d}, -NR^{2c}C(O)OR^{2d}, -OC(O)R^{2d}, -C(O)OR^{2d}, -OC(O)OR^{2d}, -OC(O)NR^{2c}R^{2d}, -SO₂R^{2d}, -NHSO₂R^{2d}, -C₁₋₃ alkylene C(O)NR^{2c}R^{2d}, -C₁₋₃ alkylene NR^{2c}C(O)R^{2d}, -C₁₋₃ alkylene NHC(O)NR^{2c}R^{2d}, -C₁₋₃ alkylene NR^{2c}C(O)OR^{2d}, -C₁₋₃ alkylene OC(O)R^{2d}, -C₁₋₃ alkylene C(O)OR^{2d}, -C₁₋₃ alkylene OC(O)NR^{2c}R^{2d}, -C₁₋₃ alkylene SO₂R^{2d}, -C₁₋₃ alkylene OSO₂R^{2d}, -C₁₋₃ alkylene NHSO₂R^{2d}, and the following groups optionally substituted with one or more R^{2b}: C₁₋₄ alkyl, C₁₋₄ alkoxy, C₁₋₄ alkylamino, di-C₁₋₄ alkylamino, 3- to 6-membered cycloalkyl, 4- to 7-membered heterocyclyl, phenyl, 5- to 6-membered heteroaryl, 3- to 6-membered cycloalkyl C₁₋₃ alkylene, 4- to 7-membered heterocyclyl C₁₋₃ alkylene, phenyl C₁₋₃ alkylene, and 5- to 6-membered heteroaryl C₁₋₃ alkylene.

In some embodiments, R^{2a} is independently selected from the group consisting of halogen, -OH, oxo, -NH₂, -CN, -C(O)NR^{2c}R^{2d}, -NR^{2c}C(O)R^{2d}, -NHC(O)NR^{2c}R^{2d}, -NR^{2c}C(O)OR^{2d}, -OC(O)R^{2d}, -C(O)OR^{2d}, -OC(O)OR^{2d}, -OC(O)NR^{2c}R^{2d}, -C₁₋₃ alkylene C(O)NR^{2c}R^{2d}, -C₁₋₃ alkylene NR^{2c}C(O)R^{2d}, -C₁₋₃ alkylene NHC(O)NR^{2c}R^{2d}, -C₁₋₃ alkylene NR^{2c}C(O)OR^{2d}, -C₁₋₃ alkylene OC(O)R^{2d}, -C₁₋₃ alkylene C(O)OR^{2d}, -C₁₋₃ alkylene OC(O)NR^{2c}R^{2d}, and the following groups optionally substituted with one or more R^{2b}: C₁₋₄ alkyl, C₁₋₄ alkoxy, C₁₋₄ alkylamino, di-C₁₋₄ alkylamino, 3- to 6-membered cycloalkyl, 4- to 7-membered heterocyclyl, phenyl, 5- to 6-membered heteroaryl, 3- to 6-membered cycloalkyl C₁₋₃ alkylene, 4- to 7-membered heterocyclyl C₁₋₃ alkylene, phenyl C₁₋₃ alkylene, and 5- to 6-membered heteroaryl C₁₋₃ alkylene.

In some embodiments, R^{2a} is independently selected from the group consisting of halogen, -OH, oxo, -NH₂, -CN, -C(O)NR^{2c}R^{2d}, -NR^{2c}C(O)R^{2d}, -OC(O)R^{2d}, -C(O)OR^{2d}, -C₁₋₃ alkylene C(O)NR^{2c}R^{2d}, -C₁₋₃ alkylene NR^{2c}C(O)R^{2d}, -C₁₋₃ alkylene OC(O)R^{2d}, -C₁₋₃ alkylene C(O)OR^{2d}, and the following groups optionally substituted with one or more R^{2b}: C₁₋₄ alkyl, C₁₋₄ alkoxy, C₁₋₄ alkylamino, di-C₁₋₄ alkylamino, 3- to 6-membered cycloalkyl, 4- to 7-membered heterocyclyl, phenyl, 5- to 6-membered heteroaryl, 3- to 6-membered cycloalkyl C₁₋₃ alkylene, 4- to 7-membered heterocyclyl C₁₋₃ alkylene, phenyl C₁₋₃ alkylene, and 5- to 6-membered heteroaryl C₁₋₃ alkylene.

In some embodiments, R^{2a} is independently selected from the group consisting of halogen, -OH, oxo, -NH₂, -CN, -C(O)NR^{2c}R^{2d}, -NR^{2c}C(O)R^{2d}, -OC(O)R^{2d}, -C(O)OR^{2d}, and the following groups optionally substituted with one or more R^{2b}: C₁₋₄ alkyl, C₁₋₄ alkoxy, C₁₋₄ alkylamino, di-C₁₋₄ alkylamino, 3- to 6-membered cycloalkyl, 4- to 7-membered heterocycloalkyl, phenyl, 5- to 6-membered heteroaryl, 3- to 6-membered cycloalkyl C₁₋₃ alkylene, 4- to 7-membered heterocycloalkyl C₁₋₃ alkylene, phenyl C₁₋₃ alkylene, and 5- to 6-membered heteroaryl C₁₋₃ alkylene.

In some embodiments, R^{2a} is independently selected from the group consisting of F, Cl, Br, -OH, oxo, -NH₂, -CN, -C(O)NR^{2c}R^{2d}, -NR^{2c}C(O)R^{2d}, -OC(O)R^{2d}, -C(O)OR^{2d}, and the following groups optionally substituted with one or more R^{2b}: methyl, ethyl, isopropyl, methoxy, ethoxy, isopropoxy, methylamino, ethylamino, isopropylamino, dimethylamino, diethylamino, 3- to 6-membered cycloalkyl, 4- to 7-membered heterocycloalkyl, phenyl, 5- to 6-membered heteroaryl, 3- to 6-membered cycloalkyl C₁₋₃ alkylene, 4- to 7-membered heterocycloalkyl C₁₋₃ alkylene, phenyl C₁₋₃ alkylene, and 5- to 6-membered heteroaryl C₁₋₃ alkylene.

In some embodiments, R^{2a} is independently selected from the group consisting of deuterium, F, -OH, -NH₂, methyl, methylamino, dimethylamino, and cyclopropanyl.

In some embodiments, R^{2a} is independently selected from the group consisting of F, -OH, -NH₂, methyl, methylamino, dimethylamino, and cyclopropanyl.

In some embodiments, R^{2a} is independently selected from the group consisting of -NH₂, methyl, methylamino, and cyclopropanyl.

In some embodiments, R^{2a} is independently selected from the group consisting of F, -OH, and dimethylamino.

In some embodiments, R^{2a} is independently selected from the group consisting of F, -NH₂, and methyl. In some embodiments, R^{2a} is independently selected from the group consisting of -NH₂ and methyl. In some embodiments, R^{2a} is independently selected from -NH₂.

In some embodiments, R^{2c} is independently selected from the group consisting of H, deuterium, and C₁₋₃ alkyl.

In some embodiments, R^{2c} may further be selected from deuterium.

In some embodiments, R^{2c} is independently selected from the group consisting of H and C₁₋₃ alkyl.

In some embodiments, R^{2c} is independently selected from the group consisting of H and methyl.

In some embodiments, R^{2d} is independently selected from the group consisting of H, deuterium, and the following groups optionally substituted with one or more R^{2e}: C₁₋₆ alkyl, C₁₋₆ heteroalkyl, 3- to 10-membered cycloalkyl, 3-to 10-membered heterocyclyl, 6- to 10-membered aryl, 5- to 10-membered heteroaryl, 3- to 10-membered cycloalkyl C₁₋₃ alkylene, 3- to 10-membered heterocyclyl C₁₋₃ alkylene, 6- to 10-membered aryl C₁₋₃ alkylene, and 5- to 10-membered heteroaryl C₁₋₃ alkylene.

In some embodiments, R^{2d} may further be selected from deuterium.

In some embodiments, R^{2d} is independently selected from the group consisting of H and the following groups optionally substituted with one or more R^{2e}: C₁₋₆ alkyl, C₁₋₆ heteroalkyl, 3- to 10-membered cycloalkyl, 3- to 10-membered heterocyclyl, 6- to 10-membered aryl, 5- to 10-membered heteroaryl, 3- to 10-membered cycloalkyl C₁₋₃ alkylene, 3- to 10-membered heterocyclyl C₁₋₃ alkylene, 6- to 10-membered aryl C₁₋₃ alkylene, and 5- to 10-membered heteroaryl C₁₋₃ alkylene.

In some embodiments, R^{2d} is independently selected from the group consisting of H and the following groups optionally substituted with one or more R^{2e}: C₁₋₆ alkyl, C₁₋₆ heteroalkyl, 3- to 10-membered cycloalkyl, 3- to 10-membered heterocyclyl, 6- to 10-membered aryl, 5- to 10-membered heteroaryl, 3- to 10-membered cycloalkyl C₁₋₃ alkylene, 5- to 10-membered heterocyclyl C₁₋₃ alkylene, 6- to 10-membered aryl C₁₋₃ alkylene, and 5- to 10-membered heteroaryl C₁₋₃ alkylene.

In some embodiments, R^{2d} is independently selected from the group consisting of H and the following groups optionally substituted with one or more R^{2e}: C₁₋₄ alkyl, C₁₋₄ heteroalkyl, 3- to 8-membered cycloalkyl, 3- to 8-membered heterocyclyl, 6- to 10-membered aryl, 5- to 10-membered heteroaryl, 3- to 8-membered cycloalkyl C₁₋₃ alkylene, 3- to 8-membered heterocyclyl C₁₋₃ alkylene, 6- to 10-membered aryl C₁₋₃ alkylene, and 5- to 10-membered heteroaryl C₁₋₃ alkylene.

In some embodiments, R^{2d} is independently selected from the group consisting of H and the following groups optionally substituted with one or more R^{2e}: C₁₋₃ alkyl, C₁₋₃ heteroalkyl, 3- to 8-membered cycloalkyl, 3- to 8-membered heterocycloalkyl, phenyl, 5- to 6-membered heteroaryl, 3- to 8-membered cycloalkyl C₁₋₃ alkylene, 3- to 8-membered heterocycloalkyl C₁₋₃ alkylene, phenyl C₁₋₃ alkylene, and 5- to 6-membered heteroaryl C₁₋₃ alkylene.

In some embodiments, R^{2e} is independently selected from the group consisting of deuterium, -F, -Cl, -Br, -OH, oxo, -NH₂, -CN, methyl, ethyl, methoxy, ethoxy, trifluoromethyl, and trifluoromethoxy.

In some embodiments, R^{2e} may further be selected from deuterium.

In some embodiments, R^{2e} is independently selected from the group consisting of -F, -Cl, -Br, -OH, oxo, -NH₂, -CN, methyl, ethyl, methoxy, ethoxy, trifluoromethyl, and trifluoromethoxy.

In some embodiments, R^{2e} is independently selected from the group consisting of -F, -Cl, -OH, oxo, -NH₂, methyl, methoxy, trifluoromethyl, and trifluoromethoxy.

In some embodiments, R^{2b} is independently selected from the group consisting of deuterium, -F, -Cl, -Br, -OH, -NH₂, -CN, methyl, ethyl, isopropyl, methoxy, ethoxy, methylamino, ethylamino, dimethylamino, diethylamino, trifluoromethyl, and trifluoromethoxy.

In some embodiments, R^{2b} may further be selected from deuterium.

In some embodiments, R^{2b} is independently selected from the group consisting of -F, -Cl, -Br, -OH, -NH₂, -CN, methyl, ethyl, isopropyl, methoxy, ethoxy, methylamino, ethylamino, dimethylamino, diethylamino, trifluoromethyl, and trifluoromethoxy.

In some embodiments, R^{2b} is independently selected from the group consisting of -F, -Cl, -OH, -NH₂, methyl, methoxy, methylamino, dimethylamino, trifluoromethyl, and trifluoromethoxy.

In some embodiments, R² is selected from the group consisting of H, deuterium, methyl, ethyl, isopropyl, *tert-*butyl*,* trifluoromethyl, cyclopropanyl, cyclobutanyl, cyclopentanyl,

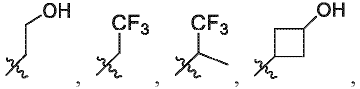

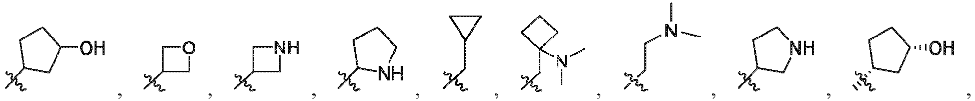

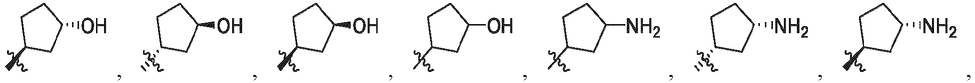

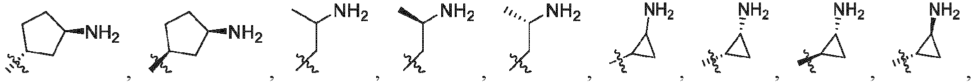

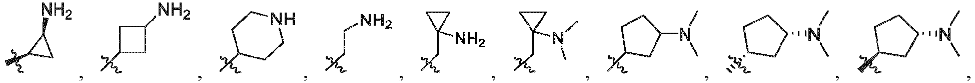

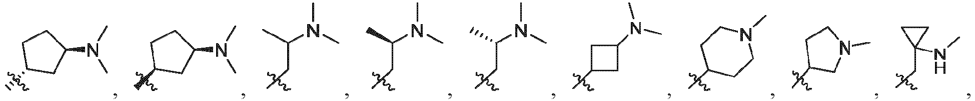

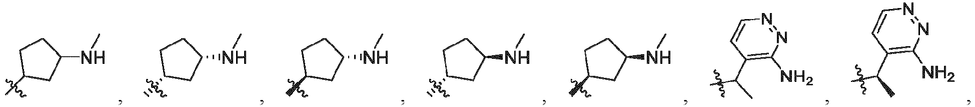

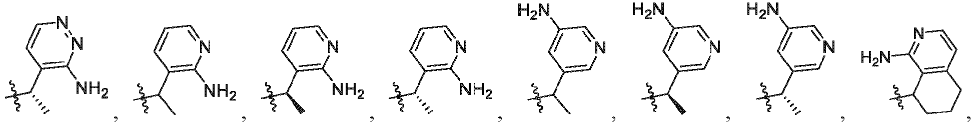

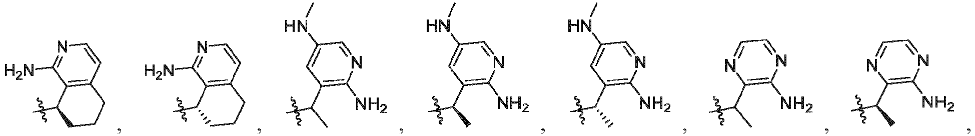

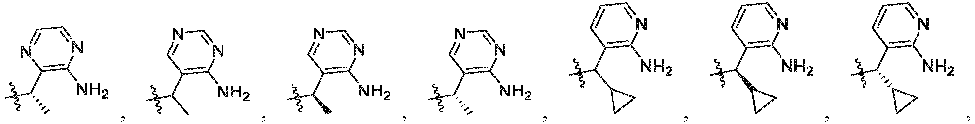

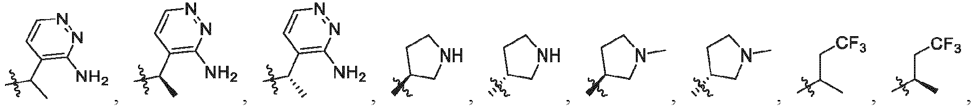

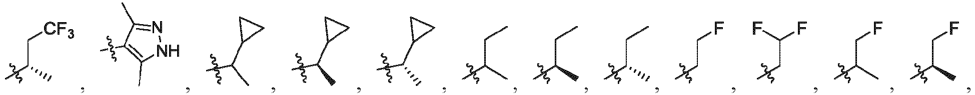

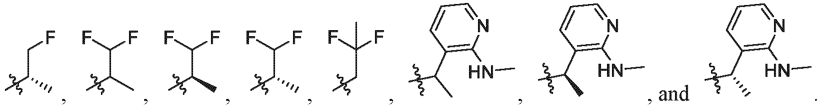

In some embodiments, R² is selected from the group consisting of H, methyl, ethyl, isopropyl, trifluoromethyl, cyclopropanyl, cyclobutanyl, cyclopentanyl, 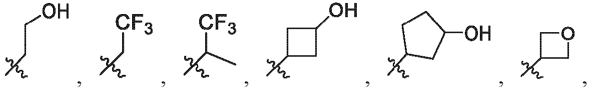 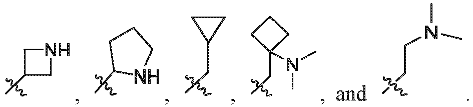 In some embodiments, R² is selected from *tert-butyl.* In some embodiments, R² is selected from the group consisting of 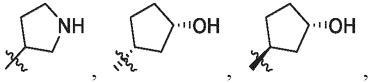 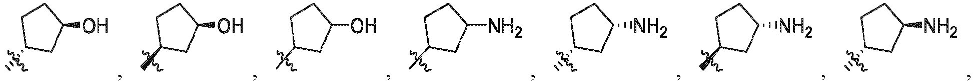 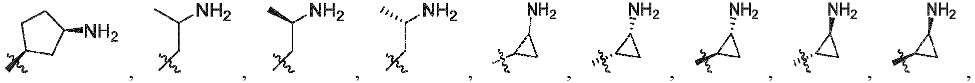 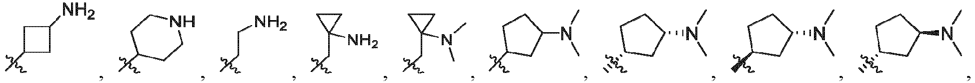 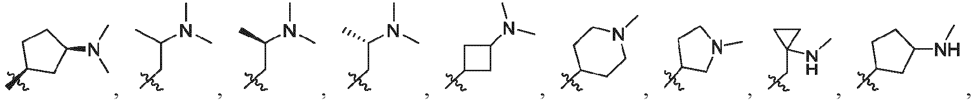 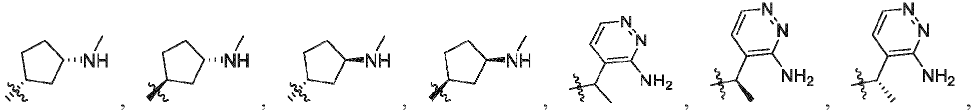 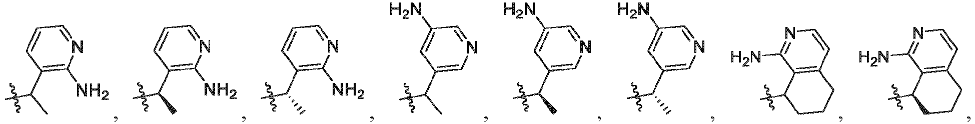 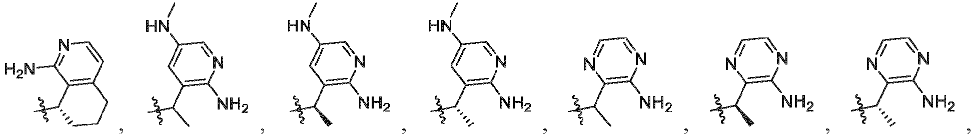 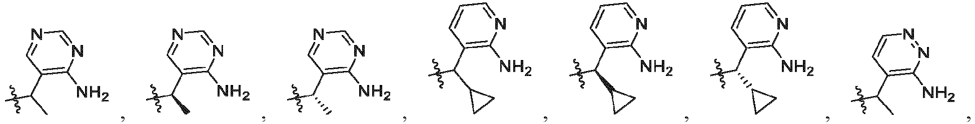 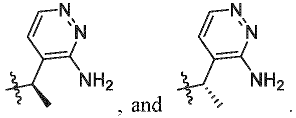 In some embodiments, R² is selected from the group consisting of 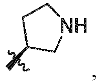 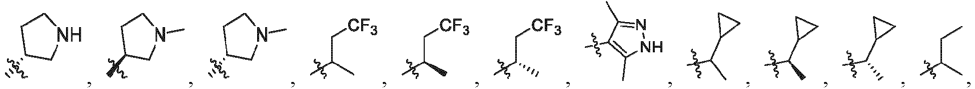 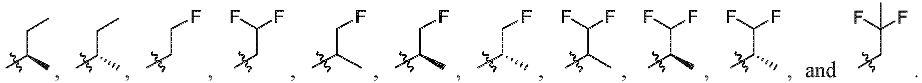 In some embodiments, R² is selected from the group consisting of

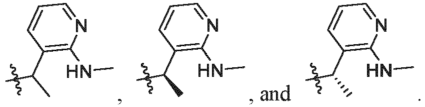

In some embodiments, R² is selected from the group consisting of H and C₁₋₄ alkyl, the C₁₋₄ alkyl optionally substituted with one or more R^{2a}.

In some embodiments, R² is selected from the group consisting of H, methyl, trifluoromethyl, 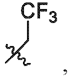 and

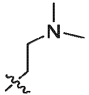

In some embodiments, R² is selected from the group consisting of H, methyl, ethyl, isopropyl, *tert-*butyl*,* trifluoromethyl,

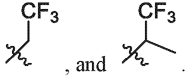

In some embodiments, R² is selected from the group consisting of H, methyl, ethyl, isopropyl, *tert-*butyl*,* trifluoromethyl,

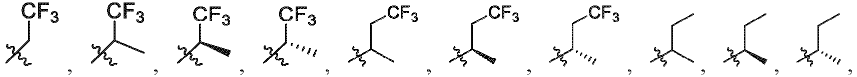

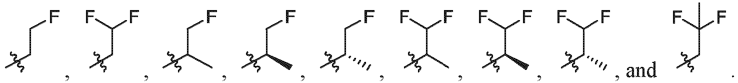

In some embodiments, R² is selected from the group consisting of 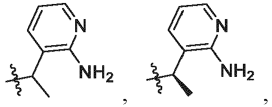 and

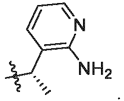

In some embodiments, R² is selected from the group consisting of isopropyl, *tert-*butyl*,* cyclopropanyl, and

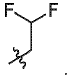

In some embodiments, when X is selected from -N-, L¹ is selected from the group consisting of -O-, -S-, -NH-, and -N(C₁₋₆ alkyl)-, R² is not selected from the group consisting of halogen, -OH, -NH₂, and -CN, and L² is not selected from the group consisting of -O-, -S-, -NH-, and -N(C₁₋₆ alkyl)-.

In some embodiments, Z is selected from the group consisting of a single bond, -S-, -O-, and the following groups optionally substituted with one or more R^{z}: -NH- and -N(C₁₋₆ alkyl)-.

In some embodiments, each R^{z} is independently selected from the group consisting of deuterium, oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, R^{z} may further be selected from deuterium.

In some embodiments, each R^{z} is independently selected from the group consisting of oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino.

In some embodiments, each R^{z} is independently selected from the group consisting of oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, and C₁₋₆ haloalkoxy.

In some embodiments, each R^{z} is independently selected from the group consisting of oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy.

In some embodiments, each R^{z} is independently selected from the group consisting of F, Cl, Br, -OH, oxo, -NH₂, -CN, C₁₋₃ alkyl, C₁₋₃ alkoxy, C₁₋₃ haloalkyl, and C₁₋₃ haloalkoxy.

In some embodiments, each R^{z} is independently selected from the group consisting of deuterium, F, Cl, Br, -OH, oxo, -NH₂, -CN, methyl, ethyl, isopropyl, methoxy, ethoxy, isopropoxy, trifluoromethyl, and trifluoromethoxy.

In some embodiments, each R^{z} is independently selected from the group consisting of F, Cl, Br, -OH, oxo, -NH₂, -CN, methyl, ethyl, isopropyl, methoxy, ethoxy, isopropoxy, trifluoromethyl, and trifluoromethoxy.

In some embodiments, Z is selected from the group consisting of a single bond, -S-, -O-, -NH-, and -N(C₁₋₁₂ alkyl)-.

In some embodiments, Z is selected from the group consisting of a single bond, -S-, -O-, -NH-, and -N(C₁₋₆ alkyl)-.

In some embodiments, Z is selected from the group consisting of a single bond, -S-, -O-, -NH-, and -N(C₁₋₃ alkyl)-.

In some embodiments, Z is selected from the group consisting of a single bond, -O-, and -N(C₁₋₃ alkyl)-.

In some embodiments, Z is selected from the group consisting of a single bond, -O-, and -N(CH₃)-.

In some embodiments, Z is selected from -O-.

In some embodiments, Z is selected from the group consisting of a single bond, -O-, -NH-, and -N(CH₃)-.

In some embodiments, R³ is selected from the group consisting of H, deuterium, and the following groups optionally substituted with one or more R^{3a}: C₁₋₆ alkyl, 3- to 10-membered cycloalkyl, 3- to 10-membered heterocyclyl, 6- to 10-membered aryl, 5- to 10-membered heteroaryl, 3- to 10-membered cycloalkyl C₁₋₄ alkylene, 3- to 10-membered heterocyclyl C₁₋₄ alkylene, 6- to 10-membered aryl C₁₋₄ alkylene, and 5- to 10-membered heteroaryl C₁₋₄ alkylene.

In some embodiments, R³ is selected from the group consisting of the following groups optionally substituted with one or more R^{3a}: 3- to 10-membered heterocyclyl and 3- to 10-membered heterocyclyl C₁₋₄ alkylene. In some embodiments, R³ is selected from the group consisting of the following groups optionally substituted with one or more R^{3a}: 4- to 10-membered heterocycloalkyl and 4- to 10-membered heterocycloalkyl C₁₋₄ alkylene.

In some embodiments, R³ is selected from deuterium.

In some embodiments, R³ is selected from the group consisting of H and the following groups optionally substituted with one or more R^{3a}: C₁₋₆ alkyl, 3- to 10-membered cycloalkyl, 3- to 10-membered heterocyclyl, 6- to 10-membered aryl, 5- to 10-membered heteroaryl, 3- to 10-membered cycloalkyl C₁₋₄ alkylene, 3- to 10-membered heterocyclyl C₁₋₄ alkylene, 6- to 10-membered aryl C₁₋₄ alkylene, and 5- to 10-membered heteroaryl C₁₋₄ alkylene.

In some embodiments, R³ is selected from the group consisting of H and the following groups optionally substituted with one or more R^{3a}: C₁₋₄ alkyl, 4- to 10-membered cycloalkyl, 4- to 10-membered heterocyclyl, phenyl, 5- to 6-membered heteroaryl, 4- to 10-membered cycloalkyl C₁₋₃ alkylene, 4- to 10-membered heterocyclyl C₁₋₃ alkylene, phenyl C₁₋₃ alkylene, and 5- to 6-membered heteroaryl C₁₋₃ alkylene. In some embodiments, R³ is selected from the group consisting of the following groups optionally substituted with one or more R^{3a}: 3-membered cycloalkyl and 3-membered cycloalkyl C₁₋₃ alkylene.

In some embodiments, R³ is selected from the group consisting of the following groups optionally substituted with one or more R^{3a}: 4- to 10-membered and 4- to 9-membered and 4- to 8-membered and 5- to 9-membered heterocyclyl C₁₋₃ alkylene. In some embodiments, R³ is selected from the group consisting of the following groups optionally substituted with one or more R^{3a}: 4- to 10-membered and 4- to 9-membered and 4- to 8-membered and 5- to 9-membered heterocyclyl.

In some embodiments, R³ is selected from 9-membered heterocyclyl C₁₋₃ alkylene optionally substituted with one or more R^{3a}. In some embodiments, R³ is selected from 9-membered heterocyclyl optionally substituted with one or more R^{3a}.

In some embodiments, R³ is selected from the group consisting of H and the following groups optionally substituted with one or more R^{3a}: C₁₋₄ alkyl, 4- to 8-membered cycloalkyl, 4- to 8-membered heterocyclyl, phenyl, 5- to 6-membered heteroaryl, 4- to 8-membered cycloalkyl C₁₋₃ alkylene, 4- to 8-membered heterocyclyl C₁₋₃ alkylene, phenyl C₁₋₃ alkylene, and 5- to 6-membered heteroaryl C₁₋₃ alkylene. In some embodiments, R³ is selected from the group consisting of the following groups optionally substituted with one or more R^{3a}: 3-membered cycloalkyl and 3-membered cycloalkyl C₁₋₃ alkylene.

In some embodiments, R³ is selected from the group consisting of H and the following groups optionally substituted with one or more R^{3a}: C₁₋₄ alkyl, 4- to 8-membered cycloalkyl, 4- to 8-membered heterocyclyl, 4- to 8-membered cycloalkyl C₁₋₃ alkylene, and 4- to 8-membered heterocyclyl C₁₋₃ alkylene. In some embodiments, R³ is selected from the group consisting of the following groups optionally substituted with one or more R^{3a}: 3-membered cycloalkyl and 3-membered cycloalkyl C₁₋₃ alkylene. In some embodiments, R³ is selected from 5- to 6-membered heteroaryl C₁₋₃ alkylene optionally substituted with one or more R^{3a}.

In some embodiments, R³ is selected from the group consisting of H and the following groups optionally substituted with one or more R^{3a}: C₁₋₄ alkyl, 4- to 8-membered heterocyclyl, 4- to 8-membered cycloalkyl C₁₋₃ alkylene, and 4- to 8-membered heterocyclyl C₁₋₃ alkylene. In some embodiments, R³ is selected from 3-membered cycloalkyl C₁₋₃ alkylene optionally substituted with one or more R^{3a}. In some embodiments, R³ is selected from 5- to 6-membered heteroaryl C₁₋₃ alkylene optionally substituted with one or more R^{3a}.

In some embodiments, the heterocyclyl is selected from heterocycloalkyl. In some embodiments, the heterocyclyl is selected from partially unsaturated heterocyclyl.

In some embodiments, the 3- to 12-membered heterocyclyl is selected from the group consisting of 3- to 12-membered heterocycloalkyl and benzo 4- to 6-membered heterocyclyl. In some embodiments, the 3- to 12-membered heterocyclyl C₁₋₆ alkylene is selected from the group consisting of 3- to 12-membered heterocycloalkyl C₁₋₆ alkylene and benzo 4- to 6-membered heterocyclyl C₁₋₆ alkylene.

In some embodiments, the 3- to 10-membered heterocyclyl is selected from the group consisting of 3- to 10-membered heterocycloalkyl and benzo 4- to 6-membered heterocyclyl. In some embodiments, the 3- to 10-membered heterocyclyl C₁₋₄ alkylene is selected from the group consisting of 3- to 10-membered heterocycloalkyl C₁₋₄ alkylene and benzo 4- to 6-membered heterocyclyl C₁₋₄ alkylene.

In some embodiments, the 4- to 10-membered heterocyclyl is selected from the group consisting of 4- to 10-membered heterocycloalkyl and benzo 4- to 6-membered heterocyclyl. In some embodiments, the 4- to 10-membered heterocyclyl C₁₋₆ alkylene is selected from the group consisting of 4- to 10-membered heterocycloalkyl C₁₋₆ alkylene and benzo 4- to 6-membered heterocyclyl C₁₋₆ alkylene. In some embodiments, the 4- to 10-membered heterocyclyl C₁₋₃ alkylene is selected from the group consisting of 4- to 10-membered heterocycloalkyl C₁₋₃ alkylene and benzo 4- to 6-membered heterocyclyl C₁₋₃ alkylene.

In some embodiments, the 4- to 10-membered and 4- to 9-membered and 4- to 8-membered and 5- to 9-membered heterocyclyl C₁₋₃ alkylene groups are selected from the group consisting of 4- to 10-membered and 4- to 9-membered and 4- to 8-membered and 5- to 9-membered heterocycloalkyl C₁₋₃ alkylene groups. In some embodiments, the 4- to 10-membered and 4- to 9-membered and 4- to 8-membered and 5- to 9-membered heterocyclyl groups are selected from the group consisting of 4- to 10-membered and 4- to 9-membered and 4- to 8-membered and 5- to 9-membered heterocycloalkyl groups.

In some embodiments, the 4- to 8-membered heterocyclyl is selected from 4- to 8-membered heterocycloalkyl. In some embodiments, the 4- to 8-membered heterocyclyl C₁₋₆ alkylene is selected from 4- to 8-membered heterocycloalkyl C₁₋₆ alkylene. In some embodiments, the 4- to 8-membered heterocyclyl C₁₋₃ alkylene is selected from 4- to 8-membered heterocycloalkyl C₁₋₃ alkylene.

In some embodiments, R³ is selected from the group consisting of H and the following groups optionally substituted with one or more R^{3a}: C₁₋₄ alkyl, 4- to 8-membered heterocycloalkyl, 4- to 8-membered cycloalkyl C₁₋₃ alkylene, and 4- to 8-membered heterocycloalkyl C₁₋₃ alkylene. In some embodiments, R³ is selected from 3-membered cycloalkyl C₁₋₃ alkylene optionally substituted with one or more R^{3a}. In some embodiments, R³ is selected from 5- to 6-membered heteroaryl C₁₋₃ alkylene optionally substituted with one or more R^{3a}.

In some embodiments, R³ is selected from the group consisting of H, deuterium, and the following groups optionally substituted with one or more R^{3a}: methyl, ethyl, propyl, butyl, cyclobutyl, cyclopentyl, cyclohexyl, azetidinyl, tetrahydropyrrolyl, morpholinyl, piperidinyl, piperazinyl, hexahydro-1H-pyrrolizinyl, cyclobutyl C₁₋₃ alkylene, cyclopentyl C₁₋₃ alkylene, cyclohexyl C₁₋₃ alkylene, azetidinyl C₁₋₃ alkylene, tetrahydropyrrolyl C₁₋₃ alkylene, morpholinyl C₁₋₃ alkylene, piperidinyl C₁₋₃ alkylene, piperazinyl C₁₋₃ alkylene, hexahydro-1H-pyrrolizinyl C₁₋₃ alkylene, hexahydro-1*H*-pyrrolo[2,1-*c*][1,4]oxazinyl, hexahydro-1*H*-pyrrolo[2,1-*c*][1,4]oxazinyl C₁₋₃ alkylene, 5-azaspiro[2.4]heptanyl, 5-azaspiro[2.4]heptanyl C₁₋₃ alkylene, cyclopropyl, cyclopropyl C₁₋₃ alkylene, n-propyl, isopropyl, n-butyl, isobutyl, sec-butyl, *tert-butyl,* and imidazolyl C₁₋₃ alkylene.

In some embodiments, R³ is selected from the group consisting of H and the following groups optionally substituted with one or more R^{3a}: methyl, ethyl, propyl, butyl, cyclobutyl, cyclopentyl, cyclohexyl, azetidinyl, tetrahydropyrrolyl, morpholinyl, piperidinyl, piperazinyl, hexahydro-1H-pyrrolizinyl, cyclobutyl C₁₋₃ alkylene, cyclopentyl C₁₋₃ alkylene, cyclohexyl C₁₋₃ alkylene, azetidinyl C₁₋₃ alkylene, tetrahydropyrrolyl C₁₋₃ alkylene, morpholinyl C₁₋₃ alkylene, piperidinyl C₁₋₃ alkylene, piperazinyl C₁₋₃ alkylene, and hexahydro-1*H*-pyrrolizinyl C₁₋₃ alkylene. In some embodiments, R³ is selected from the group consisting of the following groups optionally substituted with one or more R^{3a}: hexahydro-1*H-*pyrrolo[2,1-*c*][1,4]oxazinyl, hexahydro-1*H*-pyrrolo[2,1-*c*][1,4]oxazinyl C₁₋₃ alkylene, 5-azaspiro[2.4]heptanyl, and 5-azaspiro[2.4]heptanyl C₁₋₃ alkylene. In some embodiments, R³ is selected from the group consisting of the following groups optionally substituted with one or more R^{3a}: cyclopropyl and cyclopropyl C₁₋₃ alkylene. In some embodiments, R³ is selected from the group consisting of H and the following groups optionally substituted with one or more R^{3a}: *n*-propyl, isopropyl, *n*-butyl, isobutyl, *sec*-butyl, *tert-*butyl*,* and imidazolyl C₁₋₃ alkylene.

In some embodiments, R³ is selected from the group consisting of the following groups optionally substituted with one or more R^{3a}: methyl, piperidinyl, cyclopentyl C₁₋₃ alkylene, tetrahydropyrrolyl C₁₋₃ alkylene, and hexahydro-1*H*-pyrrolizinyl C₁₋₃ alkylene. In some embodiments, R³ is selected from the group consisting of the following groups optionally substituted with one or more R^{3a}: hexahydro-1*H*-pyrrolo[2,1-*c*][1,4]oxazinyl C₁₋₃ alkylene and 5-azaspiro[2.4]heptanyl C₁₋₃ alkylene. In some embodiments, R³ is selected from cyclopropyl C₁₋₃ alkylene optionally substituted with one or more R^{3a}. In some embodiments, R³ is selected from the group consisting of H and the following groups optionally substituted with one or more R^{3a}: ethyl, *n*-propyl, isopropyl, *n*-butyl, isobutyl, *sec*-butyl, *tert-*butyl*,* morpholinyl C₁₋₃ alkylene, imidazolyl C₁₋₃ alkylene, tetrahydropyrrolyl, piperazinyl C₁₋₃ alkylene, azetidinyl C₁₋₃ alkylene, piperidinyl C₁₋₃ alkylene, azetidinyl, cyclobutyl, cyclohexyl, cyclobutyl C₁₋₃ alkylene, and piperazinyl.

In some embodiments, R³ is selected from the group consisting of H, deuterium, and the following groups optionally substituted with one or more R^{3a}: 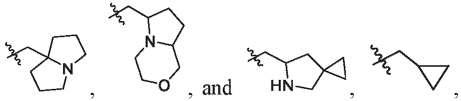 methyl, ethyl, *n*-propyl, isopropyl, *n*-butyl, isobutyl, *sec*-butyl, 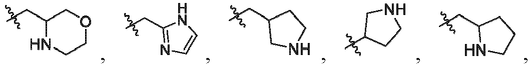 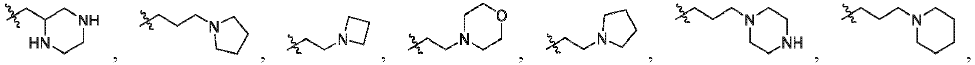 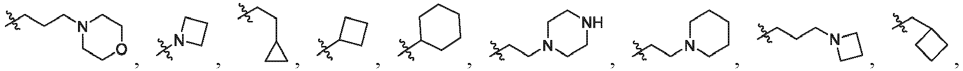 and

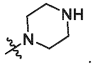

In some embodiments, R³ is selected from the group consisting of the following groups optionally substituted with one or more R^{3a}: 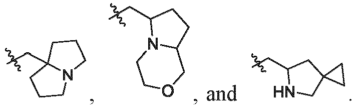 In some embodiments, R³ is selected from 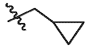 optionally substituted with one or more R^{3a}. In some embodiments, R³ is selected from the group consisting of H and the following groups optionally substituted with one or more R^{3a}: methyl, ethyl, *n*-propyl, isopropyl, *n*-butyl, isobutyl, *sec*-butyl,

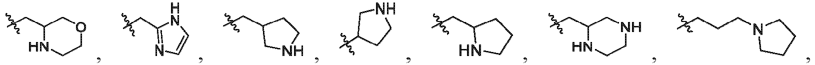

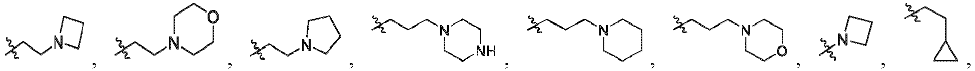

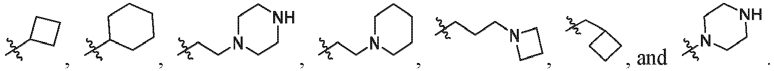

In some embodiments, R³ is selected from hexahydro-1*H*-pyrrolizinyl C₁₋₃ alkylene optionally substituted with one or more R^{3a}.

In some embodiments, the position of R^{3a} substitution is a cyclic moiety and/or an alkylene moiety in R³. In some embodiments, the position of R^{3a} substitution is a cyclic moiety in R³. In some embodiments, the position of R^{3a} substitution is an alkylene moiety in R³.

In some embodiments, R^{3a} is independently selected from the group consisting of deuterium, halogen, -OH, oxo, -NH₂, -CN, -C(O)NR^{3c}R^{3d}, -NR^{3c}C(O)R^{3d}, -NHC(O)NR^{3c}R^{3d}, -NR^{3c}C(O)OR^{3d}, -OC(O)R^{3d}, -C(O)OR^{3d}, -OC(O)OR^{3d}, -OC(O)NR^{3c}R^{3d}, -SO₂R^{3d}, -NHSO₂R^{3d}, -C₁₋₃ alkylene C(O)NR^{3c}R^{3d}, -C₁₋₃ alkylene NR^{3c}C(O)R^{3d}, -C₁₋₃ alkylene NHC(O)NR^{3c}R^{3d}, -C₁₋₃ alkylene NR^{3c}C(O)OR^{3d}, -C₁₋₃ alkylene OC(O)R^{3d}, -C₁₋₃ alkylene C(O)OR^{3d}, -C₁₋₃ alkylene OC(O)NR^{3c}R^{3d}, -C₁₋₃ alkylene SO₂R^{3d}, -C₁₋₃ alkylene OSO₂R^{3d}, -C₁₋₃ alkylene NHSO₂R^{3d}, and the following groups optionally substituted with one or more R^{3b}: C₁₋₄ alkyl, C₁₋₄ alkoxy, C₁₋₄ alkylamino, 3- to 6-membered cycloalkyl, 4- to 7-membered heterocyclyl, phenyl, 4- to 7-membered heterocyclyl C₁₋₃ alkylene, 5-to 6-membered heteroaryl, 3- to 6-membered cycloalkyl C₁₋₃ alkylene, phenyl C₁₋₃ alkylene, 5- to 6-membered heteroaryl C₁₋₃ alkylene, =NH, =CH₂, =N(C₁₋₄ alkyl), =CH(C₁₋₄ alkyl), and =C(C₁₋₄ alkyl)₂.

In some embodiments, R^{3a} may further be independently selected from deuterium.

In some embodiments, R^{3a} is independently selected from the group consisting of halogen, -OH, oxo, -NH₂, -CN, -C(O)NR^{3c}R^{3d}, -NR^{3c}C(O)R^{3d}, -NHC(O)NR^{3c}R^{3d}, -NR^{3c}C(O)OR^{3d}, -OC(O)R^{3d}, -C(O)OR^{3d}, -OC(O)OR^{3d}, -OC(O)NR^{3c}R^{3d}, -SO₂R^{3d}, -NHSO₂R^{3d}, -C₁₋₃ alkylene C(O)NR^{3c}R^{3d}, -C₁₋₃ alkylene NR^{3c}C(O)R^{3d}, -C₁₋₃ alkylene NHC(O)NR^{3c}R^{3d}, -C₁₋₃ alkylene NR^{3c}C(O)OR^{3d}, -C₁₋₃ alkylene OC(O)R^{3d}, -C₁₋₃ alkylene C(O)OR^{3d}, -C₁₋₃ alkylene OC(O)NR^{3c}R^{3d}, -C₁₋₃ alkylene SO₂R^{3d}, -C₁₋₃ alkylene OSO₂R^{3d}, -C₁₋₃ alkylene NHSO₂R^{3d}, and the following groups optionally substituted with one or more R^{3b}: C₁₋₄ alkyl, C₁₋₄ alkoxy, C₁₋₄ alkylamino, 3- to 6-membered cycloalkyl, 4- to 7-membered heterocyclyl, phenyl, 4- to 7-membered heterocyclyl C₁₋₃ alkylene, 5-to 6-membered heteroaryl, 3- to 6-membered cycloalkyl C₁₋₃ alkylene, phenyl C₁₋₃ alkylene, and 5- to 6-membered heteroaryl C₁₋₃ alkylene. In some embodiments, R^{3a} is independently selected from the group consisting of the following groups optionally substituted with one or more R^{3b}: =NH, =CH₂, =N(C₁₋₄ alkyl), =CH(C₁₋₄ alkyl), and =C(C₁₋₄ alkyl)₂.

In some embodiments, R^{3a} is independently selected from the group consisting of halogen, -OH, oxo, -NH₂, -CN, -NR^{3c}C(O)R^{3d}, -OC(O)NR^{3c}R^{3d}, -C₁₋₃ alkylene NR^{3c}C(O)R^{3d}, -C₁₋₃ alkylene NHC(O)NR^{3c}R^{3d}, -C₁₋₃ alkylene NR^{3c}C(O)OR^{3d}, -C₁₋₃ alkylene OC(O)R^{3d}, -C₁₋₃ alkylene OC(O)NR^{3c}R^{3d}, -C₁₋₃ alkylene OSO₂R^{3d}, -C₁₋₃ alkylene NHSO₂R^{3d}, and the following groups optionally substituted with one or more R^{3b}: C₁₋₄ alkyl, C₁₋₄ alkoxy, C₁₋₄ alkylamino, phenyl, benzyl, pyrazolyl, pyrazolylmethyl, tetrahydropyrrolylmethyl, and oxazolidinoneylmethyl. In some embodiments, R^{3a} is independently selected from the group consisting of the following groups optionally substituted with one or more R^{3b}: =NH, =CH₂, =N(C₁₋₄ alkyl), and =CH(C₁₋₄ alkyl). In some embodiments, R^{3a} is independently selected from morpholinomethyl optionally substituted with one or more R^{3b}. In some embodiments, R^{3a} may further be independently selected from deuterium.

In some embodiments, R^{3a} is independently selected from the group consisting of deuterium, F, Cl, Br, -OH, oxo, -NH₂, -CN, -NR^{3c}C(O)R^{3d}, -OC(O)NR^{3c}R^{3d}, -C₁₋₃ alkylene NR^{3c}C(O)R^{3d}, -C₁₋₃ alkylene NHC(O)NR^{3c}R^{3d}, -C₁₋₃ alkylene NR^{3c}C(O)OR^{3d}, -C₁₋₃ alkylene OC(O)R^{3d}, -C₁₋₃ alkylene OC(O)NR^{3c}R^{3d}, -C₁₋₃ alkylene OSO₂R^{3d}, -C₁₋₃ alkylene NHSO₂R^{3d}, and the following groups optionally substituted with one or more R^{3b}: methyl, ethyl, methoxy, ethoxy, methylamino, ethylamino, phenyl, benzyl, pyrazolylmethyl, oxazolidinoneylmethyl, =NH, =CH₂, and

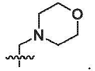

In some embodiments, R^{3a} is independently selected from the group consisting of F, Cl, Br, -OH, oxo, -NH₂, -CN, -NR^{3c}C(O)R^{3d}, -OC(O)NR^{3c}R^{3d}, -C₁₋₃ alkylene NR^{3c}C(O)R^{3d}, -C₁₋₃ alkylene NHC(O)NR^{3c}R^{3d}, -C₁₋₃ alkylene NR^{3c}C(O)OR^{3d}, -C₁₋₃ alkylene OC(O)R^{3d}, -C₁₋₃ alkylene OC(O)NR^{3c}R^{3d}, -C₁₋₃ alkylene OSO₂R^{3d}, -C₁₋₃ alkylene NHSO₂R^{3d}, and the following groups optionally substituted with one or more R^{3b}: methyl, ethyl, methoxy, ethoxy, methylamino, ethylamino, phenyl, benzyl, pyrazolylmethyl, and oxazolidinoneylmethyl. In some embodiments, R^{3a} is independently selected from the group consisting of the following groups optionally substituted with one or more R^{3b}: =NH and =CH₂. In some embodiments, R^{3a} is independently selected from 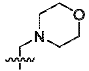 optionally substituted with one or more R^{3b}. In some embodiments, R^{3a} may further be independently selected from deuterium.

In some embodiments, R^{3a} is independently selected from =CH₂ optionally substituted with one or more R^{3b}.

In some embodiments, R^{3a} is independently selected from the group consisting of F, -C₁₋₃ alkylene OC(O)R^{3d}, -C₁₋₃ alkylene OC(O)NR^{3c}R^{3d}, methyl, methoxy, =CH₂, =CH₂ substituted with halogen, 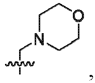 deuterium, -NH₂, dimethylamino, and dimethylaminomethylene.

In some embodiments, R^{3a} is independently selected from the group consisting of F, -C₁₋₃ alkylene OC(O)R^{3d}, -C₁₋₃ alkylene OC(O)NR^{3c}R^{3d}, methyl, and methoxy. In some embodiments, R^{3a} is independently selected from the group consisting of =CH₂ and =CH₂ substituted with halogen. In some embodiments, R^{3a} is independently selected from 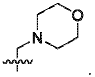 In some embodiments, R^{3a} is independently selected from the group consisting of deuterium, -NH₂, dimethylamino, and dimethylaminomethylene.

In some embodiments, R^{3a} is independently selected from the group consisting of F, -CH₂OC(O)R^{3d}, -CH₂OC(O)NR^{3c}R^{3d}, methyl, methoxy, =CH₂, =CHF, =CF₂, 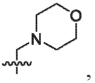 deuterium, -NH₂, dimethylamino, and dimethylaminomethylene.

In some embodiments, R^{3a} is independently selected from the group consisting of F, -CH₂OC(O)R^{3d}, -CH₂OC(O)NR^{3c}R^{3d}, methyl, and methoxy. In some embodiments, R^{3a} is independently selected from the group consisting of =CH₂, =CHF, and =CF₂. In some embodiments, R^{3a} is independently selected from 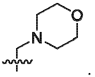 In some embodiments, R^{3a} is independently selected from the group consisting of deuterium, -NH₂, dimethylamino, and dimethylaminomethylene. In some embodiments, R^{3a} is independently selected from the group consisting of deuterium, F, and methyl.

In some embodiments, R^{3c} is independently selected from the group consisting of H, deuterium, and methyl.

In some embodiments, R^{3c} may further be independently selected from deuterium.

In some embodiments, R^{3c} is independently selected from the group consisting of H and methyl.

In some embodiments, R^{3c} is independently selected from H.

In some embodiments, R^{3d} is independently selected from the group consisting of H, deuterium, and the following groups optionally substituted with one or more R^{3e}: C₁₋₆ alkyl, C₁₋₆ heteroalkyl, 3- to 10-membered cycloalkyl, 5-to 10-membered heterocyclyl, 6- to 10-membered aryl, 5- to 10-membered heteroaryl, 3- to 10-membered cycloalkyl C₁₋₃ alkylene, 5- to 10-membered heterocyclyl C₁₋₃ alkylene, 6- to 10-membered aryl C₁₋₃ alkylene, and 5- to 10-membered heteroaryl C₁₋₃ alkylene.

In some embodiments, R^{3d} may further be independently selected from deuterium.

In some embodiments, R^{3d} is independently selected from the group consisting of H and the following groups optionally substituted with one or more R^{3e}: C₁₋₆ alkyl, C₁₋₆ heteroalkyl, 3- to 10-membered cycloalkyl, 5- to 10-membered heterocyclyl, 6- to 10-membered aryl, 5- to 10-membered heteroaryl, 3- to 10-membered cycloalkyl C₁₋₃ alkylene, 5- to 10-membered heterocyclyl C₁₋₃ alkylene, 6- to 10-membered aryl C₁₋₃ alkylene, and 5- to 10-membered heteroaryl C₁₋₃ alkylene.

In some embodiments, R^{3d} is independently selected from the group consisting of H and the following groups optionally substituted with one or more R^{3e}: C₁₋₁₂ alkyl, C₁₋₁₂ heteroalkyl, 3- to 12-membered cycloalkyl, 3- to 12-membered heterocycloalkyl, 6- to 10-membered aryl, 6- to 10-membered aryl C₁₋₆ alkylene, 5- to 10-membered heteroaryl, and 5- to 10-membered heteroaryl C₁₋₆ alkylene.

In some embodiments, R^{3d} is independently selected from the group consisting of H and the following groups optionally substituted with one or more R^{3e}: C₁₋₆ alkyl, C₁₋₆ heteroalkyl, 4- to 8-membered heterocycloalkyl, phenyl, phenyl C₁₋₃ alkylene, 5- to 6-membered heteroaryl, and 5- to 6-membered heteroaryl C₁₋₃ alkylene.

In some embodiments, R^{3d} is independently selected from the group consisting of H and the following groups optionally substituted with one or more R^{3e}: C₁₋₄ alkyl, C₁₋₄ alkoxy C₁₋₃ alkylene, phenyl, benzyl, pyrazolyl, pyridinyl, azetidinyl, tetrahydropyrrolyl, piperidinyl, piperazinyl, and morpholinyl.

In some embodiments, R^{3d} is independently selected from the group consisting of H and the following groups optionally substituted with one or more R^{3e}: C₁₋₄ alkyl, C₁₋₄ alkoxy C₁₋₃ alkylene, azetidinyl, tetrahydropyrrolyl, piperidinyl, piperazinyl, and morpholinyl.

In some embodiments, R^{3d} is independently selected from the group consisting of H, C₁₋₄ alkyl, and the following groups optionally substituted with one or more R^{3e}: tetrahydropyrrolyl, piperidinyl, and morpholinyl.

In some embodiments, R^{3d} is independently selected from the group consisting of H, methyl, and the following groups optionally substituted with one or more R^{3e}: tetrahydropyrrolyl, piperidinyl, and morpholinyl.

In some embodiments, R^{3d} is independently selected from the group consisting of H, deuterium, methyl, tetrahydropyrrolyl, piperidinyl, and morpholinyl.

In some embodiments, R^{3d} is independently selected from the group consisting of H, methyl, tetrahydropyrrolyl, piperidinyl, and morpholinyl.

In some embodiments, R^{3e} is independently selected from the group consisting of deuterium, -F, -Cl, -Br, -OH, oxo, methyl, ethyl, trifluoromethyl, and difluoromethyl.

In some embodiments, R^{3e} may further be selected from deuterium.

In some embodiments, R^{3e} is independently selected from the group consisting of -F, -Cl, -Br, -OH, oxo, methyl, ethyl, trifluoromethyl, and difluoromethyl.

In some embodiments, R^{3e} is independently selected from the group consisting of -F, -Cl, -OH, oxo, methyl, and trifluoromethyl.

In some embodiments, R^{3b} is independently selected from the group consisting of deuterium, -F, -Cl, -Br, -OH, oxo, -NH₂, -CN, methyl, ethyl, isopropyl, methoxy, ethoxy, methylamino, ethylamino, dimethylamino, and diethylamino.

In some embodiments, R^{3b} may further be independently selected from deuterium.

In some embodiments, R^{3b} is independently selected from the group consisting of -F, -Cl, -Br, -OH, oxo, -NH₂, -CN, methyl, ethyl, isopropyl, methoxy, ethoxy, methylamino, ethylamino, dimethylamino, and diethylamino.

In some embodiments, R^{3b} is independently selected from the group consisting of -F, -Cl, -NH₂, methyl, methoxy, methylamino, and dimethylamino.

In some embodiments, R³ is selected from the group consisting of H, deuterium, methyl, 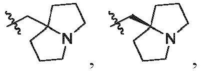 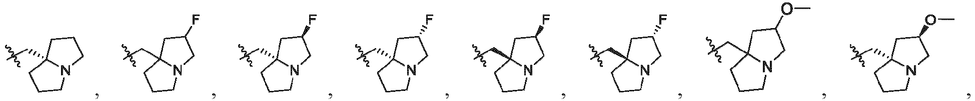 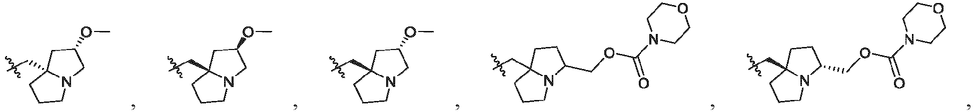 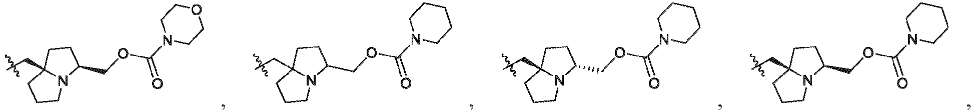 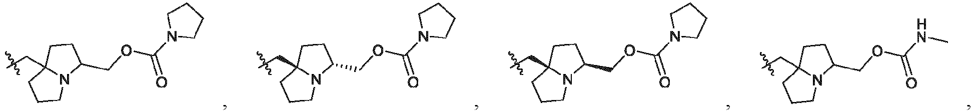 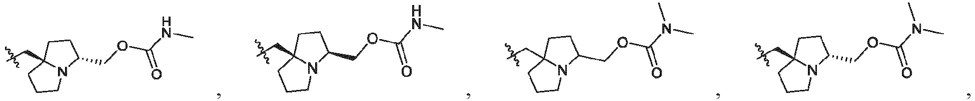 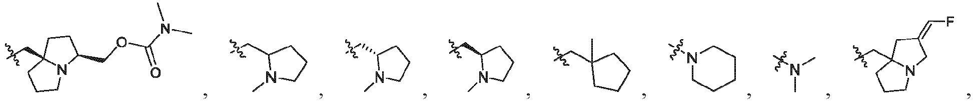 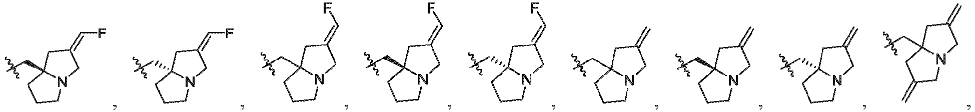 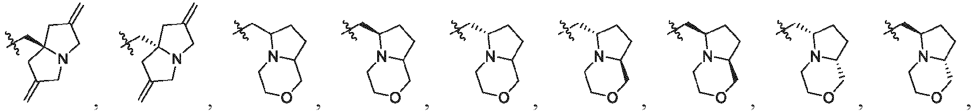 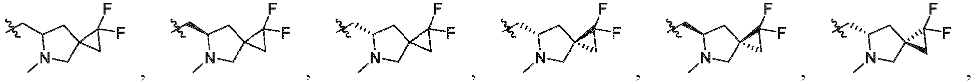 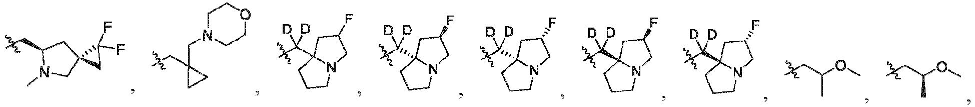 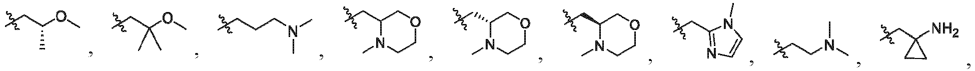 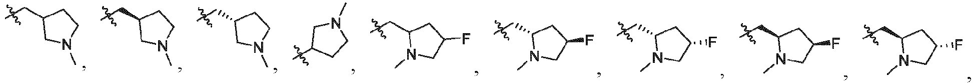 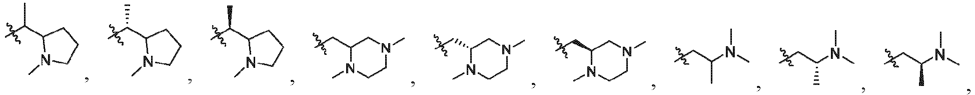 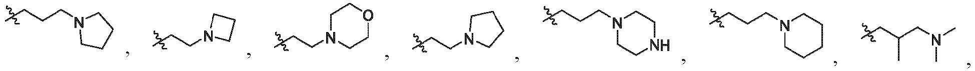 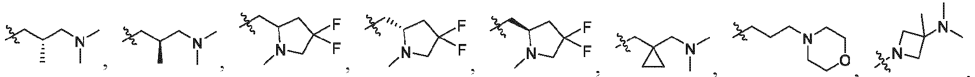 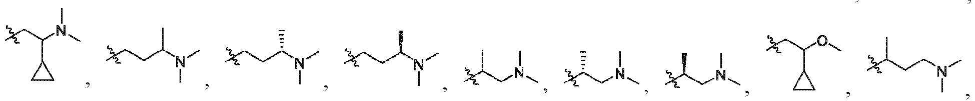 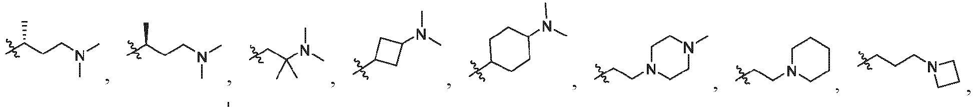 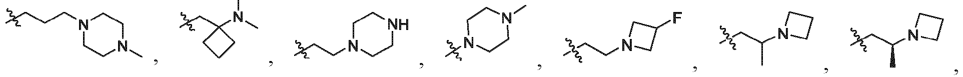 and

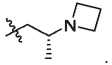

In some embodiments, R³ is selected from the group consisting of 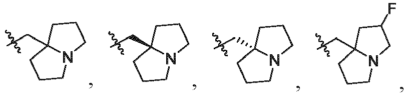 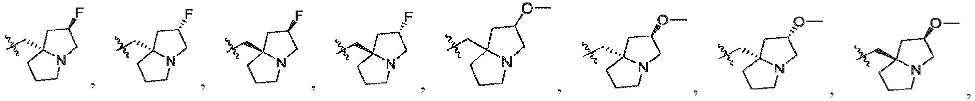 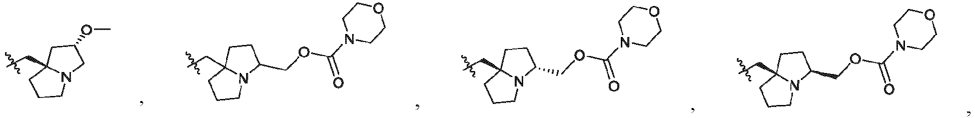 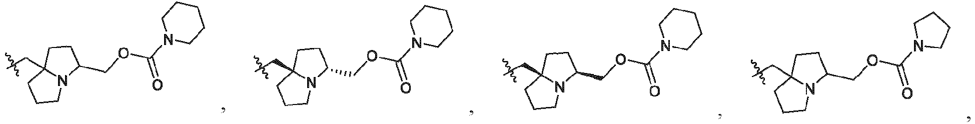 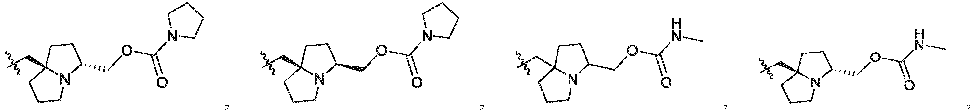 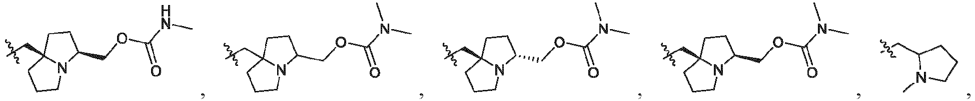 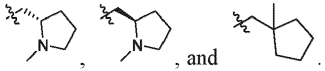 In some embodiments, R³ is selected from the group consisting of 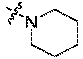 and 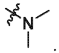 In some embodiments, R³ is selected from the group consisting of 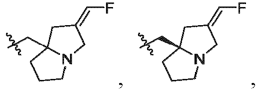 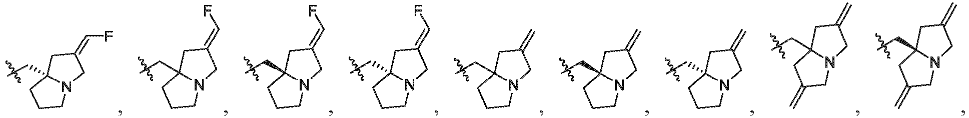 and 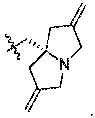 In some embodiments, R³ is selected from the group consisting of 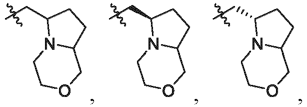 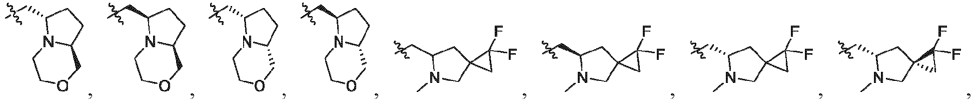 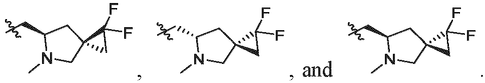 In some embodiments, R³ is selected from

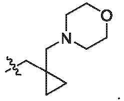

In some embodiments, R³ is selected from the group consisting of H and methyl. In some embodiments, R³ is selected from the group consisting of 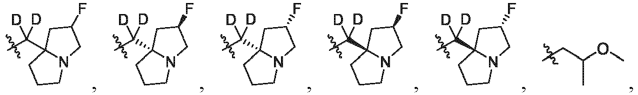 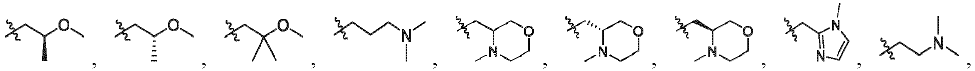 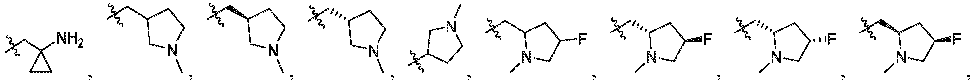 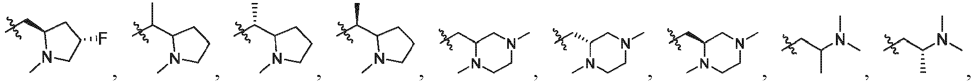 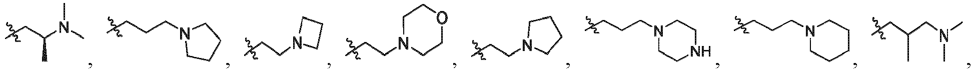 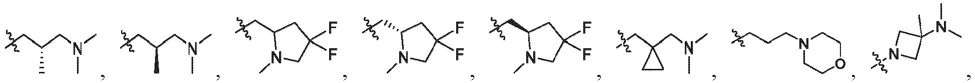 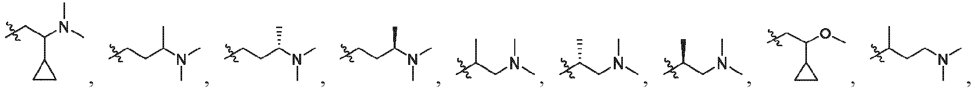 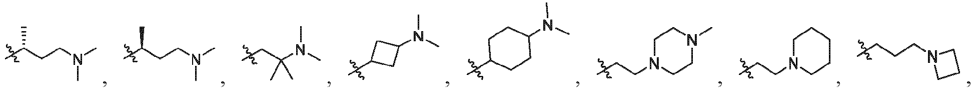 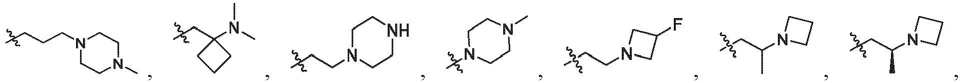 and

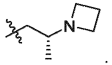

In some embodiments, R⁴ is selected from the group consisting of H, deuterium, halogen, -CN, C₁₋₆ alkyl, C₁₋₆ alkoxy, C₁₋₆ haloalkyl, C₁₋₆ haloalkoxy, 3- to 12-membered cycloalkyl, 3- to 12-membered heterocyclyl, 6- to 10-membered aryl, and 5- to 10-membered heteroaryl.

In some embodiments, R⁴ is selected from deuterium.

In some embodiments, R⁴ is selected from the group consisting of H, halogen, -CN, C₁₋₆ alkyl, C₁₋₆ alkoxy, C₁₋₆ haloalkyl, C₁₋₆ haloalkoxy, 3- to 12-membered cycloalkyl, 3- to 12-membered heterocyclyl, 6- to 10-membered aryl, and 5- to 10-membered heteroaryl.

In some embodiments, R⁴ is selected from the group consisting of H, halogen, -CN, C₁₋₄ alkyl, C₁₋₄ alkoxy, C₁₋₄ haloalkyl, C₁₋₄ haloalkoxy, 3- to 6-membered cycloalkyl, 5- to 6-membered heterocyclyl, phenyl, and 5- to 6-membered heteroaryl.

In some embodiments, R⁴ is selected from the group consisting of H, halogen, -CN, C₁₋₄ alkyl, C₁₋₄ alkoxy, C₁₋₄ haloalkyl, and C₁₋₄ haloalkoxy.

In some embodiments, R⁴ is selected from the group consisting of H, -F, -Cl, -Br, -CN, C₁₋₂ alkyl, C₁₋₂ alkoxy, C₁₋₂ haloalkyl, and C₁₋₂ haloalkoxy.

In some embodiments, R⁴ is selected from the group consisting of H, -F, -Cl, -Br, C₁₋₂ alkyl, C₁₋₂ alkoxy, and C₁₋₂ fluoroalkyl.

In some embodiments, R⁴ is selected from the group consisting of H, deuterium, -F, -Cl, -Br, methyl, methoxy, difluoromethyl, and trifluoromethyl.

In some embodiments, R⁴ is selected from the group consisting of H, -F, -Cl, -Br, methyl, methoxy, difluoromethyl, and trifluoromethyl.

In some embodiments, R⁴ is selected from the group consisting of H, -F, -Cl, and methyl.

In some embodiments, R⁴ is selected from the group consisting of H, -F, and -Cl.

In some embodiments, ring A is selected from the group consisting of the following groups optionally substituted with one or more R⁵: 3- to 6-membered cycloalkyl, 5- to 10-membered heterocyclyl, 6- to 10-membered aryl, and 5- to 10-membered heteroaryl.

In some embodiments, ring A is selected from the group consisting of the following groups optionally substituted with one or more R⁵: 6- to 10-membered aryl, benzo 4- to 6-membered cycloalkenyl, benzo 4- to 6-membered heterocyclyl, and 5- to 10-membered heteroaryl.

In some embodiments, ring A is selected from the group consisting of the following groups optionally substituted with one or more R⁵: phenyl, naphthyl, benzocyclohexenyl, benzocyclopentenyl, pyrrolyl, pyrazolyl, imidazolyl, furanyl, oxazolyl, isoxazolyl, thienyl, thiazolyl, isothiazolyl, pyranyl, pyridinyl, pyridazinyl, pyrimidinyl, pyrazinyl, indolyl, benzopyrazolyl, benzimidazolyl, benzothiazolyl, quinolyl, isoquinolyl, benzopyrimidinyl, benzothienyl, pyridopyrazolyl, and pyridopyrrolyl.

In some embodiments, ring A is selected from the group consisting of the following groups optionally substituted with one or more R⁵: phenyl, naphthyl, benzocyclohexenyl, benzocyclopentenyl, pyrrolyl, pyrazolyl, imidazolyl, furanyl, oxazolyl, isoxazolyl, thienyl, thiazolyl, isothiazolyl, pyranyl, pyridinyl, pyridazinyl, pyrimidinyl, pyrazinyl, indolyl, benzopyrazolyl, benzimidazolyl, benzothiazolyl, quinolyl, isoquinolyl, and benzopyrimidinyl. In some embodiments, ring A is selected from benzothienyl optionally substituted with one or more R⁵. In some embodiments, ring A is selected from the group consisting of the following groups optionally substituted with one or more R⁵: pyridopyrazolyl and pyridopyrrolyl.

In some embodiments, ring A is selected from the group consisting of the following groups optionally substituted with one or more R⁵: phenyl, naphthyl, benzocyclohexenyl, benzocyclopentenyl, pyridinyl, indolyl, benzopyrazolyl, benzimidazolyl, benzothiazolyl, quinolyl, and isoquinolyl. In some embodiments, ring A is selected from benzothienyl optionally substituted with one or more R⁵. In some embodiments, ring A is selected from the group consisting of the following groups optionally substituted with one or more R⁵: pyrazolyl, pyridopyrazolyl, and pyridopyrrolyl.

In some embodiments, ring A is selected from the group consisting of the following groups optionally substituted with one or more R⁵: phenyl, naphthyl, pyridinyl, benzothiazolyl, and benzothienyl. In some embodiments, ring A is selected from isoquinolyl optionally substituted with one or more R⁵. In some embodiments, ring A is selected from the group consisting of the following groups optionally substituted with one or more R⁵: benzopyrazolyl, pyrazolyl, pyridopyrazolyl, pyridopyrrolyl, quinolyl, indolyl, and benzimidazolyl.

In some embodiments, ring A is selected from the group consisting of the following groups optionally substituted with one or more R⁵: phenyl, naphthyl, and pyridinyl. In some embodiments, ring A is selected from isoquinolyl optionally substituted with one or more R⁵. In some embodiments, ring A is selected from the group consisting of the following groups optionally substituted with one or more R⁵: benzopyrazolyl, benzothiazolyl, pyrazolyl, pyridopyrazolyl, pyridopyrrolyl, quinolyl, indolyl, and benzimidazolyl.

In some embodiments, ring A is selected from the group consisting of the following groups optionally substituted with one or more R⁵: phenyl and naphthyl.

In some embodiments, each R⁵ is independently selected from the group consisting of deuterium, halogen, -CN, -OH, -NH₂, and the following groups optionally substituted with one or more R^{5a}: C₁₋₆ alkyl, C₂₋₆ alkenyl, C₂₋₆ alkynyl, C₁₋₆ alkoxy, C₁₋₆ alkylamino, di-C₁₋₆ alkylamino, C₁₋₆ alkylthio, 3- to 12-membered cycloalkyl, 6- to 10-membered aryl, 5- to 10-membered heteroaryl, and 3- to 12-membered heterocyclyl.

In some embodiments, each R⁵ may further be selected from deuterium.

In some embodiments, each R⁵ is independently selected from the group consisting of halogen, -CN, -OH, -NH₂, and the following groups optionally substituted with one or more R^{5a}: C₁₋₆ alkyl, C₂₋₆ alkenyl, C₂₋₆ alkynyl, C₁₋₆ alkoxy, C₁₋₆ alkylamino, di-C₁₋₆ alkylamino, C₁₋₆ alkylthio, 3- to 12-membered cycloalkyl, 6- to 10-membered aryl, 5- to 10-membered heteroaryl, and 3- to 12-membered heterocyclyl.

In some embodiments, each R⁵ is independently selected from the group consisting of -F, -Cl, -Br, -CN, -OH, -NH₂, and the following groups optionally substituted with one or more R^{5a}: methyl, ethyl, isopropyl, ethenyl, ethynyl, methoxy, methylamino, dimethylamino, methylthio, 3- to 6-membered cycloalkyl, and 3- to 6-membered heterocyclyl. In some embodiments, each R⁵ is independently selected from propynyl optionally substituted with one or more R^{5a}

In some embodiments, each R⁵ is independently selected from the group consisting of -F, -Cl, -OH, -NH₂, and the following groups optionally substituted with one or more R^{5a}: methyl, ethyl, isopropyl, ethenyl, ethynyl, methoxy, methylthio, and cyclopropanyl. In some embodiments, each R⁵ is independently selected from -CN. In some embodiments, each R⁵ is independently selected from propynyl optionally substituted with one or more R^{5a}

In some embodiments, each R⁵ is independently selected from the group consisting of -F, -Cl, -OH, -NH₂, methyl, ethyl, isopropyl, ethenyl, ethynyl, trifluoromethyl, hydroxymethylene, methoxy, trifluoromethoxy, methylamino, dimethylamino, methylthio, trifluoromethylthio, and cyclopropanyl optionally substituted with -F, -Cl, -Br, or methyl. In some embodiments, each R⁵ is independently selected from -CN. In some embodiments, each R⁵ is independently selected from propynyl.

In some embodiments, each R⁵ is independently selected from the group consisting of deuterium, -F, -Cl, -OH, -NH₂, methyl, ethyl, isopropyl, ethynyl, trifluoromethyl, trifluoromethoxy, methylthio, cyclopropanyl substituted with methyl, -CN, methoxy, propynyl, and dimethylamino.

In some embodiments, each R⁵ is independently selected from the group consisting of -F, -Cl, -OH, -NH₂, methyl, ethyl, isopropyl, ethynyl, trifluoromethyl, trifluoromethoxy, methylthio, and cyclopropanyl substituted with methyl. In some embodiments, each R⁵ is independently selected from -CN. In some embodiments, each R⁵ is independently selected from methoxy. In some embodiments, each R⁵ is independently selected from propynyl. In some embodiments, each R⁵ is independently selected from dimethylamino.

In some embodiments, R^{5a} is independently selected from the group consisting of deuterium, -F, -Cl, -Br, -OH, oxo, -NH₂, -CN, methyl, ethyl, isopropyl, methoxy, ethoxy, methylamino, ethylamino, dimethylamino, and diethylamino.

In some embodiments, R^{5a} may further be selected from deuterium.

In some embodiments, R^{5a} is independently selected from the group consisting of -F, -Cl, -Br, -OH, oxo, -NH₂, -CN, methyl, ethyl, isopropyl, methoxy, ethoxy, methylamino, ethylamino, dimethylamino, and diethylamino.

In some embodiments, R^{5a} is independently selected from the group consisting of -F, -Cl, -Br, -NH₂, methyl, methoxy, methylamino, and dimethylamino.

In some embodiments, R^{5a} is independently selected from the group consisting of -F, -Cl, -Br, and methyl.

In some embodiments, ring A is selected from the group consisting of 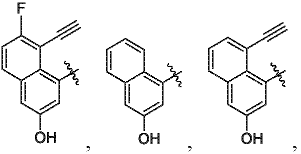 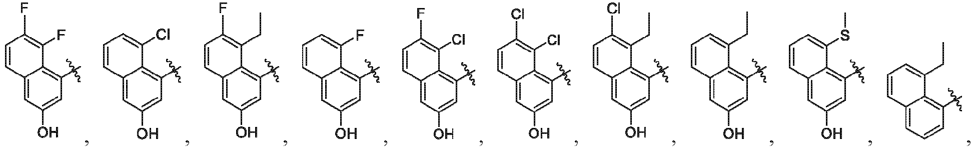 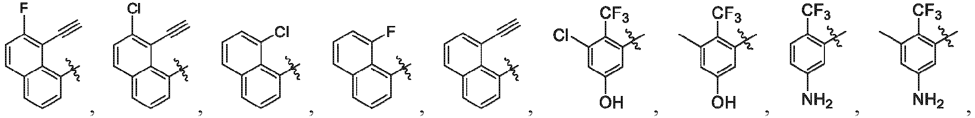 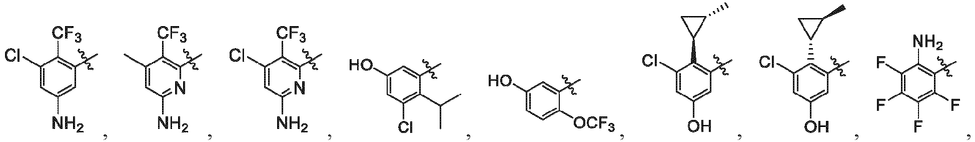 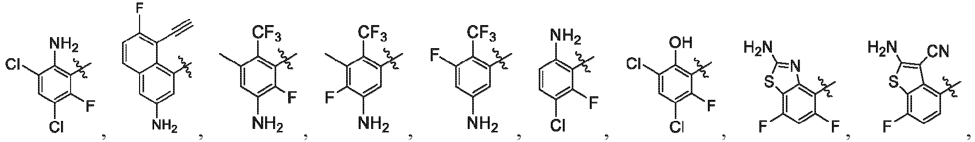 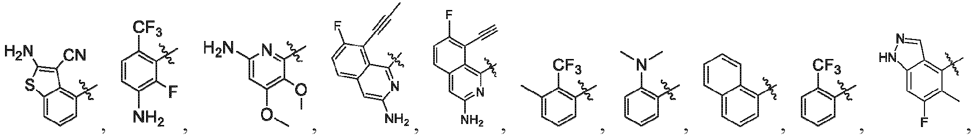 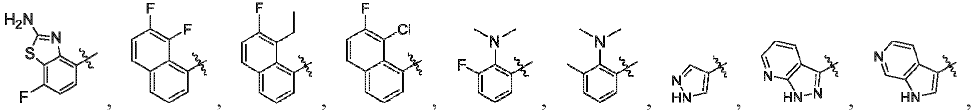 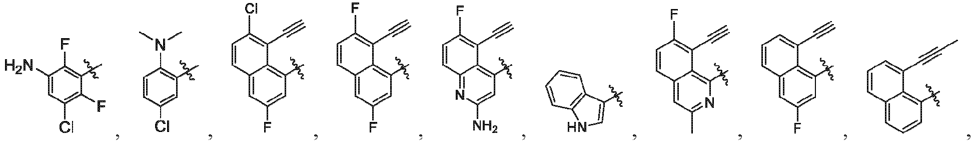 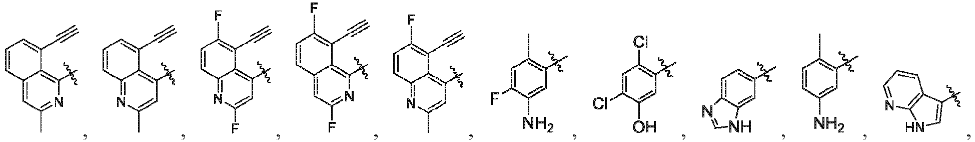 and

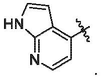

In some embodiments, ring A is selected from the group consisting of 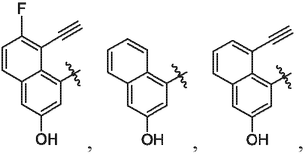 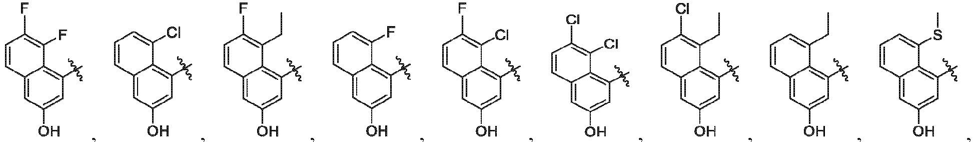 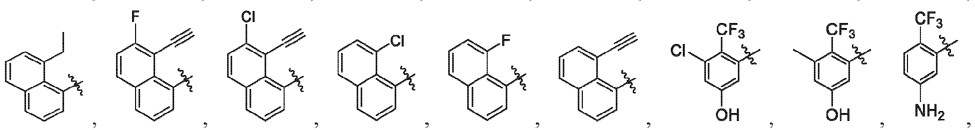 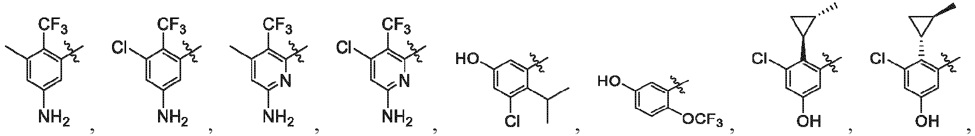 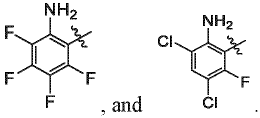 In some embodiments, ring A is selected from 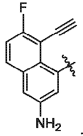 In some embodiments, ring A is selected from the group consisting of 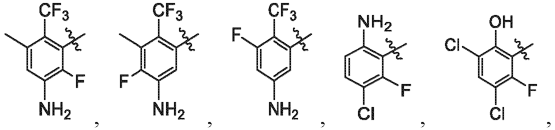 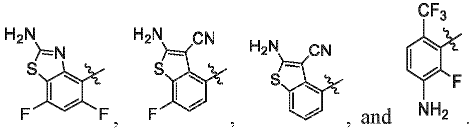 In some embodiments, ring A is selected from the group consisting of 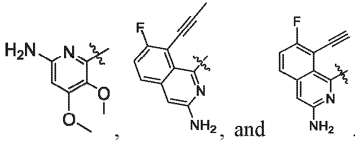 In some embodiments, ring A is selected from the group consisting of

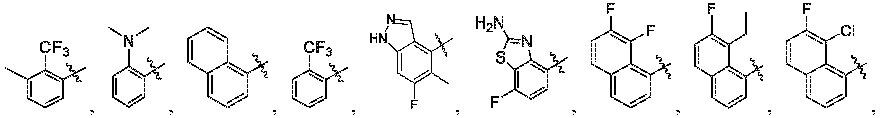

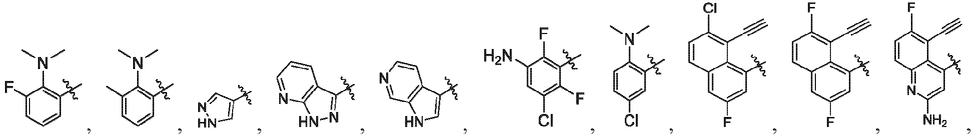

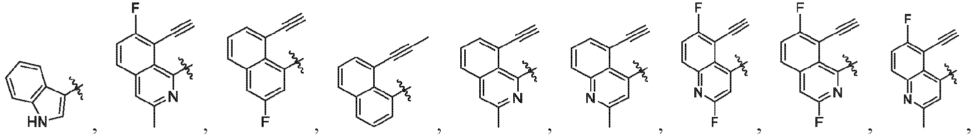

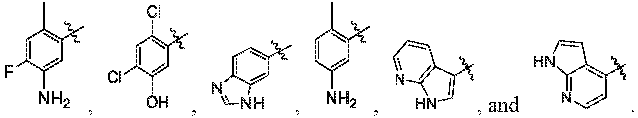

In some embodiments, C₁₋₁₂ is selected from the group consisting of C₁₋₁₀, C₁₋₈, C₁₋₆, C₁₋₄, C₁₋₃, and C₁₋₂.

In some embodiments, the C₁₋₆ alkyl is selected from the group consisting of C₁₋₄ alkyl, C₁₋₃ alkyl, and C₁₋₂ alkyl. In some embodiments, the C₁₋₆ alkylene is selected from the group consisting of C₁₋₄ alkylene, C₁₋₃ alkylene, and C₁₋₂ alkylene.

In some embodiments, the halogen is selected from the group consisting of F, Cl, Br, and I.

In some embodiments, the halo is selected from the group consisting of fluoro, chloro, and bromo. In some embodiments, the halo is selected from the group consisting of fluoro and chloro. In some embodiments, the halo is selected from fluoro.

In some embodiments, the one or more is selected from the group consisting of 1, 2, 3, 4, 5, and 6. In some embodiments, the one or more is selected from the group consisting of 1, 2, 3, 4, and 5. In some embodiments, the one or more is selected from the group consisting of 1, 2, 3, and 4. In some embodiments, the one or more is selected from the group consisting of 1, 2, and 3.

In some embodiments, the 3- to 12-membered is selected from the group consisting of 3- to 10-membered, 3- to 6-membered, 5- to 6-membered, 5- to 8-membered, and 5- to 10-membered.

In some embodiments, the heteroalkylene comprises 1 or 2 heteroatoms selected from the group consisting of N, O, and S.

In some embodiments, the heteroalkylene comprises 1 or 2 heteroatoms selected from the group consisting of N and O.

In some embodiments, the heteroalkylene comprises 1 N atom and 1 O atom.

In some embodiments, the heteroalkylene comprises 1 N atom.

In some embodiments, the heteroalkylene comprises 1 O atom.

In some embodiments, the heterocycloalkyl comprises 1 or 2 heteroatoms selected from the group consisting of N and O.

In some embodiments, the heterocycloalkyl comprises 1 N atom.

In some embodiments, the heterocycloalkyl comprises 1 O atom.

In some embodiments, the heterocycloalkyl comprises 1 N atom and 1 O atom.

In some embodiments, the heterocyclyl or heteroaryl comprises 1 or 2 heteroatoms selected from the group consisting of N, O, and S.

In some embodiments, the heterocyclyl or heteroaryl comprises 1 or 2 N atoms.

In some embodiments, the heterocyclyl or heteroaryl comprises 1 N atom and 1 O atom.

In some embodiments, the heterocyclyl or heteroaryl comprises 1 N atom and 1 S atom.

In some embodiments, the heterocyclyl or heterocycloalkyl includes a monocyclic ring, a spiro ring, a fused ring, and a bridged ring. In some embodiments, the heterocycloalkyl includes a monocyclic ring and a spiro ring. In some embodiments, the heterocyclyl or heterocycloalkyl includes a monocyclic ring and a bridged ring.

In some embodiments,
X is selected from the group consisting of -N-, -CH-, and -C(C₁₋₃ alkyl)-;
L¹ is selected from the group consisting of -S-, -O-, -NH-, -N(C₁₋₃ alkyl)-, and the following groups optionally substituted with one or more R¹: C₁₋₄ alkylene and C₁₋₄ heteroalkylene;
each R¹ is independently selected from the group consisting of deuterium, oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino;
R² is selected from the group consisting of H, deuterium, halogen, -OH, -NH₂, -CN, and the following groups optionally substituted with one or more R^{2a}: C₁₋₆ alkyl, C₁₋₆ heteroalkyl, 3- to 10-membered cycloalkyl-L²-, 4- to 10-membered heterocycloalkyl-L²-, 6- to 10-membered aryl-L²-, benzo 4- to 6-membered heterocyclyl-L²-, and 5-to 10-membered heteroaryl-L²-;
L² is selected from the group consisting of a single bond, -O-, -S-, -NH-, -N(C₁₋₆ alkyl)-, C₁₋₆ alkylene, C₁₋₆ heteroalkylene, C₂₋₆ alkenylene, and C₁₋₆ heteroalkenylene;
each R^{2a} is independently selected from the group consisting of deuterium, halogen, -OH, oxo, -NH₂, -CN, -C(O)NR^{2c}R^{2d}, -NR^{2c}C(O)R^{2d}, -NHC(O)NR^{2c}R^{2d}, -NR^{2c}C(O)OR^{2d}, -OC(O)R^{2d}, -C(O)OR^{2d}, -OC(O)OR^{2d}, -OC(O)NR^{2c}R^{2d}, -SO₂R^{2d}, -NHSO₂R^{2d}, -C₁₋₆ alkylene C(O)NR^{2c}R^{2d}, -C₁₋₆ alkylene NR^{2c}C(O)R^{2d}, -C₁₋₆ alkylene NHC(O)NR^{2c}R^{2d}, -C₁₋₆ alkylene NR^{2c}C(O)OR^{2d}, -C₁₋₆ alkylene OC(O)R^{2d}, -C₁₋₆ alkylene C(O)OR^{2d}, -C₁₋₆ alkylene OC(O)NR^{2c}R^{2d}, -C₁₋₆ alkylene SO₂R^{2d}, -C₁₋₆ alkylene OSO₂R^{2d}, -C₁₋₆ alkylene NHSO₂R^{2d}, and the following groups optionally substituted with one or more R^{2b}: C₁₋₆ alkyl, C₁₋₆ alkoxy, C₁₋₆ alkylamino, di-C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{2c} is independently selected from the group consisting of H, deuterium, and C₁₋₆ alkyl;
R^{2d} is independently selected from the group consisting of H, deuterium, and the following groups optionally substituted with one or more R^{2e}: C₁₋₆ alkyl, C₁₋₆ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{2e} is independently selected from the group consisting of deuterium, halogen, -OH, oxo, -NH₂, -CN, C₁₋₄ alkyl, C₁₋₄ alkoxy, C₁₋₄ haloalkyl, and C₁₋₄ haloalkoxy;
each R^{2b} is independently selected from the group consisting of deuterium, halogen, -OH, -NH₂, -CN, C₁₋₄ alkyl, C₁₋₄ alkoxy, C₁₋₄ alkylamino, di-C₁₋₄ alkylamino, C₁₋₄ haloalkyl, and C₁₋₄ haloalkoxy;
Z is selected from the group consisting of a single bond, -S-, -O-, -NH-, and -N(C₁₋₃ alkyl)-;
R³ is selected from the group consisting of H, deuterium, and the following groups optionally substituted with one or more R^{3a}: C₁₋₆ alkyl, 3- to 10-membered cycloalkyl, 4- to 10-membered heterocycloalkyl, 6- to 10-membered aryl, benzo 4- to 6-membered heterocyclyl, 5- to 10-membered heteroaryl, 3- to 10-membered cycloalkyl C₁₋₃ alkylene, 4- to 10-membered heterocycloalkyl C₁₋₃ alkylene, 6- to 10-membered aryl C₁₋₃ alkylene, benzo 4- to 6-membered heterocyclyl C₁₋₃ alkylene, and 5- to 10-membered heteroaryl C₁₋₃ alkylene;
each R^{3a} is independently selected from the group consisting of deuterium, halogen, -OH, oxo, -NH₂, -CN, -C(O)NR^{3c}R^{3d}, -NR^{3c}C(O)R^{3d}, -NHC(O)NR^{3c}R^{3d}, -NR^{3c}C(O)OR^{3d}, -OC(O)R^{3d}, -C(O)OR^{3d}, -OC(O)OR^{3d}, -OC(O)NR^{3c}R^{3d}, -SO₂R^{3d}, -NHSO₂R^{3d}, -C₁₋₃ alkylene C(O)NR^{3c}R^{3d}, -C₁₋₃ alkylene NR^{3c}C(O)R^{3d}, -C₁₋₃ alkylene NHC(O)NR^{3c}R^{3d}, -C₁₋₃ alkylene NR^{3c}C(O)OR^{3d}, -C₁₋₃ alkylene OC(O)R^{3d}, -C₁₋₃ alkylene C(O)OR^{3d}, -C₁₋₃ alkylene OC(O)NR^{3c}R^{3d}, -C₁₋₃ alkylene SO₂R^{3d}, -C₁₋₃ alkylene OSO₂R^{3d}, -C₁₋₃ alkylene NHSO₂R^{3d}, and the following groups optionally substituted with one or more R^{3b}: C₁₋₄ alkyl, C₁₋₄ alkoxy, C₁₋₄ alkylamino, phenyl, 5-to 6-membered heteroaryl, phenyl C₁₋₃ alkylene, 5- to 6-membered heteroaryl C₁₋₃ alkylene, and 4- to 7-membered heterocycloalkyl C₁₋₃ alkylene optionally substituted with oxo;
R^{3c} is independently selected from the group consisting of H, deuterium, and C₁₋₆ alkyl;
R^{3d} is independently selected from the group consisting of H, deuterium, C₁₋₆ alkyl, C₁₋₆ alkoxy C₁₋₃ alkylene, and the following groups optionally substituted with one or more R^{3e}: phenyl, phenyl C₁₋₃ alkylene, 5- to 6-membered heteroaryl, and 4- to 8-membered heterocycloalkyl;
each R^{3b} is independently selected from the group consisting of deuterium, halogen, -OH, oxo, -NH₂, -CN, C₁₋₄ alkyl, C₁₋₄ alkoxy, C₁₋₄ alkylamino, and di-C₁₋₄ alkylamino;
each R^{3e} is independently selected from the group consisting of deuterium, halogen, -OH, formyl, oxo, C₁₋₄ alkyl, and C₁₋₄ haloalkyl;
R⁴ is selected from the group consisting of H, deuterium, halogen, -CN, C₁₋₄ alkyl, C₁₋₄ alkoxy, C₁₋₄ haloalkyl, and C₁₋₄ haloalkoxy;
ring A is selected from the group consisting of the following groups optionally substituted with one or more R⁵: 6-to 10-membered aryl, benzo 4- to 6-membered cycloalkenyl, benzo 4- to 6-membered heterocyclyl, and 5- to 10-membered heteroaryl;
each R⁵ is independently selected from the group consisting of deuterium, halogen, -CN, -OH, -NH₂, C₁₋₆ alkyl, C₂₋₆ alkenyl, C₂₋₆ alkynyl, C₁₋₆ haloalkyl, hydroxy C₁₋₆ alkylene, C₁₋₆ alkoxy, C₁₋₆ haloalkoxy, C₁₋₆ alkylamino, di-C₁₋₆ alkylamino, C₁₋₆ alkylthio, C₁₋₆ haloalkylthio, and 3- to 7-membered cycloalkyl optionally substituted with halogen or C₁₋₃ alkyl.

In some embodiments,
X is selected from the group consisting of -N-, -CH-, and -C(C₁₋₃ alkyl)-;
L¹ is selected from the group consisting of -S-, -O-, -NH-, -N(C₁₋₃ alkyl)-, and the following groups optionally substituted with one or more R¹: C₁₋₄ alkylene and C₁₋₄ heteroalkylene;
each R¹ is independently selected from the group consisting of oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino;
R² is selected from the group consisting of H, halogen, -OH, -NH₂, -CN, and the following groups optionally substituted with one or more R^{2a}: C₁₋₆ alkyl, C₁₋₆ heteroalkyl, 3- to 10-membered cycloalkyl-L²-, 4- to 10-membered heterocycloalkyl-L²-, 6- to 10-membered aryl-L²-, benzo 4- to 6-membered heterocyclyl-L²-, and 5-to 10-membered heteroaryl-L²-;
L² is selected from the group consisting of a single bond, -O-, -S-, -NH-, -N(C₁₋₆ alkyl)-, C₁₋₆ alkylene, C₁₋₆ heteroalkylene, C₂₋₆ alkenylene, and C₁₋₆ heteroalkenylene;
each R^{2a} is independently selected from the group consisting of halogen, -OH, oxo, -NH₂, -CN, -C(O)NR^{2c}R^{2d}, -NR^{2c}C(O)R^{2d}, -NHC(O)NR^{2c}R^{2d}, -NR^{2c}C(O)OR^{2d}, -OC(O)R^{2d}, -C(O)OR^{2d}, -OC(O)OR^{2d}, -OC(O)NR^{2c}R^{2d}, -SO₂R^{2d}, -NHSO₂R^{2d}, -C₁₋₆ alkylene C(O)NR^{2c}R^{2d}, -C₁₋₆ alkylene NR^{2c}C(O)R^{2d}, -C₁₋₆ alkylene NHC(O)NR^{2c}R^{2d}, -C₁₋₆ alkylene NR^{2c}C(O)OR^{2d}, -C₁₋₆ alkylene OC(O)R^{2d}, -C₁₋₆ alkylene C(O)OR^{2d}, -C₁₋₆ alkylene OC(O)NR^{2c}R^{2d}, -C₁₋₆ alkylene SO₂R^{2d}, -C₁₋₆ alkylene OSO₂R^{2d}, -C₁₋₆ alkylene NHSO₂R^{2d}, and the following groups optionally substituted with one or more R^{2b}: C₁₋₆ alkyl, C₁₋₆ alkoxy, C₁₋₆ alkylamino, di-C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{2c} is independently selected from the group consisting of H and C₁₋₆ alkyl;
R^{2d} is independently selected from the group consisting of H and the following groups optionally substituted with one or more R^{2e}: C₁₋₆ alkyl, C₁₋₆ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{2e} is independently selected from the group consisting of halogen, -OH, oxo, -NH₂, -CN, C₁₋₄ alkyl, C₁₋₄ alkoxy, C₁₋₄ haloalkyl, and C₁₋₄ haloalkoxy;
each R^{2b} is independently selected from the group consisting of halogen, -OH, -NH₂, -CN, C₁₋₄ alkyl, C₁₋₄ alkoxy, C₁₋₄ alkylamino, di-C₁₋₄ alkylamino, C₁₋₄ haloalkyl, and C₁₋₄ haloalkoxy;
Z is selected from the group consisting of a single bond, -S-, -O-, -NH-, and -N(C₁₋₃ alkyl)-;
R³ is selected from the group consisting of H and the following groups optionally substituted with one or more R^{3a}: C₁₋₆ alkyl, 3- to 10-membered cycloalkyl, 4- to 10-membered heterocycloalkyl, 6- to 10-membered aryl, benzo 4- to 6-membered heterocyclyl, 5- to 10-membered heteroaryl, 3- to 10-membered cycloalkyl C₁₋₃ alkylene, 4- to 10-membered heterocycloalkyl C₁₋₃ alkylene, 6- to 10-membered aryl C₁₋₃ alkylene, benzo 4- to 6-membered heterocyclyl C₁₋₃ alkylene, and 5- to 10-membered heteroaryl C₁₋₃ alkylene;
each R^{3a} is independently selected from the group consisting of halogen, -OH, oxo, -NH₂, -CN, -C(O)NR^{3c}R^{3a}, -NR^{3c}C(O)R^{3d}, -NHC(O)NR^{3c}R^{3d}, -NR^{3c}C(O)OR^{3d}, -OC(O)R^{3d}, -C(O)OR^{3d}, -OC(O)OR^{3d}, -OC(O)NR^{3c}R^{3d}, -SO₂R^{3d}, -NHSO₂R^{3d}, -C₁₋₃ alkylene C(O)NR^{3c}R^{3d}, -C₁₋₃ alkylene NR^{3c}C(O)R^{3d}, -C₁₋₃ alkylene NHC(O)NR^{3c}R^{3d}, -C₁₋₃ alkylene NR^{3c}C(O)OR^{3d}, -C₁₋₃ alkylene OC(O)R^{3d}, -C₁₋₃ alkylene C(O)OR^{3d}, -C₁₋₃ alkylene OC(O)NR^{3c}R^{3d}, -C₁₋₃ alkylene SO₂R^{3d}, -C₁₋₃ alkylene OSO₂R^{3d}, -C₁₋₃ alkylene NHSO₂R^{3d}, and the following groups optionally substituted with one or more R^{3b}: C₁₋₄ alkyl, C₁₋₄ alkoxy, C₁₋₄ alkylamino, phenyl, 5- to 6-membered heteroaryl, phenyl C₁₋₃ alkylene, 5- to 6-membered heteroaryl C₁₋₃ alkylene, and 4- to 7-membered heterocycloalkyl C₁₋₃ alkylene optionally substituted with oxo;
R^{3c} is independently selected from the group consisting of H and C₁₋₆ alkyl;
R^{3d} is independently selected from the group consisting of H, C₁₋₆ alkyl, C₁₋₆ alkoxy C₁₋₃ alkylene, and the following groups optionally substituted with one or more R^{3e}: phenyl, phenyl C₁₋₃ alkylene, 5- to 6-membered heteroaryl, and 4- to 8-membered heterocycloalkyl;
each R^{3b} is independently selected from the group consisting of halogen, -OH, oxo, -NH₂, -CN, C₁₋₄ alkyl, C₁₋₄ alkoxy, C₁₋₄ alkylamino, and di-C₁₋₄ alkylamino;
each R^{3e} is independently selected from the group consisting of halogen, -OH, formyl, oxo, C₁₋₄ alkyl, and C₁₋₄ haloalkyl;
R⁴ is selected from the group consisting of H, halogen, -CN, C₁₋₄ alkyl, C₁₋₄ alkoxy, C₁₋₄ haloalkyl, and C₁₋₄ haloalkoxy;
ring A is selected from the group consisting of the following groups optionally substituted with one or more R⁵: 6-to 10-membered aryl, benzo 4- to 6-membered cycloalkenyl, benzo 4- to 6-membered heterocyclyl, and 5- to 10-membered heteroaryl;
each R⁵ is independently selected from the group consisting of halogen, -CN, -OH, -NH₂, C₁₋₆ alkyl, C₂₋₆ alkenyl, C₂₋₆ alkynyl, C₁₋₆ haloalkyl, hydroxy C₁₋₆ alkylene, C₁₋₆ alkoxy, C₁₋₆ haloalkoxy, C₁₋₆ alkylamino, di-C₁₋₆ alkylamino, C₁₋₆ alkylthio, C₁₋₆ haloalkylthio, and 3- to 7-membered cycloalkyl optionally substituted with halogen or C₁₋₃ alkyl.

In some embodiments, R^{3a} is independently selected from the group consisting of the following groups optionally substituted with one or more R^{3b}: =NH, =CH₂, =N(C₁₋₄ alkyl), =CH(C₁₋₄ alkyl), and =C(C₁₋₄ alkyl)₂.

The present disclosure relates to a compound of formula (II-1) or formula (II-2) or formula (III) or formula (III-a) or formula (III-b) or formula (III-c) or formula (IV) or formula (IV-a) or formula (IV-b) or formula (IV-c), a stereoisomer thereof, a tautomer thereof, a deuterated compound thereof, or a pharmaceutically acceptable salt thereof, 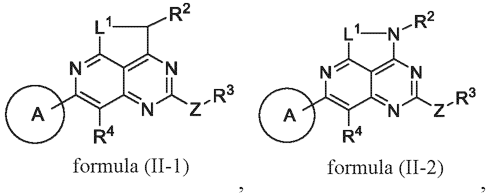 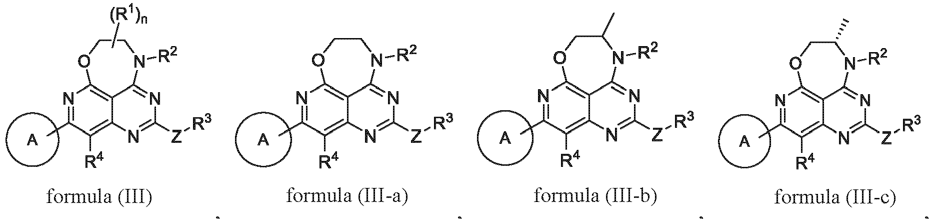 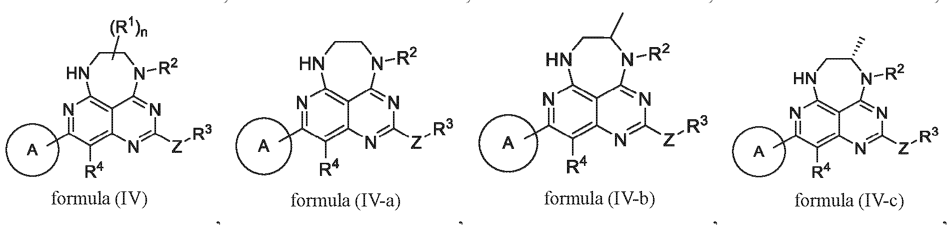 wherein, L¹, R¹, R², R³, R⁴, Z, and ring A moieties are as defined above; n is selected from the group consisting of 0, 1, 2, 3, 4, and 5.

In some embodiments, n is selected from the group consisting of 0, 1, 2, and 3.

In some embodiments, the present disclosure encompasses the variables defined above and embodiments thereof, as well as any combination thereof.

In some embodiments, the "one or more" in the present disclosure may refer to an integer ranging from one to ten. For example, "one or more" refers to one, two, three, four, five, six, seven, eight, nine, or ten; or "one or more" refers to one, two, three, four, five, or six; or "one or more" refers to one, two, or three.

The present disclosure further relates to the following compounds, stereoisomers thereof, tautomers thereof, deuterated compounds thereof, or pharmaceutically acceptable salts thereof:

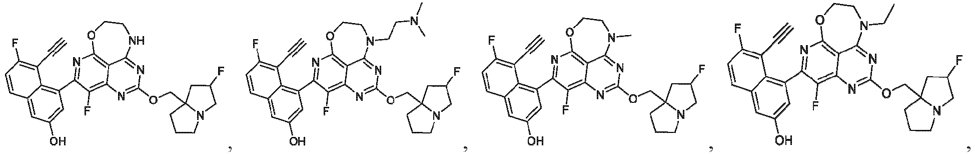

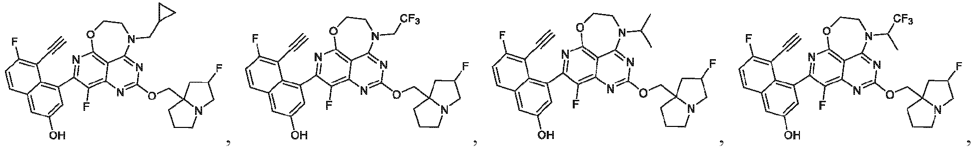

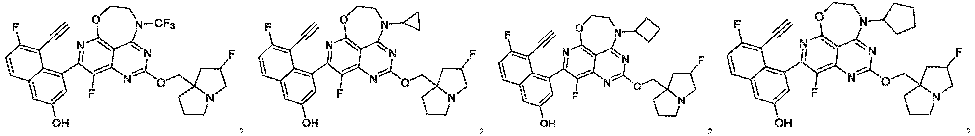

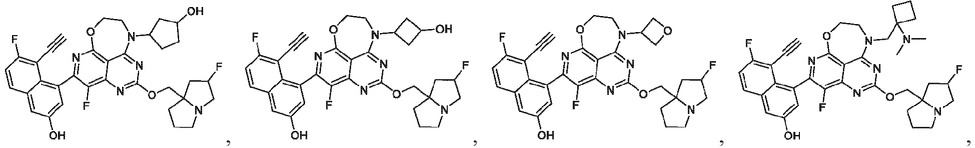

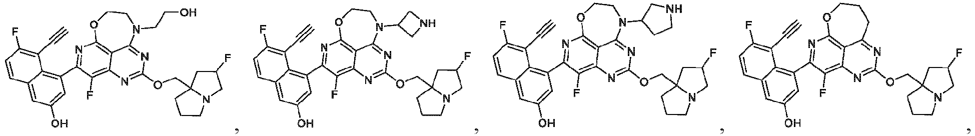

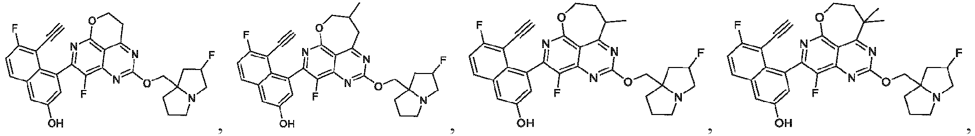

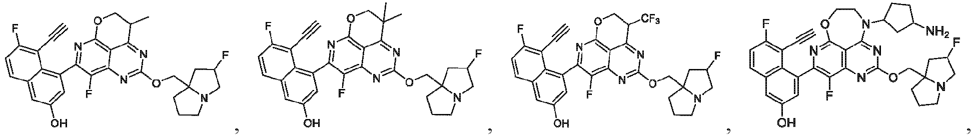

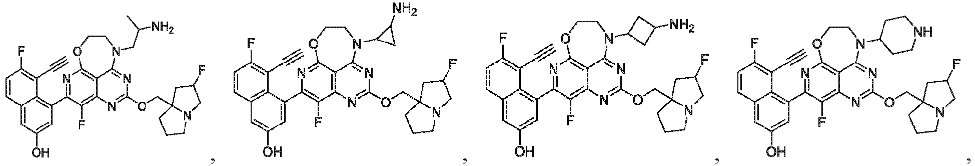

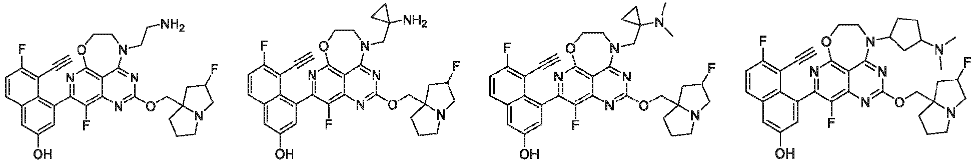

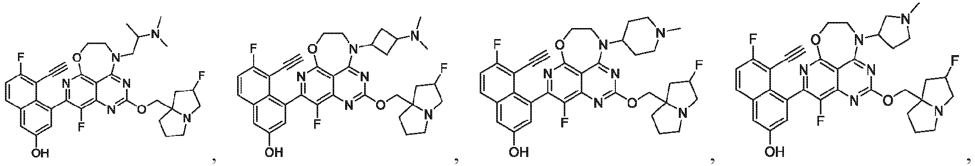

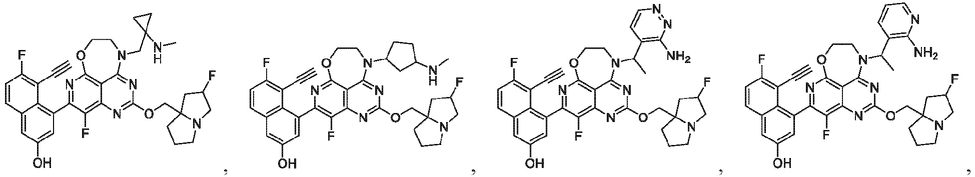

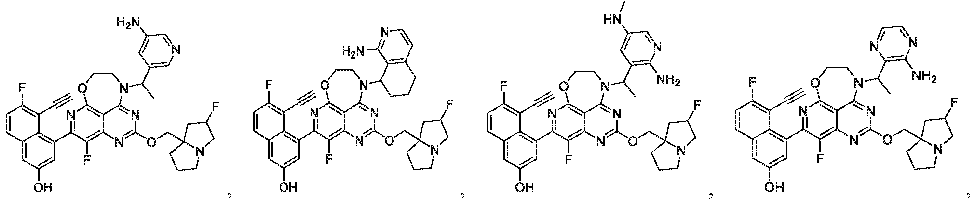

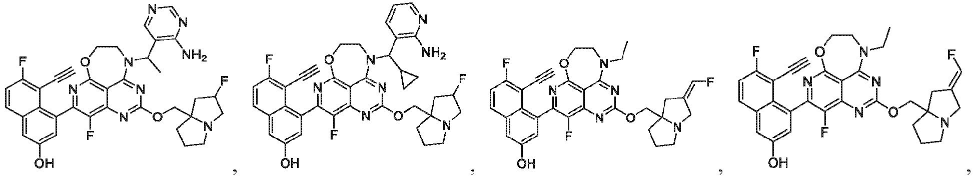

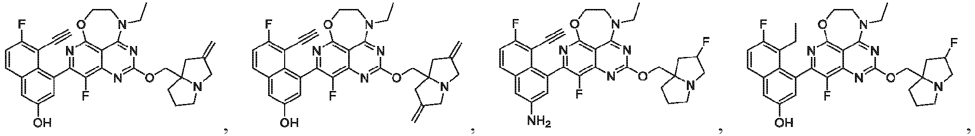

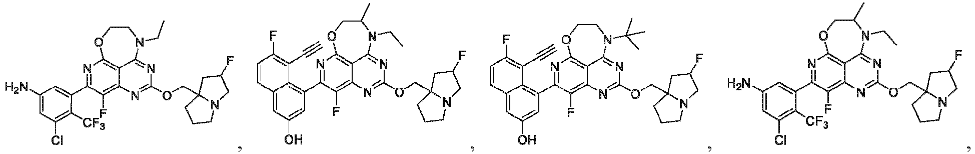

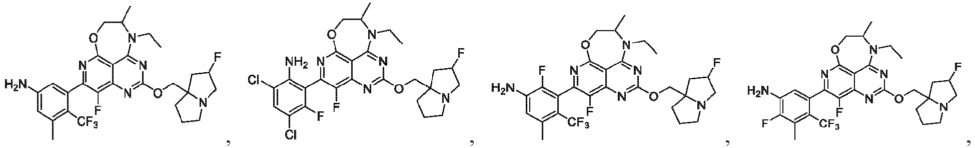

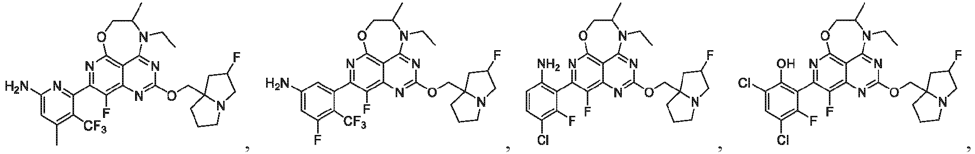

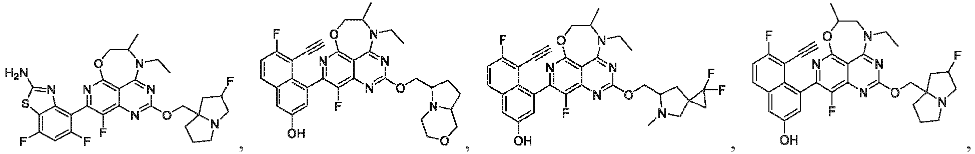

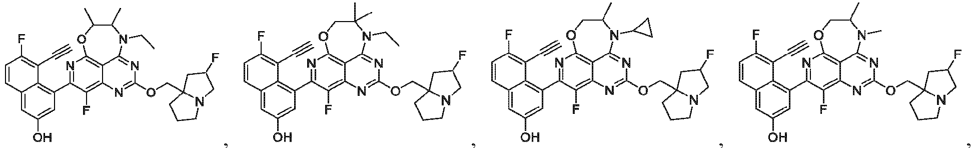

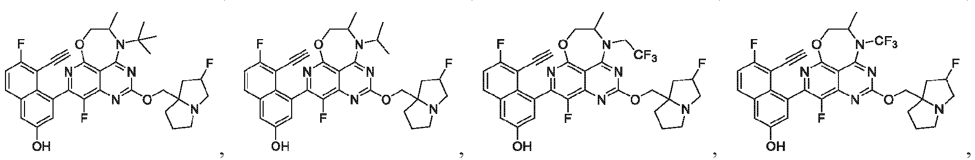

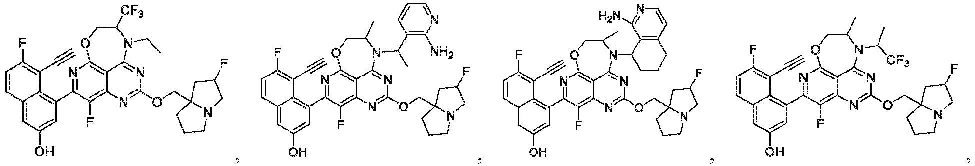

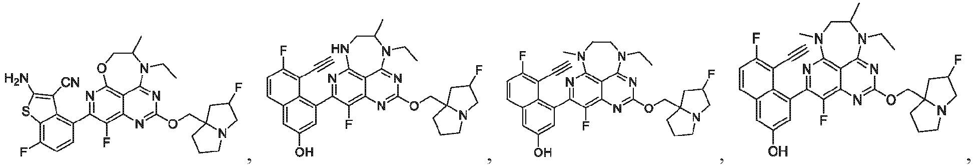

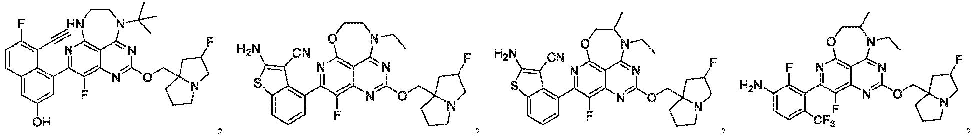

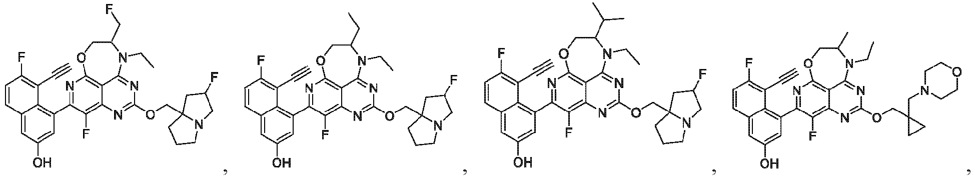

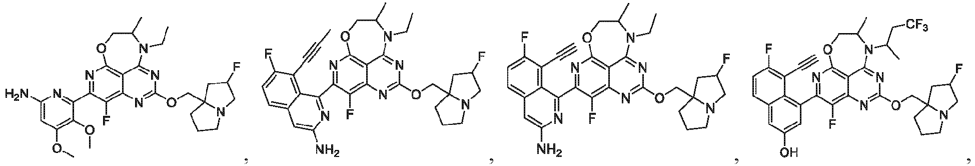

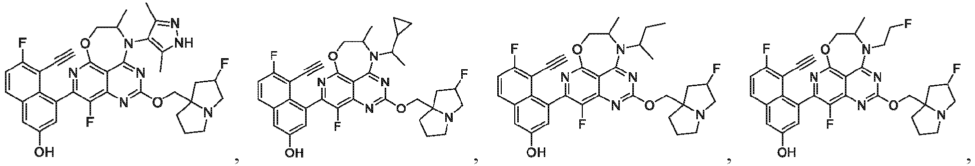

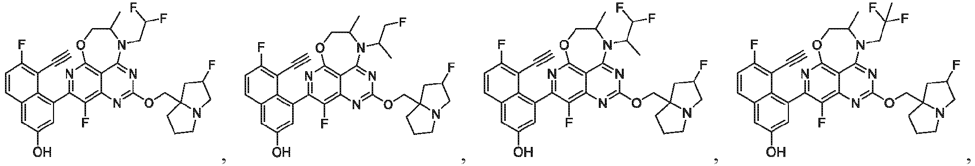

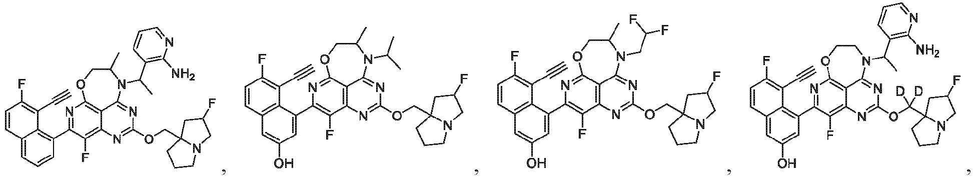

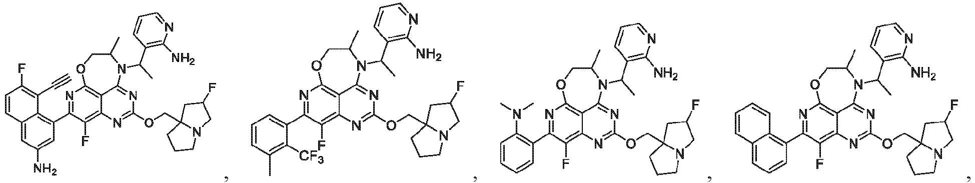

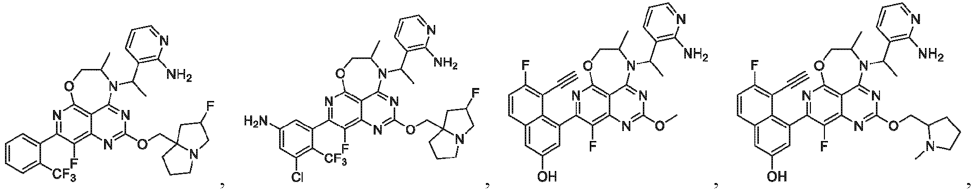

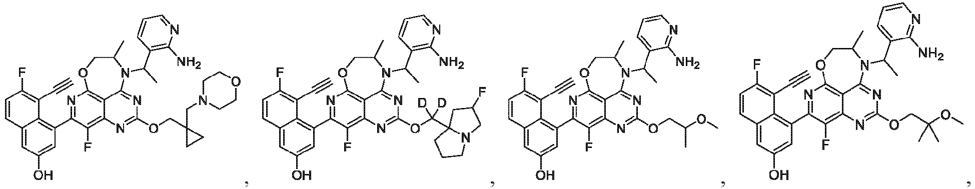

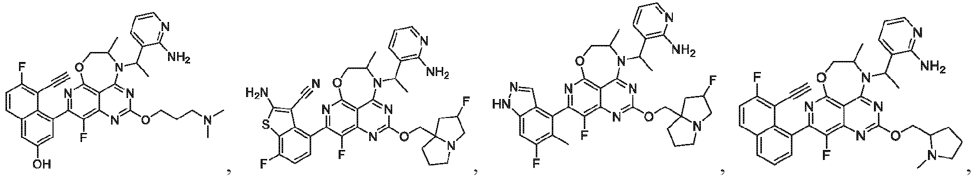

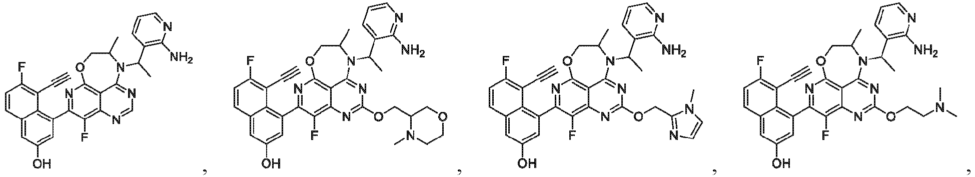

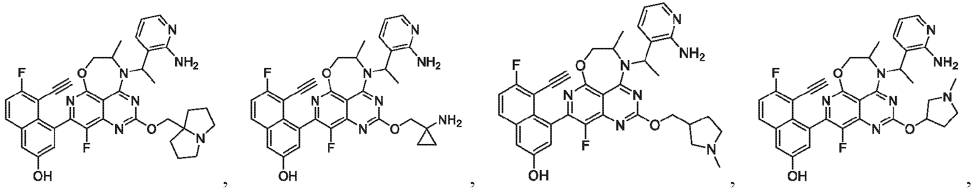

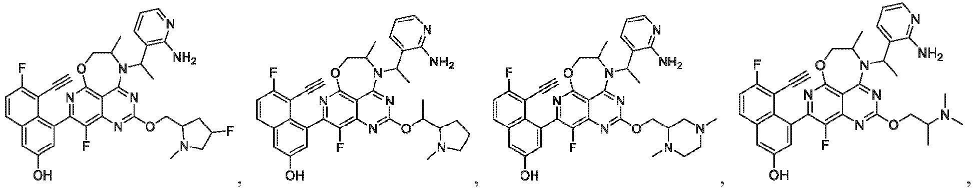

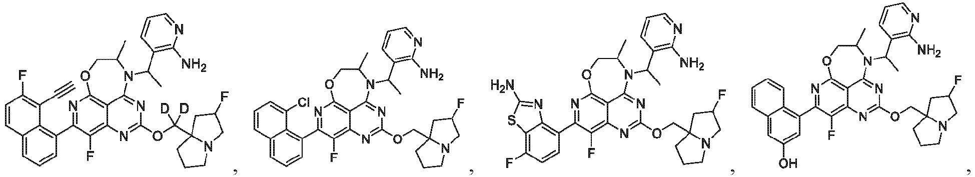

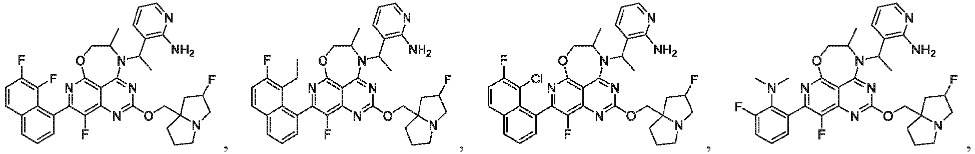

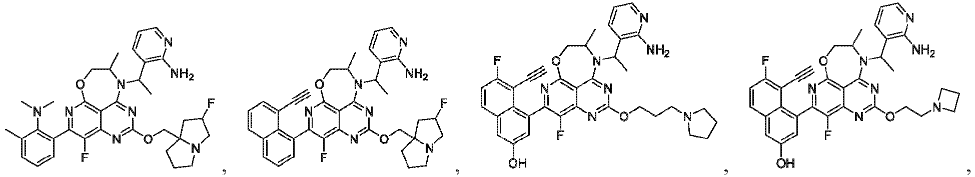

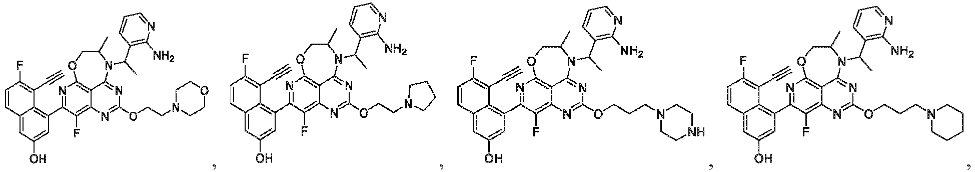

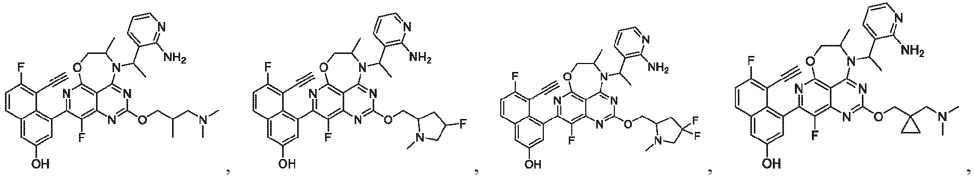

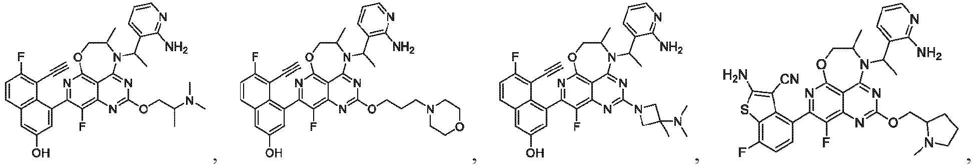

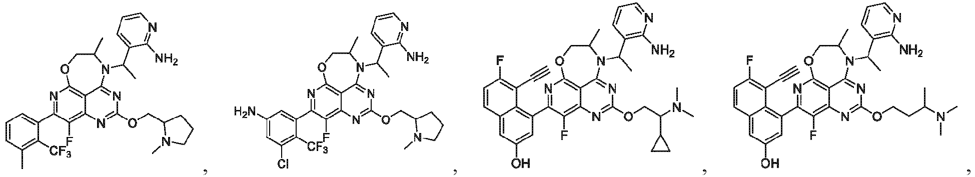

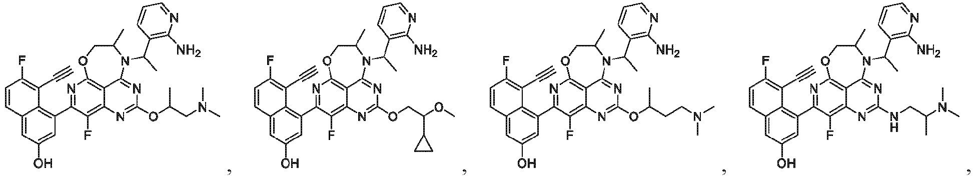

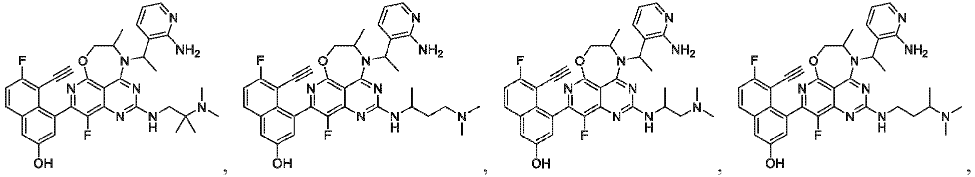

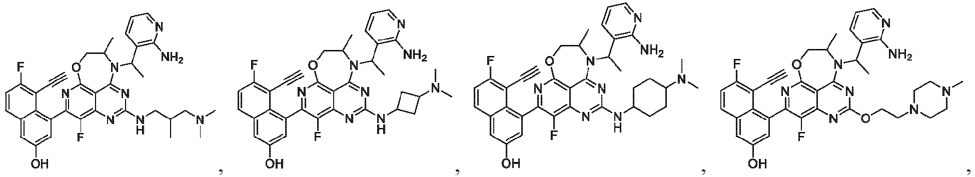

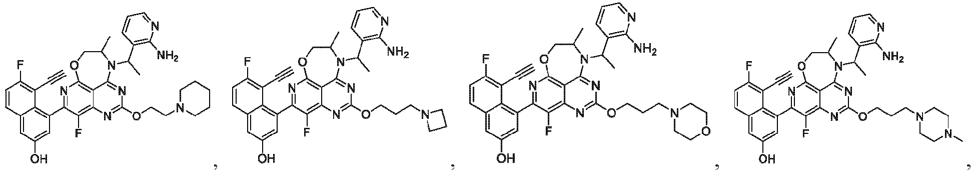

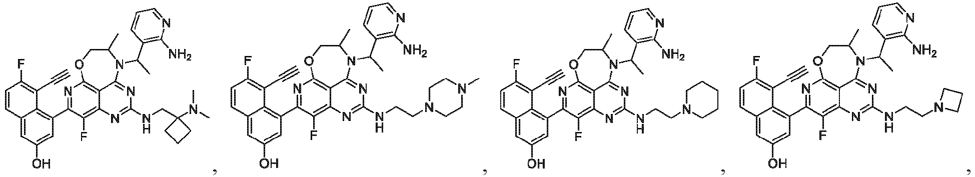

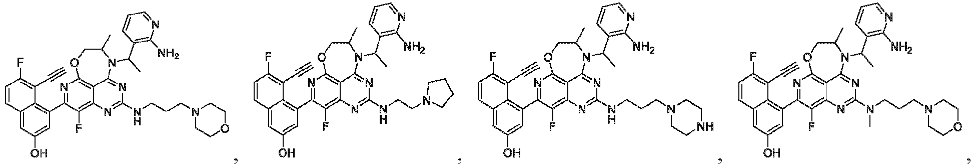

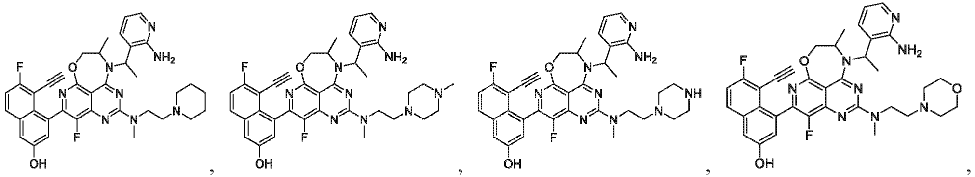

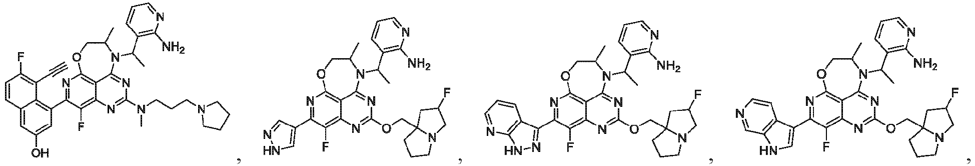

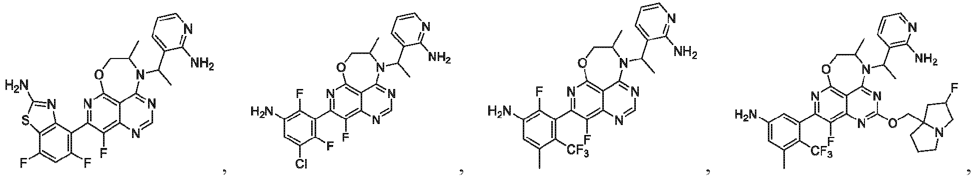

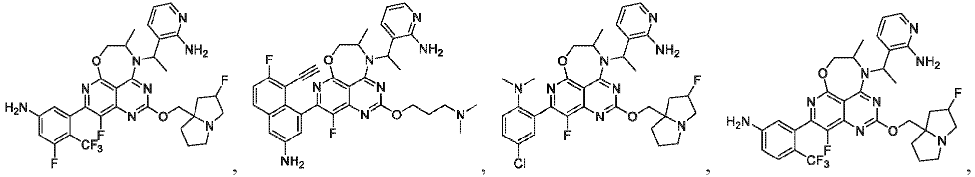

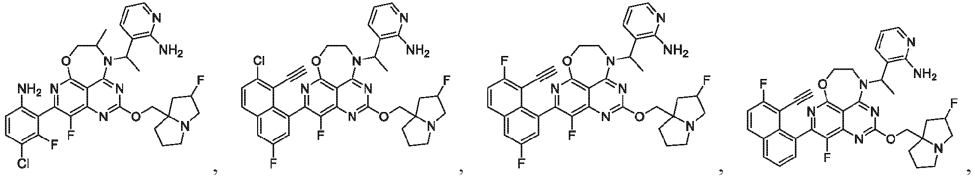

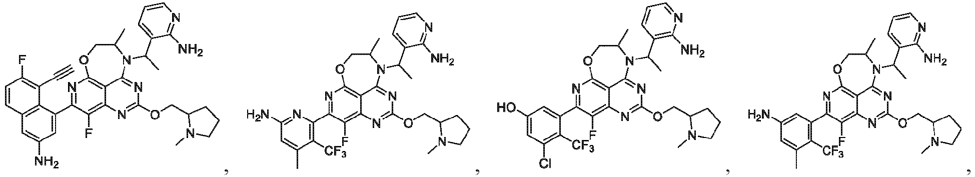

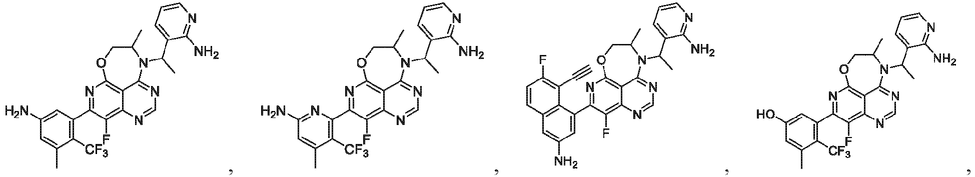

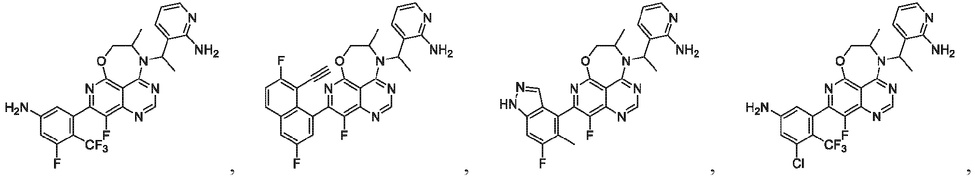

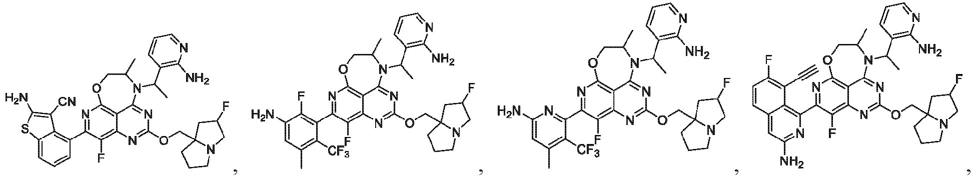

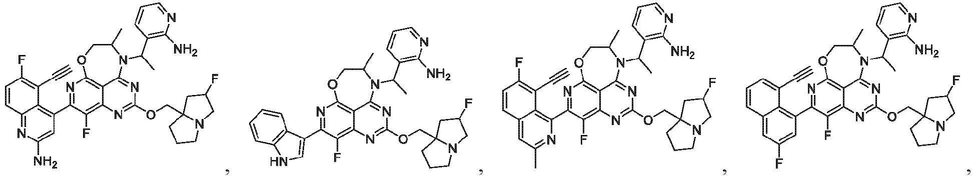

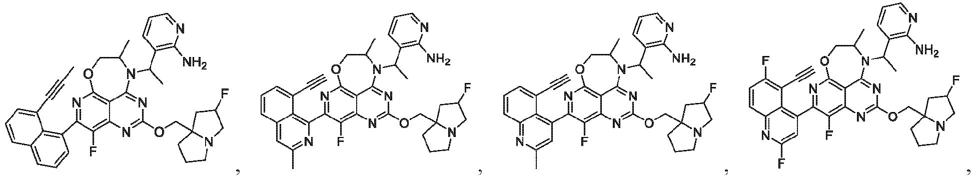

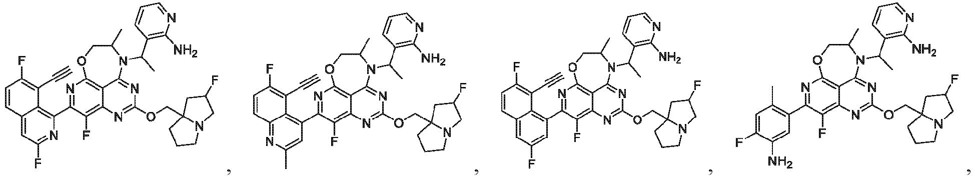

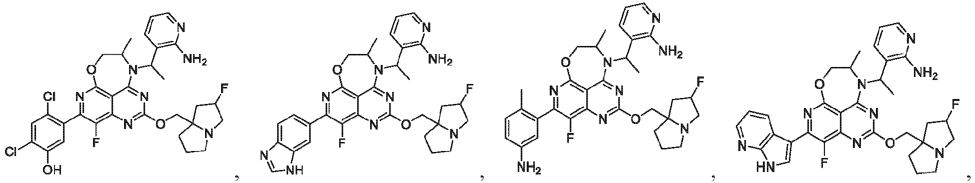

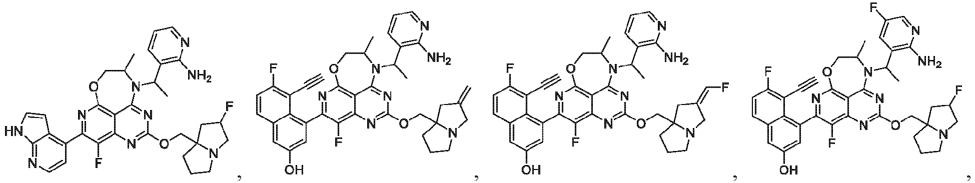

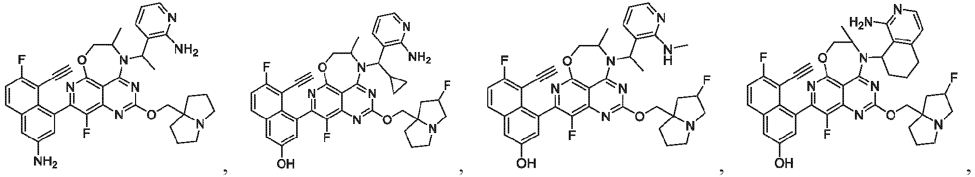

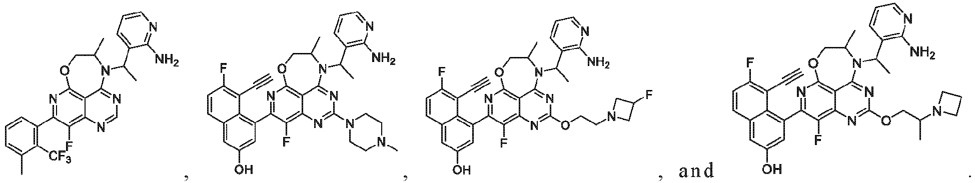

The present disclosure further relates to the following compounds, stereoisomers thereof, tautomers thereof, deuterated compounds thereof, or pharmaceutically acceptable salts thereof:

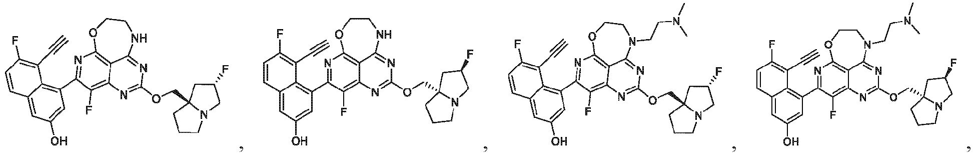

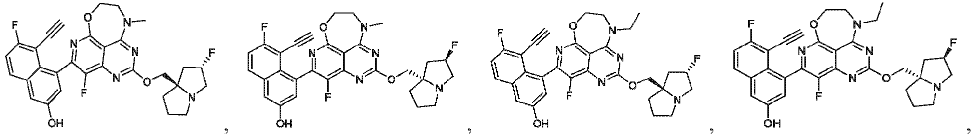

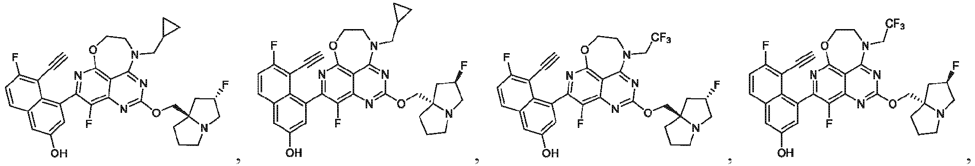

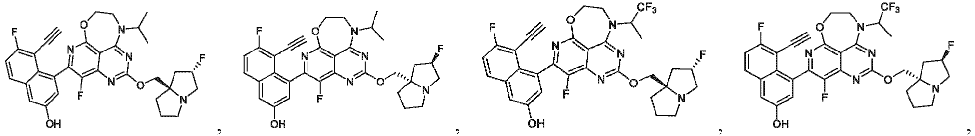

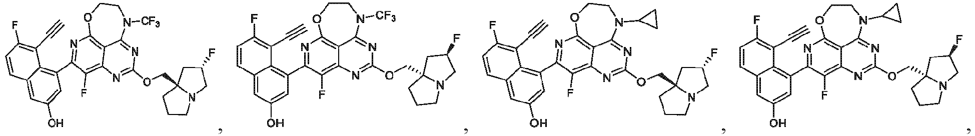

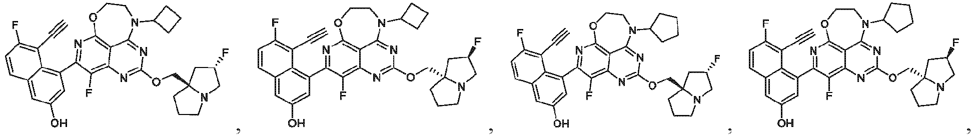

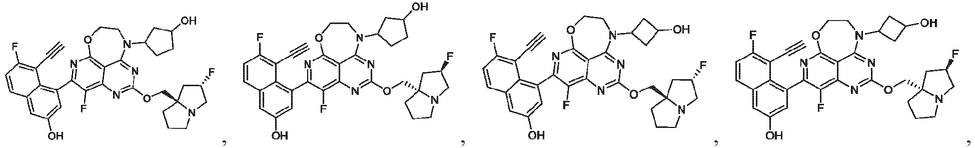

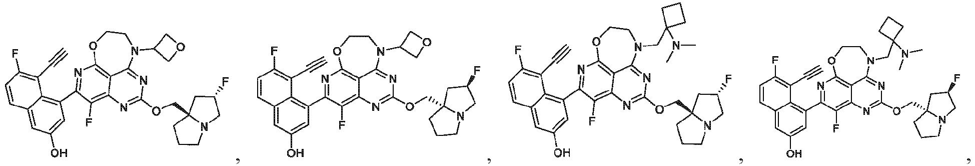

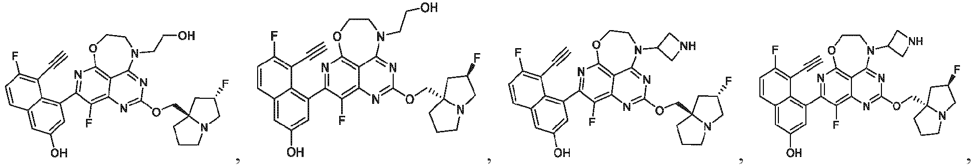

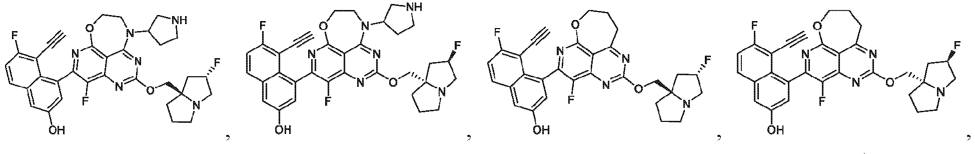

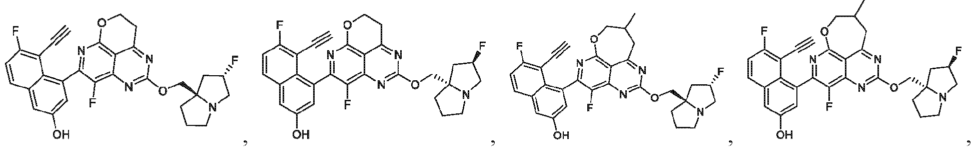

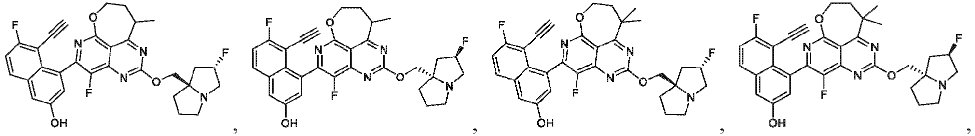

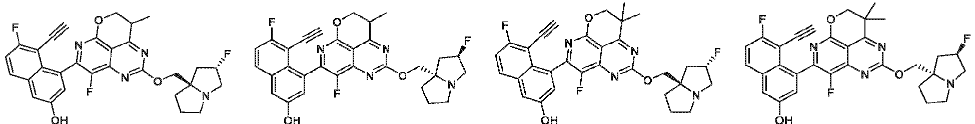

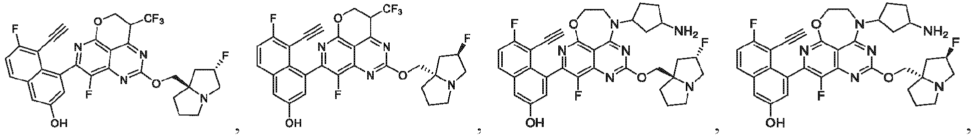

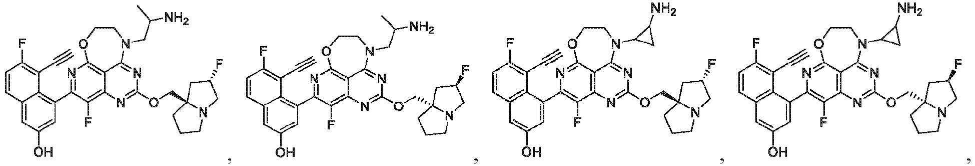

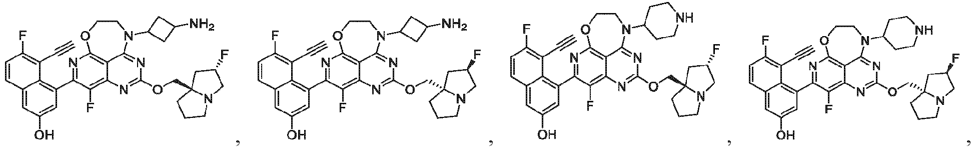

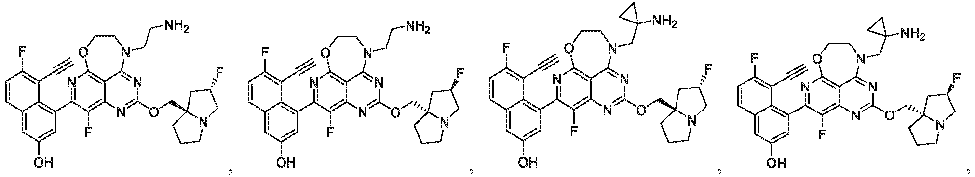

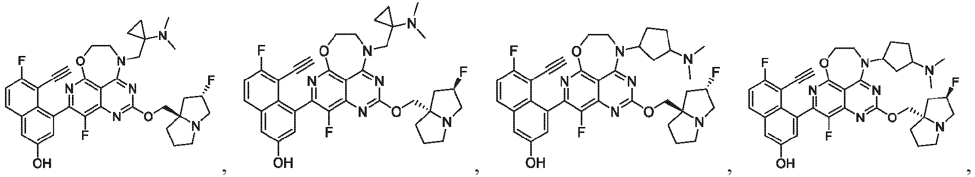

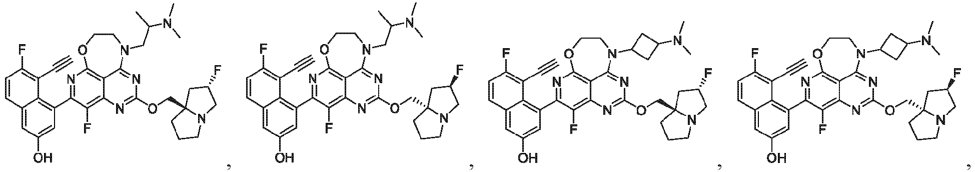

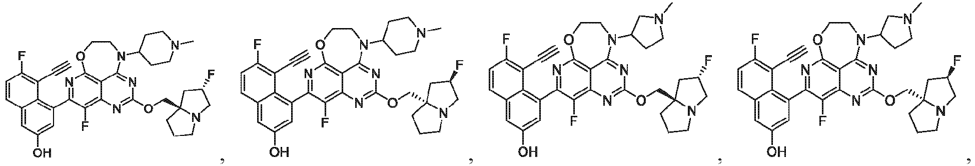

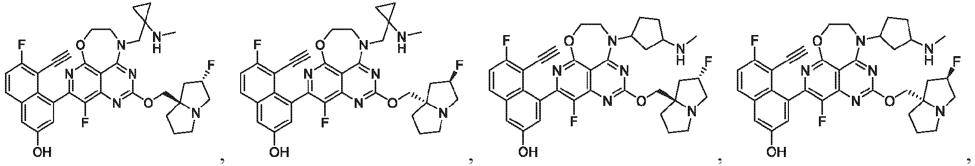

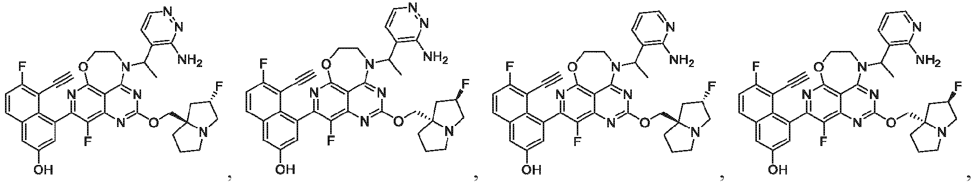

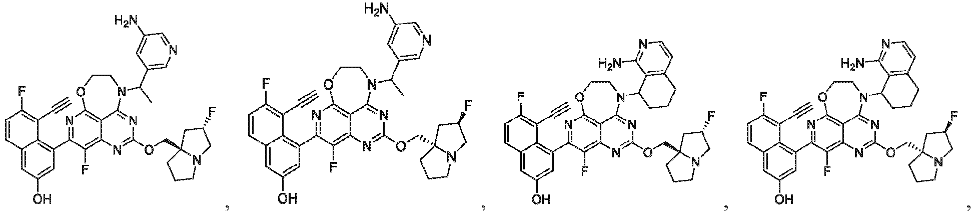

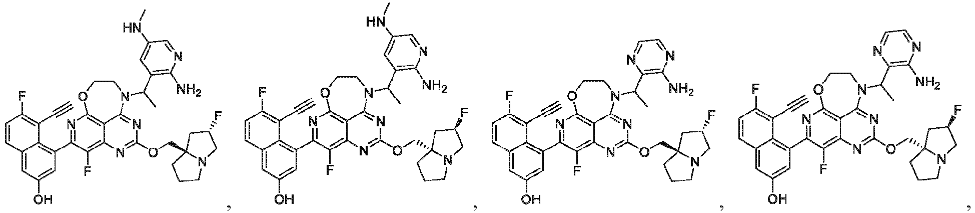

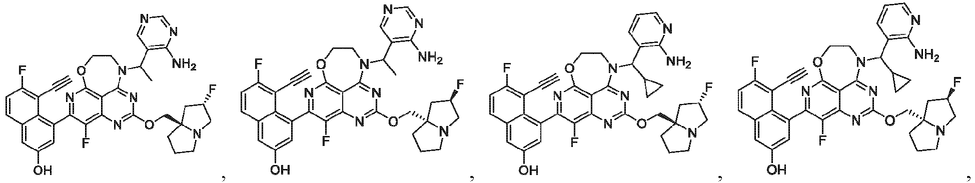

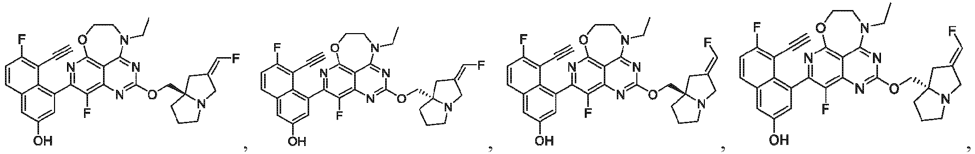

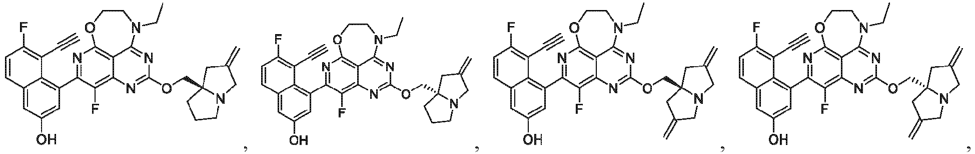

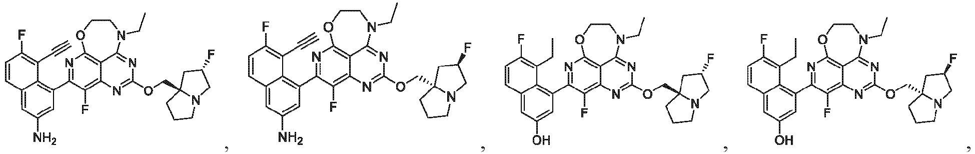

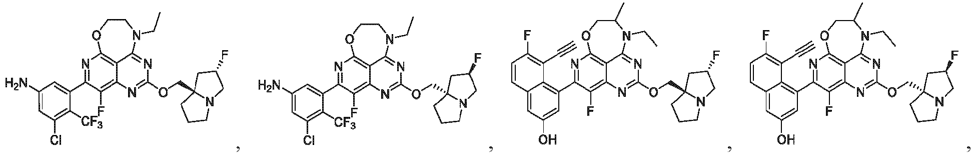

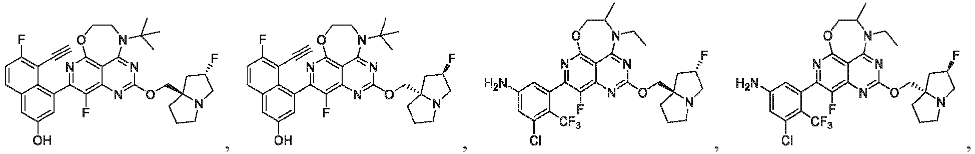

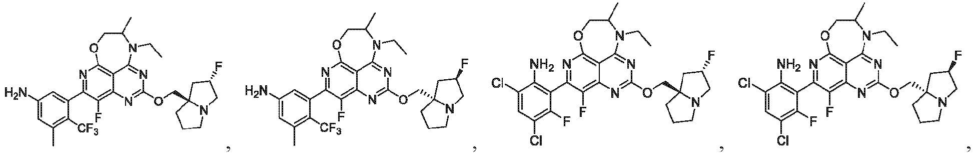

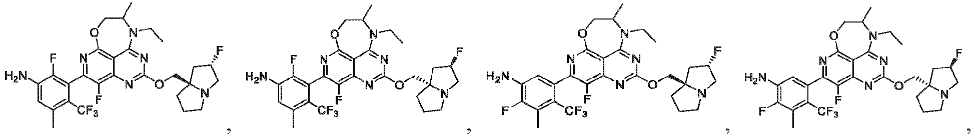

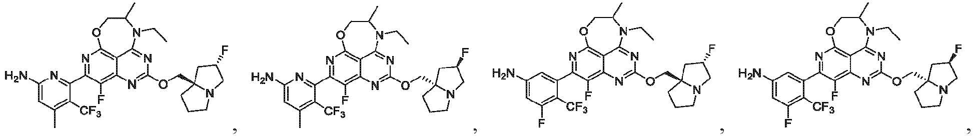

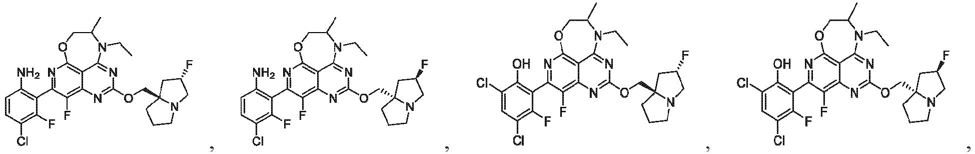

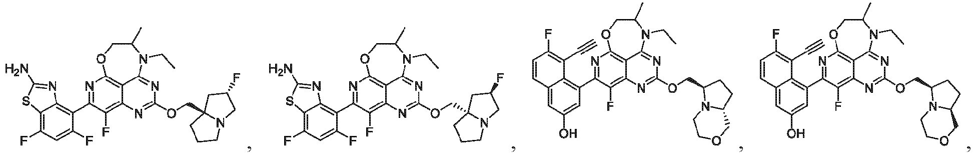

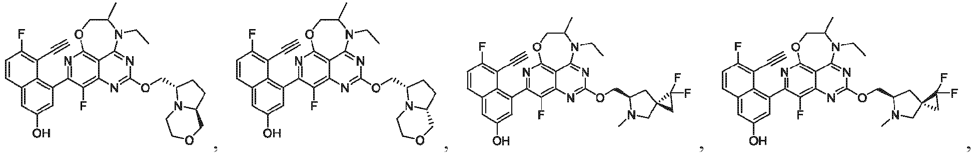

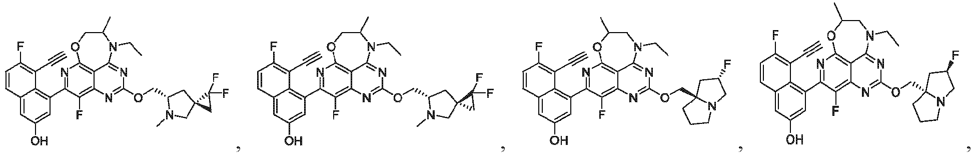

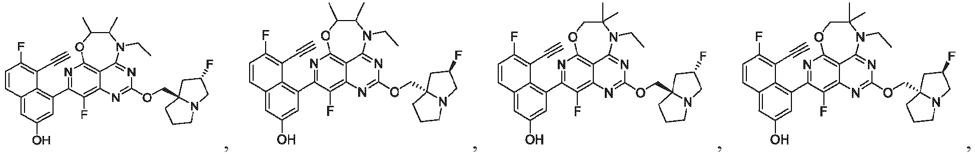

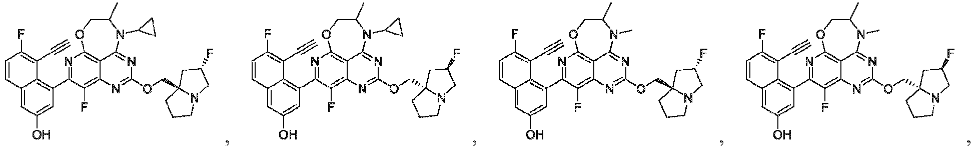

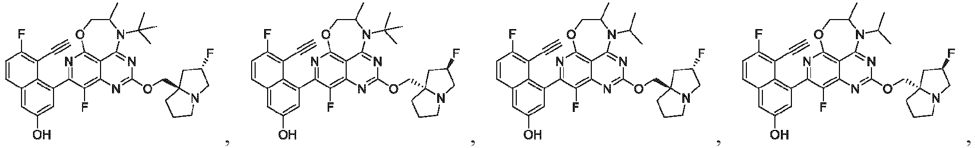

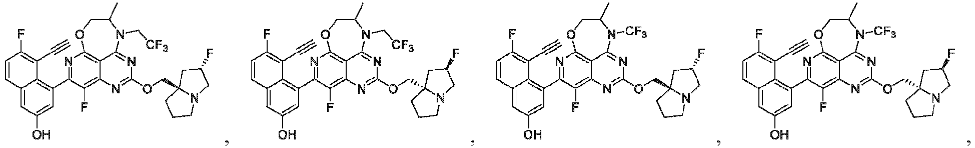

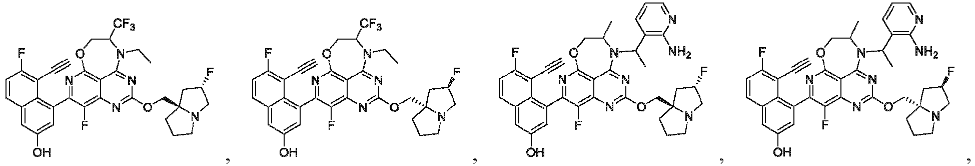

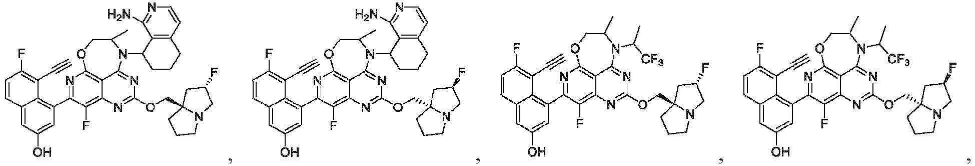

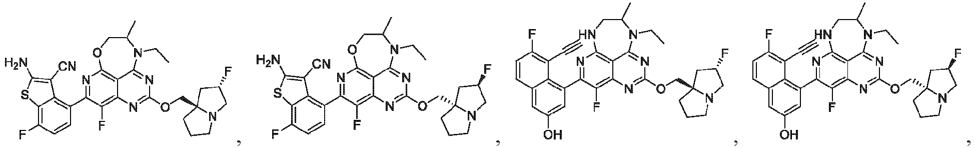

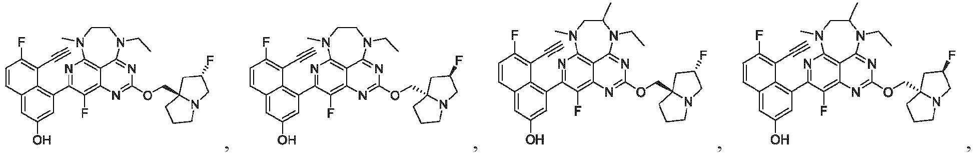

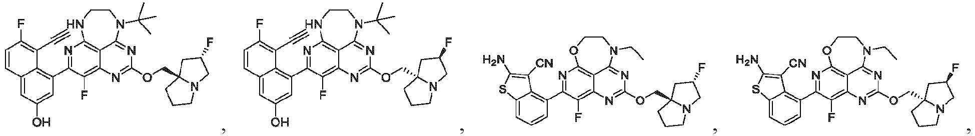

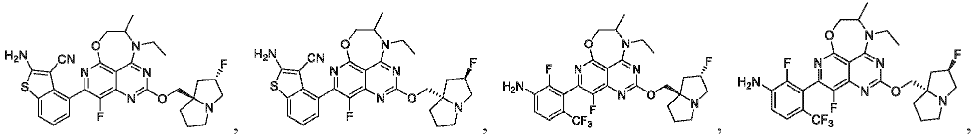

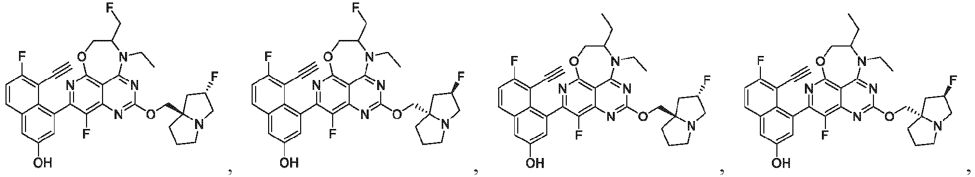

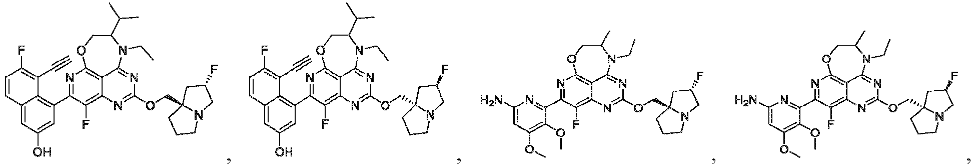

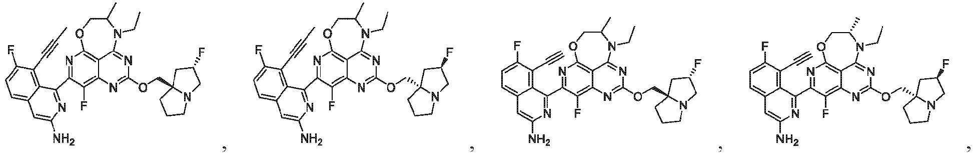

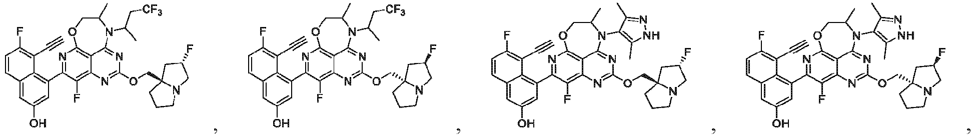

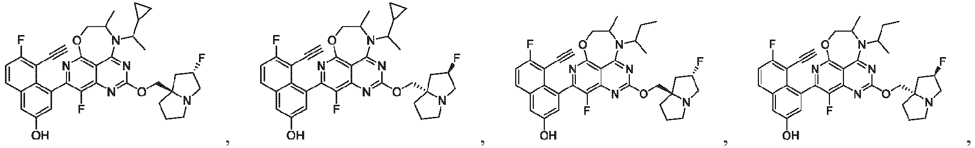

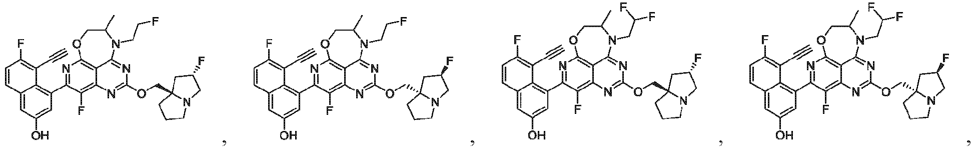

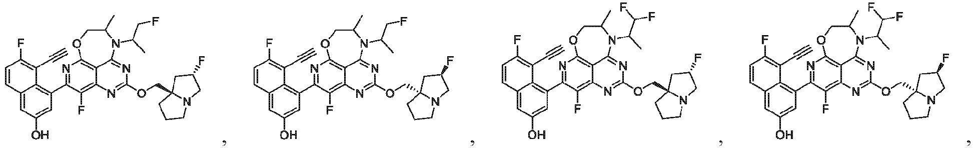

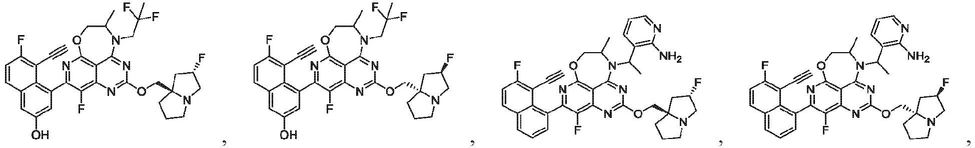

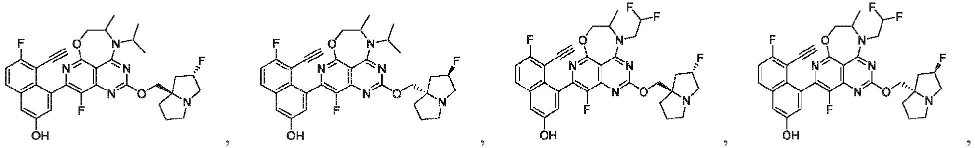

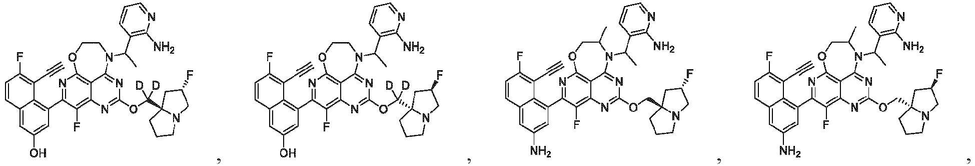

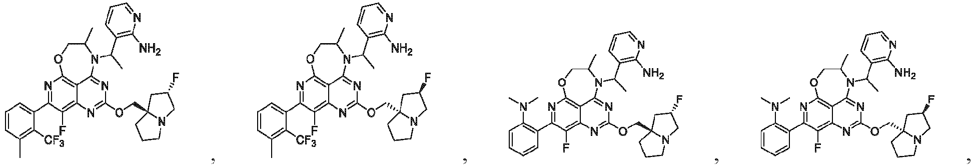

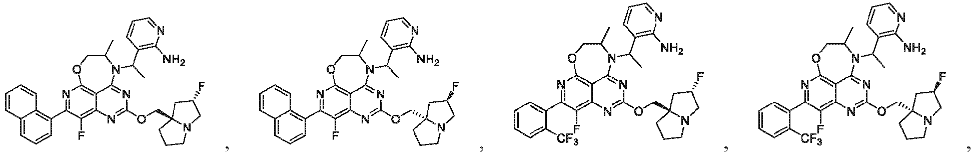

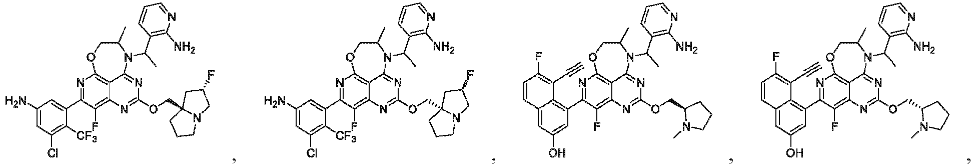

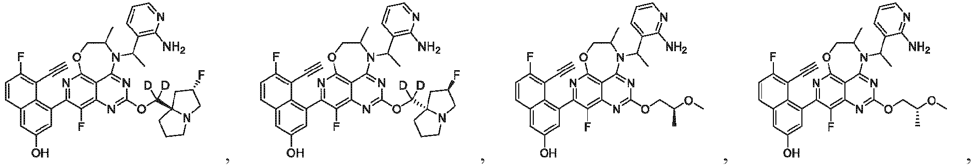

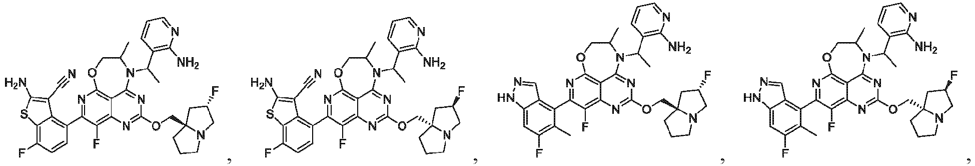

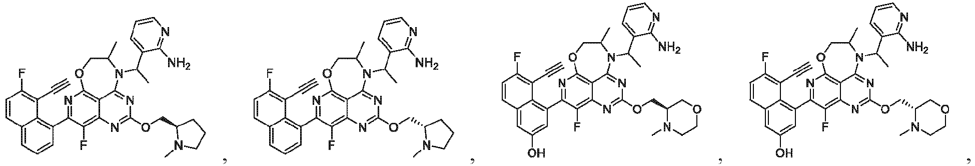

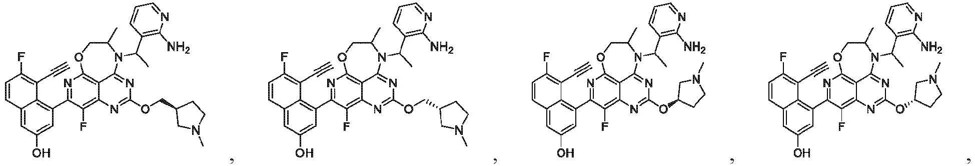

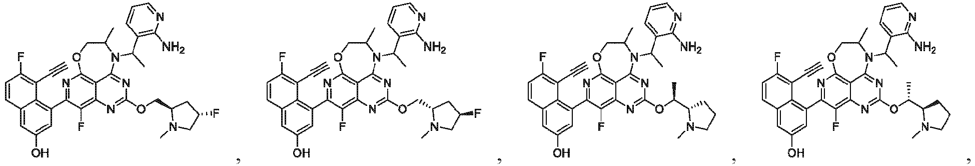

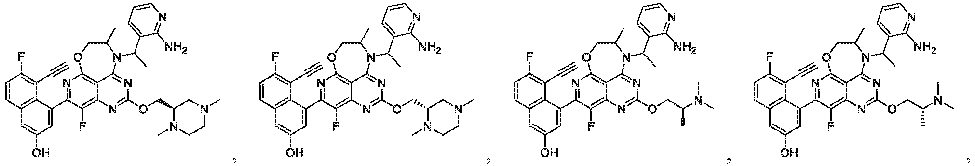

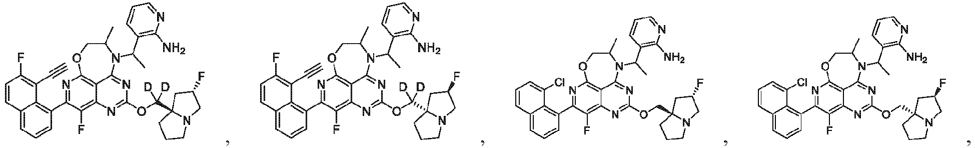

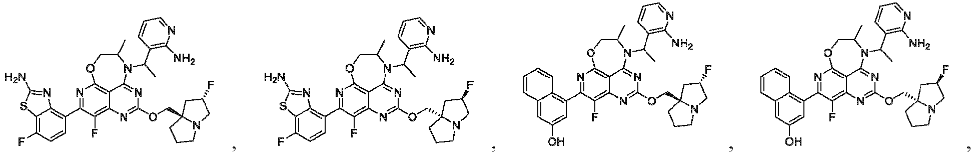

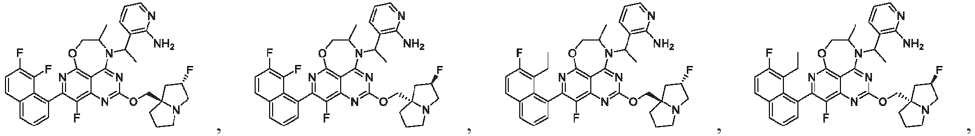

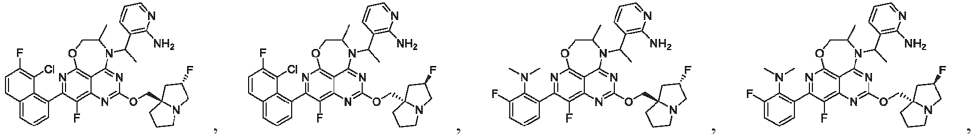

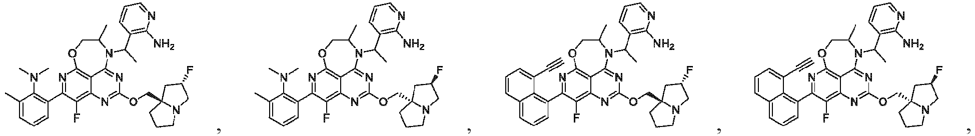

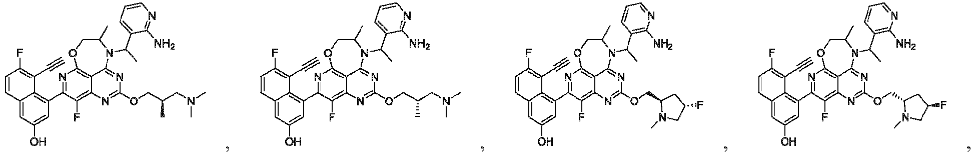

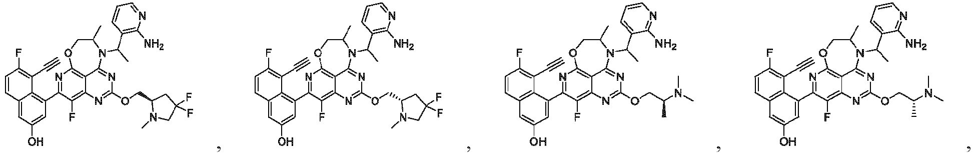

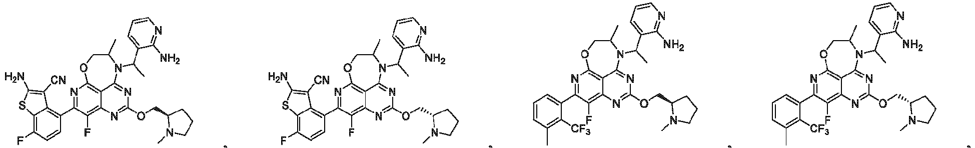

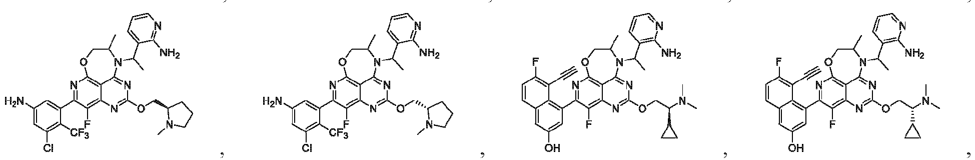

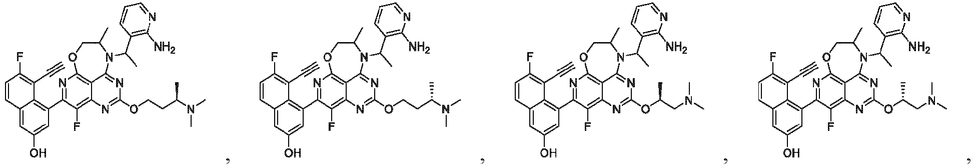

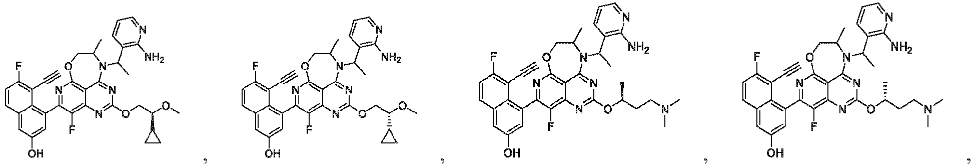

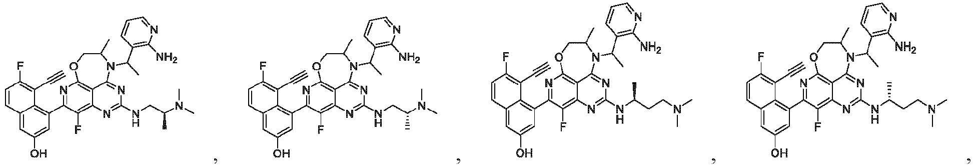

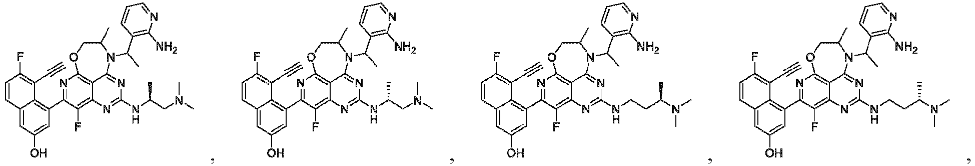

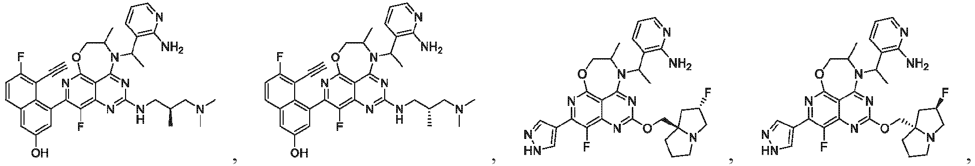

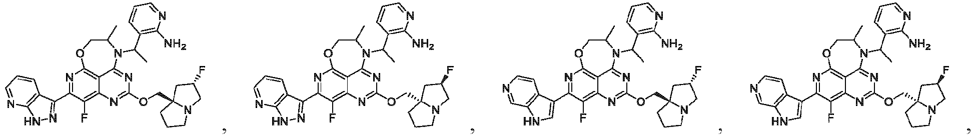

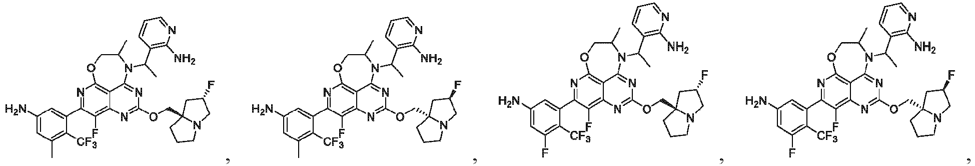

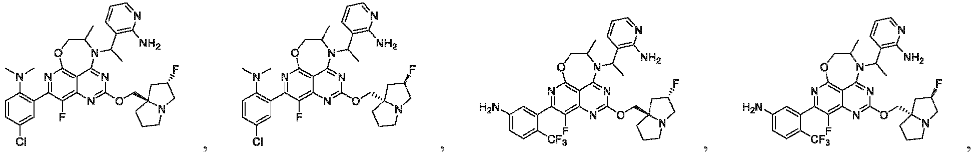

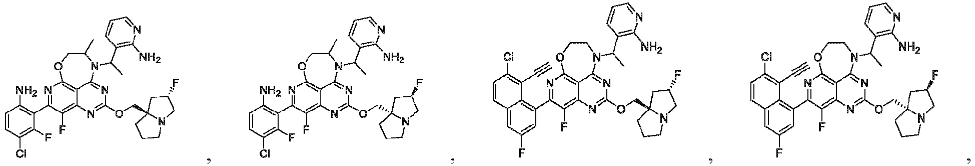

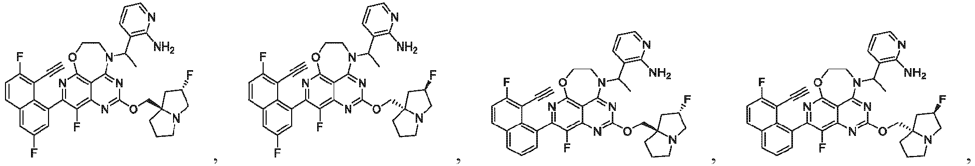

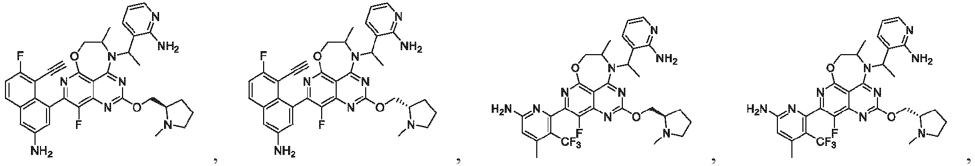

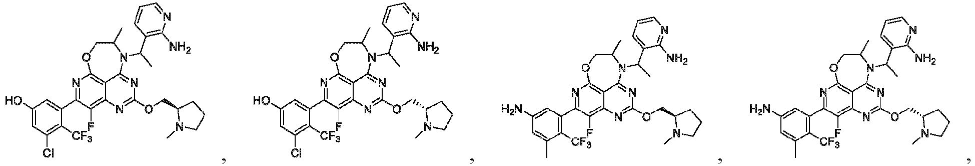

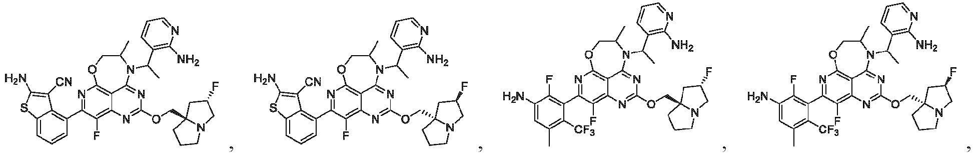

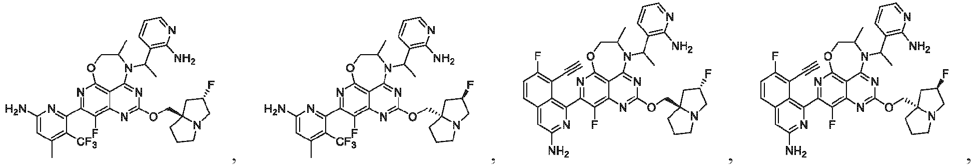

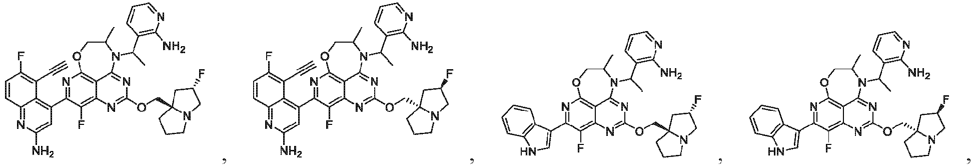

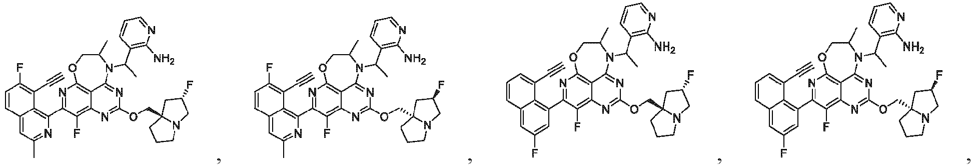

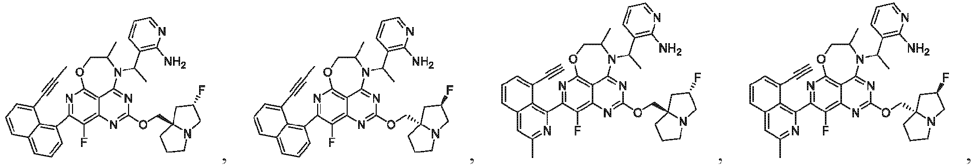

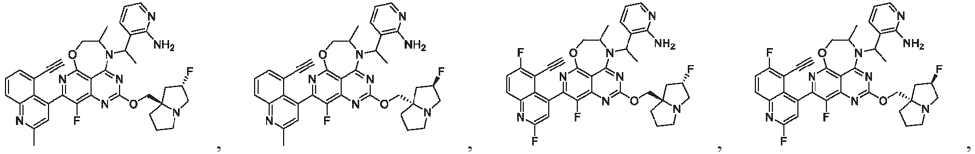

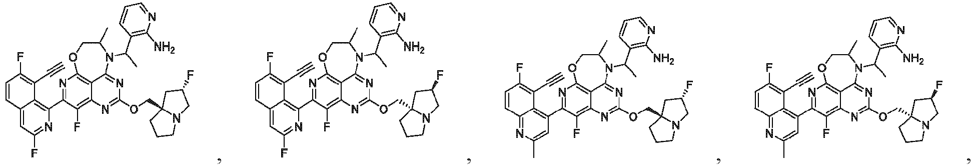

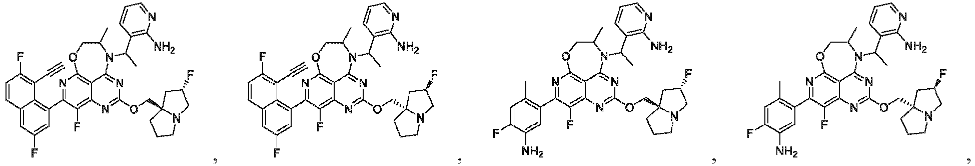

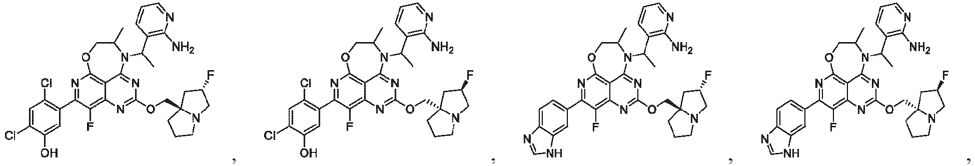

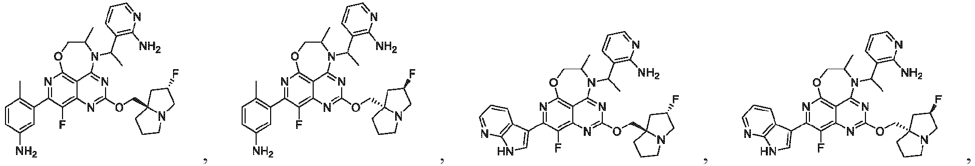

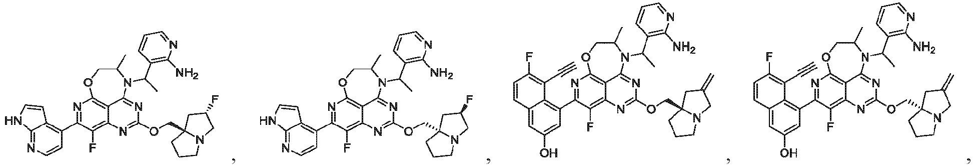

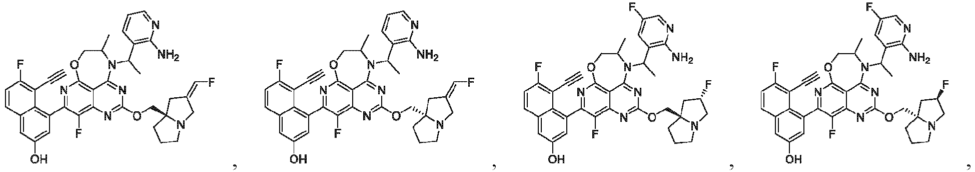

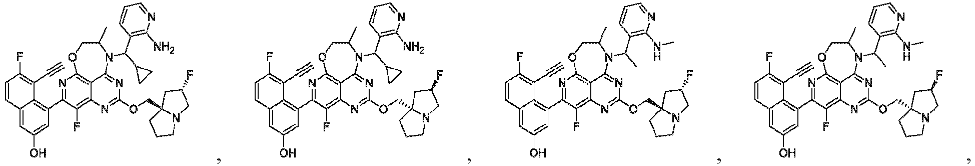

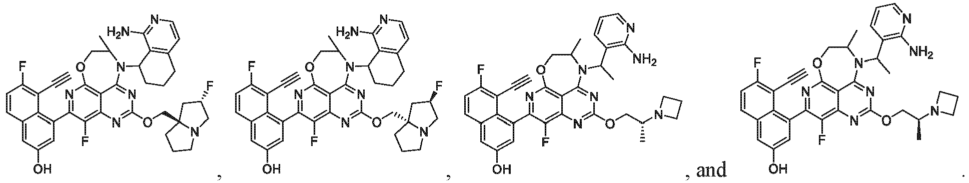

The present disclosure further relates to the following compounds, stereoisomers thereof, tautomers thereof, deuterated compounds thereof, or pharmaceutically acceptable salts thereof:

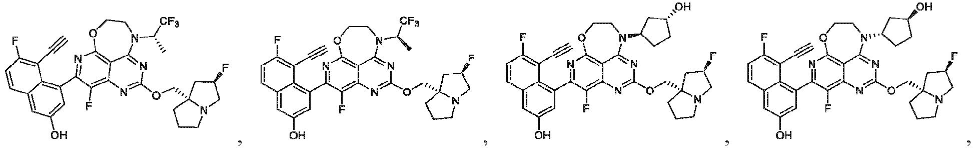

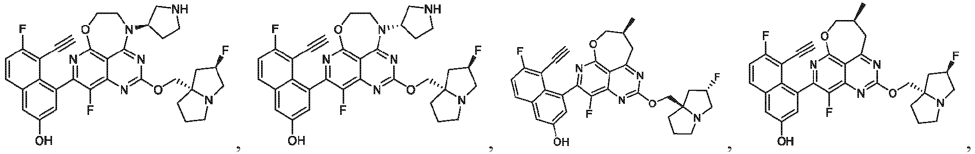

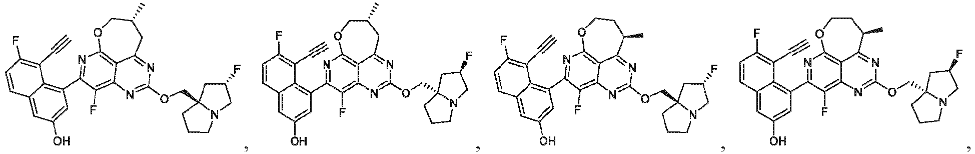

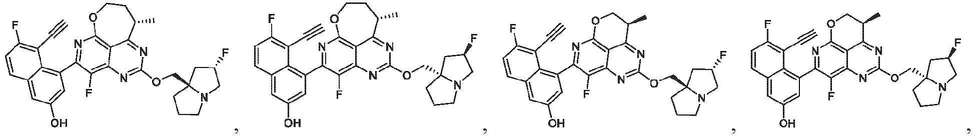

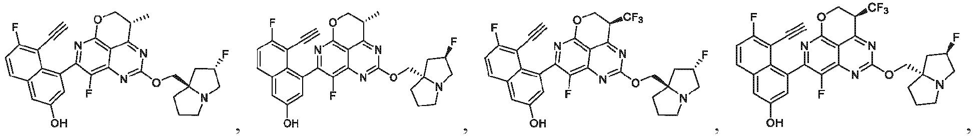

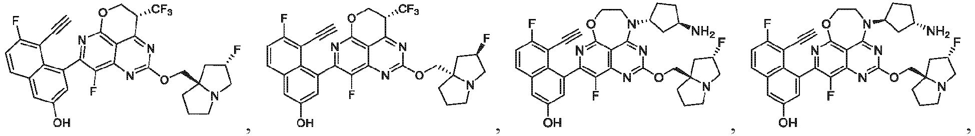

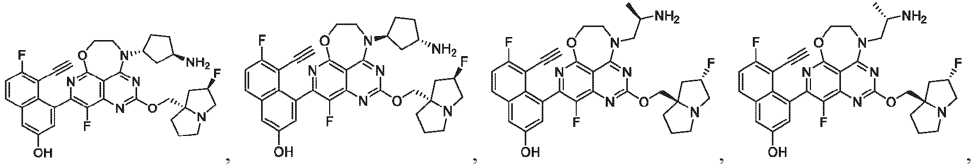

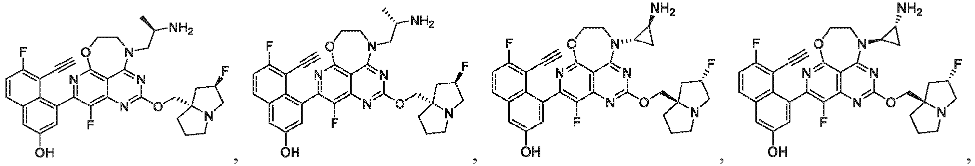

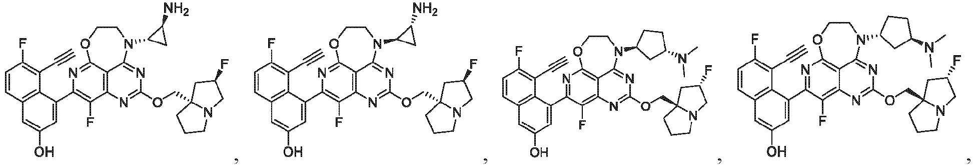

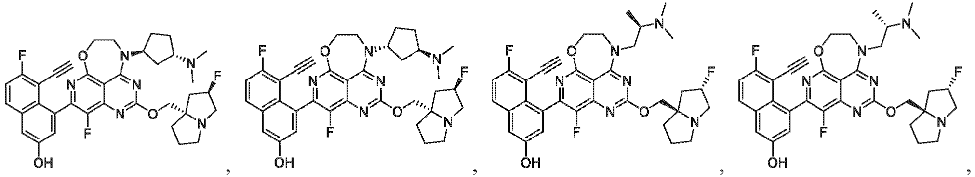

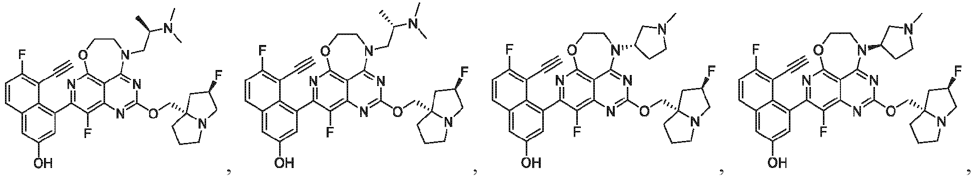

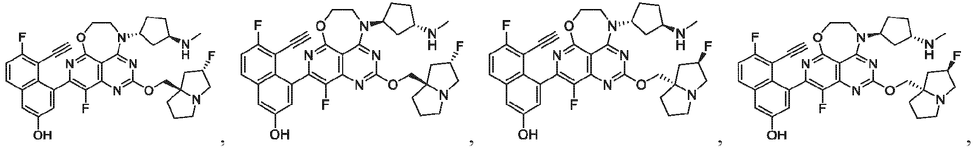

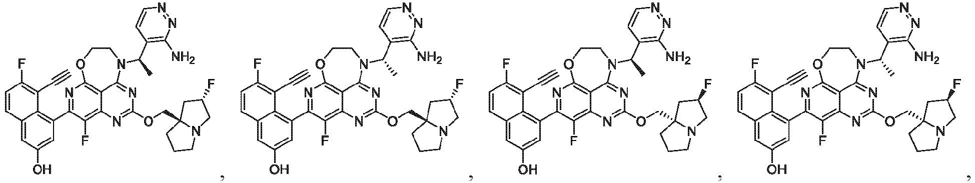

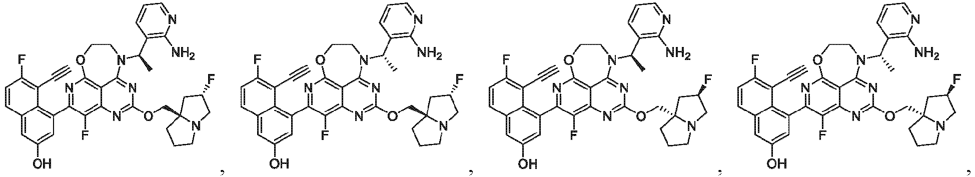

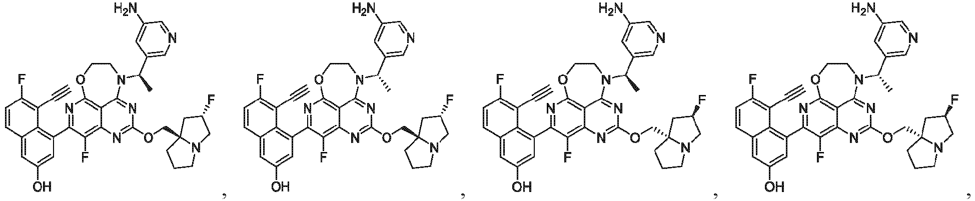

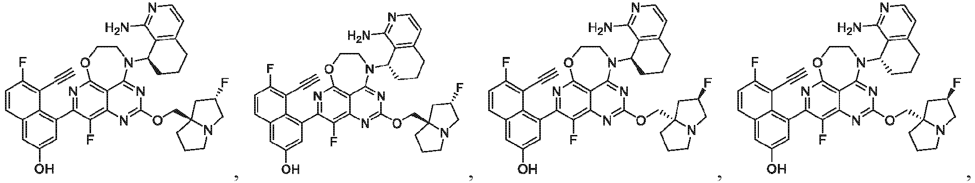

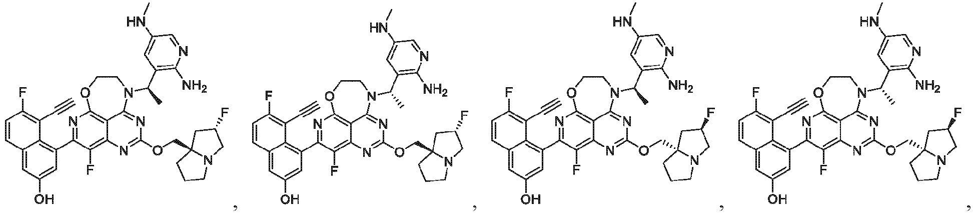

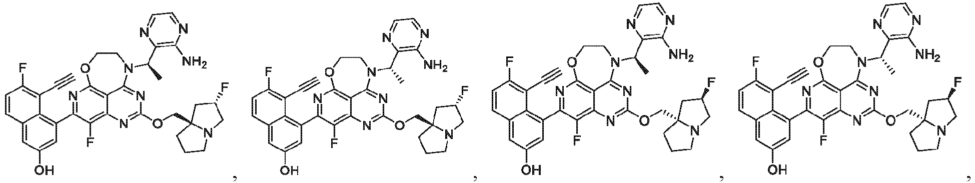

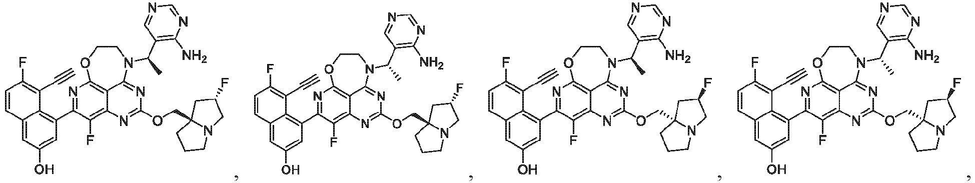

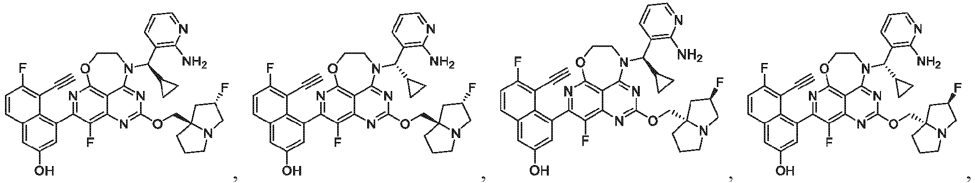

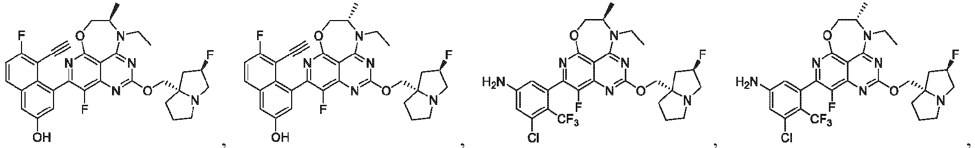

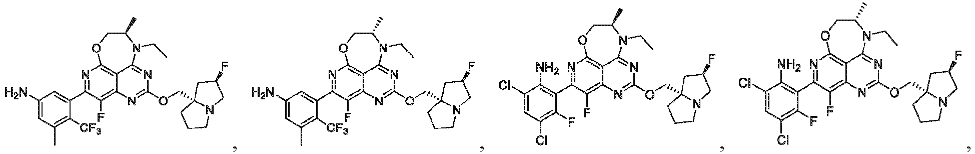

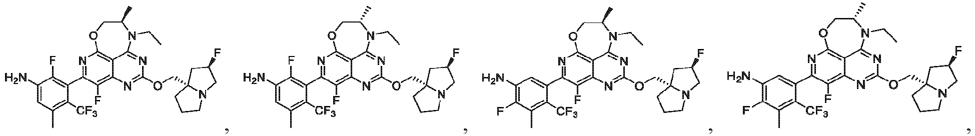

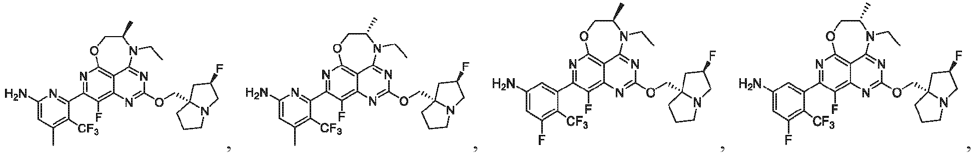

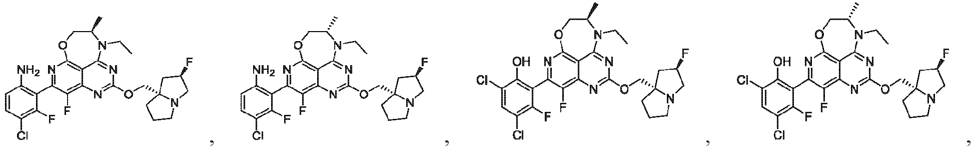

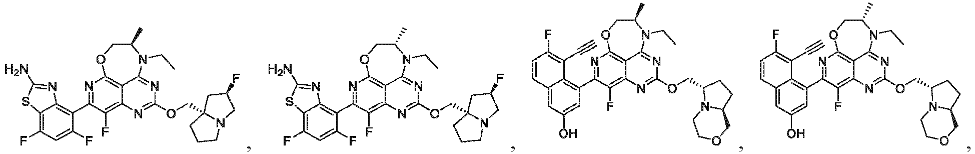

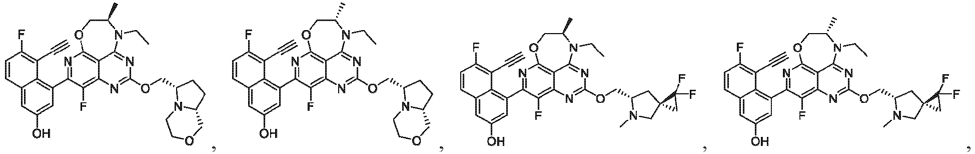

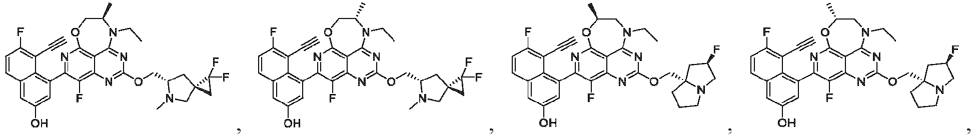

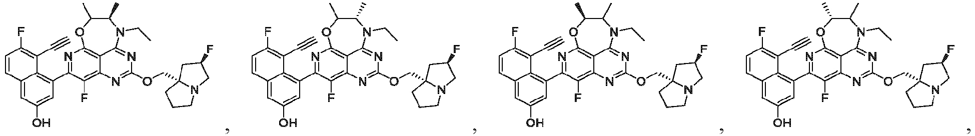

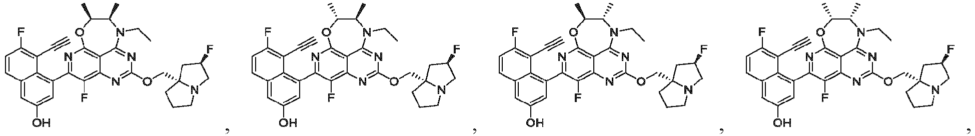

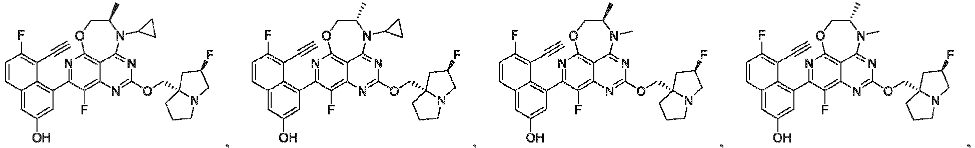

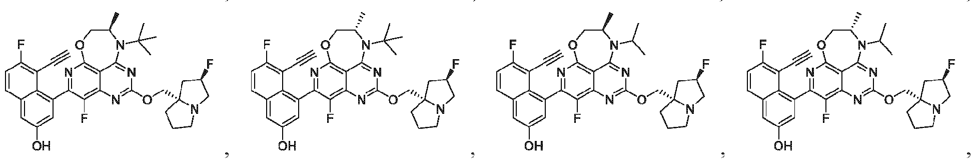

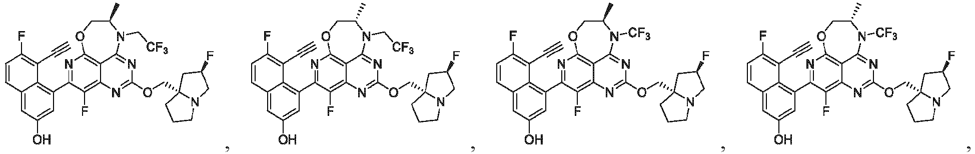

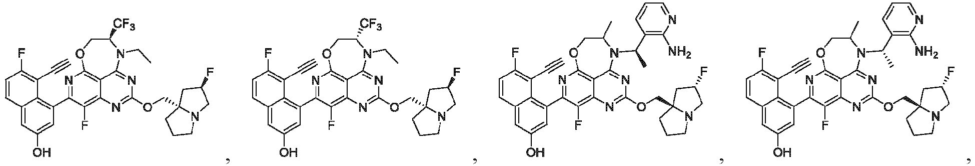

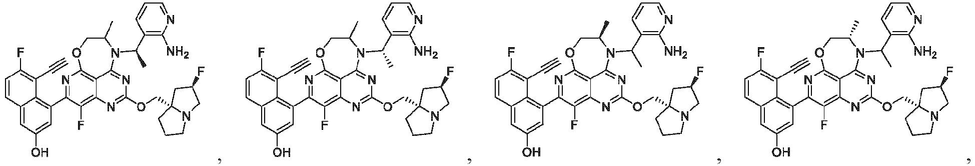

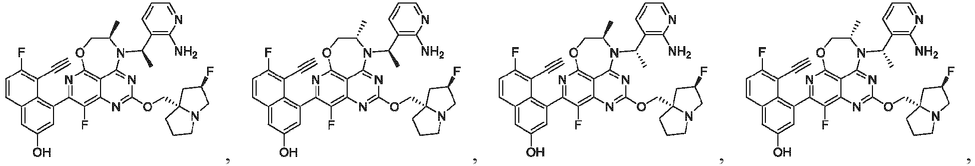

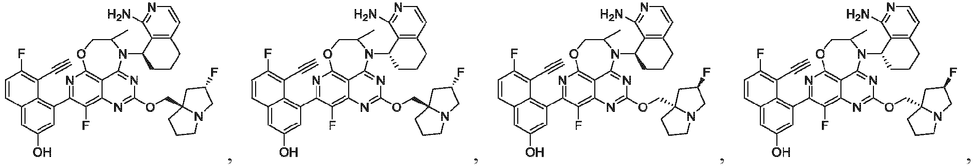

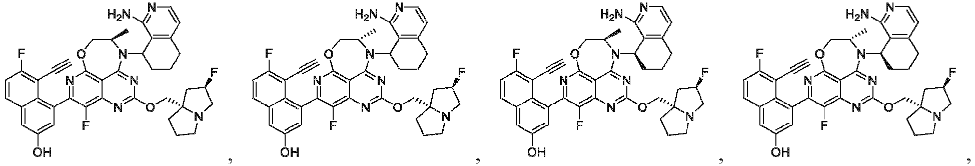

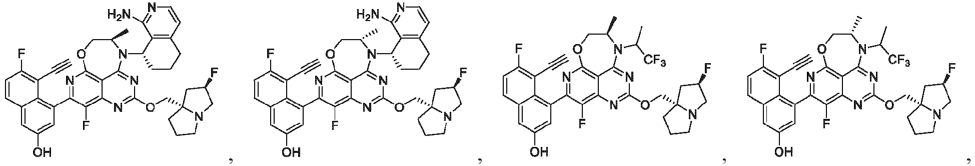

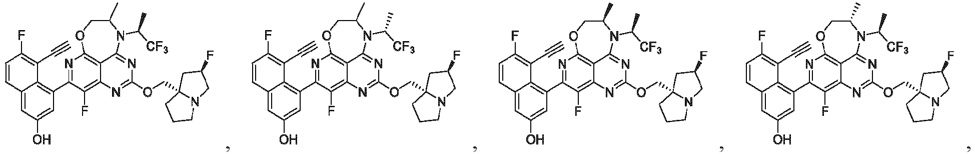

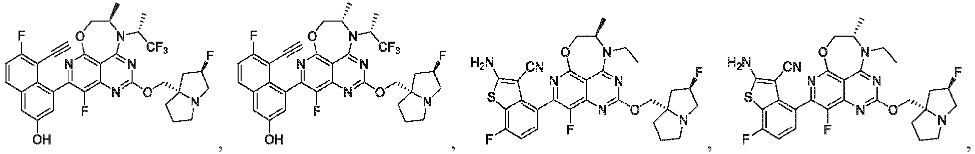

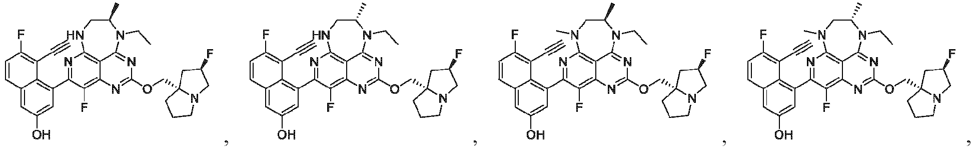

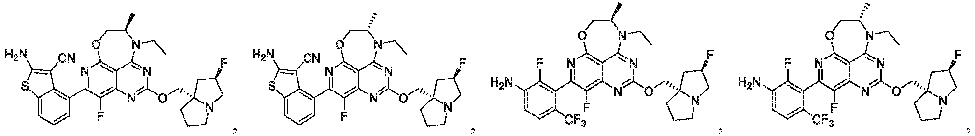

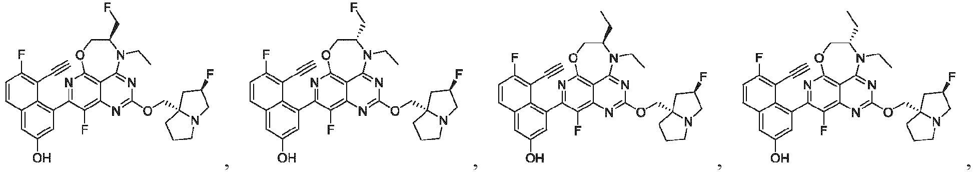

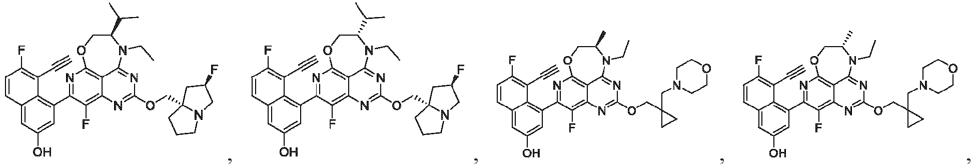

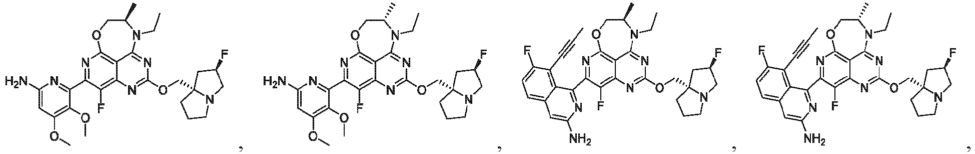

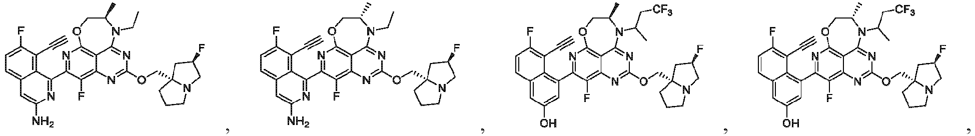

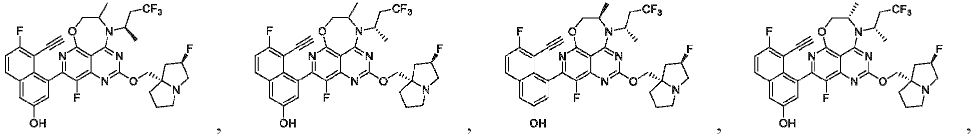

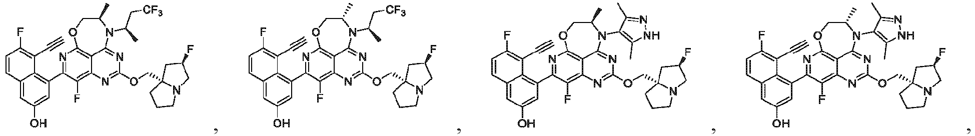

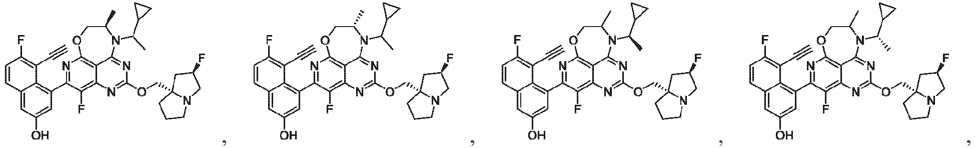

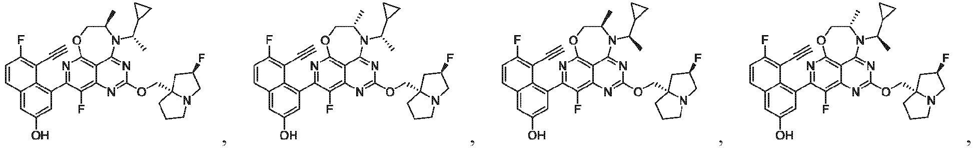

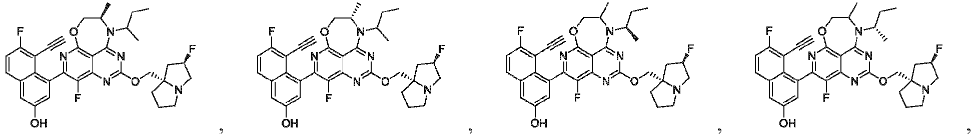

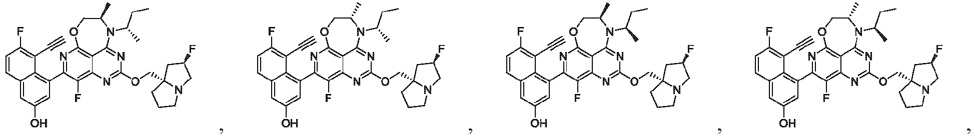

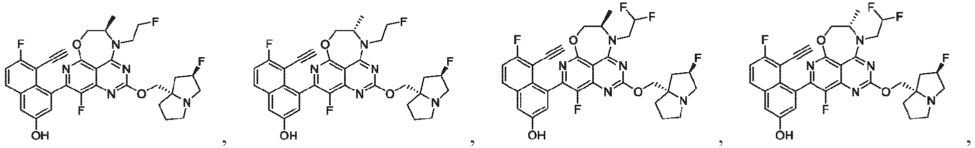

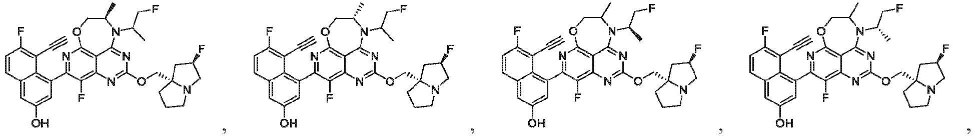

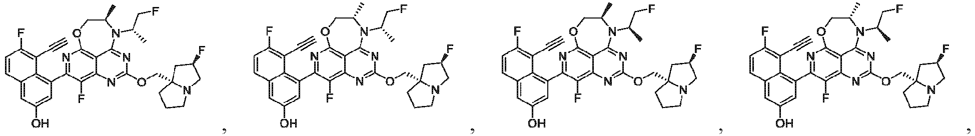

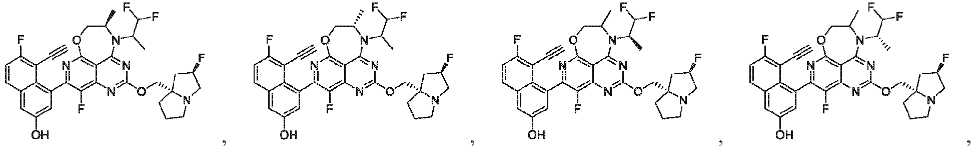

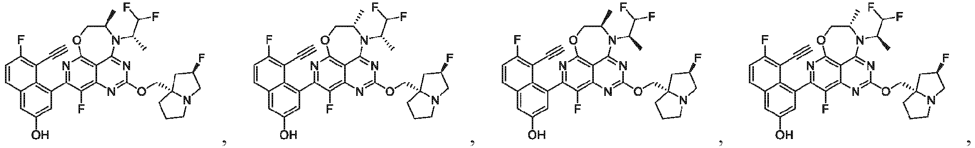

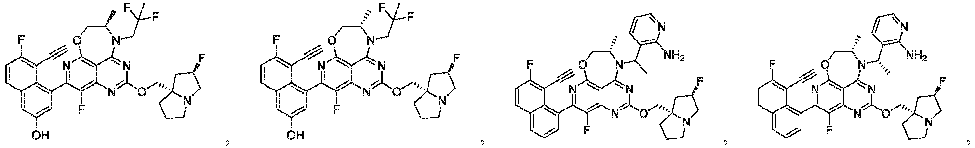

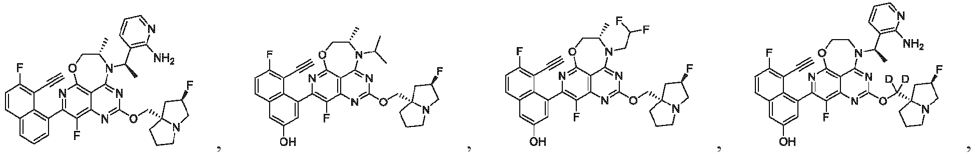

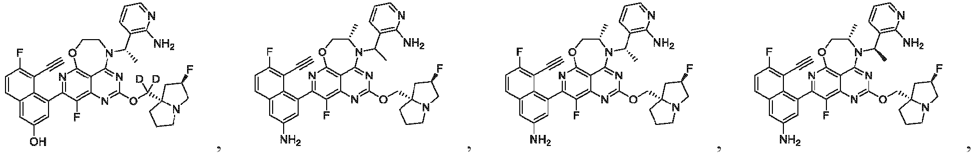

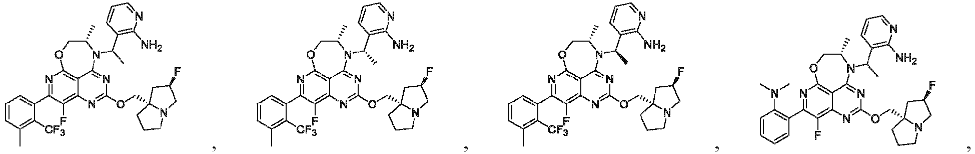

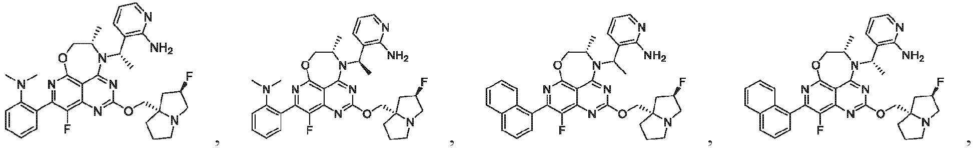

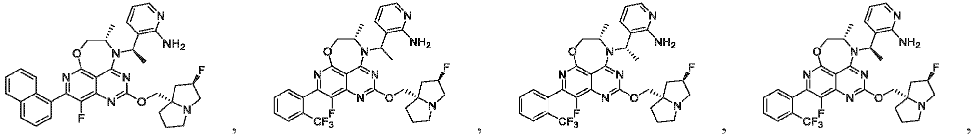

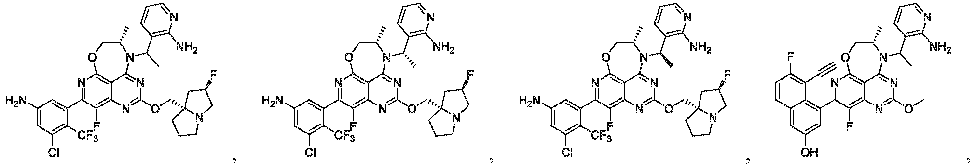

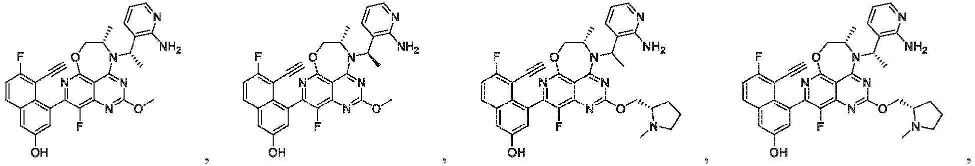

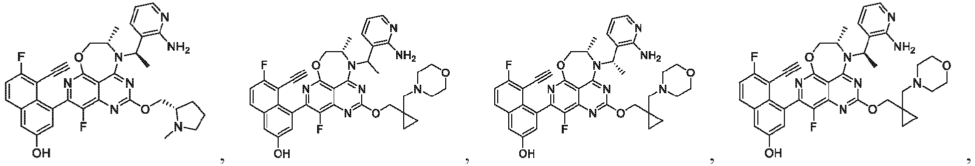

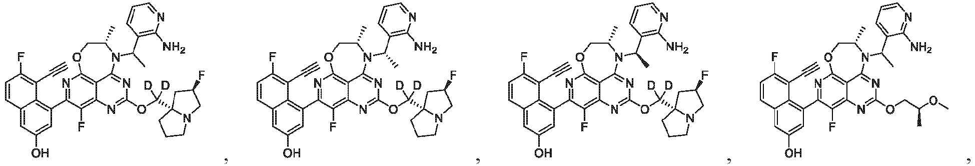

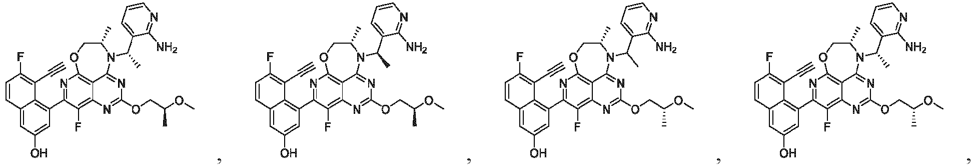

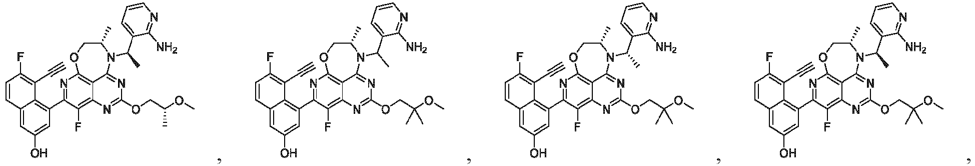

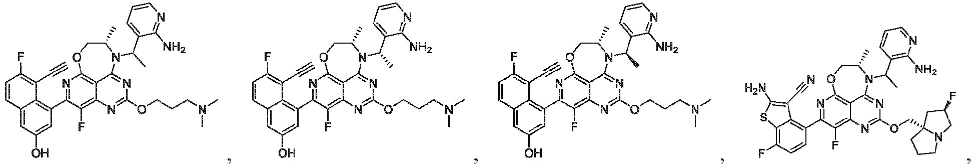

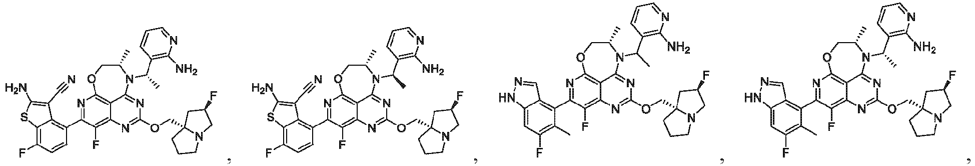

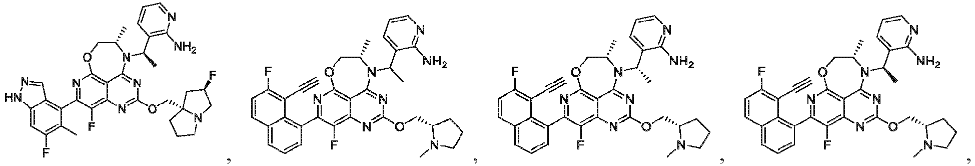

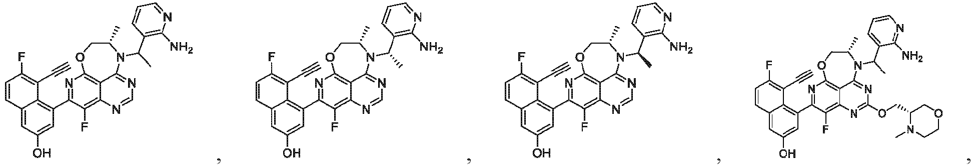

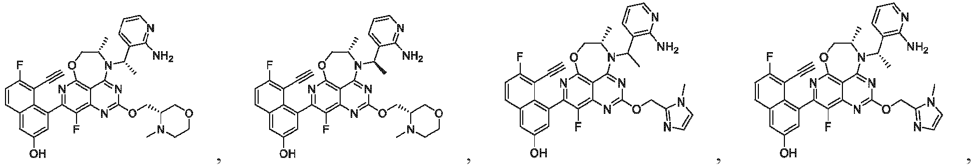

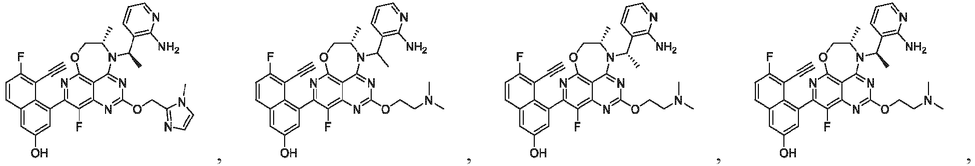

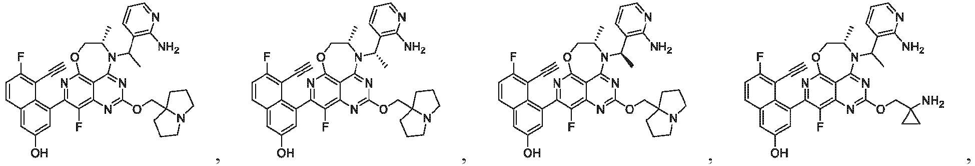

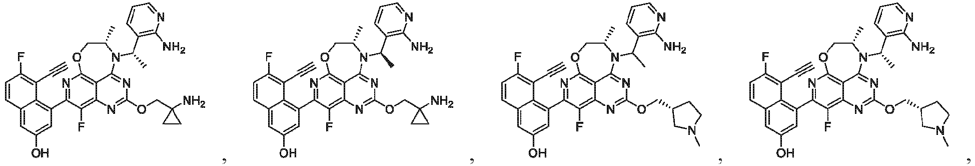

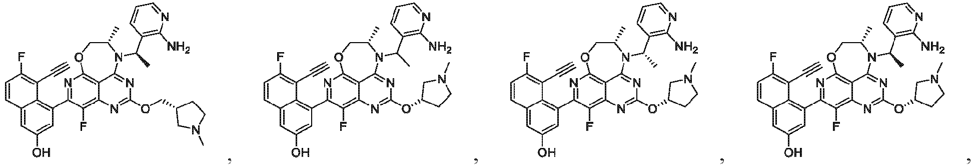

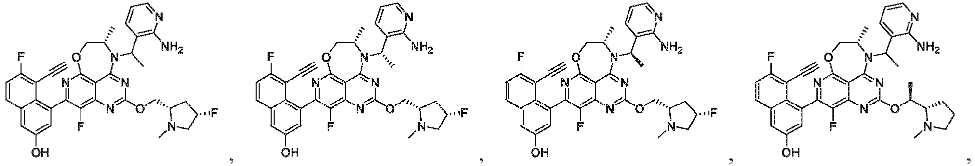

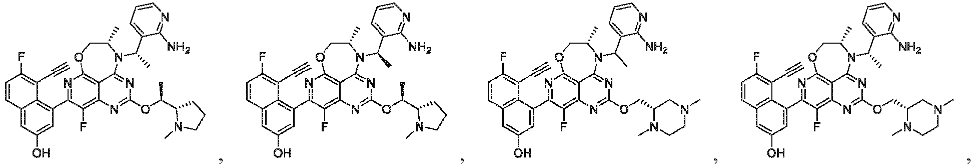

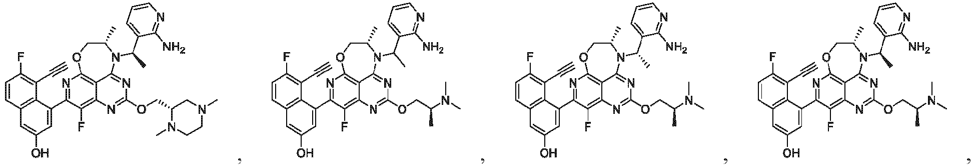

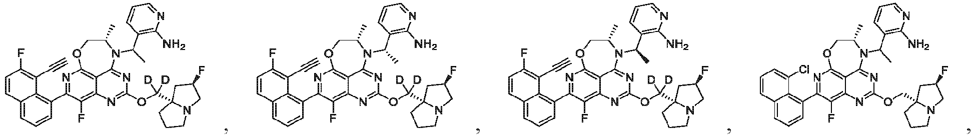

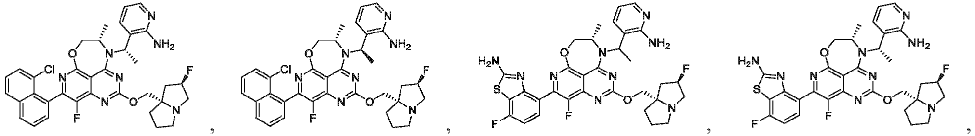

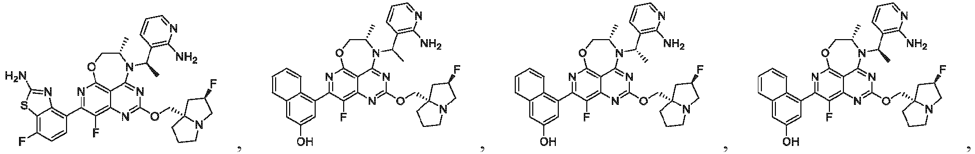

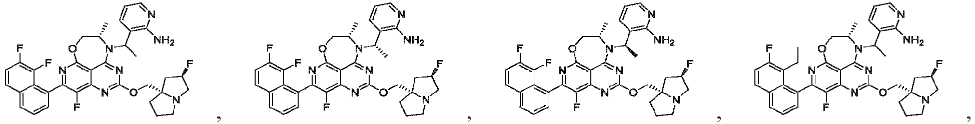

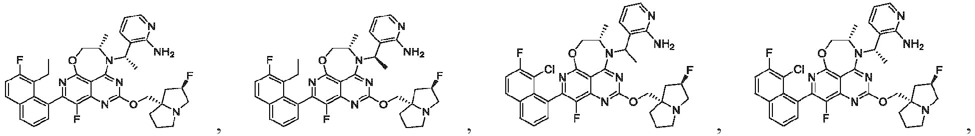

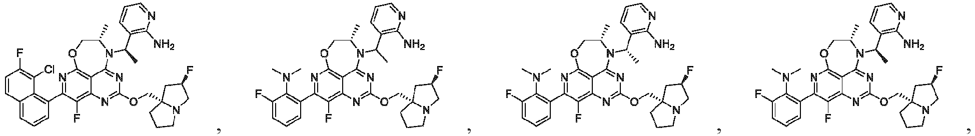

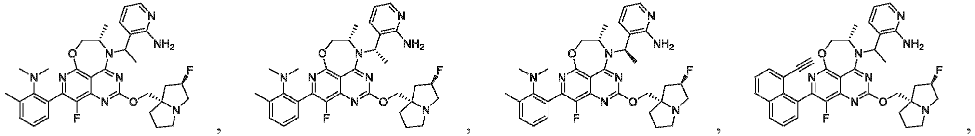

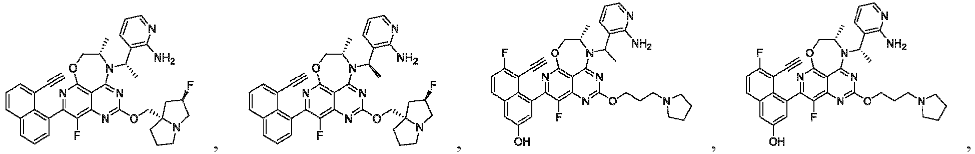

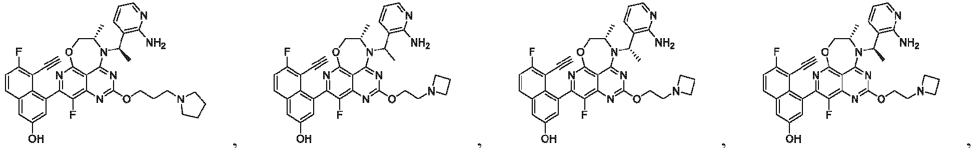

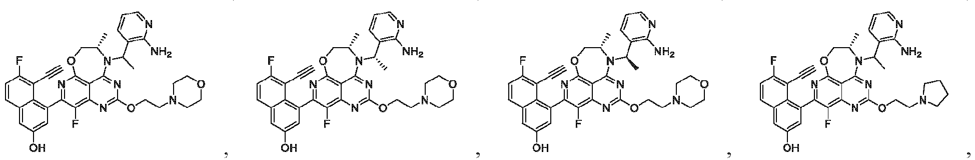

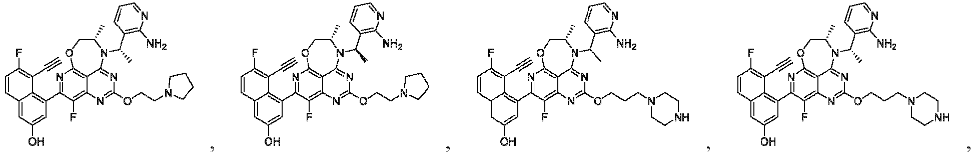

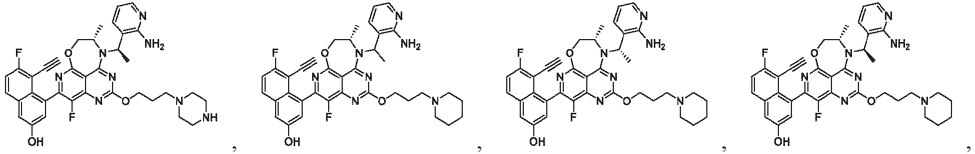

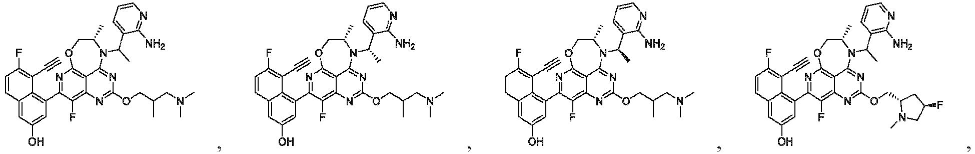

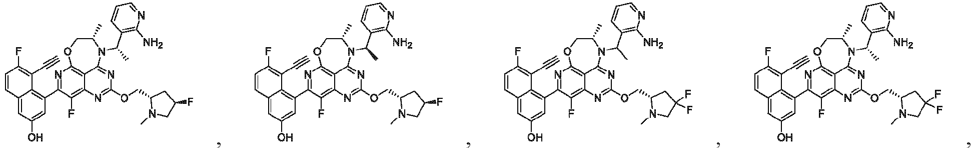

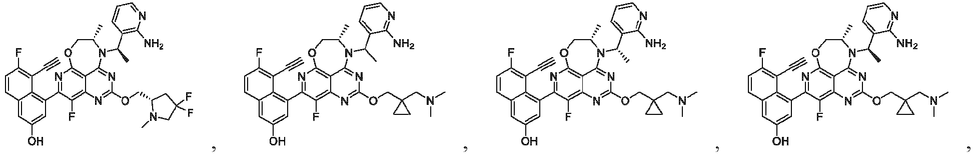

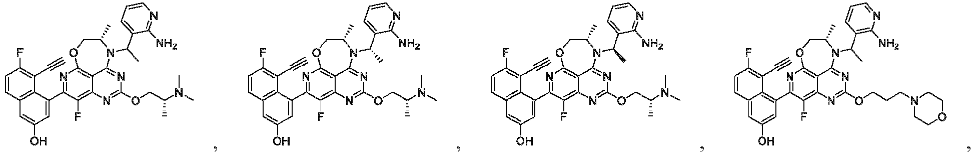

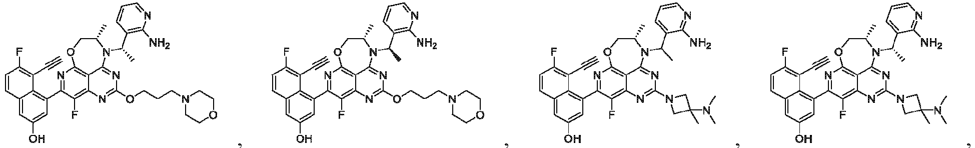

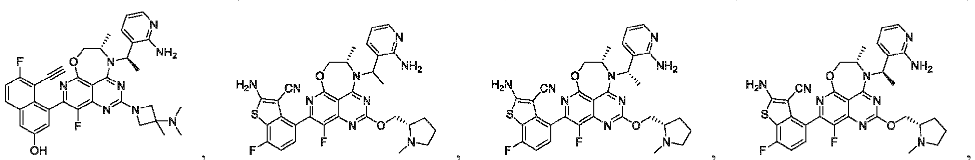

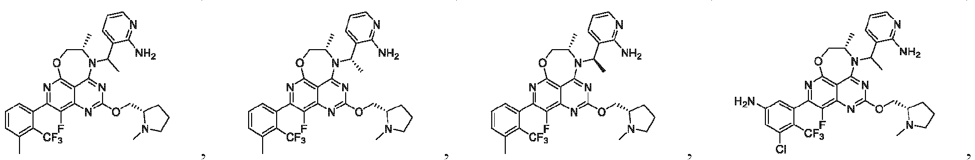

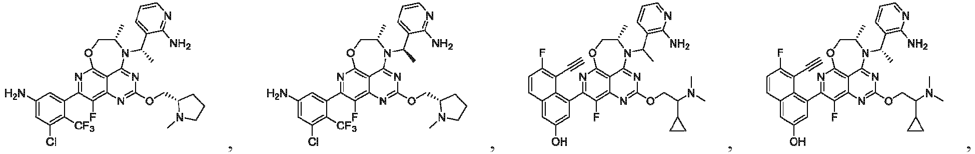

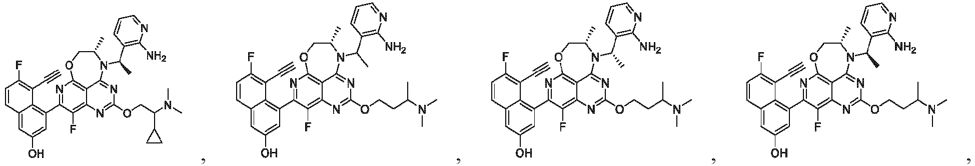

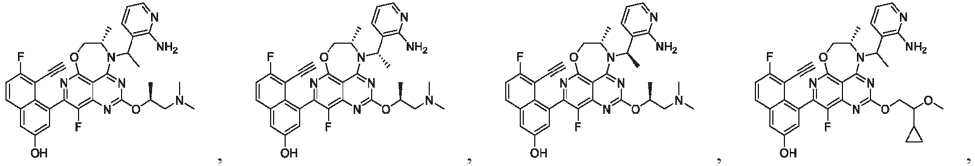

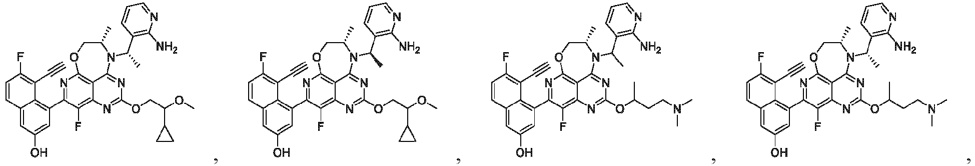

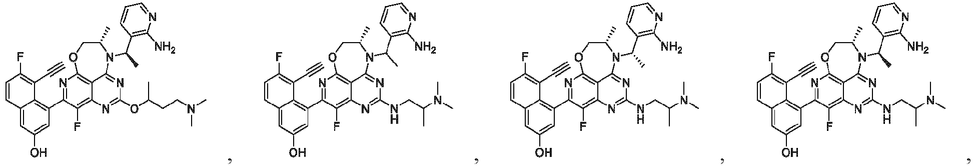

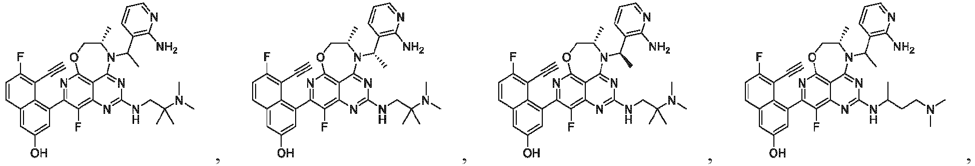

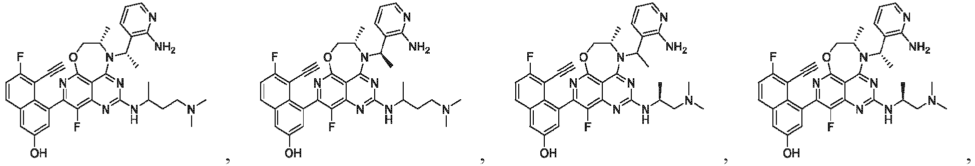

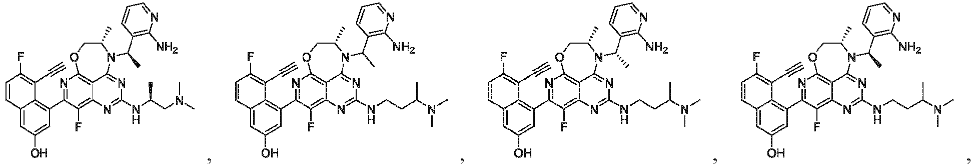

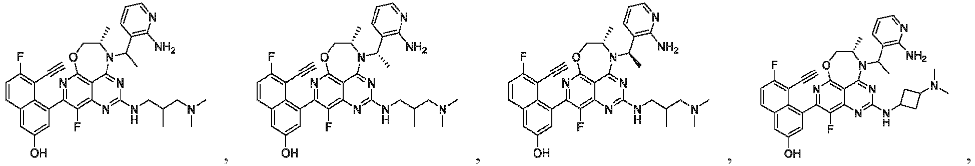

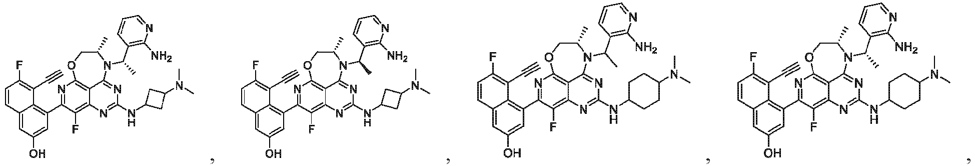

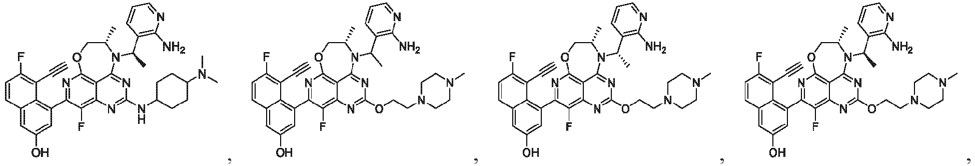

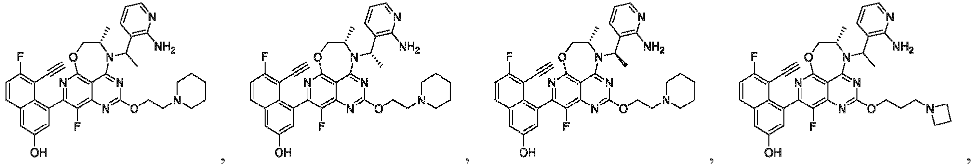

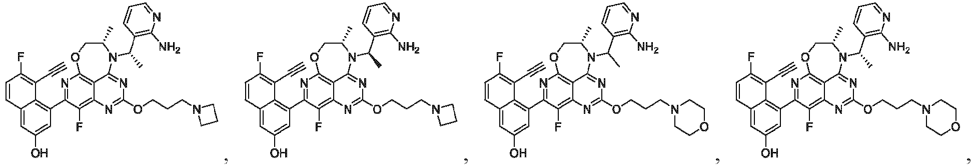

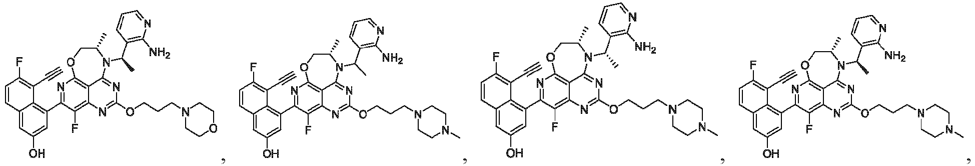

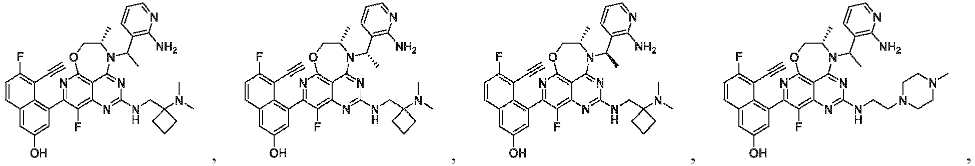

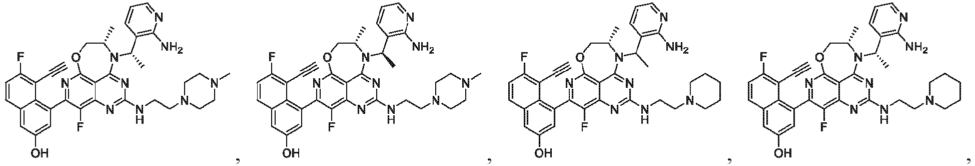

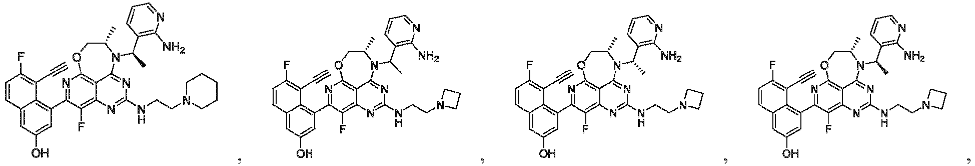

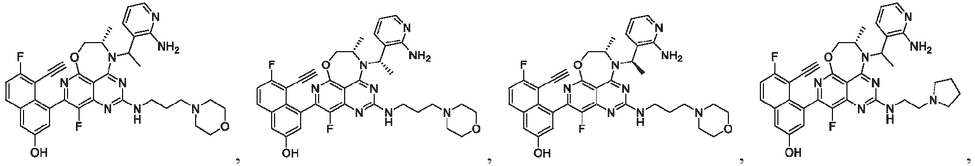

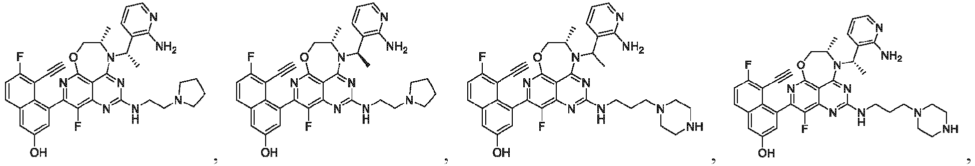

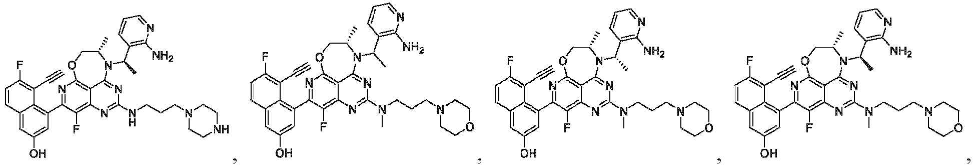

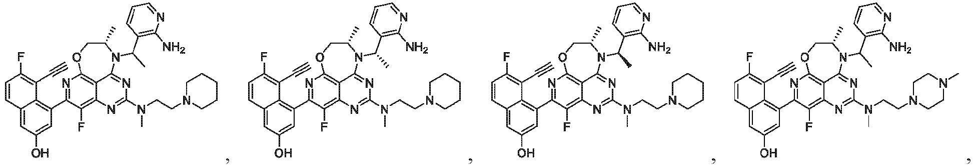

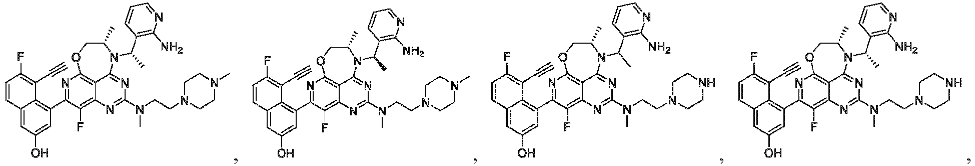

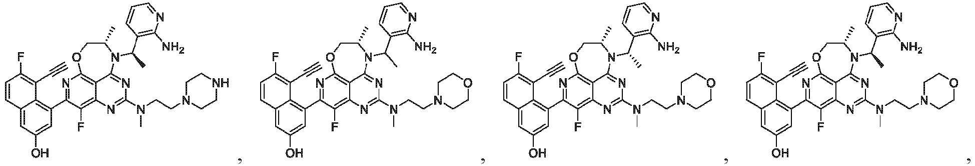

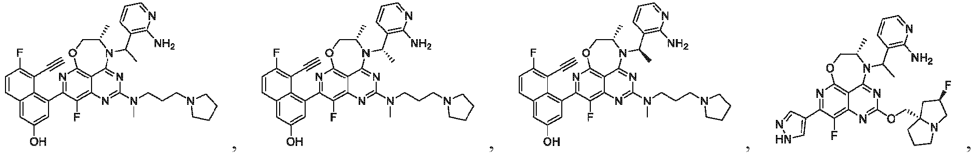

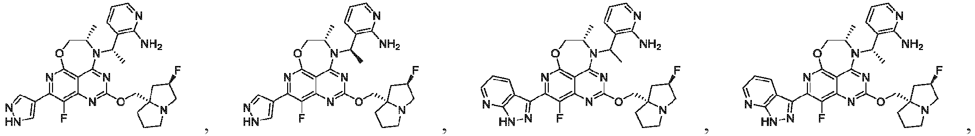

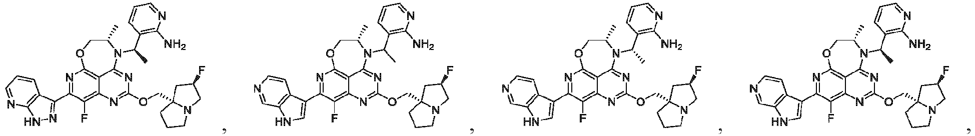

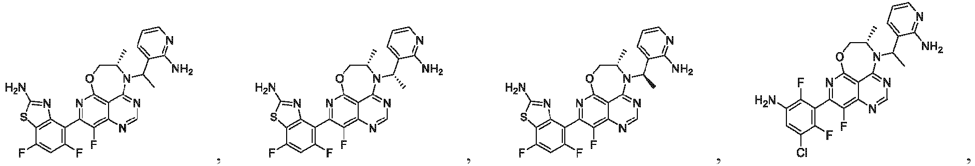

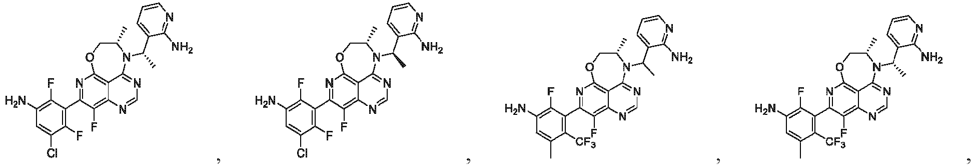

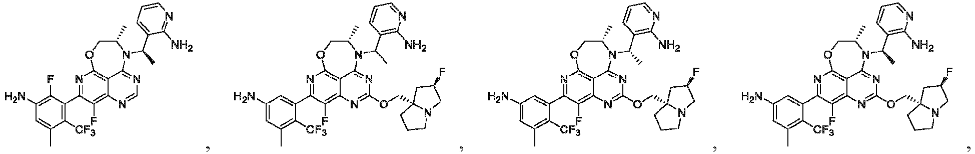

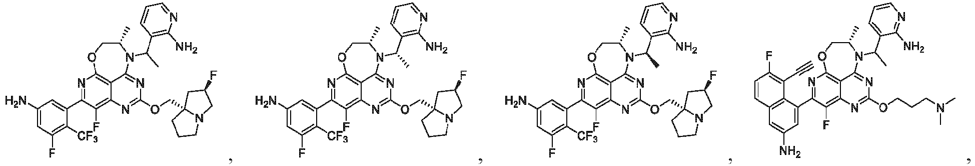

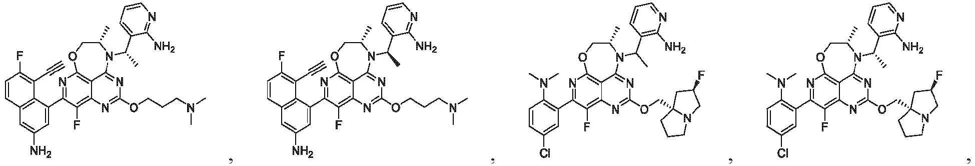

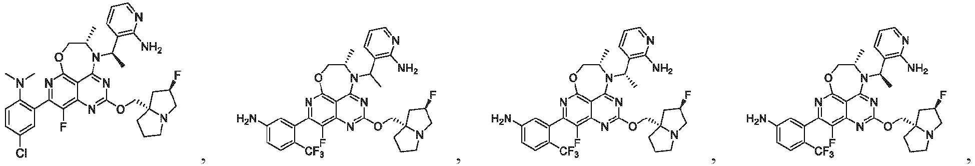

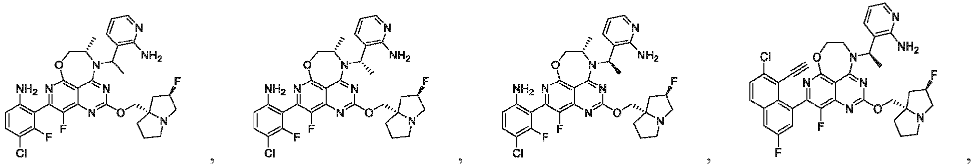

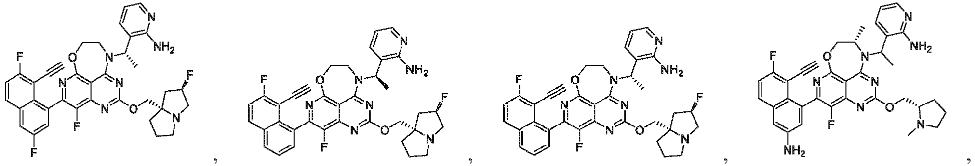

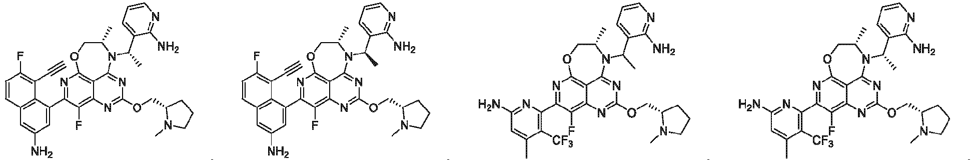

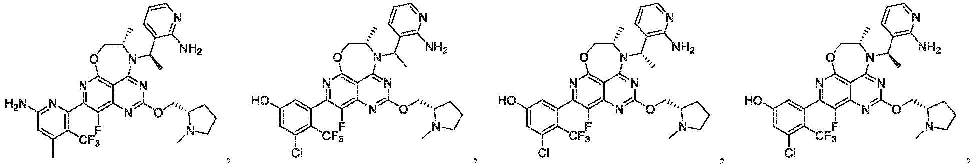

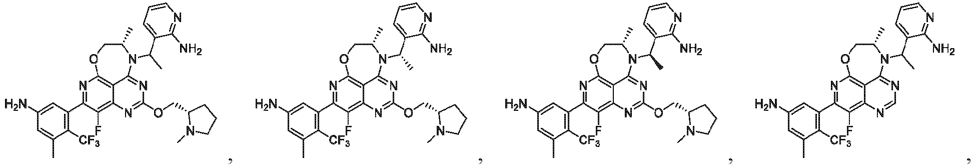

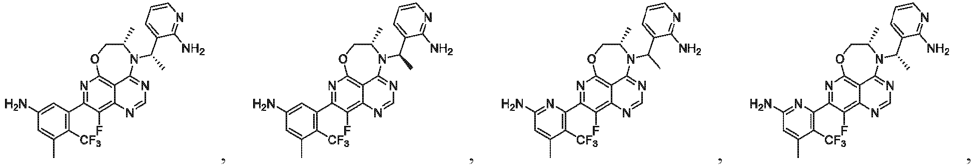

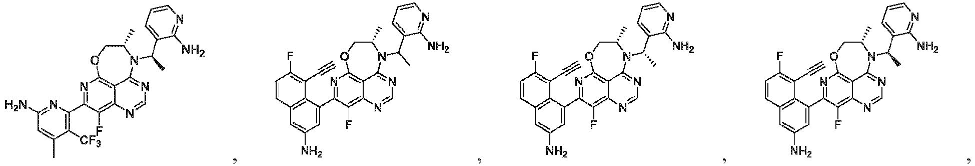

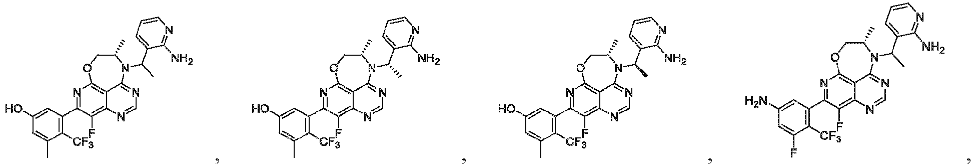

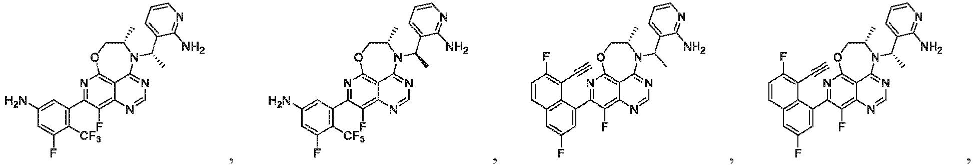

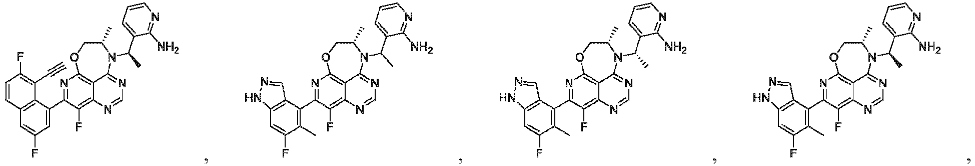

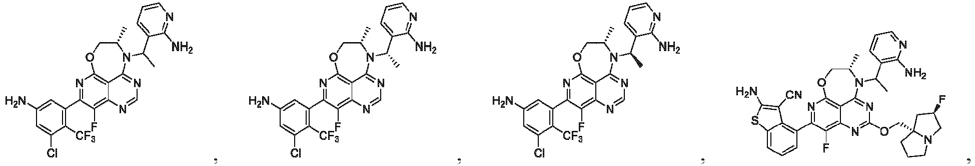

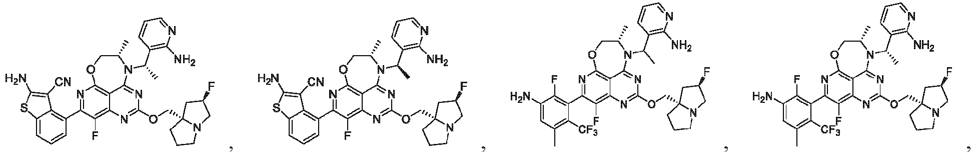

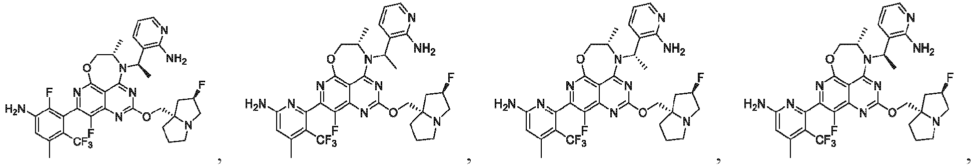

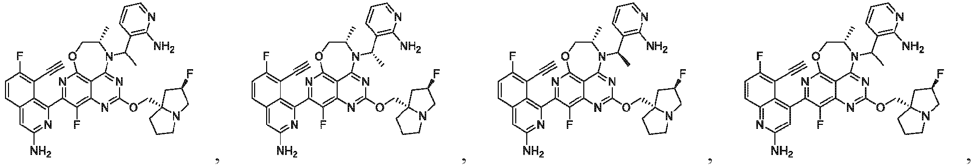

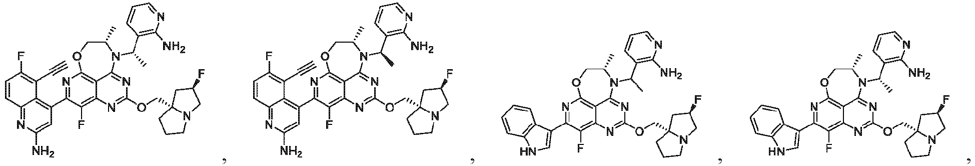

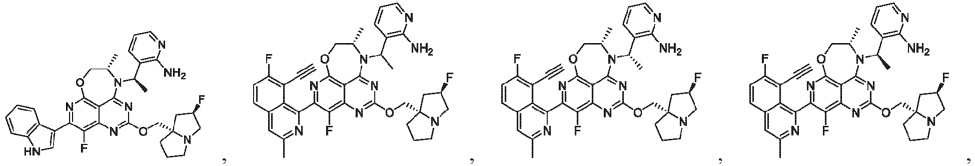

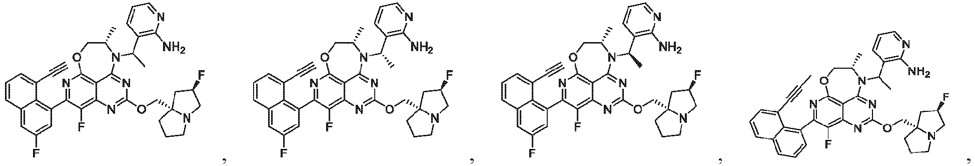

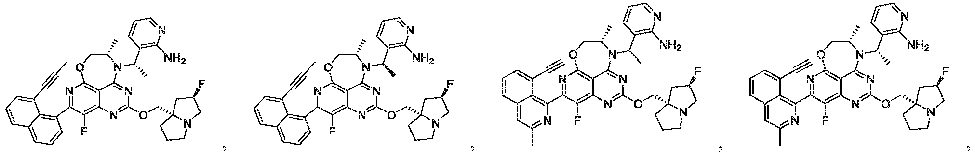

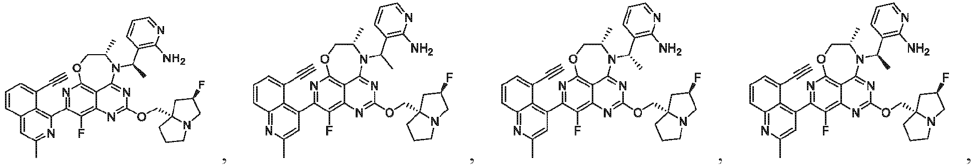

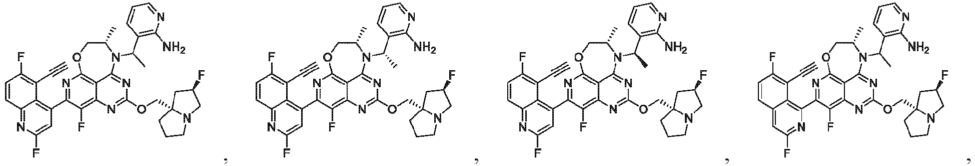

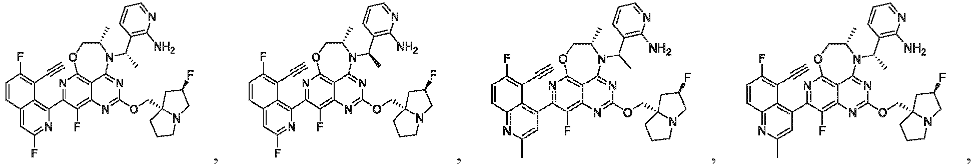

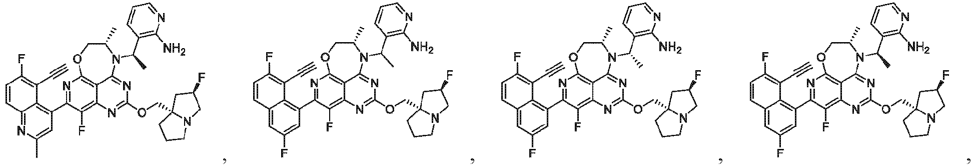

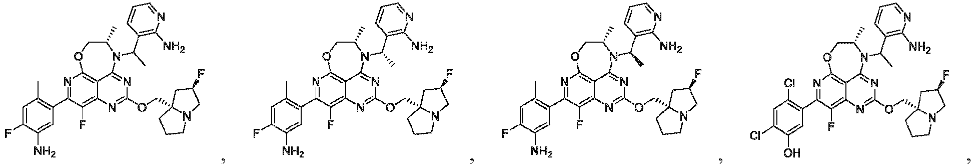

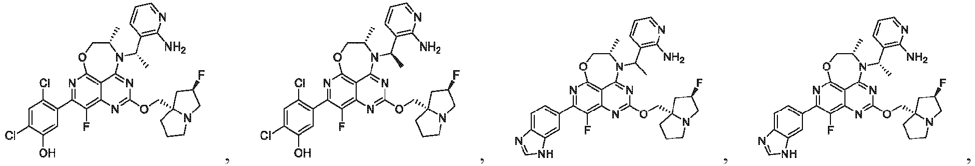

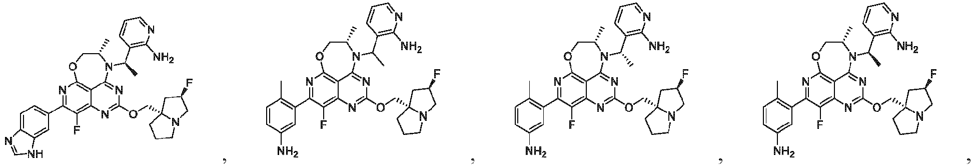

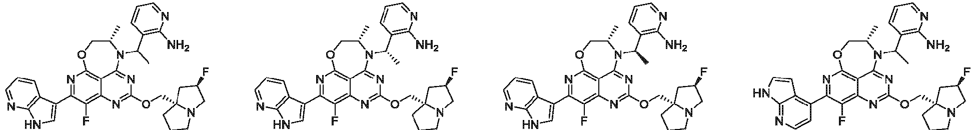

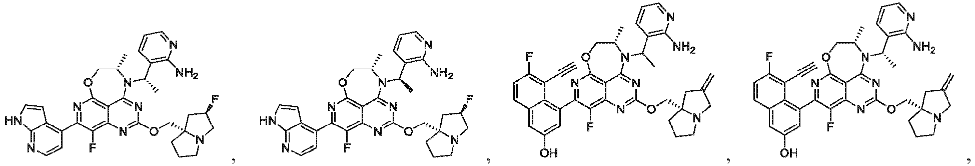

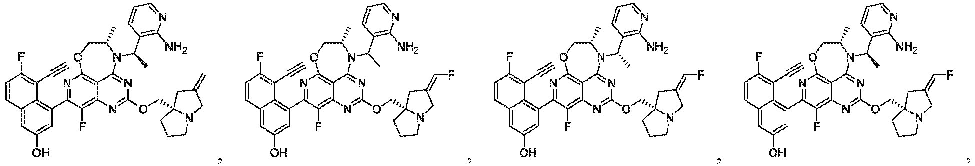

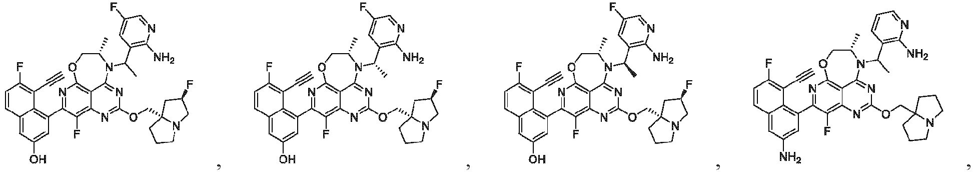

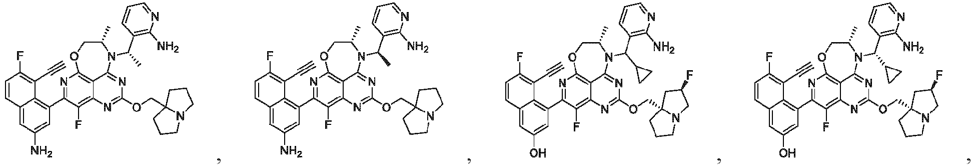

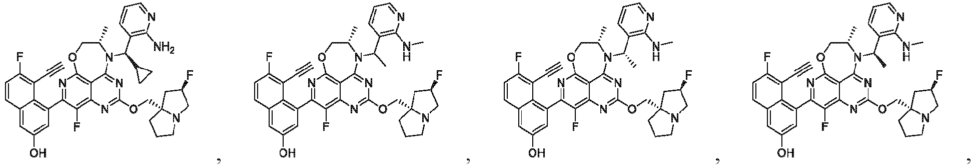

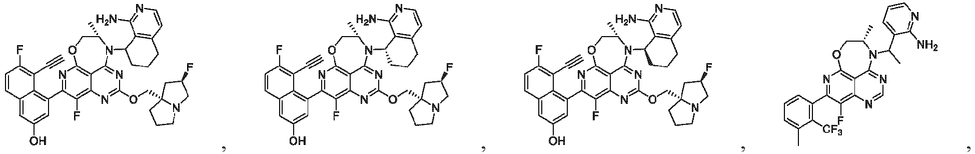

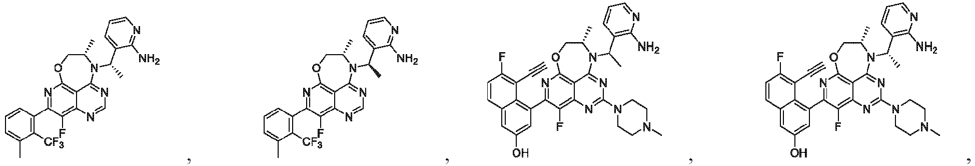

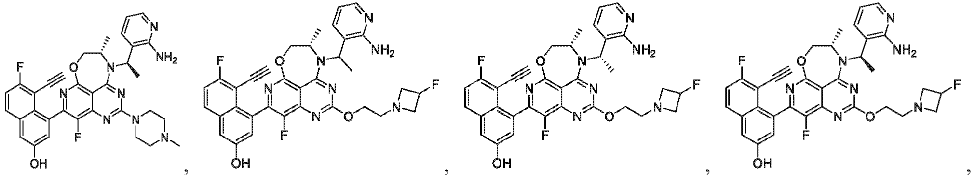

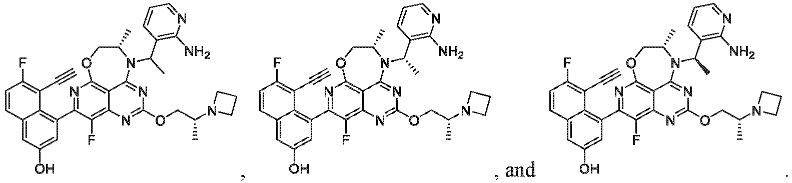

In another aspect, the present disclosure relates to a pharmaceutical composition comprising the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the tautomer thereof, the deuterated compound thereof, or the pharmaceutically acceptable salt thereof of the present disclosure. In some embodiments, the pharmaceutical composition of the present disclosure further comprises a pharmaceutically acceptable excipient.

In another aspect, the present disclosure relates to a method for treating a disease in a mammal, comprising administering to a mammal, preferably a human, in need thereof a therapeutically effective amount of the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the tautomer thereof, the deuterated compound thereof, or the pharmaceutically acceptable salt thereof, or the pharmaceutical composition of the present disclosure.

In another aspect, the present disclosure relates to use of the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the tautomer thereof, the deuterated compound thereof, or the pharmaceutically acceptable salt thereof, or the pharmaceutical composition of the present disclosure in the manufacture of a medicament for treating a disease.

In another aspect, the present disclosure relates to use of the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the tautomer thereof, the deuterated compound thereof, or the pharmaceutically acceptable salt thereof, or the pharmaceutical composition of the present disclosure in treating a disease.

In another aspect, the present disclosure relates to the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the deuterated compound thereof, the tautomer thereof, or the pharmaceutically acceptable salt thereof, or the pharmaceutical composition of the present disclosure for use in treating a disease.

In some embodiments of the present disclosure, the disease is preferably a Kras-associated disease.

In another aspect, the present disclosure relates to a method for treating a Kras-associated disease in a mammal, comprising administering to a mammal, preferably a human, in need thereof a therapeutically effective amount of the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the tautomer thereof, the deuterated compound thereof, or the pharmaceutically acceptable salt thereof, or the pharmaceutical composition of the present disclosure.

In another aspect, the present disclosure relates to use of the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the tautomer thereof, the deuterated compound thereof, or the pharmaceutically acceptable salt thereof, or the pharmaceutical composition of the present disclosure in the manufacture of a medicament for treating a Kras-associated disease.

In another aspect, the present disclosure relates to use of the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the tautomer thereof, the deuterated compound thereof, or the pharmaceutically acceptable salt thereof, or the pharmaceutical composition of the present disclosure in treating a Kras-associated disease.

In another aspect, the present disclosure relates to the compound of formula (I) or formula (II-1) or formula (II-2) or formula (III) or formula (III-a) or formula (III-b) or formula (III-c) or formula (IV) or formula (IV-a) or formula (IV-b) or formula (IV-c), the stereoisomer thereof, the deuterated compound thereof, the tautomer thereof, or the pharmaceutically acceptable salt thereof, or the pharmaceutical composition of the present disclosure for use in treating a Kras-associated disease.

In some embodiments of the present disclosure, the Kras-associated disease is preferably a Kras-mutated cancer. In some embodiments of the present disclosure, the Kras-associated disease is selected from a cancer (e.g., pancreatic cancer).

In some embodiments of the present disclosure, the Kras-associated disease is preferably a Kras ^{G12D}-associated disease.

In some embodiments of the present disclosure, the Kras ^{G12D}-associated disease is preferably a Kras ^{G12D}-mutated cancer.

In some embodiments of the present disclosure, the Kras ^{G12D}-associated disease is selected from a cancer (e.g., pancreatic cancer).

In some embodiments of the present disclosure, the Kras-associated disease is preferably a Kras ^{G12V}-associated disease.

In some embodiments of the present disclosure, the Kras ^{G12V}-associated disease is preferably a Kras ^{G12V}-mutated cancer.

In some embodiments of the present disclosure, the Kras ^{G12V}-associated disease is selected from a cancer (e.g., pancreatic cancer).

### Technical effects

The compounds of the present disclosure have high Kras inhibitory activity (e.g., Kras ^{G12D} and/or Kras ^{G12V} nucleotide exchange inhibitory activity), inhibitory activity for cell proliferation (e.g., AsPc-1 cells and/or Capan-1 cells) and inhibitory activity for *in vivo* tumor (e.g., an Aspc-1 human pancreatic cancer cell NOD-SCID mouse subcutaneous xenograft tumor model). Additionally, the compounds demonstrate good druggability in the studies of *in vivo* and *in vitro* pharmacokinetics, bioavailability, and/or pharmacodynamics.

### Definitions

Unless otherwise stated, the following terms used in the present disclosure shall have the following meanings. A certain term, unless otherwise specifically defined, should not be considered uncertain or unclear, but construed according to its common meaning in the art. When referring to a trade name, it is intended to refer to its corresponding commercial product or its active ingredient.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted, and oxo is not possible in an aromatic group.

Non-limiting examples of the "substituent" described herein include hydroxy, sulfydryl, halogen, amino, nitro, nitroso, cyano, an azide group, a sulfoxide group, a sulfone group, a sulfonamide group, carboxy, a carboxaldehyde group, an imine group, alkyl, halo-alkyl, cycloalkyl, halo-cycloalkyl, alkenyl, halo-alkenyl, cycloalkenyl, halo-cycloalkenyl, alkynyl, halo-alkynyl, cycloalkynyl, halo-cycloalkynyl, heteroalkyl, halo-heteroalkyl, alkoxy, alkylthio, aryl, aryloxy, arylthio, arylalkyl, arylalkoxy, arylalkylthio, heteroaryl, heteroaryloxy, heteroarylthio, heteroarylalkyl, heteroarylalkoxy, heteroarylalkylthio, heterocyclyl, heterocyclyloxy, heterocyclylthio, heterocyclylalkylene, heterocyclylalkoxy, heterocyclylalkylthio, acyl, acyloxy, a carbamate group, an amido group, ureido, an epoxy group, an ester group, and the like, wherein the groups are optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "replaced" means that a particular atom or group can be replaced with another atom or group as specified. For example, 1 or 2 or 3 of the -CH₂- in -CH₂CH₂CH₂- may be replaced with O, S, or NH to give -O-CH₂-CH₂-, -O-CH₂-, -CH₂-O-CH₂-, -CH₂-O-, -CH₂-CH₂-O-, -O-, or the like.

The term "optional" or "optionally" means that the subsequently described event or circumstance may or may not occur. The description includes instances where the event or circumstance occurs and instances where it does not. For example, ethyl "optionally" substituted with halogen means that the ethyl may be unsubstituted (CH₂CH₃), monosubstituted (e.g., CH₂CH₂F), polysubstituted (e.g., CHFCH₂F and CH₂CHF₂), or fully substituted (CF₂CF₃). It can be understood by those skilled in the art that for any groups comprising one or more substituents, no substitutions or substitution modes that are impossible to spatially exist and/or synthesize will be introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms.

When any variable (e.g., R) occurs once or more times in the constitution or structure of a compound, the definition of the variable in each case is independent. Therefore, for example, if a group is substituted with 2 R, the definition of each R is independent.

When the number of connecting groups is 0, for example, -(CH₂)₀-, it means that the connecting group is a covalent bond.

When a variable is selected from a covalent bond, it means that the two groups which it connects are directly connected. For example, in A-L-Z, when L represents a covalent bond, it means that the structure is actually A-Z. When the direction for connection of the listed connecting group is not specified, the direction for connection is arbitrary. For example, in A-L-Z, when the connecting group L is -M-W-, it means that the structure may be A-M-W-Z or A-W-M-Z.

When a bond of a substituent is cross-connected to two atoms on a ring, the substituent can be bonded to any atom on the ring. For example, a structural unit 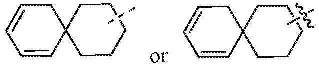 represents that substitution may occur in any one position on cyclohexyl or cyclohexadienyl.

The term "halo" or "halogen" refers to fluorine, chlorine, bromine, and iodine.

The term "hydroxy" refers to an -OH group.

The term "cyano" refers to a -CN group.

The term "sulfydryl" refers to an -SH group.

The term "amino" refers to an -NH₂ group.

The term "nitro" refers to an -NO₂ group.

The term "alkylene" refers to saturated linear or branched divalent hydrocarbyl with a general formula of CₙH₂ₙ and usually has 1 to 12, 1 to 8, 1 to 6, 1 to 4, 1 to 3, or 1 to 2 carbon atoms. For example, the term "C₁₋₆ alkylene" refers to alkylene comprising 1 to 6 carbon atoms. Non-limiting examples of alkylene include, but are not limited to, methylene (-CH₂-), ethylene (-CH₂CH₂-), propylene (-CH₂CH₂CH₂- or -CH₂CH(CH₃)-), butylene (-CH₂CH₂CH₂CH₂-, -CH₂CH(CH₃)CH₂-, or -CH₂CH₂CH(CH₃)-), and the like. The alkylene is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "heteroalkylene" refers to alkylene in which one or more carbon atoms (and the hydrogen atoms to which they are connected) are each independently replaced with the same or different heteroatom groups. Unless otherwise indicated, the heteroalkylene comprises 1, 2, or 3 heteroatom groups, non-limiting examples of which include O, S, N, and NH, and usually has 1 to 12, 1 to 8, 1 to 6, 1 to 4, 1 to 3, or 1 to 2 carbon atoms. For example, the term "C₁₋₆ heteroalkylene" refers to heteroalkylene comprising 1 to 6 carbon atoms and 1-3 heteroatom groups. The heteroatom group can be placed in any position (e.g., internal or terminal position) on the heteroalkylene, including a position where the alkylene is connected to the rest of the molecule. Usually, where more than one heteroatom group is present, the heteroatoms are not adjacent to each other. Non-limiting examples of heteroalkylene include, but are not limited to, -OCH₂-, -OCH₂CH₂-, -OCH₂CH₂CH₂-, -CH₂OCH₂-, -OCH₂O-, -OCH₂CH₂O-, -OCH₂OCH₂CH₂-, -SCH₂-, -SCH₂CH₂-, -SCH₂CH₂CH₂-, -CH₂SCH₂-, -SCH₂S-, -SCH₂CH₂S-, -SCH₂SCH₂CH₂-, -NHCH₂-, -NHCH₂CH₂-, -NHCH₂CH₂CH₂-, -CH₂NHCH₂-, -N(CH₃)CH₂-, -CH₂N(CH₃)-, -OCH₂NH-, -OCH₂CH₂NH-, -OCH₂NHCH₂CH₂-, -OCH₂N(CH₃)CH₂-, and the like. The heteroalkylene is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkyl" refers to saturated hydrocarbyl with a general formula of CₙH₂ₙ₊₁ and usually has 1 to 12, 1 to 8, 1 to 6, 1 to 4, 1 to 3, or 1 to 2 carbon atoms. The alkyl may be linear or branched and usually has 1 to 12, 1 to 8, 1 to 6, 1 to 4, or 1 to 3 carbon atoms. For example, the term "C₁₋₆ alkyl" refers to alkyl comprising 1 to 6 carbon atoms (e.g., methyl, ethyl, *n*-propyl, isopropyl, *n*-butyl, isobutyl, *sec*-butyl, *tert*-butyl*, n*-pentyl, 1-methylbutyl, 2-methylbutyl, 3-methylbutyl, neopentyl, hexyl, and 2-methylpentyl). The alkyl is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy. Similarly, the alkyl moiety (i.e., alkyl) of alkoxy, alkylamino, dialkylamino, alkylsulfonyl, and alkylthio has the same definition as those described above. The term "heteroalkyl" refers to alkyl in which one or more carbon atoms (and the hydrogen atoms to which they are connected) are each independently replaced with the same or different heteroatom groups. Unless otherwise indicated, the heteroalkyl comprises 1, 2, or 3 heteroatom groups, non-limiting examples of which include O, S, N, and NH, and usually has 1 to 12, 1 to 8, 1 to 6, 1 to 4, 1 to 3, or 1 to 2 carbon atoms. For example, the term "C₁₋₆ heteroalkyl" refers to heteroalkyl comprising 1 to 6 carbon atoms and 1-3 heteroatom groups. The heteroatom group can be placed in any position (e.g., internal or terminal position) on the heteroalkyl, including a position where the heteroalkyl is connected to the rest of the molecule. Usually, where more than one heteroatom group is present, the heteroatom groups are not adjacent to each other. Exemplary heteroalkyl includes, but is not limited to, alkoxy, alkoxyalkylene, alkylamino, alkylaminoalkylene, dialkylamino, dialkylaminoalkylene, and the like. The heteroalkyl is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkoxy" refers to -O-alkyl and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkylamino" refers to -NH-alkyl and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "dialkylamino" refers to -N(alkyl)₂ and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkylsulfonyl" refers to -SO₂-alkyl and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkylthio" refers to -S-alkyl and usually has 1 to 12, 1 to 8, 1 to 6, 1 to 4, 1 to 3, or 1 to 2 carbon atoms. The alkyl moiety is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkenyl" refers to linear or branched unsaturated aliphatic hydrocarbyl consisting of carbon atoms and hydrogen atoms and having at least one double bond, and usually has 2 to 12, 2 to 8, 2 to 6, 2 to 4, or 2 to 3 carbon atoms. Non-limiting examples of the alkenyl include, but are not limited to, ethenyl, 1-propenyl, 2-propenyl, 1-butenyl, isobutenyl, 1,3-butadienyl, and the like. The alkenyl is optionally substituted with one or more substituents selected from the group consisting of the following substituents: oxo, hydroxy, amino, nitro, halogen, cyano, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkenylene" refers to a divalent form of alkenyl. The alkenylene usually has 2 to 12, 2 to 8, 2 to 6, 2 to 4, or 2 to 3 carbon atoms. Non-limiting examples of the alkenylene include, but are not limited to, ethenylene, 1-propenylidene, 2-propenylidene, 1-butenylidene, isobutenylidene, 1,3-butadienylene, and the like. The alkenylene is optionally substituted with one or more substituents selected from the group consisting of the following substituents: oxo, hydroxy, amino, nitro, halogen, cyano, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "heteroalkenyl" refers to alkenyl in which one or more carbon atoms (and the hydrogen atoms to which they are connected) are each independently replaced with the same or different heteroatom groups. Unless otherwise indicated, the heteroalkenyl comprises 1, 2, or 3 heteroatom groups, non-limiting examples of which include O, S, N, and NH, and usually has 1 to 12, 1 to 8, 1 to 6, 1 to 4, 1 to 3, 2 to 12, 2 to 8, 2 to 6, 2 to 4, or 2 to 3 carbon atoms. For example, the term "C₁₋₄ heteroalkenyl" refers to heteroalkenyl comprising 1 to 4 carbon atoms and 1-3 heteroatom groups. The heteroatom group can be placed in any position (e.g., internal or terminal position) on the heteroalkenyl, including a position where the heteroalkenyl is connected to the rest of the molecule. Usually, where more than one heteroatom group is present, the heteroatom groups are not adjacent to each other. Exemplary heteroalkenyl includes, but is not limited to, CH₂=N-, alkenyl-O-, alkenyl-NH-, alkenyl-S-, alkenyl-O-alkylene-, alkyl-O-alkenylene-, alkenyl-NH-alkylene-, alkyl-NH-alkenylene-, alkenyl-S-alkylene-, alkyl-S-alkenylene-, alkenyl-CH=N-, alkenyl-N=CH-, alkyl-CH=N-alkenylene-, alkenyl-CH=N-alkylene-, and alkyl-NH-alkenylene-O-. In some embodiments, the double bond in the heteroalkenyl is a carbon-carbon double bond. The heteroalkenyl is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "heteroalkenylene" refers to a divalent form of heteroalkenyl. Unless otherwise indicated, the heteroalkenylene comprises 1, 2, or 3 heteroatom groups, non-limiting examples of which include O, S, N, and NH, and usually has 1 to 12, 1 to 8, 1 to 6, 1 to 4, 1 to 3, 2 to 12, 2 to 8, 2 to 6, 2 to 4, or 2 to 3 carbon atoms. For example, the term "C₁₋₄ heteroalkenylene" refers to heteroalkenylene comprising 1 to 4 carbon atoms and 1-3 heteroatom groups. The heteroatom group can be placed in any position (e.g., internal or terminal position) on the heteroalkenylene, including a position where the heteroalkenylene is connected to the rest of the molecule. Usually, where more than one heteroatom group is present, the heteroatom groups are not adjacent to each other. Exemplary heteroalkenylene includes, but is not limited to, -alkenylene-O-, -alkenylene-NH-, -alkenylene-S-, -alkenylene-O-alkylene-, -alkylene-O-alkenylene-, -alkenylene-NH-alkylene-, -alkylene-NH-alkenylene-, -alkenylene-S-alkylene-, -alkylene-S-alkenylene-, -alkenylene-CH=N-, -alkenylene-N=CH-, -alkylene-CH=N-alkenylene-, -alkenylene-CH=N-alkylene-, and -alkylene-NH-alkenylene-O-. In some embodiments, the double bond in the heteroalkenylene is a carbon-carbon double bond. The heteroalkenylene is optionally substituted with one or more substituents selected from the group consisting of the following substituents: oxo, hydroxy, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "alkynyl" refers to linear or branched unsaturated aliphatic hydrocarbyl consisting of carbon atoms and hydrogen atoms and having at least one triple bond, and usually has 2 to 12, 2 to 8, 2 to 6, 2 to 4, or 2 to 3 carbon atoms. Non-limiting examples of the alkynyl include, but are not limited to, ethynyl (-C≡CH), 1-propynyl (-C≡C-CH₃), 2-propynyl (-CH₂-C≡CH), 1,3-butadiynyl (-C≡C-C≡CH), and the like. The alkynyl is optionally substituted with one or more substituents selected from the group consisting of the following substituents: oxo, hydroxy, amino, nitro, halogen, cyano, alkenyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl, and aryloxy.

The term "cycloalkyl" refers to a carbocyclic ring that is fully saturated and may exist as a monocyclic ring, a bridged ring, or a spiro ring. Unless otherwise indicated, the carbocyclic ring is usually a 3- to 10-membered ring, a 4- to 8-membered ring, a 5- to 8-membered ring, or a 5- to 6-membered ring. Non-limiting examples of cycloalkyl include, but are not limited to, cyclopropanyl, cyclobutanyl, cyclopentanyl, cyclohexanyl, norbornyl (bicyclo[2.2.1]heptyl), bicyclo[2.2.2]octyl, adamantyl, and the like. The cycloalkyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "cycloalkenyl" refers to a non-aromatic carbocyclic ring that is not fully saturated, has at least one double bond, and may exist as a monocyclic ring, a bridged ring, or a spiro ring. Unless otherwise indicated, the carbocyclic ring is usually a 3- to 10-membered ring, a 4- to 8-membered ring, a 5- to 8-membered ring, or a 5- to 6-membered ring. Non-limiting examples of cycloalkenyl include, but are not limited to, cyclopentenyl, cyclopentadienyl, cyclohexenyl, cyclohexadienyl, cycloheptenyl, cycloheptadienyl, and the like. The cycloalkenyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "heterocyclyl" refers to a fully saturated or partially unsaturated (but not a fully unsaturated heteroaromatic group) non-aromatic ring that may exist as a monocyclic ring, a bridged ring, a fused ring, or a spiro ring. Unless otherwise indicated, the heterocyclic ring is usually a 3- to 12-membered, 3- to 10-membered, 4- to 8-membered, 5- to 8-membered, 5- to 6-membered, 3- to 7-membered, or 4- to 6-membered ring comprising 1 to 3 heteroatoms (preferably 1 or 2 heteroatoms) independently selected from the group consisting of sulfur, oxygen, nitrogen, phosphorus, silicon, and/or boron. Non-limiting examples of heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, thiomorpholinyl, tetrahydrothienyl, and the like. The heterocyclyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "heterocycloalkyl" refers to a cyclic group that is fully saturated and may exist as a monocyclic ring, a bridged ring, or a spiro ring. Unless otherwise indicated, the heterocyclic ring is usually a 3- to 12-membered, 3-to 10-membered, 4- to 8-membered, 5- to 8-membered, 5- to 6-membered, 3- to 7-membered, or 4- to 6-membered ring comprising 1 to 3 heteroatoms (preferably 1 or 2 heteroatoms) independently selected from the group consisting of sulfur, oxygen, nitrogen, phosphorus, silicon, and/or boron. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. The heterocycloalkyl is optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "aryl" refers to an all-carbon aromatic monocyclic or fused polycyclic group having a conjugated *π*-electron system. For example, aryl may have 6-20 carbon atoms, 6-14 carbon atoms, or 6-12 carbon atoms. Non-limiting examples of aryl include, but are not limited to, phenyl, naphthyl, anthryl, and the like. The aryl is optionally substituted with one or more substituents selected from the group consisting of the following substituents: hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "heteroaryl" refers to an aromatic monocyclic or fused polycyclic system comprising at least one ring atom selected from the group consisting of N, O, and S, with the remaining ring atoms being C, and usually has a 5- to 14-membered, 5- to 12-membered, 5- to 10-membered, 5- to 8-membered, 5- to 7-membered, or 5- to 6-membered ring. Preferably, heteroaryl has a single 4- to 8-membered ring, in particular a 5- to 6-membered ring, or has fused polycyclic rings comprising 5 to 14 ring atoms, in particular 5 to 10 ring atoms. Non-limiting examples of heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like. The heteroaryl is optionally substituted with one or more substituents selected from the group consisting of the following substituents: hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The group -L¹- in the present disclosure is optionally correspondingly connected to the lower group and the right group that are connected to this group in the general formula in a left-to-right reading order, e.g., when -L¹- is -OCH₂CH₂-, the structure 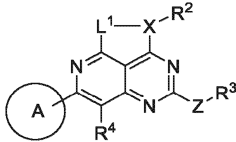 corresponds to

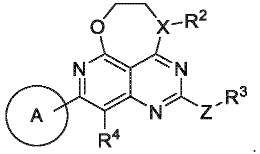

The term "treat", "treating", or "treatment" refers to administering the compound or formulation described herein to ameliorate or eliminate a disease or one or more symptoms associated with the disease, and includes:
(i) inhibiting a disease or disease state, i.e., arresting its progression; and
(ii) alleviating a disease or disease state, i.e., causing its regression.

The term "prevent", "preventing", or "prevention" means administering the compound or formulation described herein to prevent a disease or one or more symptoms associated with the disease, and includes preventing the occurrence of the disease or disease state in a mammal, particularly when such a mammal is predisposed to the disease state but has not yet been diagnosed with it.

The term "therapeutically effective amount" refers to an amount of the compound of the present disclosure for (i) treating or preventing a specific disease, condition, or disorder; (ii) relieving, ameliorating, or eliminating one or more symptoms of a specific disease, condition, or disorder, or (iii) preventing or delaying onset of one or more symptoms of the specific disease, condition, or disorder described herein. The amount of the compound of the present disclosure composing the "therapeutically effective amount" varies dependently on the compound, the disease state and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, and a salt formed with a basic or acidic amino acid.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, and water.

The word "comprise" and variations thereof such as "comprises" or "comprising" will be understood in an open, non-exclusive sense, i.e., "including but not limited to".

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons.

The present disclosure also includes isotopically labeled compounds of the present disclosure, which are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present disclosure (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. The isotopically labeled compounds of the present disclosure can generally be prepared by following procedures analogous to those disclosed in the schemes and/or examples below while substituting a non-isotopically labeled reagent with an isotopically-labeled reagent.

Furthermore, substitution with heavier isotopes such as deuterium (i.e., ²H) may provide certain therapeutic advantages (e.g., increased *in vivo* half-life or reduced dose) resulting from greater metabolic stability and hence may be preferred in some circumstances in which deuterium substitution may be partial or complete, where partial deuterium substitution refers to substitution of at least one hydrogen with at least one deuterium.

The compounds of the present disclosure can be asymmetrical, for example, has one or more stereoisomers.

Unless otherwise stated, all stereoisomers are included, for example, enantiomers and diastereoisomers. The compounds comprising asymmetric carbon atoms of the present disclosure can be isolated in an optically active pure form or in a racemic form. The optically active pure form may be separated from a racemic mixture or may be synthesized using a chiral raw material or a chiral reagent. Non-limiting examples of stereoisomers include, but are not limited to:

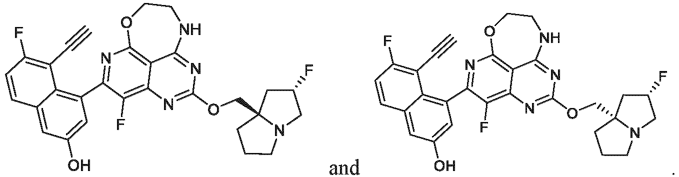

The compounds of the present disclosure may have one or more atropisomers, which, unless otherwise stated, refers to optically active isomers resulting from the hindrance of free rotation between single bonds. The compounds comprising a chiral axis of the present disclosure can be isolated in a racemic form. When the energy barrier for the single bond free rotation of the compounds comprising a chiral axis of the present disclosure is sufficiently high, the atropisomers of the compounds may be isolated in an optically active pure form.

The pharmaceutical composition of the present disclosure can be prepared by combining the compound of the present disclosure with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present disclosure include, but are not limited to, oral, rectal, topical, inhalational, parenteral, sublingual, intravaginal, intranasal, intraocular, intraperitoneal, intramuscular, subcutaneous, and intravenous administration.

The pharmaceutical composition of the present disclosure can be manufactured using methods well known in the art, such as conventional mixing, dissolving, granulating, dragee-making, levigating, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compounds of the present disclosure to be formulated into tablets, pills, pastilles, dragees, capsules, gels, slurries, suspensions, etc. for oral administration to a patient.

A solid oral composition can be prepared by conventional mixing, filling, or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners, flavoring agents, and the like.

The pharmaceutical composition may also be suitable for parenteral administration, such as a sterile solution, a suspension, or a lyophilized product in a suitable unit dosage form.

In all administration methods of the compound of general formula I described herein, the daily dose is 0.01 mg/kg of body weight to 200 mg/kg of body weight. The compounds of the present disclosure can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present disclosure.

The chemical reactions of the specific embodiments of the present disclosure are conducted in a proper solvent that must be suitable for the chemical changes in the present disclosure and the reagents and materials required. In order to acquire the compounds of the present disclosure, it is sometimes necessary for those skilled in the art to modify or select a synthesis procedure or a reaction process based on the existing embodiments.

The compound of formula (III) of the present disclosure may be prepared by those skilled in the art of organic synthesis via route 1, wherein, PG is independently selected from the group consisting of suitable commonly used protecting groups, such as tert-butoxycarbonyl; R², R³, R⁴, Z, and ring A moieties are as defined above.

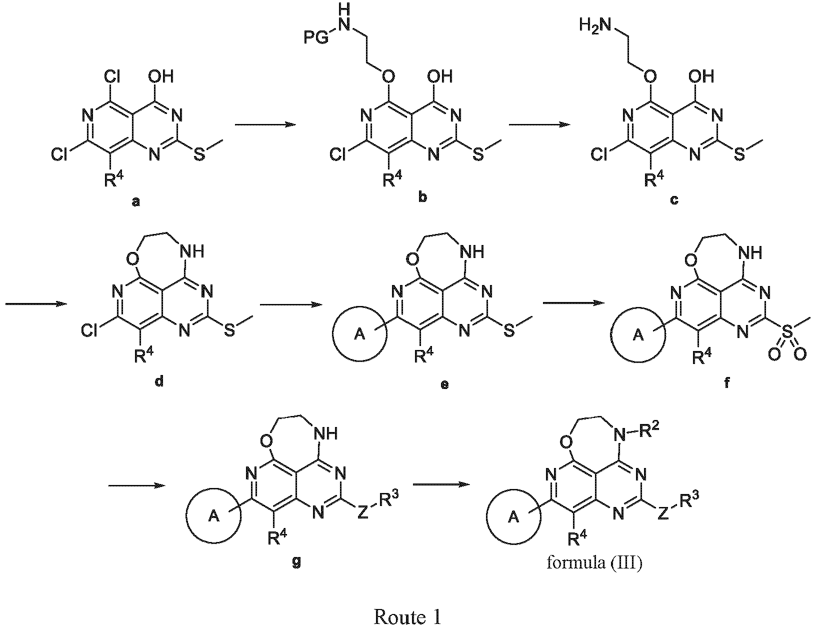

Each of the products from the reactions of the route described above may be obtained by conventional separation techniques including, but not limited to, filtration, distillation, crystallization, chromatography, and the like. The starting materials may be self-synthesized or purchased from commercial establishments (such as, but not limited to, Adrich or Sigma). These materials can be characterized using conventional means, such as physical constants and spectral data. The compounds described herein can be synthesized as a single isomer or a mixture of isomers. The following abbreviations are used in the present disclosure:
TsOH represents *p*-toluenesulfonic acid; DMF represents *N,N-*dimethylformamide; Boc represents *tert-*butoxycarbonyl; TIPS represents triisopropylsilyl; MOM represents methoxymethyl; DMSO represents dimethyl sulfoxide; TBS represents *tert*-butyldimethyl; DIPEA represents diisopropylethylamine; THF represents tetrahydrofuran; PMB represents *p*-methoxybenzyl; FA represents formic acid.

The commercially available compounds are under the supplier's catalog names.

For clarity, the present disclosure is further described with the following examples, which are, however, not intended to limit the scope of the present disclosure. The present disclosure has been described in detail herein and specific embodiments have also been disclosed, it will be apparent to those skilled in the art that various changes and modifications can be made to the embodiments without departing from the spirit and scope of the present disclosure.

All the reagents used in the present disclosure are commercially available and can be used without further purification.

### Examples

### Example 1

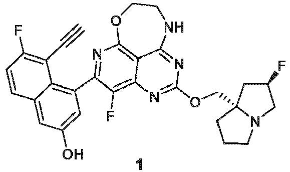

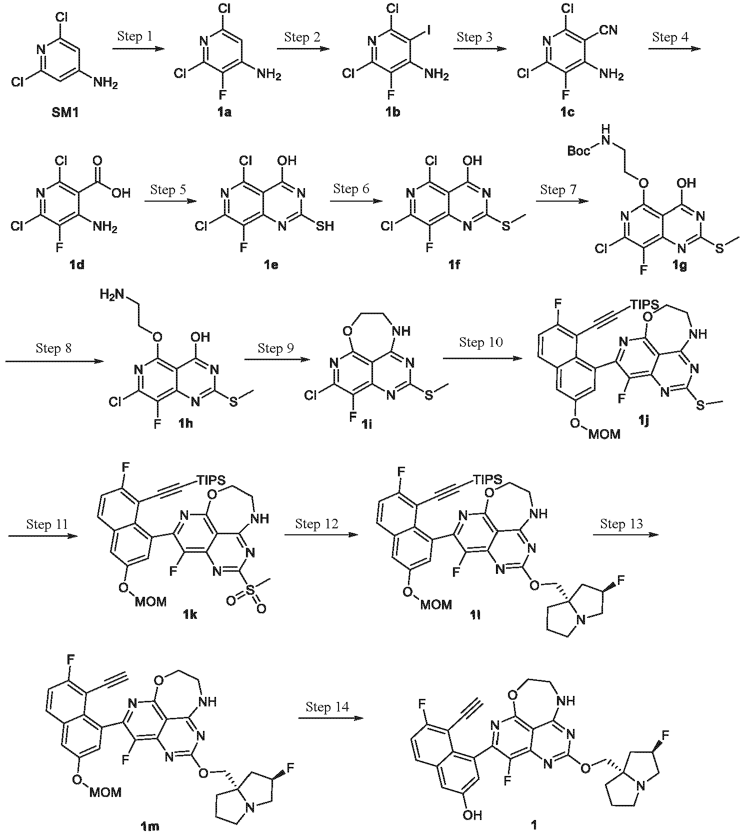

### Step 1:

Compound **SM1** (50 g), 1-chloromethyl-4-fluoro-1,4-diazoniabicyclo[2.2.2]octane bis(tetrafluoroborate) (119.5 g), methanol (450 mL), and H₂O (90 mL) were added to a reaction flask, and after being uniformly stirred, the mixture was heated to 45 °C, stirred for reaction for 24 h, and filtered. The filtrate was concentrated under reduced pressure and purified by column chromatography (petroleum ether:ethyl acetate = 50:1 to 8:1) to give compound **1a.** LC-MS: m/z = 181.01 (M + H)⁺

### Step 2:

Compound **1a** (60 g), *N*-iodosuccinimide (90 g), TsOH·H₂O (3.2 g), and acetonitrile (600 mL) were added to a reaction flask, and after being uniformly stirred, the mixture was heated to 70 °C and stirred for reaction for 4 h. The reaction solution was concentrated to dryness under reduced pressure, and 1 L of ethyl acetate was added to the residue for dissolution. The mixture was washed with a sodium thiosulfate solution, a sodium bicarbonate solution, and brine, respectively, and the organic phase was dried over anhydrous sodium sulfate and filtered. The filtrate was concentrated under reduced pressure to give compound **1b.** LC-MS: m/z = 306.84 (M + H)⁺

### Step 3:

Compound **1b** (100 g), CuCN (45 g), and DMF (1000 mL) were added to a reaction flask, and after being uniformly stirred, the mixture was heated to 100 °C and stirred for reaction for 12 h. The reaction solution was cooled to room temperature and then filtered, and the filtrate was concentrated to dryness under reduced pressure. 1 L of ethyl acetate was added to the residue, and the mixture was stirred and filtered. The filtrate was washed with ammonium hydroxide and brine, respectively, and the organic phase was dried over anhydrous sodium sulfate and filtered. The filtrate was concentrated under reduced pressure to give compound 1c. LC-MS: m/z = 206.07 (M + H)⁺

### Step 4:

NaOH (19.4 g) and H₂O (270 mL) were added to a reaction flask. After complete dissolution with stirring, compound **1c** (25 g) was added, and the mixture was heated to 80 °C and stirred for reaction for 10 h. The reaction solution was cooled to room temperature, and concentrated hydrochloric acid was added dropwise under an ice bath to adjust the pH to acidity. A large amount of solid was precipitated, and the mixture was filtered. The filter cake was dried under reduced pressure to give compound **1d.** LC-MS: m/z = 225.07 (M + H)⁺

### Step 5:

Compound **1d** (10.7 g) and thionyl chloride (100 mL) were added to a reaction flask, and after being uniformly stirred, the mixture was heated to 50 °C and stirred for reaction. After the reaction was completed, the reaction solution was concentrated to dryness under reduced pressure. Acetone (90 mL) was added, and the mixture was stirred for complete dissolution and then added dropwise to an ammonium thiocyanate solution (10.84 g/150 mL). The resulting mixture was stirred at room temperature overnight. The reaction solution was concentrated under reduced pressure and purified by column chromatography (methanol:dichloromethane = 1:50 to 1:10) to give compound **1e.** LC-MS: m/z = 264.16 (M - H)⁻

### Step 6:

Compound **1e** (9.3 g), methanol (300 mL), and an aqueous sodium hydroxide solution (2.8 g/30 mL) were added to a reaction flask. After the mixture was uniformly stirred, iodomethane (9.9 g) was added, and the resulting mixture was stirred at room temperature for 6 h. The pH of the reaction solution was adjusted to neutrality, and the reaction solution was concentrated to dryness under reduced pressure and then purified by column chromatography (ethyl acetate:petroleum ether = 1:20 to 1:2) to give compound **1f.** LC-MS: m/z = 279.97 (M + H)⁺

### Step 7:

*N*-Boc-ethanolamine (13.7 g), lithium hydride (0.68 g), and DMF (21 mL) were added to a reaction flask. After 30 min of reaction at room temperature, compound **1f** (2.8 g) and DMF (70 mL) were added, and after being uniformly stirred, the mixture was heated to 60 °C and stirred for reaction for 4 h, followed by the addition of 1 L of water to adjust the pH to neutrality and filtration. The filter cake was purified by column chromatography (ethyl acetate:petroleum ether = 1:10 to 1:3) to give compound **1g.** LC-MS: m/z = 305.03 (M + H - Boc + H)⁺

### Step 8:

Compound **1g** (12.3 g) and a solution of hydrogen chloride in ethyl acetate (123 mL) were added to a reaction flask, and after being uniformly stirred, the mixture reacted at room temperature overnight and then was filtered. The filter cake was dried under reduced pressure to give compound **1h.** LC-MS: m/z = 305.03 (M + H)⁺

### Step 9:

Compound **1h** (6.8 g), benzotriazole-1-yl-oxytripyrrolidinophosphonium hexafluorophosphate (12.5 g), triethylamine (10.1 g), and 1,4-dioxane (680 mL) were added to a reaction flask, and after being uniformly stirred in an ice-water bath, the mixture was stirred at room temperature for 4 h. After the reaction was completed, the reaction solution was concentrated to dryness under reduced pressure and purified by column chromatography (ethyl acetate:petroleum ether = 1:10 to 1:2) to give compound **1i.** LC-MS: m/z = 287.03 (M + H)⁺

### Step 10:

Compound **1i** (524 mg), ((2-fluoro-6-(methoxymethoxy)-8-(4,4,5,5-tetramethyl-1,3,2-dioxaborolan-2-yl)naphthalen-1-yl)ethynyl)triisopro pylsilane (2.33 g), chloro[4-(di-tert-butylphosphino)-N,N-dimethylaniline-2-(2'-aminobiphenyl)]palladium(II) (210 mg), tripotassium phosphate (1160 mg), 1,4-dioxane (100 mL), and purified water (20 mL) were added to a reaction flask, and after being purged with nitrogen three times, the mixture was uniformly stirred at room temperature and heated to 90 °C for reaction for 10 h. After the reaction was completed, the reaction solution was filtered, and the filtrate was concentrated to dryness under reduced pressure and purified by column chromatography (ethyl acetate:petroleum ether = 1:10 to 1:2) to give compound **1j.** LC-MS: m/z = 637.36 (M + H)⁺

### Step 11:

Compound **1j** (1010 mg), m-chloroperoxybenzoic acid (800 mg), and dichloromethane (200 mL) were added to a reaction flask, and after being uniformly stirred in an ice-water bath, the mixture reacted at room temperature for 1 h. After the reaction was completed, an aqueous sodium thiosulfate (10%) solution (100 mL) was added to the reaction flask. The resulting mixture was stirred, and the phases were separated. The organic phase was collected, concentrated to dryness under reduced pressure, and purified by column chromatography (methanol:dichloromethane = 1:500 to 1:100) to give compound **1k.** LC-MS: m/z = 669.37 (M + H)⁺

### Step 12:

Compound **1k** (360 mg), ((2*R*,7a*S*)-2-fluorotetrahydro-1*H*-pyrrolizin-7a(5*H*)-yl)methanol (420 mg), a solution of lithium bis(trimethylsilyl)amide (1 N) in tetrahydrofuran (2.7 mL), and tetrahydrofuran (150 mL) were added to a reaction flask, and after being uniformly stirred in an ice-water bath, the mixture reacted at room temperature overnight. After the reaction was completed, a saturated aqueous ammonium chloride solution (100 mL) was added to the reaction system. The mixture was stirred, and the phases were separated. The organic phase was collected, concentrated to dryness under reduced pressure, and purified by column chromatography (methanol:dichloromethane = 1:200 to 1:50) to give compound **1l.** LC-MS: m/z = 748.44 (M + H)⁺

### Step 13:

Compound **1l** (150 mg), cesium fluoride (302 mg), and DMF (15 mL) were added to a reaction flask, and the mixture was stirred for reaction at room temperature for 2 h. 100 mL of water and 100 mL of ethyl acetate were added to the reaction solution, the resulting mixture was stirred, and the phases were separated. The organic phase was collected, concentrated to dryness under reduced pressure, and purified by column chromatography (methanol:dichloromethane = 1:200 to 1:50) to give compound **1m.** LC-MS: m/z = 592.26 (M + H)⁺

### Step 14:

Compound **1m** (133 mg) and ethyl acetate (20 mL) were added to a reaction flask, and the reaction system was stirred at 0 °C to 5 °C for 5 min. Then, a solution of hydrogen chloride in ethyl acetate (2 mL) was added dropwise to the reaction system for reaction at 0 °C to 5 °C for 1 h. After the reaction was completed, the reaction solution was concentrated to dryness under reduced pressure, purified by preparative liquid phase chromatography (YMC C18 10 µm 30 × 250 chromatographic column; acetonitrile-30 mM ammonium acetate (gradient elution of 5%-5%-60%/0-5-60 min), and then desalted and purified by preparative liquid phase chromatography (YMC C18 10 µm 30 × 250 chromatographic column; acetonitrile-0.1% formic acid (gradient elution of 10%-10%-70%/0-5-60 min) to give compound 1 (35 mg, Rt = 36.4 min). LC-MS: m/z = 548.24 (M + H)^{+ 1}H NMR (500 MHz, *d*-DMSO) δ 9.190 (s, 1H), 7.966-7.936 (m,1H), 7.451 (t, 1H), 7.367 (d, 1H), 7.157 (d, 1H), 5.288 (d, 1H), 4.495 (t, 2H), 4.122-4.023 (m, 3H), 3.703 (t, 2H), 3.163-3.066 (m, 3H), 2.891-2.831 (m, 1H), 2.171-2.063 (m, 2H), 2.031-1.962(m, 1H), 1.910-1.766(m, 3H).

### Example 2

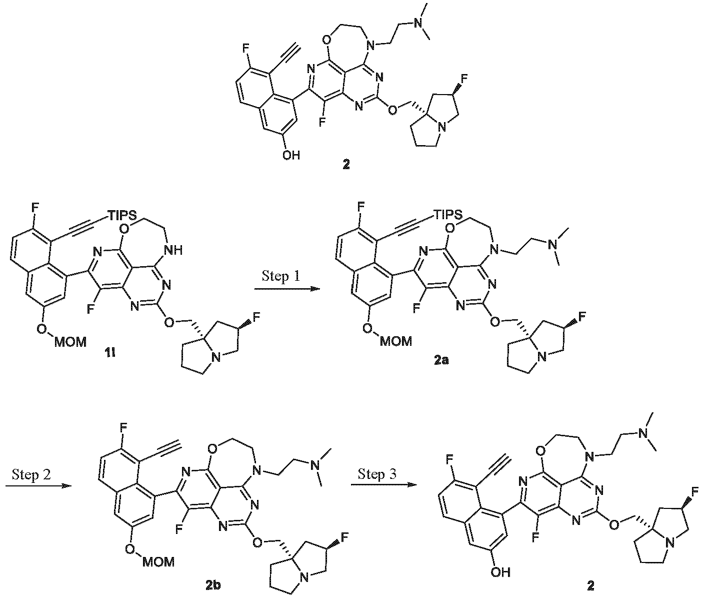

### Step 1:

Compound **1l** (150 mg), 2-(dimethylamino)ethyl iodide hydroiodide (196 mg), cesium carbonate (455 mg), and acetone (15 mL) were added to a reaction flask, and after being uniformly stirred at room temperature, the mixture was heated to 60 °C for reaction for 6 h, cooled to room temperature, and filtered. The filtrate was concentrated to dryness under reduced pressure and purified by column chromatography (methanol:dichloromethane = 1:200 to 1:50) to give compound **2a.** LC-MS: m/z = 819.52 (M + H)⁺

### Step 2:

Compound **2a** (164 mg), cesium fluoride (302 mg), and DMF (15 mL) were added to a reaction flask, and the mixture was stirred for reaction at room temperature for 2 h. 100 mL of water and 100 mL of ethyl acetate were added to the reaction solution, the resulting mixture was stirred, and the phases were separated. The organic phase was collected, concentrated to dryness under reduced pressure, and purified by column chromatography (methanol:dichloromethane = 1:200 to 1:50) to give compound **2b.** LC-MS: m/z = 663.35 (M + H)⁺

### Step 3:

Compound **2b** (133 mg) and ethyl acetate (20 mL) were added to a reaction flask, and the reaction system was stirred at 0 °C to 5 °C for 5 min. Then, a solution of hydrogen chloride in ethyl acetate (2 mL) was added dropwise to the reaction system for reaction at 0 °C to 5 °C for 1 h. After the reaction was completed, the reaction solution was concentrated to dryness under reduced pressure, purified by preparative liquid phase chromatography (YMC C18 10 µm 30 × 250 chromatographic column; acetonitrile-30 mM ammonium acetate (gradient elution of 5%-5%-60%/0-5-60 min), and then purified by preparative liquid phase chromatography (YMC C18 10 µm 30 × 250 chromatographic column; acetonitrile-0.1% formic acid (gradient elution of 10%-10%-70%/0-5-60 min) to give compound **2** (8 mg, Rt = 33.3 min). LC-MS: m/z = 619.29 (M + H)⁺

### Example 3

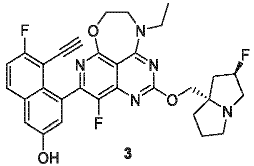

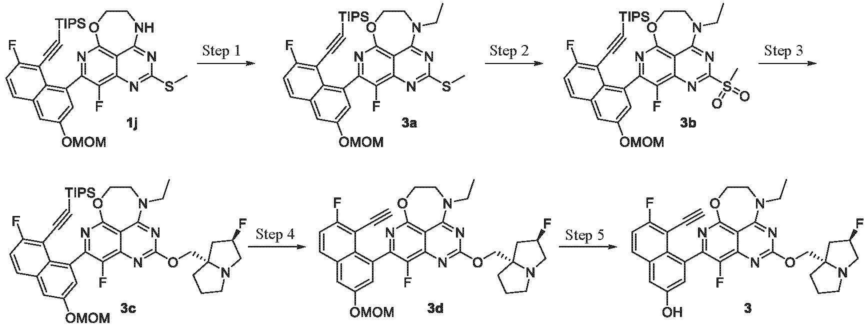

### Step 1:

**1j** (318 mg), iodoethane (775 mg), and potassium carbonate (207 mg) were dispersed in acetone (30 mL), and the mixture was heated to 60 °C and stirred for reaction for 6 h. The reaction solution was cooled to room temperature and filtered, and the filtrate was concentrated until no liquid flowed out to give compound **3a.** LC-MS: m/z = 665.35 (M + H)⁺.

### Step 2:

**3a** (190 mg) was dispersed in dichloromethane (19 mL), and the mixture was cooled to 0 °C to 5 °C. *m*-Chloroperoxybenzoic acid (145 mg) was added, and the resulting mixture was stirred for 1 h with the temperature maintained. After the reaction was completed, a 10% sodium thiosulfate solution (200 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:500 to 1:100) to give compound **3b.** LC-MS: m/z = 697.35 (M + H)⁺.

### Step 3:

**3b** (195 mg) and ((2*R*,7a*S*)-2-fluorotetrahydro-1*H*-pyrrolizin-7a(5*H*)-yl)methanol (218 mg) were dispersed in tetrahydrofuran (20 mL). Under a nitrogen atmosphere, the mixture was cooled to 0 °C, and a solution of lithium bis(trimethylsilyl)amide in tetrahydrofuran (1 N, 1.4 mL) was added dropwise. The resulting mixture was stirred for 30 min with the temperature maintained and then was allowed to be stirred at room temperature overnight. A saturated ammonium chloride solution (100 mL) was added to the reaction system, and the mixture was extracted with dichloromethane (50 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:200 to 1:50) to give compound **3c.** LC-MS: m/z = 776.45 (M + H)⁺.

### Step 4:

**3c** (170 mg) and cesium fluoride (320 mg) were dispersed in DMF (17 mL), and the mixture was stirred for reaction at room temperature for 2 h. Water (200 mL) and ethyl acetate (200 mL) were added to the reaction solution, the mixture was stirred, and the phases were separated. The organic phase was washed with 5% brine (100 mL × 4), dried over anhydrous sodium sulfate, and filtered. The filtrate was concentrated until no liquid flowed out to give compound **3d.** LC-MS: m/z = 620.31 (M + H)⁺.

### Step 5:

**3d** (130 mg) was dispersed in ethyl acetate (10 mL), and the reaction system was cooled to 0 °C to 5 °C and stirred for 5 min. A solution of hydrogen chloride in ethyl acetate (4 N, 1 mL) was added dropwise to the reaction system, and the resulting mixture reacted for 1 h with the temperature maintained. After the reaction was completed, the reaction solution was concentrated to dryness under reduced pressure and purified by column chromatography (methanol:dichloromethane = 1:20) to give compound **3.** LC-MS: m/z = 576.29 (M + H)⁺.

¹H-NMR (500 MHz, DMSO-*d₆*), *δ*: 10.16 (br, 1H), 7.95 (dd, 1H), 7.45 (t, 1H), 7.36 (d, 1H), 7.14 (d, 1H), 5.28 (d, 1H), 4.52 (t, 2H), 4.14-3.89 (m, 6H), 3.82-3.75 (m, 1H), 3.13-3.00 (m, 3H), 2.87-2.81 (m, 1H), 2.14-1.97 (m, 3H), 1.88-1.74(m, 3H), 1.25(t, 3H).

### Example 4

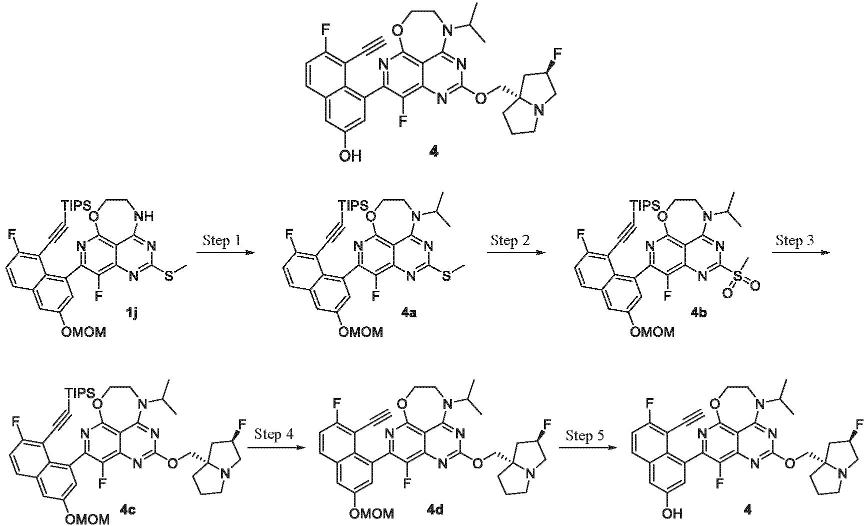

### Step 1:

**1j** (318 mg), 2-iodopropane (850 mg), and cesium carbonate (488 mg) were dispersed in acetone (32 mL), and the mixture was heated to 60 °C and stirred for reaction for 6 h. The reaction solution was cooled to room temperature and filtered, and the filtrate was concentrated until no liquid flowed out and purified by column chromatography (methanol:dichloromethane = 1:500) to give compound **4a.** LC-MS: m/z = 679.38 (M + H)⁺.

### Step 2:

**4a** (270 mg) was dispersed in dichloromethane (27 mL), and the mixture was cooled to 0 °C to 5 °C. *m*-Chloroperoxybenzoic acid (200 mg) was added, and the resulting mixture was stirred for 1 h with the temperature maintained. After the reaction was completed, a 10% sodium thiosulfate solution (200 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:500 to 1:100) to give compound **4b.** LC-MS: m/z = 711.42 (M + H)⁺.

### Step 3:

**4b** (300 mg) and ((2*R*,7a*S*)-2-fluorotetrahydro-1*H*-pyrrolizin-7a(5*H*)-yl)methanol (318 mg) were dispersed in tetrahydrofuran (30 mL). Under a nitrogen atmosphere, the mixture was cooled to 0 °C, and a solution of lithium bis(trimethylsilyl)amide in tetrahydrofuran (1 N, 2 mL) was added dropwise. The resulting mixture was stirred for 30 min with the temperature maintained and then was allowed to be stirred at room temperature for 1 h. A saturated ammonium chloride solution (100 mL) was added to the reaction system, and the mixture was extracted with ethyl acetate (80 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:200 to 1:50) to give compound **4c.** LC-MS: m/z = 790.48 (M + H)⁺.

### Step 4:

**4c** (252 mg) and cesium fluoride (482 mg) were dispersed in DMF (25 mL), and the mixture was stirred for reaction at room temperature for 2 h. Water (200 mL) and ethyl acetate (200 mL) were added to the reaction solution, the mixture was stirred, and the phases were separated. The organic phase was washed with saturated brine (100 mL × 4), dried over anhydrous sodium sulfate, and filtered. The filtrate was concentrated until no liquid flowed out to give compound 4d. LC-MS: m/z = 634.37 (M + H)⁺.

### Step 5:

**4d** (203 mg) was dispersed in ethyl acetate (10 mL), and the reaction system was cooled to 0 °C to 5 °C and stirred for 5 min. A solution of hydrogen chloride in ethyl acetate (4 N, 1 mL) was added dropwise to the reaction system, and the resulting mixture was stirred for reaction for 1 h with the temperature maintained. After the reaction was completed, the reaction solution was concentrated to dryness under reduced pressure and purified by preparative liquid phase chromatography (YMC ODS 10 µm 50 × 250 chromatographic column; acetonitrile-0.1% aqueous formic acid solution (gradient elution of 20%-50%/0-60 min) to give compound **4.** LC-MS: m/z = 590.28 (M + H)⁺.
¹H-NMR (500 MHz, DMSO-*d₆*), *δ*: 7.95 (dd, 1H), 7.45 (t, 1H), 7.37 (s, 1H), 7.14 (s, 1H), 5.44-5.38 (m, 1H), 5.28 (d, 1H), 4.54-4.46 (m, 2H), 4.15-3.72 (m, 6H), 3.13-3.05 (m, 3H), 2.87-2.83 (m, 1H), 2.14-1.97 (m, 3H), 1.88-1.74(m, 3H), 1.25(t, 6H)

### Example 5

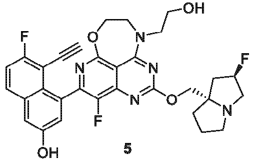

Referring to the preparation of compound **4** in Example 4, compound **5** was prepared by replacing 2-iodopropane with *tert*-butyl(2-iodoethoxy)dimethylsilane in step 1. LC-MS: m/z = 592.27 (M + H)⁺.

### Example 6

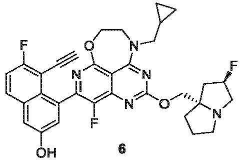

Referring to the preparation of compound **4** in Example 4, compound 6 was prepared by replacing 2-iodopropane with (iodomethyl)cyclopropane in step 1. LC-MS: m/z = 602.29 (M + H)⁺.

### Example 7

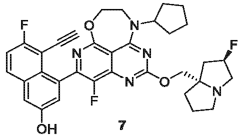

Referring to the preparation of compound **4** in Example 4, compound **7** was prepared by replacing 2-iodopropane with iodocyclopentane in step 1. LC-MS: m/z = 616.36 (M + H)⁺.

### Example 8

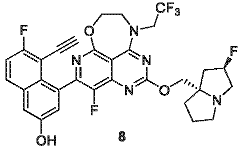

Referring to the preparation of compound **4** in Example 4, compound **8** was prepared by replacing 2-iodopropane with 2-iodo-1,1,1-trifluoroethane in step 1. LC-MS: m/z = 630.29 (M + H)⁺.

### Example 9

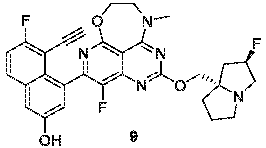

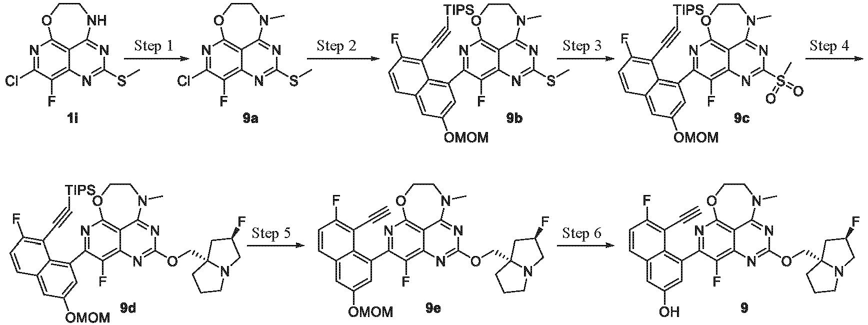

### Step 1:

**1i** (287 mg), iodomethane (1410 mg), and potassium carbonate (414 mg) were dispersed in acetone (50 mL), and the mixture was heated to 60 °C and stirred for reaction for 6 h. The reaction solution was cooled to room temperature and filtered, and the filtrate was concentrated until no liquid flowed out and purified by column chromatography (ethyl acetate:petroleum ether = 1:10 to 1:2) to give compound **9a.** LC-MS: m/z = 301.03 (M + H)⁺.

### Step 2:

**9a** (301 mg), ((2-fluoro-6-(methoxymethoxy)-8-(4,4,5,5-tetramethyl-1,3,2-dioxaborolan-2-yl)naphthalen-1-yl)ethynyl)triisopro pylsilane (1.28 g), chloro[4-(di-tert-butylphosphino)-N,N-dimethylaniline-2-(2'-aminobiphenyl)]palladium(II) (115 mg), potassium phosphate (636 mg), and water (6 mL) were dispersed in 1,4-dioxane (30 mL), and under a nitrogen atmosphere, the mixture was heated to 90 °C and stirred for reaction for 10 h. The reaction solution was cooled to room temperature and filtered, and the filtrate was concentrated to dryness under reduced pressure and purified by column chromatography (ethyl acetate:petroleum ether = 1:10 to 1:2) to give compound **9b.** LC-MS: m/z = 651.35 (M + H)⁺.

### Step 3:

**9b** (415 mg) was dispersed in dichloromethane (80 mL), and the mixture was cooled to 0 °C to 5 °C. *m*-Chloroperoxybenzoic acid (320 mg) was added, and the resulting mixture was stirred for 1 h with the temperature maintained. After the reaction was completed, a 10% sodium thiosulfate solution (200 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:500 to 1:100) to give compound **9c.** LC-MS: m/z = 683.47 (M + H)⁺.

### Step 4:

**9c** (437 mg) and ((2*R*,7a*S*)-2-fluorotetrahydro-1*H*-pyrrolizin-7a(5*H*)-yl)methanol (511 mg) were dispersed in tetrahydrofuran (80 mL). Under a nitrogen atmosphere, the mixture was cooled to 0 °C, and a solution of lithium bis(trimethylsilyl)amide in tetrahydrofuran (1 N, 3.3 mL) was added dropwise. The resulting mixture was stirred for 30 min with the temperature maintained and then was allowed to be stirred at room temperature overnight. A saturated ammonium chloride solution (100 mL) was added to the reaction system, and the mixture was extracted with dichloromethane (50 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:200 to 1:50) to give compound **9d.** LC-MS: m/z = 762.57 (M + H)⁺.

### Step 5:

**9d** (360 mg) and cesium fluoride (710 mg) were dispersed in DMF (36 mL), and the mixture was stirred for reaction at room temperature for 2 h. Water (200 mL) and ethyl acetate (200 mL) were added to the reaction solution, the mixture was stirred, and the phases were separated. The organic phase was washed with 5% brine (100 mL × 4), dried over anhydrous sodium sulfate, and filtered. The filtrate was concentrated until no liquid flowed out to give compound **9e.** LC-MS: m/z = 606.32 (M + H)⁺.

### Step 6:

**9e** (121 mg) was dispersed in ethyl acetate (20 mL), and the reaction system was cooled to 0 °C to 5 °C and stirred for 5 min. A solution of hydrogen chloride in ethyl acetate (4 N, 2 mL) was added dropwise to the reaction system, and the resulting mixture reacted for 1 h with the temperature maintained. After the reaction was completed, the reaction solution was concentrated to dryness under reduced pressure and purified by column chromatography (methanol:dichloromethane = 1:20) to give compound **9.** LC-MS: m/z = 562.26 (M + H)⁺.

### Example 10

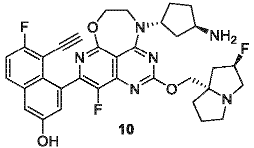

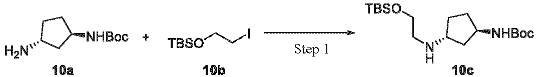

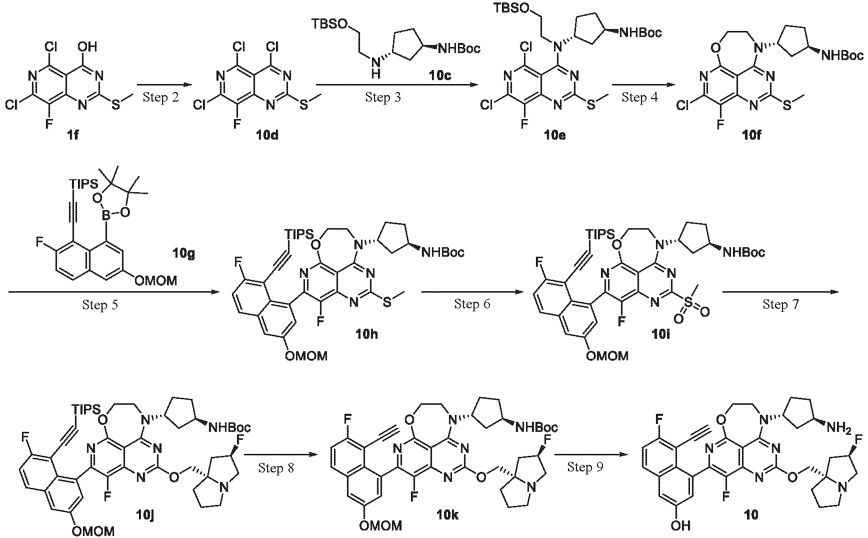

### Step 1:

**10a** (1.15 g), **10b** (1 g), and potassium carbonate (2.05 g) were dispersed in DMF (10 mL), and the mixture was stirred for reaction at room temperature for 12 h. Water (50 mL) and ethyl acetate (50 mL) were added to the reaction solution, the mixture was stirred, and the phases were separated. The aqueous phase was extracted with ethyl acetate (50 mL × 2), and the organic phases were combined, washed with 5% brine (50 mL × 3), dried over anhydrous sodium sulfate, and filtered. The filtrate was concentrated until no liquid flowed out to give compound **10c.** LC-MS: m/z = 359.37 (M + H)⁺.

### Step 2:

**1f** (5 g) was dispersed in acetonitrile (100 mL), and DIPEA (13.8 g) and POCl₃ (8.19 g) were added sequentially. Under a nitrogen atmosphere, the mixture was heated to 80 °C and stirred for reaction for 2 h. After the reaction was completed, the reaction solution was cooled to room temperature and poured into ice water (500 mL), and the mixture was stirred for crystallization for 30 min and filtered. The filter cake was dried under vacuum at 55 °C for 6 h to give compound **10d.**

### Step 3:

**10d** (1.6 g) was dispersed in THF (10 mL), and **10c** (1.6 g) and DIPEA (2.3 g) were added sequentially. The mixture was stirred for reaction at room temperature for 1 h. After the reaction was completed, the reaction solution was concentrated under reduced pressure and purified by column chromatography (methanol:dichloromethane = 1:100 to 1:20) to give compound **10e.** LC-MS: m/z = 620.32 (M + H)⁺.

### Step 4:

**10e** (1.1 g) and cesium fluoride (5.4 g) were dispersed in DMF (10 mL), and the mixture was heated to 60 °C and stirred for reaction for 3 h. After the reaction was completed, water (100 mL) and ethyl acetate (100 mL) were added to the reaction solution, the mixture was stirred, and the phases were separated. The organic phase was washed with 10% brine (100 mL × 2), dried over anhydrous sodium sulfate, and filtered. The filtrate was concentrated until no liquid flowed out and purified by column chromatography (methanol:dichloromethane = 1:100 to 1:10) to give compound **10f.** LC-MS: m/z = 470.24 (M + H)⁺.

### Step 5:

**10f** (0.8 g), **10g** (1.7 g), chloro[4-(di-tert-butylphosphino)-N ,N-dimethylaniline-2-(2'-aminobiphenyl) palladium (II) (0.2 g), potassium phosphate (1 g), and water (6.4 mL) were dispersed in 1,4-dioxane (32 mL), and under a nitrogen atmosphere, the mixture was heated to 90 °C and stirred for reaction for 2 h. The reaction solution was cooled to room temperature and filtered, and the filtrate was concentrated to dryness under reduced pressure and purified by column chromatography (ethyl acetate:petroleum ether = 1:10 to 1:1) to give compound **10h.** LC-MS: m/z = 820.74 (M + H)⁺.

### Step 6:

**10h** (550 mg) was dispersed in dichloromethane (50 mL), and the mixture was cooled to 0 °C to 5 °C. *m*-Chloroperoxybenzoic acid (290 mg) was added, and the resulting mixture was stirred for 1 h with the temperature maintained. After the reaction was completed, a 10% sodium thiosulfate solution (200 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:500 to 1:100) to give compound **10i.** LC-MS: m/z = 852.47 (M + H)⁺.

### Step 7:

**10i** (400 mg) and ((2*R*,7a*S*)-2-fluorotetrahydro-1*H*-pyrrolizin-7a(5*H*)-yl)methanol (220 mg) were dispersed in tetrahydrofuran (5 mL). The mixture was cooled to 0 °C, and a solution of lithium bis(trimethylsilyl)amide in tetrahydrofuran (1 N, 1.2 mL) was added dropwise. The resulting mixture was stirred for 30 min with the temperature maintained. After the reaction was completed, a saturated ammonium chloride solution (100 mL) was added to the reaction system, and the mixture was extracted with ethyl acetate (80 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:200 to 1:50) to give compound **10j.** LC-MS: m/z = 931.60 (M + H)⁺.

### Step 8:

**10j** (600 mg) and cesium fluoride (2.16 g) were dispersed in DMF (12 mL), and the mixture was stirred for reaction at room temperature for 2 h. Water (200 mL) and ethyl acetate (200 mL) were added to the reaction solution, the mixture was stirred, and the phases were separated. The organic phase was washed with 5% brine (100 mL × 4), dried over anhydrous sodium sulfate, and filtered. The filtrate was concentrated until no liquid flowed out to give compound **10k.** LC-MS: m/z = 775.48 (M + H)⁺.

### Step 9:

**10k** (500 mg) was dispersed in dichloromethane (10 mL), and the reaction system was cooled to 0 °C to 5 °C. A solution of hydrogen chloride in 1,4-dioxane (4 N, 2 mL) was added dropwise to the reaction system, and the resulting mixture reacted for 1 h with the temperature maintained. After the reaction was completed, the reaction solution was concentrated to dryness under reduced pressure and purified by preparative liquid phase chromatography (YMC ODS 10 µm 50 × 250 chromatographic column; acetonitrile-0.1% aqueous formic acid solution (gradient elution of 10%-40%/0-60 min) to give compound 10. LC-MS: m/z = 631.39 (M + H)⁺.

### Example 11

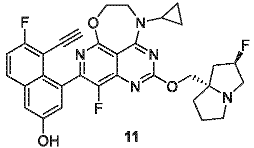

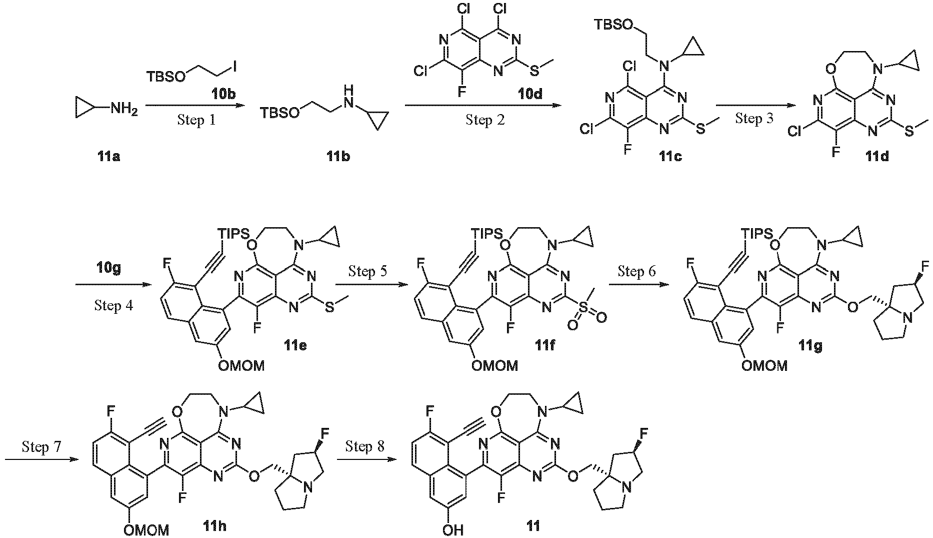

### Step 1:

**11a** (0.57 g), **10b** (2.29 g), and potassium carbonate (4.42 g) were dispersed in DMF (60 mL), and the mixture was stirred for reaction at room temperature for 12 h. Water (50 mL) and ethyl acetate (50 mL) were added to the reaction solution, the mixture was stirred, and the phases were separated. The aqueous phase was extracted with ethyl acetate (50 mL × 2), and the organic phases were combined, washed with 5% brine (50 mL × 3), dried over anhydrous sodium sulfate, and filtered. The filtrate was concentrated until no liquid flowed out to give compound **11b.** LC-MS: m/z = 216.23 (M + H)⁺.

### Step 2:

**10d** (0.6 g) was dispersed in acetonitrile (60 mL), and **11b** (1.86 g) and DIPEA (1.03 g) were added sequentially. The mixture was stirred for reaction at room temperature for 1 h. After the reaction was completed, the reaction solution was concentrated under reduced pressure and purified by column chromatography (methanol:dichloromethane = 1:100 to 1:20) to give compound **11c.** LC-MS: m/z = 477.18 (M + H)⁺.

### Step 3:

**11c** (628 mg) and cesium fluoride (2 g) were dispersed in DMF (63 mL), and the mixture was heated to 60 °C and stirred for reaction for 3 h. After the reaction was completed, water (200 mL) and ethyl acetate (200 mL) were added to the reaction solution, the mixture was stirred, and the phases were separated. The organic phase was washed with 10% brine (100 mL × 2), dried over anhydrous sodium sulfate, and filtered. The filtrate was concentrated until no liquid flowed out and purified by column chromatography (methanol:dichloromethane = 1:100 to 1:10) to give compound **11d.** LC-MS: m/z = 327.12 (M + H)⁺.

### Step 4:

**11d** (378 mg), **10g** (1.2 g), chloro[4-(di-tert-butylphosphino)-N,N-dimethylaniline-2-(2'-aminobiphenyl) palladium (II) (132 mg), potassium phosphate (735 mg), and water (8 mL) were dispersed in 1,4-dioxane (40 mL), and under a nitrogen atmosphere, the mixture was heated to 90 °C and stirred for reaction for 2 h. The reaction solution was cooled to room temperature and filtered, and the filtrate was concentrated to dryness under reduced pressure and purified by column chromatography (ethyl acetate:petroleum ether = 1:10 to 1:1) to give compound 11e. LC-MS: m/z = 677.29 (M + H)⁺.

### Step 5:

**11e** (540 mg) was dispersed in dichloromethane (54 mL), and the mixture was cooled to 0 °C to 5 °C. *m*-Chloroperoxybenzoic acid (400 mg) was added, and the resulting mixture was stirred for 1 h with the temperature maintained. After the reaction was completed, a 10% sodium thiosulfate solution (200 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:500 to 1:100) to give compound **11f.** LC-MS: m/z = 709.32 (M + H)⁺.

### Step 6:

**11f** (600 mg) and ((2*R*,7a*S*)-2-fluorotetrahydro-1*H*-pyrrolizin-7a(5*H*)-yl)methanol (636 mg) were dispersed in tetrahydrofuran (60 mL). The mixture was cooled to 0 °C, and a solution of lithium bis(trimethylsilyl)amide in tetrahydrofuran (1 N, 4 mL) was added dropwise. The resulting mixture was stirred for 30 min with the temperature maintained. After the reaction was completed, a saturated ammonium chloride solution (100 mL) was added to the reaction system, and the mixture was extracted with ethyl acetate (100 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:500 to 1:100) to give compound **11g.** LC-MS: m/z = 788.44 (M + H)⁺.

### Step 7:

**11g** (330 mg) and cesium fluoride (634 mg) were dispersed in DMF (33 mL), and the mixture was stirred for reaction at room temperature for 3 h. Water (100 mL) and ethyl acetate (100 mL) were added to the reaction solution, the mixture was stirred, and the phases were separated. The organic phase was washed with 5% brine (80 mL × 4), dried over anhydrous sodium sulfate, and filtered. The filtrate was concentrated until no liquid flowed out to give compound **11h.** LC-MS: m/z = 632.32 (M + H)⁺.

### Step 8:

**11h** (260 mg) was dispersed in ethyl acetate (10 mL), and the reaction system was cooled to 0 °C to 5 °C. A solution of hydrogen chloride in ethyl acetate (4 N, 1 mL) was added dropwise to the reaction system, and the resulting mixture reacted for 1 h with the temperature maintained. After the reaction was completed, the reaction solution was concentrated to dryness under reduced pressure and purified by column chromatography (methanol:dichloromethane = 1:20) to give compound **11.** LC-MS: m/z = 588.28 (M + H)⁺.

### Example 12

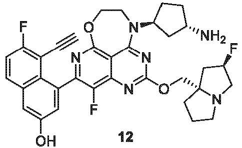

Referring to the preparation of compound **11** in Example 11, compound **12** was prepared by replacing **11a** with *tert*-butyl ((1*S*,3*S*)-3-aminocyclopentyl)carbamate in step 1. LC-MS: m/z = 631.38 (M + H)⁺.

### Example 13

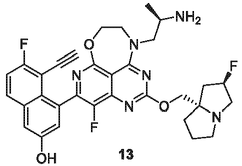

Referring to the preparation of compound **11** in Example 11, compound **13** was prepared by replacing **11a** with *tert*-butyl (*R*)-(1-aminopropan-2-yl)carbamate in step 1. LC-MS: m/z = 605.37 (M + H)⁺.

### Example 14

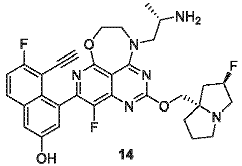

Referring to the preparation of compound **11** in Example 11, compound **14** was prepared by replacing **11a** with *tert-butyl* (*S*)-(1-aminopropan-2-yl)carbamate in step 1. LC-MS: m/z = 605.37 (M + H)⁺.

### Example 15

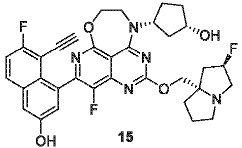

Referring to the preparation of compound **11** in Example 11, compound **15** was prepared by replacing **11a** with (1*S*,3*R*)-3-aminocyclopentanol hydrochloride in step 1. LC-MS: m/z = 632.32 (M + H)⁺.

### Example 16

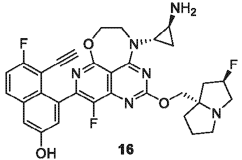

Referring to the preparation of compound **11** in Example 11, compound **16** was prepared by replacing **11a** with *tert-butyl* ((1*S*,2*S*)-2-aminocyclopropyl)carbamate in step 1. LC-MS: m/z = 603.23 (M + H)⁺.

### Example 17

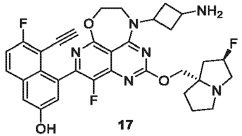

Referring to the preparation of compound **11** in Example 11, compound **17** was prepared by replacing **11a** with *tert-butyl* (3-aminocyclobutyl)carbamate in step 1. LC-MS: m/z = 617.33 (M + H)⁺.

### Example 18

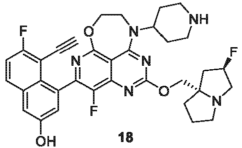

Referring to the preparation of compound **11** in Example 11, compound **18** was prepared by replacing **11a** with 4-amino-1-*tert*-butoxycarbonylpiperidine in step 1. LC-MS: m/z = 631.38 (M + H)⁺.

### Example 19

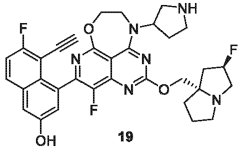

Referring to the preparation of compound **11** in Example 11, compound **19** was prepared by replacing **11a** with 3-amino-1-(*tert*-butoxycarbonyl)pyrrolidine in step 1. LC-MS: m/z = 617.45 (M + H)⁺.

### Example 20

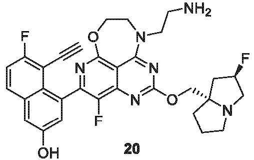

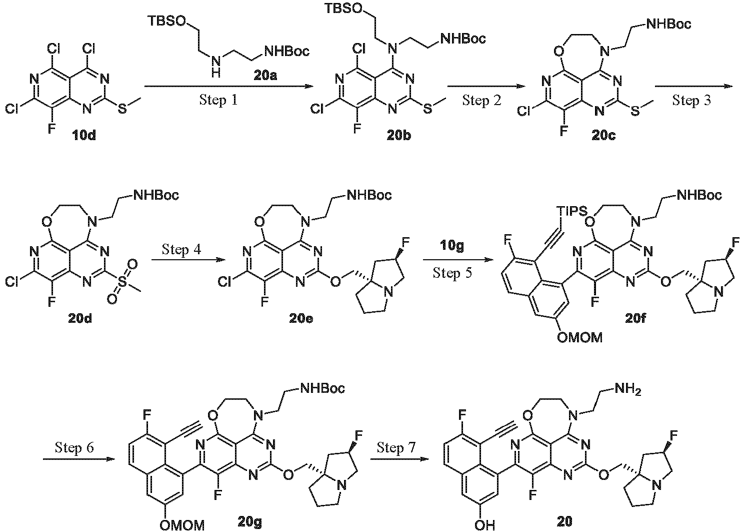

### Step 1:

**10d** (1 g) was dispersed in tetrahydrofuran (20 mL), and DIPEA (1.73 g) and **20a** (2.8 g) were added sequentially. The mixture was stirred for reaction at room temperature for 1 h. After the reaction was completed, the reaction solution was concentrated under reduced pressure and purified by column chromatography (ethyl acetate:petroleum ether = 1:100 to 1:20) to give compound **20b.** LC-MS: m/z = 580.17 (M + H)⁺.

### Step 2:

**20b** (2.62 g) and cesium fluoride (10.33 g) were dispersed in DMF (63 mL), and the mixture was stirred for reaction at room temperature for 3 h. After the reaction was completed, water (200 mL) and ethyl acetate (200 mL) were added to the reaction solution, the mixture was stirred, and the phases were separated. The organic phase was washed with 10% brine (100 mL × 2), dried over anhydrous sodium sulfate, and filtered. The filtrate was concentrated until no liquid flowed out and purified by column chromatography (ethyl acetate:petroleum ether = 1:50 to 1:10) to give compound **20c.** LC-MS: m/z = 430.11 (M + H)⁺.

### Step 3:

**20c** (451 mg) was dispersed in dichloromethane (22.5 mL), and the mixture was cooled to 0 °C to 5 °C. *m*-Chloroperoxybenzoic acid (907 mg) was added, and the resulting mixture was stirred for 1 h with the temperature maintained. After the reaction was completed, a 10% sodium thiosulfate solution (50 mL) was added to the reaction solution, and the mixture was extracted with dichloromethane (80 mL × 2). The organic phases were combined, washed with 5% brine (100 mL), dried over anhydrous sodium sulfate, and filtered. The filtrate was concentrated until no liquid flowed out and purified by column chromatography (ethyl acetate:petroleum ether = 1:50 to 1:10) to give compound **20d.** LC-MS: m/z = 462.10 (M + H)⁺.

### Step 4:

**20d** (350 mg) and ((2*R*,7a*S*)-2-fluorotetrahydro-1*H*-pyrrolizin-7a(5*H*)-yl)methanol (604 mg) were dispersed in tetrahydrofuran (9 mL). The mixture was cooled to 0 °C, and a solution of lithium bis(trimethylsilyl)amide in tetrahydrofuran (1 N, 3 mL) was added dropwise. The resulting mixture was stirred for 30 min with the temperature maintained. After the reaction was completed, a saturated ammonium chloride solution (100 mL) was added to the reaction system, and the mixture was extracted with ethyl acetate (100 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:100 to 1:30) to give compound **20e.** LC-MS: m/z = 541.21 (M + H)⁺.

### Step 5:

**20e** (170 mg), 10g (322 mg), methanesulfonato(2-dicyclohexylphosphino-2',4',6'-tri-i-propyl-1,1'-biphenyl)(2'-methylamino-1,1'-biphenyl-2-yl) palladium(II) (50 mg), potassium phosphate (200 mg), and water (1.4 mL) were dispersed in 1,4-dioxane (6.8 mL), and under a nitrogen atmosphere, the mixture was heated to 90 °C and stirred for reaction for 5 h. The reaction solution was cooled to room temperature and filtered, and the filtrate was concentrated to dryness under reduced pressure and purified by column chromatography (methanol:dichloromethane = 1:100 to 1:30) to give compound **20f.** LC-MS: m/z = 891.44 (M + H)⁺.

### Step 6:

**20f** (100 mg) and cesium fluoride (171 mg) were dispersed in DMF (2.5 mL), and the mixture was stirred for reaction at room temperature for 1 h. After the reaction was completed, water (50 mL) was added to the reaction solution, and the mixture was extracted with ethyl acetate (80 mL × 2). The organic phases were combined, washed with 5% brine (80 mL × 3), dried over anhydrous sodium sulfate, and filtered, and the filtrate was concentrated until no liquid flowed out to give compound **20g.** LC-MS: m/z = 735.31 (M + H)⁺.

### Step 7:

**20g** (100 mg) was dispersed in dichloromethane (10 mL), and the reaction system was cooled to 0 °C to 5 °C. A solution of hydrogen chloride in 1,4-dioxane (4 N, 2 mL) was added dropwise to the reaction system, and the resulting mixture reacted for 0.5 h with the temperature maintained. After the reaction was completed, the reaction solution was concentrated to dryness under reduced pressure and purified by preparative liquid phase chromatography (YMC ODS 10 µm 50 × 250 chromatographic column; acetonitrile-0.1% aqueous formic acid solution (gradient elution of 5%-35%/0-60 min) to give compound **20.** LC-MS: m/z = 591.23 (M + H)+.

### Example 21

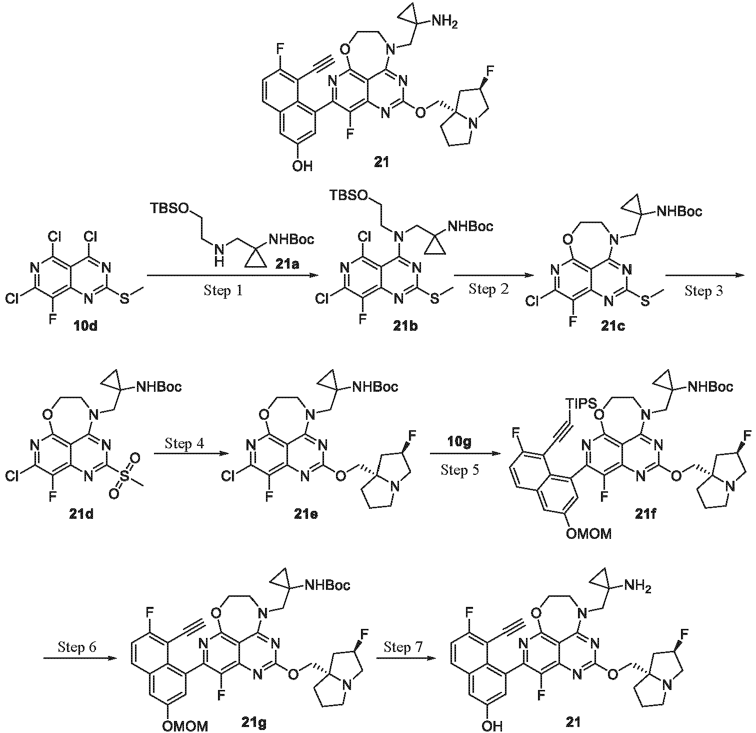

### Step 1:

**10d** (835 mg) was dispersed in tetrahydrofuran (20 mL), and DIPEA (1.08 g) and **21a** (1.45 g) were added sequentially. The mixture was stirred for reaction at room temperature for 1 h. After the reaction was completed, the reaction solution was concentrated under reduced pressure and purified by column chromatography (ethyl acetate:petroleum ether = 1:100 to 1:20) to give compound **21b.** LC-MS: m/z = 606.19 (M + H)⁺.

### Step 2:

**21b** (1.49 g) and cesium fluoride (3.75 g) were dispersed in DMF (50 mL), and the mixture was stirred for reaction at room temperature for 3 h. After the reaction was completed, water (100 mL) and ethyl acetate (200 mL) were added to the reaction solution, the mixture was stirred, and the phases were separated. The organic phase was washed with 10% brine (100 mL × 2), dried over anhydrous sodium sulfate, and filtered. The filtrate was concentrated until no liquid flowed out and purified by column chromatography (ethyl acetate:petroleum ether = 1:50 to 1:10) to give compound **21c.** LC-MS: m/z = 456.13 (M + H)⁺.

### Step 3:

**21c** (848 mg) was dispersed in dichloromethane (45 mL), and the mixture was cooled to 0 °C to 5 °C. *m*-Chloroperoxybenzoic acid (1.89 g) was added, and the resulting mixture was stirred for 1 h with the temperature maintained. After the reaction was completed, a 10% sodium thiosulfate solution (50 mL) was added to the reaction solution, and the mixture was extracted with dichloromethane (80 mL × 2). The organic phases were combined, washed with 5% brine (100 mL), dried over anhydrous sodium sulfate, and filtered. The filtrate was concentrated until no liquid flowed out to give compound **21d.** LC-MS: m/z = 488.12 (M + H)⁺.

### Step 4:

**21d** (670 mg) and ((2*R*,7a*S*)-2-fluorotetrahydro-1*H*-pyrrolizin-7a(5*H*)-yl)methanol (1093 mg) were dispersed in tetrahydrofuran (20 mL). The mixture was cooled to 0 °C, and a solution of lithium bis(trimethylsilyl)amide in tetrahydrofuran (1 N, 5.5 mL) was added dropwise. The resulting mixture was stirred for 30 min with the temperature maintained. After the reaction was completed, a saturated ammonium chloride solution (100 mL) was added to the reaction system, and the mixture was extracted with ethyl acetate (100 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:100 to 1:30) to give compound **21e.** LC-MS: m/z = 567.23 (M + H)⁺.

### Step 5:

**21e** (700 mg), **10g** (2214 mg), chloro[4-(di-tert-butylphosphino)-N,N-dimethylaniline-2-(2'-aminobiphenyl)]palladium(II) (142 mg), potassium phosphate (786 mg), and water (5.6 mL) were dispersed in 1,4-dioxane (28 mL), and under a nitrogen atmosphere, the mixture was heated to 90 °C and stirred for reaction for 5 h. The reaction solution was cooled to room temperature and filtered, and the filtrate was concentrated to dryness under reduced pressure and purified by column chromatography (methanol:dichloromethane = 1:100 to 1:30) to give compound **21f.** LC-MS: m/z = 917.46 (M + H)⁺.

### Step 6:

**21f** (892 mg) and cesium fluoride (1.5 g) were dispersed in DMF (25 mL), and the mixture was stirred for reaction at room temperature for 1 h. After the reaction was completed, water (50 mL) was added to the reaction solution, and the mixture was extracted with ethyl acetate (80 mL × 2). The organic phases were combined, washed with 5% brine (80 mL × 3), dried over anhydrous sodium sulfate, and filtered, and the filtrate was concentrated until no liquid flowed out to give compound **21g.** LC-MS: m/z = 761.33 (M + H)⁺.

### Step 7:

**21g** (100 mg) was dispersed in dichloromethane (10 mL), and the reaction system was cooled to 0 °C to 5 °C. A solution of hydrogen chloride in 1,4-dioxane (4 N, 2 mL) was added dropwise to the reaction system, and the resulting mixture reacted for 0.5 h with the temperature maintained. After the reaction was completed, the reaction solution was concentrated to dryness under reduced pressure and purified by preparative liquid phase chromatography (YMC ODS 10 µm 50 × 250 chromatographic column; acetonitrile-0.1% aqueous formic acid solution (gradient elution of 5%-35%/0-60 min) to give compound **21.** LC-MS: m/z = 617.25 (M + H)+.

### Example 22

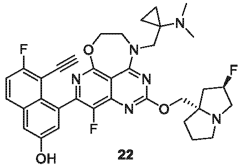

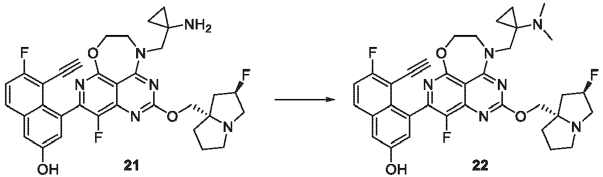

**21** (324 mg) was dispersed in methanol (15 mL), and an aqueous formaldehyde solution (1.58 g, 40%) and sodium triacetoxyborohydride (446 mg) were added sequentially. The mixture was stirred for reaction at room temperature for 1 h. After the reaction was completed, the reaction solution was concentrated to dryness under reduced pressure and purified by preparative liquid phase chromatography (YMC ODS 10 µm 50 × 250 chromatographic column; acetonitrile-0.1% ammonium hydroxide solution (gradient elution of 20%-60%/0-60 min) to give compound **22.** LC-MS: m/z = 645.28 (M + H)⁺.

### Example 23

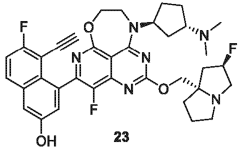

Referring to the preparation of compound **22** in Example 22, compound **23** was prepared by replacing compound **21** with compound **12.** LC-MS: m/z = 659.39 (M + H)⁺.

### Example 24

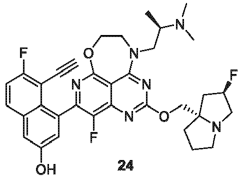

Referring to the preparation of compound **22** in Example 22, compound **24** was prepared by replacing compound **21** with compound **13.** LC-MS: m/z = 633.36 (M + H)⁺.

### Example 25

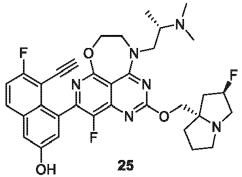

Referring to the preparation of compound **22** in Example 22, compound **25** was prepared by replacing compound **21** with compound **14.** LC-MS: m/z = 633.36 (M + H)⁺.

### Example 26

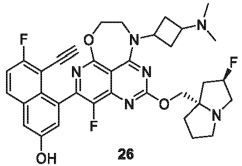

Referring to the preparation of compound **22** in Example 22, compound **26** was prepared by replacing compound **21** with compound **17.** LC-MS: m/z = 645.32 (M + H)⁺.

### Example 27

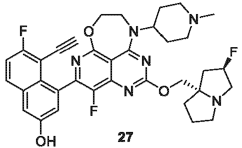

Referring to the preparation of compound **22** in Example 22, compound **27** was prepared by replacing compound **21** with compound **18.** LC-MS: m/z = 645.27 (M + H)⁺.

### Example 28

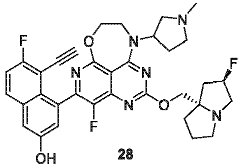

Referring to the preparation of compound **22** in Example 22, compound **28** was prepared by replacing compound **21** with compound **19.** LC-MS: m/z = 631.30 (M + H)⁺.

### Example 29

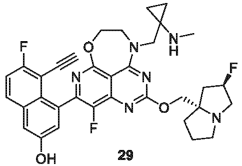

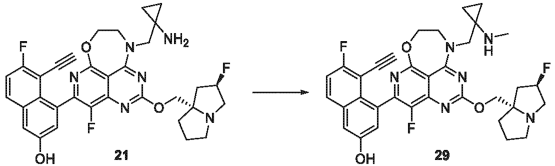

**21** (455 mg) was dispersed in hexafluoroisopropanol (4.5 mL), and iodomethane (105 mg) was slowly added dropwise. The mixture was stirred for reaction at room temperature for 18 h. After the reaction was completed, the reaction solution was purified by preparative liquid phase chromatography (YMC ODS 10 µm 50 × 250 chromatographic column; acetonitrile-30 mM aqueous ammonium acetate solution (gradient elution of 10%-50%/0-60 min) and then desalted (YMC ODS 10 µm 50 × 250 chromatographic column; acetonitrile-0.1% formic acid water (gradient elution of 10%-10%-20%-90%/0-15-16-60 min)) and purified to give compound 29.

LC-MS: m/z = 631.26 (M + H)⁺.

### Example 30

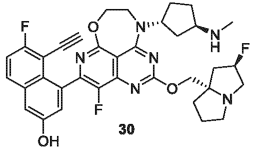

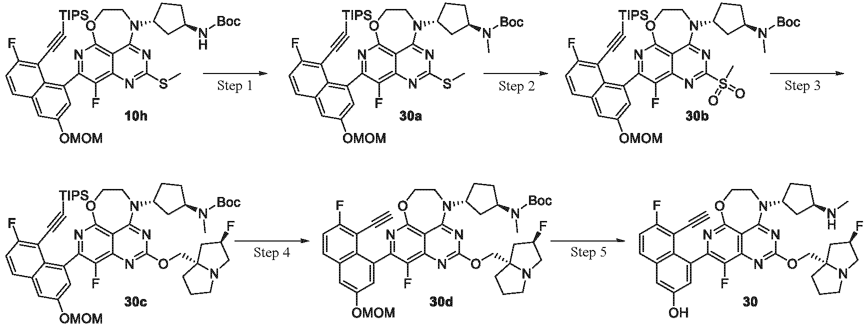

### Step 1:

**10h** (200 mg) was dispersed in DMF (5 mL), and the mixture was cooled to 0 °C to 5 °C. NaH (50 mg, 60%) and iodomethane (350 mg) were added, and the resulting mixture was stirred for reaction for 4 h with the temperature maintained. After the reaction was completed, a saturated ammonium chloride solution (30 mL) was added to the reaction system, and the mixture was extracted with ethyl acetate (50 mL × 2). The organic phases were combined, washed with 5% brine (100 mL), dried over anhydrous sodium sulfate, and filtered. The filtrate was concentrated until no liquid flowed out and purified by column chromatography (methanol:dichloromethane = 1:500 to 1:100) to give compound **30a.** LC-MS: m/z = 834.55 (M + H)⁺.

### Step 2:

**30a** (200 mg) was dispersed in dichloromethane (2 mL), and the mixture was cooled to 0 °C to 5 °C. *m*-Chloroperoxybenzoic acid (103 mg) was added, and the resulting mixture was stirred for 1 h with the temperature maintained. After the reaction was completed, a 10% sodium thiosulfate solution (50 mL) was added to the reaction solution, and the mixture was extracted with dichloromethane (80 mL × 2). The organic phases were combined, washed with 5% brine (100 mL), dried over anhydrous sodium sulfate, and filtered. The filtrate was concentrated until no liquid flowed out to give compound **30b.** LC-MS: m/z = 866.52 (M + H)⁺.

### Step 3:

**30b** (200 mg) and ((2*R*,7a*S*)-2-fluorotetrahydro-1*H*-pyrrolizin-7a(5*H*)-yl)methanol (112 mg) were dispersed in tetrahydrofuran (5 mL). The mixture was cooled to 0 °C, and a solution of lithium bis(trimethylsilyl)amide in tetrahydrofuran (1 N, 0.6 mL) was added dropwise. The resulting mixture was allowed to be stirred for reaction at room temperature overnight. After the reaction was completed, a saturated ammonium chloride solution (100 mL) was added to the reaction system, and the mixture was extracted with ethyl acetate (100 mL × 2). The organic phases were combined, washed with 5% brine (100 mL), dried over anhydrous sodium sulfate, and filtered. The filtrate was concentrated until no liquid flowed out to give compound **30c.** LC-MS: m/z = 945.62 (M + H)⁺.

### Step 4:

**30c** (390 mg) and cesium fluoride (1.25 g) were dispersed in DMF (15 mL), and the mixture was stirred for reaction at room temperature for 2 h. After the reaction was completed, water (50 mL) was added to the reaction solution, and the mixture was extracted with ethyl acetate (80 mL × 2). The organic phases were combined, washed with 5% brine (80 mL × 3), dried over anhydrous sodium sulfate, and filtered, and the filtrate was concentrated until no liquid flowed out to give compound **30d.** LC-MS: m/z = 789.40 (M + H)⁺.

### Step 5:

**30d** (250 mg) was dispersed in dichloromethane (2.5 mL), and the reaction system was cooled to 0 °C to 5 °C. A solution of hydrogen chloride in 1,4-dioxane (4 N, 2.5 mL) was added dropwise to the reaction system, and the resulting mixture reacted for 0.5 h with the temperature maintained. After the reaction was completed, the reaction solution was concentrated to dryness under reduced pressure and purified by preparative liquid phase chromatography (YMC AQ C18 50 × 250 chromatographic column; acetonitrile-0.05% acetic acid (gradient elution of 10%-70%/0-60 min) to give compound **30.** LC-MS: m/z = 645.32 (M + H)⁺.

### Example 31

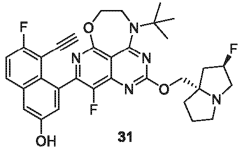

Referring to the preparation method of compound **11** in Example 11, compound **31** was prepared by replacing **11a** with tert-butylamine in step 1. LC-MS: m/z = 604.30 (M + H)⁺.

¹H-NMR (500 MHz, DMSO-*d₆*), *δ*: 7.96 (dd, *J = 9.2* Hz, *J* = 6.0 Hz, 1H), 7.46 (t, *J* = 9.0 Hz, 1H), 7.37 (d, *J =* 2.4 Hz, 1H), 7.15 (d, *J* = 2.4 Hz, 1H), 5.30 (d, *J* = 54.0 Hz, 1H), 4.61-4.50 (m, 2H), 4.17 (d, *J* = 3.3 Hz, 1H), 4.16-4.06 (m, 2H), 4.01-3.95 (m, 1H), 3.75-3.68 (m, 1H), 3.16-3.07 (m, 3H), 2.89-2.84 (m, 1H), 2.17-1.99 (m, 3H), 1.90-1.76(m, 3H), 1.65(s, 9H).

### Example 32 and Example 33

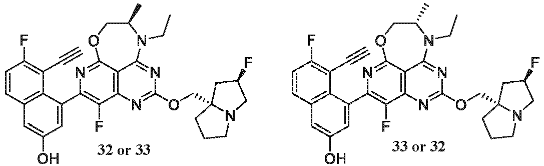

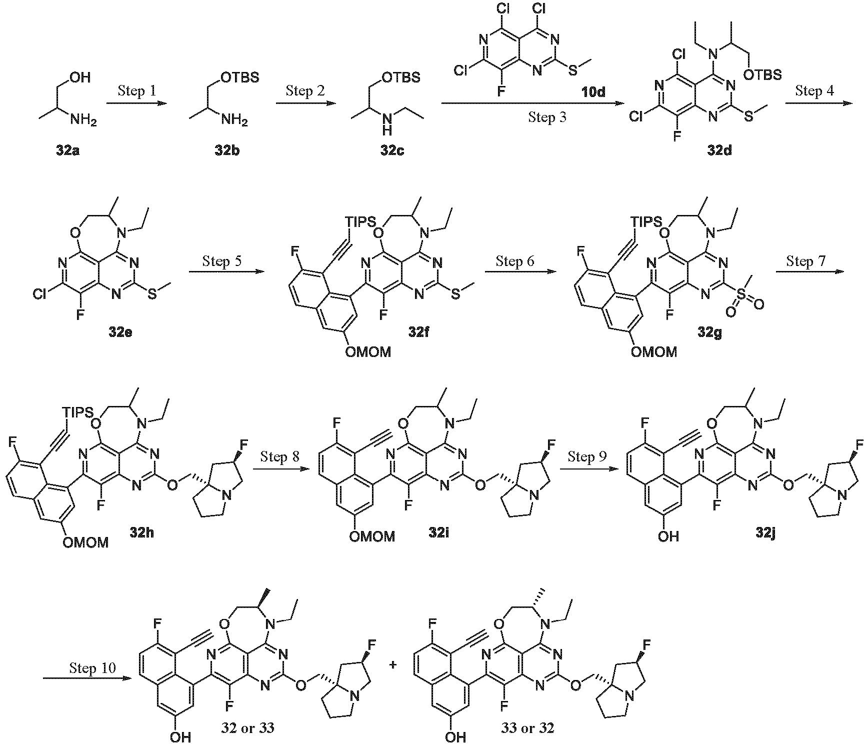

### Step 1:

**32a** (2 g) and imidazole (5.4 g) were dispersed in DMF (40 mL), and the mixture was stirred at room temperature for 30 min. *tert*-Butyldimethylsilyl chloride (6 g) was added, and the resulting mixture was stirred for reaction for another 12 h. Water (100 mL) and ethyl acetate (150 mL) were added to the reaction solution, the mixture was stirred, and the phases were separated. The aqueous phase was extracted with ethyl acetate (100 mL × 2), and the organic phases were combined, washed with 5% brine (100 mL × 2), dried over anhydrous sodium sulfate, and filtered. The filtrate was concentrated until no liquid flowed out to give compound **32b.** LC-MS: m/z = 190.16 (M + H)⁺.

### Step 2:

**32b** (2 g) was dispersed in DMF (200 mL). At room temperature, iodoethane (1.47 g) was added dropwise, and the mixture was stirred for reaction for 12 h. Water (200 mL) and ethyl acetate (250 mL) were added to the reaction solution, the mixture was stirred, and the phases were separated. The aqueous phase was extracted with ethyl acetate (100 mL × 2), and the organic phases were combined, washed with 5% brine (150 mL × 2), dried over anhydrous sodium sulfate, and filtered. The filtrate was concentrated until no liquid flowed out to give compound **32c.** LC-MS: m/z = 218.19 (M + H)⁺.

### Step 3:

**10d** (530 mg) was dispersed in tetrahydrofuran (20 mL), and DIPEA (923 mg) and **32c** (774 mg) were slowly added sequentially. The mixture was stirred for reaction at room temperature for 1 h. After the reaction was completed, the reaction solution was concentrated under reduced pressure and purified by column chromatography (ethyl acetate:petroleum ether = 1:100 to 1:20) to give compound **32d.** LC-MS: m/z = 479.13 (M + H)⁺.

### Step 4:

**32d** (532 mg) and cesium fluoride (2029 mg) were dispersed in DMF (22 mL), and the mixture was heated to 80 °C and stirred for reaction for 3 h. After the reaction was completed, water (100 mL) and ethyl acetate (100 mL) were added to the reaction solution, the mixture was stirred, and the phases were separated. The organic phase was washed with 10% brine (80 mL × 2), dried over anhydrous sodium sulfate, and filtered. The filtrate was concentrated until no liquid flowed out and purified by column chromatography (ethyl acetate:petroleum ether = 1:50 to 1:5) to give compound **32e.** LC-MS: m/z = 329.06 (M + H)⁺.

### Step 5:

**32e** (172 mg), **10g** (938 mg), chloro[4-(di-tert-butylphosphino)-N,N-dimethylaniline-2-(2'-aminobiphenyl)]palladium(II) (60.2 mg), potassium phosphate (333 mg), and water (1.72 mL) were dispersed in 1,4-dioxane (8.6 mL), and under a nitrogen atmosphere, the mixture was heated to 90 °C and stirred for reaction for 5 h. The reaction solution was cooled to room temperature and filtered, and the filtrate was concentrated to dryness under reduced pressure and purified by column chromatography (methanol:dichloromethane = 1:100 to 1:30) to give compound **32f.** LC-MS: m/z = 679.29 (M + H)⁺.

### Step 6:

**32f** (275 mg) was dispersed in dichloromethane (15 mL), and the mixture was cooled to 0 °C to 5 °C. *m*-Chloroperoxybenzoic acid (85%, 412 mg) was added, and the resulting mixture was stirred for 1 h with the temperature maintained. After the reaction was completed, a 10% sodium thiosulfate solution (100 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out to give compound **32g.** LC-MS: m/z = 711.28 (M + H)⁺.

### Step 7:

**32g** (245 mg) and ((2*R*,7a*S*)-2-fluorotetrahydro-1*H*-pyrrolizin-7a(5*H*)-yl)methanol (275 mg) were dispersed in tetrahydrofuran (8 mL). The mixture was cooled to 0 °C, and a solution of lithium bis(trimethylsilyl)amide in tetrahydrofuran (1 N, 1.4 mL) was added dropwise. The resulting mixture was stirred for 30 min with the temperature maintained. After the reaction was completed, a saturated ammonium chloride solution (50 mL) was added to the reaction system, and the mixture was extracted with ethyl acetate (80 mL × 2). The organic phases were combined, washed with 5% brine (80 mL), dried over anhydrous sodium sulfate, and filtered. The filtrate was concentrated until no liquid flowed out to give compound **32h.** LC-MS: m/z = 790.40 (M + H)⁺.

### Step 8:

**32h** (285 mg) and cesium fluoride (550 mg) were dispersed in DMF (10 mL), and the mixture was stirred for reaction at room temperature for 2 h. Water (50 mL) and ethyl acetate (80 mL) were added to the reaction solution, the mixture was stirred, and the phases were separated. The aqueous phase was extracted with ethyl acetate (50 mL × 2), and the organic phases were combined, washed with 5% brine (100 mL × 2), dried over anhydrous sodium sulfate, and filtered. The filtrate was concentrated until no liquid flowed out to give compound **32i.** LC-MS: m/z = 634.26 (M + H)⁺.

### Step 9:

**32i** (280 mg) was dispersed in dichloromethane (20 mL), and the reaction system was cooled to 0 °C to 5 °C. A solution of hydrogen chloride in 1,4-dioxane (4 N, 4 mL) was added dropwise to the reaction system, and the resulting mixture reacted for 1 h with the temperature maintained. After the reaction was completed, the reaction solution was concentrated to dryness under reduced pressure and purified by preparative liquid phase chromatography (YMC ODS 10 µm 50 × 250 chromatographic column; acetonitrile-0.1% FA (gradient elution of 10%-40%/0-60 min) to give compound **32j.** LC-MS: m/z = 590.24 (M + H)⁺.

### Step 10:

Compound **32j** was sequentially purified by preparative liquid phase chromatography (YMC Cellulose-SC 10 µm 30 × 250 chromatographic column; ethanol-n-hexane (gradient elution of 40%-100%/0-30 min) to give compound **32** (Rt 13.1 min, 61 mg) and compound **33** (Rt 15.6 min, 58 mg).

**Compound 32:** LC-MS: m/z = 590.24 (M + H)⁺.

¹H-NMR (500 MHz, DMSO-*d₆*), *δ*: 7.96-7.95 (m, 1H), 7.48-7.43 (m, 1H), 7.37 (d, *J* = 2.2 Hz, 1H), 7.20-7.11 (m, 1H), 5.28 (d, *J* = 53.6 Hz, 1H), 4.58-4.51 (m, 1H), 4.35-4.31 (m, 1H), 4.19-4.00 (m, 5H), 3.55-3.47 (m, 1H), 3.12-3.02 (m, 3H), 2.86-2.81 (m, 1H), 2.14-1.97 (m, 3H), 1.88-1.73(m, 3H), 1.31-1.27 (m, 6H).

**Compound 33:** LC-MS: m/z = 590.24 (M + H)⁺.

¹H-NMR (500 MHz, DMSO-*d₆*), *δ*: 10.11 (s, 1H), 7.95 (dd, *J* = 9.0 Hz, *J* = 6.0 Hz, 1H), 7.48-7.43 (m, 1H), 7.37-7.36 (m, 1H), 7.20-7.11 (m, 1H), 5.28 (d, *J* = 53.8 Hz, 1H), 4.58-4.51 (m, 1H), 4.34 (d, *J* = 12.9 Hz, 1H), 4.19-4.00 (m, 5H), 3.55-3.47 (m, 1H), 3.13-3.02 (m, 3H), 2.87-2.81 (m, 1H), 2.14-1.96 (m, 3H), 1.88-1.73(m, 3H), 1.31-1.26 (m, 6H).

The retention time in chiral chromatographic column of compound 32 is short compared to that of compound 33, and the retention time in chiral chromatographic column of compound 33 is long compared to that of compound 32.

### Example 34

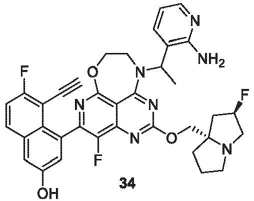

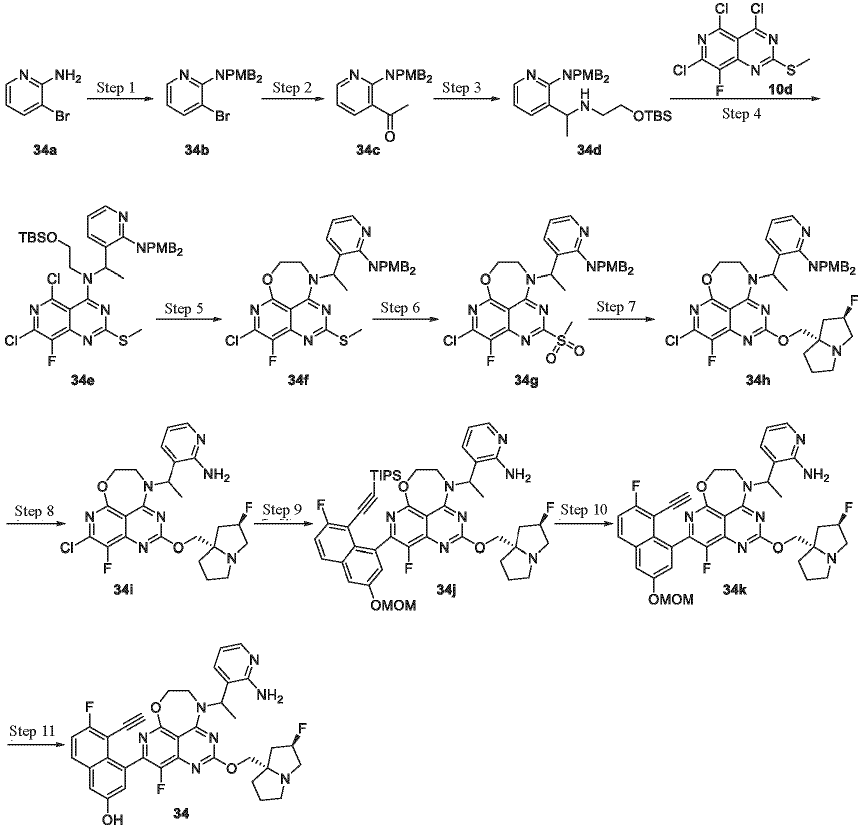

### Step 1:

**34a** (7.25 g) and *p*-methoxybenzyl chloride (26.3 g) were dispersed in DMF (80 mL), and sodium hydride (60%, 5.9 g) was added under stirring at room temperature. The mixture was stirred for reaction for 2 h. After the reaction was completed, an ammonium chloride solution (20 mL) was added to quench the reaction, and water (100 mL) and ethyl acetate (150 mL) were added. The mixture was stirred, and the phases were separated. The aqueous phase was extracted with ethyl acetate (100 mL × 2), and the organic phases were combined, washed with 5% brine (100 mL × 2), dried over anhydrous sodium sulfate, and filtered. The filtrate was concentrated until no liquid flowed out and purified by column chromatography (ethyl acetate:petroleum ether = 1:100 to 1:10) to give compound **34b.** LC-MS: m/z = 413.16 (M + H)⁺.

### Step 2:

**34b** (1.25 g), bis(diphenylphosphino)palladium(II) dichloride (0.21 g), and tributyl(1-ethoxyvinyl)tin (3.1 mL) were dispersed in DMF (10 mL), and under a nitrogen atmosphere, the mixture was heated to 80 °C and stirred for reaction for 8 h. After the reaction was completed, the reaction solution was cooled to room temperature, and the pH was adjusted to acidity with diluted hydrochloric acid. Water (100 mL) and ethyl acetate (100 mL) were added, the mixture was stirred, and the phases were separated. The aqueous phase was extracted with ethyl acetate (100 mL × 2), and the organic phases were combined, washed once with a 10% sodium bicarbonate solution (100 mL) and once with 5% brine (100 mL), dried over anhydrous sodium sulfate, and filtered. The filtrate was concentrated until no liquid flowed out and purified by column chromatography (ethyl acetate:petroleum ether = 1:50 to 1:5) to give compound **34c.** LC-MS: m/z = 377.26 (M + H)⁺.

### Step 3:

**34c** (0.7 g), 2-(*tert*-butyldimethylsilyloxy)ethanamine (1.12 g), and titanium tetraisopropanolate (1.2 mL) were dispersed in methanol (14 mL), and the mixture was heated to 80 °C and stirred for reaction for 6 h. The reaction solution was cooled to 0 °C, and sodium borohydride (240 mg) was added. The resulting mixture was allowed to be stirred for reaction at room temperature for 4 h. After the reaction was completed, the reaction solution was filtered by diatomite, and the filtrate was concentrated until no liquid flowed out and purified by column chromatography (ethyl acetate:petroleum ether = 1:20 to 1:2) to give compound **34d.** LC-MS: m/z = 536.39 (M + H)⁺.

### Step 4:

**10d** (800 mg), **34d** (980 mg), and DIPEA (580 mg) were dispersed in tetrahydrofuran (20 mL), and the mixture was stirred for reaction at room temperature for 1.5 h. After the reaction was completed, the reaction solution was concentrated under reduced pressure and purified by column chromatography (ethyl acetate:petroleum ether = 1:100 to 1:10) to give compound **34e.** LC-MS: m/z = 797.65 (M + H)⁺.

### Step 5:

**34e** (809 mg) and cesium fluoride (1.6 g) were dispersed in DMF (16 mL), and the mixture was heated to 60 °C and stirred for reaction for 2 h. After the reaction was completed, water (100 mL) and ethyl acetate (100 mL) were added to the reaction solution, the mixture was stirred, and the phases were separated. The aqueous phase was extracted with ethyl acetate (100 mL × 2), and the organic phase was washed with 10% brine (100 mL × 2), dried over anhydrous sodium sulfate, and filtered. The filtrate was concentrated until no liquid flowed out to give compound **34f.** LC-MS: m/z = 647.28 (M + H)⁺.

### Step 6:

**34f** (650 mg) was dispersed in dichloromethane (30 mL), and the mixture was cooled to 0 °C to 5 °C. *m*-Chloroperoxybenzoic acid (85%, 860 mg) was added, and the resulting mixture was stirred for 1 h with the temperature maintained. After the reaction was completed, a 10% sodium thiosulfate solution (100 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out to give compound **34g.** LC-MS: m/z = 679.27 (M + H)⁺.

### Step 7:

**34g** (725 mg) and ((2*R*,7a*S*)-2-fluorotetrahydro-1*H*-pyrrolizin-7a(5*H*)-yl)methanol (850 mg) were dispersed in tetrahydrofuran (15 mL). The mixture was cooled to 0 °C, and a solution of lithium bis(trimethylsilyl)amide in tetrahydrofuran (1 N, 4.3 mL) was added dropwise. The resulting mixture was stirred for 1 h with the temperature maintained. After the reaction was completed, a saturated ammonium chloride solution (20 mL) was added to the reaction system to quench the reaction, and water (50 mL) and ethyl acetate (80 mL) were added. The mixture was stirred, and the phases were separated. Extraction was performed with ethyl acetate (80 mL × 2), and the organic phases were combined, washed with 5% brine (80 mL), dried over anhydrous sodium sulfate, and filtered. The filtrate was concentrated until no liquid flowed out and purified by column chromatography (methanol:dichloromethane = 1:100 to 1:20) to give compound **34h.** LC-MS: m/z = 758.33 (M + H)⁺.

### Step 8:

**34h** (200 mg) was dispersed in trifluoroacetic acid (10 mL), and the mixture was cooled to 0 °C. Methanesulfonic acid (1 mL) was added dropwise, and the resulting mixture was allowed to be stirred for reaction at room temperature for 2 h. After the reaction was completed, the reaction solution was concentrated under reduced pressure until no liquid flowed out. The residue was dissolved in dichloromethane (10 mL), and the pH of the system was adjusted to alkalinity with a saturated sodium bicarbonate solution under an ice bath. Water (50 mL) and dichloromethane (50 mL) were added, the mixture was stirred, and the phases were separated. Extraction was performed with dichloromethane (50 mL × 2), and the organic phases were combined, washed with 5% brine (100 mL), dried over anhydrous sodium sulfate, and filtered. The filtrate was concentrated until no liquid flowed out to give compound **34i.** LC-MS: m/z = 518.21 (M + H)⁺.

### Step 9:

**34i** (166 mg), **10g** (327 mg), chloro[4-(di-tert-butylphosphino)-N,N-dimethylaniline-2-(2'-aminobiphenyl)]palladium(II) (37 mg), potassium phosphate (198 mg), and water (2.4 mL) were dispersed in 1,4-dioxane (12 mL), and under a nitrogen atmosphere, the mixture was heated to 90 °C and stirred for reaction for 1 h. The reaction solution was cooled to room temperature and filtered, and the filtrate was concentrated to dryness under reduced pressure and purified by column chromatography (methanol:dichloromethane = 1:100 to 1:15) to give compound **34j.** LC-MS: m/z = 868.91 (M + H)⁺.

### Step 10:

**34j** (175 mg) and cesium fluoride (306 mg) were dispersed in DMF (10 mL), and the mixture was stirred for reaction at room temperature for 2 h. Water (50 mL) and ethyl acetate (80 mL) were added to the reaction solution, the mixture was stirred, and the phases were separated. The aqueous phase was extracted with ethyl acetate (50 mL × 2), and the organic phases were combined, washed with 5% brine (80 mL × 3), dried over anhydrous sodium sulfate, and filtered. The filtrate was concentrated until no liquid flowed out to give compound **34k.** LC-MS: m/z = 712.50 (M + H)⁺.

### Step 11:

**34k** (168 mg) was dispersed in dichloromethane (10 mL), and the reaction system was cooled to 0 °C to 5 °C. A solution of hydrogen chloride in 1,4-dioxane (4 N, 2 mL) was added dropwise to the reaction system, and the resulting mixture was stirred for reaction for 1 h with the temperature maintained. After the reaction was completed, the reaction solution was concentrated to dryness under reduced pressure and purified by preparative liquid phase chromatography (YMC ODS 10 µm 30 × 250 chromatographic column; acetonitrile-0.1% FA (gradient elution of 5%-5%/0-60 min)) to give compound **34.** LC-MS: m/z = 668.42 (M + H)⁺.

### Example 35 and Example 36

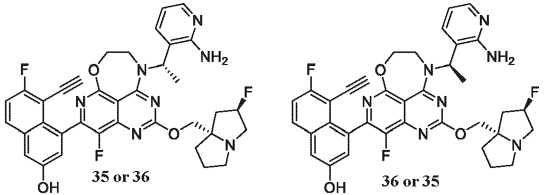

Compound **34** was sequentially purified by preparative liquid phase chromatography (YMC Cellulose-SC 10 µm 30 × 250 chromatographic column; ethanol-*n*-hexane (gradient elution of 30%-100%/0-40 min)) to give compound **35** (Rt 16.2 min, 44 mg) and compound **36** (Rt 22.3 min, 57 mg).

**Compound 35:** LC-MS: m/z = 668.40 (M + H)⁺.

¹H-NMR (500 MHz, DMSO-*d₆*), *δ*: 10.15 (br, 1H), 7.99-7.94 (m, 2H), 7.65 (d, *J =* 6.2 Hz, 1H), 7.45 (t, *J* = 8.6 Hz, 1H), 7.37 (s, 1H), 7.14 (s, 1H), 6.68 (s, 1H), 6.34 (dd, *J* = 29.2 Hz, *J =* 5.6 Hz, 1H), 5.79 (s, 1H), 5.72 (s, 1H), 5.30 (d, *J =* 54.6 Hz, 1H), 4.47-4.16 (m, 3H), 4.11-3.96 (m, 2H), 3.80-3.65 (m, 1H), 3.47-3.41 (m, 1H), 3.14-3.03 (m, 3H), 2.86-2.81 (m, 1H), 2.18-1.99 (m, 3H), 1.88-1.75(m, 3H), 1.62-1.58 (m, 3H).

**Compound 36:** LC-MS: m/z = 668.41 (M + H)⁺.

¹H-NMR (500 MHz, DMSO-*d₆*), *δ*: 10.15 (br, 1H), 7.99-7.94 (m, 2H), 7.65 (d, *J* = 7.4 Hz, 1H), 7.46 (t, *J* = 8.6 Hz, 1H), 7.37 (s, 1H), 7.15 (s, 1H), 6.68 (t, *J* = 5.6 Hz, 1H), 6.39-6.29 (m, 1H), 5.80 (s, 1H), 5.72 (s, 1H), 5.30 (d, *J = 53.8* Hz, 1H), 4.46-4.21 (m, 2H), 4.16-3.97 (m, 3H), 3.80-3.65 (m, 1H), 3.47-3.42 (m, 1H), 3.16-3.05 (m, 3H), 2.88-2.82 (m, 1H), 2.20-2.02 (m, 3H), 1.91-1.78(m, 3H), 1.60 (t, *J* = 6.2 Hz, 3H).

The retention time in chiral chromatographic column of compound 35 is short compared to that of compound 36, and the retention time in chiral chromatographic column of compound 36 is long compared to that of compound 35.

### Example 37

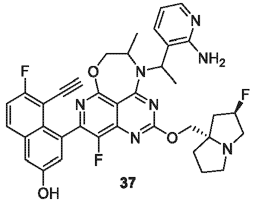

Referring to the preparation of compound **34** in Example 34, compound **37** was prepared by replacing 2-(*tert*-butyldimethylsilyloxy)ethanamine with DL-alaninol in step 3. LC-MS: m/z = 682.42 (M + H)⁺.

### Example 38

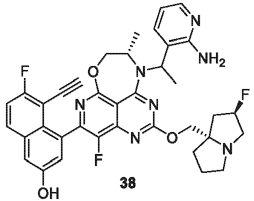

Referring to the preparation of compound **34** in Example 34, compound **38** was prepared by replacing 2-(*tert*-butyldimethylsilyloxy)ethanamine with L-alaninol in step 3. LC-MS: m/z = 682.42 (M + H)⁺.

### Example 39 and Example 40

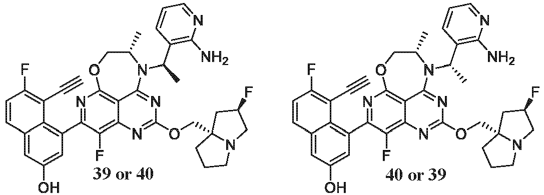

Compound **38** was sequentially purified by liquid phase chromatography (CHIRALPAK ID 5.0 µm 4.6 × 250 mm; *n*-hexane-0.1% ethanolamine/ethanol (50%/isocratic elution for 20 min)) to give compound **39** (Rt 11.6 min, LC-MS: m/z = 682.42 (M + H)⁺) and compound **40** (Rt 14.9 min, LC-MS: m/z = 682.42 (M + H)⁺).

**Compound 39:** ¹H-NMR (500 MHz, DMSO-*d₆*), *δ*: 10.30-9.97 (m, 1H), 8.15-7.85 (m, 2H), 7.83-7.60 (m, 1H), 7.59-7.30 (m, 2H), 7.28-7.04 (m, 1H), 6.83-6.37 (m, 2H), 5.78-5.44 (m, 2H), 5.43-5.15 (m, 1H), 4.64-4.41 (m, 1H), 4.38-3.72 (m, 5H), 3.21-2.94 (m, 3H), 2.93-2.76 (m, 1H), 2.27-1.96 (m, 3H), 1.95-1.71 (m, 3H), 1.70-1.52 (m, 3H), 0.74-0.44 (m, 3H).

**Compound 40:** ¹H-NMR (500 MHz, DMSO-*d₆*), *δ*: 10.30-9.97 (m, 1H), 8.10-7.86 (m, 2H), 7.73 (d, *J* = 7.2 Hz, 1H), 7.56-7.42 (m, 1H), 7.41-7.30 (m, 1H), 7.25-7.08 (m, 1H), 6.82-6.62 (m, 1H), 6.60-6.36 (m, 1H), 5.82-5.53 (m, 2H), 5.43-5.15 (m, 1H), 4.46-4.26 (m, 1H), 4.25-4.15 (m, 1H), 4.14-4.00 (m, 2H), 3.98-3.79 (m, 2H), 3.21-2.98 (m, 3H), 2.92-2.76 (m, 1H), 2.23-1.95 (m, 3H), 1.93-1.73 (m, 3H), 1.72-1.58 (m, 3H), 1.28-1.17 (m, 3H).

The retention time in chiral chromatographic column of compound 39 is short compared to that of compound 40, and the retention time in chiral chromatographic column of compound 40 is long compared to that of compound 39.

### Example 41

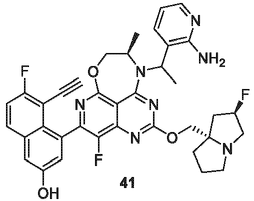

Referring to the preparation of compound **34** in Example 34, compound **41** was prepared by replacing 2-(*tert-*butyldimethylsilyloxy)ethanamine with D-alaninol in step 3. LC-MS: m/z = 682.42 (M + H)⁺.

### Example 42 and Example 43

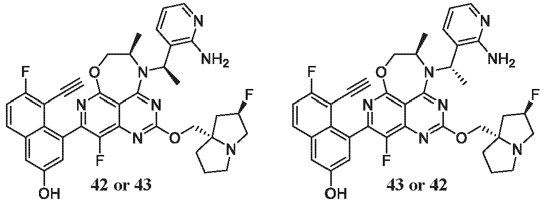

Compound **41** was sequentially purified by liquid phase chromatography (CHIRALPAK ID 5.0 µm 4.6 × 250 mm; *n*-hexane-0.1% ethanolamine/ethanol (50%/isocratic elution for 20 min)) to give compound **42** (Rt 13.7 min, LC-MS: m/z = 682.42 (M + H)⁺) and compound **43** (Rt 16.6 min, LC-MS: m/z = 682.42 (M + H)⁺).

**Compound 42:** ¹H-NMR (500 MHz, DMSO-*d₆*), *δ*: 10.16(br, 1H), 8.10-7.90 (m, 2H), 7.71 (d, *J* = 7.4 Hz, 1H), 7.52-7.42 (m, 1H), 7.41-7.32 (m, 1H), 7.26-7.07 (m, 1H), 6.79-6.64 (m, 1H), 6.63-6.41 (m, 1H), 5.80-5.46 (m, 2H), 5.42-5.14 (m, 1H), 4.69-4.43 (m, 1H), 4.37-4.15 (m, 2H), 4.14-3.75 (m, 3H), 3.20-3.00 (m, 3H), 2.90-2.78 (m, 1H), 2.22-1.96 (m, 3H), 1.93-1.73 (m, 3H), 1.69-1.55 (m, 3H), 0.72-0.49 (m, 3H).

**Compound 43:** ¹H-NMR (500 MHz, DMSO-*d₆*), *δ*: 10.15(br, 1H), 8.10-7.89 (m, 2H), 7.73 (d, *J* = 7.4 Hz, 1H), 7.56-7.43 (m, 1H), 7.38 (d, *J* = 2.4 Hz, 1H), 7.26-7.10 (m, 1H), 6.78-6.63 (m, 1H), 6.61-6.38 (m, 1H), 5.79-5.56 (m, 2H), 5.42-5.18 (m, 1H), 4.44-4.26 (m, 1H), 4.21-3.79 (m, 5H), 3.20-2.99 (m, 3H), 2.90-2.77 (m, 1H), 2.22-1.97 (m, 3H), 1.94-1.73 (m, 3H), 1.72-1.57 (m, 3H), 1.27-1.17 (m, 3H).

The retention time in chiral chromatographic column of compound 42 is short compared to that of compound 43, and the retention time in chiral chromatographic column of compound 43 is long compared to that of compound **42.**

### Example 44

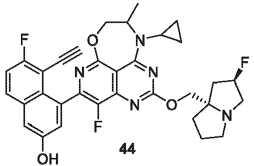

Referring to the preparation of compound **32j** in Example 32, compound **44** was prepared by replacing iodoethane with iodocyclopropane in step 2. LC-MS: m/z = 602.22 (M + H)⁺.

### Example 45 and Example 46

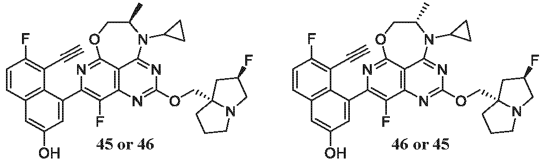

Compound 44 was sequentially purified by preparative liquid phase chromatography (YMC Cellulose-SC 10 µm 30 × 250 chromatographic column; ethanol-n-hexane (gradient elution of 20%-80%/0-40 min)) to give compound 45 (Rt 15.1 min, LC-MS: m/z = 602.22 (M + H)⁺) and compound 46 (Rt 22.6 min, LC-MS: m/z = 602.22 (M + H)⁺).

The retention time in chiral chromatographic column of compound 45 is short compared to that of compound 46, and the retention time in chiral chromatographic column of compound 46 is long compared to that of compound 45.

### Example 47 and Example 48

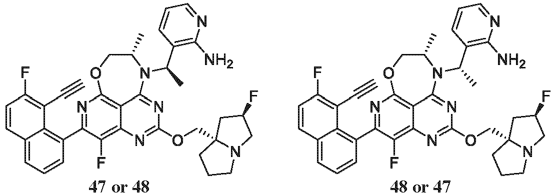

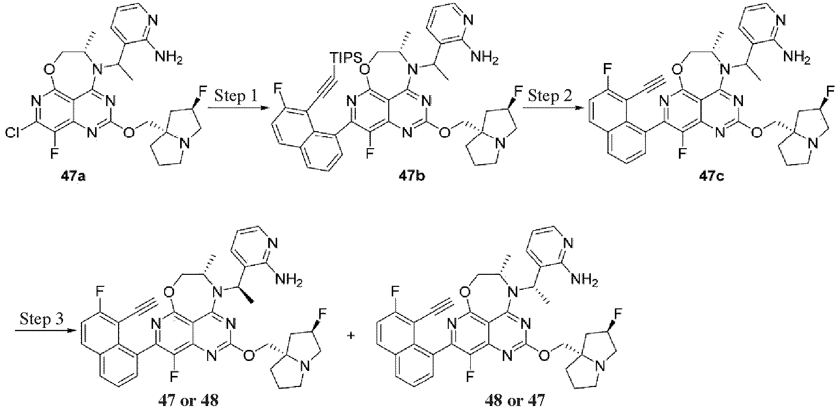

Referring to the preparation of compound **34i** in Example 34, compound **47a** was prepared by replacing 2-(*tert*-butyldimethylsilyloxy)ethanamine with L-alaninol in step 3. LC-MS: m/z = 532.20 (M + H)⁺.

### Step 1:

**47a** (160 mg), ((2-fluoro-8-(4,4,5,5-tetramethyl-1,3,2-dioxaborolan-2-yl)naphthalen-1-yl)ethynyl)triisopropylsilane (271 mg), chloro[4-(di-tert-butylphosphino)-N,N-dimethylaniline-2-(2'-aminobiphenyl)]palladium(II) (35 mg), potassium phosphate (190 mg), and water (3 mL) were dispersed in 1,4-dioxane (15 mL), and under a nitrogen atmosphere, the mixture was heated to 90 °C and stirred for reaction for 2 h. The reaction solution was cooled to room temperature and filtered, and the filtrate was concentrated to dryness under reduced pressure and purified by column chromatography (ethyl acetate:petroleum ether = 1:10 to 1:2) to give compound **47b.** LC-MS: m/z = 822.77 (M + H)⁺.

### Step 2:

**47b** (157 mg) and cesium fluoride (300 mg) were dispersed in DMF (16 mL), and the mixture was stirred for reaction at room temperature for 2 h. Water (200 mL) and ethyl acetate (200 mL) were added to the reaction solution, the mixture was stirred, and the phases were separated. The organic phase was washed with 5% brine (100 mL × 4), dried over anhydrous sodium sulfate, and filtered. The filtrate was concentrated until no liquid flowed out to give compound **47c.** LC-MS: m/z = 666.40 (M + H)⁺.

### Step 3:

Compound **47c** was sequentially purified by preparative liquid phase chromatography (YMC Cellulose-SB 10 µm 30 × 250 chromatographic column; ethanol-n-hexane (gradient elution of 10%-90%/0-40 min)) to give compound **47** (Rt 16.3 min, LC-MS: m/z = 666.40 (M + H)⁺) and compound **48** (Rt 18.4 min, LC-MS: m/z = 666.40 (M + H)⁺).

The retention time in chiral chromatographic column of compound 47 is short compared to that of compound 48, and the retention time in chiral chromatographic column of compound 48 is long compared to that of compound 47.

### Example 49

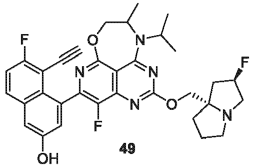

Referring to the preparation of compound **32j** in Example 32, compound **49** was prepared by replacing iodoethane with 2-iodopropane in step 2. LC-MS: m/z = 604.34 (M + H)⁺.

### Example 50

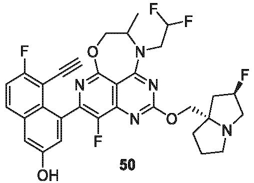

Referring to the preparation of compound 32j in Example 32, compound **50** was prepared by replacing iodoethane with 1,1-difluoro-2-iodoethane in step 2. LC-MS: m/z = 626.30 (M + H)⁺.

### Example 51

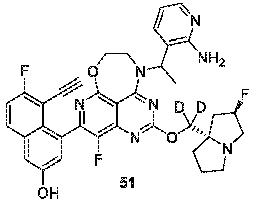

Referring to the preparation of compound **34** in Example 34, compound **51** was prepared by replacing ((2*R*,7a*S*)-2-fluorotetrahydro-1*H*-pyrrolizin-7a(5*H*)-yl)methanol with ((2*R*,7a*S*)-2-fluorotetrahydro-1*H*-pyrrolizin-7a(5*H*)-yl)methane-*d*₂-ol in step 7. LC-MS: m/z = 670.38 (M + H)⁺.

### Example 52

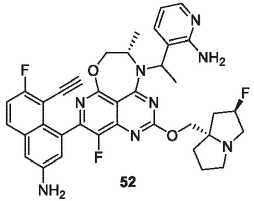

Referring to the preparation of compound **47c** in Example 47, compound **52** was prepared by replacing ((2-fluoro-8-(4,4,5,5-tetramethyl-1,3,2-dioxaborolan-2-yl)naphthalen-1-yl)ethynyl)triisopropylsilane with 6-fluoro-4-(4,4,5,5-tetramethyl-1,3,2-dioxaborolan-2-yl)-5-((triisopropylsilyl)ethynyl)naphthalen-2-amine in step 1. LC-MS: m/z = 681.32 (M + H)⁺.

### Example 53 and Example 54

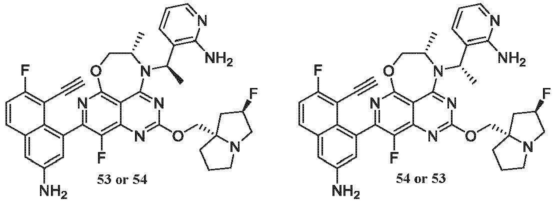

Compound **52** was sequentially purified by preparative liquid phase chromatography (YMC C18 10 µm 50 × 250 chromatographic column; acetonitrile-0.1% ammonium hydroxide (gradient elution of 60%-90%/0-60 min)) to give compound **53** (Rt 22 min, LC-MS: m/z = 681.32 (M + H)⁺) and compound **54** (Rt 25 min, LC-MS: m/z = 681.34 (M + H)⁺).

The retention time in chiral chromatographic column of compound 53 is short compared to that of compound 54, and the retention time in chiral chromatographic column of compound 54 is long compared to that of compound 53.

### Example 55 and Example 56

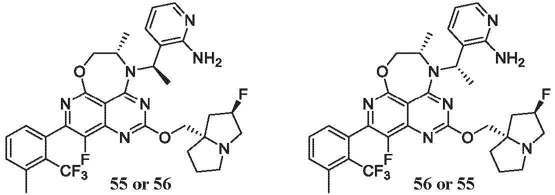

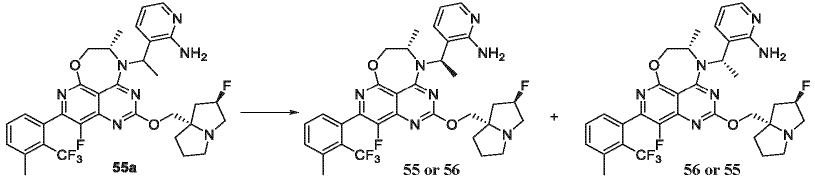

Referring to the preparation of compound **47b** in Example 47, compound **55a** was prepared by replacing ((2-fluoro-8-(4,4,5,5-tetramethyl-1,3,2-dioxaborolan-2-yl)naphthalen-1-yl)ethynyl)triisopropylsilane with (3-methyl-2-(trifluoromethyl)phenyl)boronic acid in step 1.

Compound **55a** was sequentially purified by preparative liquid phase chromatography (YMC C18 10 µm 50 × 250 chromatographic column; acetonitrile-0.1% ammonium hydroxide (gradient elution of 60%-90%/0-60 min)) to give compound **55** (Rt 25 min, LC-MS: m/z = 656.28 (M + H)⁺) and compound **56** (Rt 28 min, LC-MS: m/z = 656.28 (M + H)⁺).

**Compound 55:** ¹H-NMR (500 MHz, DMSO-*d₆*), *δ*: 7.99 (dd, *J =* 4.8 Hz, *J =* 1.6 Hz, 1H), 7.69 (d, *J =* 7.6, 1H), 7.67-7.60 (m, 1H), 7.57 (d, *J =* 7.6 Hz, 1H), 7.30 (br, 1H), 6.69 (dd, *J =* 7.4 Hz, *J =* 5.0 Hz, 1H), 6.52 (q, *J* = 6.6 Hz, 1H), 5.60 (s, 2H), 5.38-5.19 (m, 1H), 4.52 (dd, *J* = 12.6 Hz, *J* = 5.2 Hz, 1H), 4.38-4.25 (m, 1H), 4.13 (s, 2H), 4.06-3.98 (m, 1H), 3.14-2.98 (m, 3H), 2.87-2.79 (m, 1H), 2.57-2.53 (m, 3H), 2.17-1.95 (m, 3H), 1.89-1.74 (m, 3H), 1.60 (d, *J* = 6.8 Hz, 3H), 0.54 (d, *J* = 6.8 Hz, 3H).

**Compound 56:** ¹H-NMR (500 MHz, DMSO-d₆), *δ*: 7.99 (dd, *J =* 4.8 Hz, *J =* 1.5 Hz, 1H), 7.72 (d, *J =* 7.6 Hz, 1H), 7.64 (t, *J =* 7.6 Hz, 1H), 7.57 (d, *J =* 7.6 Hz, 1H), 7.31 (br, 1H), 6.70 (dd, *J* = 7.4 Hz, *J* = 4.9 Hz, 1H), 6.45 (q, *J =* 6.6 Hz, 1H), 5.60 (s, 2H), 5.38-5.20 (m, 1H), 4.35 (dd, *J =* 12.4 Hz, *J* = 5.6 Hz, 1H), 4.20 (d, *J* = 10.4 Hz, 1H), 4.09-4.00 (m, 1H), 3.95-3.84 (m, 2H), 3.13-2.98 (m, 3H), 2.87-2.77 (m, 1H), 2.57-2.53 (m, 3H), 2.17-1.96 (m, 3H), 1.90-1.72 (m, 3H), 1.62 (d, *J* = 6.9 Hz, 3H), 1.17 (d, *J* = 6.5 Hz, 3H).

The retention time in chiral chromatographic column of compound 55 is short compared to that of compound 56, and the retention time in chiral chromatographic column of compound 56 is long compared to that of compound 55.

### Example 57 and Example 58

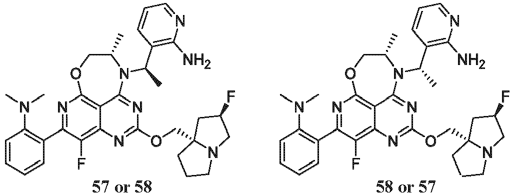

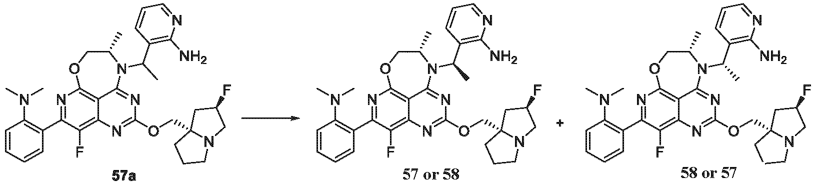

Referring to the preparation of compound **47b** in Example 47, compound **57a** was prepared by replacing ((2-fluoro-8-(4,4,5,5-tetramethyl-1,3,2-dioxaborolan-2-yl)naphthalen-1-yl)ethynyl)triisopropylsilane with 2-(dimethylamino)phenylboronic acid pinacol ester in step 1.

Compound **57a** was sequentially purified by preparative liquid phase chromatography (YMC C18 10 µm 50 × 250 chromatographic column; acetonitrile-0.1% ammonium hydroxide (gradient elution of 50%-90%/0-60 min)) to give compound **57** (Rt 49 min) and compound **58** (Rt 52 min).

**Compound 57:** LC-MS: m/z = 617.41 (M + H)⁺.

¹H-NMR (500 MHz, DMSO-*d₆*), *δ*: 7.99 (s, 1H), 7.69 (d, *J =* 6.8 Hz, 1H), 7.36 (t, *J* = 7.0 Hz, 1H), 7.24 (d, *J* = 7.0 Hz, 1H), 7.09 (d, *J =* 8.0 Hz, 1H), 6.99 (t, *J =* 7.0 Hz, 1H), 6.72-6.65 (m, 1H), 6.57-6.49 (m, 1H), 5.64 (s, 2H), 5.42-5.17 (m, 1H), 4.57-4.47 (m, 1H), 4.29 (d, *J =* 12.4 Hz, 1H), 4.14 (s, 2H), 4.06-3.97 (m, 1H), 3.16-3.00 (m, 3H), 2.89-2.79 (m, 1H), 2.53 (s, 6H), 2.21-1.98 (m, 3H), 1.91-1.74 (m, 3H), 1.59 (d, *J* = 5.8 Hz, 3H) , 0.55 (d, *J*= 6.2 Hz, 3H).

**Compound 58:** LC-MS: m/z = 617.38 (M + H)⁺.

¹H-NMR (500 MHz, DMSO-*d₆*), *δ*: 8.00 (s, 1H), 7.72 (d, *J* = 5.8 Hz, 1H), 7.41-7.30 (m, 1H), 7.25 (d, *J =* 5.8 Hz, 1H), 7.16-7.04 (m, 1H), 7.03-6.94 (m, 1H), 6.75-6.64 (m, 1H), 6.54-6.41 (m, 1H), 5.62 (s, 2H), 5.42-5.17 (m, 1H), 4.41-4.27 (m, 1H), 4.20 (d, *J =* 9.6 Hz, 1H), 4.06 (d, *J =* 9.8 Hz, 1H), 3.90 (d, *J =* 10.2 Hz, 2H), 3.16-2.99 (m, 3H), 2.89-2.77 (m, 1H), 2.54 (s, 6H), 2.21-1.95 (m, 3H), 1.91-1.71 (m, 3H), 1.70-1.53 (m, 3H) , 1.27-1.10 (m, 3H).

The retention time in chiral chromatographic column of compound 57 is short compared to that of compound 58, and the retention time in chiral chromatographic column of compound 58 is long compared to that of compound 57.

### Example 59 and Example 60

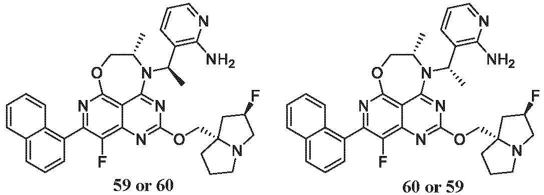

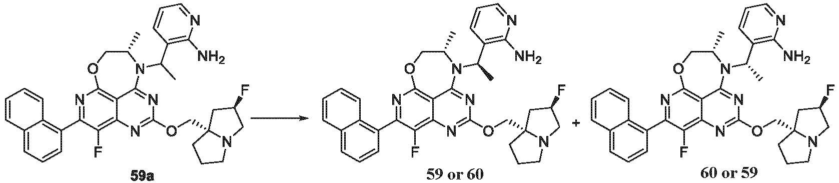

Referring to the preparation of compound **47b** in Example 47, compound **59a** was prepared by replacing ((2-fluoro-8-(4,4,5,5-tetramethyl-1,3,2-dioxaborolan-2-yl)naphthalen-1-yl)ethynyl)triisopropylsilane with 1-naphthylboronic acid in step 1.

Compound **59a** was sequentially purified by preparative liquid phase chromatography (YMC C18 10 µm 50 × 250 chromatographic column; acetonitrile-0.1% ammonium hydroxide (gradient elution of 60%-90%/0-60 min)) to give compound **59** (Rt 49 min, LC-MS: m/z = 624.40 (M + H)⁺) and compound **60** (Rt 55 min, LC-MS: m/z = 624.37 (M + H)⁺).

The retention time in chiral chromatographic column of compound 59 is short compared to that of compound 60, and the retention time in chiral chromatographic column of compound 60 is long compared to that of compound 59.

### Example 61 and Example 62

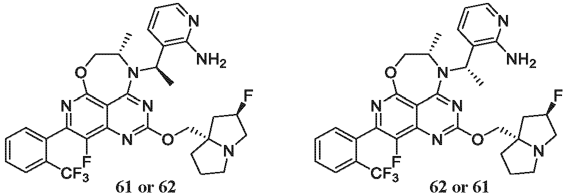

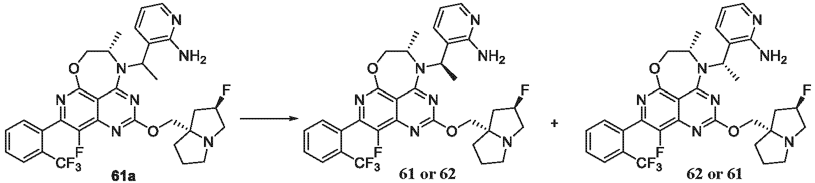

Referring to the preparation of compound **47b** in Example 47, compound **61a** was prepared by replacing ((2-fluoro-8-(4,4,5,5-tetramethyl-1,3,2-dioxaborolan-2-yl)naphthalen-1-yl)ethynyl)triisopropylsilane with 2-(trifluoromethyl)phenylboronic acid in step 1.

Compound **61a** was sequentially purified by preparative liquid phase chromatography (YMC C18 10 µm 50 × 250 chromatographic column; acetonitrile-0.1% ammonium hydroxide (gradient elution of 60%-90%/0-60 min)) to give compound **61** (Rt 35 min, LC-MS: m/z = 642.34 (M + H)⁺) and compound **62** (Rt 40 min, LC-MS: m/z = 642.34 (M + H)⁺).

**Compound 61:** ¹H-NMR (500 MHz, DMSO-*d₆*), *δ*: 7.99 (d, *J =* 4.2 Hz, 1H), 7.89 (d, *J* = 7.8, 1H), 7.80 (t, *J =* 7.4 Hz, 1H), 7.77-7.66 (m, 2H), 7.57 (d, *J* = 7.4 Hz, 1H), 6.74-6.64 (m, 1H), 6.58-6.46 (m, 1H), 5.59 (s, 2H), 5.38-5.19 (m, 1H), 4.58-4.48 (m, 1H), 4.37-4.27 (m, 1H), 4.14 (s, 2H), 4.08-3.97 (m, 1H), 3.16-2.99 (m, 3H), 2.88-2.77 (m, 1H), 2.20-1.97 (m, 3H), 1.91-1.72 (m, 3H), 1.60 (d, *J* = 6.6 Hz, 3H), 0.54 (d, *J =* 6.6 Hz, 3H).

**Compound 62:** ¹H-NMR (500 MHz, DMSO-*d₆*), *δ*: 7.99 (dd, *J =* 4.8 Hz, *J =* 1.6 Hz, 1H), 7.89 (d, *J =* 7.8 Hz, 1H), 7.81 (t, *J* = 7.4 Hz, 1H), 7.76-7.69 (m, 2H), 7.58 (d, *J* = 7.6 Hz, 1H), 6.70 (dd, *J =* 7.4 Hz, *J* = 5.0 Hz, 1H), 6.45 (q, *J =* 7.0 Hz, 1H), 5.60 (s, 2H), 5.38-5.20 (m, 1H), 4.35 (dd, *J =* 12.6 Hz, *J =* 5.6 Hz, 1H), 4.23-4.17 (m, 1H), 4.09-4.03 (m, 1H), 3.95-3.84 (m, 2H), 3.13-2.99 (m, 3H), 2.87-2.74 (m, 1H), 2.20-1.96 (m, 3H), 1.89-1.72 (m, 3H), 1.62 (d, *J* = 6.8 Hz, 3H), 1.17 (d, *J* = 6.8 Hz, 3H).

The retention time in chiral chromatographic column of compound 61 is short compared to that of compound 62, and the retention time in chiral chromatographic column of compound 62 is long compared to that of compound 61.

### Example 63 and Example 64

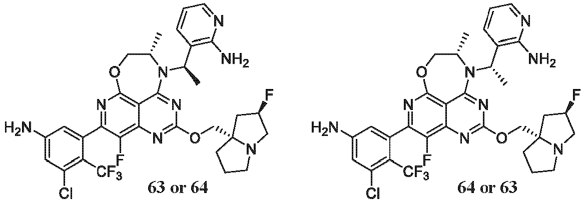

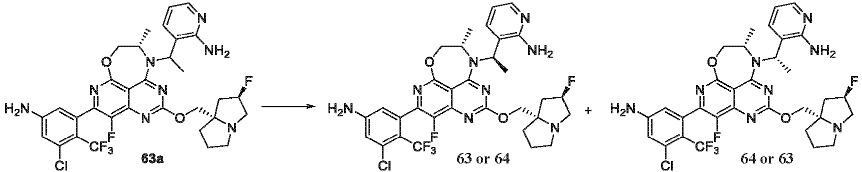

Referring to the preparation of compound **47b** in Example 47, compound **63a** was prepared by replacing ((2-fluoro-8-(4,4,5,5-tetramethyl-1,3,2-dioxaborolan-2-yl)naphthalen-1-yl)ethynyl)triisopropylsilane with 3-chloro-5-(4,4,5,5-tetramethyl-1,3,2-dioxaborolan-2-yl)-4-(trifluoromethyl)aniline in step 1.

Compound **63a** was sequentially purified by preparative liquid phase chromatography (YMC C18 10 µm 50 × 250 chromatographic column; acetonitrile-0.1% ammonium hydroxide (gradient elution of 60%-90%/0-60 min)) to give compound **63** (Rt 24 min, LC-MS: m/z = 691.23 (M + H)⁺) and compound **64** (Rt 27 min, LC-MS: m/z = 691.23 (M + H)⁺).

**Compound 63:** ¹H-NMR (500 MHz, DMSO-*d₆*), *δ*: 8.02-7.95 (m, 1H), 7.69 (d, *J* = 7.4 Hz, 1H), 6.89-6.83 (m, 1H), 6.69 (dd, *J* = 7.4 Hz, *J=* 5.0 Hz, 1H), 6.57-6.38 (m, 2H), 6.37-6.19 (m, 2H), 5.59 (s, 2H), 5.38-5.19 (m, 1H), 4.56-4.47 (m, 1H), 4.36-4.25 (m, 1H), 4.13 (s, 2H), 4.06-3.98 (m, 1H), 3.14-3.00 (m, 3H), 2.87-2.79 (m, 1H), 2.19-1.97 (m, 3H), 1.89-1.74 (m, 3H), 1.59 (d, *J* = 6.8 Hz, 3H), 0.53 (d, *J* = 6.8 Hz, 3H).

**Compound 64:** ¹H-NMR (500 MHz, DMSO-*d₆*), *δ*: 8.02-7.96 (m, 1H), 7.76-7.67 (m, 1H), 6.86 (d, *J* = 1.9 Hz, 1H), 6.70 (dd, *J* = 7.4 Hz, *J* = 4.9 Hz, 1H), 6.56-6.38 (m, 2H), 6.30 (br, 2H), 5.60 (s, 2H), 5.38-5.20 (m, 1H), 4.38-4.31 (m, 1H), 4.23-4.16 (m, 1H), 4.09-4.02 m, 1H), 3.98-3.85 (m, 2H), 3.13-3.00 (m, 3H), 2.86-2.79 (m, 1H), 2.19-1.97 (m, 3H), 1.89-1.72 (m, 3H), 1.62 (d, *J* = 6.8 Hz, 3H), 1.16 (d, *J* = 6.8 Hz, 3H).

The retention time in chiral chromatographic column of compound 63 is short compared to that of compound 64, and the retention time in chiral chromatographic column of compound 64 is long compared to that of compound 63.

### Example 65 and Example 66

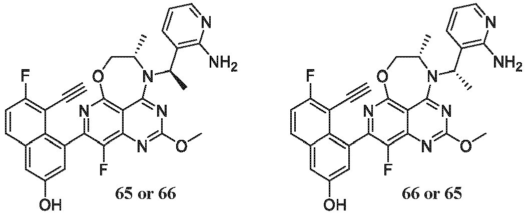

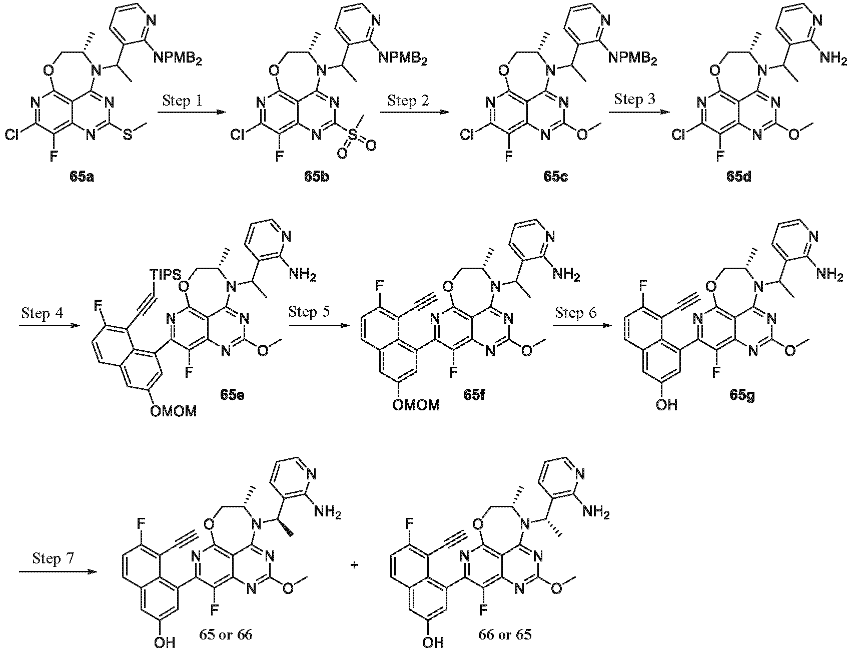

Referring to the preparation of compound **34f** in Example 34, compound **65a** was prepared by replacing 2-(*tert*-butyldimethylsilyloxy)ethanamine with L-alaninol in step 3. LC-MS: m/z = 661.33 (M + H)⁺.

### Step 1:

**65a** (1.5 g) was dispersed in dichloromethane (30 mL), and the mixture was cooled to 0 °C to 5 °C. *m*-Chloroperoxybenzoic acid (80%, 1.82 g) was added, and the resulting mixture was stirred for 1 h with the temperature maintained. After the reaction was completed, a 10% sodium thiosulfate solution (100 mL) was added to the reaction solution, and the mixture was extracted with dichloromethane (100 mL × 2). The organic phases were combined, washed with 5% brine (100 mL), dried over anhydrous sodium sulfate, and filtered. The filtrate was concentrated until no liquid flowed out to give compound **65b.** LC-MS: m/z = 693.31 (M + H)⁺.

### Step 2:

**65b** (600 mg) and methanol (83 mg) were dispersed in tetrahydrofuran (10 mL). The mixture was cooled to 0 °C, and a solution of lithium bis(trimethylsilyl)amide in tetrahydrofuran (1 N, 2.6 mL) was added dropwise. The resulting mixture was stirred for 1 h with the temperature maintained. After the reaction was completed, a saturated ammonium chloride solution (20 mL) was added to the reaction system to quench the reaction, and water (50 mL) and ethyl acetate (80 mL) were added. The mixture was stirred, and the phases were separated. Extraction was performed with ethyl acetate (80 mL × 2), and the organic phases were combined, washed with 5% brine (80 mL), dried over anhydrous sodium sulfate, and filtered. The filtrate was concentrated until no liquid flowed out and purified by column chromatography (methanol:dichloromethane = 1:150 to 1:50) to give compound **65c.** LC-MS: m/z = 645.31 (M + H)⁺.

### Step 3:

**65c** (500 mg) was dispersed in trifluoroacetic acid (10 mL), and the mixture was cooled to 0 °C. Methanesulfonic acid (1 mL) was added dropwise, and the resulting mixture was allowed to be stirred for reaction at room temperature for 2 h. After the reaction was completed, the reaction solution was concentrated under reduced pressure until no liquid flowed out. The residue was dissolved in dichloromethane (10 mL), and the pH of the system was adjusted to alkalinity with a saturated sodium bicarbonate solution under an ice bath. Water (50 mL) and dichloromethane (50 mL) were added, the mixture was stirred, and the phases were separated. Extraction was performed with dichloromethane (50 mL × 2), and the organic phases were combined, washed with saturated brine (100 mL), dried over anhydrous sodium sulfate, and filtered. The filtrate was concentrated until no liquid flowed out to give compound **65d.** LC-MS: m/z = 405.24 (M + H)⁺.

### Step 4:

**65d** (450 mg), **10g** (1.14 g), chloro[4-(di-tert-butylphosphino)-N,N-dimethylaniline-2-(2'-aminobiphenyl)]palladium(II) (128 mg), potassium phosphate (706 mg), and water (4 mL) were dispersed in 1,4-dioxane (20 mL), and under a nitrogen atmosphere, the mixture was heated to 90 °C and stirred for reaction for 3 h. The reaction solution was cooled to room temperature and filtered, and the filtrate was concentrated to dryness under reduced pressure and purified by column chromatography (methanol:dichloromethane = 1:200 to 1:100) to give compound **65e.** LC-MS: m/z = 755.40 (M + H)⁺.

### Step 5:

**65e** (550 mg) and cesium fluoride (1.66 g) were dispersed in DMF (20 mL), and the mixture was stirred for reaction at room temperature for 2 h. Water (50 mL) and ethyl acetate (80 mL) were added to the reaction solution, the mixture was stirred, and the phases were separated. The aqueous phase was extracted with ethyl acetate (50 mL × 2), and the organic phases were combined, washed with 5% brine (80 mL × 3), dried over anhydrous sodium sulfate, and filtered. The filtrate was concentrated until no liquid flowed out to give compound **65f.** LC-MS: m/z = 599.33 (M + H)⁺.

### Step 6:

**65f** (438 mg) was dispersed in dichloromethane (5 mL), and the reaction system was cooled to 0 °C to 5 °C. A solution of hydrogen chloride in 1,4-dioxane (4 N, 1 mL) was added dropwise to the reaction system, and the resulting mixture was stirred for reaction for 1 h with the temperature maintained. After the reaction was completed, the reaction solution was concentrated under reduced pressure until no liquid flowed out to give compound **65g.** LC-MS: m/z = 555.25 (M + H)⁺.

### Step 7:

Compound **65g** was sequentially purified by preparative liquid phase chromatography (YMC C18 10 µm 50 × 250 chromatographic column; acetonitrile-0.1% ammonium hydroxide (gradient elution of 40%-70%/0-60 min)) to give compound **65** (Rt 28 min, LC-MS: m/z = 555.25 (M + H)⁺) and compound **66** (Rt 32 min, LC-MS: m/z = 555.24 (M + H)⁺).

**Compound 65:** ¹H-NMR (500 MHz, DMSO-*d₆*), *δ*: 10.16 (br, 1H), 8.06-7.89 (m, 2H), 7.71 (d, *J* = 7.4 Hz, 1H), 7.50-7.42 (m, 1H), 7.40-7.33 (m, 1H), 7.24-7.09 (m, 1H), 6.75-6.65 (m, 1H), 6.64-6.45 (m, 1H), 5.67-5.48 (m, 2H), 4.59-4.46 (m, 1H), 4.32-4.17 (m, 1H), 4.10-3.77 (m, 5H), 1.68-1.54 (m, 3H), 0.67-0.52 (m, 3H).

**Compound 66:** ¹H-NMR (500 MHz, DMSO-*d₆*), *δ*: 10.13 (br, 1H), 8.03-7.92 (m, 2H), 7.77-7.70 (m, 1H), 7.49-7.42 (m, 1H), 7.37 (d, *J =* 2.5 Hz, 1H), 7.22-7.12 (m, 1H), 6.73-6.66 (m, 1H), 6.58-6.43 (m, 1H), 5.72-5.57 (m, 2H), 4.41-4.27 (m, 1H), 4.10-3.79 (m, 6H), 1.70-1.59 (m, 3H), 1.27-1.18 (m, 3H).

The retention time in chiral chromatographic column of compound 65 is short compared to that of compound 66, and the retention time in chiral chromatographic column of compound 66 is long compared to that of compound 65.

### Example 67 and Example 68

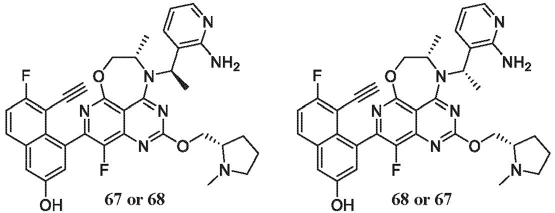

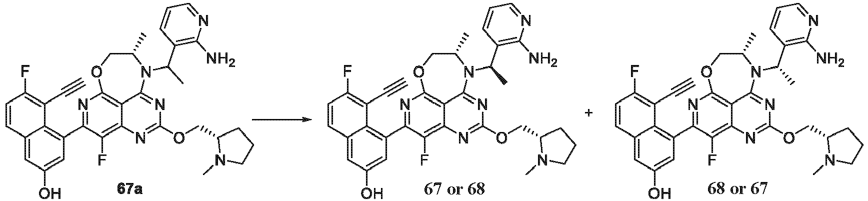

Referring to the preparation of compound **65g** in Example 65, compound **67a** was prepared by replacing methanol with *N*-methyl-*L*-prolinol in step 2.

Compound **67a** was sequentially purified by preparative liquid phase chromatography (YMC TA C18 10 µm 30 × 250 chromatographic column; acetonitrile-0.05% aqueous trifluoroacetic acid solution (gradient elution of 10%-65%/0-60 min)) to give compound **67** (Rt 23.1 min, LC-MS: m/z = 638.37 (M + H)⁺) and compound **68** (Rt 24.4 min, LC-MS: m/z = 638.37 (M + H)⁺).

The retention time in chiral chromatographic column of compound 67 is short compared to that of compound 68, and the retention time in chiral chromatographic column of compound 68 is long compared to that of compound 67.

### Example 69 and Example 70

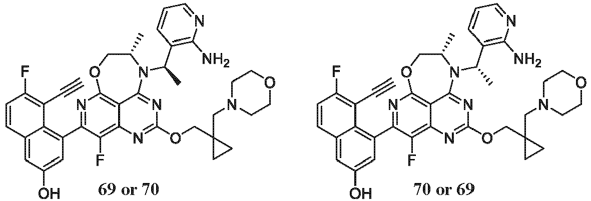

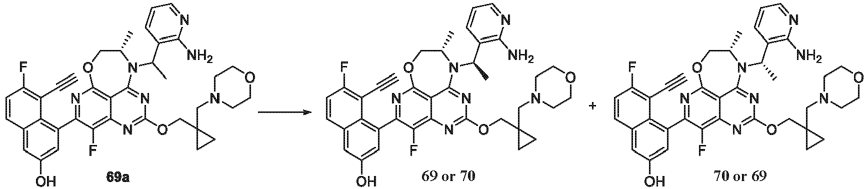

Referring to the preparation of compound **65g** in Example 65, compound **69a** was prepared by replacing methanol with (1-(morpholinomethyl)cyclopropyl)methanol in step 2.

Compound **69a** was sequentially purified by preparative liquid phase chromatography (YMC TA C18 10 µm 30 × 250 chromatographic column; acetonitrile-0.05% aqueous trifluoroacetic acid solution (gradient elution of 10%-60%/0-60 min)) to give compound **69** (Rt 24.6 min, LC-MS: m/z = 694.40 (M + H)⁺) and compound **70** (Rt 26.5 min, LC-MS: m/z = 694.38 (M + H)⁺).

**Compound 69:** ¹H-NMR (500 MHz, DMSO-*d₆*), *δ*: 10.50-9.46 (m, 1H), 8.28-8.20 (m, 1H), 8.15 (d, *J =* 6.2 Hz, 1H), 8.02-7.94 (m, 1H), 7.85-7.56 (m, 2H), 7.51-7.43 (m, 1H), 7.41-7.36 (m, 1H), 7.22-7.04 (m, 2H), 6.63-6.47 (m, 1H), 4.64-4.42 (m, 3H), 4.36-3.91 (m, 6H), 3.83-3.56 (m, 4H), 3.43-3.02 (m, 3H), 1.77-1.60 (m, 3H), 0.98-0.62 (m, 7H).

**Compound 70:** ¹H-NMR (500 MHz, DMSO-*d₆*), *δ*: 10.16-10.08 (m, 1H), 8.03-7.92 (m, 2H), 7.72 (d, *J* = 7.4 Hz, 1H), 7.50-7.42 (m, 1H), 7.37 (d, *J* = 2.5 Hz, 1H), 7.21-7.10 (m, 1H), 6.75-6.65 (m, 1H), 6.56-6.37 (m, 1H), 5.77-5.62 (m, 2H), 4.43-4.21 (m, 3H), 4.10-3.82 (m, 3H), 3.70-3.44 (m, 4H), 2.47-2.26 (m, 2H), 1.70-1.58 (m, 3H), 1.38-1.14 (m, 7H), 0.72-0.61 (m, 2H), 0.53-0.33 (m, 2H).

The retention time in chiral chromatographic column of compound 69 is short compared to that of compound 70, and the retention time in chiral chromatographic column of compound 70 is long compared to that of compound 69.

### Example 71 and Example 72

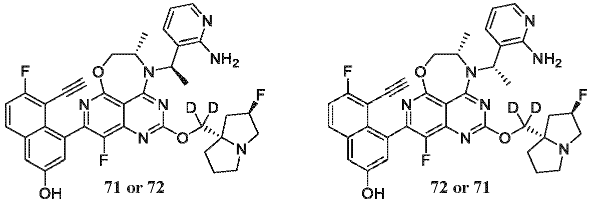

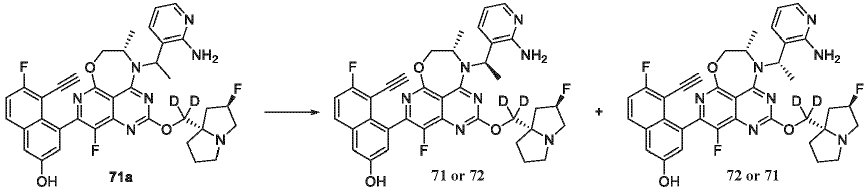

Referring to the preparation of compound **65g** in Example 65, compound **71a** was prepared by replacing methanol with ((2*R*,7a*S*)-2-fluorotetrahydro-1*H*-pyrrolizin-7a(5*H*)-yl)methane-*d*₂-ol in step 2.

Compound **71a** was sequentially purified by preparative liquid phase chromatography (YMC C18 10 µm 50 × 250 chromatographic column; acetonitrile-0.1% ammonium hydroxide (gradient elution of 40%-80%/0-60 min)) to give compound **71** (Rt 40 min, LC-MS: m/z = 684.36 (M + H)⁺) and compound **72** (Rt 43 min, LC-MS: m/z = 684.37 (M + H)⁺).

**Compound 71:** ¹H-NMR (500 MHz, DMSO-*d₆*), *δ*: 10.36-9.87 (m, 1H), 8.19-7.83 (m, 2H), 7.81-7.59 (m, 1H), 7.57-7.29 (m, 2H), 7.28-7.02 (m, 1H), 6.89-6.33 (m, 2H), 5.81-5.45 (m, 2H), 5.44-5.09 (m, 1H), 4.72-3.70 (m, 4H), 3.20-2.70 (m, 3H), 2.34-1.46 (m, 10H), 0.85-0.41 (m, 3H).

**Compound 72:** ¹H-NMR (500 MHz, DMSO-*d₆*), *δ*: 10.23-10.11 (m, 1H), 8.06-7.91 (m, 2H), 7.74 (d, *J* = 7.4 Hz, 1H), 7.51-7.43 (m, 1H), 7.38 (d, *J* = 2.4 Hz, 1H), 7.23-7.11 (m, 1H), 6.76-6.67 (m, 1H), 6.54-6.37 (m, 1H), 5.87-5.62 (m, 2H), 5.61-5.37 (m, 1H), 4.50-4.31 (m, 1H), 4.14-3.83 (m, 3H), 3.79-2.96 (m, 4H), 2.47-2.15 (m,3H), 2.14-1.87 (m, 3H), 1.76-1.57 (m, 3H), 1.33-1.15 (m, 3H).

The retention time in chiral chromatographic column of compound 71 is short compared to that of compound 72, and the retention time in chiral chromatographic column of compound 72 is long compared to that of compound 71.

### Example 73 and Example 74

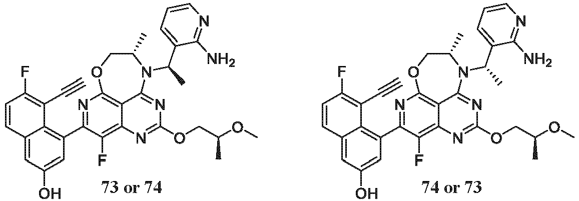

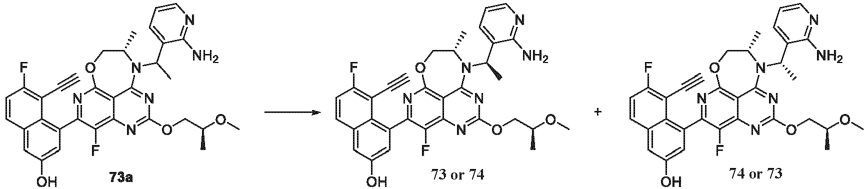

Referring to the preparation of compound **65g** in Example 65, compound **73a** was prepared by replacing methanol with (2S)-2-methoxy-1-propanol in step 2.

Compound **73a** was sequentially purified by preparative liquid phase chromatography (YMC TA C18 10 µm 30 × 250 chromatographic column; acetonitrile-0.05% aqueous trifluoroacetic acid solution (gradient elution of 20%-80%/0-40 min)) to give compound **73** (Rt 27.1 min, LC-MS: m/z = 613.31 (M + H)⁺) and compound **74** (Rt 29.3 min, LC-MS: m/z = 613.27 (M + H)⁺).

The retention time in chiral chromatographic column of compound 73 is short compared to that of compound 74, and the retention time in chiral chromatographic column of compound 74 is long compared to that of compound 73.

### Example 75 and Example 76

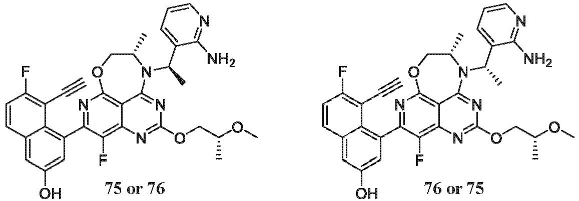

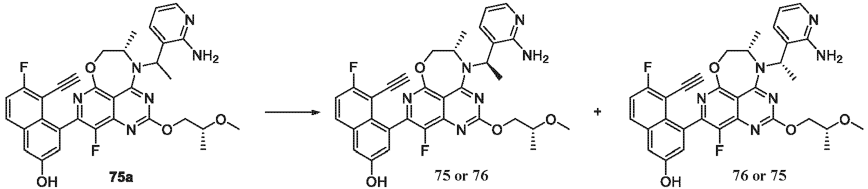

Referring to the preparation of compound **65g** in Example 65, compound **75a** was prepared by replacing methanol with (*R*)-2-methoxy-1-propanol in step 2.

Compound **75a** was sequentially purified by preparative liquid phase chromatography (YMC C18 10 µm 50 × 250 chromatographic column; acetonitrile-0.1% ammonium hydroxide (gradient elution of 40%-70%/0-60 min)) to give compound **75** (Rt 28 min, LC-MS: m/z = 613.33 (M + H)⁺) and compound **76** (Rt 31 min, LC-MS: m/z = 613.33 (M + H)⁺).

**Compound 75:** ¹H-NMR (500 MHz, DMSO-*d₆*), *δ*: 10.19 (br, 1H), 8.05-7.91 (m, 2H), 7.71 (d, *J =* 7.0 Hz, 1H), 7.51-7.42 (m, 1H), 7.41-7.35 (m, 1H), 7.27-7.11 (m, 1H), 6.76-6.66 (m, 1H), 6.62-6.44 (m, 1H), 5.67-5.47 (m, 2H), 4.60-4.46 (m, 1H), 4.44-4.34 (m, 2H), 4.33-4.21 (m, 1H), 4.12-3.78 (m, 2H), 3.79-3.70 (m, 1H), 3.34 (s, 3H), 1.72-1.55 (m, 3H), 1.29-1.14 (m, 3H), 0.67-0.63 (m, 3H).

**Compound 76:** ¹H-NMR (500 MHz, DMSO-*d₆*), *δ*: 10.20 (br, 1H), 8.09-7.89 (m, 2H), 7.74 (d, *J =* 7.4 Hz, 1H), 7.53-7.42 (m, 1H), 7.39 (d, *J =* 2.4 Hz, 1H), 7.26-7.14 (m, 1H), 6.77-6.65 (m, 1H), 6.62-6.41 (m, 1H), 5.78-5.52 (m, 2H), 4.46-4.28 (m, 3H), 4.14-3.83 (m, 3H), 3.79-3.68 (m, 1H), 3.33 (s, 3H), 1.75-1.58 (m, 3H), 1.32-1.14 (m, 6H).

The retention time in chiral chromatographic column of compound 75 is short compared to that of compound 76, and the retention time in chiral chromatographic column of compound 76 is long compared to that of compound 75.

### Example 77 and Example 78

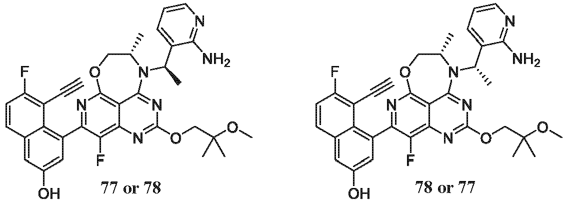

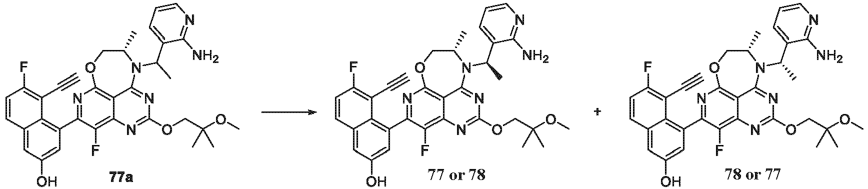

Referring to the preparation of compound **65g** in Example 65, compound **77a** was prepared by replacing methanol with 2-methoxy-2-methylpropan-1-ol in step 2.

Compound **77a** was purified by preparative liquid phase chromatography (YMC C18 10 µm 50 × 250 chromatographic column; acetonitrile-30 mM ammonium acetate (gradient elution of 50%-80%/0-60 min)) and desalted with acetonitrile/0.1% formic acid to sequentially give compound **77** (Rt 21 min, LC-MS: m/z = 627.32 (M + H)⁺) and compound **78** (Rt 24 min, LC-MS: m/z = 627.33 (M + H)⁺).

**Compound 77:** ¹H-NMR (500 MHz, DMSO-*d₆*), *δ*: 8.08-7.90 (m, 2H), 7.71 (d, *J* = 6.9 Hz, 1H), 7.54-7.32 (m, 2H), 7.27-7.09 (m, 1H), 6.78-6.41 (m, 2H), 5.72-5.48 (m, 2H), 4.63-4.43 (m, 1H), 4.39-3.76 (m, 6H), 3.19 (s, 3H), 1.73-1.51 (m, 3H), 1.31-1.18 (m, 6H), 0.70-0.48 (m, 3H).

**Compound 78:** ¹H-NMR (500 MHz, DMSO-*d₆*), *δ*: 8.08-7.90 (m, 2H), 7.74 (d, *J* = 7.0 Hz, 1H), 7.54-7.33 (m, 2H), 7.28-7.10 (m, 1H), 6.84-6.64 (m, 1H), 6.63-6.39 (m, 1H), 5.80-5.50 (m, 2H), 4.45-3.80 (m, 7H), 3.20 (s, 3H), 1.77-1.59 (m, 3H), 1.33-1.18 (m, 9H).

The retention time in chiral chromatographic column of compound 77 is short compared to that of compound 78, and the retention time in chiral chromatographic column of compound 77 is long compared to that of compound 78.

### Example 79 and Example 80

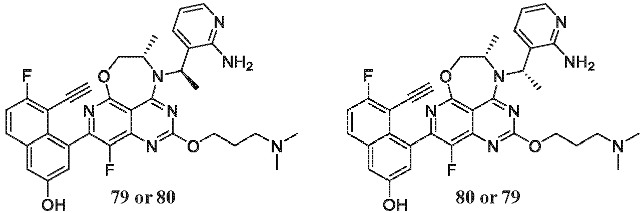

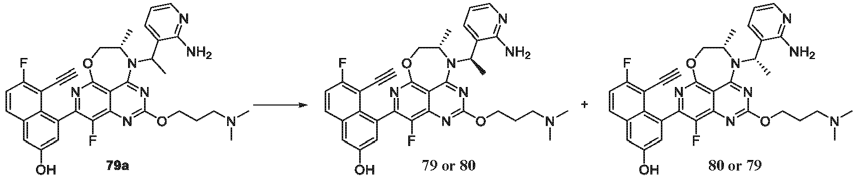

Referring to the preparation of compound 65g in Example 65, compound 79a was prepared by replacing methanol with 3-dimethylamino-1-propanol in step 2.

Compound 79a was sequentially purified by preparative liquid phase chromatography (YMC C18 10 µm 50 × 250 chromatographic column; acetonitrile-0.1% ammonium hydroxide (gradient elution of 50%-80%/0-60 min)) to give compound **79** (Rt 32 min, LC-MS: m/z = 626.36 (M + H)⁺) and compound **80** (Rt 36 min, LC-MS: m/z = 626.36 (M + H)⁺).

**Compound 79:** ¹H-NMR (500 MHz, DMSO-*d₆*), *δ*: 10.18 (br, 1H), 8.07-7.88 (m, 2H), 7.71 (d, *J =* 7.4 Hz, 1H), 7.51-7.42 (m, 1H), 7.41-7.34 (m, 1H), 7.26-7.10 (m, 1H), 6.77-6.67 (m, 1H), 6.61-6.43 (m, 1H), 5.66-5.45 (m, 2H), 4.62-4.38 (m, 3H), 4.34-4.18 (m, 1H), 4.12-3.77 (m, 2H), 2.42-2.33 (m, 2H), 2.22-2.10 (m, 6H), 1.96-1.87 (m, 2H), 1.69-1.53 (m, 3H), 0.71-0.50 (m, 3H).

**Compound 80:** ¹H-NMR (500 MHz, DMSO-*d₆*), *δ*: 10.17 (br, 1H), 8.07-7.90 (m, 2H), 7.73 (d, *J =* 7.4 Hz, 1H), 7.52-7.42 (m, 1H), 7.38 (d, *J =* 2.5 Hz, 1H), 7.24-7.13 (m, 1H), 6.77-6.63 (m, 1H), 6.58-6.40 (m, 1H), 5.75-5.51 (m, 2H), 4.49-4.25 (m, 3H), 4.13-3.80 (m, 3H), 2.45-2.35 (m, 2H), 2.17 (s, 6H), 1.97-1.85 (m, 2H), 1.70-1.58 (m, 3H), 1.27-1.17 (m, 3H).

The retention time in chiral chromatographic column of compound 79 is short compared to that of compound 80, and the retention time in chiral chromatographic column of compound 80 is long compared to that of compound 79.

### Example 81

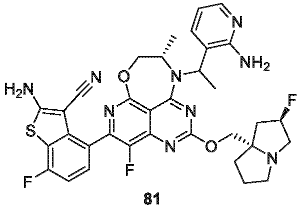

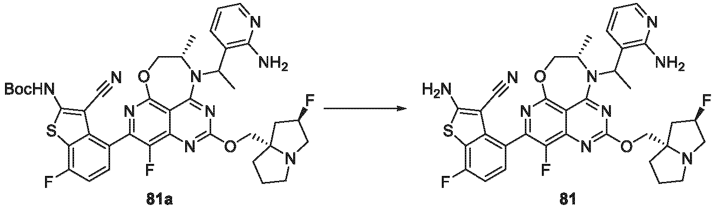

Referring to the preparation of compound **47b** in Example 47, compound **81a** was prepared by replacing ((2-fluoro-8-(4,4,5,5-tetramethyl-1,3,2-dioxaborolan-2-yl)naphthalen-1-yl)ethynyl)triisopropylsilane with *tert*-butyl (3-cyano-7-fluoro-4-(4,4,5,5-tetramethyl-1,3,2-dioxaborolan-2-yl)benzo[*b*]thiophen-2-yl)carbamate in step 1. LC-MS: m/z = 788.41 (M + H)⁺.

**81a** (430 mg) was dispersed in dichloromethane (10 mL), and the mixture was cooled to 0 °C to 5 °C. Trifluoroacetic acid (4 mL) was added, and the resulting mixture was stirred for reaction for 2 h with the temperature maintained. After the reaction was completed, the reaction solution was concentrated under reduced pressure until no liquid flowed out. The residue was dissolved in dichloromethane (10 mL), and the pH of the system was adjusted to alkalinity with a saturated sodium bicarbonate solution under an ice bath. Water (50 mL) and dichloromethane (50 mL) were added, the mixture was stirred, and the phases were separated. Extraction was performed with dichloromethane (50 mL × 2), and the organic phases were combined, washed with saturated brine (100 mL), dried over anhydrous sodium sulfate, and filtered. The filtrate was concentrated until no liquid flowed out to give compound **81.** LC-MS: m/z = 688.30 (M + H)⁺.

### Example 82 and Example 83

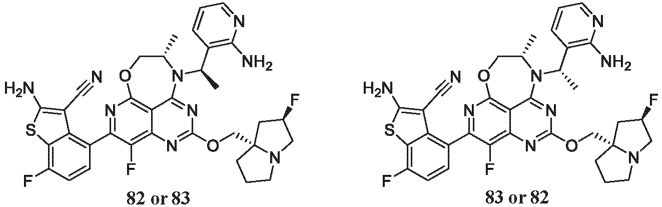

Compound **81** was sequentially purified by preparative liquid phase chromatography (YMC C18 10 µm 50 × 250 chromatographic column; acetonitrile-0.1% ammonium hydroxide (gradient elution of 70%-90%/0-60 min)) to give compound **82** (Rt 23 min, LC-MS: m/z = 688.35 (M + H)⁺) and compound **83** (Rt 24 min, LC-MS: m/z = 688.30 (M + H)⁺).

The retention time in chiral chromatographic column of compound 82 is short compared to that of compound 83, and the retention time in chiral chromatographic column of compound 83 is long compared to that of compound 82.

### Example 84

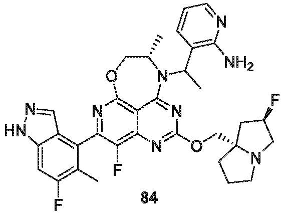

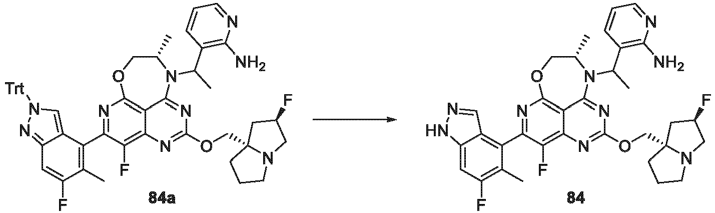

Referring to the preparation of compound **47b** in Example 47, compound **84a** was prepared by replacing ((2-fluoro-8-(4,4,5,5-tetramethyl-1,3,2-dioxaborolan-2-yl)naphthalen-1-yl)ethynyl)triisopropylsilane with 6-fluoro-5-methyl-4-(4,4,5,5-tetramethyl-1,3,2-dioxaborolan-2-yl)-2-(triphenylmethyl)-2*H*-indazole (synthesized with reference to WO2022173032A1) in step 1. LC-MS: m/z = 888.60 (M + H)⁺.

**84a** (200 mg) was dispersed in methanol (10 mL). At room temperature, *p*-toluenesulfonic acid (10 mg) was added dropwise, and the mixture was stirred for reaction for 1 h. After the reaction was completed, the reaction solution was purified by preparative liquid phase chromatography (YMC C18 10 µm 50 × 250 chromatographic column: acetonitrile-0.1% ammonium hydroxide (gradient elution of 50%-90%/0-60 min)) to give compound **84.** LC-MS: m/z = 646.48 (M + H)⁺.

### Example 85 and Example 86

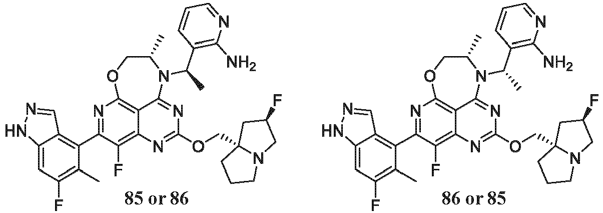

Compound **84** was sequentially purified by preparative liquid phase chromatography (YMC C18 10 µm 50 × 250 chromatographic column; acetonitrile-0.1% ammonium hydroxide (gradient elution of 50%-90%/0-60 min)) to give compound **85** (Rt 43 min, LC-MS: m/z = 646.48 (M + H)⁺) and compound **86** (Rt 47 min, LC-MS: m/z = 646.48 (M + H)⁺).

**Compound 85:** ¹H-NMR (500 MHz, DMSO-*d₆*), *δ*: 13.18 (br, 1H), 8.00 (s, 1H), 7.86-7.60 (m, 2H), 7.56-7.33 (m, 1H), 6.88-6.40 (m, 2H), 5.83-5.48 (m, 2H), 5.46-5.15 (m, 1H), 4.68-4.47 (m, 1H), 4.46-3.90 (m, 4H), 3.19-2.97 (m, 3H), 2.92-2.73 (m, 1H), 2.31-1.95 (m, 6H), 1.94-1.72 (m, 3H), 1.71-1.50 (m, 3H), 0.77-0.48 (m, 3H).

**Compound 86:** ¹H-NMR (500 MHz, DMSO-*d₆*), *δ*: 13.18 (br, 1H), 8.09-7.95 (m, 1H), 7.87-7.66 (m, 2H), 7.54-7.40 (m, 1H), 6.80-6.68 (m, 1H), 6.58-6.43 (m, 1H), 5.80-5.49 (m, 2H), 5.44-5.17 (m, 1H), 4.46-4.33 (m, 1H), 4.30-4.19 (m, 1H), 4.14-4.03 (m, 1H), 4.02-3.85 (m, 2H), 3.16-2.99 (m, 3H), 2.91-2.77 (m, 1H), 2.26-1.94 (m, 6H), 1.92-1.73 (m, 3H), 1.72-1.56 (m, 3H), 1.33-1.15 (m, 3H).

The retention time in chiral chromatographic column of compound 85 is short compared to that of compound 86, and the retention time in chiral chromatographic column of compound 86 is long compared to that of compound 85.

### Example 87 and Example 88

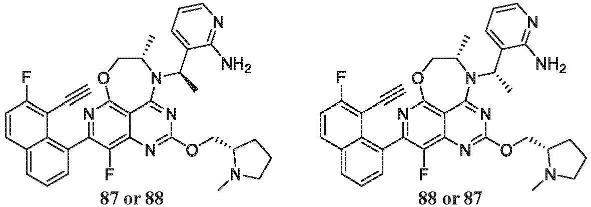

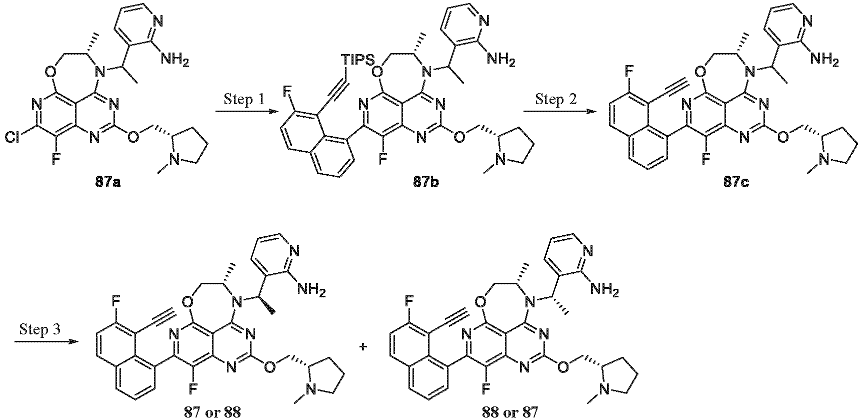

Referring to the preparation of compound **65d** in Example 65, compound **87a** was prepared by replacing methanol with *N*-methyl-*L*-prolinol in step 2. LC-MS: m/z = 488.23 (M + H)⁺.

### Step 1:

**87a** (132 mg), ((2-fluoro-8-(4,4,5,5-tetramethyl-1,3,2-dioxaborolan-2-yl)naphthalen-1-yl)ethynyl)triisopropylsilane (244 mg), chloro[4-(di-tert-butylphosphino)-N,N-dimethylaniline-2-(2'-aminobiphenyl)]palladium(II) (31 mg), potassium phosphate (171 mg), and water (3 mL) were dispersed in 1,4-dioxane (15 mL), and under a nitrogen atmosphere, the mixture was heated to 90 °C and stirred for reaction for 2 h. The reaction solution was cooled to room temperature and filtered, and the filtrate was concentrated to dryness under reduced pressure and purified by column chromatography (ethyl acetate:petroleum ether = 1:10 to 1:2) to give compound **87b.** LC-MS: m/z = 778.59 (M + H)⁺.

### Step 2:

**87b** (130 mg) and cesium fluoride (253 mg) were dispersed in DMF (13 mL), and the mixture was stirred for reaction at room temperature for 2 h. Water (50 mL) and ethyl acetate (80 mL) were added to the reaction solution, the mixture was stirred, and the phases were separated. The aqueous phase was extracted with ethyl acetate (50 mL × 2), and the organic phases were combined, washed with 5% brine (80 mL × 3), dried over anhydrous sodium sulfate, and filtered. The filtrate was concentrated until no liquid flowed out to give compound **87c.** LC-MS: m/z = 622.37 (M + H)⁺.

### Step 3:

Compound **87c** was sequentially purified by preparative liquid phase chromatography (YMC C18 10 µm 50 × 250 chromatographic column; acetonitrile-0.1% ammonium hydroxide (gradient elution of 50%-80%/0-60 min)) to give compound **87** (Rt 32 min, LC-MS: m/z = 622.37 (M + H)⁺) and compound **88** (Rt 35 min, LC-MS: m/z = 622.37 (M + H)⁺).

**Compound 87:** ¹H-NMR (500 MHz, DMSO-*d₆*), *δ*: 8.27-8.12 (m, 2H), 8.05-7.95 (m, 1H), 7.78-7.50 (m, 4H), 6.75-6.65 (m, 1H), 6.61-6.43 (m, 1H), 5.69-5.44 (m, 2H), 4.61-4.38 (m, 2H), 4.34-3.81 (m, 4H), 3.04-2.92 (m, 1H), 2.72-2.58 (m, 1H), 2.38 (s, 3H), 2.28-2.16 (m, 1H), 2.05-1.91 (m, 1H), 1.79-1.54 (m, 6H), 0.70-0.48 (m, 3H).

**Compound 88:** ¹H-NMR (500 MHz, DMSO-*d₆*), *δ*: 8.44-8.09 (m, 2H), 7.99 (s, 1H), 7.89-7.36 (m, 4H), 6.87-6.40 (m, 2H), 5.64 (s, 2H), 4.70-3.66 (m, 6H), 3.07-2.86 (m, 1H), 2.73-2.56 (m, 1H), 2.37 (s, 3H), 2.28-2.12 (m, 1H), 2.08-1.89 (m, 1H), 1.83-1.52 (m, 6H), 1.36-1.01 (m, 3H).

The retention time in chiral chromatographic column of compound 87 is short compared to that of compound 88, and the retention time in chiral chromatographic column of compound 88 is long compared to that of compound 87.

### Example 89 and Example 90

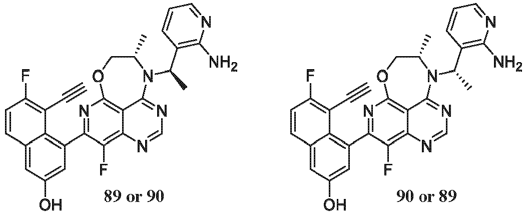

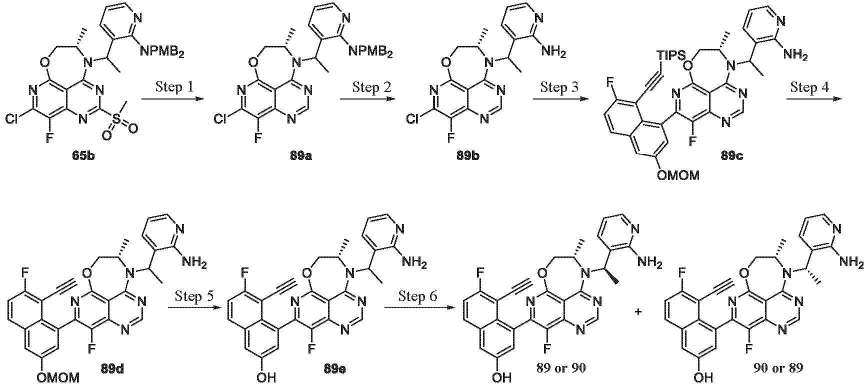

### Step 1:

**65b** (600 mg) and ethanol (10 mL) were dispersed in dichloromethane (10 mL), and the mixture was cooled to 0 °C. Sodium borohydride (65 mg) was added slowly, and the resulting mixture was allowed to be stirred for reaction at room temperature for 1 h. After the reaction was completed, a saturated ammonium chloride solution (20 mL) was added to the reaction system to quench the reaction, and water (50 mL) and ethyl acetate (80 mL) were added. The mixture was stirred, and the phases were separated. Extraction was performed with ethyl acetate (80 mL × 2), and the organic phases were combined, washed with 5% brine (80 mL), dried over anhydrous sodium sulfate, and filtered. The filtrate was concentrated until no liquid flowed out to give compound **89a.** LC-MS: m/z = 615.30 (M + H)⁺.

### Step 2:

**89a** (420 mg) was dispersed in trifluoroacetic acid (10 mL), and the mixture was cooled to 0 °C. Methanesulfonic acid (1 mL) was added dropwise, and the resulting mixture was allowed to be stirred for reaction at room temperature for 2 h. After the reaction was completed, the reaction solution was concentrated under reduced pressure until no liquid flowed out. The residue was dissolved in dichloromethane (10 mL), and the pH of the system was adjusted to alkalinity with a saturated sodium bicarbonate solution under an ice bath. Water (50 mL) and dichloromethane (50 mL) were added, the mixture was stirred, and the phases were separated. Extraction was performed with dichloromethane (50 mL × 2), and the organic phases were combined, washed with saturated brine (100 mL), dried over anhydrous sodium sulfate, and filtered. The filtrate was concentrated until no liquid flowed out to give compound **89b.** LC-MS: m/z = 375.25 (M + H)⁺.

### Step 3:

**89b** (180 mg), **10g** (492 mg), chloro[4-(di-tert-butylphosphino)-N,N-dimethylaniline-2-(2'-aminobiphenyl)]palladium(II) (55 mg), potassium phosphate (305 mg), and water (3 mL) were dispersed in 1,4-dioxane (15 mL), and under a nitrogen atmosphere, the mixture was heated to 90 °C and stirred for reaction for 3 h. The reaction solution was cooled to room temperature and filtered, and the filtrate was concentrated to dryness under reduced pressure and purified by column chromatography (methanol:dichloromethane = 1:200 to 1:100) to give compound **89c.** LC-MS: m/z = 725.35 (M + H)⁺.

### Step 4:

**89c** (220 mg) and cesium fluoride (592 mg) were dispersed in DMF (5 mL), and the mixture was stirred for reaction at room temperature for 2 h. Water (50 mL) and ethyl acetate (80 mL) were added to the reaction solution, the mixture was stirred, and the phases were separated. The aqueous phase was extracted with ethyl acetate (50 mL × 2), and the organic phases were combined, washed with 5% brine (80 mL × 3), dried over anhydrous sodium sulfate, and filtered. The filtrate was concentrated until no liquid flowed out to give compound 89d. LC-MS: m/z = 569.25 (M + H)⁺.

### Step 5:

**89d** (220 mg) was dispersed in dichloromethane (5 mL), and the reaction system was cooled to 0 °C to 5 °C. A solution of hydrogen chloride in 1,4-dioxane (4 N, 1 mL) was added dropwise to the reaction system, and the resulting mixture was stirred for reaction for 1 h with the temperature maintained. After the reaction was completed, the reaction solution was concentrated under reduced pressure until no liquid flowed out to give compound **89e.** LC-MS: m/z = 525.20 (M + H)⁺.

### Step 6:

Compound **89e** was sequentially purified by preparative liquid phase chromatography (YMC C18 10 µm 50 × 250 chromatographic column; acetonitrile-0.1% ammonium hydroxide (gradient elution of 55%-85%/0-60 min)) to give compound **89** (Rt 21 min, LC-MS: m/z = 525.20 (M + H)⁺) and compound **90** (Rt 24 min, LC-MS: m/z = 525.22 (M + H)⁺).

**Compound 89:** ¹H-NMR (500 MHz, DMSO-*d₆*), *δ*: 10.24-10.00 (m, 1H), 8.77-8.71 (m, 1H), 8.03-7.94 (m, 2H), 7.76-7.70 (m, 1H), 7.50-7.43 (m, 1H), 7.41-7.36 (m, 1H), 7.25-7.13 (m, 1H), 6.74-6.60 (m, 2H), 5.64-5.45 (m, 2H), 4.60-4.50 (m, 1H), 4.39-4.24 (m, 1H), 4.12-4.04 (m, 1H), 4.03-3.81(m, 1H), 1.69-1.55 (m, 3H), 0.67-0.52 (m, 3H).

**Compound 90:** ¹H-NMR (500 MHz, DMSO-*d₆*), *δ*: 10.13 (br, 1H), 8.73 (s, 2H), 8.04-7.93 (m, 2H), 7.79-7.72 (m, 1H), 7.50-7.42 (m, 1H), 7.38 (d, *J* = 2.5 Hz, 1H), 7.24-7.14 (m, 1H), 6.75-6.60 (m, 2H), 5.70-5.58 (m, 1H), 4.43-4.29 (m, 1H), 4.06-3.80 (m, 3H), 1.72-1.59 (m, 3H), 1.28-1.16 (m, 3H).

The retention time in chiral chromatographic column of compound 89 is short compared to that of compound 90, and the retention time in chiral chromatographic column of compound 90 is long compared to that of compound 89.

### Example 91 and Example 92

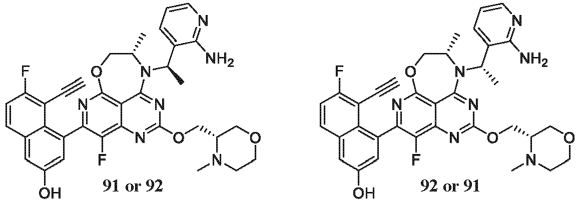

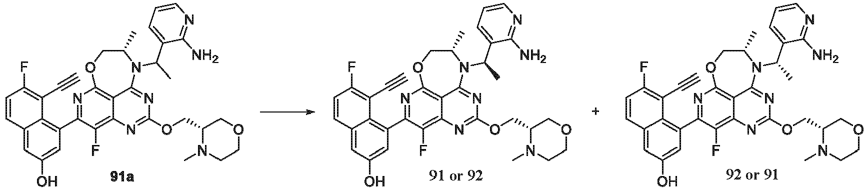

Referring to the preparation of compound **65g** in Example 65, compound **91a** was prepared by replacing methanol with (R)-(4-methylmorpholin-3-yl)methanol in step 2.

Compound 91a was sequentially purified by preparative liquid phase chromatography (YMC C18 10 µm 50 × 250 chromatographic column; acetonitrile-0.1% ammonium hydroxide (gradient elution of 40%-70%/0-60 min)) to give compound **91** (Rt 28 min, LC-MS: m/z = 654.34 (M + H)⁺) and compound **92** (Rt 30 min, LC-MS: m/z = 654.35 (M + H)⁺).

The retention time in chiral chromatographic column of compound 91 is short compared to that of compound 92, and the retention time in chiral chromatographic column of compound 92 is long compared to that of compound 91.

### Example 93 and Example 94

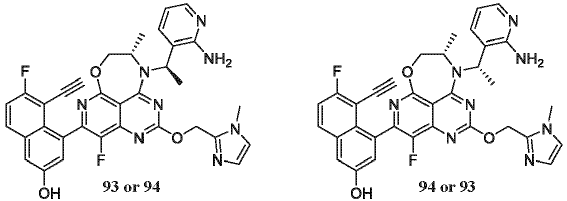

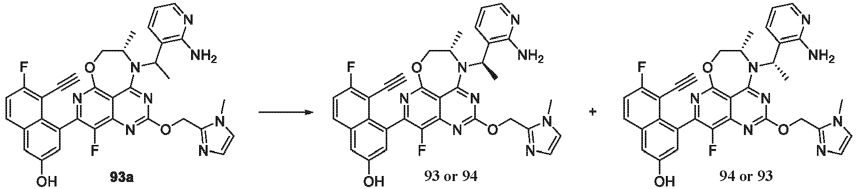

Referring to the preparation of compound **65g** in Example 65, compound **93a** was prepared by replacing methanol with 1-methyl-1*H*-imidazole-2-methanol in step 2.

Compound **93a** was sequentially purified by preparative liquid phase chromatography (YMC C18 10 µm 50 × 250 chromatographic column; acetonitrile-0.1% ammonium hydroxide (gradient elution of 35%-65%/0-60 min)) to give compound **93** (Rt 26 min, LC-MS: m/z = 635.35 (M + H)⁺) and compound **94** (Rt 30 min, LC-MS: m/z = 635.33 (M + H)⁺).

The retention time in chiral chromatographic column of compound 93 is short compared to that of compound 94, and the retention time in chiral chromatographic column of compound 94 is long compared to that of compound 93.

### Example 95 and Example 96

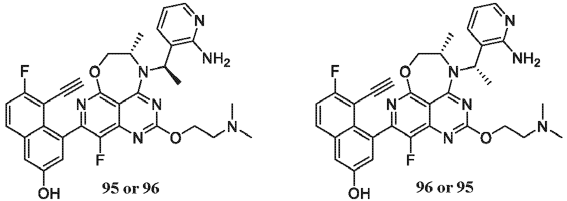

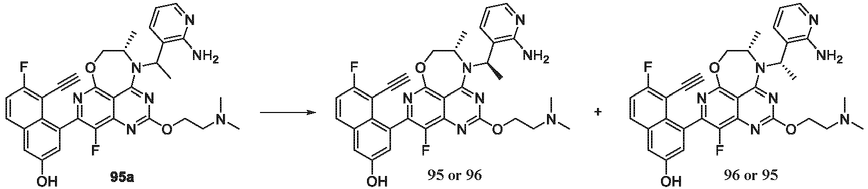

Referring to the preparation of compound **65g** in Example 65, compound **95a** was prepared by replacing methanol with *N,N-*dimethylethanolamine in step 2.

Compound **95a** was sequentially purified by preparative liquid phase chromatography (YMC C18 10 µm 50 × 250 chromatographic column; acetonitrile-0.1% ammonium hydroxide (gradient elution of 40%-70%/0-60 min)) to give compound **95** (Rt 25 min, LC-MS: m/z = 612.34 (M + H)⁺) and compound **96** (Rt 28 min, LC-MS: m/z = 612.34 (M + H)⁺).

The retention time in chiral chromatographic column of compound 95 is short compared to that of compound 96, and the retention time in chiral chromatographic column of compound 96 is long compared to that of compound 95.

### Example 97 and Example 98

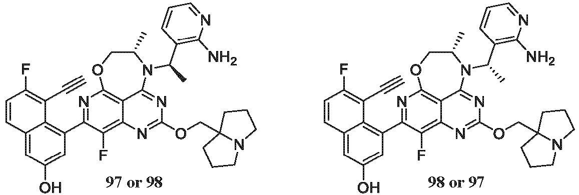

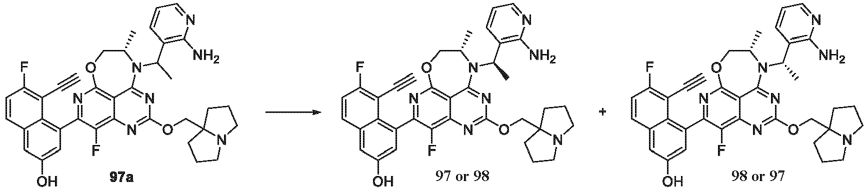

Referring to the preparation of compound **65g** in Example 65, compound **97a** was prepared by replacing methanol with (hexahydro-1*H*-pyrrolizin-7a-yl)methanol in step 2.

Compound **97a** was sequentially purified by preparative liquid phase chromatography (YMC C18 10 µm 50 × 250 chromatographic column; acetonitrile-0.1% ammonium hydroxide (gradient elution of 50%-70%-90%/0-20-60 min)) to give compound 97 (Rt 33 min, LC-MS: m/z = 664.36 (M + H)⁺) and compound 98 (Rt 36 min, LC-MS: m/z = 664.36 (M + H)⁺).

The retention time in chiral chromatographic column of compound 97 is short compared to that of compound 98, and the retention time in chiral chromatographic column of compound 98 is long compared to that of compound 97.

### Example 99 and Example 100

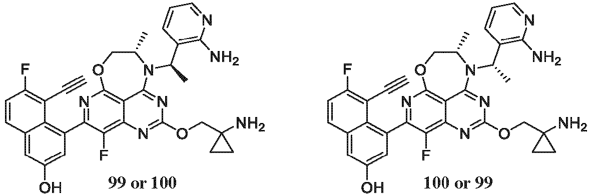

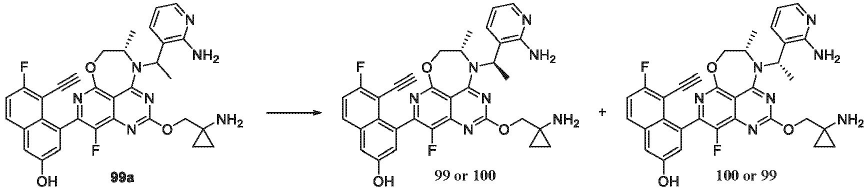

Referring to the preparation of compound **65g** in Example 65, compound **99a** was prepared by replacing methanol with 1-(*tert*-butoxycarbonylamino)cyclopropylmethanol in step 2.

Compound **99a** was sequentially purified by preparative liquid phase chromatography (YMC C18 10 µm 50 × 250 chromatographic column; acetonitrile-0.1% ammonium hydroxide (gradient elution of 40%-90%/0-60 min)) to give compound **99** (Rt 34 min, LC-MS: m/z = 610.32 (M + H)⁺) and compound **100** (Rt 39 min, LC-MS: m/z = 610.32 (M + H)⁺).

The retention time in chiral chromatographic column of compound 99 is short compared to that of compound 100, and the retention time in chiral chromatographic column of compound 100 is long compared to that of compound 99.

### Example 101 and Example 102

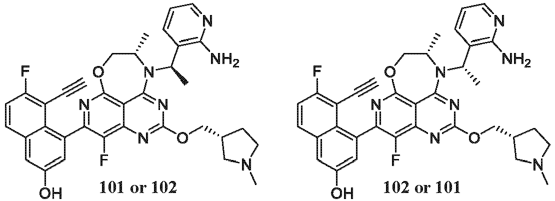

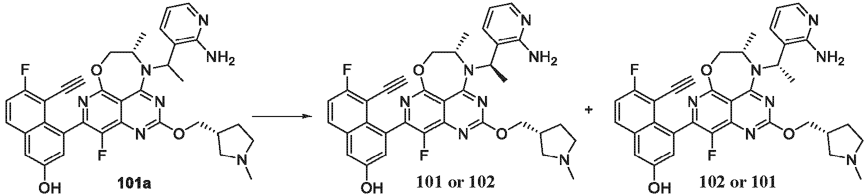

Referring to the preparation of compound 65g in Example 65, compound 101a was prepared by replacing methanol with (R)-(1-methylpyrrolidin-3-yl)methanol in step 2.

Compound **101a** was sequentially purified by preparative liquid phase chromatography (YMC C18 10 µm 50 × 250 chromatographic column; methanol-0.1% ammonium hydroxide (gradient elution of 40%-70%/0-60 min)) to give compound **101** (Rt 54 min, LC-MS: m/z = 638.37 (M + H)⁺) and compound **102** (Rt 58 min, LC-MS: m/z = 638.40 (M + H)⁺).

The retention time in chiral chromatographic column of compound 101 is short compared to that of compound 102, and the retention time in chiral chromatographic column of compound 102 is long compared to that of compound 101.

### Example 103 and Example 104

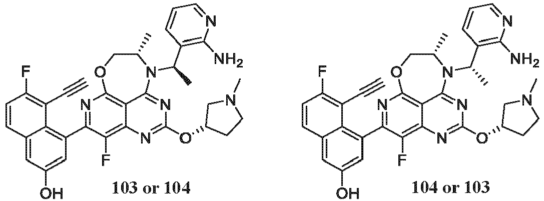

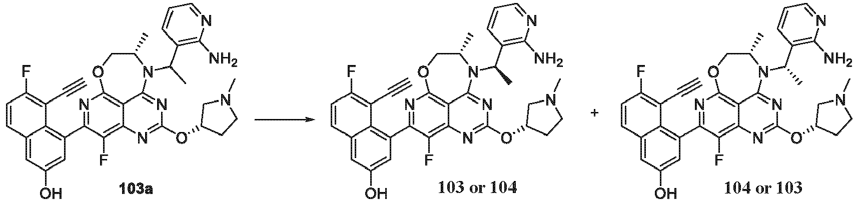

Referring to the preparation of compound **65g** in Example 65, compound **103a** was prepared by replacing methanol with (3*S*)-1-methylpyrrolidin-3-ol in step 2.

Compound **103a** was sequentially purified by preparative liquid phase chromatography (YMC TA C18 10 µm 30 × 250 chromatographic column; methanol-0.1% aqueous ammonium hydroxide solution (gradient elution of 40%-100%/0-60 min)) to give compound **103** (Rt 28.0 min, LC-MS: m/z = 624.36 (M + H)⁺) and compound **104** (Rt 29.9 min, LC-MS: m/z = 624.36 (M + H)⁺).

The retention time in chiral chromatographic column of compound 103 is short compared to that of compound 104, and the retention time in chiral chromatographic column of compound 104 is long compared to that of compound 103.

### Example 105 and Example 106

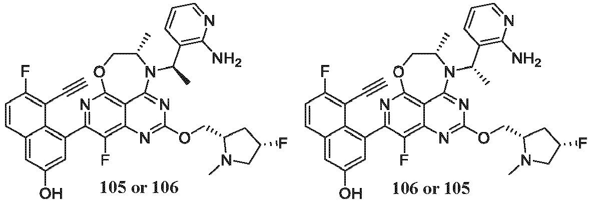

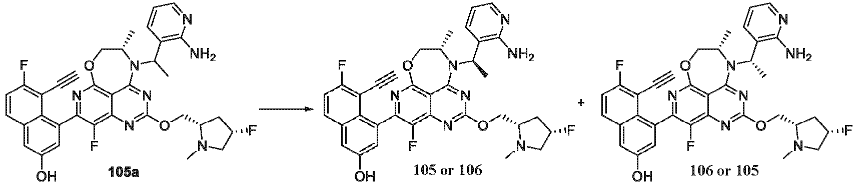

Referring to the preparation of compound **65g** in Example 65, compound **105a** was prepared by replacing methanol with ((2S,4S)-4-fluoro-1-methylpyrrolidin-2-yl)methanol in step 2.

Compound **105a** was sequentially purified by preparative liquid phase chromatography (YMC TA C18 10 µm 50 × 250 chromatographic column; methanol-0.1% aqueous ammonium hydroxide solution (gradient elution of 40%-70%/0-60 min)) to give compound **105** (Rt 37.2 min, LC-MS: m/z = 656.33 (M + H)⁺) and compound **106** (Rt 47.1 min, LC-MS: m/z = 656.33 (M + H)⁺).

The retention time in chiral chromatographic column of compound 105 is short compared to that of compound 106, and the retention time in chiral chromatographic column of compound 106 is long compared to that of compound 105.

### Example 107 and Example 108

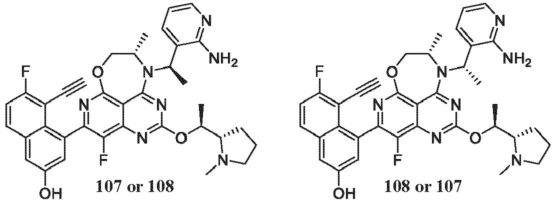

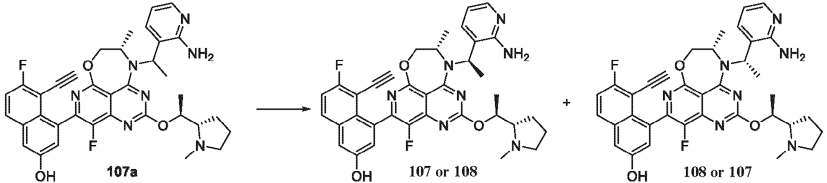

Referring to the preparation of compound **65g** in Example 65, compound **107a** was prepared by replacing methanol with (*S*)-1-((*S*)-1-methylpyrrolidin-2-yl)ethanol in step 2.

Compound **107a** was sequentially purified by preparative liquid phase chromatography (YMC TA C18 10 µm 30 × 250 chromatographic column; methanol-0.1% aqueous ammonium hydroxide solution (gradient elution of 40%-100%/0-60 min)) to give compound **107** (Rt 31.8 min, LC-MS: m/z = 652.36 (M + H)⁺) and compound **108** (Rt 34.8 min, LC-MS: m/z = 652.36 (M + H)⁺).

The retention time in chiral chromatographic column of compound 107 is short compared to that of compound 108, and the retention time in chiral chromatographic column of compound 108 is long compared to that of compound 107.

### Example 109 and Example 110

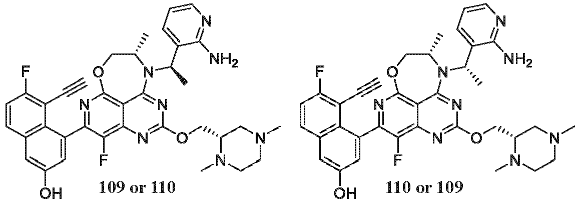

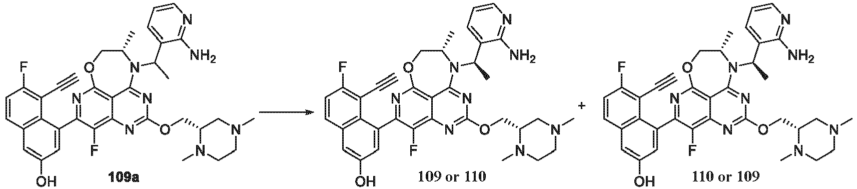

Referring to the preparation of compound 65g in Example 65, compound 109a was prepared by replacing methanol with (*S*)-(1,4-dimethylpiperazin-2-yl)methanol in step 2.

Compound **109a** was sequentially purified by preparative liquid phase chromatography (YMC TA C18 10 µm 50 × 250 chromatographic column; methanol-0.1% aqueous ammonium hydroxide solution (gradient elution of 40%-90%/0-60 min)) to give compound **109** (Rt 32.4 min, LC-MS: m/z = 667.35 (M + H)⁺) and compound **110** (Rt 37.1 min, LC-MS: m/z = 667.35 (M + H)⁺).

The retention time in chiral chromatographic column of compound 109 is short compared to that of compound 110, and the retention time in chiral chromatographic column of compound 110 is long compared to that of compound 109.

### Example 111 and Example 112

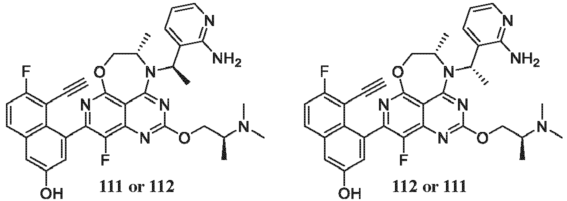

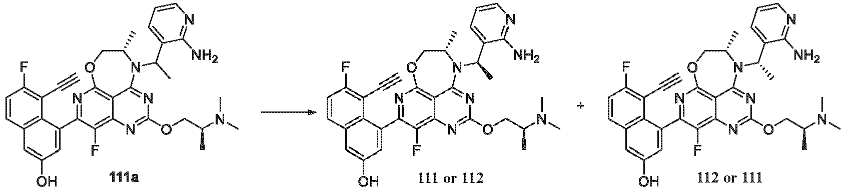

Referring to the preparation of compound **65g** in Example 65, compound **111a** was prepared by replacing methanol with (*S*)-2-(dimethylamino)propanol in step 2.

Compound **111a** was sequentially purified by preparative liquid phase chromatography (YMC TA C18 10 µm 30 × 250 chromatographic column; methanol-0.1% aqueous ammonium hydroxide solution (gradient elution of 40%-100%/0-60 min)) to give compound **111** (Rt 25.0 min, LC-MS: m/z = 626.43 (M + H)⁺) and compound **112** (Rt 27.1 min, LC-MS: m/z = 626.43 (M + H)⁺).

**Compound 111:** ¹H-NMR (500 MHz, DMSO-*d₆*), *δ*: 8.07-7.91 (m, 2H), 7.71 (d, *J =* 7.4 Hz, 1H), 7.50-7.41 (m, 1H), 7.40-7.34 (m, 1H), 7.25-7.10 (m, 1H), 6.75-6.67 (m, 1H), 6.60-6.44 (m, 1H), 5.64-5.47 (m, 2H), 4.60-4.43 (m, 2H), 4.35-3.76 (m, 5H), 3.02-2.93 (m, 1H), 2.24 (s, 6H), 1.68-1.57 (m, 3H), 1.09-1.00 (m, 3H), 0.66-0.52 (m, 3H).

**Compound 112:** ¹H-NMR (500 MHz, DMSO-*d₆*), *δ*: 8.06-7.88 (m, 2H), 7.78-7.69 (m, 1H), 7.50-7.42 (m, 1H), 7.37 (d, *J=* 2.5 Hz, 1H), 7.23-7.12 (m, 1H), 6.75-6.67 (m, 1H), 6.59-6.41 (m, 1H), 5.71-5.55 (m, 2H), 4.55-4.17 (m, 3H), 4.11-3.81 (m, 4H), 3.01-2.91 (m, 1H), 2.24 (s, 6H), 1.70-1.60 (m, 3H), 1.26-1.21 (m, 3H), 1.07-1.00 (m, 3H).

The retention time in chiral chromatographic column of compound 111 is short compared to that of compound 112, and the retention time in chiral chromatographic column of compound 112 is long compared to that of compound 111.

### Example 113 and Example 114

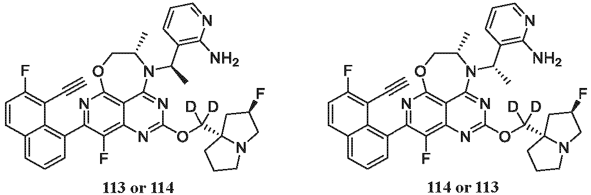

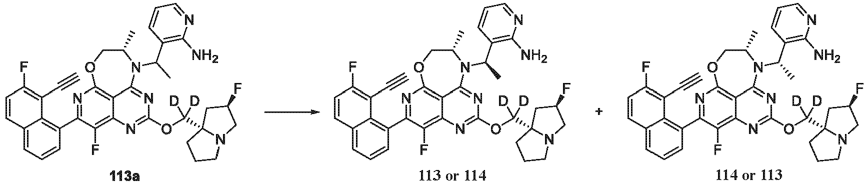

Referring to the preparation of compound **65f** in Example 65, compound **113a** was prepared by replacing methanol with ((2*R*,7a*S*)-2-fluorotetrahydro-1*H*-pyrrolizin-7a(5*H*)-yl)methane-d₂-ol in step 2 and replacing **10g** with ((2-fluoro-8-(4,4,5,5-tetramethyl-1,3,2-dioxaborolan-2-yl)naphthalen-1-yl)ethynyl)triisopropylsilane in step 4.

Compound **113a** was sequentially purified by preparative liquid phase chromatography (YMC TA C18 10 µm 50 × 250 chromatographic column; methanol-0.1% aqueous ammonium hydroxide solution (gradient elution of 60%-90%/0-60 min)) to give compound **113** (Rt 35.1 min, LC-MS: m/z = 668.39 (M + H)⁺) and compound **114** (Rt 37.1 min, LC-MS: m/z = 668.35 (M + H)⁺).

**Compound 113:** ¹H-NMR (500 MHz, DMSO-*d₆*), *δ*: 8.39-8.12 (m, 2H), 8.09-7.92 (m, 1H), 7.81-7.51 (m, 4H), 6.80-6.64 (m, 1H), 6.62-6.38 (m, 1H), 5.73-5.52 (m, 2H), 5.43-5.16 (m, 1H), 4.69-4.42 (m, 1H), 4.38-4.20 (m, 1H), 4.19-3.84 (m, 2H), 3.16-2.96 (m, 3H), 2.92-2.76 (m, 1H), 2.25-1.96 (m, 3H), 1.92-1.71 (m, 3H), 1.70-1.53 (m, 3H), 0.71-0.47 (m, 3H).

**Compound 114:** ¹H-NMR (500 MHz, DMSO-*d₆*), *δ*: 8.32-8.09 (m, 2H), 8.08-7.94 (m, 1H), 7.82-7.50 (m, 4H), 6.80-6.63 (m, 1H), 6.62-6.35 (m, 1H), 5.83-5.53 (m, 2H), 5.45-5.17 (m, 1H), 4.44-4.27 (m, 1H), 4.21-3.78 (m, 3H), 3.18-2.99 (m, 3H), 2.92-2.75 (m, 1H), 2.22-1.96 (m, 3H), 1.92-1.72 (m, 3H), 1.72-1.57 (m, 3H), 1.32-1.15 (m, 3H).

The retention time in chiral chromatographic column of compound 113 is short compared to that of compound 114, and the retention time in chiral chromatographic column of compound 114 is long compared to that of compound 113.

### Example 115

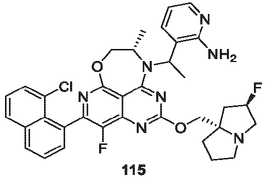

Referring to the preparation of compound **47b** in Example 47, compound **115** was prepared by replacing ((2-fluoro-8-(4,4,5,5-tetramethyl-1,3,2-dioxaborolan-2-yl)naphthalen-1-yl)ethynyl)triisopropylsilane with 2-(8-chloronaphthalen-1-yl)-4,4,5,5-tetramethyl-1,3,2-dioxaborolane in step 1. LC-MS: m/z = 658.32 (M + H)⁺.

### Example 116 and Example 117

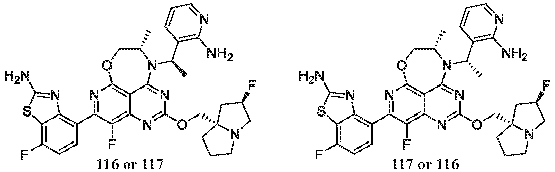

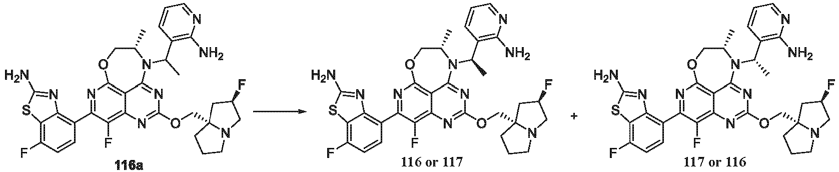

Referring to the preparation of compound **47b** in Example 47, compound **116a** was prepared by replacing ((2-fluoro-8-(4,4,5,5-tetramethyl-1,3,2-dioxaborolan-2-yl)naphthalen-1-yl)ethynyl)triisopropylsilane with (2-amino-7-fluorobenzo[*d*]thiazol-4-yl)boronic acid in step 1.

Compound **116a** was sequentially purified by preparative liquid phase chromatography (YMC C18 10 µm 50 × 250 chromatographic column; acetonitrile-0.1% ammonium hydroxide (gradient elution of 50%-70%-90%/0-20-60 min)) to give compound **116** (Rt 41 min, LC-MS: m/z = 664.34 (M + H)⁺) and compound **117** (Rt 44 min, LC-MS: m/z = 664.33 (M + H)⁺).

The retention time in chiral chromatographic column of compound 116 is short compared to that of compound 117, and the retention time in chiral chromatographic column of compound 117 is long compared to that of compound 116.

### Example 118 and Example 119

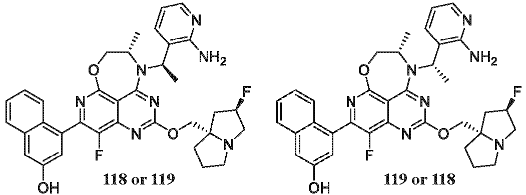

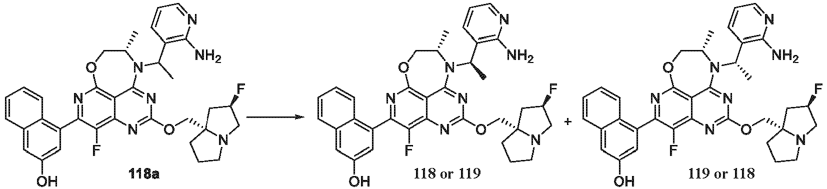

Referring to the preparation of compound **47b** in Example 47, compound **118a** was prepared by replacing ((2-fluoro-8-(4,4,5,5-tetramethyl-1,3,2-dioxaborolan-2-yl)naphthalen-1-yl)ethynyl)triisopropylsilane with 4-(4,4,5,5-tetramethyl-1,3,2-dioxaborolan-2-yl)naphthalen-2-ol in step 1.

Compound **118a** was sequentially purified by preparative liquid phase chromatography (YMC C18 10 µm 50 × 250 chromatographic column; acetonitrile-0.1% ammonium hydroxide (gradient elution of 50%-70%-90%/0-20-60 min)) to give compound **118** (Rt 34 min, LC-MS: m/z = 640.40 (M + H)⁺) and compound **119** (Rt 39 min, LC-MS: m/z = 640.41 (M + H)⁺).

The retention time in chiral chromatographic column of compound 118 is short compared to that of compound 119, and the retention time in chiral chromatographic column of compound 119 is long compared to that of compound 118.

### Example 120 and Example 121

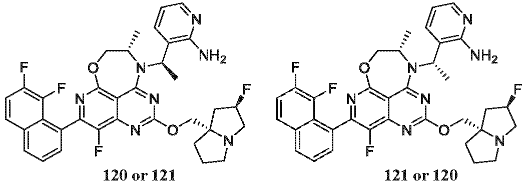

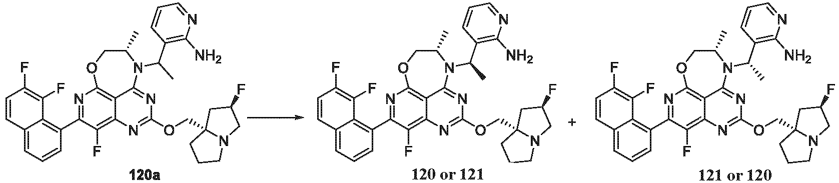

Referring to the preparation of compound **47b** in Example 47, compound **120a** was prepared by replacing ((2-fluoro-8-(4,4,5,5-tetramethyl-1,3,2-dioxaborolan-2-yl)naphthalen-1-yl)ethynyl)triisopropylsilane with 2-(7,8-difluoronaphthalen-1-yl)-4,4,5,5-tetramethyl-1,3,2-dioxaborolane in step 1.

Compound **120a** was sequentially purified by preparative liquid phase chromatography (YMC C18 10 µm 50 × 250 chromatographic column; acetonitrile-0.1% ammonium hydroxide (gradient elution of 50%-70%-90%/0-20-60 min)) to give compound **120** (Rt 43 min, LC-MS: m/z = 660.35 (M + H)⁺) and compound **121** (Rt 47 min, LC-MS: m/z = 660.35 (M + H)⁺).

**Compound 120:** ¹H-NMR (500 MHz, DMSO-*d₆*), *δ*: 8.31-8.11 (m, 1H), 8.10-7.90 (m, 2H), 7.90-7.43 (m, 4H), 6.98-6.34 (m, 2H), 5.88-5.50 (m, 2H), 5.46-5.15 (m, 1H), 4.81-3.86 (m, 5H), 3.24-2.74 (m, 4H), 2.26-1.51 (m, 9H), 0.78-0.40 (m, 3H).

**Compound 121:** ¹H-NMR (500 MHz, DMSO-*d₆*), *δ*: 8.30-7.90 (m, 3H), 7.89-7.44 (m, 4H), 6.79-6.42 (m, 2H), 5.76-5.50 (m, 2H), 5.44-5.16 (m, 1H), 4.49-3.84 (m, 5H), 3.20-2.73 (m,4H), 2.27-1.54 (m, 9H), 1.36-1.11 (m, 3H).

The retention time in chiral chromatographic column of compound 120 is short compared to that of compound 121, and the retention time in chiral chromatographic column of compound 121 is long compared to that of compound 120.

### Example 122 and Example 123

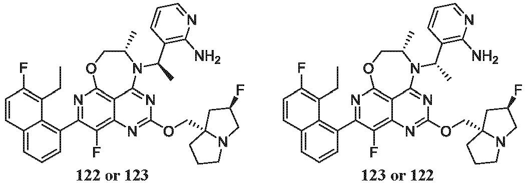

Compound **47** (30 mg) and Pd/C (10%, 24 mg) were dispersed in methanol (6 mL), and the mixture was purged with hydrogen three times and stirred for reaction at room temperature overnight under a hydrogen atmosphere. After the reaction was completed, filtration was performed, and the reaction solution was purified by preparative liquid phase chromatography (YMC C18 10 µm 50 × 250 chromatographic column: acetonitrile-0.1% aqueous formic acid solution (gradient elution of 5%-35%/0-60 min)) to give compound **122.** LC-MS: m/z = 670.38 (M + H)⁺.

Referring to the preparation of compound **122,** compound **123** was prepared by replacing compound **47** with compound **48.** LC-MS: m/z = 670.38 (M + H)⁺.

All chiral carbon atom configurations of compound 122 are identical to those of compound 47. All chiral carbon atom configurations of compound 123 are identical to those of compound 48.

### Example 124 and Example 125

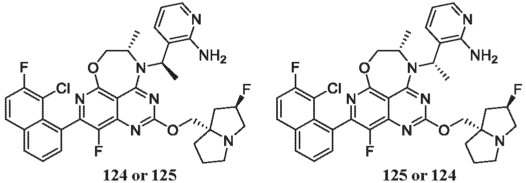

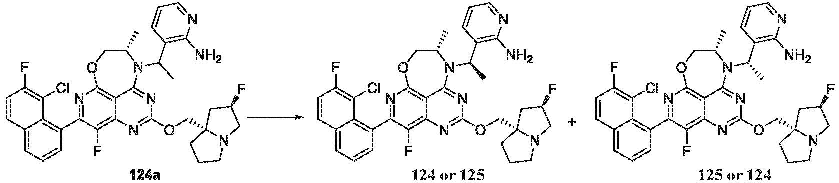

Referring to the preparation of compound **47b** in Example 47, compound **124a** was prepared by replacing ((2-fluoro-8-(4,4,5,5-tetramethyl-1,3,2-dioxaborolan-2-yl)naphthalen-1-yl)ethynyl)triisopropylsilane with 2-(8-chloro-7-fluoronaphthalen-1-yl)-4,4,5,5-tetramethyl-1,3,2-dioxaborolane in step 1.

Compound **124a** was purified by preparative liquid phase chromatography (YMC-TA-C18, 10 µm, 50 × 250 chromatographic column; methanol-0.1% aqueous ammonium hydroxide solution (gradient elution of 40%-100%/0-50 min)) to give compound **125** (Rt 39 min, LC-MS: m/z = 676.30 (M + H)⁺) and purified by preparative liquid phase chromatography (YMC-TA-C18, 10 µm, 50 × 250 chromatographic column; methanol-0.1% aqueous ammonium hydroxide solution (gradient elution of 50%-100%/0-40 min)) to give compound **124** (Rt 28 min, LC-MS: m/z = 676.32 (M + H)⁺).

### Example 126 and Example 127

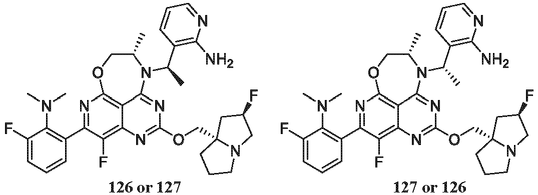

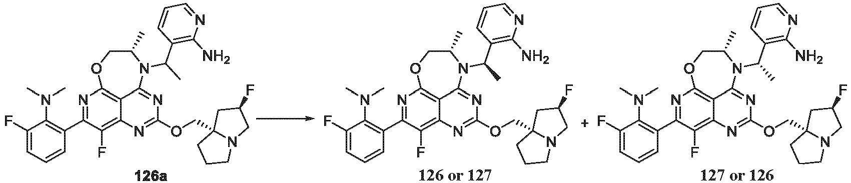

Referring to the preparation of compound **47b** in Example 47, compound **126a** was prepared by replacing ((2-fluoro-8-(4,4,5,5-tetramethyl-1,3,2-dioxaborolan-2-yl)naphthalen-1-yl)ethynyl)triisopropylsilane with (2-(dimethylamino)-3-fluorophenyl)boronic acid in step 1.

Compound **126a** was purified by preparative liquid phase chromatography (YMC-AQ-C18, 10 µm, 50 × 250 chromatographic column; acetonitrile-0.1% aqueous TFA solution (gradient elution of 10%-70%/0-60 min)) to give compound **126** (Rt 22 min, LC-MS: m/z = 635.40 (M + H)⁺) and purified by preparative liquid phase chromatography (YMC-AQ-C18, 10 µm, 50 × 250 chromatographic column; methanol-0.1% aqueous TFA solution (gradient elution of 10%-70%/0-60 min)) to give compound **127** (Rt 43 min, LC-MS: m/z = 635.40 (M + H)⁺).

### Example 128 and Example 129

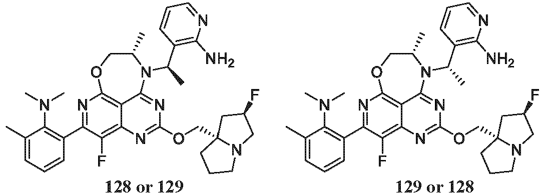

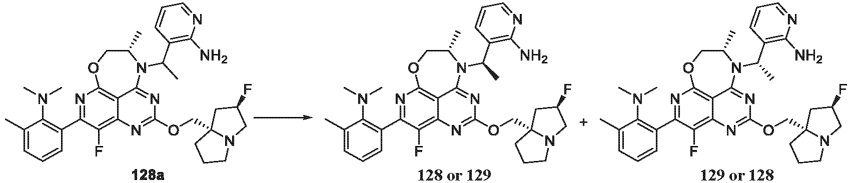

Referring to the preparation of compound **47b** in Example 47, compound **128a** was prepared by replacing ((2-fluoro-8-(4,4,5,5-tetramethyl-1,3,2-dioxaborolan-2-yl)naphthalen-1-yl)ethynyl)triisopropylsilane with (2-(dimethylamino)-3-methylphenyl)boronic acid in step 1.

Compound **128a** was sequentially purified by preparative liquid phase chromatography (YMC TA C18 10 µm 30 × 250 chromatographic column; methanol-0.1% aqueous ammonium hydroxide solution (gradient elution of 40%-100%/0-60 min)) to give compound **128** (Rt 29.5 min, LC-MS: m/z = 631.45 (M + H)⁺) and compound **129** (Rt 33.4 min, LC-MS: m/z = 631.46 (M + H)⁺).

The retention time in chiral chromatographic column of compound 128 is short compared to that of compound 129, and the retention time in chiral chromatographic column of compound 129 is long compared to that of compound 128.

### Example 130 and Example 131

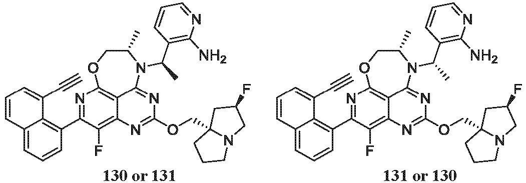

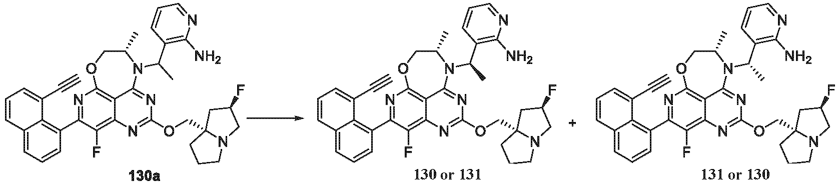

Referring to the preparation of compound **47c** in Example 47, compound **130a** was prepared by replacing ((2-fluoro-8-(4,4,5,5-tetramethyl-1,3,2-dioxaborolan-2-yl)naphthalen-1-yl)ethynyl)triisopropylsilane with triisopropyl((8-(4,4,5,5-tetramethyl-1,3,2-dioxaborolan-2-yl)naphthalen-1-yl)ethynyl)silane in step 1.

Compound **130a** was sequentially purified by preparative liquid phase chromatography (YMC TA C18 10 µm 30 × 250 chromatographic column; methanol-0.1% aqueous formic acid solution (gradient elution of 20%-80%/0-60 min)) to give compound **130** (Rt 16.9 min, LC-MS: m/z = 648.36 (M + H)⁺) and compound **131** (Rt 18.0 min, LC-MS: m/z = 648.36 (M + H)⁺).

The retention time in chiral chromatographic column of compound 130 is short compared to that of compound 131, and the retention time in chiral chromatographic column of compound 131 is long compared to that of compound 130.

### Example 132 and Example 133

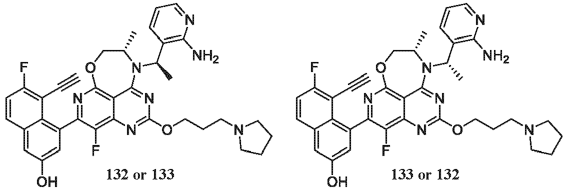

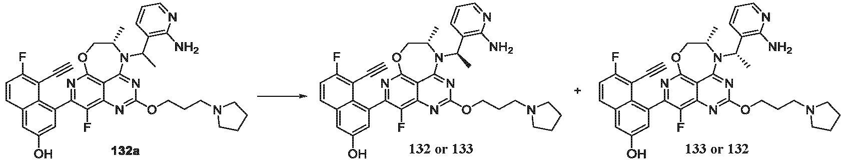

Referring to the preparation of compound **65g** in Example 65, compound **132a** was prepared by replacing methanol with 3-(1-pyrrolidinyl)-1-propanol in step 2.

Compound **132a** was purified by preparative liquid phase chromatography (Novasep C18 250 × 50 mm,10 µm chromatographic column; methanol-0.1% aqueous ammonium hydroxide solution (gradient elution of 40%-90%/0-60 min)) and then purified by preparative liquid phase chromatography (Novasep C18 250 × 50 mm,10 µm chromatographic column; acetonitrile-0.1% aqueous acetic acid solution (gradient elution of 15%-45%/0-60 min)) to give compound **132** (Rt 40 min, LC-MS: m/z = 652.37 (M + H)⁺) and compound **133** (Rt 38 min, LC-MS: m/z = 652.36 (M + H)⁺).

The retention time in chiral chromatographic column of compound 133 is short compared to that of compound 132, and the retention time in chiral chromatographic column of compound 132 is long compared to that of compound 133.

### Example 134 and Example 135

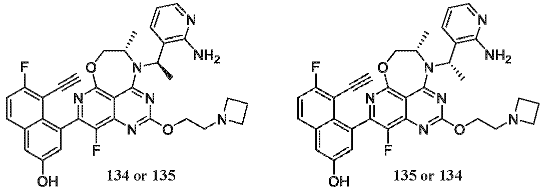

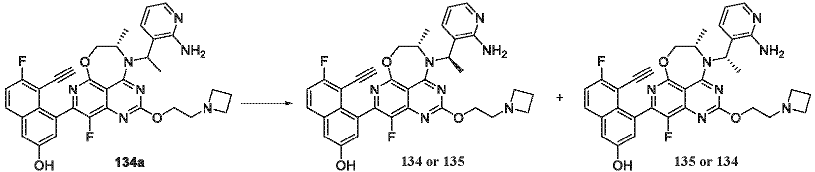

Referring to the preparation of compound **65g** in Example 65, compound **134a** was prepared by replacing methanol with 2-(azetidin-1-yl)ethanol in step 2.

Compound **134a** was sequentially purified by preparative liquid phase chromatography (YMC TA C18 10 µm 50 × 250 chromatographic column; methanol-0.1% aqueous ammonium hydroxide solution (gradient elution of 40%-60%-90%/0-15-60 min)) to give compound **134** (Rt 31.56 min, LC-MS: m/z = 624.33 (M + H)⁺) and compound **135** (Rt 35.48 min, LC-MS: m/z = 624.33 (M + H)⁺).

The retention time in chiral chromatographic column of compound 134 is short compared to that of compound 135, and the retention time in chiral chromatographic column of compound 135 is long compared to that of compound 134.

### Example 136 and Example 137

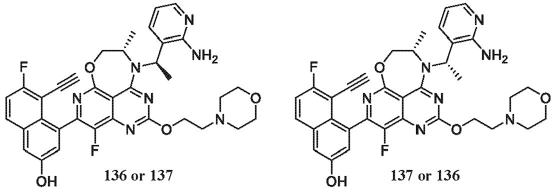

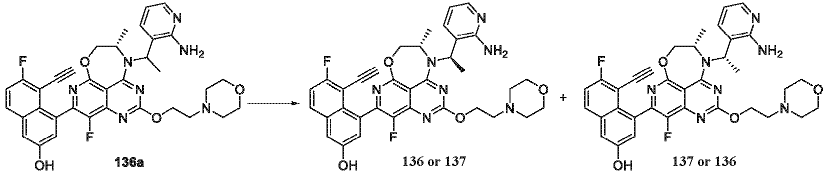

Referring to the preparation of compound **65g** in Example 65, compound **136a** was prepared by replacing methanol with 2-morpholinoethanol in step 2.

Compound **136a** was sequentially purified by preparative liquid phase chromatography (YMC TA C18 10 µm 30 × 250 chromatographic column; methanol-0.1% aqueous ammonium hydroxide solution (gradient elution of 30%-90%/0-60 min)) to give compound **136** (Rt 29 min, LC-MS: m/z = 654.34 (M + H)⁺) and compound **137** (Rt 33 min, LC-MS: m/z = 654.34 (M + H)⁺).

The retention time in chiral chromatographic column of compound 136 is short compared to that of compound 137, and the retention time in chiral chromatographic column of compound 137 is long compared to that of compound 136.

### Example 138 and Example 139

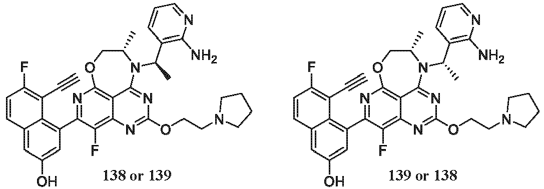

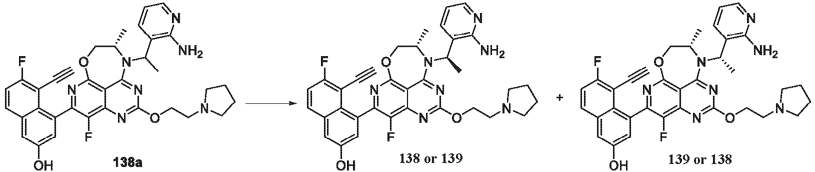

Referring to the preparation of compound **65g** in Example 65, compound **138a** was prepared by replacing methanol with 1-(2-hydroxyethyl)pyrrolidine in step 2.

Compound **138a** was sequentially purified by preparative liquid phase chromatography (YMC TA C18 10 µm 50 × 250 chromatographic column; methanol-0.1% aqueous ammonium hydroxide solution (gradient elution of 50%-80%/0-60 min)) to give compound **138** (Rt 26.21 min, LC-MS: m/z = 638.38 (M + H)⁺) and compound **139** (Rt 38.27 min, LC-MS: m/z = 638.39 (M + H)⁺).

The retention time in chiral chromatographic column of compound 138 is short compared to that of compound 139, and the retention time in chiral chromatographic column of compound 139 is long compared to that of compound 138.

### Example 140 and Example 141

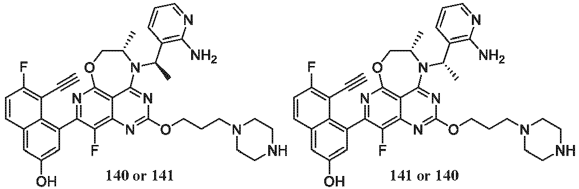

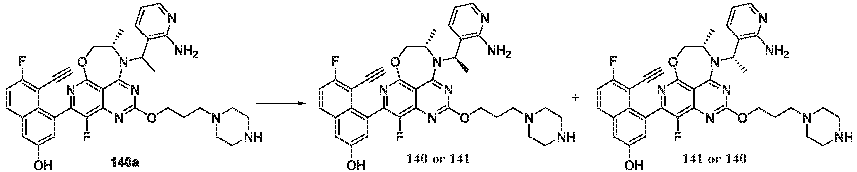

Referring to the preparation of compound **65g** in Example 65, compound **140a** was prepared by replacing methanol with 1-*tert*-butoxycarbonyl-4-(3-hydroxypropyl)piperazine in step 2.

Compound **140a** was sequentially purified by preparative liquid phase chromatography (YMC TA C18 10 µm 50 × 250 chromatographic column; methanol-0.1% aqueous ammonium hydroxide solution (gradient elution of 35%-65%-65%/0-60-70 min)) to give compound **140** (Rt 56.35 min, LC-MS: m/z = 667.38 (M + H)⁺) and compound **141** (Rt 62.30 min, LC-MS: m/z = 667.36 (M + H)⁺).

The retention time in chiral chromatographic column of compound 140 is short compared to that of compound 141, and the retention time in chiral chromatographic column of compound 141 is long compared to that of compound 140.

### Example 142 and Example 143

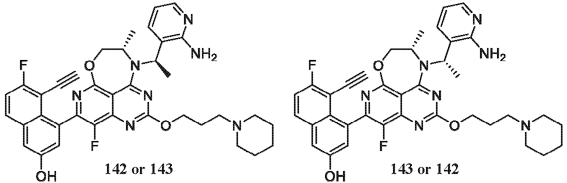

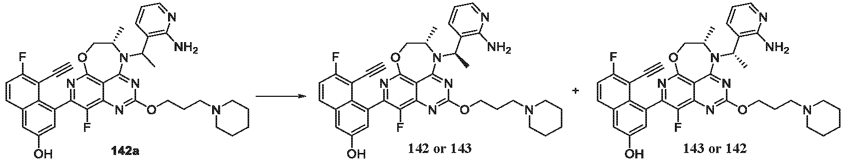

Referring to the preparation of compound **65g** in Example 65, compound **142a** was prepared by replacing methanol with 1-piperidinepropanol in step 2.

Compound **142a** was sequentially purified by preparative liquid phase chromatography (Novasep C18 250 × 50 mm,10 µm chromatographic column; methanol-0.1% aqueous ammonium hydroxide solution (gradient elution of 45%-85%/0-60 min)) and then by preparative liquid phase chromatography (Novasep C18 250 × 50 mm,10 µm chromatographic column; acetonitrile-0.1% aqueous acetic acid solution (gradient elution of 15%-45%/0-60 min)) to give compound **142** (Rt 28 min, LC-MS: m/z = 666.38 (M + H)⁺) and compound **143** (Rt 29 min, LC-MS: m/z = 666.40 (M + H)⁺).

The retention time in chiral chromatographic column of compound 142 is short compared to that of compound 143, and the retention time in chiral chromatographic column of compound 143 is long compared to that of compound 142.

### Example 144 and Example 145

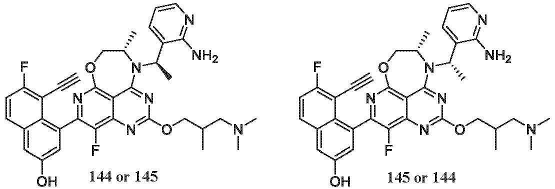

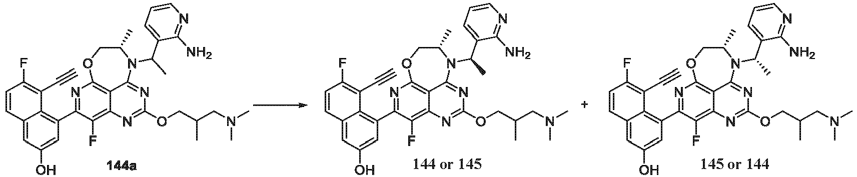

Referring to the preparation of compound **65g** in Example 65, compound **144a** was prepared by replacing methanol with 3-(dimethylamino)-2-methyl-1-propanol in step 2.

Compound **144a** was sequentially purified by preparative liquid phase chromatography (YMC TA C18 10 µm 30 × 250 chromatographic column; methanol-0.1% aqueous ammonium hydroxide solution (gradient elution of 40%-70%/0-60 min)) to give compound **144** (Rt 36.54 min, LC-MS: m/z = 640.38 (M + H)⁺) and compound **145** (Rt 47.57 min, LC-MS: m/z = 640.38 (M + H)⁺).

The retention time in chiral chromatographic column of compound 144 is short compared to that of compound 145, and the retention time in chiral chromatographic column of compound 145 is long compared to that of compound 144.

### Example 146 and Example 147

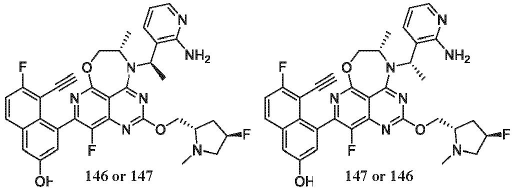

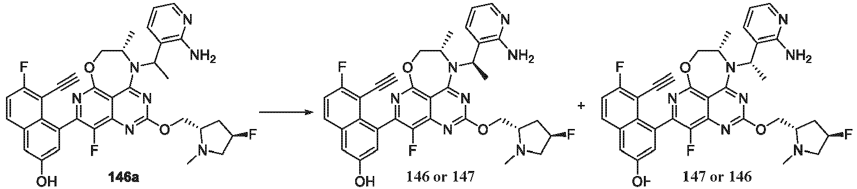

Referring to the preparation of compound **65g** in Example 65, compound **146a** was prepared by replacing methanol with ((2*S*,4*R*)-4-fluoro-1-methylpyrrolidin-2-yl)methanol in step 2.

Compound **146a** was sequentially purified by preparative liquid phase chromatography (YMC TA C18 10 µm 30 × 250 chromatographic column; methanol-0.1% aqueous ammonium hydroxide solution (gradient elution of 40%-70%/0-60 min)) to give compound **146** (Rt 35.37 min, LC-MS: m/z = 656.32 (M + H)⁺) and compound **147** (Rt 42.08 min, LC-MS: m/z = 656.33 (M + H)⁺).

The retention time in chiral chromatographic column of compound 146 is short compared to that of compound 147, and the retention time in chiral chromatographic column of compound 147 is long compared to that of compound 146.

### Example 148 and Example 149

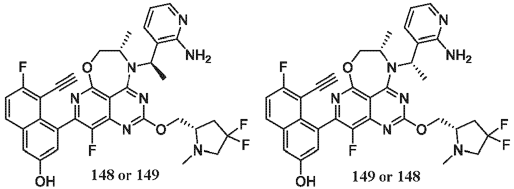

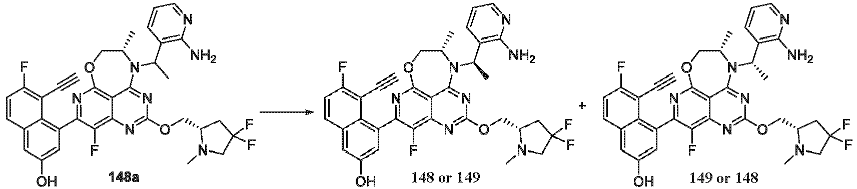

Referring to the preparation of compound **65g** in Example 65, compound **148a** was prepared by replacing methanol with (*S*)-(4,4-difluoro-1-methylpyrrolidin-2-yl)methanol in step 2.

Compound **148a** was sequentially purified by preparative liquid phase chromatography (YMC TA C18 10 µm 30 × 250 chromatographic column; methanol-0.1% aqueous ammonium hydroxide solution (gradient elution of 40%-70%/0-60 min)) to give compound **148** (Rt 30.18 min, LC-MS: m/z = 674.32 (M + H)⁺) and compound **149** (Rt 49.50 min, LC-MS: m/z = 674.32 (M + H)⁺).

The retention time in chiral chromatographic column of compound 148 is short compared to that of compound 149, and the retention time in chiral chromatographic column of compound 149 is long compared to that of compound 148.

### Example 150 and Example 151

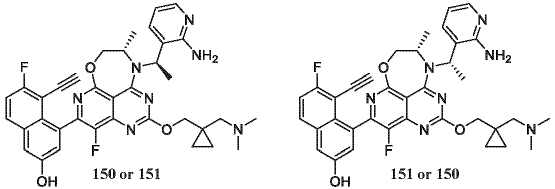

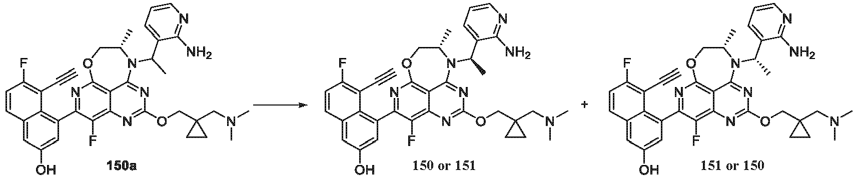

Referring to the preparation of compound **65g** in Example 65, compound **150a** was prepared by replacing methanol with (1-((dimethylamino)methyl)cyclopropyl)methanol in step 2.

Compound **150a** was sequentially purified by preparative liquid phase chromatography (YMC TA C18 10 µm 30 × 250 chromatographic column; methanol-0.1% aqueous ammonium hydroxide solution (gradient elution of 40%-100%/0-60 min)) to give compound **150** (Rt 37 min, LC-MS: m/z = 652.36 (M + H)⁺) and compound **151** (Rt 38 min, LC-MS: m/z = 652.34 (M + H)⁺).

The retention time in chiral chromatographic column of compound 150 is short compared to that of compound 151, and the retention time in chiral chromatographic column of compound 151 is long compared to that of compound 150.

### Example 152 and Example 153

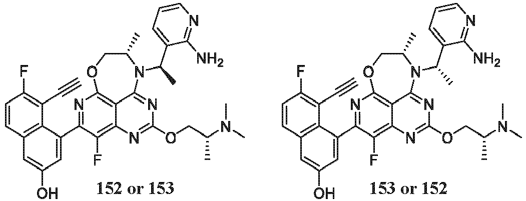

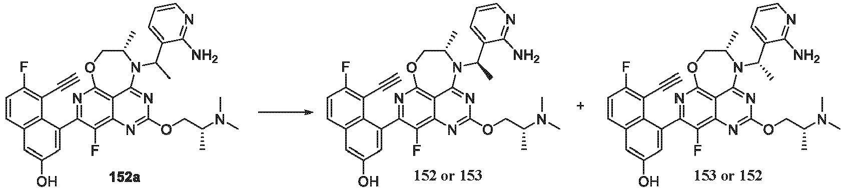

Referring to the preparation of compound **65g** in Example 65, compound **152a** was prepared by replacing methanol with (*R*)-2-(dimethylamino)propanol in step 2.

Compound **152a** was sequentially purified by preparative liquid phase chromatography (YMC TA C18 10 µm 30 × 250 chromatographic column; methanol-0.1% aqueous formic acid solution (gradient elution of 20%-80%/0-60 min)) to give compound **152** (Rt 13.9 min, LC-MS: m/z = 626.33 (M + H)⁺) and compound **153** (Rt 16.7 min, LC-MS: m/z = 626.33 (M + H)⁺).

The retention time in chiral chromatographic column of compound 152 is short compared to that of compound 153, and the retention time in chiral chromatographic column of compound 153 is long compared to that of compound 152.

### Example 154 and Example 155

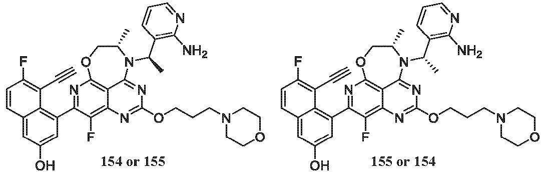

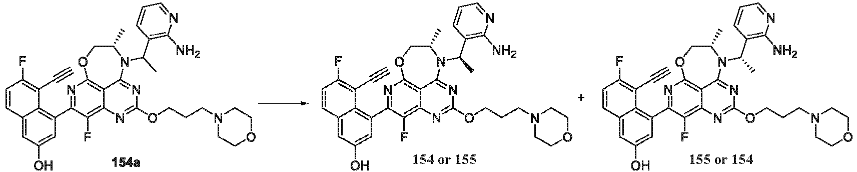

Referring to the preparation of compound **65g** in Example 65, compound **154a** was prepared by replacing methanol with 3-(4-morpholinyl)-1-propanol in step 2.

Compound **154a** was sequentially purified by preparative liquid phase chromatography (YMC TA C18 10 µm 30 × 250 chromatographic column; methanol-0.1% aqueous formic acid solution (gradient elution of 20%-80%/0-60 min)) to give compound **154** (Rt 13.8 min, LC-MS: m/z = 668.34 (M + H)⁺) and compound **155** (Rt 15.4 min, LC-MS: m/z = 668.32 (M + H)⁺).

The retention time in chiral chromatographic column of compound 154 is short compared to that of compound 155, and the retention time in chiral chromatographic column of compound 155 is long compared to that of compound 154.

### Example 156 and Example 157

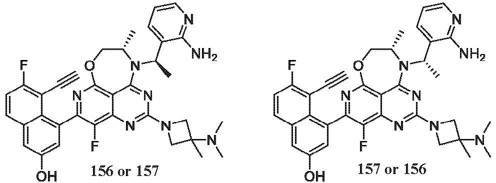

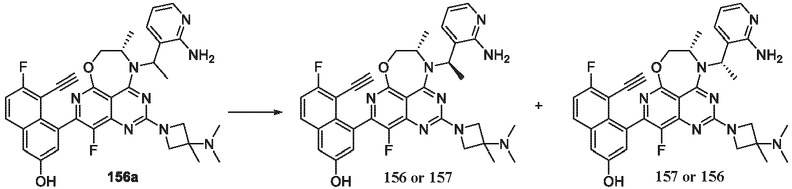

Referring to the preparation of compound **65g** in Example 65, compound **156a** was prepared by replacing methanol with *N*,*N*,3-trimethylazetidin-3-amine dihydrochloride in step 2.

Compound **156a** was sequentially purified by preparative liquid phase chromatography (YMC TA C18 10 µm 30 × 250 chromatographic column; methanol-0.1% aqueous acetic acid solution (gradient elution of 5%-45%/0-80 min)) to give compound **156** (Rt 55.26 min, LC-MS: m/z = 637.38 (M + H)⁺) and compound **157** (Rt 63.01 min, LC-MS: m/z = 637.39 (M + H)⁺).

The retention time in chiral chromatographic column of compound 156 is short compared to that of compound 157, and the retention time in chiral chromatographic column of compound 157 is long compared to that of compound 156.

### Example 158 and Example 159

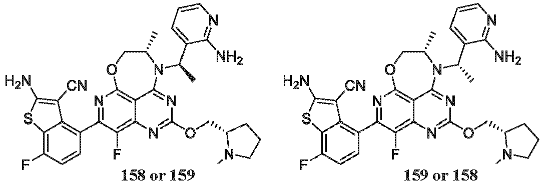

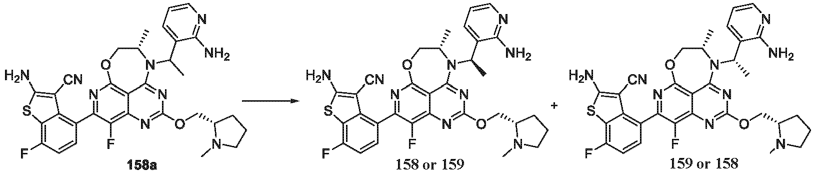

Referring to the preparation of compound **87b** in Example 87, compound **158a** was prepared by replacing ((2-fluoro-8-(4,4,5,5-tetramethyl-1,3,2-dioxaborolan-2-yl)naphthalen-1-yl)ethynyl)triisopropylsilane with *tert*-butyl (3-cyano-7-fluoro-4-(4,4,5,5-tetramethyl-1,3,2-dioxaborolan-2-yl)benzo[*b*]thiophen-2-yl)carbamate in step 1.

Compound **158a** was sequentially purified by preparative liquid phase chromatography (YMC TA C18 10 µm 30 × 250 chromatographic column; methanol-0.1% aqueous acetic acid solution (gradient elution of 10%-45%/0-80 min)) to give compound **158** (Rt 54.7 min, LC-MS: m/z = 644.34 (M + H)⁺) and compound **159** (Rt 59.37 min, LC-MS: m/z = 644.36 (M + H)⁺).

The retention time in chiral chromatographic column of compound 158 is short compared to that of compound 159, and the retention time in chiral chromatographic column of compound 159 is long compared to that of compound 158.

### Example 160 and Example 161

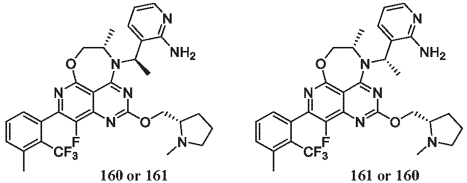

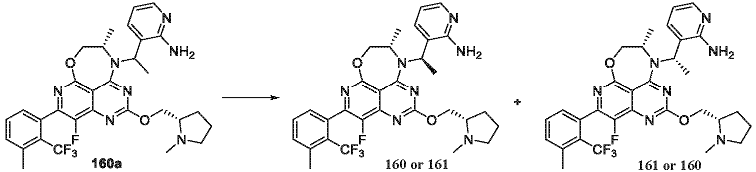

Referring to the preparation of compound **87b** in Example 87, compound **160a** was prepared by replacing ((2-fluoro-8-(4,4,5,5-tetramethyl-1,3,2-dioxaborolan-2-yl)naphthalen-1-yl)ethynyl)triisopropylsilane with (3-methyl-2-(trifluoromethyl)phenyl)boronic acid in step 1.

Compound **160a** was sequentially purified by preparative liquid phase chromatography (Novasep C18 250 × 50 mm, 10µm chromatographic column; methanol-0.1% aqueous acetic acid solution (gradient elution of 20%-50%/0-60 min)) to give compound **160** (Rt 45 min, LC-MS: m/z = 612.34 (M + H)⁺) and compound **161** (Rt 48 min, LC-MS: m/z = 612.33 (M + H)⁺).

The retention time in chiral chromatographic column of compound 160 is short compared to that of compound 161, and the retention time in chiral chromatographic column of compound 161 is long compared to that of compound 160.

### Example 162 and Example 163

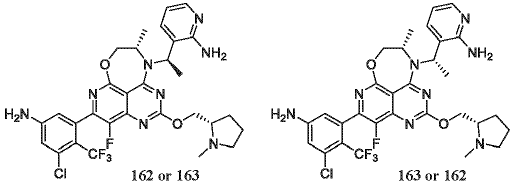

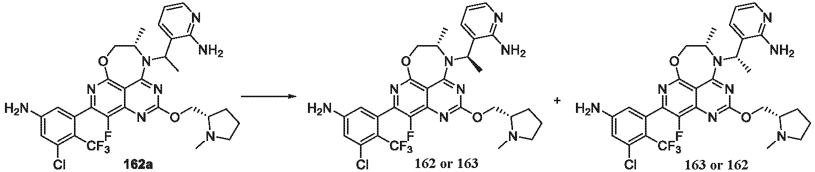

Referring to the preparation of compound **87b** in Example 87, compound **162a** was prepared by replacing ((2-fluoro-8-(4,4,5,5-tetramethyl-1,3,2-dioxaborolan-2-yl)naphthalen-1-yl)ethynyl)triisopropylsilane with 3-chloro-5-(4,4,5,5-tetramethyl-1,3,2-dioxaborolan-2-yl)-4-(trifluoromethyl)aniline in step 1.

Compound **162a** was sequentially purified by preparative liquid phase chromatography (YMC TA C18 10 µm 30 × 250 chromatographic column; methanol-0.1% aqueous ammonium hydroxide solution (gradient elution of 50%-70%-100%/0-20-60 min)) to give compound **162** (Rt 36.12 min, LC-MS: m/z = 647.27 (M + H)⁺) and compound **163** (Rt 39.01 min, LC-MS: m/z = 647.27 (M + H)⁺).

The retention time in chiral chromatographic column of compound 162 is short compared to that of compound 163, and the retention time in chiral chromatographic column of compound 163 is long compared to that of compound 162.

### Example 164 and Example 165

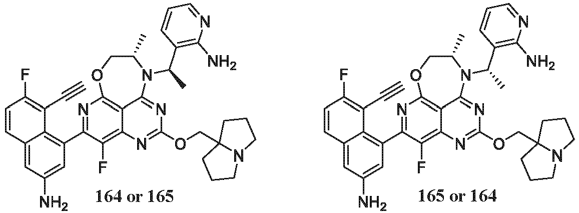

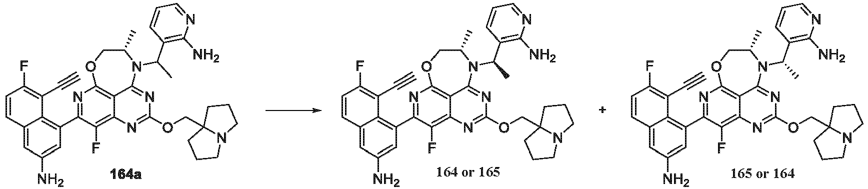

Referring to the preparation of compound **65f** in Example 65, compound **164a** was prepared by replacing methanol with (hexahydro-1*H*-pyrrolizin-7a-yl)methanol in step 2 and replacing 10g with 6-fluoro-4-(4,4,5,5-tetramethyl-1,3,2-dioxaborolan-2-yl)-5-((triisopropylsilyl)ethynyl)naphthalen-2-amine in step 4.

Compound **164a** was sequentially purified by preparative liquid phase chromatography (YMC-AQ-C18, 10 µm, 50 × 250 chromatographic column; methanol-0.1% aqueous acetic acid solution (gradient elution of 10%-70%/0-60 min)) to give compound **164** (Rt 27.34 min, LC-MS: m/z = 663.37 (M + H)⁺) and compound **165** (Rt 32.16 min, LC-MS: m/z = 663.38 (M + H)⁺).

The retention time in chiral chromatographic column of compound 164 is short compared to that of compound 165, and the retention time in chiral chromatographic column of compound 165 is long compared to that of compound 164.

### Example 166 and Example 167

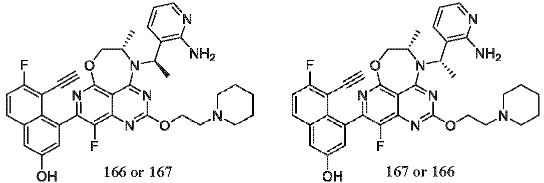

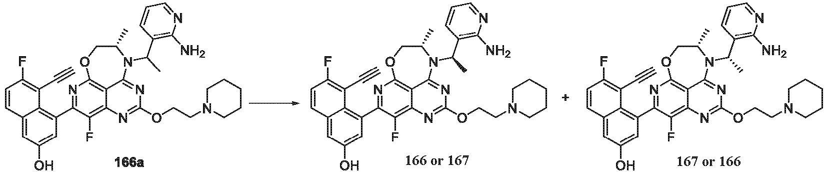

Referring to the preparation of compound **65g** in Example 65, compound **166a** was prepared by replacing methanol with 2-piperidinoethanol in step 2.

Compound **166a** was sequentially purified by preparative liquid phase chromatography (YMC TA C18 10 µm 50 × 250 chromatographic column; methanol-0.1% aqueous ammonium hydroxide solution (gradient elution of 40%-60%-90%/0-15-60 min)) to give compound **166** (Rt 34.34 min, LC-MS: m/z = 652.35 (M + H)⁺) and compound **167** (Rt 38.08 min, LC-MS: m/z = 652.36 (M + H)⁺).

The retention time in chiral chromatographic column of compound 166 is short compared to that of compound 167, and the retention time in chiral chromatographic column of compound 167 is long compared to that of compound 166.

### Example 168 and Example 169

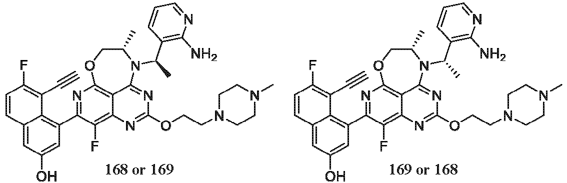

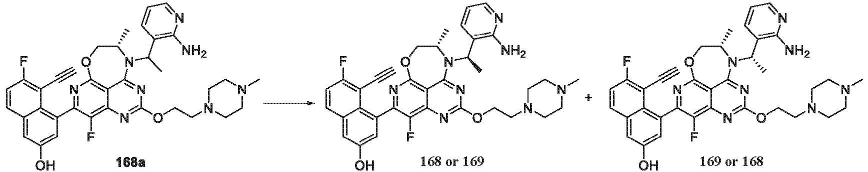

Referring to the preparation of compound **65g** in Example 65, compound **168a** was prepared by replacing methanol with 1-(2-hydroxyethyl)-4-methylpiperazine in step 2.

Compound **168a** was sequentially purified by preparative liquid phase chromatography (YMC TA C18 10 µm 50 × 250 chromatographic column; methanol-0.1% aqueous ammonium hydroxide solution (gradient elution of 40%-60%-90%/0-15-60 min)) to give compound **168** (Rt 28.56 min, LC-MS: m/z = 667.37 (M + H)⁺) and compound **169** (Rt 32.12 min, LC-MS: m/z = 667.37 (M + H)⁺).

The retention time in chiral chromatographic column of compound 168 is short compared to that of compound 169, and the retention time in chiral chromatographic column of compound 169 is long compared to that of compound 168.

### Example 170 and Example 171

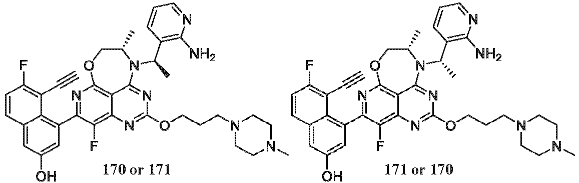

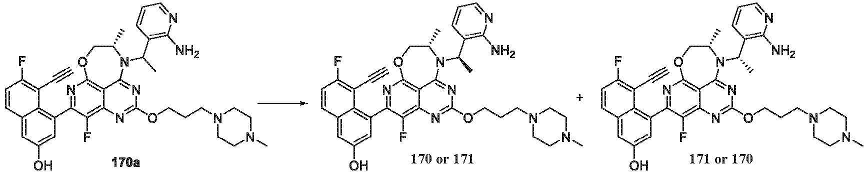

Referring to the preparation of compound **65g** in Example 65, compound **170a** was prepared by replacing methanol with 1-(3-hydroxypropyl)-4-methylpiperazine in step 2.

Compound **170a** was sequentially purified by preparative liquid phase chromatography (YMC TA C18 10 µm 50 × 250 chromatographic column; methanol-0.1% aqueous ammonium hydroxide solution (gradient elution of 40%-60%-90%/0-15-60 min)) to give compound **170** (Rt 27.54 min, LC-MS: m/z = 681.39 (M + H)⁺) and compound **171** (Rt 31.58 min, LC-MS: m/z = 681.37 (M + H)⁺).

The retention time in chiral chromatographic column of compound 170 is short compared to that of compound 171, and the retention time in chiral chromatographic column of compound 171 is long compared to that of compound 170.

### Example 172 and Example 173

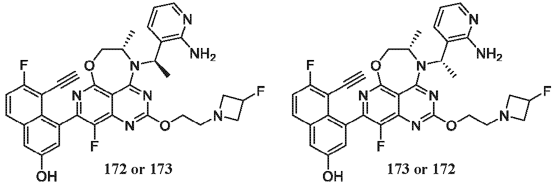

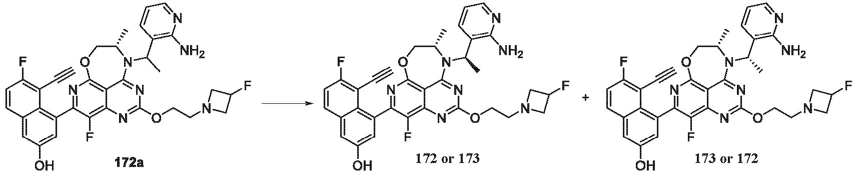

Referring to the preparation of compound **65g** in Example 65, compound **172a** was prepared by replacing methanol with 2-(3-fluoroazetidin-1-yl)ethanol in step 2.

Compound **172a** was sequentially purified by preparative liquid phase chromatography (YMC TA C18 10 µm 50 × 250 chromatographic column; methanol-0.1% aqueous ammonium hydroxide solution (gradient elution of 40%-60%-90%/0-15-60 min)) to give compound **172** (Rt 24.14 min, LC-MS: m/z = 642.34 (M + H)⁺) and compound **173** (Rt 28.07 min, LC-MS: m/z = 642.35 (M + H)⁺).

The retention time in chiral chromatographic column of compound 172 is short compared to that of compound 173, and the retention time in chiral chromatographic column of compound 173 is long compared to that of compound 172.

### Example 174 and Example 175

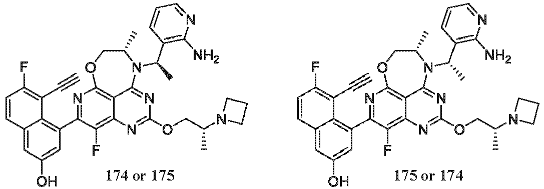

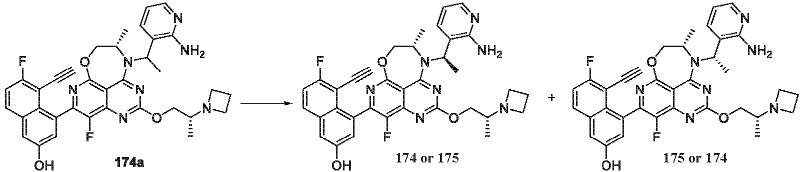

Referring to the preparation of compound **65g** in Example 65, compound **174a** was prepared by replacing methanol with (*R*)-2-(azetidin-1-yl)propanol in step 2.

Compound **174a** was sequentially purified by preparative liquid phase chromatography (YMC TA C18 10 µm 50 × 250 chromatographic column; methanol-0.1% aqueous ammonium hydroxide solution (gradient elution of 40%-60%-90%/0-15-60 min)) to give compound **174** (Rt 33.57 min, LC-MS: m/z = 638.38 (M + H)⁺) and compound **175** (Rt 37.53 min, LC-MS: m/z = 638.26 (M + H)⁺).

The retention time in chiral chromatographic column of compound 174 is short compared to that of compound 175, and the retention time in chiral chromatographic column of compound 175 is long compared to that of compound 174.

### Example 176 and Example 177

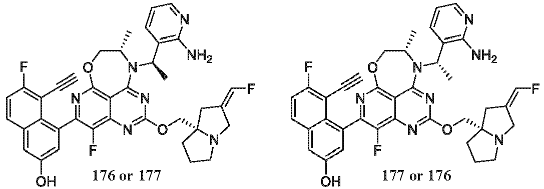

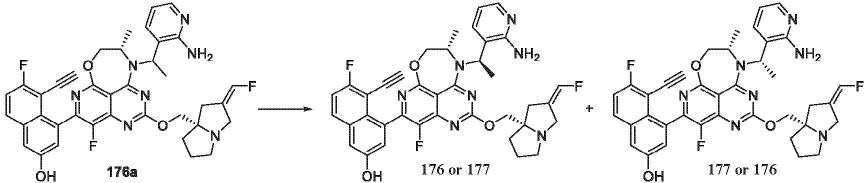

Referring to the preparation of compound **65g** in Example 65, compound **176a** was prepared by replacing methanol with (*S*,*Z*)-(2-(fluoromethylene)tetrahydro-1*H*-pyrrolizin-7a(5*H*)-yl)methanol in step 2.

Compound **176a** was sequentially purified by preparative liquid phase chromatography (YMC TA C18 10 µm 50 × 250 chromatographic column; methanol-0.1% aqueous acetic acid solution (gradient elution of 30%-90%/0-60 min)) to give compound **176** (Rt 19.34 min, LC-MS: m/z = 694.36 (M + H)⁺) and compound **177** (Rt 24.16 min, LC-MS: m/z = 694.35 (M + H)⁺).

The retention time in chiral chromatographic column of compound 176 is short compared to that of compound 177, and the retention time in chiral chromatographic column of compound 177 is long compared to that of compound 176.

### Example 178 and Example 179

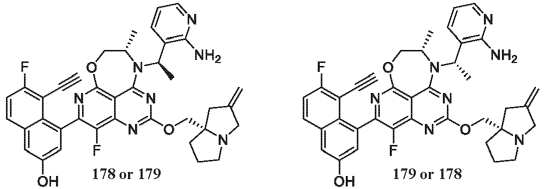

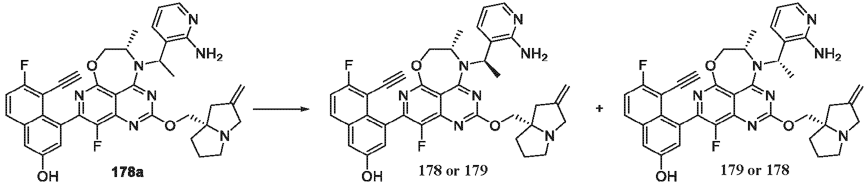

Referring to the preparation of compound **65g** in Example 65, compound **178a** was prepared by replacing methanol with (*S*)-(2-methylenetetrahydro-1*H*-pyrrolizin-7a(5*H*)-yl)methanol in step 2.

Compound **178a** was sequentially purified by preparative liquid phase chromatography (YMC TA C18 10 µm 50 × 250 chromatographic column; methanol-0.1% aqueous acetic acid solution (gradient elution of 20%-80%/0-60 min)) to give compound **178** (Rt 26.35 min, LC-MS: m/z = 676.30 (M + H)⁺) and compound **179** (Rt 34.01 min, LC-MS: m/z = 676.32 (M + H)⁺).

The retention time in chiral chromatographic column of compound 178 is short compared to that of compound 179, and the retention time in chiral chromatographic column of compound 179 is long compared to that of compound 178.

### Example 180 to Example 425

The following compounds were prepared by reference to the route 1 and examples 1-179 of the present application:

| Example | | | LC-MS: m/z (M + H)⁺ (compound No.) |
|---|---|---|---|
| 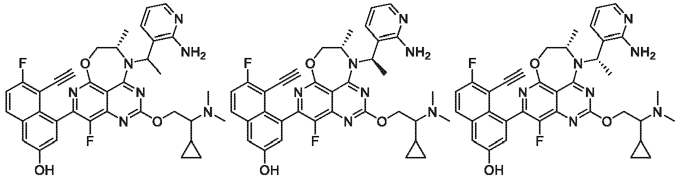 | | | 652.28 (180) |
| | | | 652.28 (181) |
| | | | 652.28 (182) |
| 180 | 181 or 182 | 182 or 181 | |
| 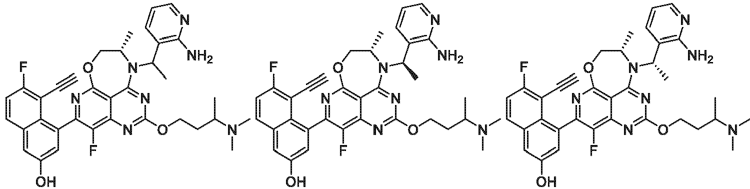 | | | 640.28 (183) |
| | | | 640.28 (184) |
| | | | 640.28 (185) |
| 183 | 184 or 185 | 185 or 184 | |
| 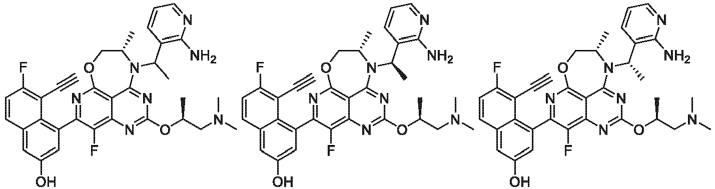 | | | 626.27 (186) |
| | | | 626.27 (187) |
| | | | 626.27 (188) |
| 186 | 187 or 188 | 188 or 187 | |
| 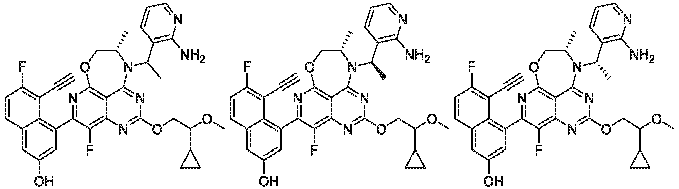 | | | 639.25 (189) |
| | | | 639.25 (190) |
| | | | 639.25 (191) |
| 189 | 190 or 191 | 191 or 190 | |
| 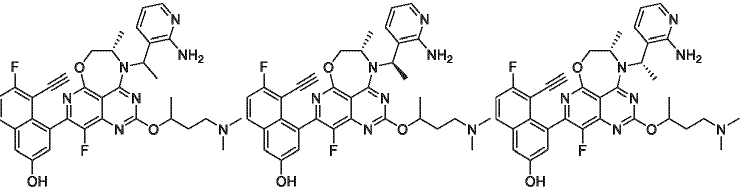 | | | 640.28 (192) |
| | | | 640.28 (193) |
| | | | 640.28 (194) |
| 192 | 193 or 194 | 194 or 193 | |
| 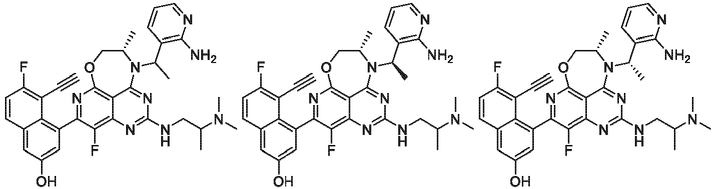 | | | 625.28 (195) |
| | | | 625.28 (196) |
| | | | 625.28 (197) |
| 195 | 196 or 197 | 197 or 196 | |
| 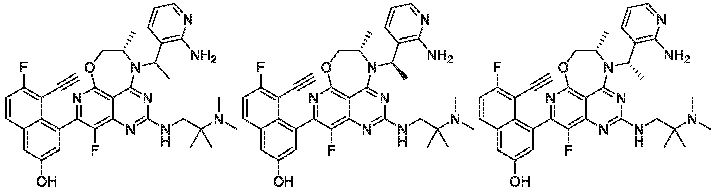 | | | 639.30 (198) |
| | | | 639.30 (199) |
| | | | 639.30 (200) |
| 198 | 199 or 200 | 200 or 199 | |
| 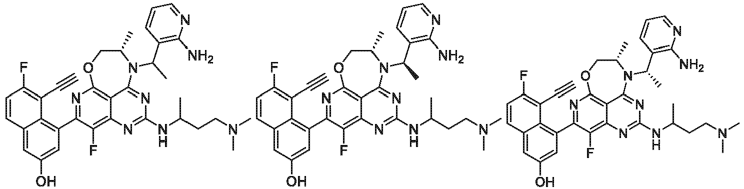 | | | 639.30 (201) |
| | | | 639.30 (202) |
| | | | 639.30 (203) |
| 201 | 202 or 203 | 203 or 202 | |
| 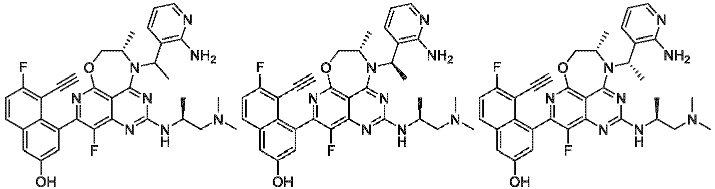 | | | 625.28 (204) |
| | | | 625.28 (205) |
| | | | 625.28 (206) |
| 204 | 205 or 206 | 206 or 205 | |
| 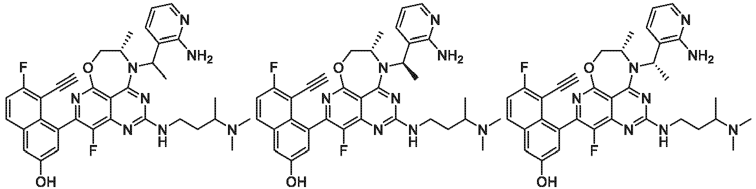 | | | 639.30 (207) |
| | | | 639.30 (208) |
| | | | 639.30 (209) |
| 207 | 208 or 209 | 209 or 208 | |
| 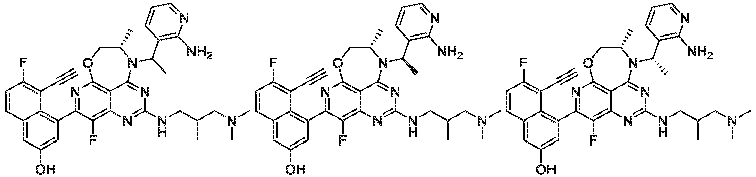 | | | 639.30 (210) |
| | | | 639.30 (211) |
| | | | 639.30 (212) |
| 210 | 211 or 212 | 212 or 211 | |
| 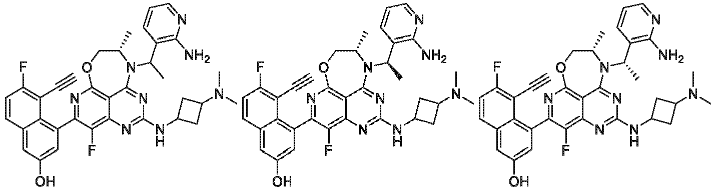 | | | 637.28 (213) |
| | | | 637.28 (214) |
| | | | 637.28 (215) |
| 213 | 214 or 215 | 215 or 214 | |
| 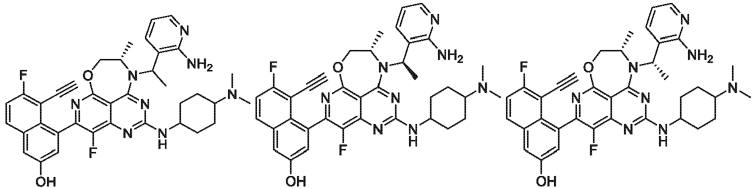 | | | 665.32 (216) |
| | | | 665.32 (217) |
| | | | 665.32 (218) |
| 216 | 217 or 218 | 218 or 217 | |
| 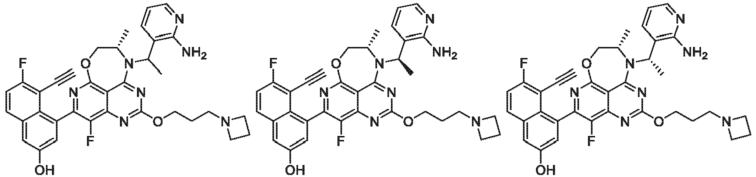 | | | 638.27 (219) |
| | | | 638.27 (220) |
| | | | 638.27 (221) |
| 219 | 220 or 221 | 221 or 220 | |
| 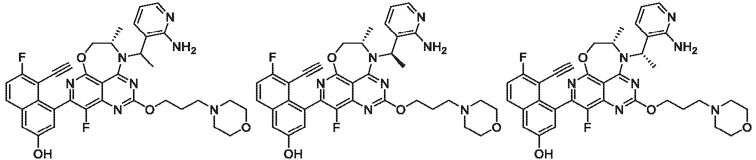 | | | 668.28 (222) |
| | | | 668.28 (223) |
| | | | 668.28 (224) |
| 222 | 223 or 224 | 224 or 223 | |
| 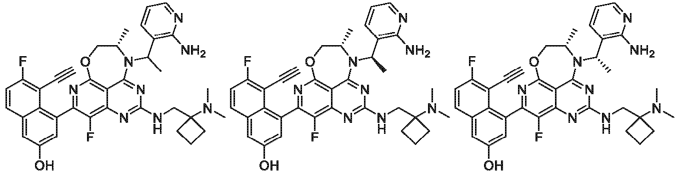 | | | 651.30 (225) |
| | | | 651.30 (226) |
| | | | 651.30 (227) |
| 225 | 226 or 227 | 227 or 226 | |
| 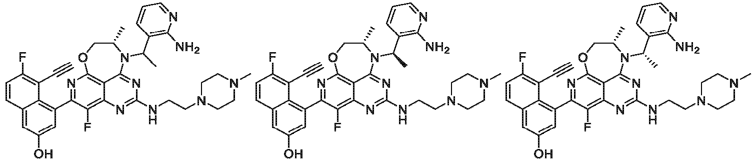 | | | 666.31 (228) |
| | | | 666.31 (229) |
| | | | 666.31 (230) |
| 228 | 229 or 230 | 230 or 229 | |
| 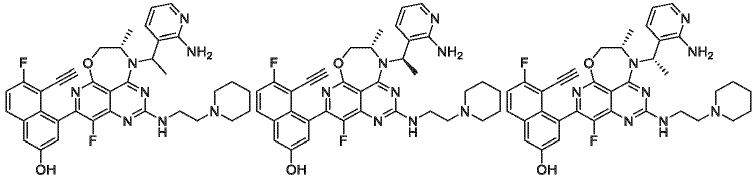 | | | 651.30 (231) |
| | | | 651.30 (232) |
| | | | 651.30 (233) |
| 231 | 232 or 233 | 233 or 232 | |
| 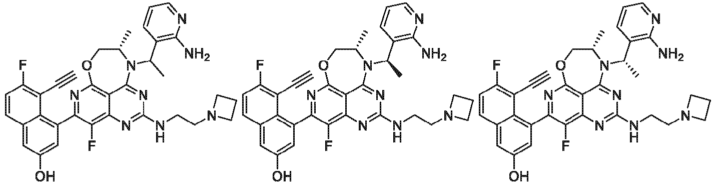 | | | 623.27 (234) |
| | | | 623.27 (235) |
| | | | 623.27 (236) |
| 234 | 235 or 236 | 236 or 235 | |
| 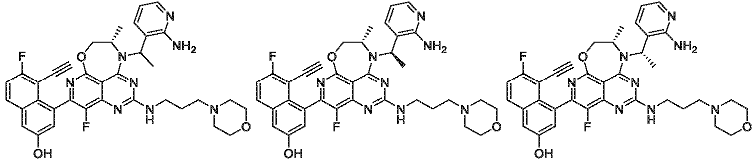 | | | 667.30 (237) |
| | | | 667.30 (238) |
| | | | 667.30 (239) |
| 237 | 238 or 239 | 239 or 238 | |
| 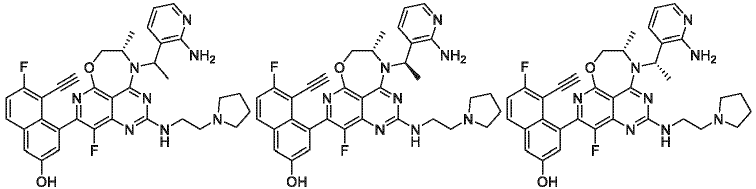 | | | 637.28 (240) |
| | | | 637.28 (241) |
| | | | 637.28 (242) |
| 240 | 241 or 242 | 242 or 241 | |
| 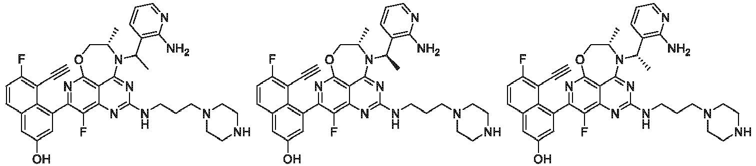 | | | 666.31 (243) |
| | | | 666.31 (244) |
| | | | 666.31 (245) |
| 243 | 244 or 245 | 245 or 244 | |
| 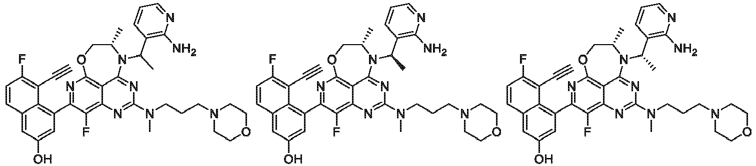 | | | 681.31 (246) |
| | | | 681.31 (247) |
| | | | 681.31 (248) |
| 246 | 247 or 248 | 248 or 247 | |
| 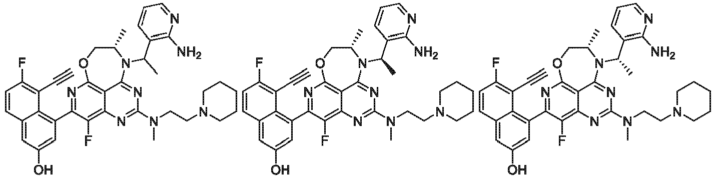 | | | 665.32 (249) |
| | | | 665.32 (250) |
| | | | 665.32 (251) |
| 249 | 250 or 251 | 251 or 250 | |
| 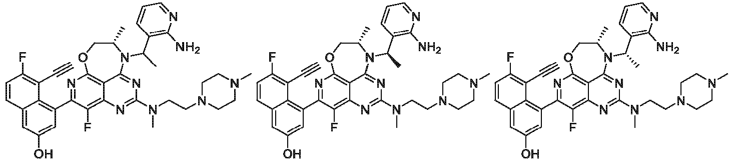 | | | 680.33 (252) |
| | | | 680.33 (253) |
| | | | 680.33 (254) |
| 252 | 253 or 254 | 254 or 253 | |
| 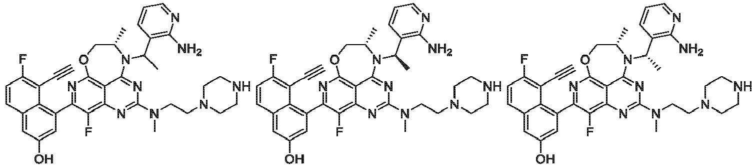 | | | 666.31 (255) |
| | | | 666.31 (256) |
| | | | 666.31 (257) |
| 255 | 256 or 257 | 257 or 256 | |
| 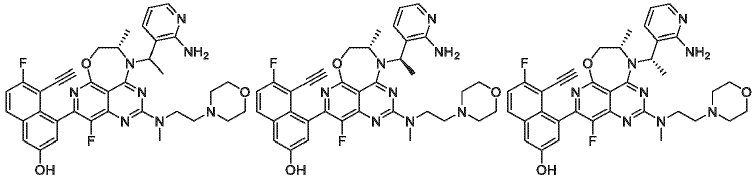 | | | 667.30 (258) |
| | | | 667.30 (259) |
| | | | 667.30 (260) |
| 258 | 259 or 260 | 260 or 259 | |
| 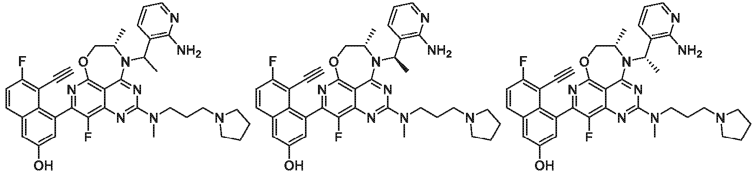 | | | 665.32 (261) |
| | | | 665.32 (262) |
| | | | 665.32 (263) |
| 261 | 262 or 263 | 263 or 262 | |
| 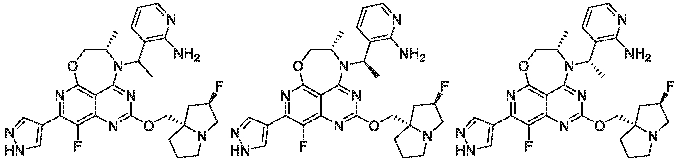 | | | 564.26 (264) |
| | | | 564.26 (265) |
| | | | 564.26 (266) |
| 264 | 265 or 266 | 266 or 265 | |
| 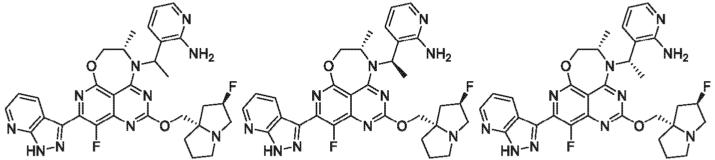 | | | 615.28 (267) |
| | | | 615.28 (268) |
| | | | 615.28 (269) |
| 267 | 268 or 269 | 269 or 268 | |
| 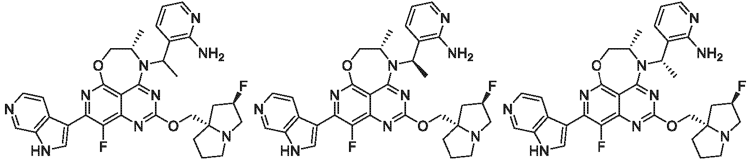 | | | 614.28 (270) |
| | | | 614.28 (271) |
| | | | 614.28 (272) |
| 270 | 271 or 272 | 272 or 271 | |
| 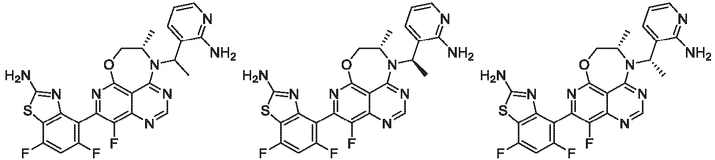 | | | 525.14 (273) |
| | | | 525.14 (274) |
| | | | 525.14 (275) |
| 273 | 274 or 275 | 275 or 274 | |
| 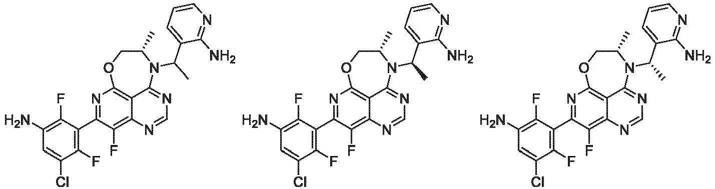 | | | 502.14 (276) |
| | | | 502.14 (277) |
| | | | 502.14 (278) |
| 276 | 277 or 278 | 278 or 277 | |
| 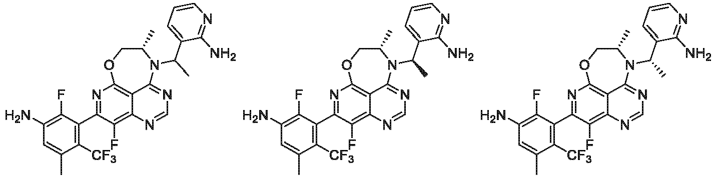 | | | 532.19 (279) |
| | | | 532.19 (280) |
| | | | 532.19 (281) |
| 279 | 280 or 281 | 281 or 280 | |
| 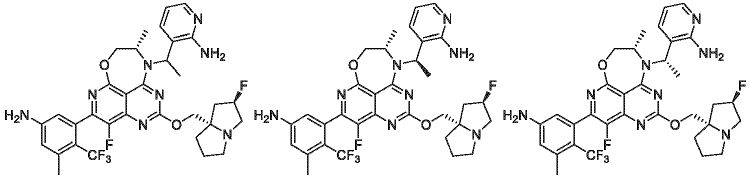 | | | 671.29 (282) |
| | | | 671.29 (283) |
| | | | 671.29 (284) |
| 282 | 283 or 284 | 284 or 283 | |
| 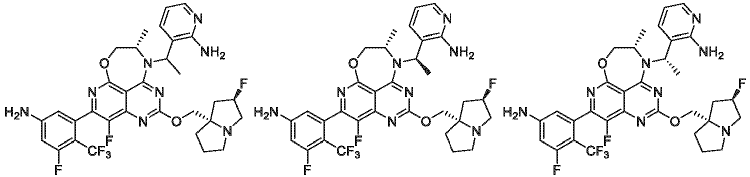 | | | 675.26 (285) |
| | | | 675.26 (286) |
| | | | 675.26 (287) |
| 285 | 286 or 287 | 287 or 286 | |
| 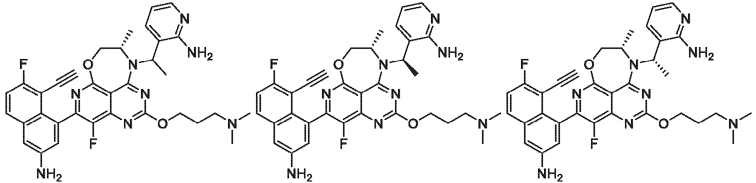 | | | 625.28 (288) |
| | | | 625.28 (289) |
| | | | 625.28 (290) |
| 288 | 289 or 290 | 290 or 289 | |
| 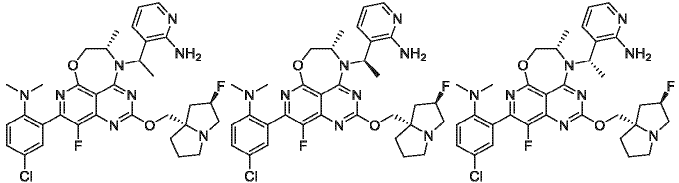 | | | 651.28 (291) |
| | | | 651.28 (292) |
| | | | 651.28 (293) |
| 291 | 292 or 293 | 293 or 292 | |
| 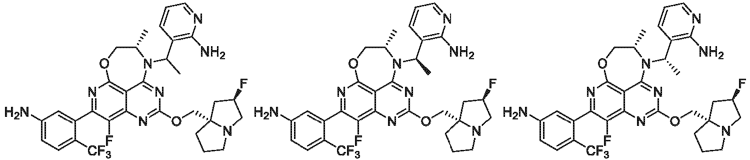 | | | 657.27 (294) |
| | | | 657.27 (295) |
| | | | 657.27 (296) |
| 294 | 295 or 296 | 296 or 295 | |
| 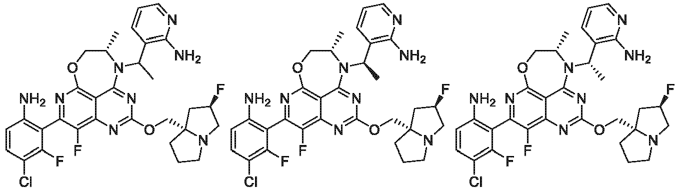 | | | 641.24 (297) |
| | | | 641.24 (298) |
| | | | 641.24 (299) |
| 297 | 298 or 299 | 299 or 298 | |
| 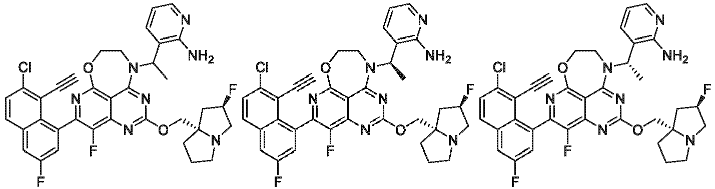 | | | 686.23 (300) |
| | | | 686.23 (301) |
| | | | 686.23 (302) |
| 300 | 301 or 302 | 302 or 301 | |
| 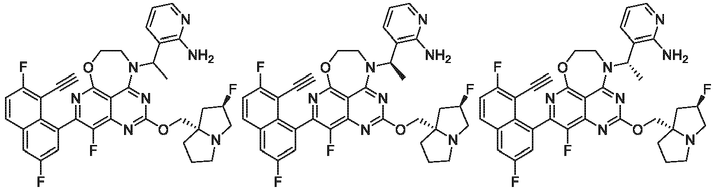 | | | 670.25 (303) |
| | | | 670.25 (304) |
| | | | 670.25 (305) |
| 303 | 304 or 305 | 305 or 304 | |
| 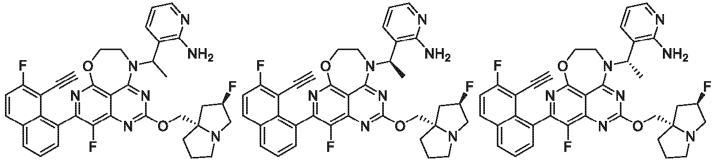 | | | 652.26 (306) |
| | | | 652.26 (307) |
| | | | 652.26 (308) |
| 306 | 307 or 308 | 308 or 307 | |
| 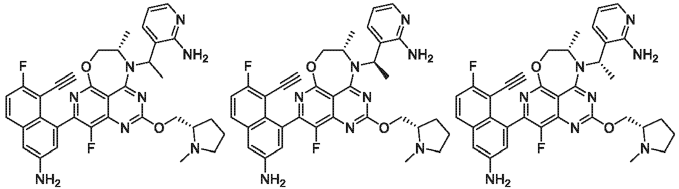 | | | 637.28 (309) |
| | | | 637.28 (310) |
| | | | 637.28 (311) |
| 309 | 310 or 311 | 311 or 310 | |
| 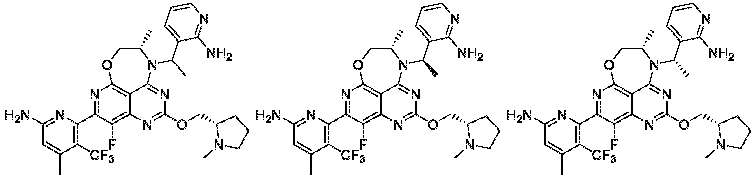 | | | 628.28 (312) |
| | | | 628.28 (313) |
| | | | 628.28 (314) |
| 312 | 313 or 314 | 314 or 313 | |
| 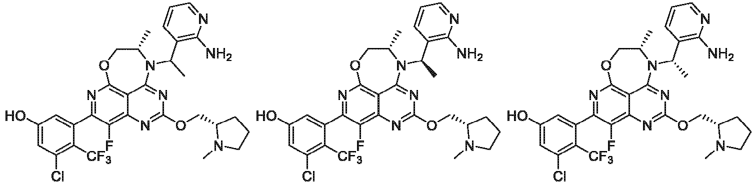 | | | 648.21 (315) |
| | | | 648.21 (316) |
| | | | 648.21 (317) |
| 315 | 316 or 317 | 317 or 316 | |
| 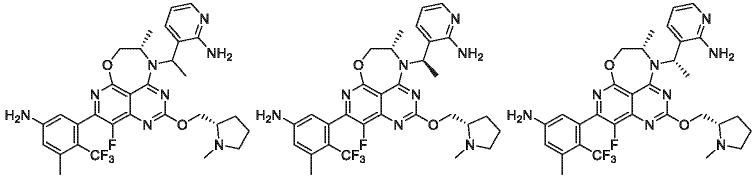 | | | 627.28 (318) |
| | | | 627.28 (319) |
| | | | 627.28 (320) |
| 318 | 319 or 320 | 320 or 319 | |
| 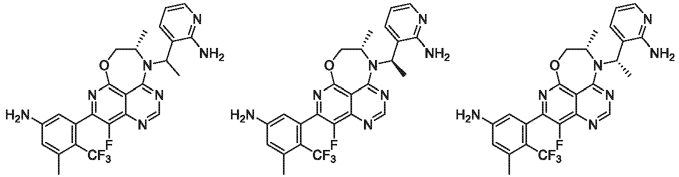 | | | 514.20 (321) |
| | | | 514.20 (322) |
| | | | 514.20 (323) |
| 321 | 322 or 323 | 323 or 322 | |
| 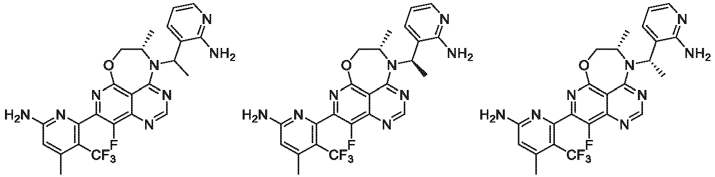 | | | 515.19 (324) |
| | | | 515.19 (325) |
| | | | 515.19 (326) |
| 324 | 325 or 326 | 326 or 325 | |
| 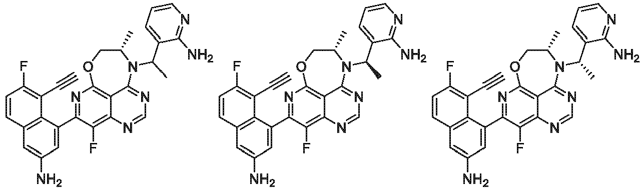 | | | 524.20 (327) |
| | | | 524.20 (328) |
| | | | 524.20 (329) |
| 327 | 328 or 329 | 329 or 328 | |
| 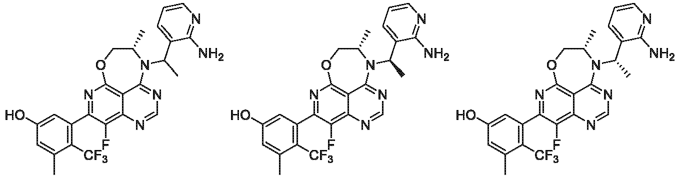 | | | 515.18 (330) |
| | | | 515.18 (331) |
| | | | 515.18 (332) |
| 330 | 331 or 332 | 332 or 331 | |
| 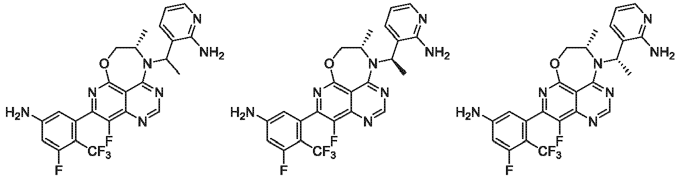 | | | 518.17 (333) |
| | | | 518.17 (334) |
| | | | 518.17 (335) |
| 333 | 334 or 335 | 335 or 334 | |
| 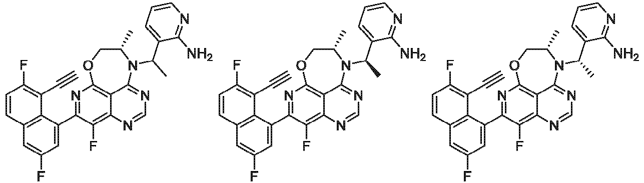 | | | 527.18 (336) |
| | | | 527.18 (337) |
| | | | 527.18 (338) |
| 336 | 337 or 338 | 338 or 337 | |
| 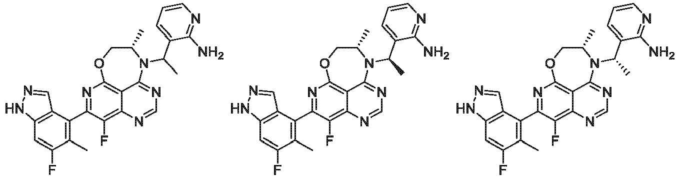 | | | 489.20 (339) |
| | | | 489.20 (340) |
| | | | 489.20 (341) |
| 339 | 340 or 341 | 341 or 340 | |
| 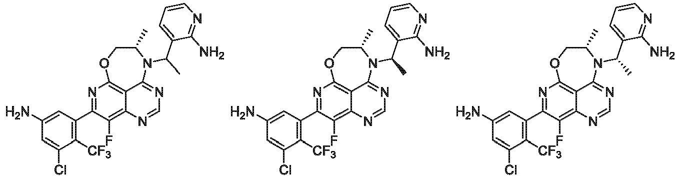 | | | 534.14 (342) |
| | | | 534.14 (343) |
| | | | 534.14 (344) |
| 342 | 343 or 344 | 344 or 343 | |
| 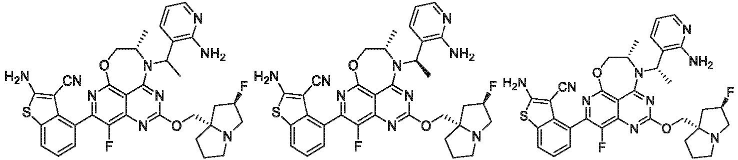 | | | 670.25 (345) |
| | | | 670.25 (346) |
| | | | 670.25 (347) |
| 345 | 346 or 347 | 347 or 346 | |
| 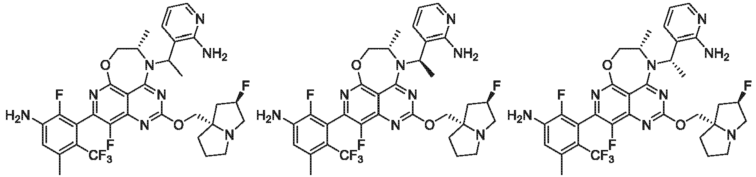 | | | 689.28 (348) |
| | | | 689.28 (349) |
| | | | 689.28 (350) |
| 348 | 349 or 350 | 350 or 349 | |
| 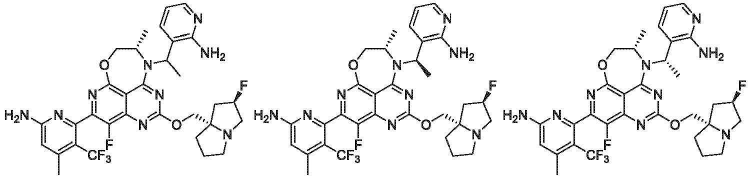 | | | 672.28 (351) |
| | | | 672.28 (352) |
| | | | 672.28 (353) |
| 351 | 352 or 353 | 353 or 352 | |
| 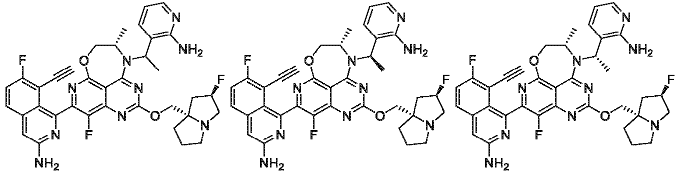 | | | 682.29 (354) |
| | | | 682.29 (355) |
| | | | 682.29 (356) |
| 354 | 355 or 356 | 356 or 355 | |
| 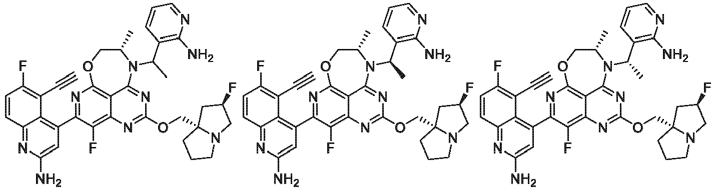 | | | 682.29 (357) |
| | | | 682.29 (358) |
| | | | 682.29 (359) |
| 357 | 358 or 359 | 359 or 358 | |
| 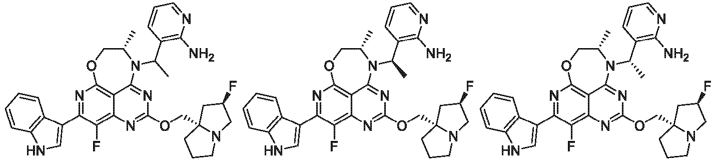 | | | 613.28 (360) |
| | | | 613.28 (361) |
| | | | 613.28 (362) |
| 360 | 361 or 362 | 362 or 361 | |
| 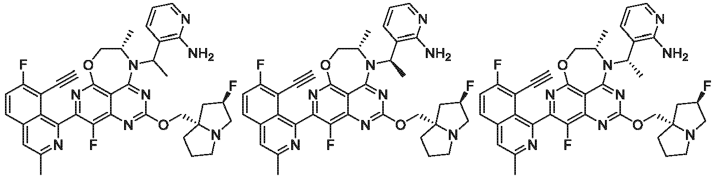 | | | 681.29 (363) |
| | | | 681.29 (364) |
| | | | 681.29 (365) |
| 363 | 364 or 365 | 365 or 364 | |
| 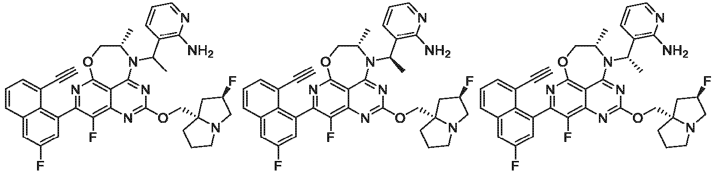 | | | 666.28 (366) |
| | | | 666.28 (367) |
| | | | 666.28 (368) |
| 366 | 367 or 368 | 368 or 367 | |
| 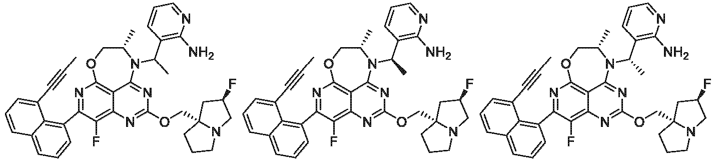 | | | 662.30 (369) |
| | | | 662.30 (370) |
| | | | 662.30 (371) |
| 369 | 370 or 371 | 371 or 370 | |
| 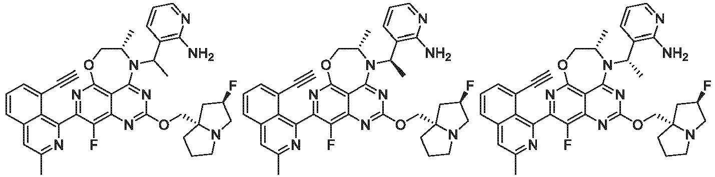 | | | 663.30 (372) |
| | | | 663.30 (373) |
| | | | 663.30 (374) |
| 372 | 373 or 374 | 374 or 373 | |
| 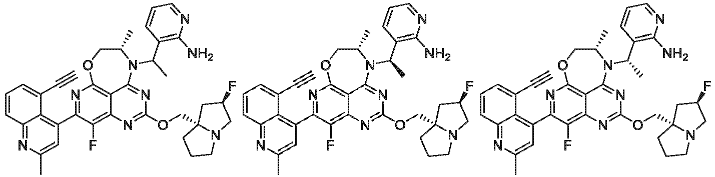 | | | 663.30 (375) |
| | | | 663.30 (376) |
| | | | 663.30 (377) |
| 375 | 376 or 377 | 377 or 376 | |
| 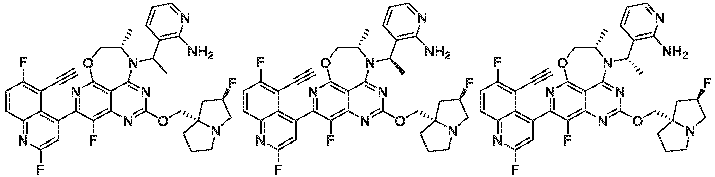 | | | 685.27 (378) |
| | | | 685.27 (379) |
| | | | 685.27 (380) |
| 378 | 379 or 380 | 380 or 379 | |
| 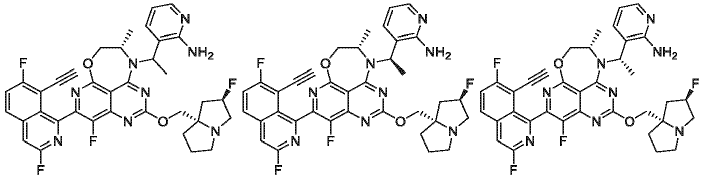 | | | 685.27 (381) |
| | | | 685.27 (382) |
| | | | 685.27 (383) |
| 381 | 382 or 383 | 383 or 382 | |
| 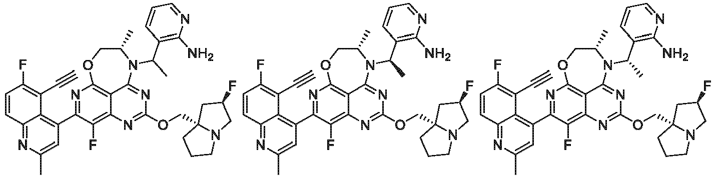 | | | 681.29 (384) |
| | | | 681.29 (385) |
| | | | 681.29 (386) |
| 384 | 385 or 386 | 386 or 385 | |
| 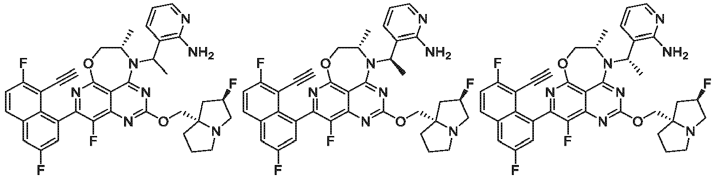 | | | 684.27 (387) |
| | | | 684.27 (388) |
| | | | 684.27 (389) |
| 387 | 388 or 389 | 389 or 388 | |
| 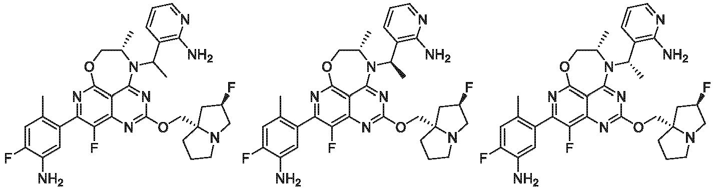 | | | 621.29 (390) |
| | | | 621.29 (391) |
| | | | 621.29 (392) |
| 390 | 391 or 392 | 392 or 391 | |
| 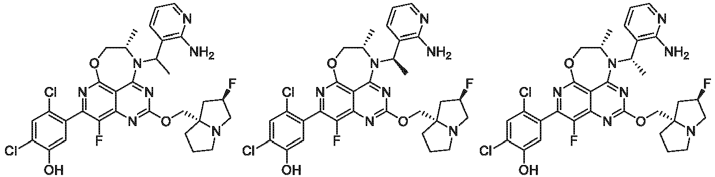 | | | 658.19 (393) |
| | | | 658.19 (394) |
| | | | 658.19 (395) |
| 393 | 394 or 395 | 395 or 394 | |
| 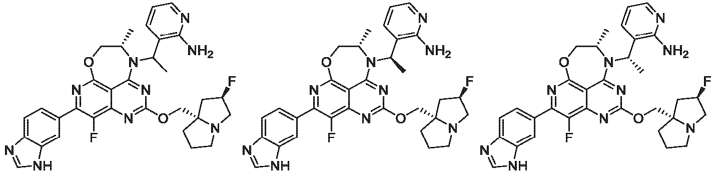 | | | 614.28 (396) |
| | | | 614.28 (397) |
| | | | 614.28 (398) |
| 396 | 397 or 398 | 398 or 397 | |
| 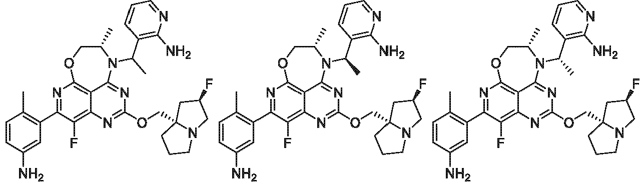 | | | 603.30 (399) |
| | | | 603.30 (400) |
| | | | 603.30 (401) |
| 399 | 400 or 401 | 401 or 400 | |
| 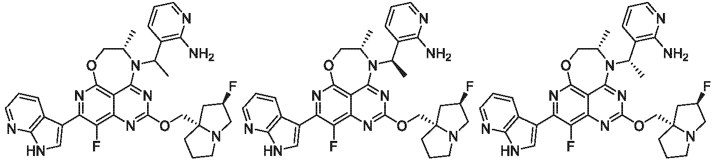 | | | 614.28 (402) |
| | | | 614.28 (403) |
| | | | 614.28 (404) |
| 402 | 403 or 404 | 404 or 403 | |
| 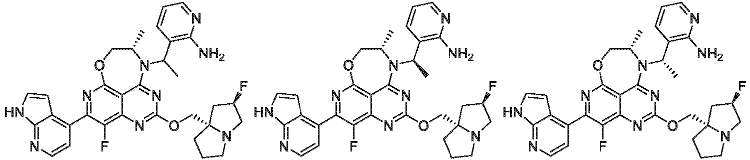 | | | 614.28 (405) |
| | | | 614.28 (406) |
| | | | 614.28 (407) |
| 405 | 406 or 407 | 407 or 406 | |
| 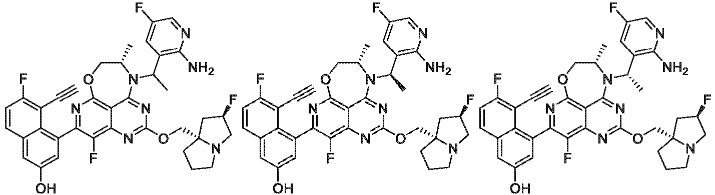 | | | 700.27 (408) |
| | | | 700.27 (409) |
| | | | 700.27 (410) |
| 408 | 409 or 410 | 410 or 409 | |
| 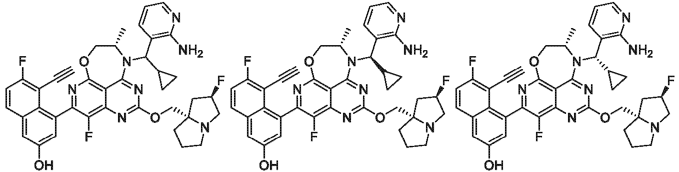 | | | 708.29 (411) |
| | | | 708.29 (412) |
| | | | 708.29 (413) |
| 411 | 412 or 413 | 413 or 412 | |
| 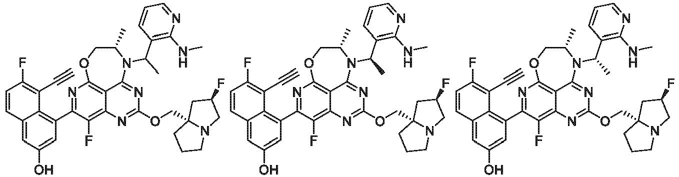 | | | 696.29 (414) |
| | | | 696.29 (415) |
| | | | 696.29 (416) |
| 414 | 415 or 416 | 416 or 415 | |
| 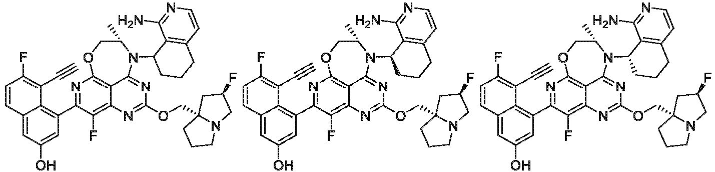 | | | 708.29 (417) |
| | | | 708.29 (418) |
| | | | 708.29 (419) |
| 417 | 418 or 419 | 419 or 418 | |
| 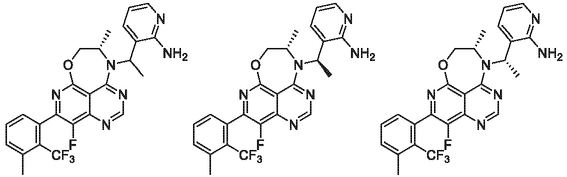 | | | 499.19 (420) |
| | | | 499.19 (421) |
| | | | 499.19 (422) |
| 420 | 421 or 422 | 422 or 421 | |
| 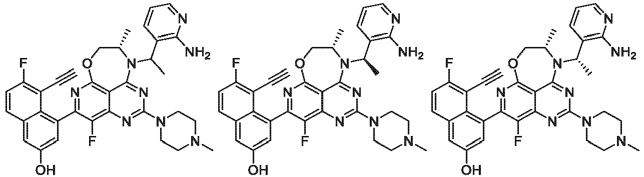 | | | 623.27 (423) |
| | | | 623.27 (424) |
| | | | 623.27 (425) |
| 423 | 424 or 425 | 425 or 424 | |

### Test Example 1: Experimental Method for Nucleotide Exchange

1. Reagents: His-KRAS G12D (1-169); SOS1^{cat} (564-1049); GDP (purchased from Sigma); Anti-6HIS-Cryptate (purchased from Cisbio); EDA-GTP-DY-647P1 (purchased from Jena Bioscience)
2. Buffer preparation:
   1) assay buffer: HEPES pH 7.4, NaCl, MgCl₂, DTT, BSA, and Igepal;
   2) KRAS G12D working solution: A KRAS G12D working solution containing 100 nM His-KRAS G12D and 2 nM Anti-6HIS-Cryptate was prepared using the assay buffer;
   3) SOS1^{cat} working solution: An SOS1^{cat} working solution containing 20 nM SOS1^{cat} and 200 nM EDA-GTP-DY-647P1 was prepared using the assay buffer;
   4) blank control working solution: A blank control solution containing 2 nM Anti-6HIS-Cryptate was prepared using the assay buffer.
3. Testing procedure:
   The entire experimental process was completed at room temperature. Using a black clear-bottom 384-well plate, 5 µL of KRAS G12D working solution was added to each well for the experimental and negative groups, while 5 µL of blank control working solution was added to each well for the blank group. The plate was incubated at room temperature for 10 min. Then, the compounds (at an initial concentration of 100 µM, 8 concentration gradients by 1:2 serial dilution) were added to the experimental groups using an ultra microsyringe and incubated at room temperature for 30 min. Finally, 5 µL of SOS1^{cat} working solution was added to each well, and after incubation at room temperature for 15 min, signal values were detected at 665 nm/620 nm using a PerkinElmer Envision HTS multi-label plate reader. The inhibition rate was calculated by the following formula: inhibition rate (%) = (mean value of negative control group - mean value of experimental group)/(mean value of negative control group - mean value of blank group) × 100%. The curves were fitted using the four-parameter logistic model with the logarithm of the compound concentration as the abscissa and the inhibition rate as the ordinate, and IC ₅₀ values were calculated.

### Test Example 2: Assay for Inhibitory Activity for AsPc-1 Cell Proliferation

AsPc-1 cells in a well-growing state were collected into a centrifuge tube, adjusted to a cell density of 3 × 10⁴ cells/mL, and inoculated onto a 96-well plate (100 µL/well). The compounds were added using a nanoliter pipettor such that the final concentrations of the compounds were 5000 nM-0.31 nM (the addition was performed in duplicate). Meanwhile, a control was set. After 72 h of incubation in the cell incubator, the assay reagent CCK-8 (manufacturer: Dojindo Laboratories; 10 µL/well) was added. After 3 h of incubation in the cell incubator, the absorbance was measured at 450 nm on a PerkinElmer Envision microplate reader. A four-parameter analysis was performed, a dose-response curve was fitted, and IC₅₀ was calculated.

### Test Example 3: Assay for Inhibitory Activity for Capan-1 Cell Proliferation

Capan-1 cells in a well-growing state were collected into a centrifuge tube, adjusted to a cell density of 1.5 × 10⁴ cells/mL, and inoculated onto a 96-well plate (100 µL/well). The compounds were added using a nanoliter pipettor such that the final concentrations of the compounds were 20000 nM-9.1 nM (the addition was performed in duplicate). Meanwhile, a control was set. After 5 days of incubation in the cell incubator, the assay reagent CCK-8 (manufacturer: Dojindo Laboratories, Beijing; 10 µL/well) was added. After 4 h of incubation in the cell incubator, the absorbance was measured at 450 nm on a PerkinElmer Envision microplate reader. A four-parameter analysis was performed, a dose-response curve was fitted, and IC₅₀ was calculated.

The above testing results for the part of the compounds of the present disclosure are shown in Table 1.

**Table 1. Testing results for part of compounds**

| Compound | Nucleotide exchange inhibitory activity IC₅₀ (nM) | Inhibitory activity IC₅₀ for AsPc-1 cell proliferation (nM) | Inhibitory activity IC₅₀ for Capan-1 cell proliferation (nM) |
|---|---|---|---|
| Example 1 | A | | |
| Example 2 | A | | |
| Example 3 | | B | B |
| Example 4 | A | c | B |
| Example 6 | A | | |
| Example 7 | A | | |
| Example 8 | A | c | B |
| Example 9 | A | | |
| Example 10 | c | | |
| Example 13 | A | | |
| Example 14 | A | | |
| Example 15 | A | | |
| Example 16 | A | | |
| Example 17 | A | | |
| Example 22 | A | | |
| Example 23 | B | | |
| Example 24 | A | | |
| Example 25 | A | | |
| Example 26 | B | | |
| Example 31 | | B | B |
| Example 32 | | A | A |
| Example 34 | | A | A |
| Example 36 | | A | A |
| Example 38 | | A | A |
| Example 39 | | A | A |
| Example 40 | | A | A |
| Example 41 | | | A |
| Example 45 | | | A |
| Example 47 | | A | A |
| Example 48 | | | A |
| Example 49 | | A | |
| Example 50 | | | A |
| Example 51 | | A | A |
| Example 53 | | A | A |
| Example 54 | | A | A |
| Example 55 | | | A |
| Example 57 | | | A |
| Example 59 | | | A |
| Example 61 | | | A |
| Example 63 | | | A |
| Example 64 | | | A |
| Example 65 | | | A |
| Example 67 | | A | A |
| Example 69 | | A | A |
| Example 70 | | | A |
| Example 71 | | A | A |
| Example 72 | | | A |
| Example 73 | | | A |
| Example 75 | | | A |
| Example 77 | | | A |
| Example 79 | | | A |
| Example 81 | | | A |
| Example 82 | | | A |
| Example 85 | | | A |
| Example 87 | | | A |
| Example 88 | | | A |
| Example 89 | | | A |
| Example 91 | | | A |
| Example 92 | | | A |
| Example 93 | | | A |
| Example 95 | | | A |
| Example 97 | | A | A |
| Example 99 | | | A |
| Example 101 | | | A |
| Example 103 | | | A |
| Example 105 | | A | A |
| Example 107 | | | A |
| Example 109 | | | A |
| Example 111 | | | A |
| Example 113 | | A | A |
| Example 114 | | A | A |
| Example 115 | | A | A |
| Example 116 | | | A |
| Example 118 | | A | A |
| Example 120 | | | A |
| Example 122 | | A | A |
| Example 124 | | A | A |
| Example 126 | | | A |
| Example 128 | | | A |
| Example 130 | | A | A |
| Example 131 | | | A |
| Example 132 | | | A |
| Example 134 | | | A |
| Example 136 | | | A |
| Example 138 | | A | A |
| Example 140 | | | A |
| Example 142 | | | A |
| Example 144 | | | A |
| Example 146 | | A | A |
| Example 147 | | | A |
| Example 148 | | A | A |
| Example 150 | | A | A |
| Example 152 | | | A |
| Example 154 | | | A |
| Example 156 | | A | A |
| Example 158 | | | A |
| Example 160 | | | A |
| Example 162 | | | A |
| Example 164 | | A | |
| Example 166 | | | A |
| Example 168 | | | A |
| Example 170 | | | A |
| Example 172 | | A | A |
| Example 174 | | | A |
| Example 176 | | A | A |
| Example 177 | | A | A |
| Example 178 | | A | |

| | | | |
|---|---|---|---|
| Note: A represents IC₅₀ ≤ 100 nM; B represents 100 nM < IC₅₀ ≤ 500 nM; C represents 500 nM < IC₅₀ ≤ 999 nM; - represents the IC₅₀ value not detected. | | | |

### Test Example 4: In Vitro Liver Microsomal Stability

Liver microsome incubation samples (species: human, monkey, rat, and mouse) were prepared by mixing a PBS buffer (pH 7.4), a liver microsome solution (0.5 mg/mL), a test compound, and an NADPH + MgCl₂ solution and incubated at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and the supernatants were prepared by protein precipitation, diluted, and then assayed by LC/MS/MS. The results are shown in Table 2.

**Table 2. Results for in vitro liver microsomal metabolic stability**

| **Compound** | The remaining amount % (T = 60 min) (0.5 mg/mL) | |
|---|---|---|
| | Human liver microsome | Mouse liver microsome |
| Example 79 | | >60 |
| Example 95 | | >60 |
| Example 97 | >40 | |
| Example 101 | | >60 |
| Example 103 | | >60 |
| Example 107 | >40 | |
| Example 111 | | >60 |
| Example 134 | | >60 |
| Example 140 | | >60 |
| Example 162 | >40 | |

### Test Example 5: Evaluation for In Vivo Pharmacokinetics in Mice

ICR mice weighing 20 g to 24 g were randomized into groups with 9 mice in each group after 3 to 5 days of acclimatization, and intragastric administration of the experimental compounds was performed at a dose of 10 mg/kg.

The test animals (ICR mice) were fasted for 12 h before administration and fed 4 h after administration, and were given free access to water before, after, and during the experiment.

After intragastric administration, about 0.1 mL of blood was collected via the orbital sinus at 0.25 h (15 min), 0.5 h (30 min), 1 h, 2 h, 4 h, 6 h, 8 h, and 10 h (each mouse was subjected to blood collection at 3 to 4 time points, with 3 mice per time point). Following anticoagulation with EDTA-K2, the blood samples were transferred to a centrifuge within 30 min and then centrifuged at 4000 rpm for 10 min at 4 °C to separate the plasma. All the plasma samples were collected and immediately stored at -20 °C for testing.

20 µL of the plasma sample to be tested and the standard curve sample were pipetted, and 400 µL of acetonitrile solution containing an internal standard (20 ng/mL) was added. The mixture was shaken and mixed well for 10 min, and centrifuged at 13000 rpm for 10 min. 50 µL of the supernatant was taken and diluted with 100 µL of ultrapure water. After being mixed well, 0.5 µL of the resulting sample was subjected to LC/MS/MS, and a chromatogram was recorded.

Oral exposure of the compounds of the present disclosure was evaluated by *in vivo* pharmacokinetic experiments in mice. The experimental results are shown in Table 3.

**Table 3**

| PK parameter | Compound 85 IG | Compound 87 IG | Compound 162 IG |
|---|---|---|---|
| Dose | 10 mg/kg | | |
| AUC(0-t h) (ng*h/mL) | 197 | 266 | 156*^{a}* |
| AUC(0-∞) (ng*h/mL) | 214 | 309 | / |
| MRT(0-t) (h) | 3.40 | 4.93 | / |
| t1/2(h) | 2.77 | 3.66 | / |
| Tmax (h) | 0.25 | 0.25 | 1.00 |
| Cmax (ng/mL) | 50.2 | 49.0 | 38.4 |

| | | | |
|---|---|---|---|
| *^{a}* gavage parameters were fitted to 6 h. | | | |

### Test Example 6: Evaluation for In Vivo Pharmacokinetics in Rats

SD rats weighing 180 g to 220 g were randomized into 3 groups with 3 rats in each group after 3 to 5 days of acclimatization, and intragastric administration of the compounds was performed at a dose of 10 mg/kg.

The test animals (SD rats) were fasted for 12 h before administration and fed 4 h after administration, and were given free access to water before, after, and during the experiment.

After intragastric administration, about 0.2 mL of blood was collected via the orbital sinus at 0 min, 15 min, 30 min, 1 h, 2 h, 4 h, 6 h, 8 h, 10 h, and 24 h. Following anticoagulation with EDTA-K2, the blood samples were transferred to a centrifuge within 30 min and then centrifuged at 4000 rpm for 10 min at 4 °C to separate the plasma. All the plasma samples were collected and immediately stored at -20 °C for testing.

50 µL of the plasma sample to be tested and the standard curve sample were pipetted, and 300 µL of acetonitrile solution containing an internal standard (20 mg/mL diazepam) was added. The mixture was shaken and mixed well for 5 min, and centrifuged at 13000 rpm for 10 min. 75 µL of the supernatant was taken and diluted with 75 µL of ultrapure water. After being mixed well, 2 µL of the resulting sample was subjected to LC/MS/MS, and a chromatogram was recorded.

### Test Example 7: Pharmacodynamic Evaluation for Aspc-1 Human Pancreatic Cancer Cell NOD-SCID Mouse Subcutaneous Xenograft Tumor Model

SPF female NOD-SCID mice (source: Jiangsu *Huachuang Sino* Pharmaceutical Technology Co., Ltd.) were inoculated subcutaneously at the right axilla with Aspc-1 human pancreatic cancer cells at 1 × 107 cells/mouse. When the mean tumor volume reached about 200 mm3, the animals were grouped.

The day of grouping was defined as day 0, and intraperitoneal injection was started on day 1 and performed once a day. The tumor volume was measured 2-3 times a week, and meanwhile, the mice were weighed and the data were recorded; the general behavior of the mice was observed and recorded every day. After the experiment was completed, the tumors were removed, weighed, and photographed.

The detection index and the calculation formulas are as follows:
tumor volume TV (mm3) = 1/2 × (a × b2), where a represents the long diameter of the tumor, and b represents the short diameter of the tumor;
relative tumor volume RTV = TVt/TV0, where TV0 represents the tumor volume on day 0, and TVt represents the tumor volume at each measurement;
relative tumor proliferation rate T/C (%) = (TRTV/CRTV) × 100%, where TRTV represents RTV of the treatment group, and CRTV represents RTV of the vehicle control group;
tumor growth inhibition rate TGI (%) = (1 - TW/TW0) × 100%; where TW represents the tumor weight of the treatment group, and TW0 represents the tumor weight of the vehicle control group;
weight change rate WCR (%) = (Wtt - Wt0)/Wt0 × 100%, where Wt0 represents the body weight of the mouse on day 0, and Wtt represents the body weight of the mouse at each measurement.

## Claims

1. A compound of formula (I), a stereoisomer thereof, a tautomer thereof, a deuterated compound thereof, or a pharmaceutically acceptable salt thereof, 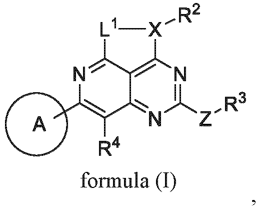 wherein,
X is selected from the group consisting of -N- and -CH-, the -CH- optionally substituted with R^{x};
R^{x} is selected from the group consisting of deuterium, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
L¹ is selected from the group consisting of -O-, -S-, and the following groups optionally substituted with one or more R¹: -NH-, C₁₋₅ alkylene, C₁₋₄ heteroalkylene, C₂₋₅ alkenylene, and C₁₋₄ heteroalkenylene;
each R¹ is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R² is selected from the group consisting of H, deuterium, halogen, -OH, -NH₂, -CN, and the following groups optionally substituted with one or more R^{2a}: C₁₋₁₂ alkyl, C₁₋₁₂ heteroalkyl, 3- to 12-membered cycloalkyl-L²-, 4- to 12-membered heterocyclyl-L²-, 6- to 10-membered aryl-L²-, and 5- to 10-membered heteroaryl-L²-;
L² is selected from the group consisting of a single bond, -O-, -S-, -NH-, -N(C₁₋₆ alkyl)-, C₁₋₆ alkylene, C₁₋₆ heteroalkylene, C₂₋₆ alkenylene, and C₁₋₆ heteroalkenylene;
each R^{2a} is independently selected from the group consisting of deuterium, halogen, -OH, oxo, -NH₂, -CN, -C(O)NR^{2c}R^{2d}, -NR^{2c}C(O)R^{2d}, -NHC(O)NR^{2c}R^{2d}, -NR^{2c}C(O)OR^{2d}, -OC(O)R^{2d}, -C(O)OR^{2d}, -OC(O)OR^{2d}, -OC(O)NR^{2c}R^{2d}, -SO₂R^{2d}, -NHSO₂R^{2d}, -C₁₋₆ alkylene C(O)NR^{2c}R^{2d}, -C₁₋₆ alkylene NR^{2c}C(O)R^{2d}, -C₁₋₆ alkylene NHC(O)NR^{2c}R^{2d}, -C₁₋₆ alkylene NR^{2c}C(O)OR^{2d}, -C₁₋₆ alkylene OC(O)R^{2d}, -C₁₋₆ alkylene C(O)OR^{2d}, -C₁₋₆ alkylene OC(O)NR^{2c}R^{2d}, -C₁₋₆ alkylene SO₂R^{2d}, -C₁₋₆ alkylene OSO₂R^{2d}, -C₁₋₆ alkylene NHSO₂R^{2d}, and the following groups optionally substituted with one or more R^{2b}: C₁₋₆ alkyl, C₁₋₆ alkoxy, C₁₋₆ alkylamino, di-C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{2c} is independently selected from the group consisting of H, deuterium, and C₁₋₆ alkyl;
R^{2d} is independently selected from the group consisting of H, deuterium, and the following groups optionally substituted with one or more R^{2e}: C₁₋₆ alkyl, C₁₋₆ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{2e} is independently selected from the group consisting of deuterium, halogen, -OH, oxo, -NH₂, -CN, C₁₋₄ alkyl, C₁₋₄ alkoxy, C₁₋₄ haloalkyl, and C₁₋₄ haloalkoxy;
each R^{2b} is independently selected from the group consisting of deuterium, halogen, -OH, -NH₂, -CN, C₁₋₄ alkyl, C₁₋₄ alkoxy, C₁₋₄ alkylamino, di-C₁₋₄ alkylamino, C₁₋₄ haloalkyl, and C₁₋₄ haloalkoxy;
Z is selected from the group consisting of a single bond, -S-, -O-, and the following groups optionally substituted with one or more R^{z}: -NH- and -N(C₁₋₁₂ alkyl)-;
each R^{z} is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
R³ is selected from the group consisting of H, deuterium, and the following groups optionally substituted with one or more R^{3a}: C₁₋₁₂ alkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3a} is independently selected from the group consisting of deuterium, halogen, -OH, oxo, -NH₂, -CN, -C(O)NR^{3c}R^{3d}, -NR^{3c}C(O)R^{3d}, -NHC(O)NR^{3c}R^{3d}, -NR^{3c}C(O)OR^{3d}, -OC(O)R^{3d}, -C(O)OR^{3d}, -OC(O)OR^{3d}, -OC(O)NR^{3c}R^{3d}, -SO₂R^{3d}, -NHSO₂R^{3d}, -C₁₋₆ alkylene C(O)NR^{3c}R^{3d}, -C₁₋₆ alkylene NR^{3c}C(O)R^{3d}, -C₁₋₆ alkylene NHC(O)NR^{3c}R^{3d}, -C₁₋₆ alkylene NR^{3c}C(O)OR^{3d}, -C₁₋₆ alkylene OC(O)R^{3d}, -C₁₋₆ alkylene C(O)OR^{3d}, -C₁₋₆ alkylene OC(O)NR^{3c}R^{3d}, -C₁₋₆ alkylene SO₂R^{3d}, -C₁₋₆ alkylene OSO₂R^{3d}, -C₁₋₆ alkylene NHSO₂R^{3d}, and the following groups optionally substituted with one or more R^{3b}: =NH, =CH₂, =N(C₁₋₆ alkyl), =CH(C₁₋₆ alkyl), =C(C₁₋₆ alkyl)₂, C₁₋₆ alkyl, C₁₋₆ alkoxy, C₁₋₆ alkylamino, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
R^{3c} is independently selected from the group consisting of H, deuterium, and C₁₋₆ alkyl;
R^{3d} is independently selected from the group consisting of H, deuterium, and the following groups optionally substituted with one or more R^{3e}: C₁₋₁₂ alkyl, C₁₋₁₂ heteroalkyl, 3- to 12-membered cycloalkyl, 3- to 12-membered heterocyclyl, 6- to 10-membered aryl, 5- to 10-membered heteroaryl, 3- to 12-membered cycloalkyl C₁₋₆ alkylene, 3- to 12-membered heterocyclyl C₁₋₆ alkylene, 6- to 10-membered aryl C₁₋₆ alkylene, and 5- to 10-membered heteroaryl C₁₋₆ alkylene;
each R^{3b} is independently selected from the group consisting of deuterium, halogen, -OH, oxo, -NH₂, -CN, C₁₋₄ alkyl, C₁₋₄ alkoxy, C₁₋₄ alkylamino, and di-C₁₋₄ alkylamino;
each R^{3e} is independently selected from the group consisting of deuterium, halogen, -OH, formyl, oxo, C₁₋₄ alkyl, and C₁₋₄ haloalkyl;
R⁴ is selected from the group consisting of H, deuterium, halogen, -CN, C₁₋₁₂ alkyl, C₁₋₁₂ alkoxy, C₁₋₁₂ haloalkyl, C₁₋₁₂ haloalkoxy, 3- to 12-membered cycloalkyl, 3- to 12-membered heterocyclyl, 6- to 10-membered aryl, and 5-to 10-membered heteroaryl;
ring A is selected from the group consisting of the following groups optionally substituted with one or more R⁵: 3-to 12-membered cycloalkyl, 3- to 12-membered heterocyclyl, 6- to 10-membered aryl, and 5- to 10-membered heteroaryl;
each R⁵ is independently selected from the group consisting of deuterium, halogen, -CN, -OH, -NH₂, and the following groups optionally substituted with one or more R^{5a}: C₁₋₁₂ alkyl, C₂₋₁₂ alkenyl, C₂₋₁₂ alkynyl, C₁₋₁₂ alkoxy, C₁₋₁₂ alkylamino, di-C₁₋₁₂ alkylamino, C₁₋₁₂ alkylthio, 3- to 12-membered cycloalkyl, 6- to 10-membered aryl, 5-to 10-membered heteroaryl, and 3- to 12-membered heterocyclyl;
each R^{5a} is independently selected from the group consisting of deuterium, halogen, -OH, oxo, -NH₂, -CN, C₁₋₄ alkyl, C₁₋₄ alkoxy, C₁₋₄ alkylamino, and di-C₁₋₄ alkylamino;
each L¹, L², R¹, R², R^{2a}, R^{2b}, R^{2c}, R^{2d}, R^{2e}, R³, R^{3a}, R^{3b}, R^{3c}, R^{3d}, R^{3e}, R⁴, R⁵, R^{5a}, R^{x}, or R^{z} is independently optionally substituted with one or more substituents.

2. The compound of formula (I), the stereoisomer thereof, the tautomer thereof, the deuterated compound thereof, or the pharmaceutically acceptable salt thereof according to claim 1, wherein R^{x} is selected from the group consisting of deuterium, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino;
or R^{x} is selected from the group consisting of deuterium, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, and C₁₋₆ haloalkoxy;
or R^{x} is selected from the group consisting of deuterium, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy;
or R^{x} is selected from the group consisting of deuterium, F, Cl, Br, -OH, -NH₂, -CN, C₁₋₃ alkyl, C₁₋₃ alkoxy, C₁₋₃ haloalkyl, and C₁₋₃ haloalkoxy;
or R^{x} is selected from the group consisting of deuterium, F, Cl, Br, -OH, -NH₂, -CN, methyl, ethyl, isopropyl, methoxy, ethoxy, isopropoxy, trifluoromethyl, and trifluoromethoxy;
or X is selected from the group consisting of -N-, -CH-, -C(C₁₋₆ alkyl)-, and -C(C₁₋₆ haloalkyl)-;
or X is selected from the group consisting of -N-, -CH-, -C(C₁₋₃ alkyl)-, and -C(C₁₋₃ haloalkyl)-;
or X is selected from -N-.

3. The compound of formula (I), the stereoisomer thereof, the tautomer thereof, the deuterated compound thereof, or the pharmaceutically acceptable salt thereof according to claim 1 or 2, wherein L¹ is selected from the group consisting of -O-, -S-, and the following groups optionally substituted with one or more R¹: -NH-, C₁₋₅ alkylene, C₁₋₄ heteroalkylene, C₂₋₅ alkenylene, and C₂₋₄ heteroalkenylene;
or L¹ is selected from the group consisting of -O- and the following groups optionally substituted with one or more R¹: -NH-, C₂₋₄ alkylene, C₁₋₃ heteroalkylene, C₂₋₄ alkenylene, and C₁₋₃ heteroalkenylene;
or L¹ is selected from the group consisting of -O- and the following groups optionally substituted with one or more R¹: -NH-, C₂₋₄ alkylene, C₁₋₃ heteroalkylene, C₂₋₄ alkenylene, and C₂₋₃ heteroalkenylene;
or L¹ is selected from the group consisting of -O- and the following groups optionally substituted with one or more R¹: -NH-, C₂₋₄ alkylene, and C₁₋₃ heteroalkylene;
or L¹ is selected from the group consisting of -O- and the following groups optionally substituted with one or more R¹: -NH-, -CH₂CH₂-, -(CH₂)₃-, -(CH₂)₄-, -OCH₂-, -CH₂O-, -OCH₂CH₂-, -CH₂OCH₂-, -CH₂CH₂O-, -O(CH₂)₃-, -CH₂OCH₂CH₂-, -CH₂CH₂OCH₂-, -CH₂CH₂CH₂O-, -NHCH₂-, -NHCH₂CH₂-, -CH₂NHCH₂-, -CH₂CH₂NH-, -NH(CH₂)₃-, -CH₂NHCH₂CH₂-, -CH₂CH₂NHCH₂-, and -CH₂CH₂CH₂NH-;
or L¹ is selected from the group consisting of -O- and the following groups optionally substituted with one or more R¹: -NH-, -CH₂CH₂-, -(CH₂)₃-, -(CH₂)₄-, -OCH₂-, -OCH₂CH₂-, -O(CH₂)₃-, -NHCH₂-, -NHCH₂CH₂-, and -NH(CH₂)₃-;
or L¹ is selected from the group consisting of -O-, -NH-, -N(C₁₋₃ alkyl)-, and the following groups optionally substituted with one or more R¹: -CH₂CH₂-, -(CH₂)₃-, -(CH₂)₄-, -OCH₂-, -OCH₂CH₂-, -O(CH₂)₃-, -NHCH₂-, -NHCH₂CH₂-, and -NH(CH₂)₃-;
or L¹ is selected from the group consisting of -O-, -NH-, -N(CH₃)-, and the following groups optionally substituted with one or more R¹: -CH₂CH₂-, -(CH₂)₃-, -(CH₂)₄-, -OCH₂-, -OCH₂CH₂-, -O(CH₂)₃-, -NHCH₂-, -NHCH₂CH₂-, and -NH(CH₂)₃-;
or L¹ is selected from the group consisting of the following groups optionally substituted with one or more R¹: -(CH₂)₃-, -OCH₂CH₂-, and -NHCH₂CH₂-;
or L¹ is selected from -OCH₂CH₂- optionally substituted with one or more R¹.

4. The compound of formula (I), the stereoisomer thereof, the tautomer thereof, the deuterated compound thereof, or the pharmaceutically acceptable salt thereof according to any one of claims 1-3, wherein each R¹ is independently selected from the group consisting of deuterium, oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, C₁₋₃ haloalkoxy, C₁₋₃ haloalkylthio, C₁₋₃ haloalkylamino, and di-C₁₋₃ haloalkylamino;
or each R¹ is independently selected from the group consisting of deuterium, oxo, halogen, -OH, -NH₂, -CN, C₁₋₆ alkyl, C₁₋₆ alkoxy, C₁₋₆ alkylthio, C₁₋₆ alkylamino, di-C₁₋₆ alkylamino, C₁₋₆ haloalkyl, and C₁₋₆ haloalkoxy;
or each R¹ is independently selected from the group consisting of deuterium, oxo, halogen, -OH, -NH₂, -CN, C₁₋₃ alkyl, C₁₋₃ alkoxy, C₁₋₃ alkylthio, C₁₋₃ alkylamino, di-C₁₋₃ alkylamino, C₁₋₃ haloalkyl, and C₁₋₃ haloalkoxy;
or each R¹ is independently selected from the group consisting of deuterium, F, Cl, Br, -OH, oxo, -NH₂, -CN, C₁₋₃ alkyl, C₁₋₃ alkoxy, C₁₋₃ haloalkyl, and C₁₋₃ haloalkoxy;
or each R¹ is independently selected from the group consisting of deuterium, F, Cl, Br, -OH, oxo, -NH₂, -CN, methyl, ethyl, isopropyl, methoxy, ethoxy, isopropoxy, trifluoromethyl, trifluoromethoxy, and -CH₂F;
or each R¹ is independently selected from the group consisting of deuterium, methyl, ethyl, isopropyl, trifluoromethyl, and -CH₂F.

5. The compound of formula (I), the stereoisomer thereof, the tautomer thereof, the deuterated compound thereof, or the pharmaceutically acceptable salt thereof according to any one of claims 1-4, wherein R² is selected from the group consisting of H, deuterium, halogen, -OH, -NH₂, -CN, and the following groups optionally substituted with one or more R^{2a}: C₁₋₆ alkyl, C₁₋₆ heteroalkyl, 3- to 10-membered cycloalkyl-L²-, 4- to 10-membered heterocyclyl-L²-, 6- to 10-membered aryl-L²-, and 5- to 10-membered heteroaryl-L²-;
or R² is selected from the group consisting of H, deuterium, halogen, -OH, -NH₂, -CN, and the following groups optionally substituted with one or more R^{2a}: C₁₋₄ alkyl, C₁₋₄ heteroalkyl, 3- to 10-membered cycloalkyl-L²-, 4- to 10-membered heterocyclyl-L²-, phenyl-L²-, naphthyl-L²-, 5- to 6-membered heteroaryl-L²-, and benzo 5- to 6-membered heteroaryl-L²-;
or R² is selected from the group consisting of H, deuterium, F, Cl, Br, -OH, -NH₂, -CN, and the following groups optionally substituted with one or more R^{2a}: C₁₋₄ alkyl, C₁₋₄ heteroalkyl, 3- to 8-membered cycloalkyl-L²-, 4- to 8-membered heterocyclyl-L²-, phenyl-L²-, naphthyl-L²-, 5- to 6-membered heteroaryl-L²-, benzo 5- to 6-membered heterocyclyl-L²-, benzo 5- to 6-membered heteroaryl-L²-, and 5- to 6-membered heteroaryl-fused 5-to 6-membered cycloalkenyl-L²-;
or R² is selected from the group consisting of H, deuterium, F, Cl, Br, -OH, -NH₂, -CN, and the following groups optionally substituted with one or more R^{2a}: C₁₋₄ alkyl, C₁₋₄ heteroalkyl, 3- to 8-membered cycloalkyl-L²-, 4- to 8-membered heterocycloalkyl-L²-, phenyl-L²-, naphthyl-L²-, 5- to 6-membered heteroaryl-L²-, benzo 5- to 6-membered heterocyclyl-L²-, benzo 5- to 6-membered heteroaryl-L²-, and 5- to 6-membered heteroaryl-fused 5-to 6-membered cycloalkenyl-L²-;
or R² is selected from the group consisting of H, deuterium, F, Cl, Br, -OH, -NH₂, -CN, and the following groups optionally substituted with one or more R^{2a}: methyl, ethyl, n-propyl, isopropyl, n-butyl, isobutyl, sec-butyl, *tert-butyl,* C₁₋₄ heteroalkyl, cyclopropanyl, cyclobutanyl, cyclopentanyl, 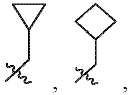 oxetanyl, azetidinyl, pyrrolidinyl, piperidinyl, pyrazolyl, 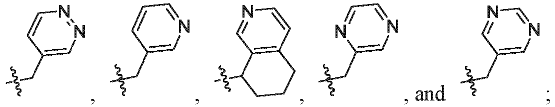
or R² is selected from the group consisting of H, deuterium, methyl, ethyl, isopropyl, *tert-butyl,* trifluoromethyl, cyclopropanyl, cyclobutanyl, cyclopentanyl,

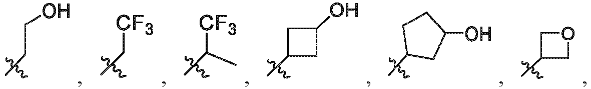

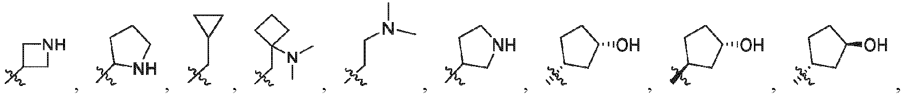

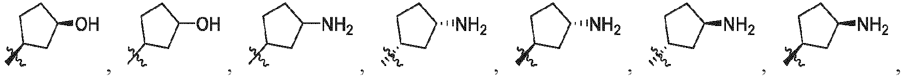

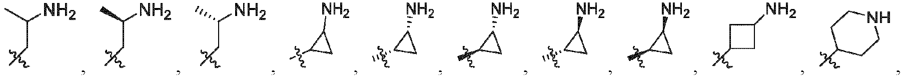

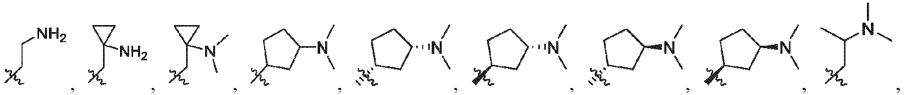

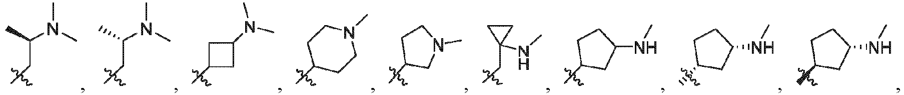

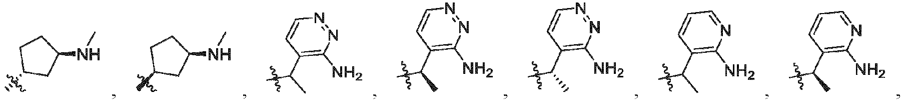

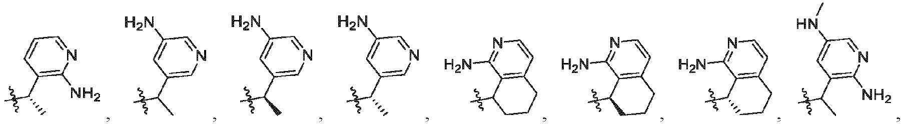

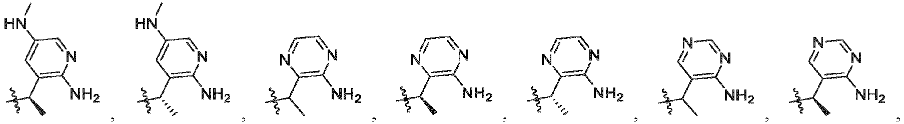

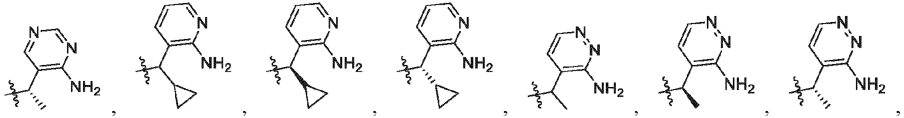

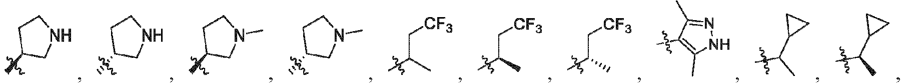

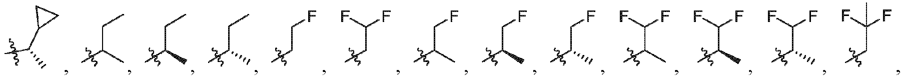

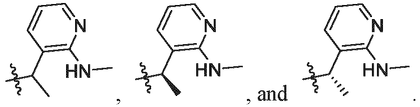

6. The compound of formula (I), the stereoisomer thereof, the tautomer thereof, the deuterated compound thereof, or the pharmaceutically acceptable salt thereof according to any one of claims 1-5, wherein Z is selected from the group consisting of a single bond, -S-, -O-, and the following groups optionally substituted with one or more R^{z}: -NH- and -N(C₁₋₆ alkyl)-;
or Z is selected from the group consisting of a single bond, -S-, -O-, -NH-, and -N(C₁₋₁₂ alkyl)-;
or Z is selected from the group consisting of a single bond, -S-, -O-, -NH-, and -N(C₁₋₆ alkyl)-;
or Z is selected from the group consisting of a single bond, -S-, -O-, -NH-, and -N(C₁₋₃ alkyl)-;
or Z is selected from the group consisting of a single bond, -O-, and -N(C₁₋₃ alkyl)-;
or Z is selected from the group consisting of a single bond, -O-, -NH-, and -N(CH₃)-;
or Z is selected from the group consisting of a single bond, -O-, and -N(CH₃)-;
or Z is selected from -O-.

7. The compound of formula (I), the stereoisomer thereof, the tautomer thereof, the deuterated compound thereof, or the pharmaceutically acceptable salt thereof according to any one of claims 1-6, wherein R³ is selected from the group consisting of H, deuterium, and the following groups optionally substituted with one or more R^{3a}: C₁₋₆ alkyl, 3- to 10-membered cycloalkyl, 3- to 10-membered heterocyclyl, 6- to 10-membered aryl, 5- to 10-membered heteroaryl, 3- to 10-membered cycloalkyl C₁₋₄ alkylene, 3- to 10-membered heterocyclyl C₁₋₄ alkylene, 6- to 10-membered aryl C₁₋₄ alkylene, and 5- to 10-membered heteroaryl C₁₋₄ alkylene;
or R³ is selected from the group consisting of H, deuterium, and the following groups optionally substituted with one or more R^{3a}: C₁₋₄ alkyl, 3- to 10-membered cycloalkyl, **4-** to 10-membered heterocyclyl, phenyl, 5- to 6-membered heteroaryl, 3- to 10-membered cycloalkyl C₁₋₃ alkylene, **4-** to 10-membered heterocyclyl C₁₋₃ alkylene, phenyl C₁₋₃ alkylene, and 5- to 6-membered heteroaryl C₁₋₃ alkylene;
or R³ is selected from the group consisting of H, deuterium, and the following groups optionally substituted with one or more R^{3a}: C₁₋₄ alkyl, 3- to 8-membered cycloalkyl, **4-** to 8-membered heterocyclyl, phenyl, 5- to 6-membered heteroaryl, 3- to 8-membered cycloalkyl C₁₋₃ alkylene, **4-** to 8-membered heterocyclyl C₁₋₃ alkylene, phenyl C₁₋₃ alkylene, and 5- to 6-membered heteroaryl C₁₋₃ alkylene;
or R³ is selected from the group consisting of H, deuterium, and the following groups optionally substituted with one or more R^{3a}: C₁₋₄ alkyl, 3- to 8-membered cycloalkyl, **4-** to 8-membered heterocyclyl, 3- to 8-membered cycloalkyl C₁₋₃ alkylene, **4-** to 8-membered heterocyclyl C₁₋₃ alkylene, and 5- to 6-membered heteroaryl C₁₋₃ alkylene;
or R³ is selected from the group consisting of H, deuterium, and the following groups optionally substituted with one or more R^{3a}: C₁₋₄ alkyl, **4-** to 8-membered heterocyclyl, 3- to 8-membered cycloalkyl C₁₋₃ alkylene, **4- to** 8-membered heterocyclyl C₁₋₃ alkylene, and 5- to 6-membered heteroaryl C₁₋₃ alkylene;
or R³ is selected from the group consisting of H, deuterium, and the following groups optionally substituted with one or more R^{3a}: C₁₋₄ alkyl, **4-** to 8-membered heterocycloalkyl, 3- to 8-membered cycloalkyl C₁₋₃ alkylene, 4- to 8-membered heterocycloalkyl C₁₋₃ alkylene, and 5- to 6-membered heteroaryl C₁₋₃ alkylene;
or R³ is selected from the group consisting of H, deuterium, and the following groups optionally substituted with one or more R^{3a}: methyl, ethyl, propyl, butyl, cyclopropyl, cyclobutyl, cyclopentyl, cyclohexyl, azetidinyl, tetrahydropyrrolyl, morpholinyl, piperidinyl, piperazinyl, hexahydro-1H-pyrrolizinyl, cyclopropyl C₁₋₃ alkylene, cyclobutyl C₁₋₃ alkylene, cyclopentyl C₁₋₃ alkylene, cyclohexyl C₁₋₃ alkylene, azetidinyl C₁₋₃ alkylene, tetrahydropyrrolyl C₁₋₃ alkylene, morpholinyl C₁₋₃ alkylene, piperidinyl C₁₋₃ alkylene, piperazinyl C₁₋₃ alkylene, hexahydro-1H-pyrrolizinyl C₁₋₃ alkylene, hexahydro-1*H*-pyrrolo[2,1-*c*][1,4]oxazinyl, hexahydro-1*H*-pyrrolo[2,1-*c*][1,4]oxazinyl C₁₋₃ alkylene, 5-azaspiro[2.4]heptanyl, 5-azaspiro[2.4]heptanyl C₁₋₃ alkylene, n-propyl, isopropyl, n-butyl, isobutyl, sec-butyl, *tert*-butyl, and imidazolyl C₁₋₃ alkylene;
or R³ is selected from the group consisting of H, deuterium, and the following groups optionally substituted with one or more R^{3a}: methyl, piperidinyl, cyclopropyl C₁₋₃ alkylene, cyclopentyl C₁₋₃ alkylene, tetrahydropyrrolyl C₁₋₃ alkylene, hexahydro-1H-pyrrolizinyl C₁₋₃ alkylene, hexahydro-1*H*-pyrrolo[2,1-*c*][1,4]oxazinyl C₁₋₃ alkylene, 5-azaspiro[2.4]heptanyl C₁₋₃ alkylene, ethyl, n-propyl, isopropyl, n-butyl, isobutyl, sec-butyl, *tert*-butyl, morpholinyl C₁₋₃ alkylene, imidazolyl C₁₋₃ alkylene, tetrahydropyrrolyl, piperazinyl C₁₋₃ alkylene, azetidinyl C₁₋₃ alkylene, piperidinyl C₁₋₃ alkylene, azetidinyl, cyclobutyl, cyclohexyl, cyclobutyl C₁₋₃ alkylene, and piperazinyl; or R³ is selected from the group consisting of H, deuterium, and the following groups optionally substituted with one or more R^{3a}: 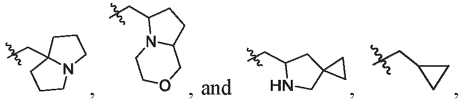 methyl, ethyl, n-propyl, isopropyl, n-butyl, isobutyl, sec-butyl, 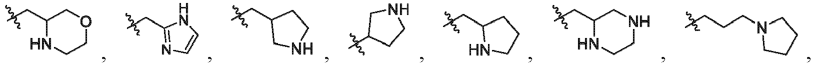 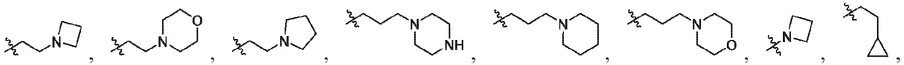 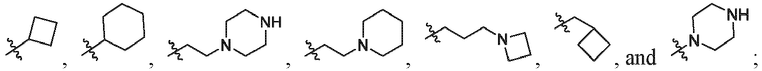
or R³ is selected from the group consisting of H, deuterium, methyl,

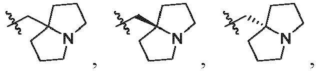

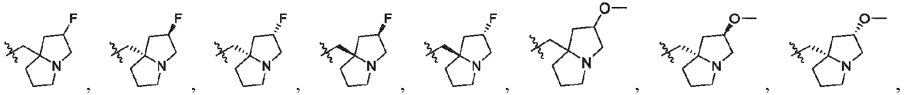

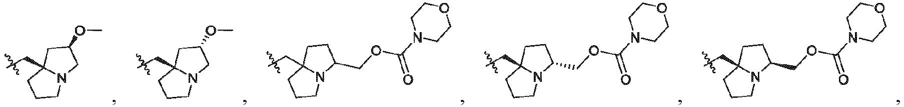

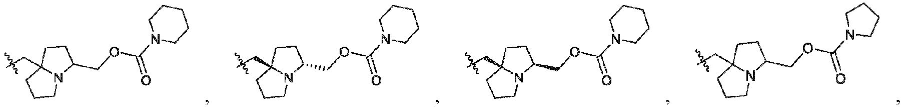

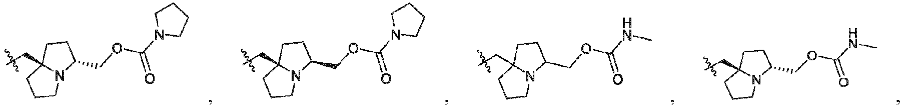

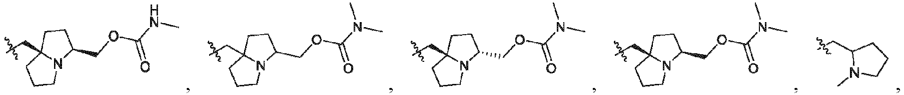

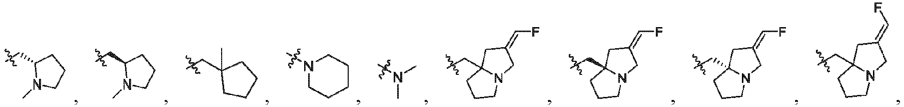

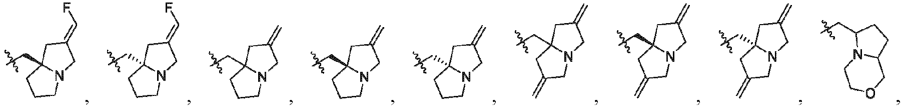

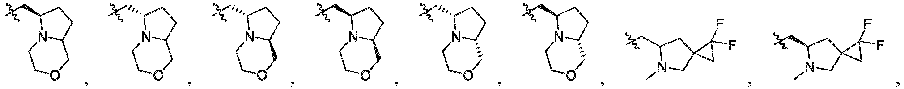

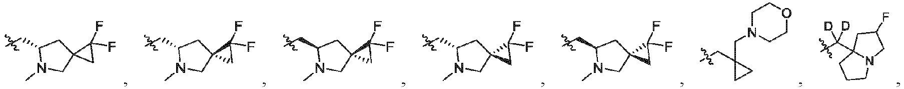

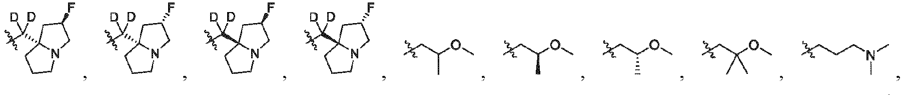

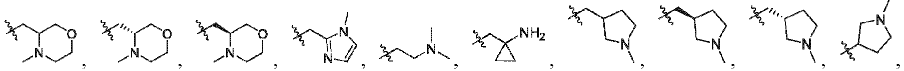

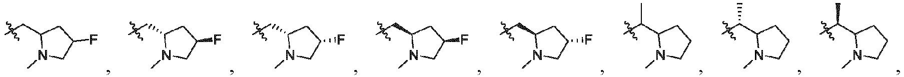

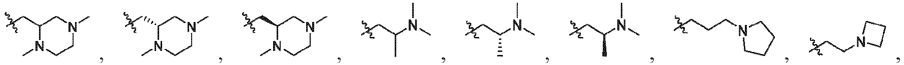

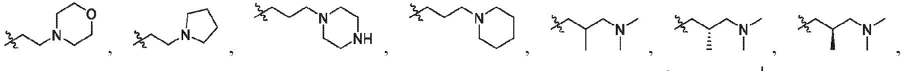

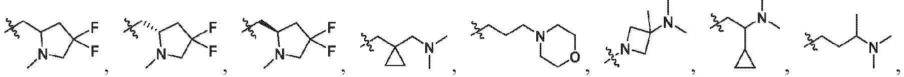

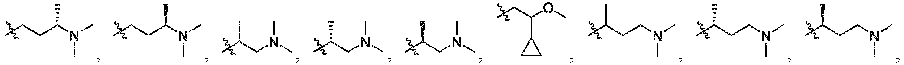

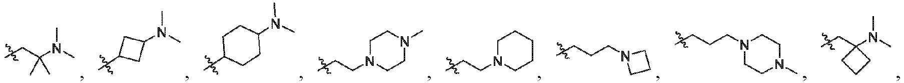

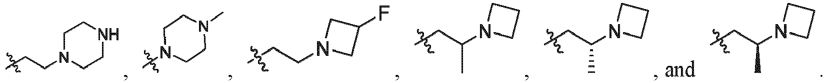

8. The compound of formula (I), the stereoisomer thereof, the tautomer thereof, the deuterated compound thereof, or the pharmaceutically acceptable salt thereof according to any one of claims 1-7, wherein R⁴ is selected from the group consisting of H, deuterium, halogen, -CN, C₁₋₆ alkyl, C₁₋₆ alkoxy, C₁₋₆ haloalkyl, C₁₋₆ haloalkoxy, 3- to 12-membered cycloalkyl, 3- to 12-membered heterocyclyl, 6- to 10-membered aryl, and 5- to 10-membered heteroaryl;
or R⁴ is selected from the group consisting of H, deuterium, halogen, -CN, C₁₋₄ alkyl, C₁₋₄ alkoxy, C₁₋₄ haloalkyl, C₁₋₄ haloalkoxy, 3- to 6-membered cycloalkyl, 5- to 6-membered heterocyclyl, phenyl, and 5- to 6-membered heteroaryl;
or R⁴ is selected from the group consisting of H, deuterium, halogen, -CN, C₁₋₄ alkyl, C₁₋₄ alkoxy, C₁₋₄ haloalkyl, and C₁₋₄ haloalkoxy;
or R⁴ is selected from the group consisting of H, deuterium, -F, -Cl, -Br, -CN, C₁₋₂ alkyl, C₁₋₂ alkoxy, C₁₋₂ haloalkyl, and C₁₋₂ haloalkoxy;
or R⁴ is selected from the group consisting of H, deuterium, -F, -Cl, -Br, C₁₋₂ alkyl, C₁₋₂ alkoxy, and C₁₋₂ fluoroalkyl;
or R⁴ is selected from the group consisting of H, deuterium, -F, -Cl, -Br, methyl, methoxy, difluoromethyl, and trifluoromethyl;
or R⁴ is selected from the group consisting of H, deuterium, -F, -Cl, and methyl;
or R⁴ is selected from the group consisting of H, deuterium, -F, and -Cl.

9. The compound of formula (I), the stereoisomer thereof, the tautomer thereof, the deuterated compound thereof, or the pharmaceutically acceptable salt thereof according to any one of claims 1-8, wherein ring A is selected from the group consisting of the following groups optionally substituted with one or more R⁵: 3- to 6-membered cycloalkyl, 5- to 10-membered heterocyclyl, 6- to 10-membered aryl, and 5- to 10-membered heteroaryl;
or ring A is selected from the group consisting of the following groups optionally substituted with one or more R⁵: 6- to 10-membered aryl, benzo 4- to 6-membered cycloalkenyl, benzo 4- to 6-membered heterocyclyl, and 5- to 10-membered heteroaryl;
or ring A is selected from the group consisting of the following groups optionally substituted with one or more R⁵: phenyl, naphthyl, benzocyclohexenyl, benzocyclopentenyl, pyrrolyl, pyrazolyl, imidazolyl, furanyl, oxazolyl, isoxazolyl, thienyl, thiazolyl, isothiazolyl, pyranyl, pyridinyl, pyridazinyl, pyrimidinyl, pyrazinyl, indolyl, benzopyrazolyl, benzimidazolyl, benzothiazolyl, quinolyl, isoquinolyl, benzopyrimidinyl, benzothienyl, pyridopyrazolyl, and pyridopyrrolyl;
or ring A is selected from the group consisting of the following groups optionally substituted with one or more R⁵: phenyl, naphthyl, benzocyclohexenyl, benzocyclopentenyl, pyridinyl, indolyl, benzopyrazolyl, benzimidazolyl, benzothiazolyl, quinolyl, isoquinolyl, benzothienyl, pyrazolyl, pyridopyrazolyl, and pyridopyrrolyl;
or ring A is selected from the group consisting of the following groups optionally substituted with one or more R⁵: phenyl, naphthyl, pyridinyl, benzothiazolyl, benzothienyl, isoquinolyl, benzopyrazolyl, pyrazolyl, pyridopyrazolyl, pyridopyrrolyl, quinolyl, indolyl, and benzimidazolyl;
or ring A is selected from the group consisting of the following groups optionally substituted with one or more R⁵: phenyl, naphthyl, pyridinyl, isoquinolyl, benzopyrazolyl, benzothiazolyl, pyrazolyl, pyridopyrazolyl, pyridopyrrolyl, quinolyl, indolyl, and benzimidazolyl;
or ring A is selected from the group consisting of

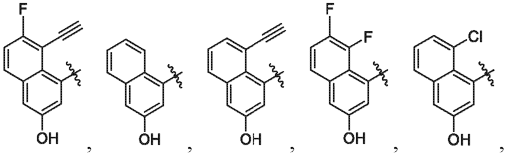

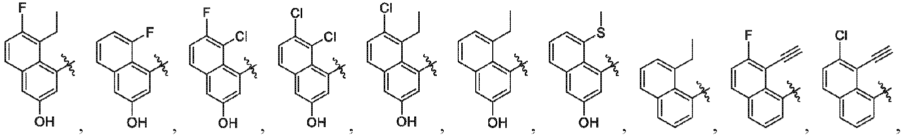

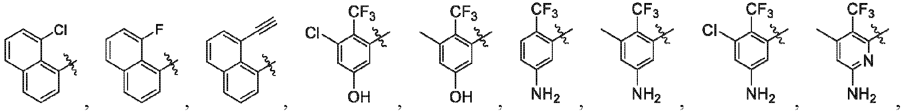

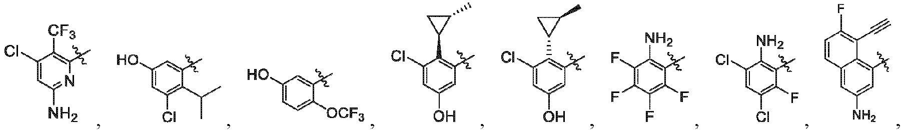

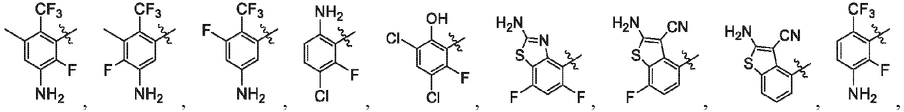

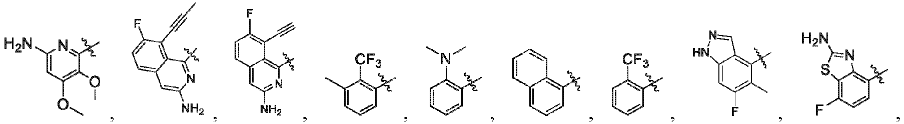

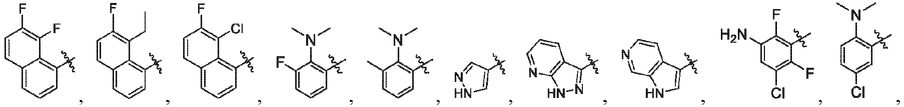

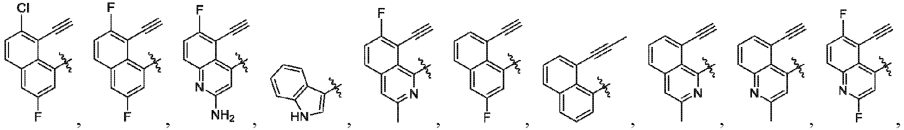

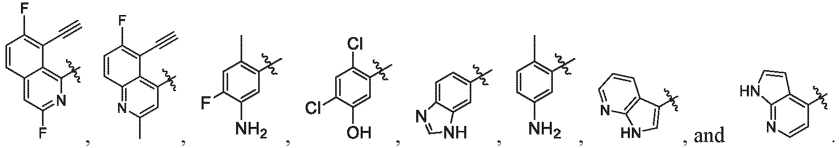

10. The compound of formula (I), the stereoisomer thereof, the tautomer thereof, the deuterated compound thereof, or the pharmaceutically acceptable salt thereof according to any one of claims 1-9, wherein each R⁵ is independently selected from the group consisting of deuterium, halogen, -CN, -OH, -NH₂, and the following groups optionally substituted with one or more R^{5a}: C₁₋₆ alkyl, C₂₋₆ alkenyl, C₂₋₆ alkynyl, C₁₋₆ alkoxy, C₁₋₆ alkylamino, di-C₁₋₆ alkylamino, C₁₋₆ alkylthio, 3- to 12-membered cycloalkyl, 6- to 10-membered aryl, 5- to 10-membered heteroaryl, and 3- to 12-membered heterocyclyl;
or each R⁵ is independently selected from the group consisting of deuterium, -F, -Cl, -Br, -CN, -OH, -NH₂, and the following groups optionally substituted with one or more R^{5a}: methyl, ethyl, isopropyl, ethenyl, ethynyl, propynyl, methoxy, methylamino, dimethylamino, methylthio, 3- to 6-membered cycloalkyl, and 3- to 6-membered heterocyclyl;
or each R⁵ is independently selected from the group consisting of deuterium, -F, -Cl, -CN, -OH, -NH₂, and the following groups optionally substituted with one or more R^{5a}: methyl, ethyl, isopropyl, ethenyl, ethynyl, propynyl, methoxy, methylthio, and cyclopropanyl;
or each R⁵ is independently selected from the group consisting of deuterium, -F, -Cl, -CN, -OH, -NH₂, methyl, ethyl, isopropyl, ethenyl, ethynyl, propynyl, trifluoromethyl, hydroxymethylene, methoxy, trifluoromethoxy, methylamino, dimethylamino, methylthio, trifluoromethylthio, and cyclopropanyl optionally substituted with -F, -Cl, -Br, or methyl;
or each R⁵ is independently selected from the group consisting of deuterium, -F, -Cl, -CN, -OH, -NH₂, methyl, ethyl, isopropyl, ethynyl, propynyl, trifluoromethyl, methoxy, trifluoromethoxy, methylthio, and cyclopropanyl substituted with methyl.

11. The compound of formula (I), the stereoisomer thereof, the tautomer thereof, the deuterated compound thereof, or the pharmaceutically acceptable salt thereof according to any one of claims 1-10, wherein the compound of formula (I), the stereoisomer thereof, the tautomer thereof, the deuterated compound thereof, or the pharmaceutically acceptable salt thereof is selected from the group consisting of a compound of formula (II-1) or formula (II-2) or formula (III) or formula (III-a) or formula (III-b) or formula (III-c) or formula (IV) or formula (IV-a) or formula (IV-b) or formula (IV-c), a stereoisomer thereof, a tautomer thereof, a deuterated compound thereof, and a pharmaceutically acceptable salt thereof, 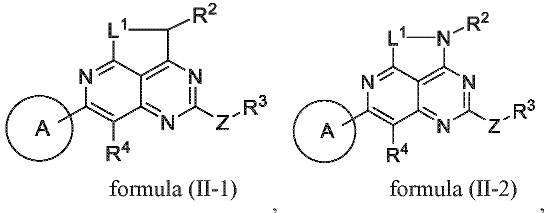 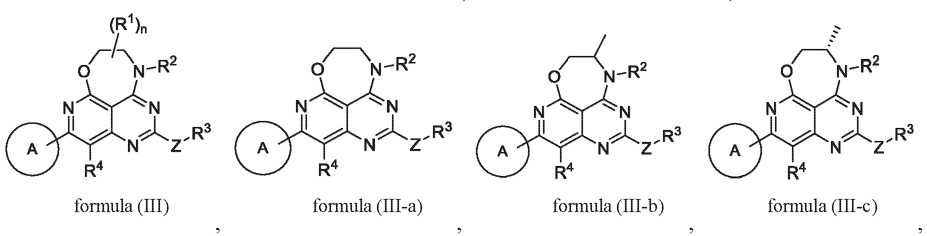 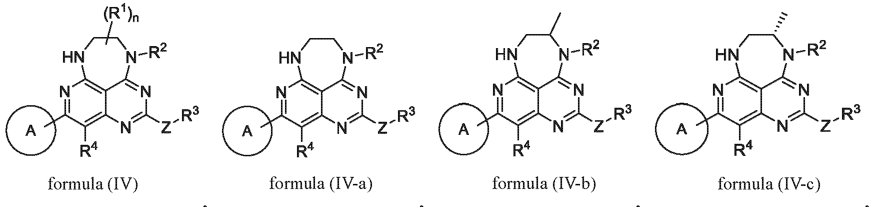 wherein L¹, R¹, R², R³, R⁴, Z, and ring A moieties are as defined in any one of claims 1-10; n is selected from the group consisting of 0, 1, 2, 3, 4, and 5.

12. The compound of formula (I), the stereoisomer thereof, the tautomer thereof, the deuterated compound thereof, or the pharmaceutically acceptable salt thereof according to any one of claims 1-11, wherein the compound of formula (I), the stereoisomer thereof, the tautomer thereof, the deuterated compound thereof, or the pharmaceutically acceptable salt thereof is selected from the group consisting of: 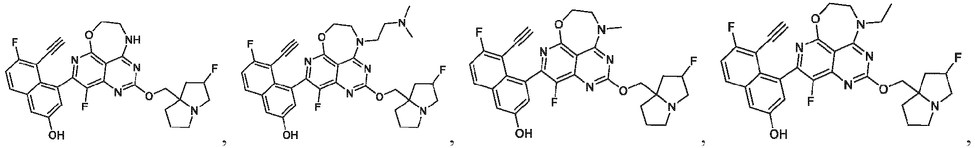 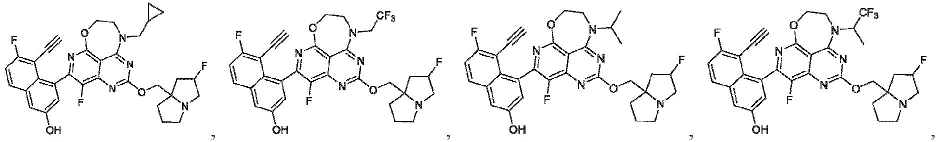 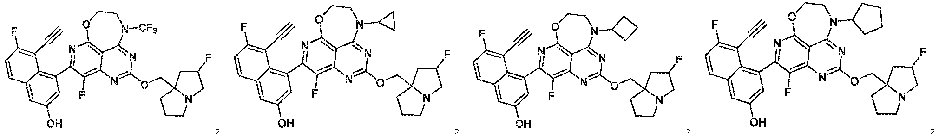 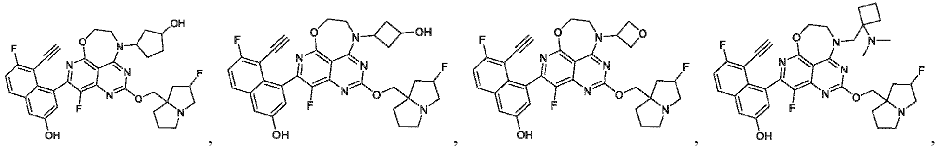 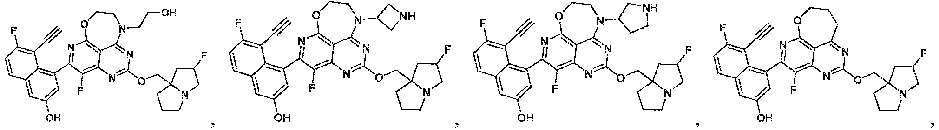 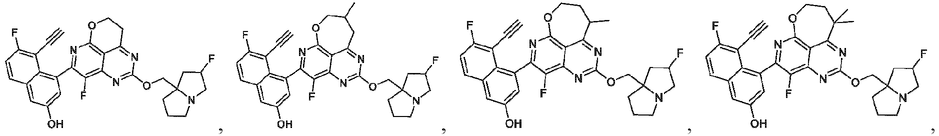 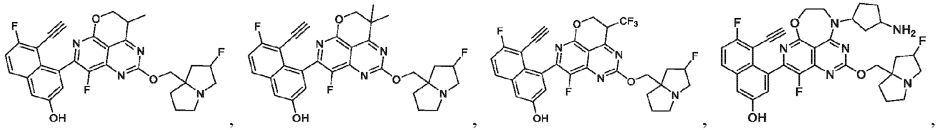 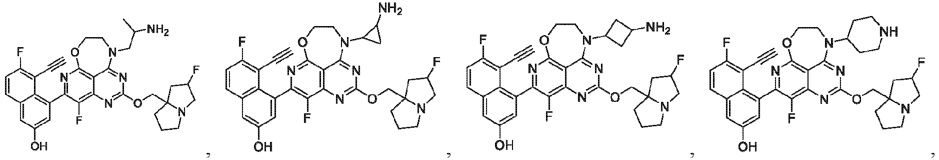 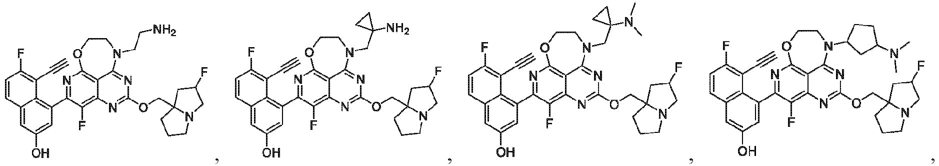 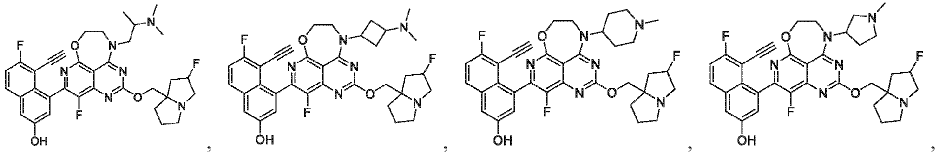 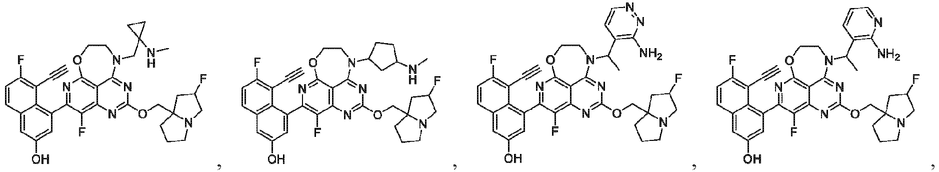 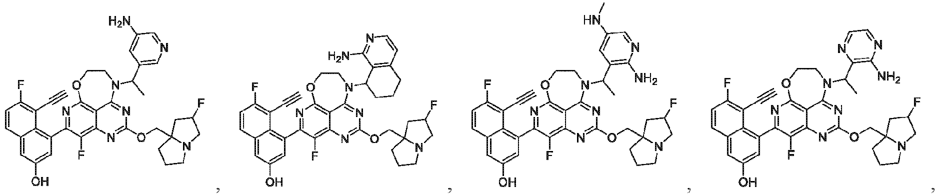 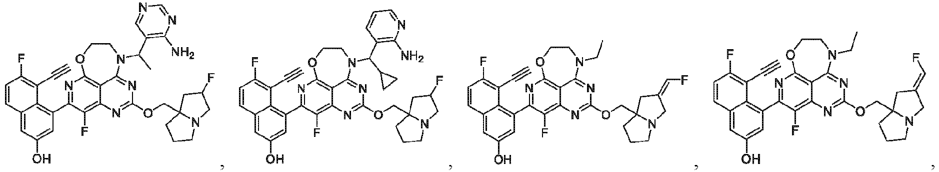 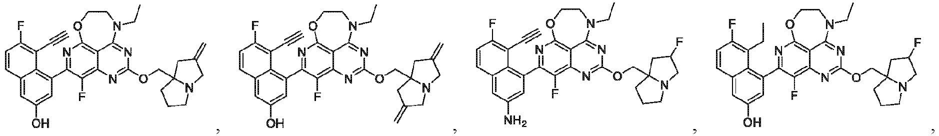 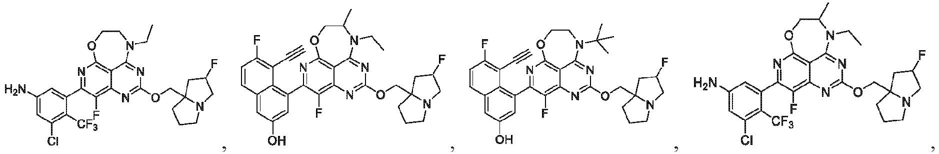 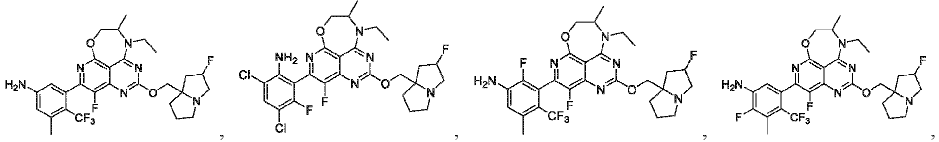 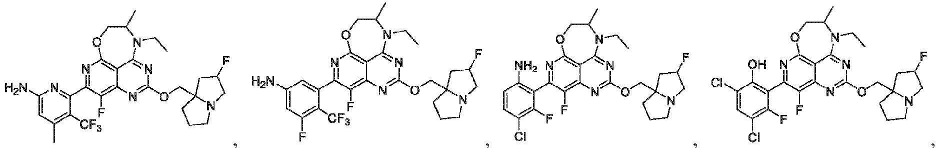 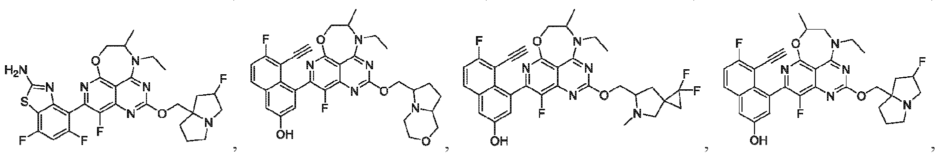 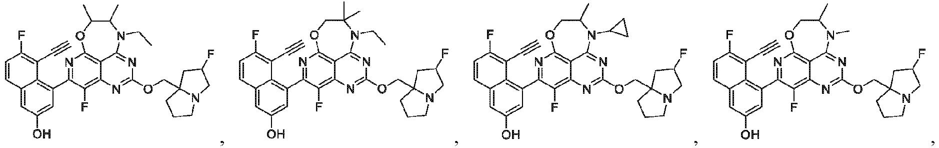 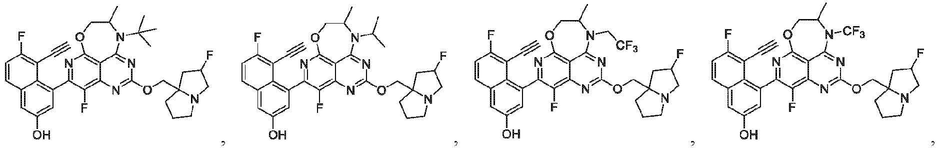 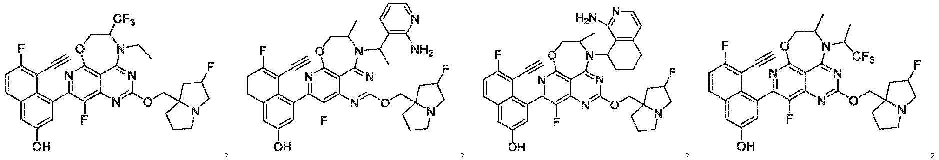 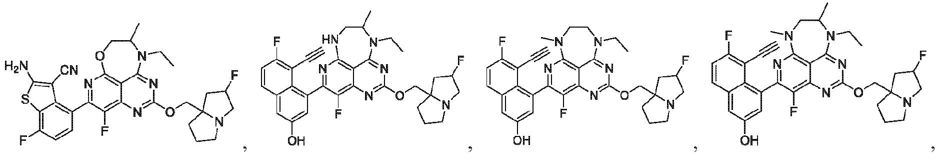 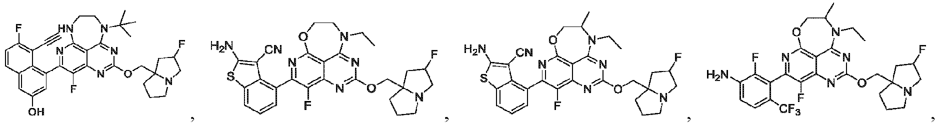 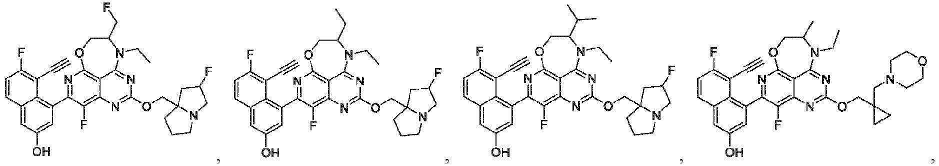 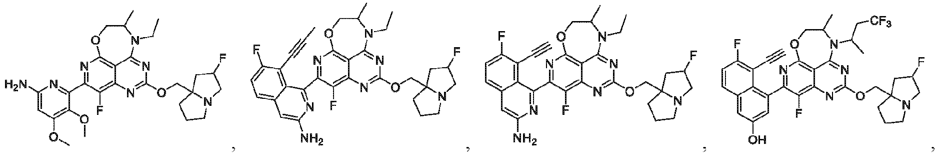 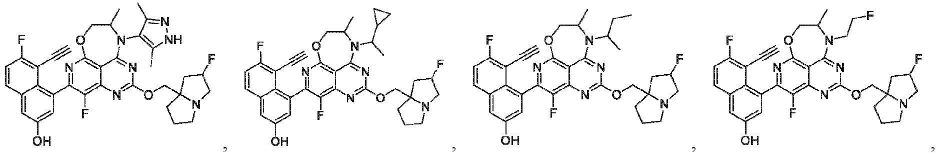 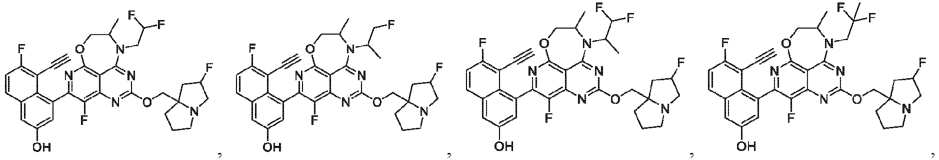 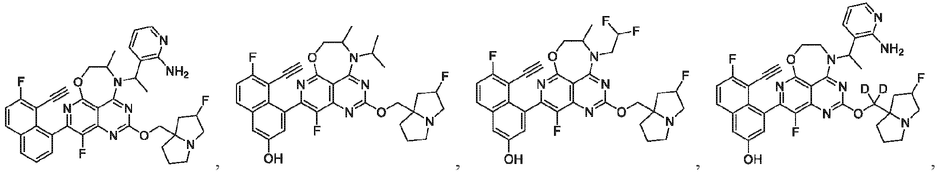 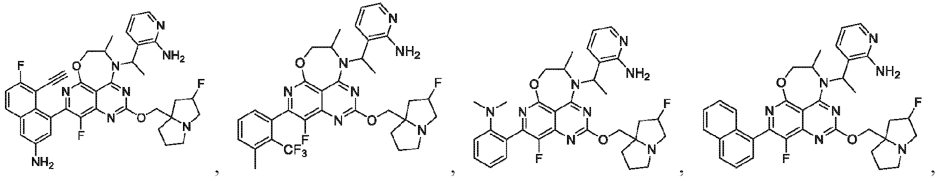 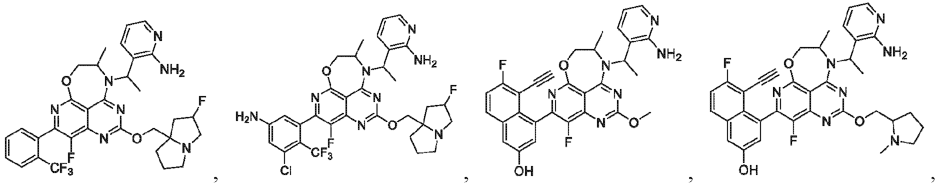 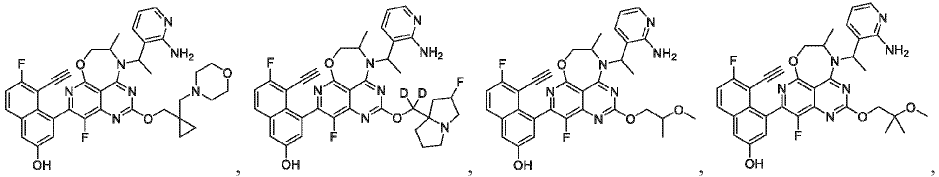 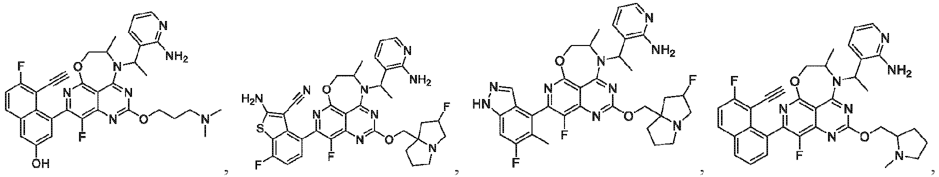 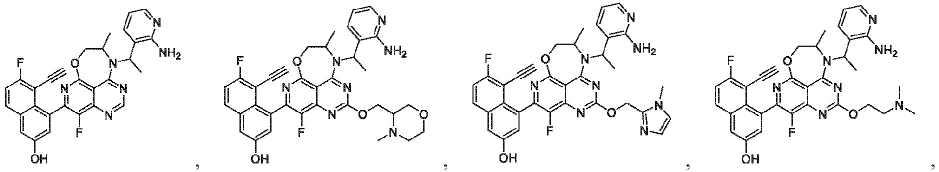 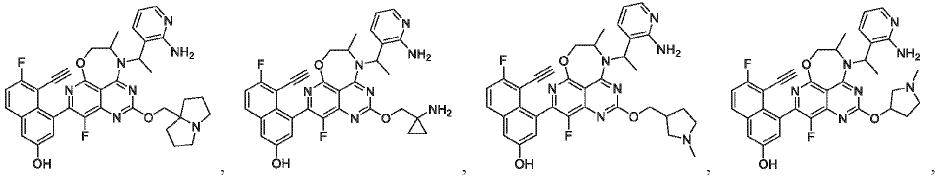 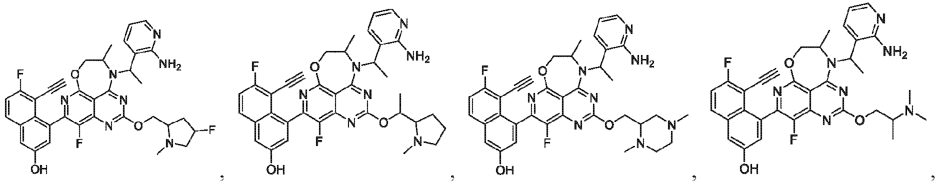 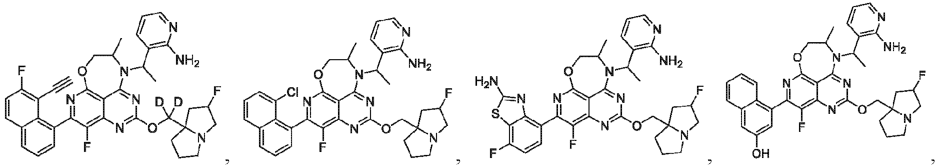 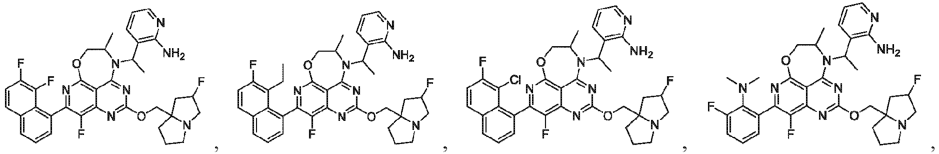 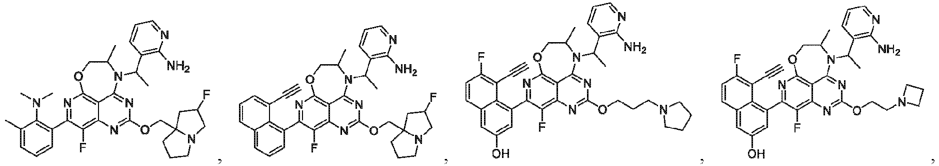 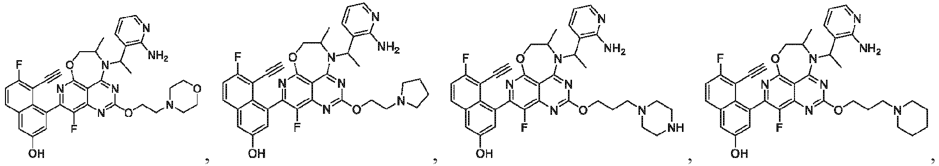 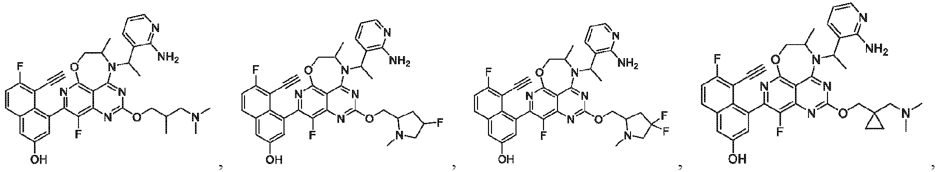 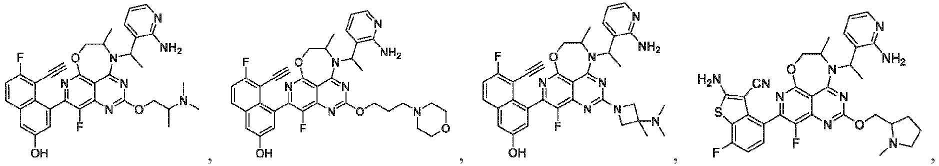 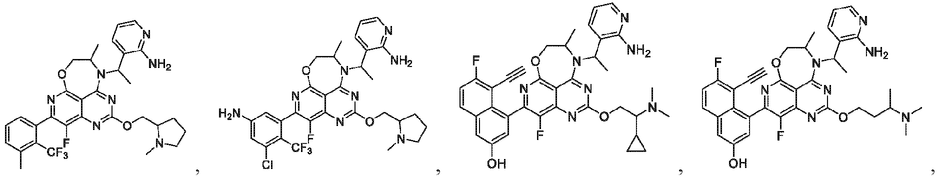 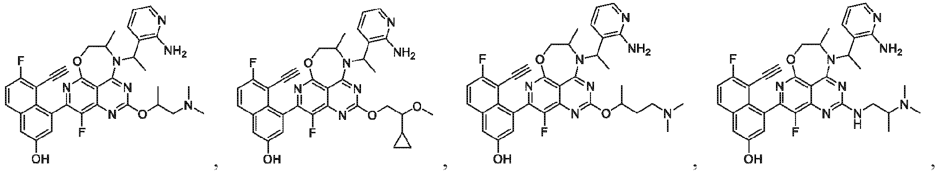 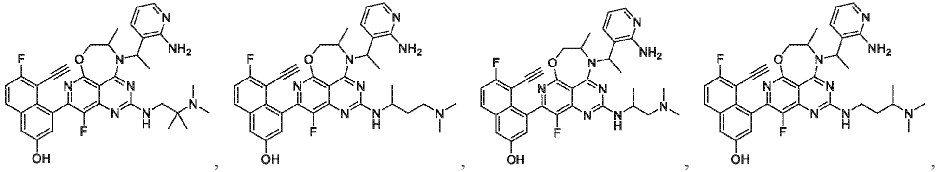 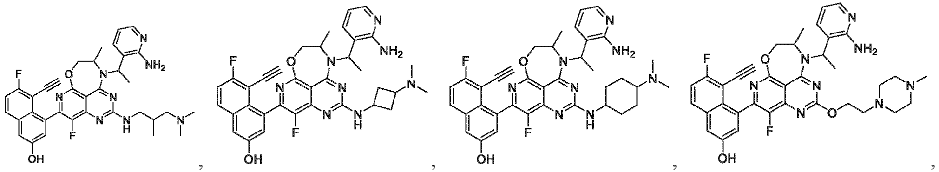 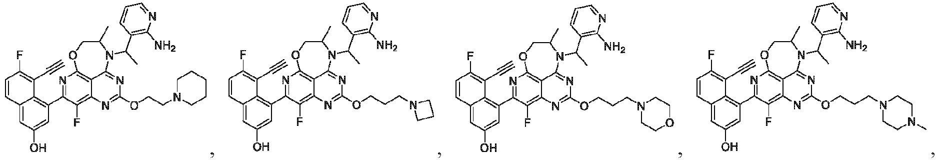 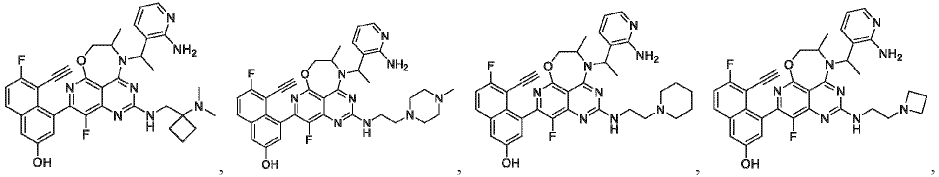 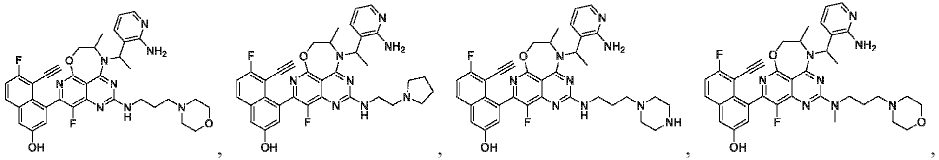 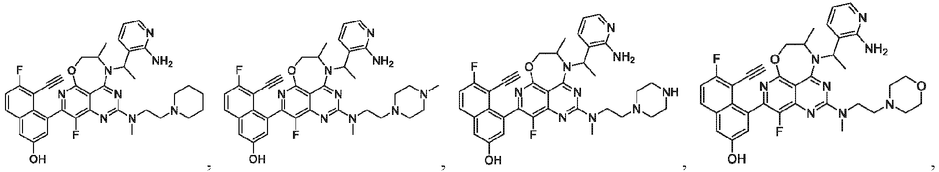 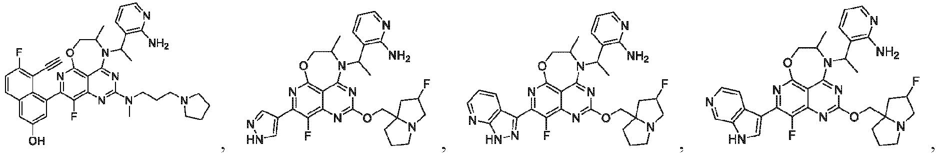 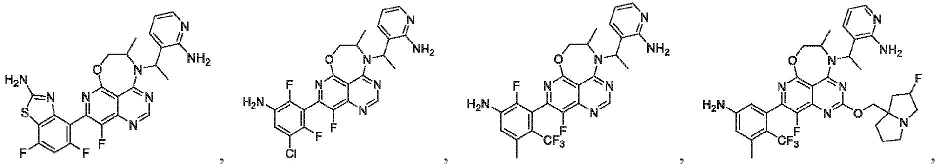 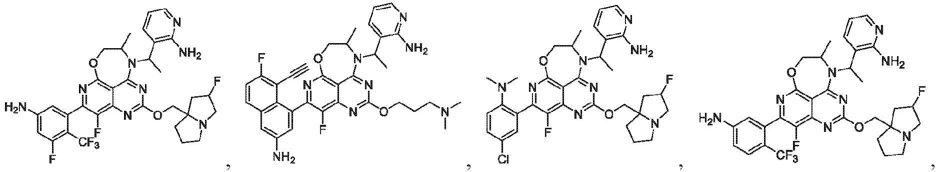 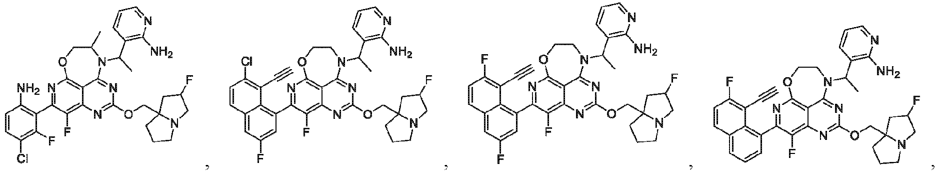 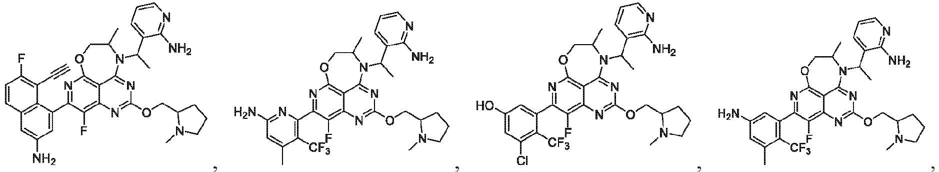 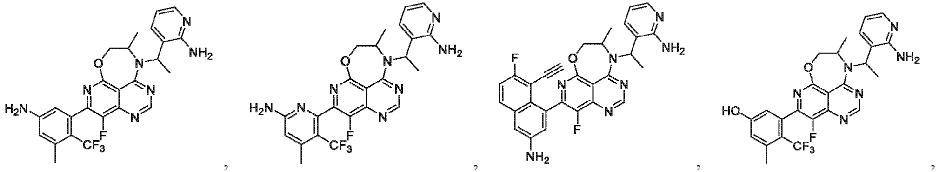 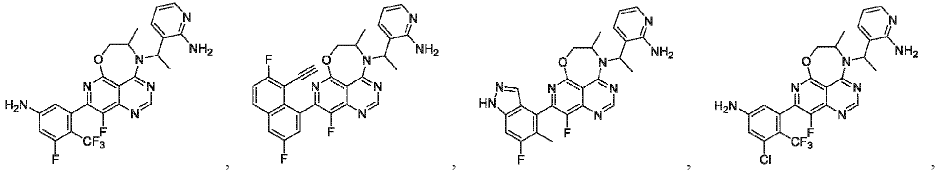 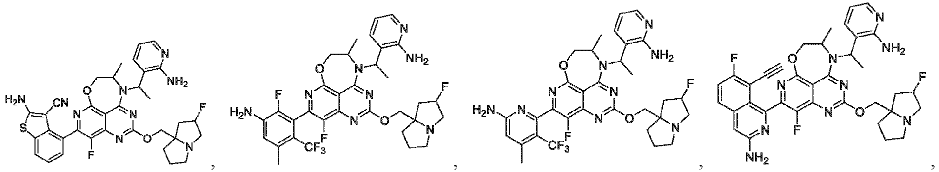 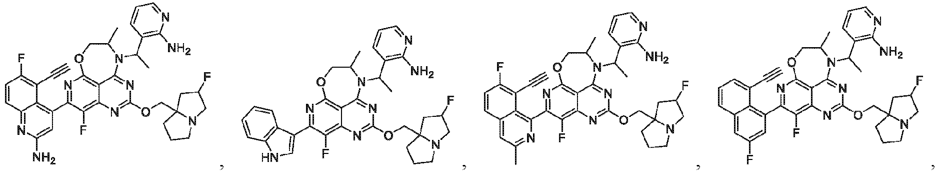 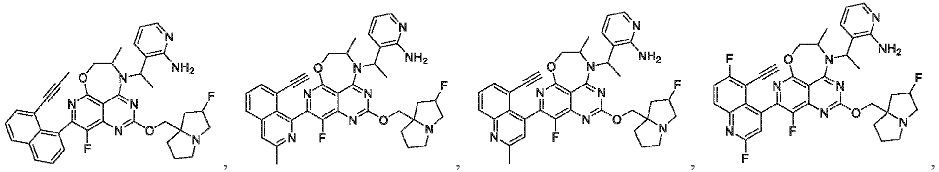 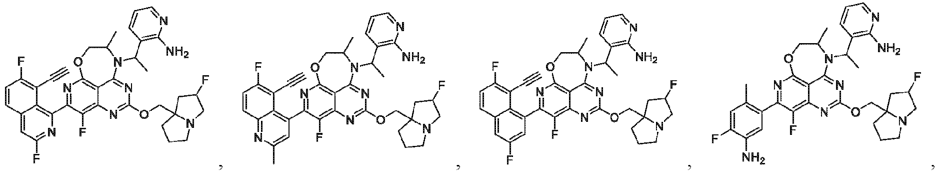 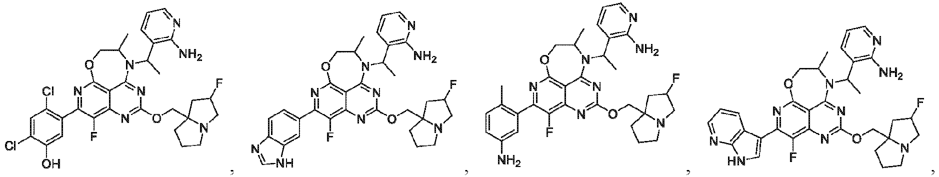 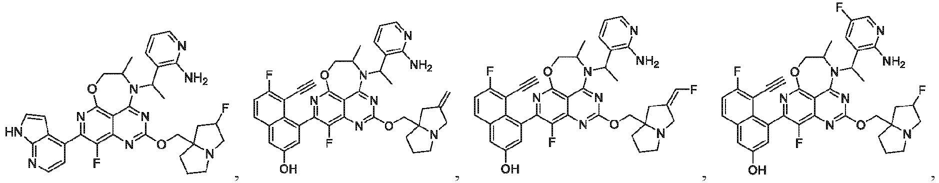 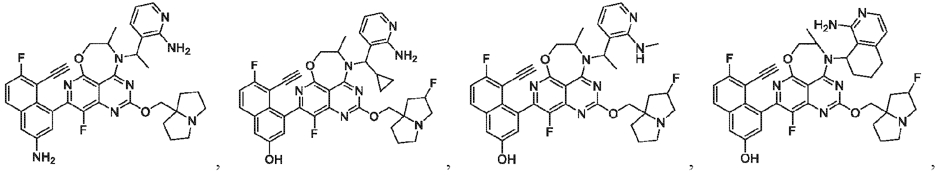 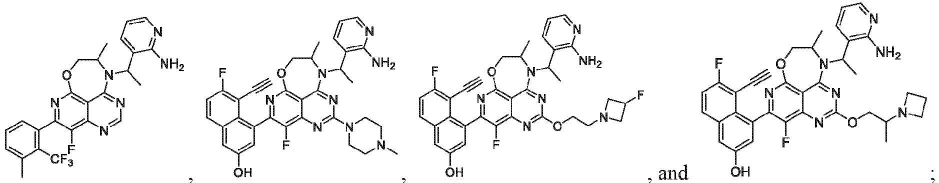 or the compound of formula (I), the stereoisomer thereof, the tautomer thereof, the deuterated compound thereof, or the pharmaceutically acceptable salt thereof is selected from the group consisting of: 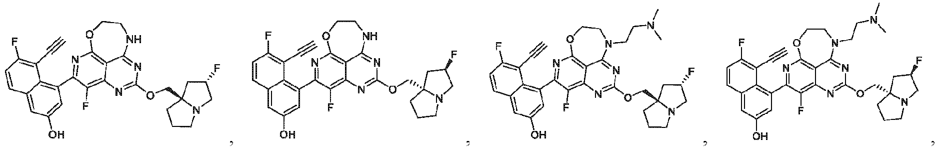 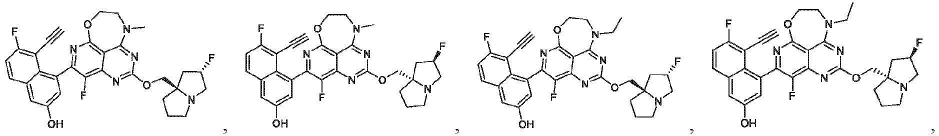 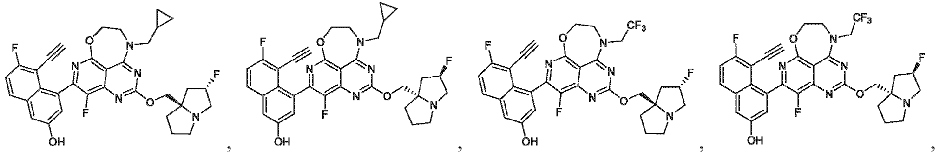 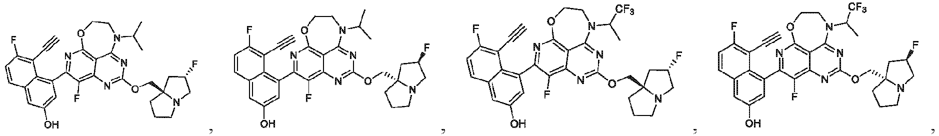 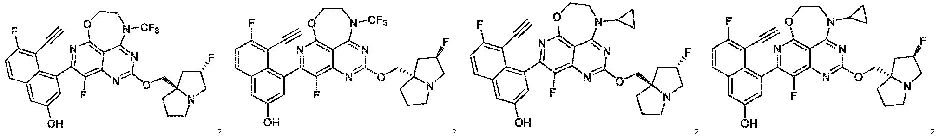 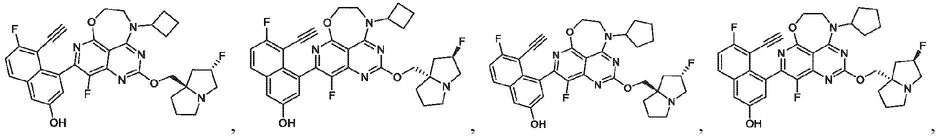 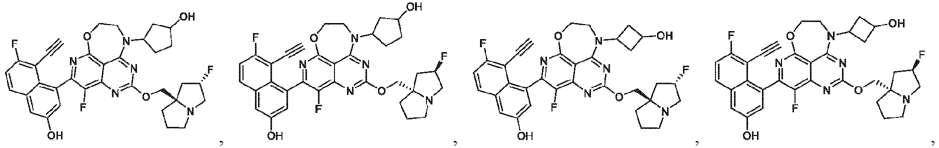 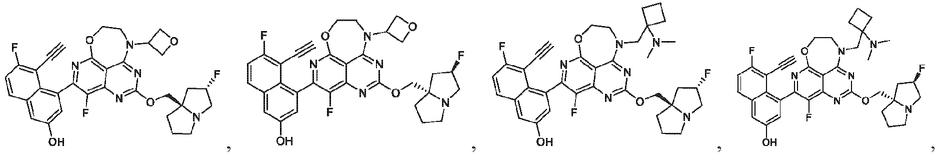 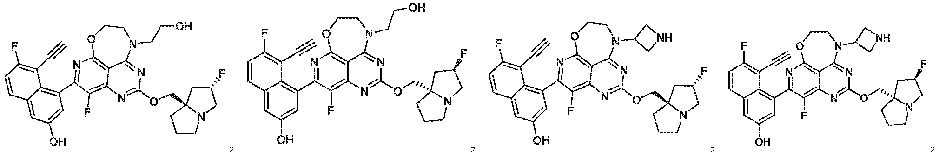 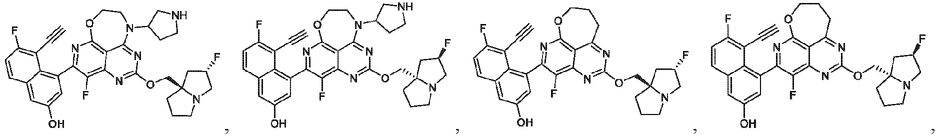 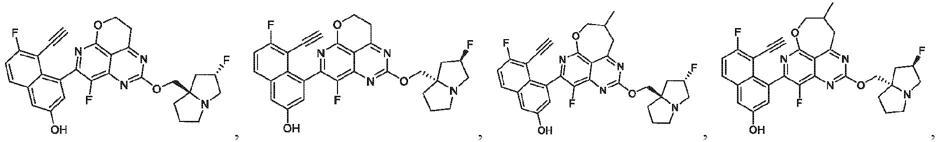 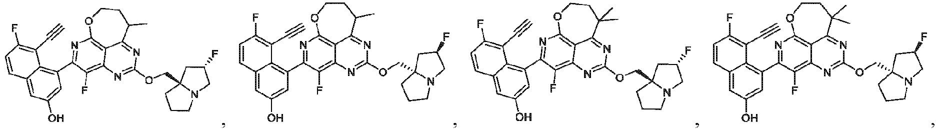 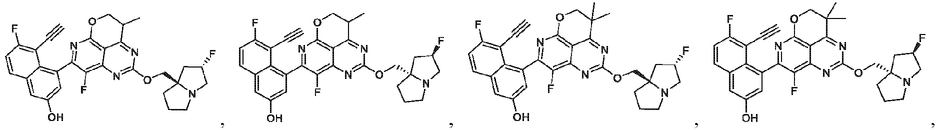 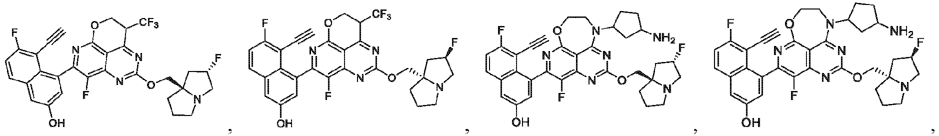 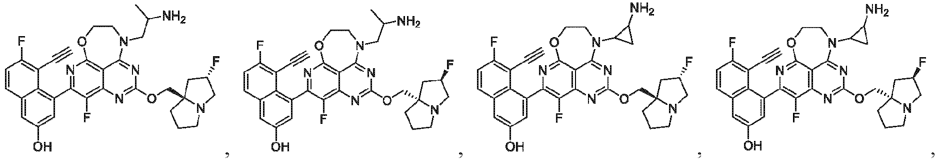 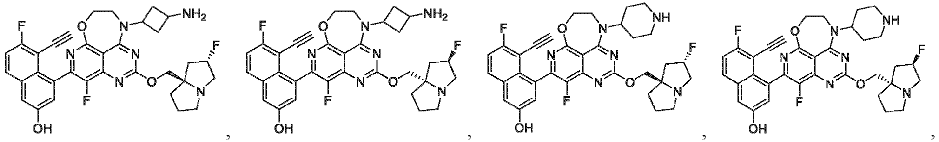 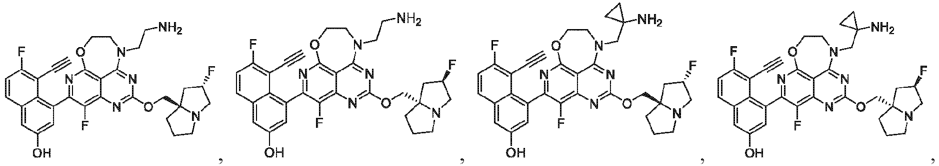 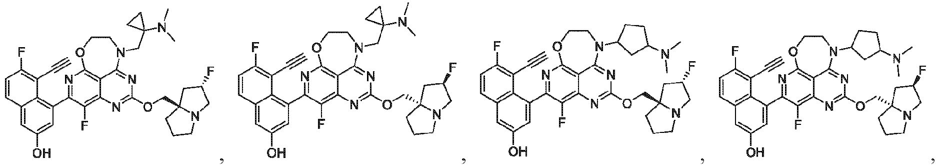 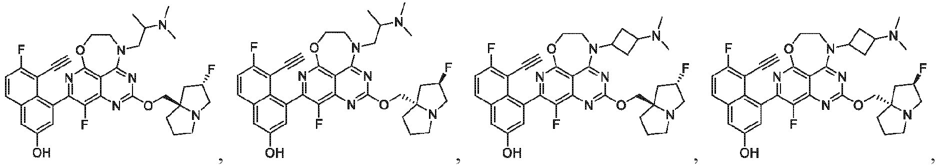 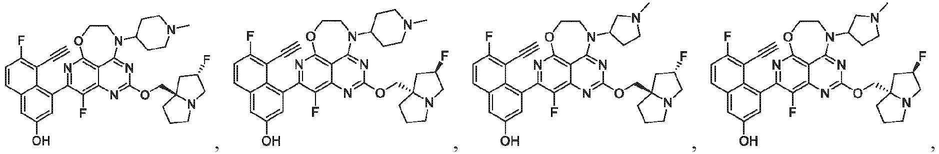 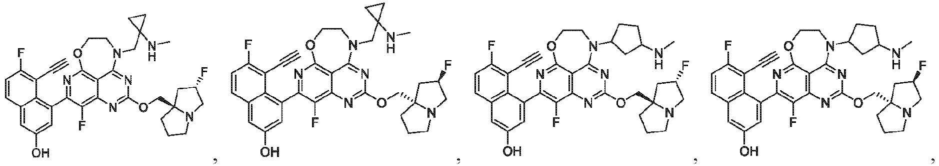 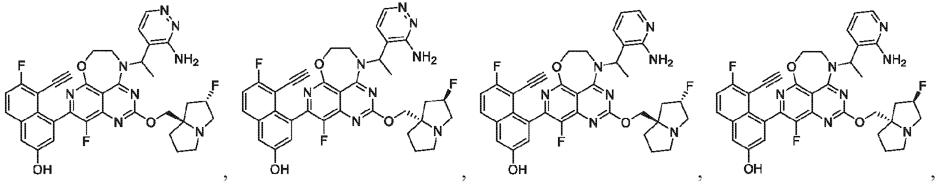 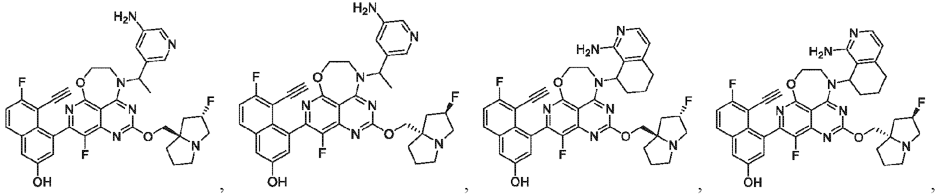 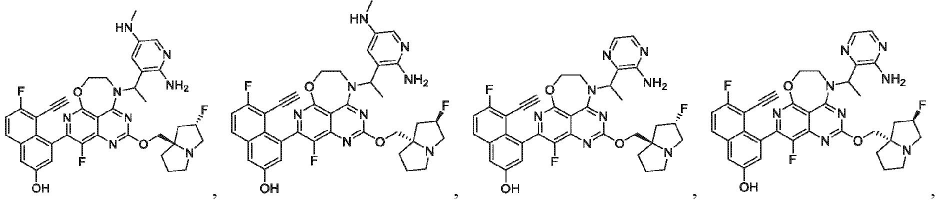 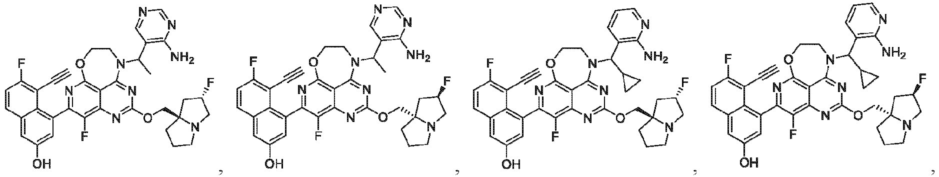 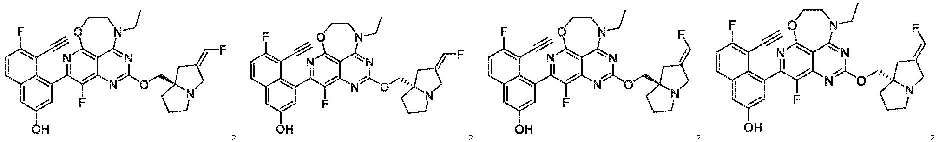 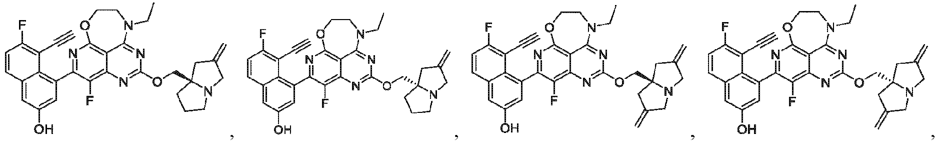 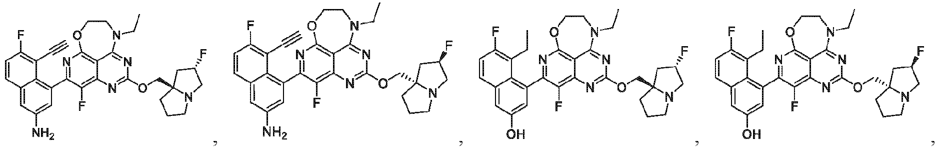 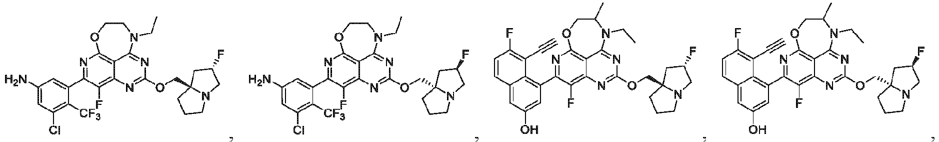 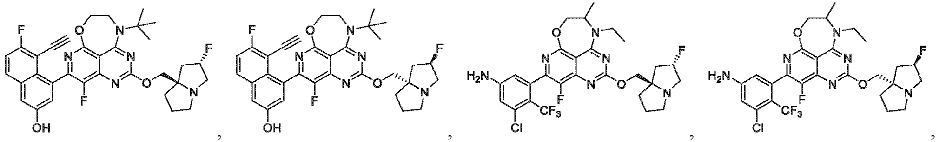 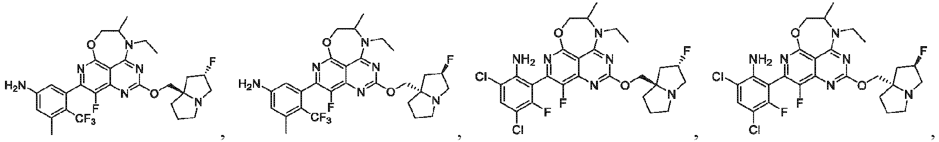 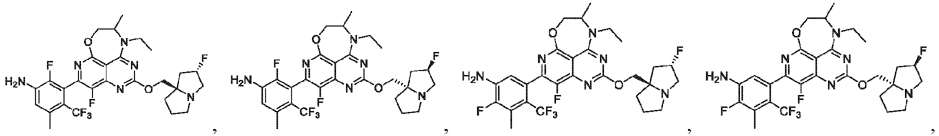 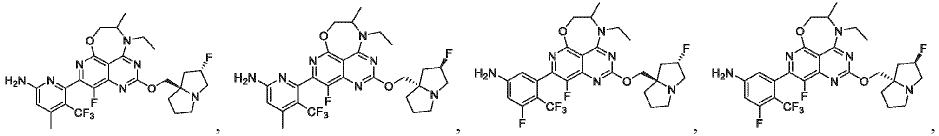 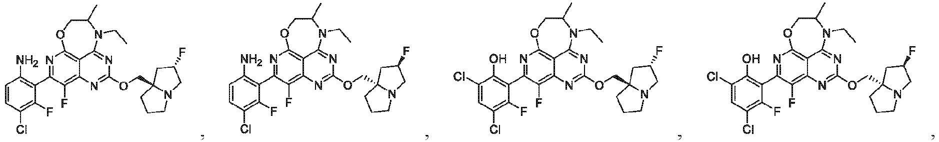 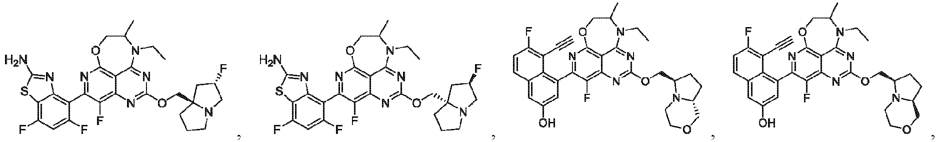 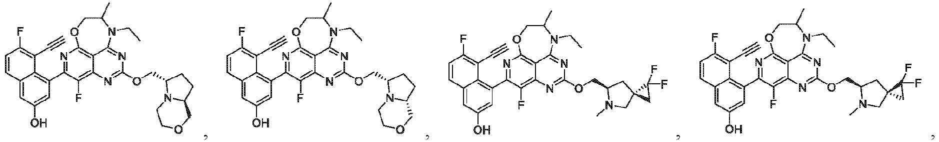 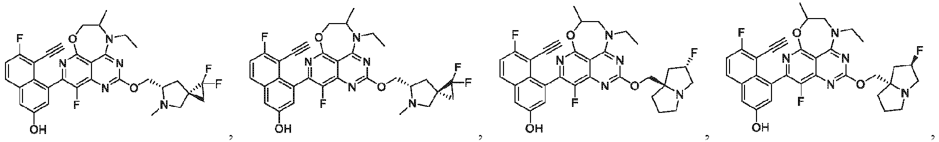 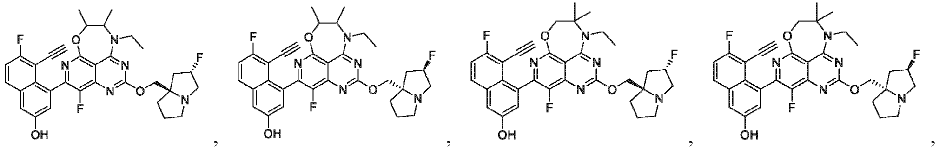 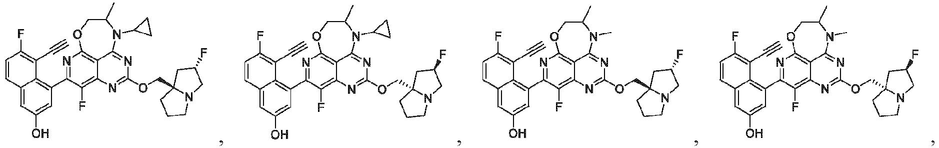 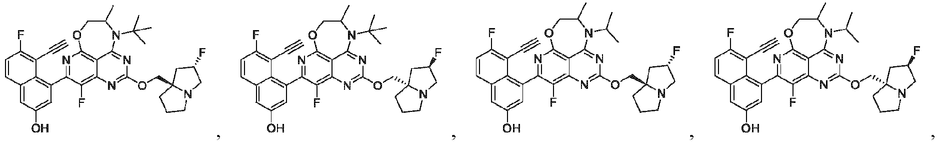 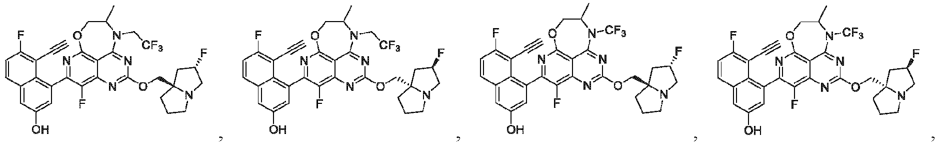 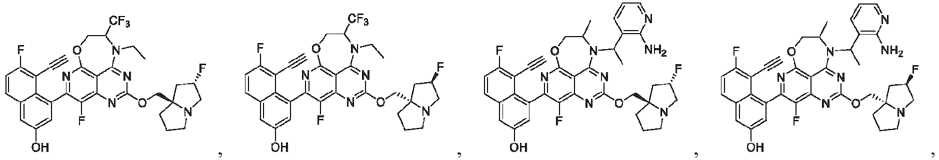 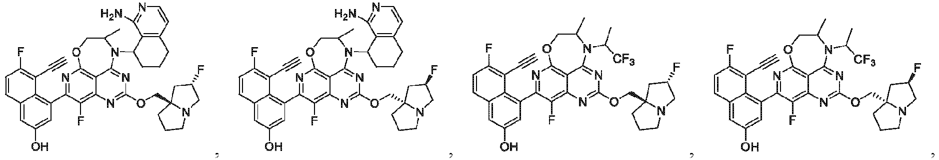 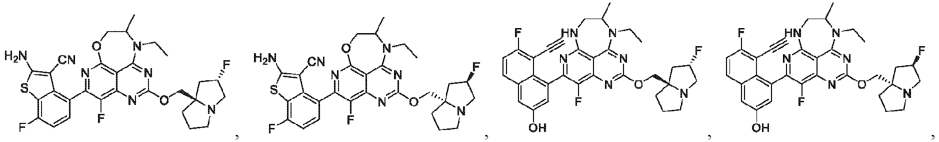 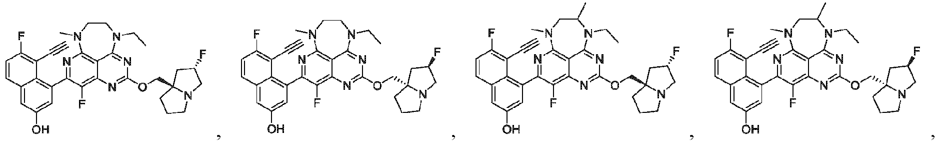 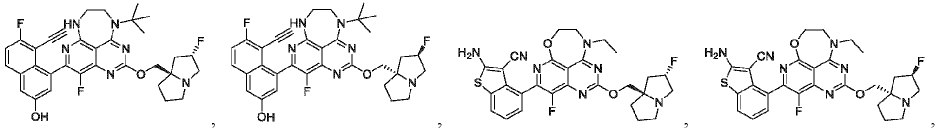 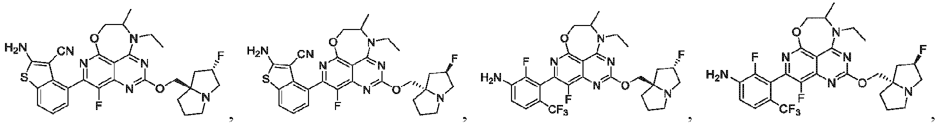 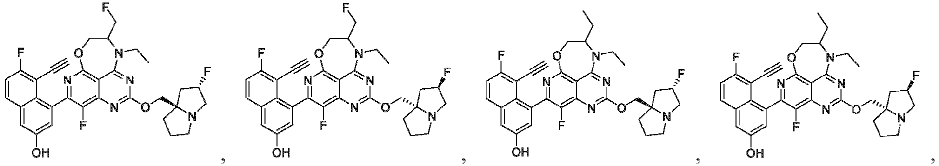 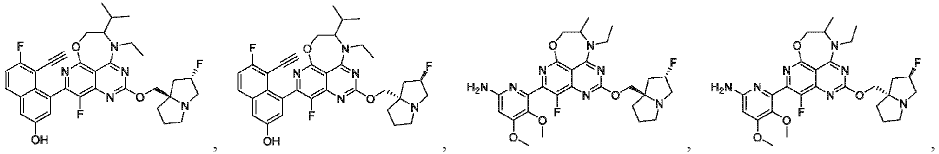 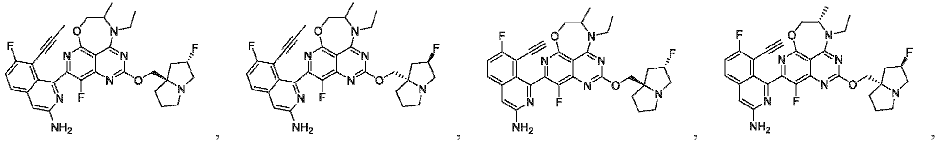 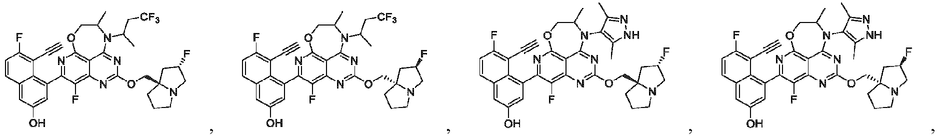 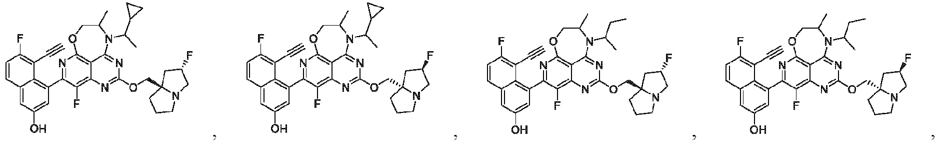 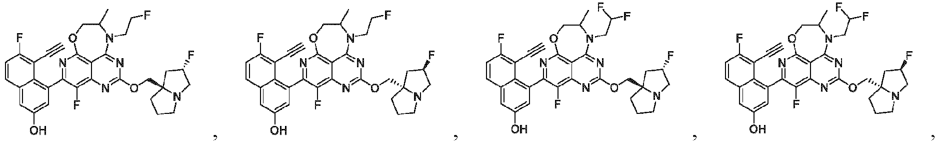 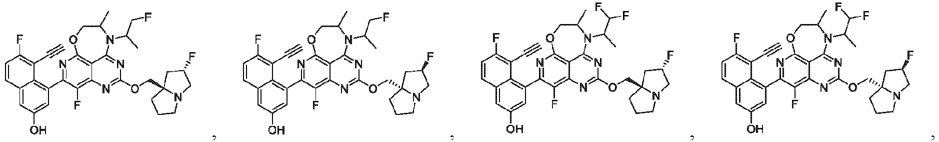 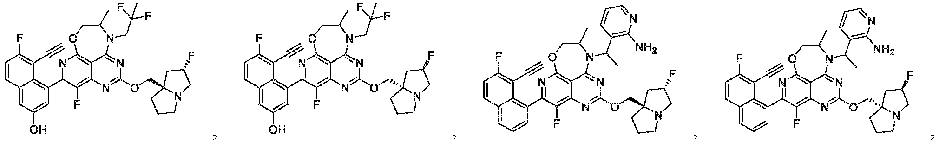 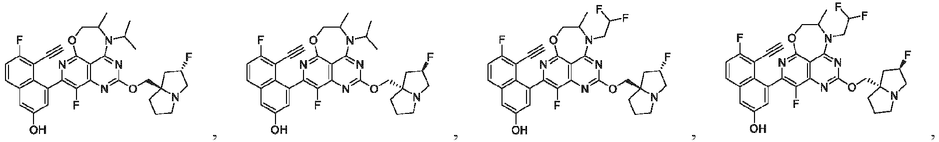 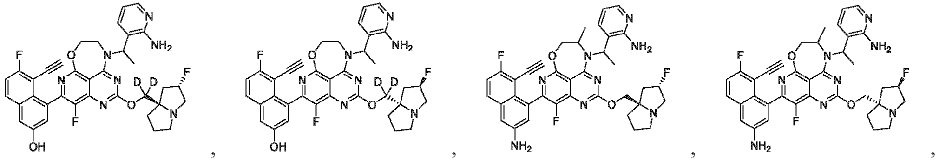 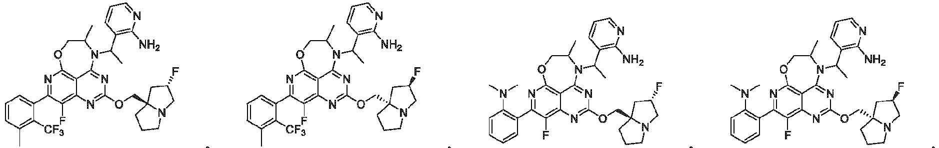 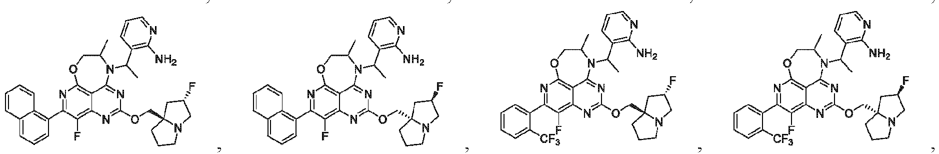 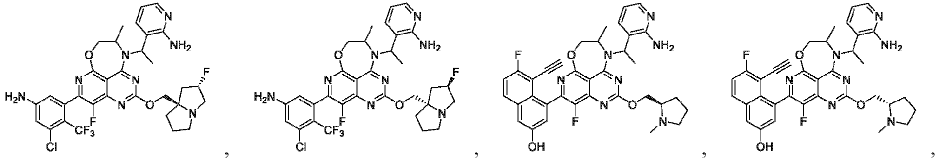 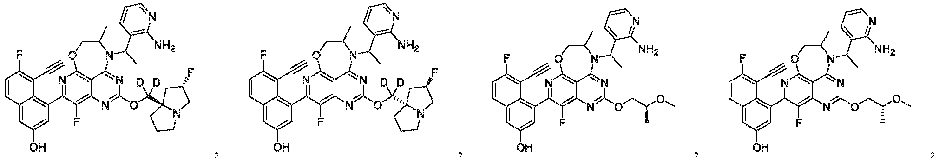 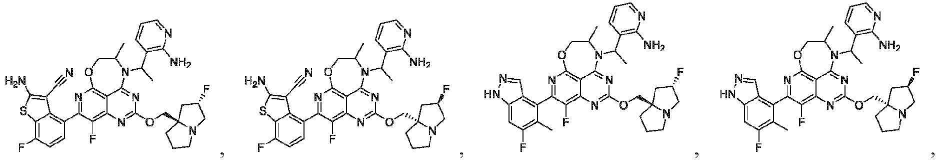 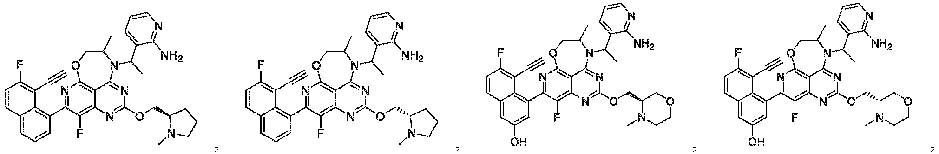 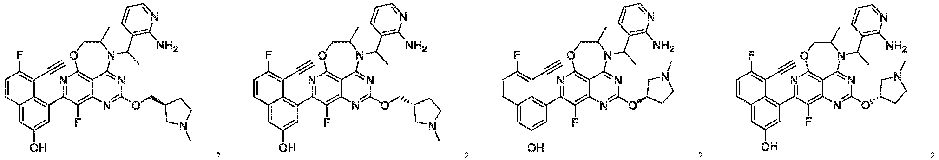 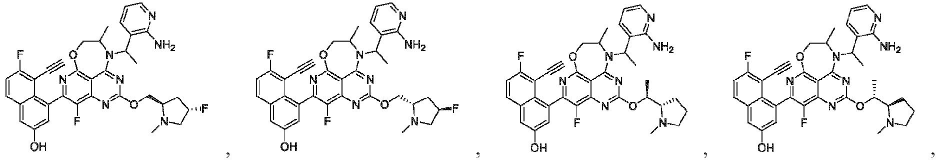 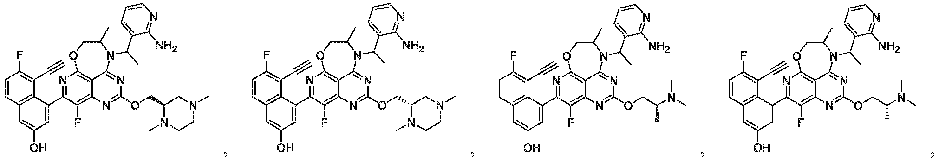 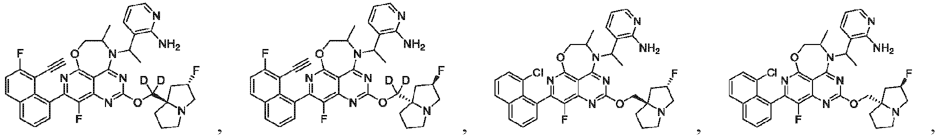 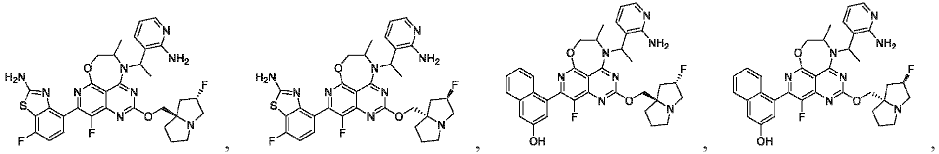 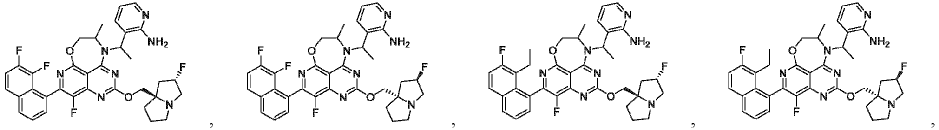 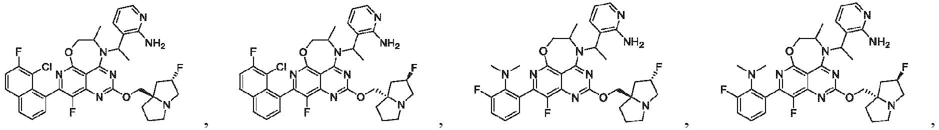 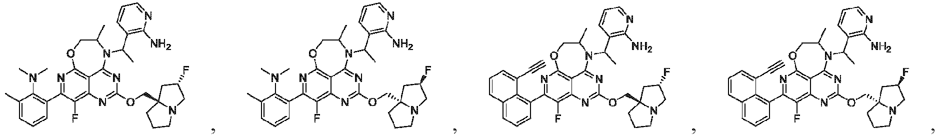 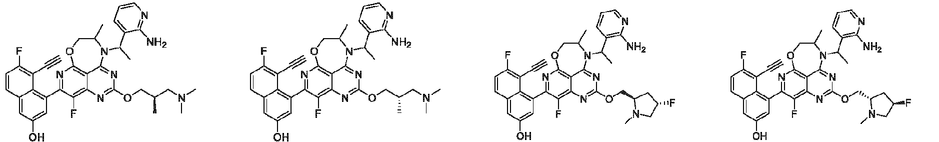 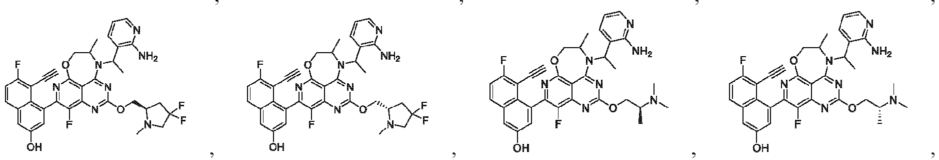 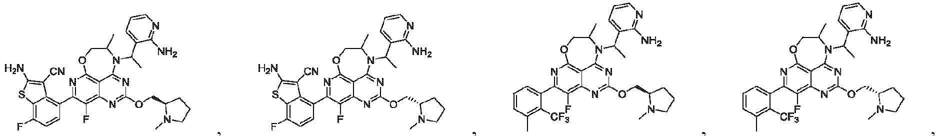 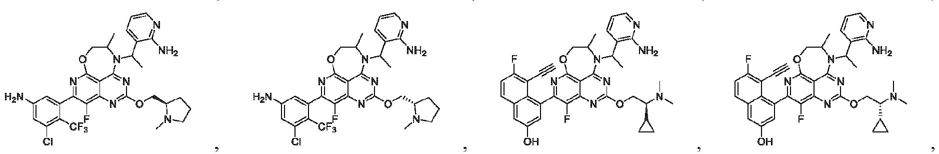 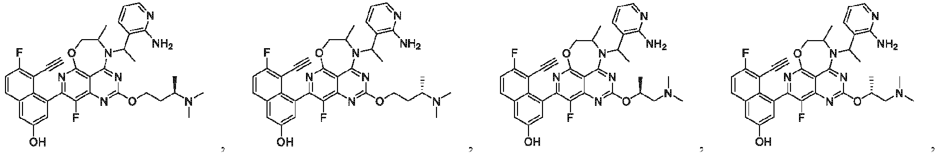 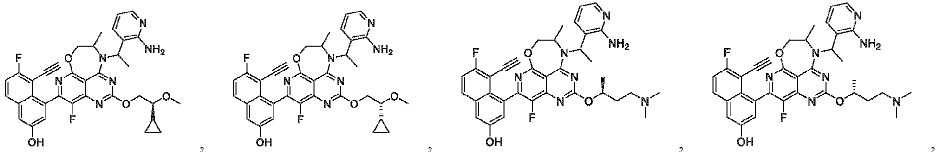 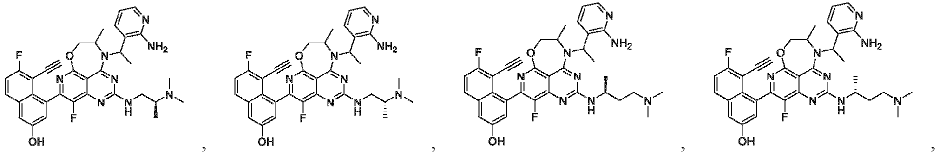 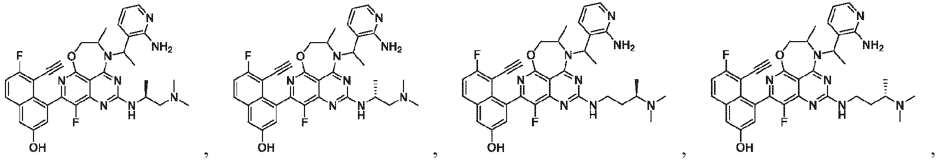 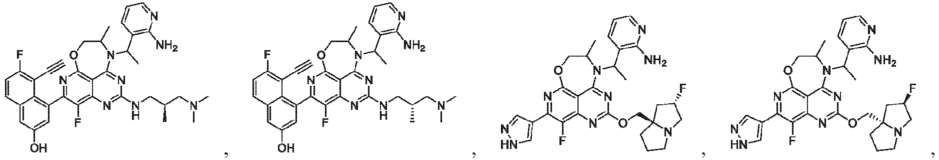 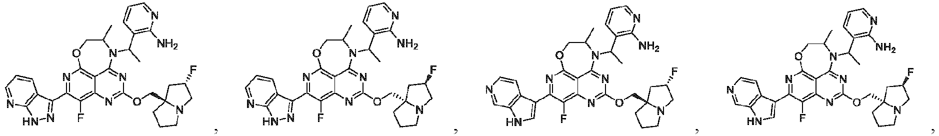 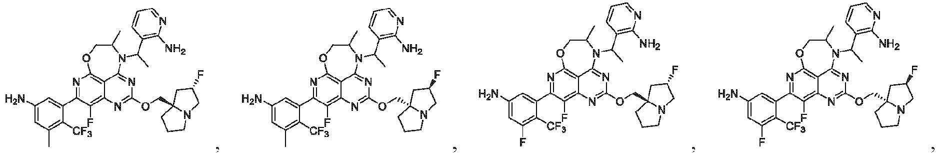 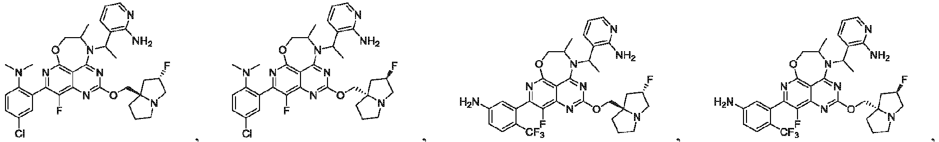 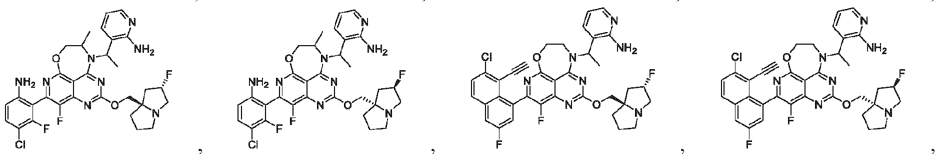 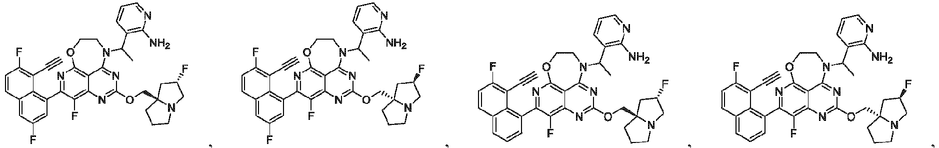 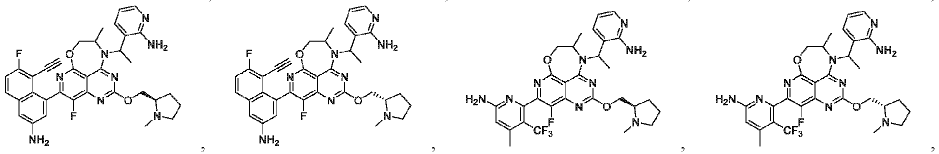 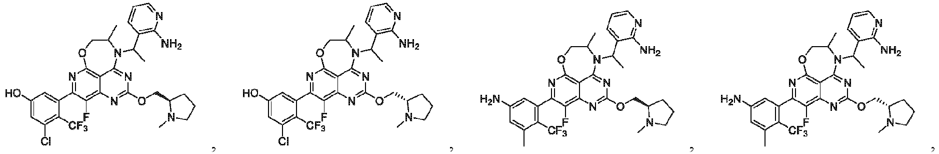 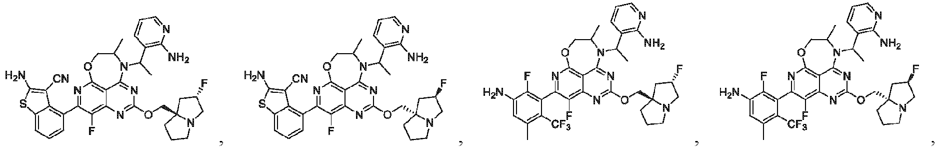 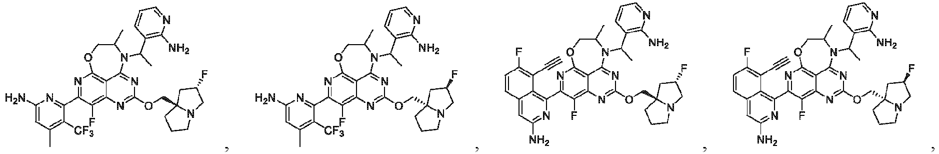 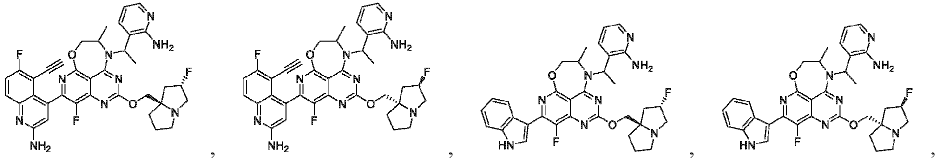 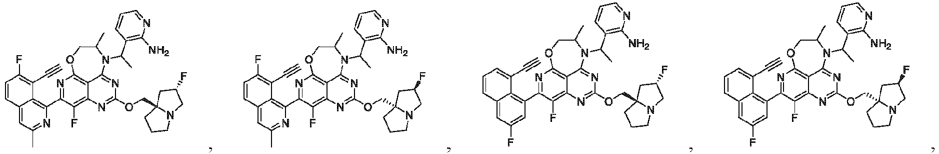 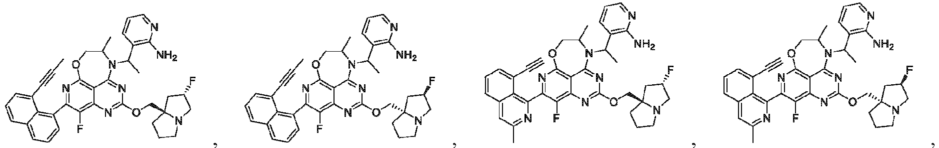 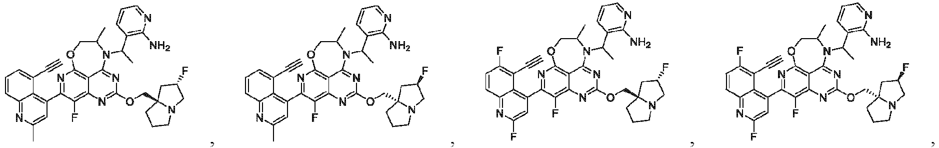 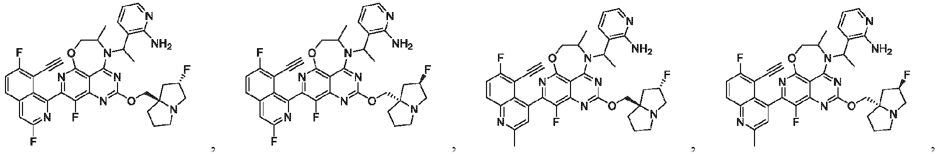 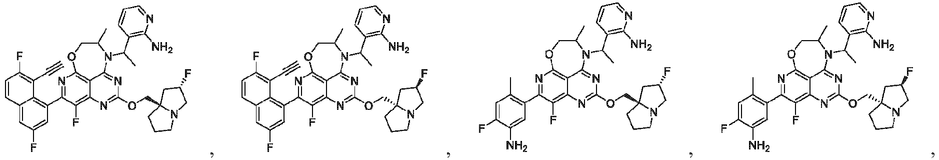 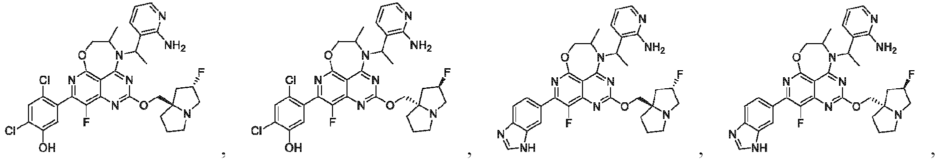 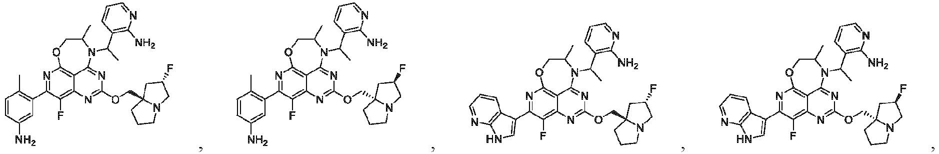 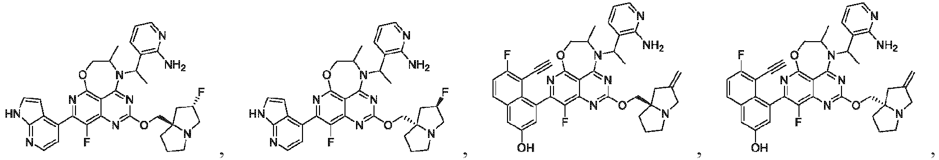 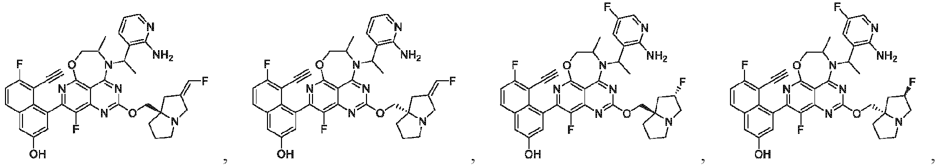 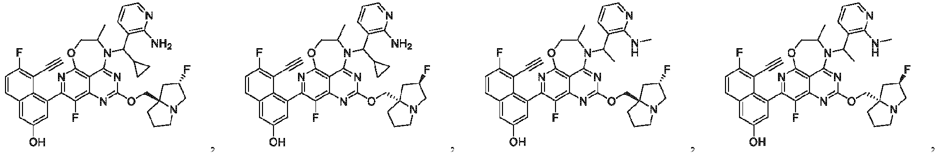 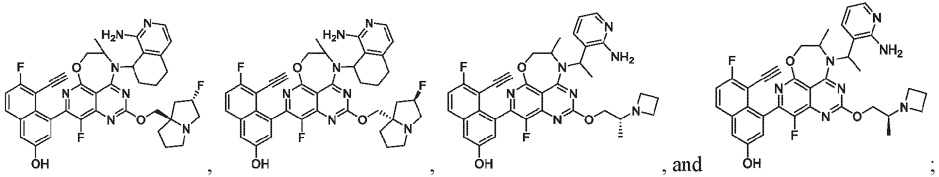 or the compound of formula (I), the stereoisomer thereof, the tautomer thereof, the deuterated compound thereof, or the pharmaceutically acceptable salt thereof is selected from the group consisting of:

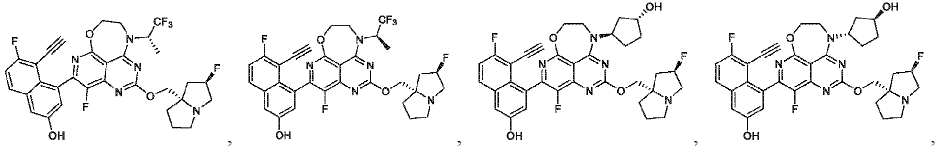

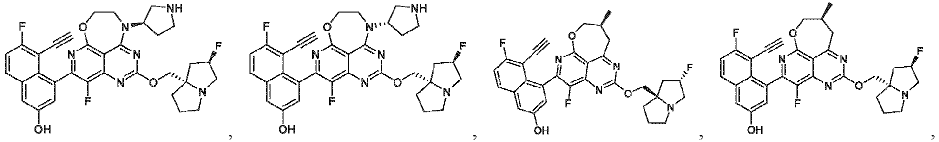

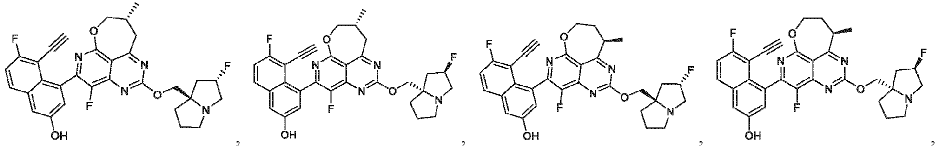

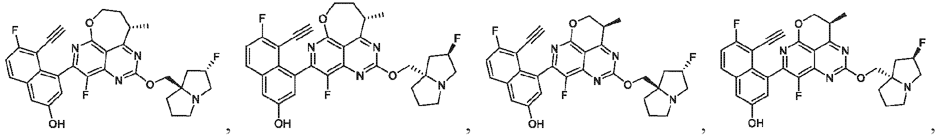

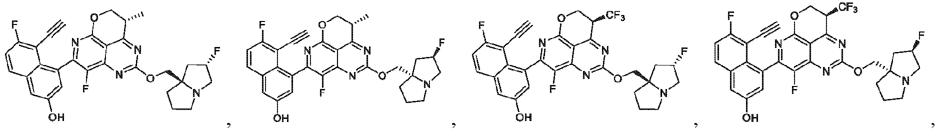

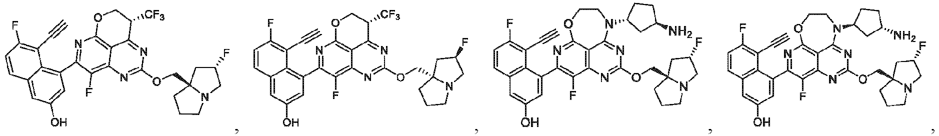

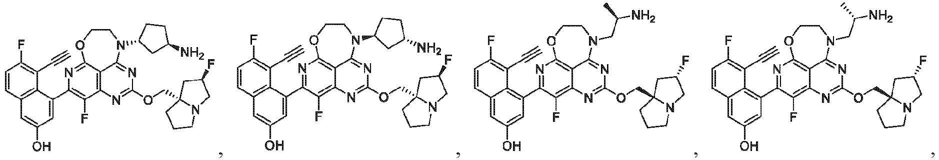

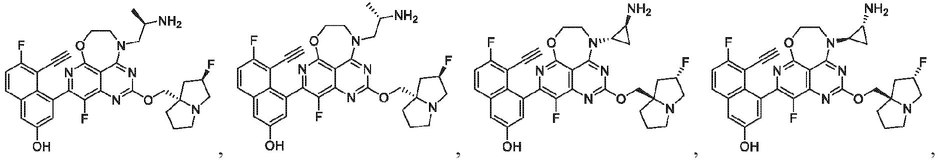

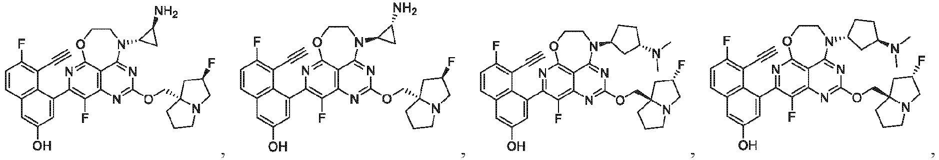

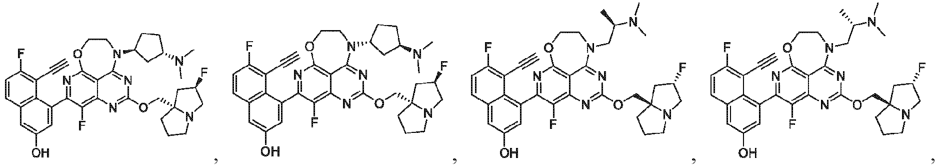

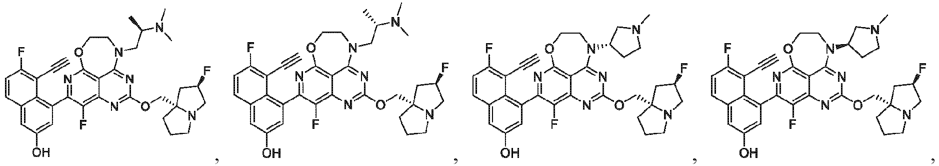

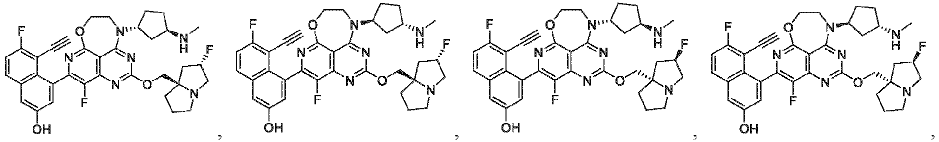

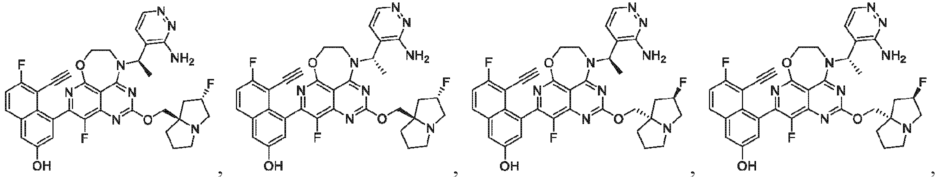

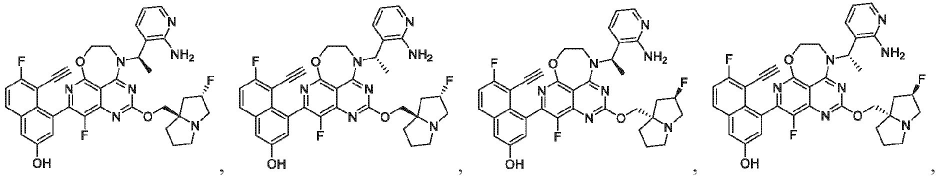

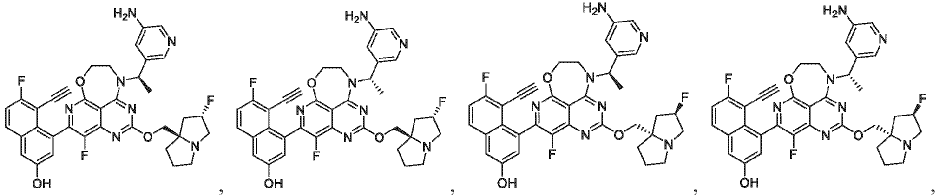

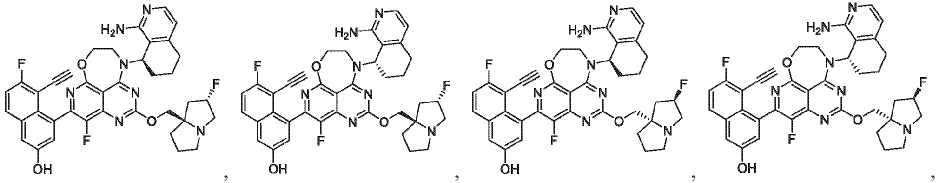

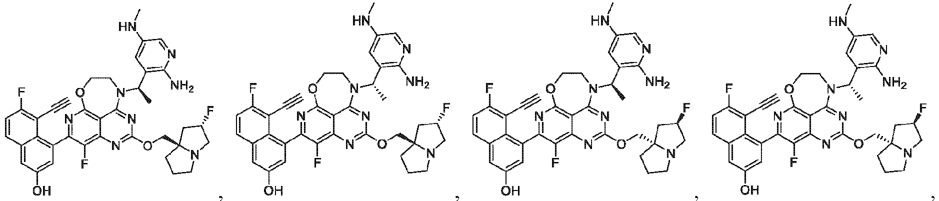

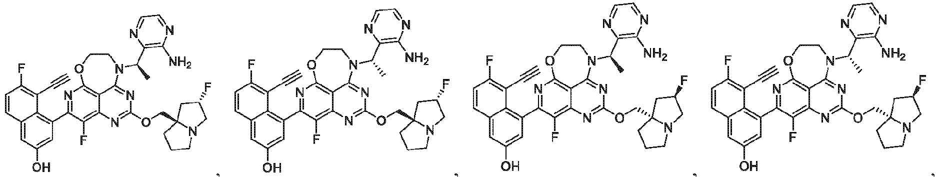

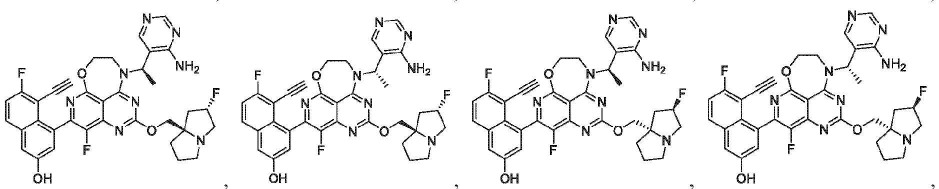

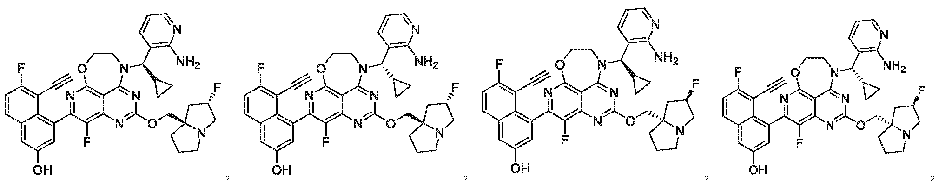

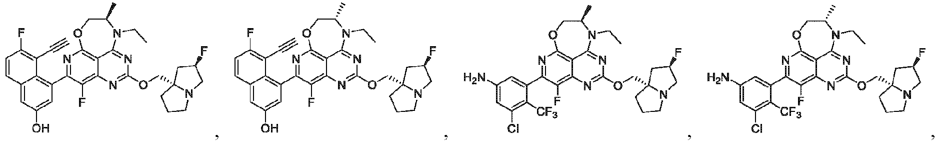

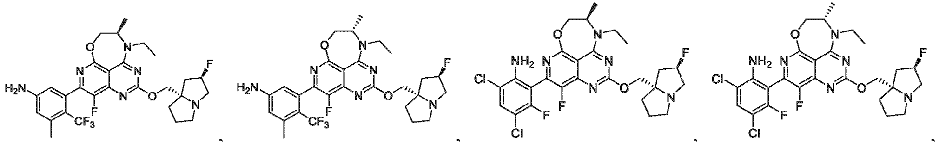

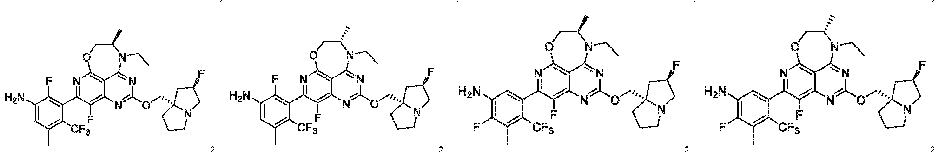

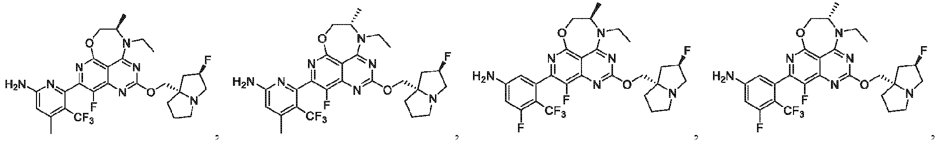

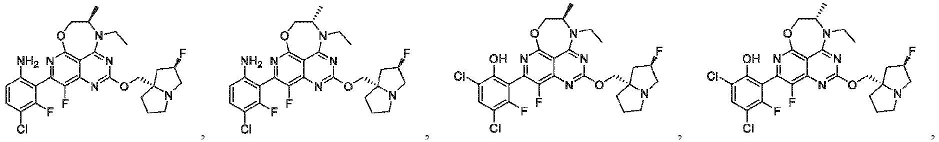

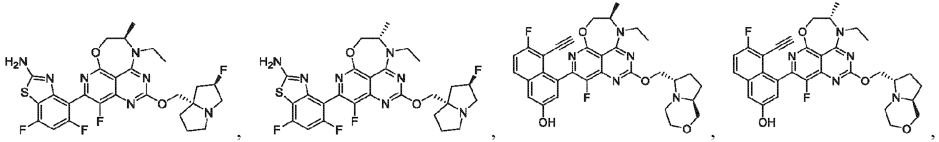

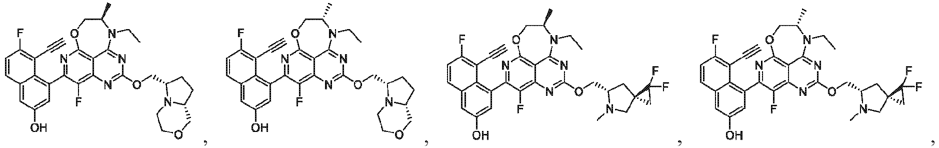

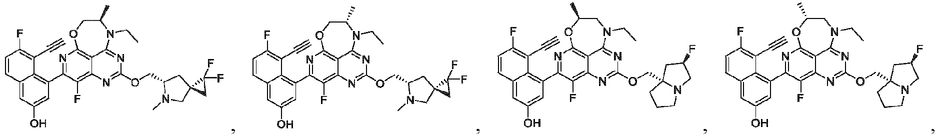

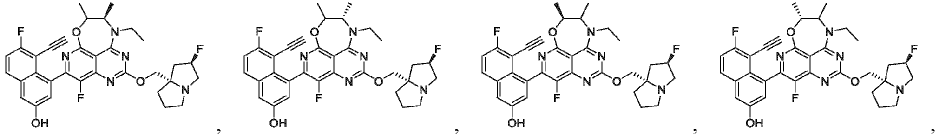

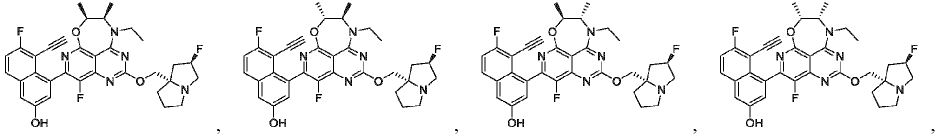

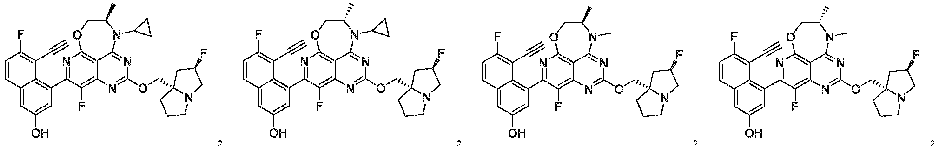

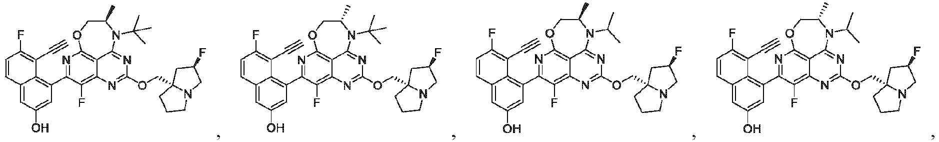

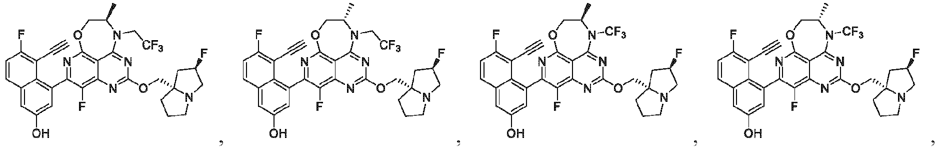

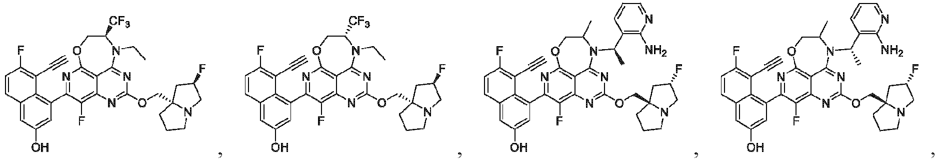

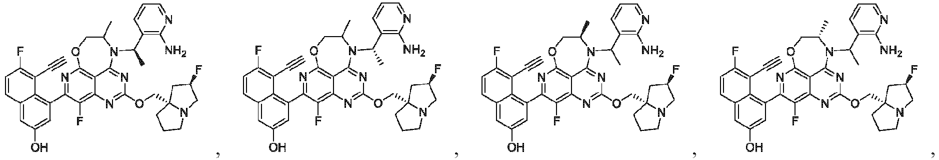

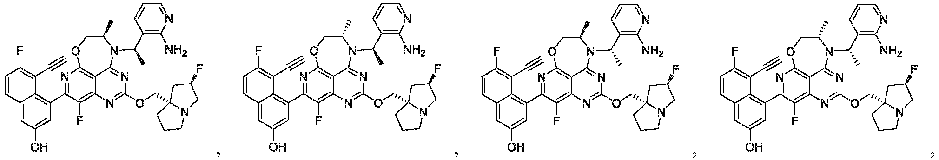

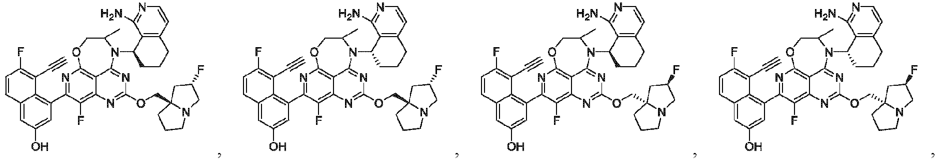

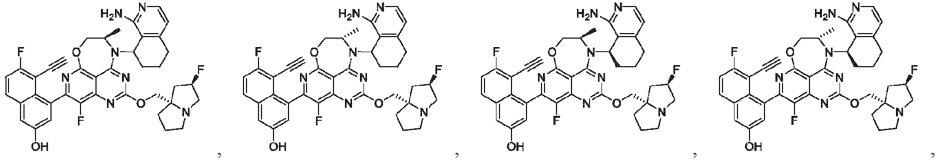

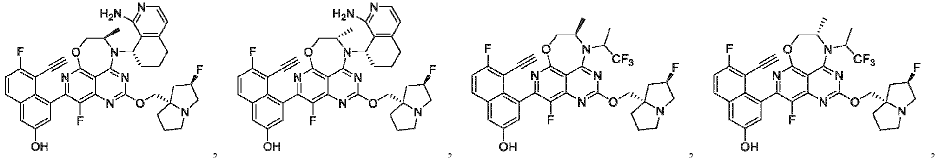

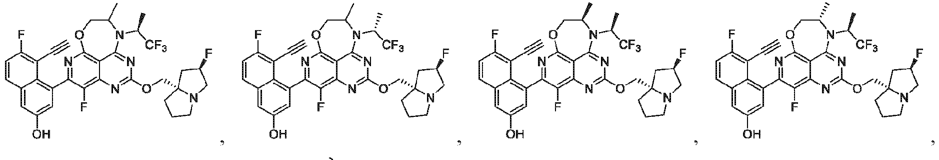

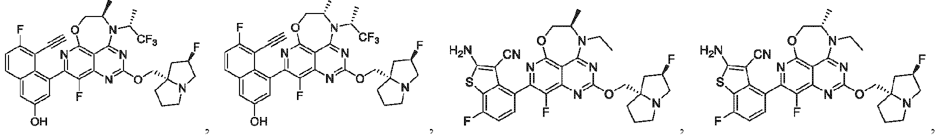

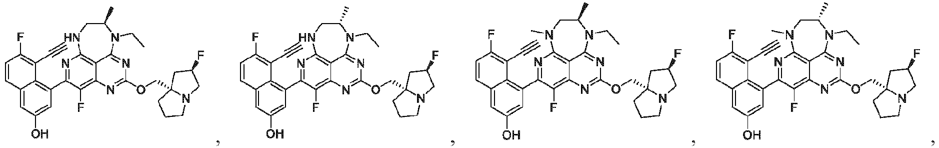

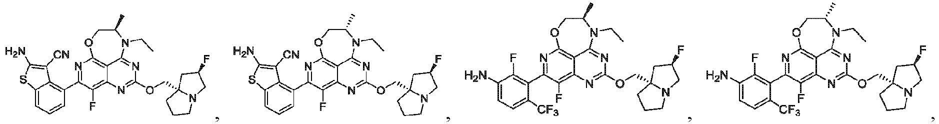

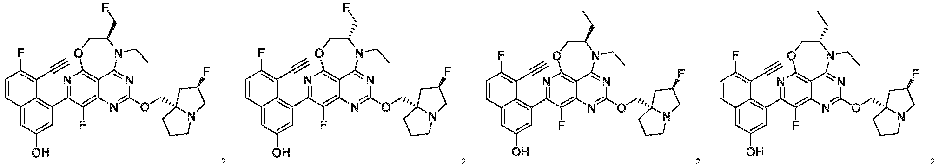

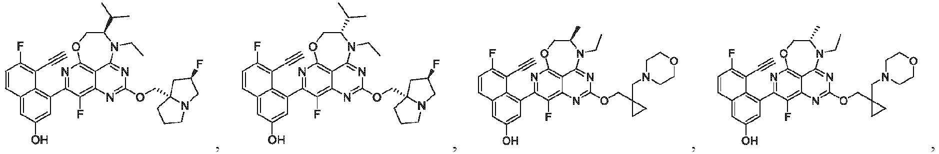

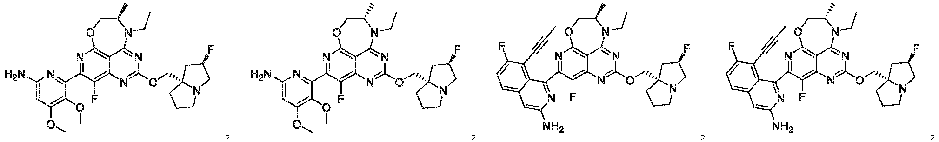

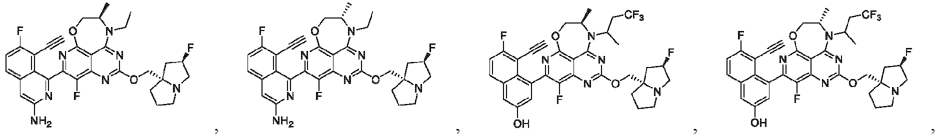

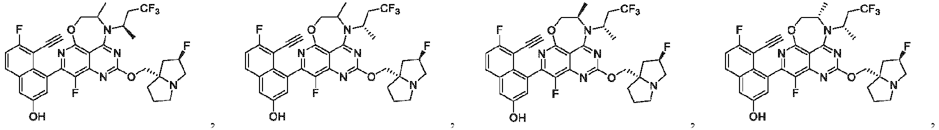

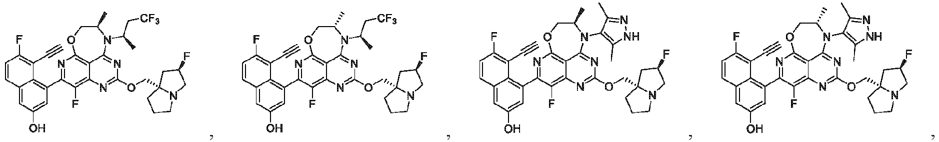

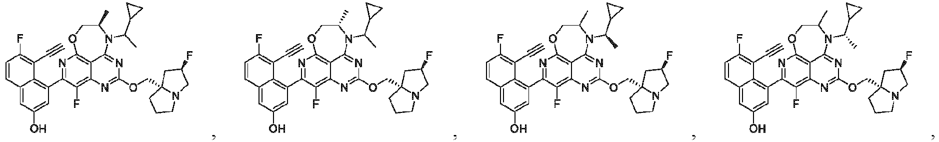

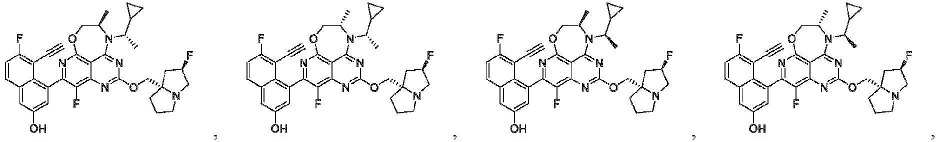

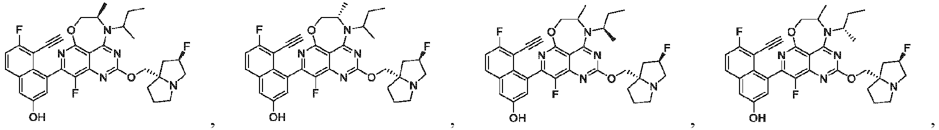

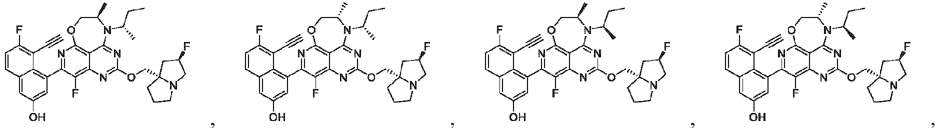

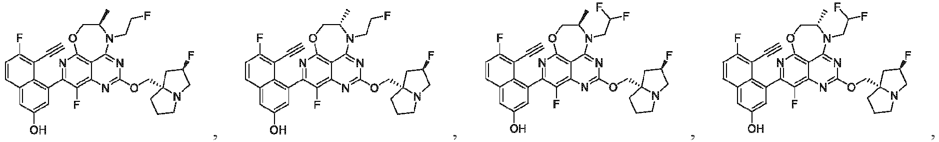

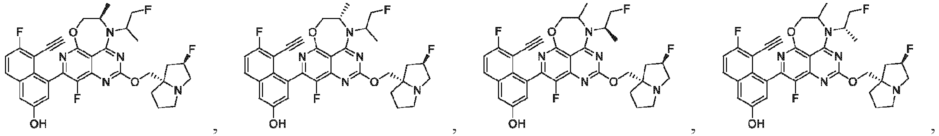

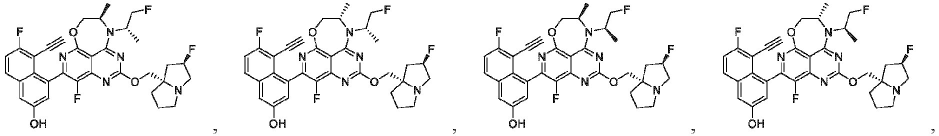

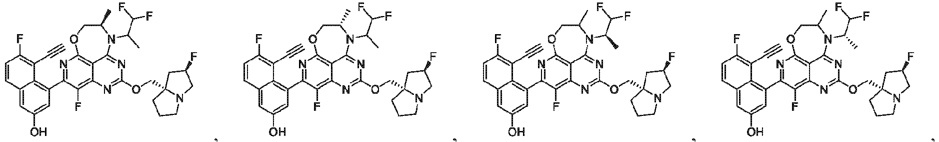

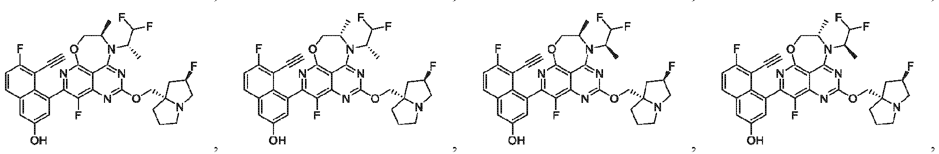

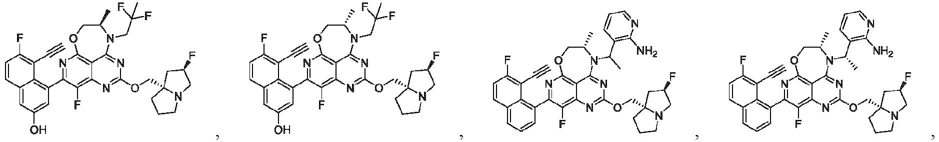

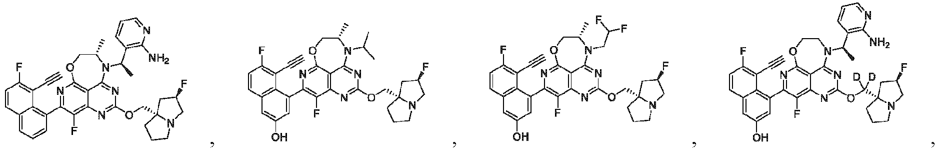

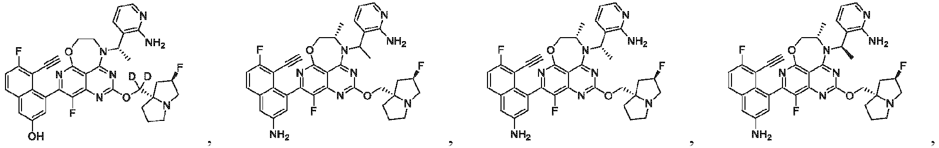

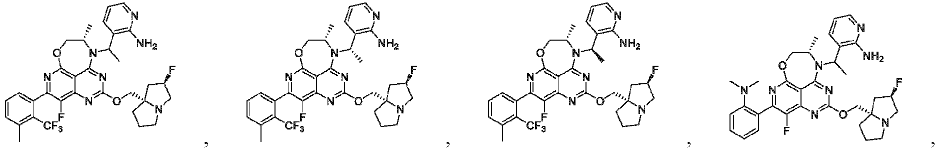

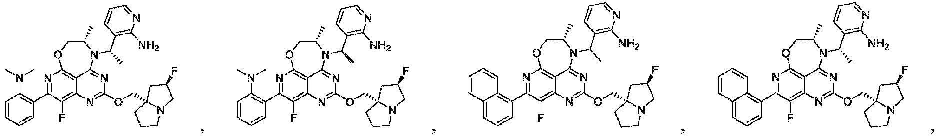

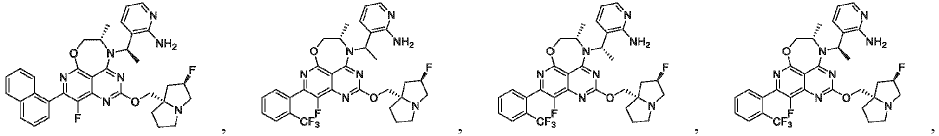

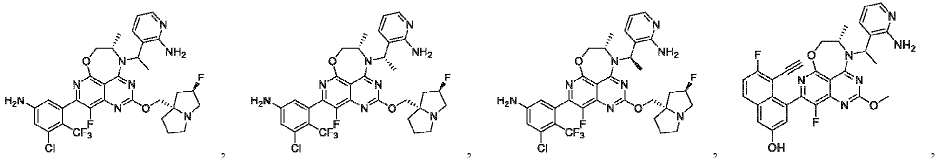

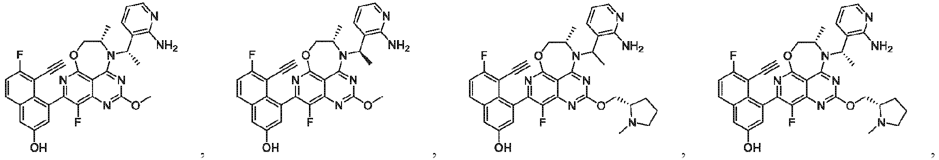

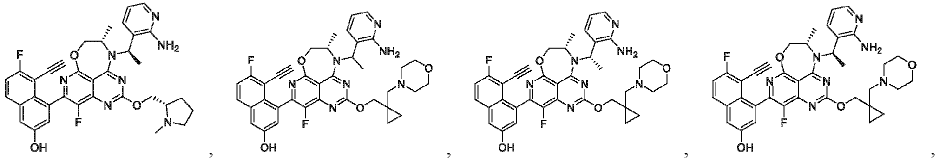

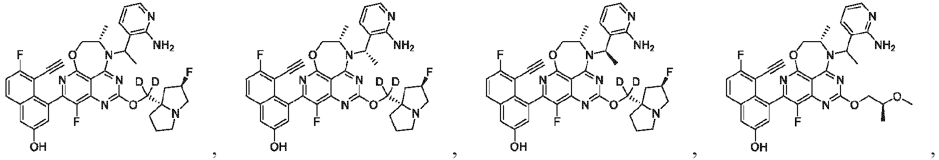

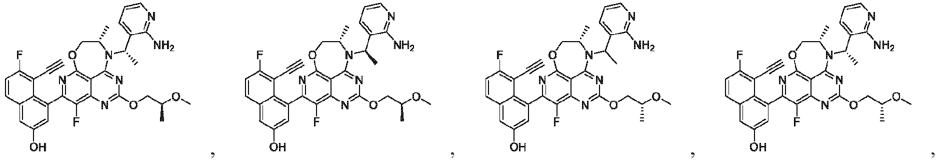

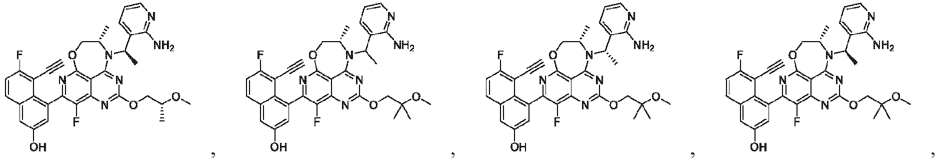

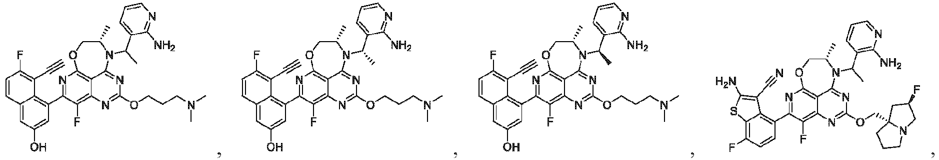

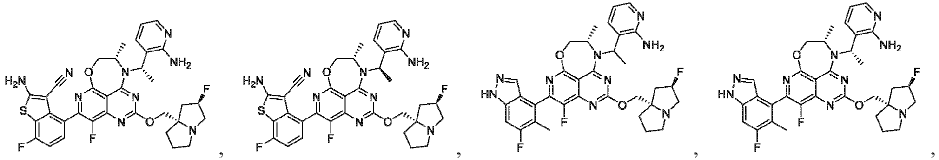

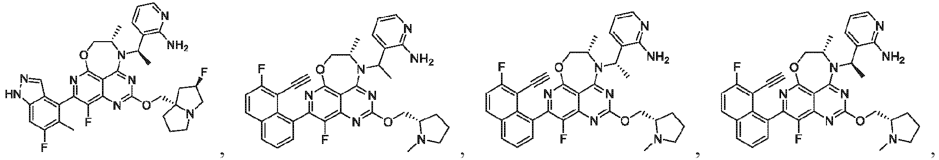

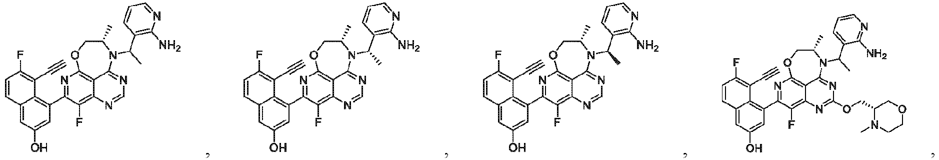

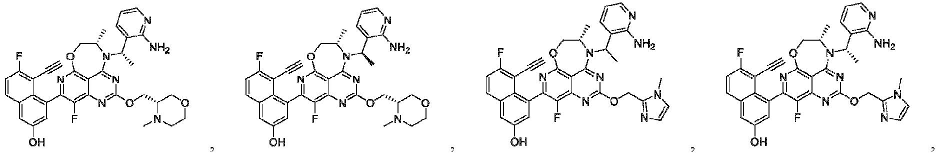

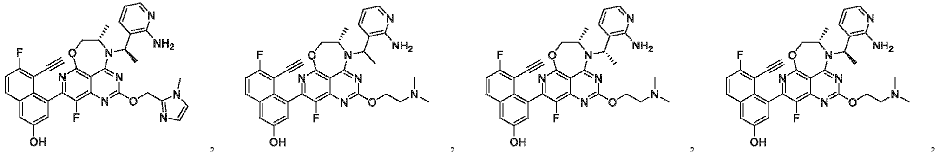

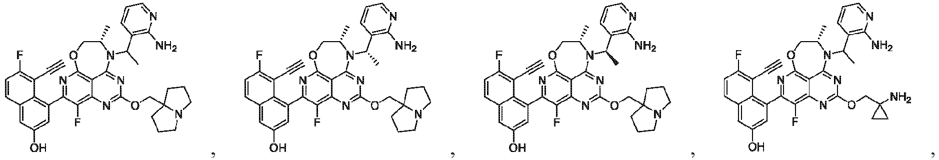

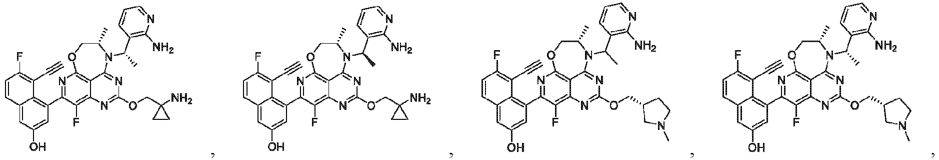

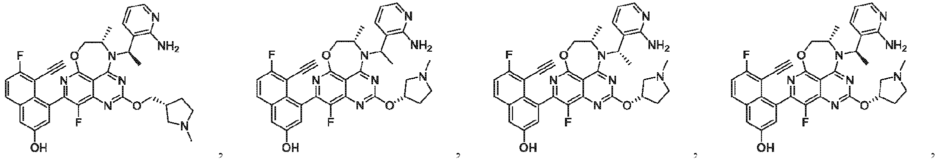

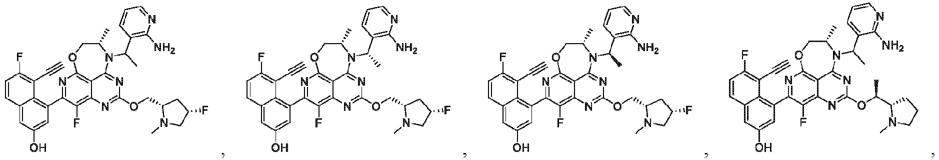

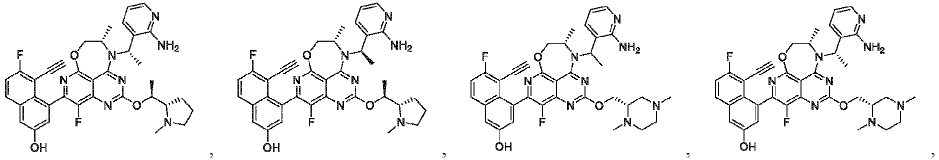

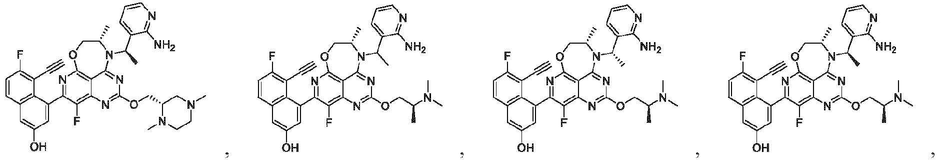

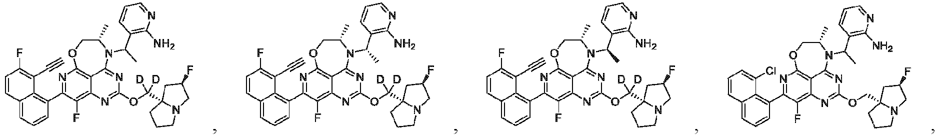

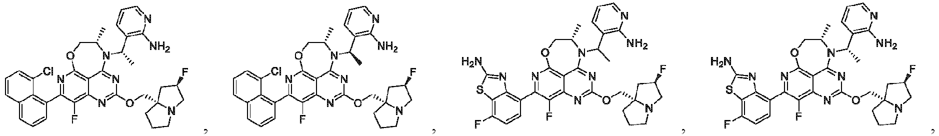

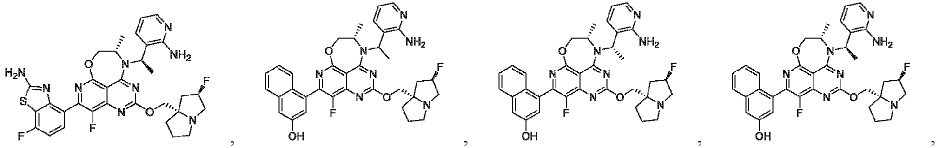

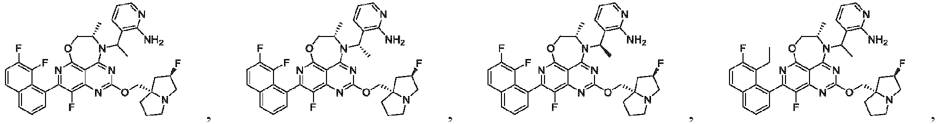

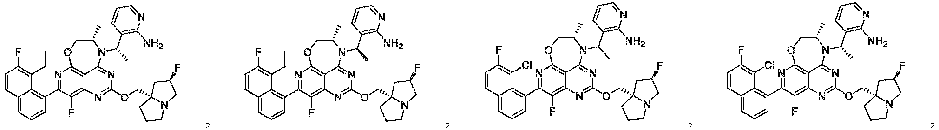

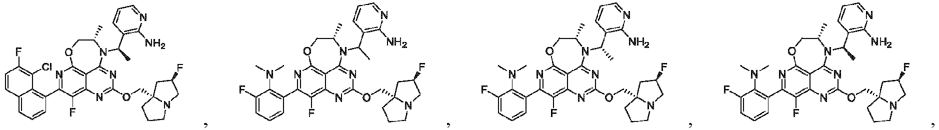

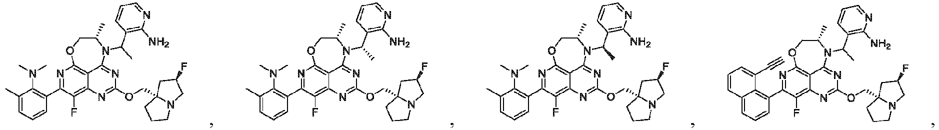

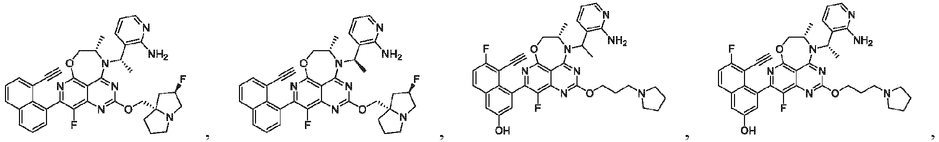

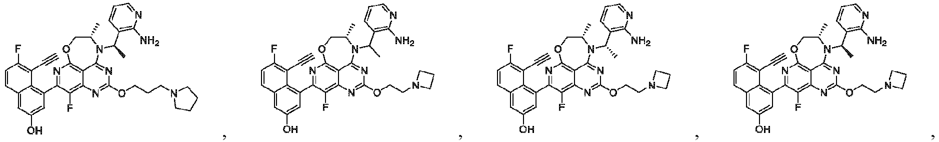

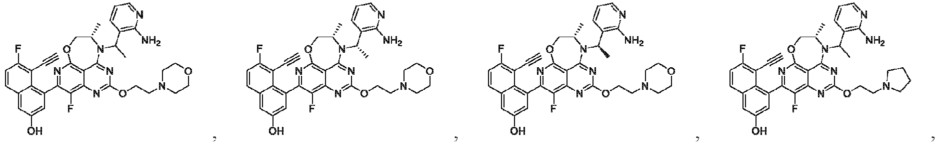

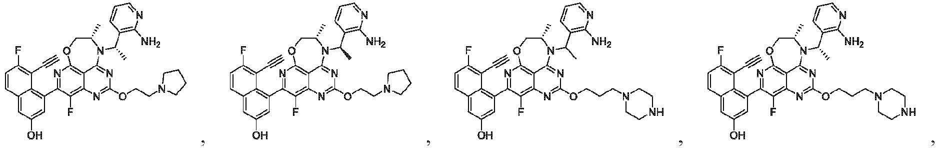

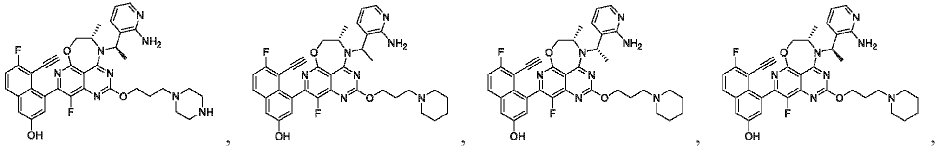

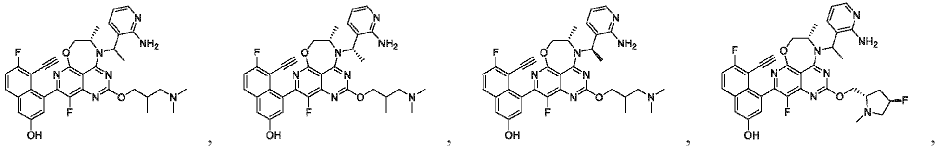

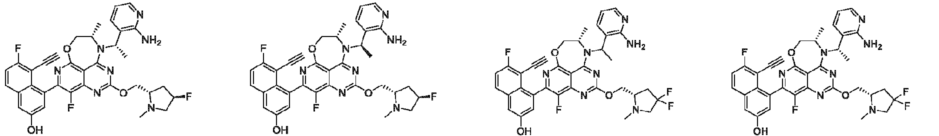

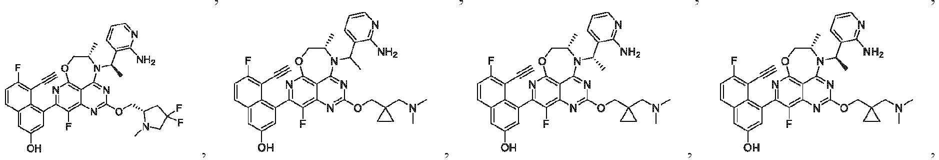

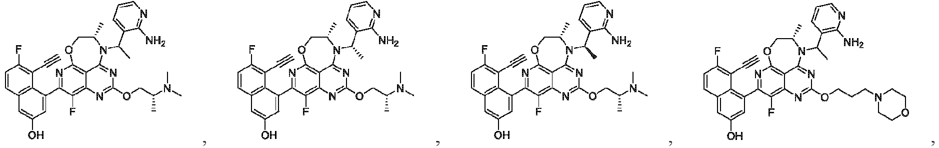

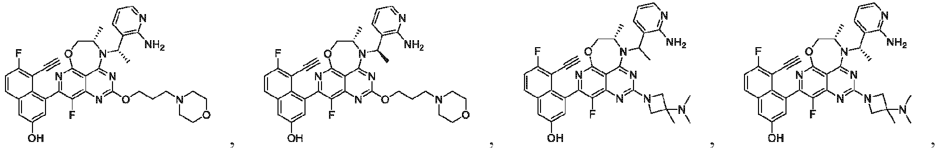

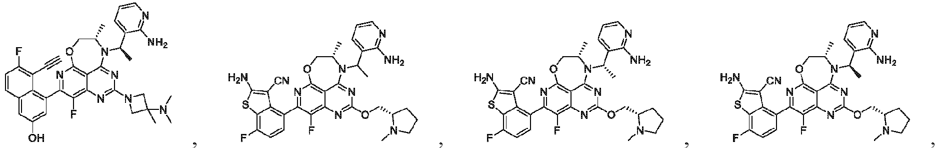

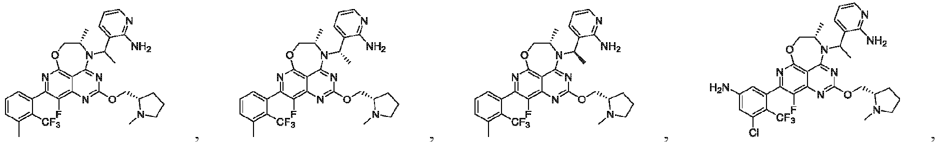

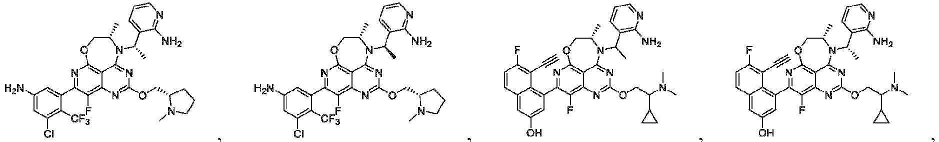

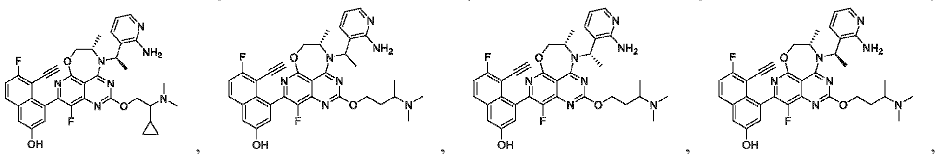

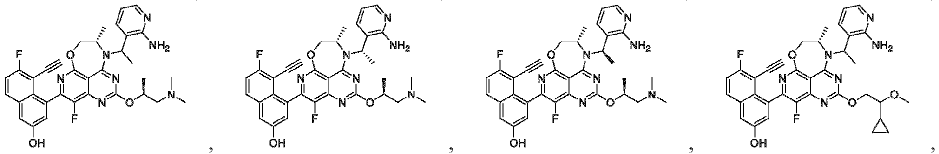

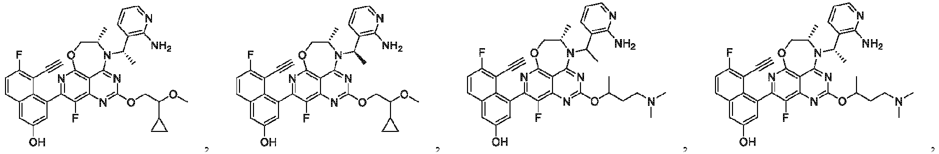

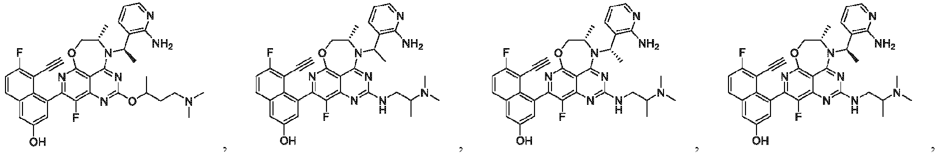

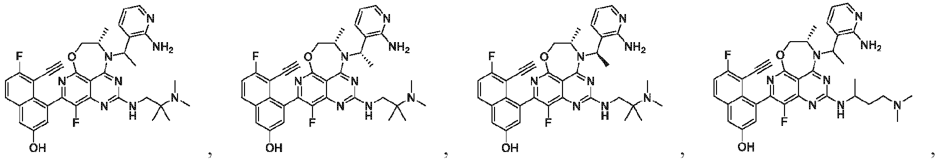

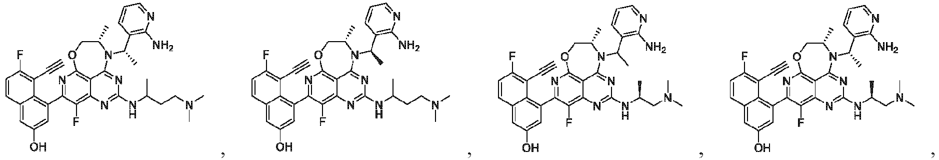

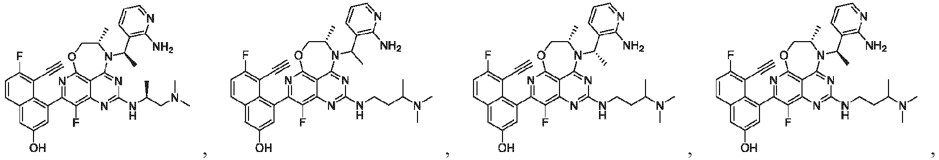

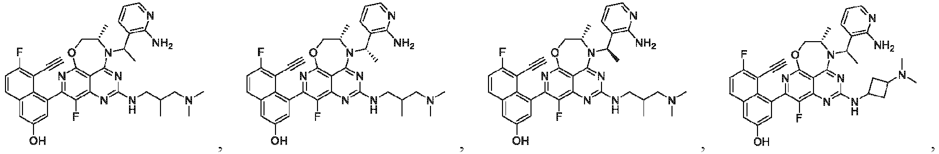

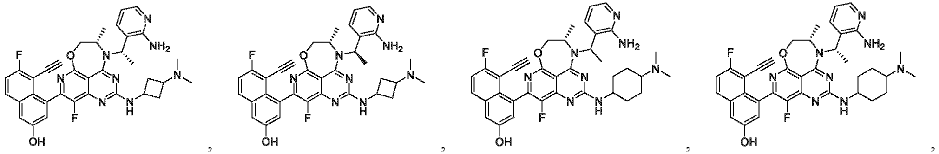

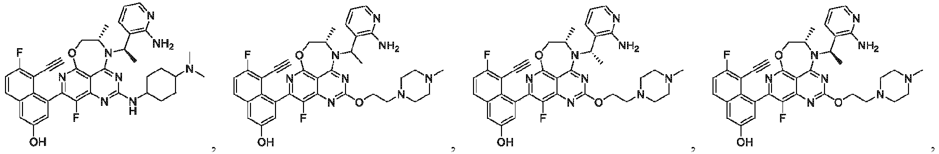

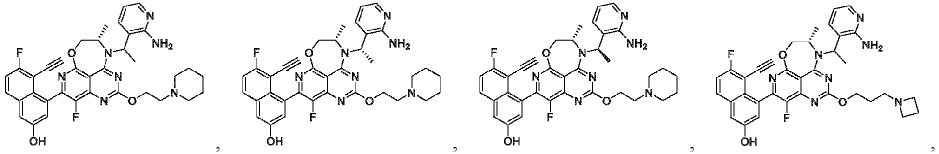

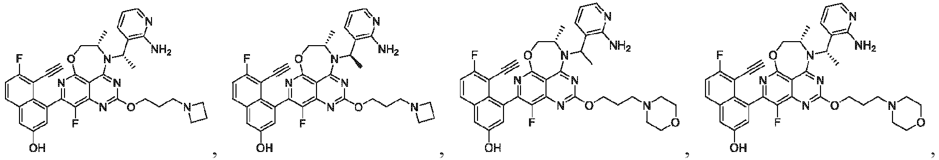

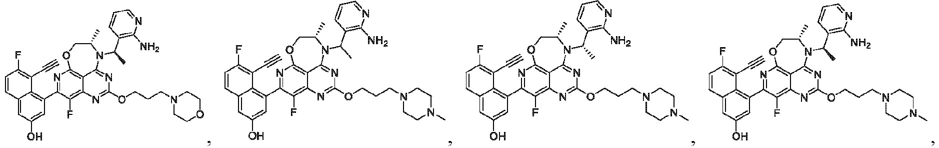

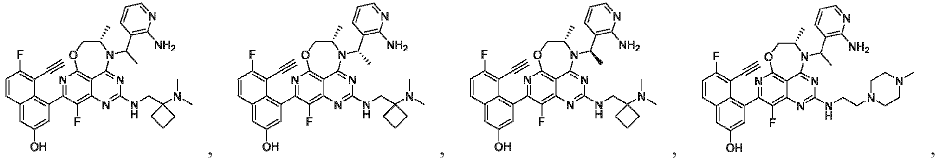

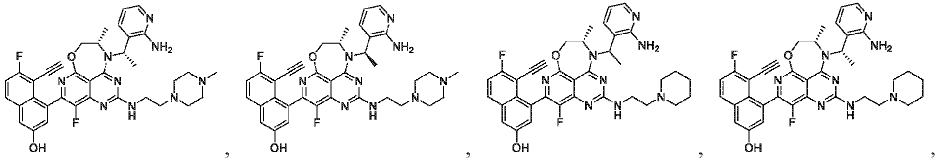

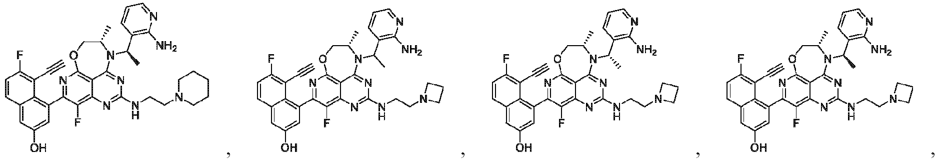

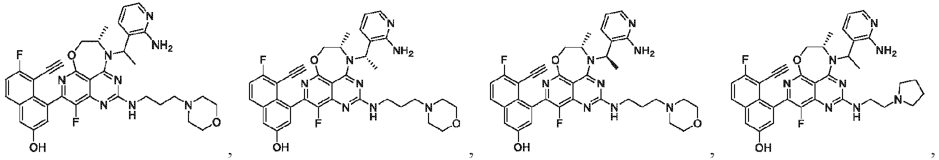

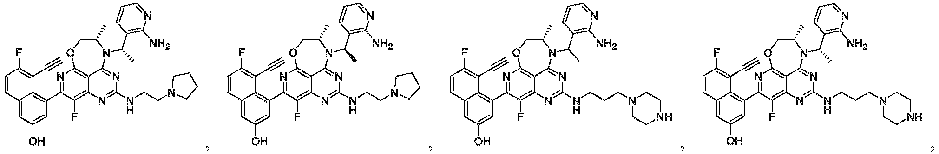

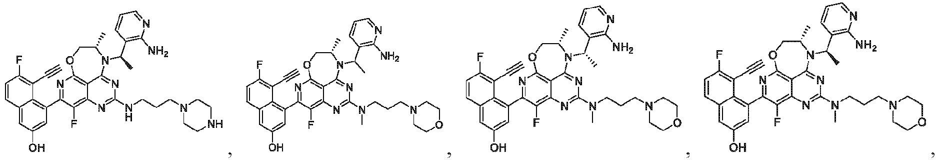

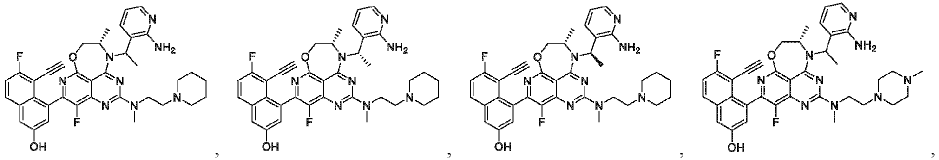

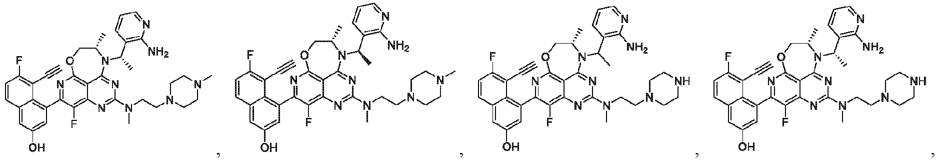

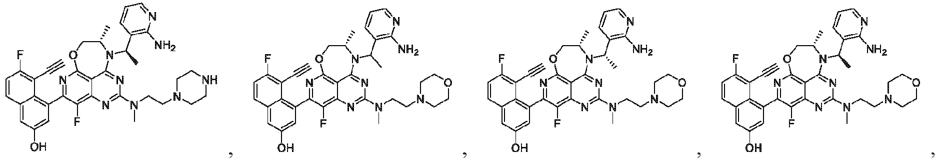

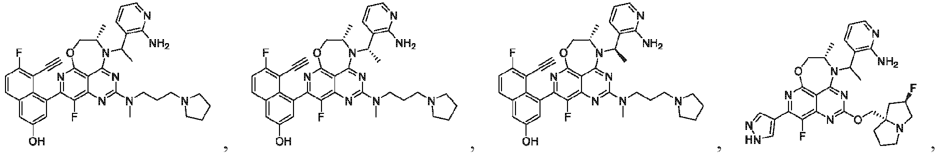

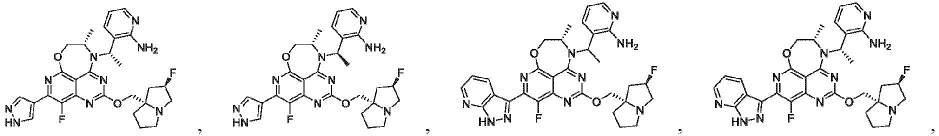

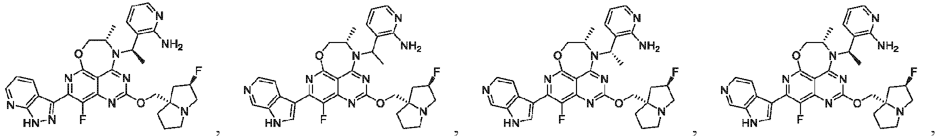

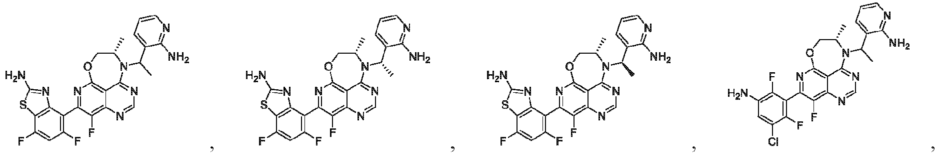

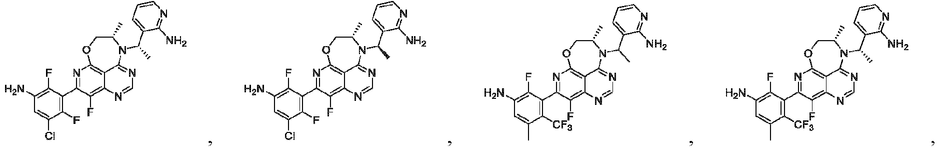

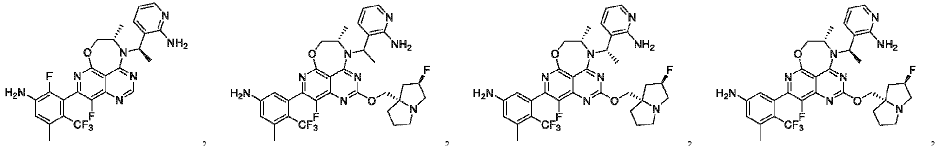

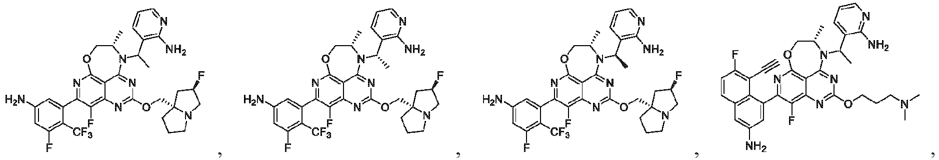

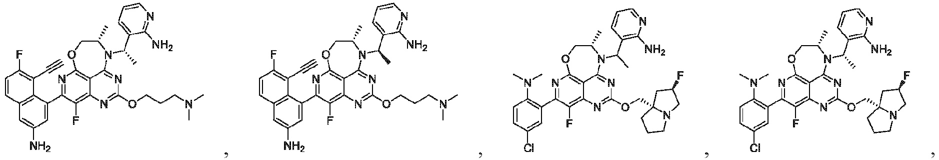

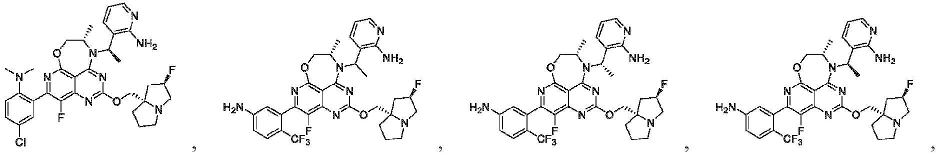

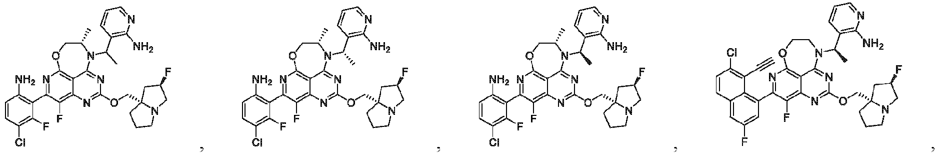

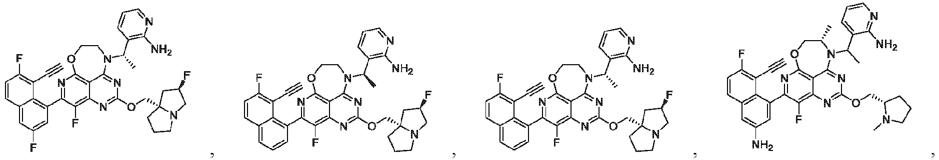

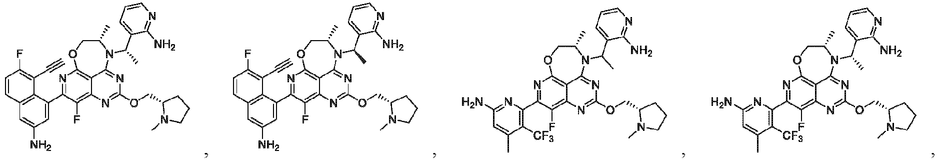

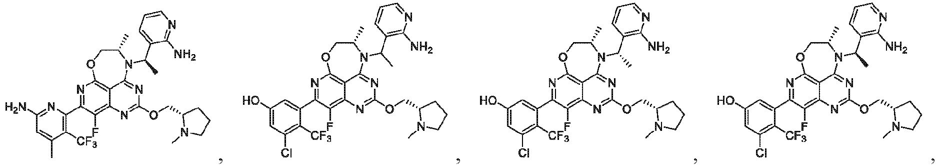

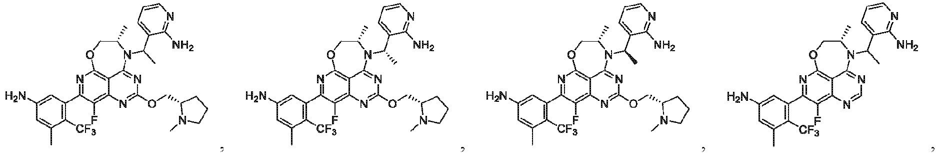

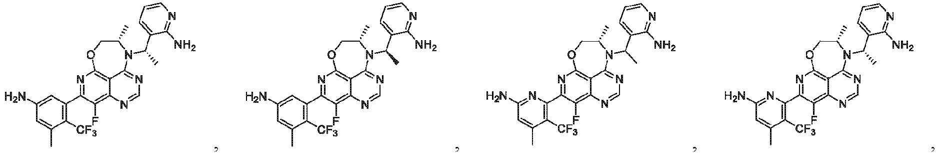

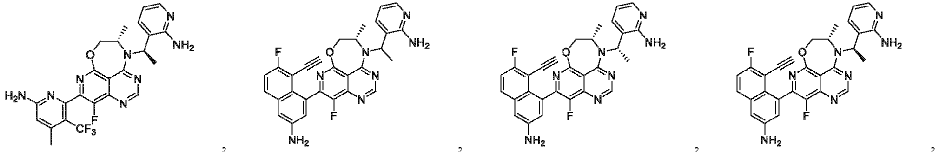

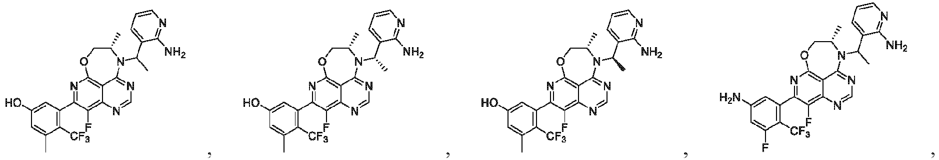

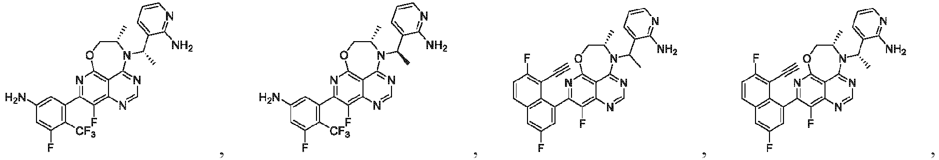

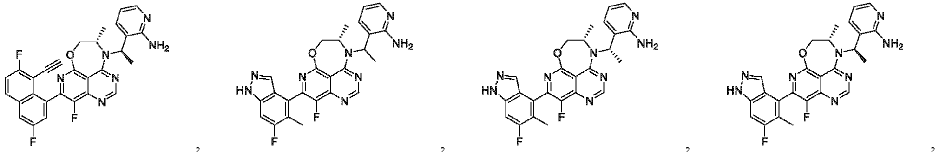

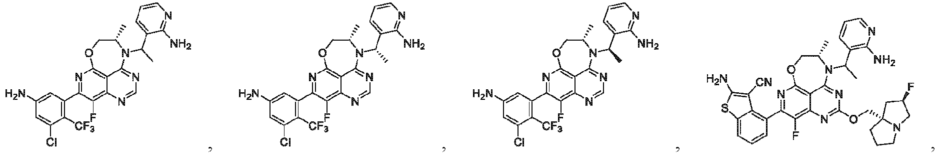

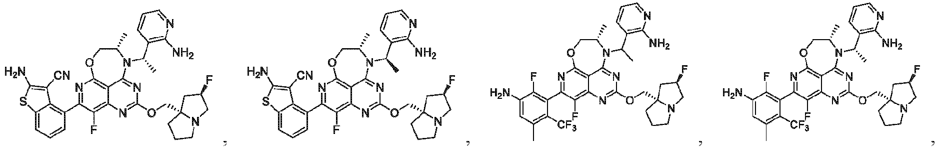

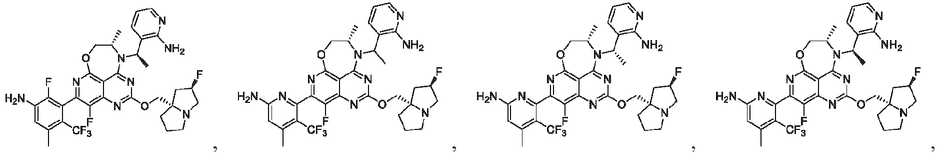

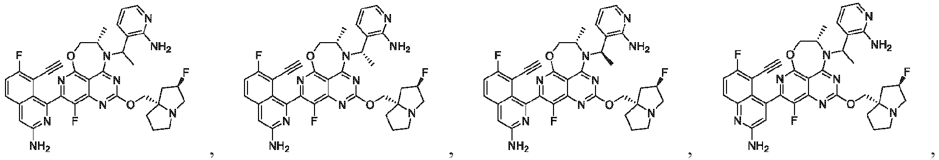

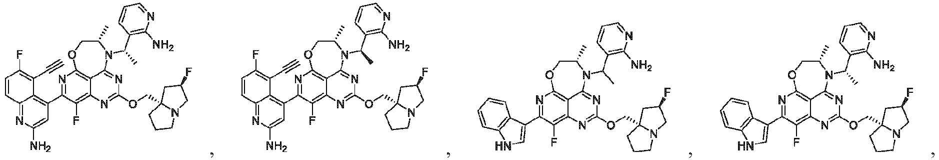

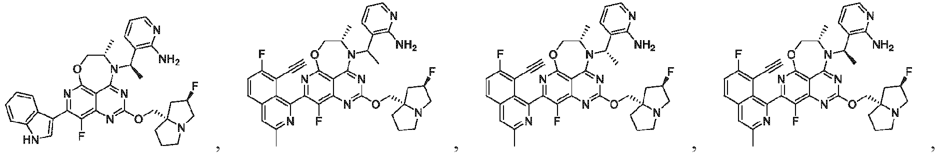

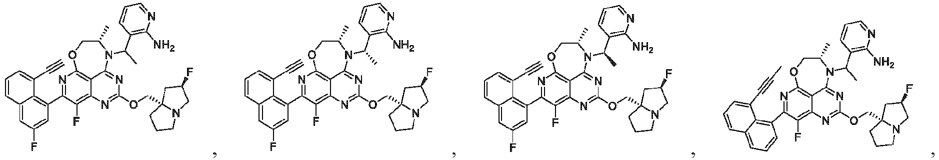

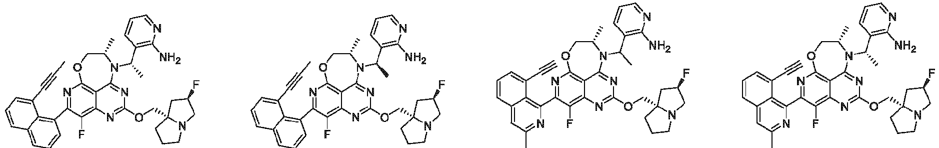

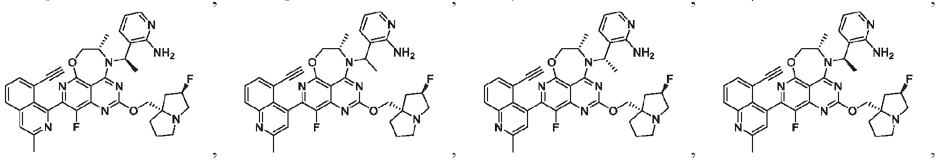

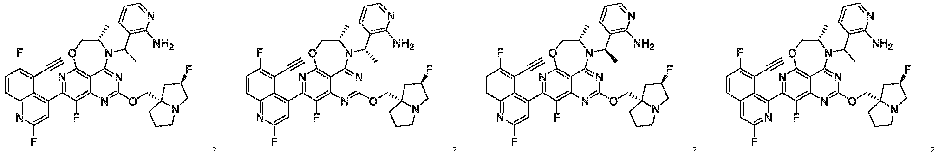

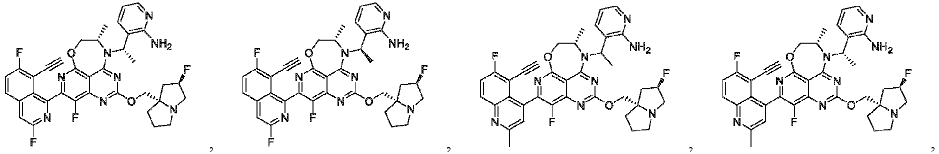

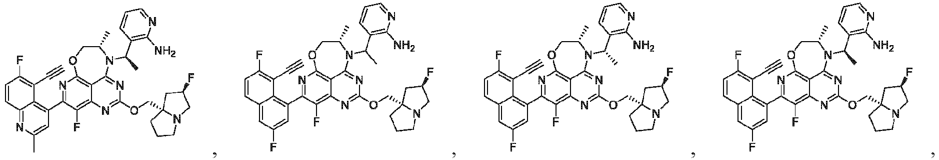

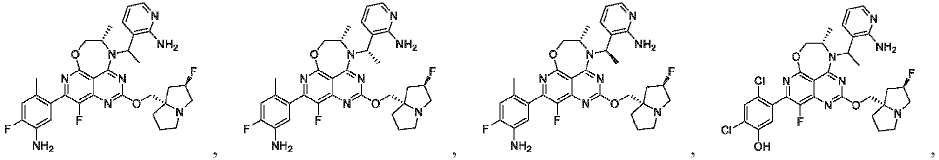

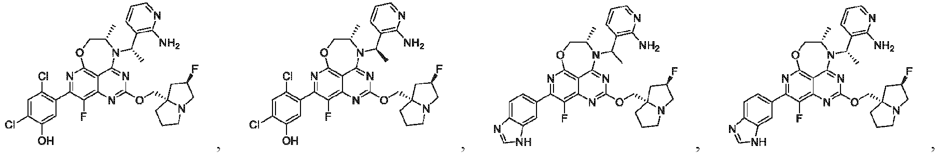

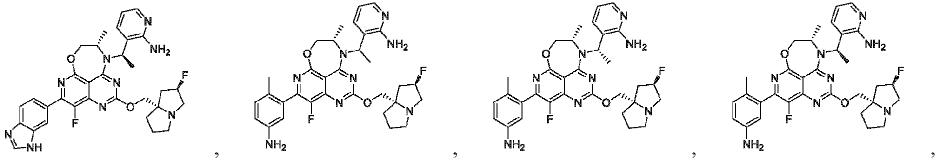

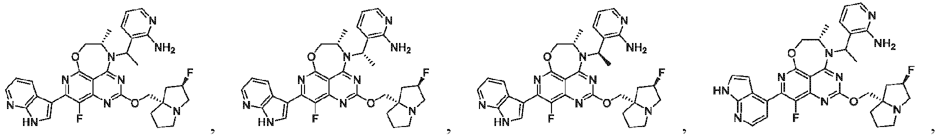

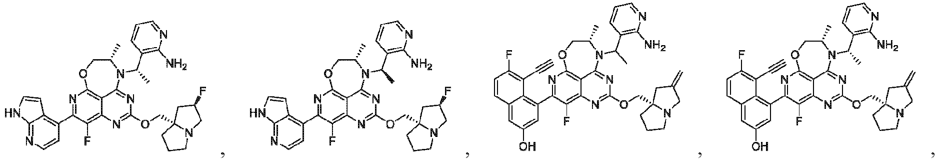

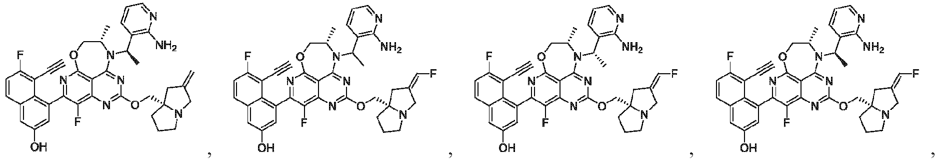

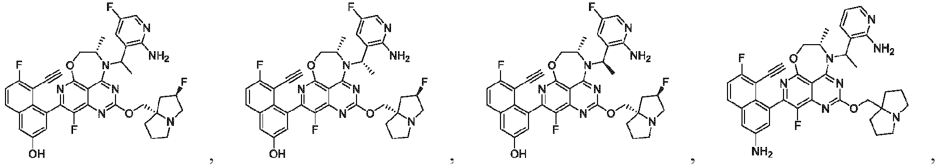

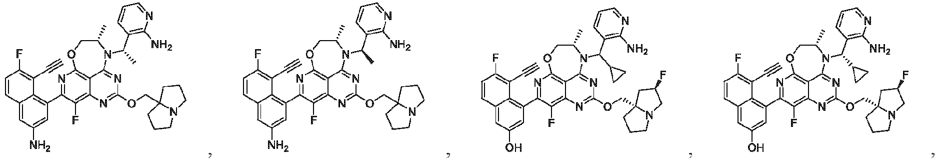

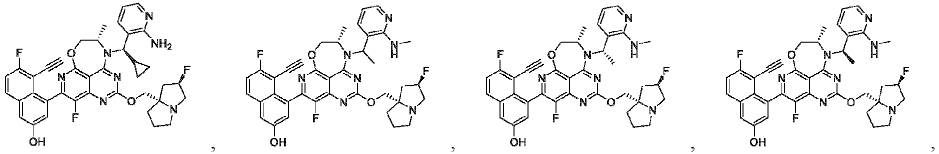

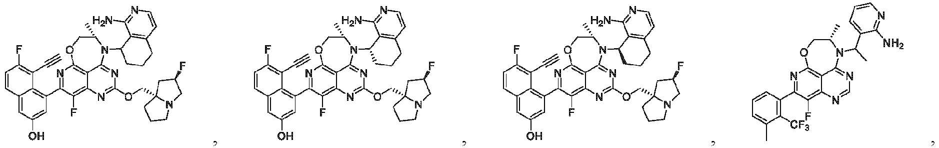

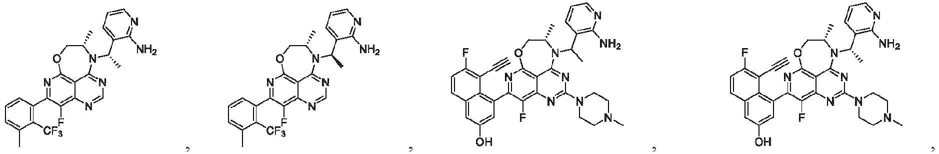

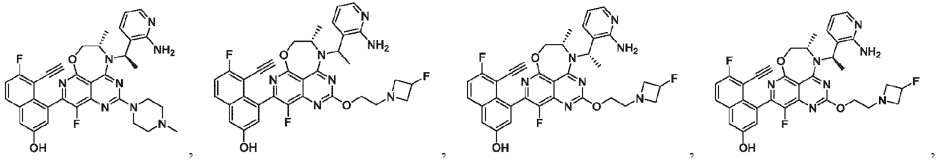

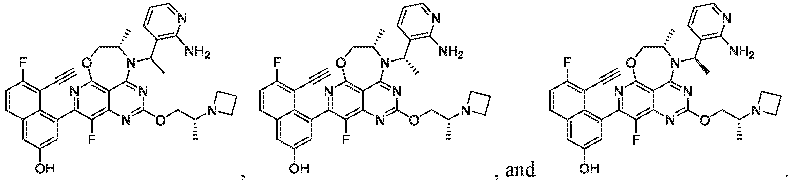

13. A pharmaceutical composition, comprising the compound of formula (I), the stereoisomer thereof, the tautomer thereof, the deuterated compound thereof, or the pharmaceutically acceptable salt thereof according to any one of claims 1-12, and optionally further comprising a pharmaceutically acceptable excipient.

14. Use of the compound of formula (I), the stereoisomer thereof, the tautomer thereof, the deuterated compound thereof, or the pharmaceutically acceptable salt thereof according to any one of claims 1-12, or the pharmaceutical composition according to claim 13 in the manufacture of a medicament for treating a disease.
